(19) Europäisches Patentamt
European Patent Office
Office européen des brevets

(11) EP 3 590 956 A1

(12) EUROPEAN PATENT APPLICATION

(43) Date of publication:
08.01.2020 Bulletin 2020/02

(21) Application number: 19175007.4

(22) Date of filing: 11.06.2009

(51) Int Cl.:
*C07K 14/195* (2006.01) *A61P 35/00* (2006.01)
*C12N 9/52* (2006.01) *A61K 38/48* (2006.01)
*C07K 1/00* (2006.01)

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **12.06.2008 GB 0810785**
**12.06.2008 GB 0810782**
**14.11.2008 GB 0820884**
**17.11.2008 GB 0820965**

(62) Document number(s) of the earlier application(s) in accordance with Art. 76 EPC:
**13179177.4 / 2 719 392**
**09762018.1 / 2 310 028**

(71) Applicant: **Ipsen Bioinnovation Limited**
**Abingdon, Oxfordshire OX14 4RY (GB)**

(72) Inventors:
- **JOHNSTONE, Stephen**
  **Abingdon, Oxfordshire OX14 4RY (GB)**
- **MARKS, Philip**
  **Abingdon, Oxfordshire OX14 4RY (GB)**
- **FOSTER, Keith**
  **Abingdon, Oxfordshire OX14 4RY (GB)**

(74) Representative: **Hobson, David James et al**
**Mathys & Squire LLP**
**The Shard**
**32 London Bridge Street**
**London SE1 9SG (GB)**

Remarks:
•This application was filed on 16.05.2019 as a divisional application to the application mentioned under INID code 62.
•Claims filed after the date of receipt of the divisional application (Rule 68(4) EPC).

# (54) SUPPRESSION OF NEUROENDOCRINE DISEASES

(57) The present invention relates to a method for suppressing neuroendocrine disease. The therapy employs use of a non-cytotoxic protease, which is targeted to a neuroendocrine tumour cell, preferably via a somatostatin or cortistatin receptor, a GHRH receptor, a ghrelin receptor, a bombesin receptor, a urotensin receptor a melanin-concentrating hormone receptor 1; a KiSS-1 receptor or a prolactin-releasing peptide receptor. When so delivered, the protease is internalised and inhibits secretion from said tumour cell. The present invention also relates to polypeptides and nucleic acids for use in said methods.

Figure 3a

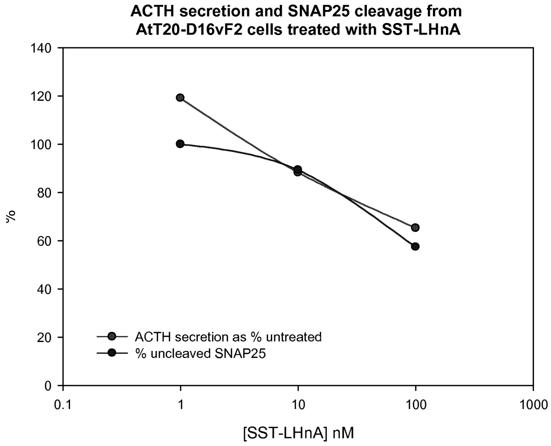


EP 3 590 956 A1

Figure 3b

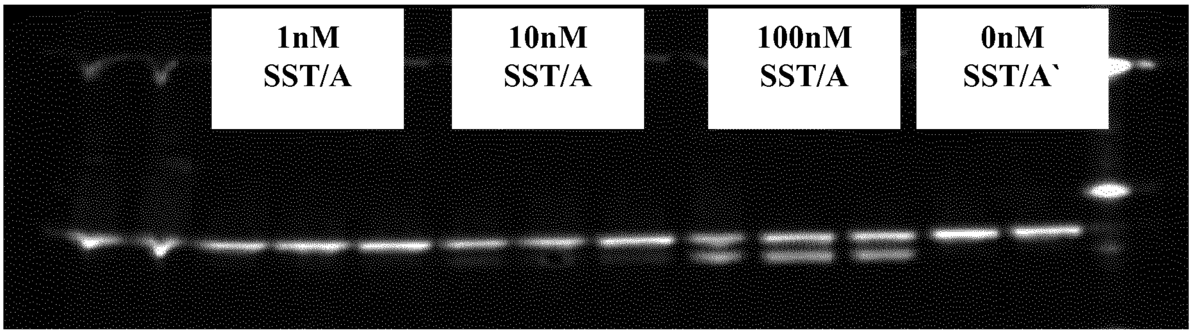

SEQUENCE LISTING

<110> IPSEN BIOINNOVATION LIMITED

<120> SUPPRESSION OF NEUROENDOCRINE DISEASES

<130> P30900EP-D2-PCT

<140> Not Yet Assigned
<141> 2009-06-11

<150> GB 0810785.6
<151> 2008-06-12

<150> GB 0810782.3
<151> 2008-06-12

<160> 206

<170> PatentIn version 3.5

<210> 1
<211> 2611
<212> DNA
<213> Artificial Sequence

<220>
<221> source
<223> /note="Description of Artificial Sequence: Synthetic polynucleotide"

<400> 1
ggatccatgg agttcgttaa caaacagttc aactataaag acccagttaa cggtgttgac 60

attgcttaca tcaaaatccc gaacgctggc cagatgcagc cggtaaaggc attcaaaatc 120

cacaacaaaa tctgggttat cccggaacgt gataccttta ctaacccgga agaaggtgac 180

ctgaacccgc caccggaagc gaaacaggtg ccggtatctt actatgactc cacctacctg 240

tctaccgata acgaaaagga caactacctg aaaggtgtta ctaaactgtt cgagcgtatt 300

tactccaccg acctgggccg tatgctgctg actagcatcg ttcgcggtat cccgttctgg 360

ggcggttcta ccatcgatac cgaactgaaa gtaatcgaca ctaactgcat caacgttatt 420

cagccggacg gttcctatcg ttccgaagaa ctgaacctgg tgatcatcgg cccgtctgct 480

gatatcatcc agttcgagtg tctgagcttt ggtcacgaag ttctgaacct cacccgtaac 540

ggctacggtt ccactcagta catccgtttc tctccggact tcaccttcgg ttttgaagaa 600

tccctggaag tagacacgaa cccactgctg ggcgctggta aattcgcaac tgatcctgcg 660

gttaccctgg ctcacgaact gattcatgca ggccaccgcc tgtacggtat cgccatcaat 720

ccgaaccgtg tcttcaaagt taacaccaac gcgtattacg agatgtccgg tctggaagtt 780

agcttcgaag aactgcgtac ttttggcggt cacgacgcta aattcatcga ctctctgcaa 840

gaaaacgagt tccgtctgta ctactataac aagttcaaag atatcgcatc caccctgaac 900

aaagcgaaat ccatcgtggg taccactgct tctctccagt acatgaagaa cgtttttaaa 960

gaaaaatacc tgctcagcga agacacctcc ggcaaattct ctgtagacaa gttgaaattc 1020

gataaacttt acaaaatgct gactgaaatt tacaccgaag acaacttcgt taagttcttt 1080

aaagttctga accgcaaaac ctatctgaac ttcgacaagg cagtattcaa aatcaacatc 1140

gtgccgaaag ttaactacac tatctacgat ggtttcaacc tgcgtaacac caacctggct 1200

gctaatttta acggccagaa cacggaaatc aacaacatga acttcacaaa actgaaaaac 1260

ttcactggtc tgttcgagtt ttacaagctg ctgtgcgtcg acggcatcat tacctccaaa 1320

actaaatctg acgatgacga taaaaacaaa gcgctgaacc tgcagtgtat caaggttaac 1380

aactgggatt tattcttcag cccgagtgaa gacaacttca ccaacgacct gaacaaaggt 1440

gaagaaatca cctcagatac taacatcgaa gcagccgaag aaaacatctc gctggacctg 1500

atccagcagt actacctgac ctttaatttc gacaacgagc cggaaaacat ttctatcgaa 1560

aacctgagct ctgatatcat cggccagctg gaactgatgc cgaacatcga acgtttccca 1620

aacggtaaaa agtacgagct ggacaaatat accatgttcc actacctgcg cgcgcaggaa 1680

tttgaacacg gcaaatcccg tatcgcactg actaactccg ttaacgaagc tctgctcaac 1740

ccgtcccgtg tatacacctt cttctctagc gactacgtga aaaaggtcaa caaagcgact 1800

gaagctgcaa tgttcttggg ttgggttgaa cagcttgttt atgattttac cgacgagacg 1860

tccgaagtat ctactaccga caaaattgcg gatatcacta tcatcatccc gtacatcggt 1920

ccggctctga acattggcaa catgctgtac aaagacgact tcgttggcgc actgatcttc 1980

tccggtgcgg tgatcctgct ggagttcatc ccggaaatcg ccatcccggt actgggcacc 2040

tttgctctgg tttcttacat tgcaaacaag gttctgactg tacaaaccat cgacaacgcg 2100

ctgagcaaac gtaacgaaaa atgggatgaa gtttacaaat atatcgtgac caactggctg 2160

gctaaggtta atactcagat cgacctcatc cgcaaaaaaa tgaaagaagc actggaaaac 2220

caggcggaag ctaccaaggc aatcattaac taccagtaca accagtacac cgaggaagaa 2280

aaaaacaaca tcaacttcaa catcgacgat ctgtcctcta aactgaacga atccatcaac 2340

aaagctatga tcaacatcaa caagttcctg aaccagtgct ctgtaagcta tctgatgaac 2400

tccatgatcc cgtacggtgt taaacgtctg gaggacttcg atgcgtctct gaaagacgcc 2460

ctgctgaaat acatttacga caaccgtggc actctgatcg gtcaggttga tcgtctgaag 2520

gacaaagtga acaatacctt atcgaccgac atccctttc agctcagtaa atatgtcgat 2580

aaccaacgcc ttttgtccac ttaataagct t 2611

<210> 2
<211> 2635
<212> DNA
<213> Artificial Sequence

<220>
<221> source
<223> /note="Description of Artificial Sequence: Synthetic
      polynucleotide"

<400> 2
ggatccatgc cggttaccat caacaacttc aactacaacg acccgatcga caacaacaac 60

atcattatga tggaaccgcc gttcgcacgt ggtaccggac gttactacaa ggcttttaag 120

atcaccgacc gtatctggat catcccggaa cgttacacct tcggttacaa acctgaggac 180

ttcaacaaga gtagcgggat tttcaatcgt gacgtctgcg agtactatga tccagattat 240

ctgaatacca acgataagaa gaacatattc cttcagacta tgattaaact cttcaaccgt 300

atcaaaagca aaccgctcgg tgaaaaactc ctcgaaatga ttatcaacgg tatcccgtac 360

ctcggtgacc gtcgtgtccc gcttgaagag ttcaacacca acatcgcaag cgtcaccgtc 420

aacaaactca tcagcaaccc aggtgaagtc gaacgtaaaa aaggtatctt cgcaaacctc 480

atcatcttcg gtccgggtcc ggtcctcaac gaaaacgaaa ccatcgacat cggtatccag 540

aaccacttcg caagccgtga aggtttcggt ggtatcatgc agatgaaatt ctgcccggaa 600

tacgtcagtg tcttcaacaa cgtccaggaa aacaaaggtg caagcatctt caaccgtcgt 660

ggttacttca gcgacccggc actcatcctc atgcatgaac tcatccacgt cctccacggt 720

ctctacggta tcaaagttga cgacctcccg atcgtcccga acgagaagaa attcttcatg 780

cagagcaccg acgcaatcca ggctgaggaa ctctacacct tcggtggcca agacccaagt 840

atcataaccc cgtccaccga caaaagcatc tacgacaaag tcctccagaa cttcaggggt 900

atcgtggaca gactcaacaa agtcctcgtc tgcatcagcg acccgaacat caatatcaac 960

atatacaaga acaagttcaa agacaagtac aaattcgtcg aggacagcga aggcaaatac 1020

agcatcgacg tagaaagttt cgacaagctc tacaaaagcc tcatgttcgg tttcaccgaa 1080

accaacatcg ccgagaacta caagatcaag acaagggcaa gttacttcag cgacagcctc 1140

ccgcctgtca aaatcaagaa cctcttagac aacgagattt acacaattga agagggcttc 1200

aacatcagtg acaaagacat ggagaaggaa tacagaggtc agaacaaggc tatcaacaaa 1260

caggcatacg aggagatcag caaagaacac ctcgcagtct acaagatcca gatgtgcgtc 1320

gacggcatca ttacctccaa aactaaatct gacgatgacg ataaaaacaa agcgctgaac 1380

ctgcagtgca tcgacgttga caacgaagac ctgttcttca tcgctgacaa aaacagcttc 1440

agtgacgacc tgagcaaaaa cgaacgtatc gaatacaaca cccagagcaa ctacatcgaa 1500

aacgacttcc cgatcaacga actgatcctg gacaccgacc tgataagtaa aatcgaactg 1560

ccgagcgaaa acaccgaaag tctgaccgac ttcaacgttg acgttccggt ttacgaaaaa 1620

cagccggcta tcaagaaaat cttcaccgac gaaaacacca tcttccagta cctgtacagc 1680

cagaccttcc cgctggacat ccgtgacatc agtctgacca gcagtttcga cgacgctctg 1740

```
ctgttcagca acaaagttta cagtttcttc agcatggact acatcaaaac cgctaacaaa     1800

gttgttgaag cagggctgtt cgctggttgg gttaaacaga tcgttaacga cttcgttatc     1860

gaagctaaca aaagcaacac tatggacaaa atcgctgaca tcagtctgat cgttccgtac     1920

atcggtctgg ctctgaacgt tggtaacgaa accgctaaag gtaactttga aaacgctttc     1980

gagatcgctg gtgcaagcat cctgctggag ttcatcccgg aactgctgat cccggttgtt     2040

ggtgctttcc tgctggaaag ttacatcgac aacaaaaaca agatcatcaa aaccatcgac     2100

aacgctctga ccaaacgtaa cgaaaaatgg agtgatatgt acggtctgat cgttgctcag     2160

tggctgagca ccgtcaacac ccagttctac accatcaaag aaggtatgta caaagctctg     2220

aactaccagg ctcaggctct ggaagagatc atcaaatacc gttacaacat ctacagtgag     2280

aaggaaaaga gtaacatcaa catcgacttc aacgacatca acagcaaact gaacgaaggt     2340

atcaaccagg ctatcgacaa catcaacaac ttcatcaacg gttgcagtgt tagctacctg     2400

atgaagaaga tgatcccgct ggctgttgaa aaactgctgg acttcgacaa caccctgaaa     2460

aagaacctgc tgaactacat cgacgaaaac aagctgtacc tgatcggtag tgctgaatac     2520

gaaaaaagta aagtgaacaa atacctgaag accatcatgc cgttcgacct gagtatctac     2580

accaacgaca ccatcctgat cgaaatgttc aacaaataca actcttaata agctt          2635
```

<210> 3
<211> 2614
<212> DNA
<213> Artificial Sequence

<220>
<221> source
<223> /note="Description of Artificial Sequence: Synthetic polynucleotide"

<400> 3

```
ggatccatgc cgatcaccat caacaacttc aactacagcg atccggtgga taacaaaaac       60

atcctgtacc tggataccca tctgaatacc ctggcgaacg aaccggaaaa agcgtttcgt      120

atcaccggca acatttgggt tattccggat cgttttagcc gtaacagcaa cccgaatctg      180

aataaaccgc cgcgtgttac cagcccgaaa agcggttatt acgatccgaa ctatctgagc      240

accgatagcg ataaagatac cttcctgaaa gaaatcatca aactgttcaa acgcatcaac      300

agccgtgaaa ttggcgaaga actgatctat cgcctgagca ccgatattcc gtttccgggc      360

aacaacaaca ccccgatcaa cacctttgat ttcgatgtgg atttcaacag cgttgatgtt      420

aaaacccgcc agggtaacaa ttgggtgaaa accggcagca ttaacccgag cgtgattatt      480

accggtccgc gcgaaaacat tattgatccg gaaaccagca cctttaaact gaccaacaac      540

acctttgcgg cgcaggaagg ttttggcgcg ctgagcatta ttagcattag cccgcgcttt      600
```

atgctgacct atagcaacgc gaccaacgat gttggtgaag gccgtttcag caaaagcgaa 660
ttttgcatgg acccgatcct gatcctgatg catgaactga accatgcgat gcataacctg 720
tatggcatcg cgattccgaa cgatcagacc attagcagcg tgaccagcaa catcttttac 780
agccagtaca acgtgaaact ggaatatgcg gaaatctatg cgtttggcgg tccgaccatt 840
gatctgattc cgaaaagcgc gcgcaaatac ttcgaagaaa aagcgctgga ttactatcgc 900
agcattgcga aacgtctgaa cagcattacc accgcgaatc cgagcagctt caacaaatat 960
atcggcgaat ataaacagaa actgatccgc aaatatcgct ttgtggtgga aagcagcggc 1020
gaagttaccg ttaaccgcaa taaattcgtg gaactgtaca acgaactgac ccagatcttc 1080
accgaattta actatgcgaa aatctataac gtgcagaacc gtaaaatcta cctgagcaac 1140
gtgtataccc cggtgaccgc gaatattctg gatgataacg tgtacgatat ccagaacggc 1200
tttaacatcc cgaaaagcaa cctgaacgtt ctgtttatgg gccagaacct gagccgtaat 1260
ccggcgctgc gtaaagtgaa cccggaaaac atgctgtacc tgttcaccaa attttgcgtc 1320
gacgcgattg atggtcgtag cctgtacaac aaaaccctgc agtgtcgtga actgctggtg 1380
aaaaacaccg atctgccgtt tattggcgat atcagcgatg tgaaaaccga tatcttcctg 1440
cgcaaagata tcaacgaaga aaccgaagtg atctactacc cggataacgt gagcgttgat 1500
caggtgatcc tgagcaaaaa caccagcgaa catggtcagc tggatctgct gtatccgagc 1560
attgatagcg aaagcgaaat tctgccgggc gaaaaccagg tgttttacga taaccgtacc 1620
cagaacgtgg attacctgaa cagctattac tacctggaaa gccagaaact gagcgataac 1680
gtggaagatt ttacctttac ccgcagcatt gaagaagcgc tggataacag cgcgaaagtt 1740
tacacctatt ttccgaccct ggcgaacaaa gttaatgcgg gtgttcaggg cggtctgttt 1800
ctgatgtggg cgaacgatgt ggtggaagat ttcaccacca acatcctgcg taaagatacc 1860
ctggataaaa tcagcgatgt tagcgcgatt attccgtata ttggtccggc gctgaacatt 1920
agcaatagcg tgcgtcgtgg caattttacc gaagcgtttg cggttaccgg tgtgaccatt 1980
ctgctggaag cgtttccgga atttaccatt ccggcgctgg gtgcgtttgt gatctatagc 2040
aaagtgcagg aacgcaacga aatcatcaaa accatcgata actgcctgga acagcgtatt 2100
aaacgctgga aagatagcta tgaatggatg atgggcacct ggctgagccg tattatcacc 2160
cagttcaaca acatcagcta ccagatgtac gatagcctga actatcaggc gggtgcgatt 2220
aaagcgaaaa tcgatctgga atacaaaaaa tacagcggca gcgataaaga aaacatcaaa 2280
agccaggttg aaaacctgaa aaacagcctg gatgtgaaaa ttagcgaagc gatgaataac 2340
atcaacaaat tcatccgcga atgcagcgtg acctacctgt tcaaaaacat gctgccgaaa 2400
gtgatcgatg aactgaacga atttgatcgc aacaccaaag cgaaactgat caacctgatc 2460
gatagccaca acattattct ggtgggcgaa gtggataaac tgaaagcgaa agttaacaac 2520

agcttccaga acaccatccc gtttaacatc ttcagctata ccaacaacag cctgctgaaa 2580

gatatcatca acgaatactt caattaataa gctt 2614

<210> 4
<211> 2632
<212> DNA
<213> Artificial Sequence

<220>
<221> source
<223> /note="Description of Artificial Sequence: Synthetic polynucleotide"

<400> 4
ggatccatga cgtggccagt taaggatttc aactactcag atcctgtaaa tgacaacgat 60

attctgtacc ttcgcattcc acaaaataaa ctgatcacca caccagtcaa agcattcatg 120

attactcaaa acatttgggt cattccagaa cgcttttcta gtgacacaaa tccgagttta 180

tctaaacctc cgcgtccgac gtccaaatat cagagctatt acgatccctc atatctcagt 240

acggacgaac aaaaagatac tttccttaaa ggtatcatta aactgtttaa gcgtattaat 300

gagcgcgata tcgggaaaaa gttgattaat tatcttgttg tgggttcccc gttcatgggc 360

gatagctcta cccccgaaga cacttttgat tttacccgtc atacgacaaa catcgcggta 420

gagaagtttg agaacggatc gtggaaagtc acaaacatca ttacacctag cgtcttaatt 480

tttggtccgc tgccaaacat cttagattat acagccagcc tgactttgca ggggcaacag 540

tcgaatccga gtttcgaagg ttttggtacc ctgagcattc tgaaagttgc cccggaattt 600

ctgctcactt tttcagatgt caccagcaac cagagctcag cagtattagg aaagtcaatt 660

ttttgcatgg acccggttat tgcactgatg cacgaactga cgcactctct gcatcaactg 720

tatgggatca acatccccag tgacaaacgt attcgtcccc aggtgtctga aggatttttc 780

tcacaggatg ggccgaacgt ccagttcgaa gagttgtata ctttcggagg cctggacgta 840

gagatcattc cccagattga gcgcagtcag ctgcgtgaga aggcattggg ccattataag 900

gatattgcaa aacgcctgaa taacattaac aaaacgattc catcttcgtg gatctcgaat 960

attgataaat ataagaaaat ttttagcgag aaatataatt ttgataaaga taatacaggt 1020

aactttgtgg ttaacattga caaattcaac tccctttaca gtgatttgac gaatgtaatg 1080

agcgaagttg tgtatagttc ccaatacaac gttaagaatc gtacccatta cttctctcgt 1140

cactacctgc cggttttcgc gaacatcctt gacgataata tttacactat tcgtgacggc 1200

tttaacttga ccaacaaggg cttcaatatt gaaaattcag gccagaacat tgaacgcaac 1260

ccggccttgc agaaactgtc gagtgaatcc gtggttgacc tgtttaccaa agtctgcgtc 1320

gacaaaagcg aagagaagct gtacgatgac gatgacaaag atcgttgggg atcgtccctg 1380

cagtgtatta aagtgaaaaa caatcggctg ccttatgtag cagataaaga tagcattagt 1440

caggagattt tcgaaaataa aattatcact gacgaaacca atgttcagaa ttattcagat 1500

aaattttcac tggacgaaag catcttagat ggccaagttc cgattaaccc ggaaattgtt 1560

gatccgttac tgccgaacgt gaatatggaa ccgttaaacc tccctggcga agagatcgta 1620

ttttatgatg acattacgaa atatgtggac taccttaatt cttattacta tttggaaagc 1680

cagaaactgt ccaataacgt ggaaaacatt actctgacca caagcgtgga agaggcttta 1740

ggctactcaa ataagattta taccttcctc ccgtcgctgg cggaaaaagt aaataaaggt 1800

gtgcaggctg gtctgttcct caactgggcg aatgaagttg tcgaagactt taccacgaat 1860

attatgaaaa aggataccct ggataaaatc tccgacgtct cggttattat cccatatatt 1920

ggccctgcgt taaatatcgg taatagtgcg ctgcggggga attttaacca ggcctttgct 1980

accgcgggcg tcgcgttcct cctggagggc tttcctgaat ttactatccc ggcgctcggt 2040

gtttttacat tttactcttc catccaggag cgtgagaaaa ttatcaaaac catcgaaaac 2100

tgcctggagc agcgggtgaa acgctggaaa gattcttatc aatggatggt gtcaaactgg 2160

ttatctcgca tcacgaccca attcaaccat attaattacc agatgtatga tagtctgtcg 2220

taccaagctg acgccattaa agccaaaatt gatctggaat ataaaaagta ctctggtagc 2280

gataaggaga acatcaaaag ccaggtggag aaccttaaga atagtctgga tgtgaaaatc 2340

tctgaagcta tgaataacat taacaaattc attcgtgaat gttcggtgac gtacctgttc 2400

aagaatatgc tgccaaaagt tattgatgaa ctgaataaat ttgatctgcg taccaaaacc 2460

gaacttatca acctcatcga ctcccacaac attatccttg tgggcgaagt ggatcgtctg 2520

aaggccaaag taaacgagag ctttgaaaat acgatgccgt ttaatatttt ttcatatacc 2580

aataactcct tgctgaaaga tatcatcaat gaatatttca attaataagc tt 2632

<210> 5
<211> 240
<212> DNA
<213> Artificial Sequence

<220>
<221> source
<223> /note="Description of Artificial Sequence: Synthetic polynucleotide"

<400> 5
catatgggat ccggtttaaa cgtcgacggc atcattacct ccaaaactaa atctgacgat 60

gacgataaaa gcgccaattc aaatcctgca atggcgccac gcgaacgcaa agctggttgc 120

aaaaacttct tctggaaaac cttcacctct tgcgcgctag cgggcggtgg cggtagcggc 180

ggtggcggta gcggcggtgg cggtagcgca ctagtgctgc agctagaata atgaaagctt 240

<210> 6
<211> 195
<212> DNA
<213> Artificial Sequence

<220>
<221> source
<223> /note="Description of Artificial Sequence: Synthetic polynucleotide"

<400> 6
ggatccgtcg acctgcaggg tctagaaggc ggtggcggta gcggcggtgg cggtagcggc 60

ggtggcggta gcggcggtgg cggtagcgca ctagtgcagg aaagacctcc attacaacaa 120

cctccacatc gcgataagaa accatgtaag aatttctttt ggaaaacatt tagcagttgc 180

aaatgataaa agctt 195

<210> 7
<211> 902
<212> PRT
<213> Artificial Sequence

<220>
<221> source
<223> /note="Description of Artificial Sequence: Synthetic polypeptide"

<400> 7
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
    130                 135                 140

Trp Lys Val Thr Asn Ile Ile Thr Pro Ser Val Leu Ile Phe Gly Pro
145                 150                 155                 160

Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
225                 230                 235                 240

Asn Ile Pro Ser Asp Lys Arg Ile Arg Pro Gln Val Ser Glu Gly Phe
                245                 250                 255

Phe Ser Gln Asp Gly Pro Asn Val Gln Phe Glu Glu Leu Tyr Thr Phe
            260                 265                 270

Gly Gly Leu Asp Val Glu Ile Ile Pro Gln Ile Glu Arg Ser Gln Leu
        275                 280                 285

Arg Glu Lys Ala Leu Gly His Tyr Lys Asp Ile Ala Lys Arg Leu Asn
    290                 295                 300

Asn Ile Asn Lys Thr Ile Pro Ser Ser Trp Ile Ser Asn Ile Asp Lys
305                 310                 315                 320

Tyr Lys Lys Ile Phe Ser Glu Lys Tyr Asn Phe Asp Lys Asp Asn Thr
                325                 330                 335

Gly Asn Phe Val Val Asn Ile Asp Lys Phe Asn Ser Leu Tyr Ser Asp
            340                 345                 350

Leu Thr Asn Val Met Ser Glu Val Val Tyr Ser Ser Gln Tyr Asn Val
        355                 360                 365

Lys Asn Arg Thr His Tyr Phe Ser Arg His Tyr Leu Pro Val Phe Ala
    370                 375                 380

```
Asn Ile Leu Asp Asp Asn Ile Tyr Thr Ile Arg Asp Gly Phe Asn Leu
385                 390                 395                 400

Thr Asn Lys Gly Phe Asn Ile Glu Asn Ser Gly Gln Asn Ile Glu Arg
                405                 410                 415

Asn Pro Ala Leu Gln Lys Leu Ser Ser Glu Ser Val Val Asp Leu Phe
            420                 425                 430

Thr Lys Val Cys Val Asp Gly Ile Ile Thr Ser Lys Thr Lys Ser Asp
        435                 440                 445

Asp Asp Asp Lys Pro Cys Lys Asn Phe Phe Trp Lys Thr Phe Ser Ser
    450                 455                 460

Cys Lys Ala Leu Ala Gly Gly Gly Gly Ser Gly Gly Gly Gly Ser Gly
465                 470                 475                 480

Gly Gly Gly Ser Ala Leu Val Leu Gln Cys Ile Lys Val Lys Asn Asn
                485                 490                 495

Arg Leu Pro Tyr Val Ala Asp Lys Asp Ser Ile Ser Gln Glu Ile Phe
            500                 505                 510

Glu Asn Lys Ile Ile Thr Asp Glu Thr Asn Val Gln Asn Tyr Ser Asp
        515                 520                 525

Lys Phe Ser Leu Asp Glu Ser Ile Leu Asp Gly Gln Val Pro Ile Asn
    530                 535                 540

Pro Glu Ile Val Asp Pro Leu Leu Pro Asn Val Asn Met Glu Pro Leu
545                 550                 555                 560

Asn Leu Pro Gly Glu Glu Ile Val Phe Tyr Asp Asp Ile Thr Lys Tyr
                565                 570                 575

Val Asp Tyr Leu Asn Ser Tyr Tyr Tyr Leu Glu Ser Gln Lys Leu Ser
            580                 585                 590

Asn Asn Val Glu Asn Ile Thr Leu Thr Thr Ser Val Glu Glu Ala Leu
        595                 600                 605

Gly Tyr Ser Asn Lys Ile Tyr Thr Phe Leu Pro Ser Leu Ala Glu Lys
    610                 615                 620

Val Asn Lys Gly Val Gln Ala Gly Leu Phe Leu Asn Trp Ala Asn Glu
625                 630                 635                 640
```

```
Val Val Glu Asp Phe Thr Thr Asn Ile Met Lys Lys Asp Thr Leu Asp
                645                 650                 655

Lys Ile Ser Asp Val Ser Val Ile Ile Pro Tyr Ile Gly Pro Ala Leu
            660                 665                 670

Asn Ile Gly Asn Ser Ala Leu Arg Gly Asn Phe Asn Gln Ala Phe Ala
        675                 680                 685

Thr Ala Gly Val Ala Phe Leu Leu Glu Gly Phe Pro Glu Phe Thr Ile
    690                 695                 700

Pro Ala Leu Gly Val Phe Thr Phe Tyr Ser Ser Ile Gln Glu Arg Glu
705                 710                 715                 720

Lys Ile Ile Lys Thr Ile Glu Asn Cys Leu Glu Gln Arg Val Lys Arg
                725                 730                 735

Trp Lys Asp Ser Tyr Gln Trp Met Val Ser Asn Trp Leu Ser Arg Ile
            740                 745                 750

Thr Thr Gln Phe Asn His Ile Asn Tyr Gln Met Tyr Asp Ser Leu Ser
        755                 760                 765

Tyr Gln Ala Asp Ala Ile Lys Ala Lys Ile Asp Leu Glu Tyr Lys Lys
    770                 775                 780

Tyr Ser Gly Ser Asp Lys Glu Asn Ile Lys Ser Gln Val Glu Asn Leu
785                 790                 795                 800

Lys Asn Ser Leu Asp Val Lys Ile Ser Glu Ala Met Asn Asn Ile Asn
                805                 810                 815

Lys Phe Ile Arg Glu Cys Ser Val Thr Tyr Leu Phe Lys Asn Met Leu
            820                 825                 830

Pro Lys Val Ile Asp Glu Leu Asn Lys Phe Asp Leu Arg Thr Lys Thr
        835                 840                 845

Glu Leu Ile Asn Leu Ile Asp Ser His Asn Ile Ile Leu Val Gly Glu
    850                 855                 860

Val Asp Arg Leu Lys Ala Lys Val Asn Glu Ser Phe Glu Asn Thr Met
865                 870                 875                 880

Pro Phe Asn Ile Phe Ser Tyr Thr Asn Asn Ser Leu Leu Lys Asp Ile
```

```
            885                 890                 895

Ile Asn Glu Tyr Phe Asn
        900

<210> 8
<211> 912
<212> PRT
<213> Artificial Sequence

<220>
<221> source
<223> /note="Description of Artificial Sequence: Synthetic
      polypeptide"

<400> 8
Thr Trp Pro Val Lys Asp Phe Asn Tyr Ser Asp Pro Val Asn Asp Asn
1               5                   10                  15

Asp Ile Leu Tyr Leu Arg Ile Pro Gln Asn Lys Leu Ile Thr Thr Pro
            20                  25                  30

Val Lys Ala Phe Met Ile Thr Gln Asn Ile Trp Val Ile Pro Glu Arg
        35                  40                  45

Phe Ser Ser Asp Thr Asn Pro Ser Leu Ser Lys Pro Pro Arg Pro Thr
    50                  55                  60

Ser Lys Tyr Gln Ser Tyr Tyr Asp Pro Ser Tyr Leu Ser Thr Asp Glu
65                  70                  75                  80

Gln Lys Asp Thr Phe Leu Lys Gly Ile Ile Lys Leu Phe Lys Arg Ile
                85                  90                  95

Asn Glu Arg Asp Ile Gly Lys Lys Leu Ile Asn Tyr Leu Val Val Gly
            100                 105                 110

Ser Pro Phe Met Gly Asp Ser Ser Thr Pro Glu Asp Thr Phe Asp Phe
        115                 120                 125

Thr Arg His Thr Thr Asn Ile Ala Val Glu Lys Phe Glu Asn Gly Ser
    130                 135                 140

Trp Lys Val Thr Asn Ile Ile Thr Pro Ser Val Leu Ile Phe Gly Pro
145                 150                 155                 160

Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
```

180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

```
Thr Lys Val Cys Val Asp Gly Ile Ile Thr Ser Lys Thr Lys Ser Asp
        435                 440                 445

Asp Asp Asp Lys Pro Cys Lys Asn Phe Phe Trp Lys Thr Phe Ser Ser
    450                 455                 460

Cys Lys Ala Leu Ala Gly Gly Gly Gly Ser Gly Gly Gly Gly Ser Gly
465                 470                 475                 480

Gly Gly Gly Ser Gly Gly Gly Gly Ser Gly Gly Gly Gly Ser Ala Leu
                485                 490                 495

Ala Leu Gln Cys Ile Lys Val Lys Asn Asn Arg Leu Pro Tyr Val Ala
            500                 505                 510

Asp Lys Asp Ser Ile Ser Gln Glu Ile Phe Glu Asn Lys Ile Ile Thr
        515                 520                 525

Asp Glu Thr Asn Val Gln Asn Tyr Ser Asp Lys Phe Ser Leu Asp Glu
    530                 535                 540

Ser Ile Leu Asp Gly Gln Val Pro Ile Asn Pro Glu Ile Val Asp Pro
545                 550                 555                 560

Leu Leu Pro Asn Val Asn Met Glu Pro Leu Asn Leu Pro Gly Glu Glu
                565                 570                 575

Ile Val Phe Tyr Asp Asp Ile Thr Lys Tyr Val Asp Tyr Leu Asn Ser
            580                 585                 590

Tyr Tyr Tyr Leu Glu Ser Gln Lys Leu Ser Asn Asn Val Glu Asn Ile
        595                 600                 605

Thr Leu Thr Thr Ser Val Glu Glu Ala Leu Gly Tyr Ser Asn Lys Ile
    610                 615                 620

Tyr Thr Phe Leu Pro Ser Leu Ala Glu Lys Val Asn Lys Gly Val Gln
625                 630                 635                 640

Ala Gly Leu Phe Leu Asn Trp Ala Asn Glu Val Val Glu Asp Phe Thr
                645                 650                 655

Thr Asn Ile Met Lys Lys Asp Thr Leu Asp Lys Ile Ser Asp Val Ser
            660                 665                 670

Val Ile Ile Pro Tyr Ile Gly Pro Ala Leu Asn Ile Gly Asn Ser Ala
        675                 680                 685
```

Leu Arg Gly Asn Phe Asn Gln Ala Phe Ala Thr Ala Gly Val Ala Phe
    690                 695                 700

Leu Leu Glu Gly Phe Pro Glu Phe Thr Ile Pro Ala Leu Gly Val Phe
705                 710                 715                 720

Thr Phe Tyr Ser Ser Ile Gln Glu Arg Glu Lys Ile Ile Lys Thr Ile
                725                 730                 735

Glu Asn Cys Leu Glu Gln Arg Val Lys Arg Trp Lys Asp Ser Tyr Gln
            740                 745                 750

Trp Met Val Ser Asn Trp Leu Ser Arg Ile Thr Thr Gln Phe Asn His
        755                 760                 765

Ile Asn Tyr Gln Met Tyr Asp Ser Leu Ser Tyr Gln Ala Asp Ala Ile
    770                 775                 780

Lys Ala Lys Ile Asp Leu Glu Tyr Lys Lys Tyr Ser Gly Ser Asp Lys
785                 790                 795                 800

Glu Asn Ile Lys Ser Gln Val Glu Asn Leu Lys Asn Ser Leu Asp Val
                805                 810                 815

Lys Ile Ser Glu Ala Met Asn Asn Ile Asn Lys Phe Ile Arg Glu Cys
            820                 825                 830

Ser Val Thr Tyr Leu Phe Lys Asn Met Leu Pro Lys Val Ile Asp Glu
        835                 840                 845

Leu Asn Lys Phe Asp Leu Arg Thr Lys Thr Glu Leu Ile Asn Leu Ile
    850                 855                 860

Asp Ser His Asn Ile Ile Leu Val Gly Glu Val Asp Arg Leu Lys Ala
865                 870                 875                 880

Lys Val Asn Glu Ser Phe Glu Asn Thr Met Pro Phe Asn Ile Phe Ser
                885                 890                 895

Tyr Thr Asn Asn Ser Leu Leu Lys Asp Ile Ile Asn Glu Tyr Phe Asn
            900                 905                 910

<210> 9
<211> 917
<212> PRT
<213> Artificial Sequence

<220>

<221> source
<223> /note="Description of Artificial Sequence: Synthetic
polypeptide"

<400> 9
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Gln Glu Arg Pro Pro Leu Gln Gln Pro Pro His Arg
450 455 460

Asp Lys Lys Pro Cys Lys Asn Phe Phe Trp Lys Thr Phe Ser Ser Cys
465 470 475 480

Lys Ala Leu Ala Gly Gly Gly Gly Ser Gly Gly Gly Gly Ser Gly Gly

```
                485                 490                 495

Gly Gly Ser Ala Leu Val Leu Gln Cys Ile Lys Val Lys Asn Asn Arg
            500                 505                 510

Leu Pro Tyr Val Ala Asp Lys Asp Ser Ile Ser Gln Glu Ile Phe Glu
        515                 520                 525

Asn Lys Ile Ile Thr Asp Glu Thr Asn Val Gln Asn Tyr Ser Asp Lys
    530                 535                 540

Phe Ser Leu Asp Glu Ser Ile Leu Asp Gly Gln Val Pro Ile Asn Pro
545                 550                 555                 560

Glu Ile Val Asp Pro Leu Leu Pro Asn Val Asn Met Glu Pro Leu Asn
                565                 570                 575

Leu Pro Gly Glu Glu Ile Val Phe Tyr Asp Asp Ile Thr Lys Tyr Val
            580                 585                 590

Asp Tyr Leu Asn Ser Tyr Tyr Tyr Leu Glu Ser Gln Lys Leu Ser Asn
        595                 600                 605

Asn Val Glu Asn Ile Thr Leu Thr Thr Ser Val Glu Glu Ala Leu Gly
    610                 615                 620

Tyr Ser Asn Lys Ile Tyr Thr Phe Leu Pro Ser Leu Ala Glu Lys Val
625                 630                 635                 640

Asn Lys Gly Val Gln Ala Gly Leu Phe Leu Asn Trp Ala Asn Glu Val
                645                 650                 655

Val Glu Asp Phe Thr Thr Asn Ile Met Lys Lys Asp Thr Leu Asp Lys
            660                 665                 670

Ile Ser Asp Val Ser Val Ile Ile Pro Tyr Ile Gly Pro Ala Leu Asn
        675                 680                 685

Ile Gly Asn Ser Ala Leu Arg Gly Asn Phe Asn Gln Ala Phe Ala Thr
    690                 695                 700

Ala Gly Val Ala Phe Leu Leu Glu Gly Phe Pro Glu Phe Thr Ile Pro
705                 710                 715                 720

Ala Leu Gly Val Phe Thr Phe Tyr Ser Ser Ile Gln Glu Arg Glu Lys
                725                 730                 735
```

Ile Ile Lys Thr Ile Glu Asn Cys Leu Glu Gln Arg Val Lys Arg Trp
740 745 750

Lys Asp Ser Tyr Gln Trp Met Val Ser Asn Trp Leu Ser Arg Ile Thr
755 760 765

Thr Gln Phe Asn His Ile Asn Tyr Gln Met Tyr Asp Ser Leu Ser Tyr
770 775 780

Gln Ala Asp Ala Ile Lys Ala Lys Ile Asp Leu Glu Tyr Lys Lys Tyr
785 790 795 800

Ser Gly Ser Asp Lys Glu Asn Ile Lys Ser Gln Val Glu Asn Leu Lys
805 810 815

Asn Ser Leu Asp Val Lys Ile Ser Glu Ala Met Asn Asn Ile Asn Lys
820 825 830

Phe Ile Arg Glu Cys Ser Val Thr Tyr Leu Phe Lys Asn Met Leu Pro
835 840 845

Lys Val Ile Asp Glu Leu Asn Lys Phe Asp Leu Arg Thr Lys Thr Glu
850 855 860

Leu Ile Asn Leu Ile Asp Ser His Asn Ile Ile Leu Val Gly Glu Val
865 870 875 880

Asp Arg Leu Lys Ala Lys Val Asn Glu Ser Phe Glu Asn Thr Met Pro
885 890 895

Phe Asn Ile Phe Ser Tyr Thr Asn Asn Ser Leu Leu Lys Asp Ile Ile
900 905 910

Asn Glu Tyr Phe Asn
915

<210> 10
<211> 927
<212> PRT
<213> Artificial Sequence

<220>
<221> source
<223> /note="Description of Artificial Sequence: Synthetic polypeptide"

<400> 10
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Gln Glu Arg Pro Pro Leu Gln Gln Pro Pro His Arg
450 455 460

Asp Lys Lys Pro Cys Lys Asn Phe Phe Trp Lys Thr Phe Ser Ser Cys
465 470 475 480

Lys Ala Leu Ala Gly Gly Gly Gly Ser Gly Gly Gly Gly Ser Gly Gly
485 490 495

Gly Gly Ser Gly Gly Gly Gly Ser Gly Gly Gly Gly Ser Ala Leu Val
500 505 510

Leu Gln Cys Ile Lys Val Lys Asn Asn Arg Leu Pro Tyr Val Ala Asp
515 520 525

Lys Asp Ser Ile Ser Gln Glu Ile Phe Glu Asn Lys Ile Ile Thr Asp
530 535 540

Glu Thr Asn Val Gln Asn Tyr Ser Asp Lys Phe Ser Leu Asp Glu Ser
545 550 555 560

Ile Leu Asp Gly Gln Val Pro Ile Asn Pro Glu Ile Val Asp Pro Leu
565 570 575

Leu Pro Asn Val Asn Met Glu Pro Leu Asn Leu Pro Gly Glu Glu Ile
580 585 590

Val Phe Tyr Asp Asp Ile Thr Lys Tyr Val Asp Tyr Leu Asn Ser Tyr
595 600 605

Tyr Tyr Leu Glu Ser Gln Lys Leu Ser Asn Asn Val Glu Asn Ile Thr
610 615 620

Leu Thr Thr Ser Val Glu Glu Ala Leu Gly Tyr Ser Asn Lys Ile Tyr
625 630 635 640

Thr Phe Leu Pro Ser Leu Ala Glu Lys Val Asn Lys Gly Val Gln Ala
645 650 655

Gly Leu Phe Leu Asn Trp Ala Asn Glu Val Val Glu Asp Phe Thr Thr
660 665 670

Asn Ile Met Lys Lys Asp Thr Leu Asp Lys Ile Ser Asp Val Ser Val
675 680 685

Ile Ile Pro Tyr Ile Gly Pro Ala Leu Asn Ile Gly Asn Ser Ala Leu
690 695 700

Arg Gly Asn Phe Asn Gln Ala Phe Ala Thr Ala Gly Val Ala Phe Leu
705 710 715 720

Leu Glu Gly Phe Pro Glu Phe Thr Ile Pro Ala Leu Gly Val Phe Thr
725 730 735

Phe Tyr Ser Ser Ile Gln Glu Arg Glu Lys Ile Ile Lys Thr Ile Glu
740 745 750

Asn Cys Leu Glu Gln Arg Val Lys Arg Trp Lys Asp Ser Tyr Gln Trp
755 760 765

Met Val Ser Asn Trp Leu Ser Arg Ile Thr Thr Gln Phe Asn His Ile

770 775 780

Asn Tyr Gln Met Tyr Asp Ser Leu Ser Tyr Gln Ala Asp Ala Ile Lys
785 790 795 800

Ala Lys Ile Asp Leu Glu Tyr Lys Lys Tyr Ser Gly Ser Asp Lys Glu
805 810 815

Asn Ile Lys Ser Gln Val Glu Asn Leu Lys Asn Ser Leu Asp Val Lys
820 825 830

Ile Ser Glu Ala Met Asn Asn Ile Asn Lys Phe Ile Arg Glu Cys Ser
835 840 845

Val Thr Tyr Leu Phe Lys Asn Met Leu Pro Lys Val Ile Asp Glu Leu
850 855 860

Asn Lys Phe Asp Leu Arg Thr Lys Thr Glu Leu Ile Asn Leu Ile Asp
865 870 875 880

Ser His Asn Ile Ile Leu Val Gly Glu Val Asp Arg Leu Lys Ala Lys
885 890 895

Val Asn Glu Ser Phe Glu Asn Thr Met Pro Phe Asn Ile Phe Ser Tyr
900 905 910

Thr Asn Asn Ser Leu Leu Lys Asp Ile Ile Asn Glu Tyr Phe Asn
915 920 925

<210> 11
<211> 902
<212> PRT
<213> Artificial Sequence

<220>
<221> source
<223> /note="Description of Artificial Sequence: Synthetic polypeptide"

<400> 11
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr

```
        50                  55                  60

    Ser Lys Tyr Gln Ser Tyr Tyr Asp Pro Ser Tyr Leu Ser Thr Asp Glu
    65                  70                  75                  80

    Gln Lys Asp Thr Phe Leu Lys Gly Ile Ile Lys Leu Phe Lys Arg Ile
                    85                  90                  95

    Asn Glu Arg Asp Ile Gly Lys Lys Leu Ile Asn Tyr Leu Val Val Gly
                100                 105                 110

    Ser Pro Phe Met Gly Asp Ser Ser Thr Pro Glu Asp Thr Phe Asp Phe
            115                 120                 125

    Thr Arg His Thr Thr Asn Ile Ala Val Glu Lys Phe Glu Asn Gly Ser
        130                 135                 140

    Trp Lys Val Thr Asn Ile Ile Thr Pro Ser Val Leu Ile Phe Gly Pro
    145                 150                 155                 160

    Leu Pro Asn Ile Leu Asp Tyr Thr Ala Ser Leu Thr Leu Gln Gly Gln
                    165                 170                 175

    Gln Ser Asn Pro Ser Phe Glu Gly Phe Gly Thr Leu Ser Ile Leu Lys
                180                 185                 190

    Val Ala Pro Glu Phe Leu Leu Thr Phe Ser Asp Val Thr Ser Asn Gln
            195                 200                 205

    Ser Ser Ala Val Leu Gly Lys Ser Ile Phe Cys Met Asp Pro Val Ile
        210                 215                 220

    Ala Leu Met His Glu Leu Thr His Ser Leu His Gln Leu Tyr Gly Ile
    225                 230                 235                 240

    Asn Ile Pro Ser Asp Lys Arg Ile Arg Pro Gln Val Ser Glu Gly Phe
                    245                 250                 255

    Phe Ser Gln Asp Gly Pro Asn Val Gln Phe Glu Glu Leu Tyr Thr Phe
                260                 265                 270

    Gly Gly Leu Asp Val Glu Ile Ile Pro Gln Ile Glu Arg Ser Gln Leu
            275                 280                 285

    Arg Glu Lys Ala Leu Gly His Tyr Lys Asp Ile Ala Lys Arg Leu Asn
        290                 295                 300
```

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Ala Gly Cys Lys Asn Phe Phe Trp Lys Thr Phe Thr
450 455 460

Ser Cys Ala Leu Ala Gly Gly Gly Gly Ser Gly Gly Gly Gly Ser Gly
465 470 475 480

Gly Gly Gly Ser Ala Leu Val Leu Gln Cys Ile Lys Val Lys Asn Asn
485 490 495

Arg Leu Pro Tyr Val Ala Asp Lys Asp Ser Ile Ser Gln Glu Ile Phe
500 505 510

Glu Asn Lys Ile Ile Thr Asp Glu Thr Asn Val Gln Asn Tyr Ser Asp
515 520 525

Lys Phe Ser Leu Asp Glu Ser Ile Leu Asp Gly Gln Val Pro Ile Asn
530 535 540

Pro Glu Ile Val Asp Pro Leu Leu Pro Asn Val Asn Met Glu Pro Leu
545 550 555 560

Asn Leu Pro Gly Glu Glu Ile Val Phe Tyr Asp Asp Ile Thr Lys Tyr
565 570 575

Val Asp Tyr Leu Asn Ser Tyr Tyr Tyr Leu Glu Ser Gln Lys Leu Ser
580 585 590

Asn Asn Val Glu Asn Ile Thr Leu Thr Thr Ser Val Glu Glu Ala Leu
595 600 605

Gly Tyr Ser Asn Lys Ile Tyr Thr Phe Leu Pro Ser Leu Ala Glu Lys
610 615 620

Val Asn Lys Gly Val Gln Ala Gly Leu Phe Leu Asn Trp Ala Asn Glu
625 630 635 640

Val Val Glu Asp Phe Thr Thr Asn Ile Met Lys Lys Asp Thr Leu Asp
645 650 655

Lys Ile Ser Asp Val Ser Val Ile Ile Pro Tyr Ile Gly Pro Ala Leu
660 665 670

Asn Ile Gly Asn Ser Ala Leu Arg Gly Asn Phe Asn Gln Ala Phe Ala
675 680 685

Thr Ala Gly Val Ala Phe Leu Leu Glu Gly Phe Pro Glu Phe Thr Ile
690 695 700

Pro Ala Leu Gly Val Phe Thr Phe Tyr Ser Ser Ile Gln Glu Arg Glu
705 710 715 720

Lys Ile Ile Lys Thr Ile Glu Asn Cys Leu Glu Gln Arg Val Lys Arg
725 730 735

Trp Lys Asp Ser Tyr Gln Trp Met Val Ser Asn Trp Leu Ser Arg Ile
740 745 750

Thr Thr Gln Phe Asn His Ile Asn Tyr Gln Met Tyr Asp Ser Leu Ser
755 760 765

Tyr Gln Ala Asp Ala Ile Lys Ala Lys Ile Asp Leu Glu Tyr Lys Lys
770 775 780

Tyr Ser Gly Ser Asp Lys Glu Asn Ile Lys Ser Gln Val Glu Asn Leu
785 790 795 800

Lys Asn Ser Leu Asp Val Lys Ile Ser Glu Ala Met Asn Asn Ile Asn
805 810 815

```
Lys Phe Ile Arg Glu Cys Ser Val Thr Tyr Leu Phe Lys Asn Met Leu
            820                 825                 830

Pro Lys Val Ile Asp Glu Leu Asn Lys Phe Asp Leu Arg Thr Lys Thr
        835                 840                 845

Glu Leu Ile Asn Leu Ile Asp Ser His Asn Ile Ile Leu Val Gly Glu
    850                 855                 860

Val Asp Arg Leu Lys Ala Lys Val Asn Glu Ser Phe Glu Asn Thr Met
865                 870                 875                 880

Pro Phe Asn Ile Phe Ser Tyr Thr Asn Asn Ser Leu Leu Lys Asp Ile
                885                 890                 895

Ile Asn Glu Tyr Phe Asn
            900


<210> 12
<211> 912
<212> PRT
<213> Artificial Sequence

<220>
<221> source
<223> /note="Description of Artificial Sequence: Synthetic
      polypeptide"

<400> 12
Thr Trp Pro Val Lys Asp Phe Asn Tyr Ser Asp Pro Val Asn Asp Asn
1               5                   10                  15

Asp Ile Leu Tyr Leu Arg Ile Pro Gln Asn Lys Leu Ile Thr Thr Pro
            20                  25                  30

Val Lys Ala Phe Met Ile Thr Gln Asn Ile Trp Val Ile Pro Glu Arg
        35                  40                  45

Phe Ser Ser Asp Thr Asn Pro Ser Leu Ser Lys Pro Pro Arg Pro Thr
    50                  55                  60

Ser Lys Tyr Gln Ser Tyr Tyr Asp Pro Ser Tyr Leu Ser Thr Asp Glu
65                  70                  75                  80

Gln Lys Asp Thr Phe Leu Lys Gly Ile Ile Lys Leu Phe Lys Arg Ile
                85                  90                  95

Asn Glu Arg Asp Ile Gly Lys Lys Leu Ile Asn Tyr Leu Val Val Gly
            100                 105                 110
```

Ser Pro Phe Met Gly Asp Ser Ser Thr Pro Glu Asp Thr Phe Asp Phe
        115                 120                 125

Thr Arg His Thr Thr Asn Ile Ala Val Glu Lys Phe Glu Asn Gly Ser
    130                 135                 140

Trp Lys Val Thr Asn Ile Ile Thr Pro Ser Val Leu Ile Phe Gly Pro
145                 150                 155                 160

Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
225                 230                 235                 240

Asn Ile Pro Ser Asp Lys Arg Ile Arg Pro Gln Val Ser Glu Gly Phe
                245                 250                 255

Phe Ser Gln Asp Gly Pro Asn Val Gln Phe Glu Glu Leu Tyr Thr Phe
            260                 265                 270

Gly Gly Leu Asp Val Glu Ile Ile Pro Gln Ile Glu Arg Ser Gln Leu
        275                 280                 285

Arg Glu Lys Ala Leu Gly His Tyr Lys Asp Ile Ala Lys Arg Leu Asn
    290                 295                 300

Asn Ile Asn Lys Thr Ile Pro Ser Ser Trp Ile Ser Asn Ile Asp Lys
305                 310                 315                 320

Tyr Lys Lys Ile Phe Ser Glu Lys Tyr Asn Phe Asp Lys Asp Asn Thr
                325                 330                 335

Gly Asn Phe Val Val Asn Ile Asp Lys Phe Asn Ser Leu Tyr Ser Asp
            340                 345                 350

Leu Thr Asn Val Met Ser Glu Val Val Tyr Ser Ser Gln Tyr Asn Val

355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Ala Gly Cys Lys Asn Phe Phe Trp Lys Thr Phe Thr
450 455 460

Ser Cys Ala Leu Ala Gly Gly Gly Gly Ser Gly Gly Gly Gly Ser Gly
465 470 475 480

Gly Gly Gly Ser Gly Gly Gly Gly Ser Gly Gly Gly Gly Ser Ala Leu
485 490 495

Ala Leu Gln Cys Ile Lys Val Lys Asn Asn Arg Leu Pro Tyr Val Ala
500 505 510

Asp Lys Asp Ser Ile Ser Gln Glu Ile Phe Glu Asn Lys Ile Ile Thr
515 520 525

Asp Glu Thr Asn Val Gln Asn Tyr Ser Asp Lys Phe Ser Leu Asp Glu
530 535 540

Ser Ile Leu Asp Gly Gln Val Pro Ile Asn Pro Glu Ile Val Asp Pro
545 550 555 560

Leu Leu Pro Asn Val Asn Met Glu Pro Leu Asn Leu Pro Gly Glu Glu
565 570 575

Ile Val Phe Tyr Asp Asp Ile Thr Lys Tyr Val Asp Tyr Leu Asn Ser
580 585 590

Tyr Tyr Tyr Leu Glu Ser Gln Lys Leu Ser Asn Asn Val Glu Asn Ile
595 600 605

Thr Leu Thr Thr Ser Val Glu Glu Ala Leu Gly Tyr Ser Asn Lys Ile
    610                 615                 620

Tyr Thr Phe Leu Pro Ser Leu Ala Glu Lys Val Asn Lys Gly Val Gln
625                 630                 635                 640

Ala Gly Leu Phe Leu Asn Trp Ala Asn Glu Val Val Glu Asp Phe Thr
                645                 650                 655

Thr Asn Ile Met Lys Lys Asp Thr Leu Asp Lys Ile Ser Asp Val Ser
            660                 665                 670

Val Ile Ile Pro Tyr Ile Gly Pro Ala Leu Asn Ile Gly Asn Ser Ala
        675                 680                 685

Leu Arg Gly Asn Phe Asn Gln Ala Phe Ala Thr Ala Gly Val Ala Phe
    690                 695                 700

Leu Leu Glu Gly Phe Pro Glu Phe Thr Ile Pro Ala Leu Gly Val Phe
705                 710                 715                 720

Thr Phe Tyr Ser Ser Ile Gln Glu Arg Glu Lys Ile Ile Lys Thr Ile
                725                 730                 735

Glu Asn Cys Leu Glu Gln Arg Val Lys Arg Trp Lys Asp Ser Tyr Gln
            740                 745                 750

Trp Met Val Ser Asn Trp Leu Ser Arg Ile Thr Thr Gln Phe Asn His
        755                 760                 765

Ile Asn Tyr Gln Met Tyr Asp Ser Leu Ser Tyr Gln Ala Asp Ala Ile
    770                 775                 780

Lys Ala Lys Ile Asp Leu Glu Tyr Lys Lys Tyr Ser Gly Ser Asp Lys
785                 790                 795                 800

Glu Asn Ile Lys Ser Gln Val Glu Asn Leu Lys Asn Ser Leu Asp Val
                805                 810                 815

Lys Ile Ser Glu Ala Met Asn Asn Ile Asn Lys Phe Ile Arg Glu Cys
            820                 825                 830

Ser Val Thr Tyr Leu Phe Lys Asn Met Leu Pro Lys Val Ile Asp Glu
        835                 840                 845

Leu Asn Lys Phe Asp Leu Arg Thr Lys Thr Glu Leu Ile Asn Leu Ile
    850                 855                 860

Asp Ser His Asn Ile Ile Leu Val Gly Glu Val Asp Arg Leu Lys Ala
865 870 875 880

Lys Val Asn Glu Ser Phe Glu Asn Thr Met Pro Phe Asn Ile Phe Ser
885 890 895

Tyr Thr Asn Asn Ser Leu Leu Lys Asp Ile Ile Asn Glu Tyr Phe Asn
900 905 910

<210> 13
<211> 916
<212> PRT
<213> Artificial Sequence

<220>
<221> source
<223> /note="Description of Artificial Sequence: Synthetic polypeptide"

<400> 13
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

```
Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
225                 230                 235                 240

Asn Ile Pro Ser Asp Lys Arg Ile Arg Pro Gln Val Ser Glu Gly Phe
                245                 250                 255

Phe Ser Gln Asp Gly Pro Asn Val Gln Phe Glu Glu Leu Tyr Thr Phe
            260                 265                 270

Gly Gly Leu Asp Val Glu Ile Ile Pro Gln Ile Glu Arg Ser Gln Leu
        275                 280                 285

Arg Glu Lys Ala Leu Gly His Tyr Lys Asp Ile Ala Lys Arg Leu Asn
    290                 295                 300

Asn Ile Asn Lys Thr Ile Pro Ser Ser Trp Ile Ser Asn Ile Asp Lys
305                 310                 315                 320

Tyr Lys Lys Ile Phe Ser Glu Lys Tyr Asn Phe Asp Lys Asp Asn Thr
                325                 330                 335

Gly Asn Phe Val Val Asn Ile Asp Lys Phe Asn Ser Leu Tyr Ser Asp
            340                 345                 350

Leu Thr Asn Val Met Ser Glu Val Val Tyr Ser Ser Gln Tyr Asn Val
        355                 360                 365

Lys Asn Arg Thr His Tyr Phe Ser Arg His Tyr Leu Pro Val Phe Ala
    370                 375                 380

Asn Ile Leu Asp Asp Asn Ile Tyr Thr Ile Arg Asp Gly Phe Asn Leu
385                 390                 395                 400

Thr Asn Lys Gly Phe Asn Ile Glu Asn Ser Gly Gln Asn Ile Glu Arg
                405                 410                 415
```

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Ser Ala Asn Ser Asn Pro Ala Met Ala Pro Arg Glu
450 455 460

Arg Lys Ala Gly Cys Lys Asn Phe Phe Trp Lys Thr Phe Thr Ser Cys
465 470 475 480

Ala Leu Ala Gly Gly Gly Gly Ser Gly Gly Gly Gly Ser Gly Gly Gly
485 490 495

Gly Ser Ala Leu Val Leu Gln Cys Ile Lys Val Lys Asn Asn Arg Leu
500 505 510

Pro Tyr Val Ala Asp Lys Asp Ser Ile Ser Gln Glu Ile Phe Glu Asn
515 520 525

Lys Ile Ile Thr Asp Glu Thr Asn Val Gln Asn Tyr Ser Asp Lys Phe
530 535 540

Ser Leu Asp Glu Ser Ile Leu Asp Gly Gln Val Pro Ile Asn Pro Glu
545 550 555 560

Ile Val Asp Pro Leu Leu Pro Asn Val Asn Met Glu Pro Leu Asn Leu
565 570 575

Pro Gly Glu Glu Ile Val Phe Tyr Asp Asp Ile Thr Lys Tyr Val Asp
580 585 590

Tyr Leu Asn Ser Tyr Tyr Tyr Leu Glu Ser Gln Lys Leu Ser Asn Asn
595 600 605

Val Glu Asn Ile Thr Leu Thr Thr Ser Val Glu Glu Ala Leu Gly Tyr
610 615 620

Ser Asn Lys Ile Tyr Thr Phe Leu Pro Ser Leu Ala Glu Lys Val Asn
625 630 635 640

Lys Gly Val Gln Ala Gly Leu Phe Leu Asn Trp Ala Asn Glu Val Val
645 650 655

Glu Asp Phe Thr Thr Asn Ile Met Lys Lys Asp Thr Leu Asp Lys Ile

```
                660                 665                 670

Ser Asp Val Ser Val Ile Ile Pro Tyr Ile Gly Pro Ala Leu Asn Ile
            675                 680                 685

Gly Asn Ser Ala Leu Arg Gly Asn Phe Asn Gln Ala Phe Ala Thr Ala
        690                 695                 700

Gly Val Ala Phe Leu Leu Glu Gly Phe Pro Glu Phe Thr Ile Pro Ala
705                 710                 715                 720

Leu Gly Val Phe Thr Phe Tyr Ser Ser Ile Gln Glu Arg Glu Lys Ile
                725                 730                 735

Ile Lys Thr Ile Glu Asn Cys Leu Glu Gln Arg Val Lys Arg Trp Lys
            740                 745                 750

Asp Ser Tyr Gln Trp Met Val Ser Asn Trp Leu Ser Arg Ile Thr Thr
        755                 760                 765

Gln Phe Asn His Ile Asn Tyr Gln Met Tyr Asp Ser Leu Ser Tyr Gln
    770                 775                 780

Ala Asp Ala Ile Lys Ala Lys Ile Asp Leu Glu Tyr Lys Lys Tyr Ser
785                 790                 795                 800

Gly Ser Asp Lys Glu Asn Ile Lys Ser Gln Val Glu Asn Leu Lys Asn
                805                 810                 815

Ser Leu Asp Val Lys Ile Ser Glu Ala Met Asn Asn Ile Asn Lys Phe
            820                 825                 830

Ile Arg Glu Cys Ser Val Thr Tyr Leu Phe Lys Asn Met Leu Pro Lys
        835                 840                 845

Val Ile Asp Glu Leu Asn Lys Phe Asp Leu Arg Thr Lys Thr Glu Leu
    850                 855                 860

Ile Asn Leu Ile Asp Ser His Asn Ile Ile Leu Val Gly Glu Val Asp
865                 870                 875                 880

Arg Leu Lys Ala Lys Val Asn Glu Ser Phe Glu Asn Thr Met Pro Phe
                885                 890                 895

Asn Ile Phe Ser Tyr Thr Asn Asn Ser Leu Leu Lys Asp Ile Ile Asn
            900                 905                 910
```

Glu Tyr Phe Asn
        915

<210> 14
<211> 926
<212> PRT
<213> Artificial Sequence

<220>
<221> source
<223> /note="Description of Artificial Sequence: Synthetic
      polypeptide"

<400> 14
Thr Trp Pro Val Lys Asp Phe Asn Tyr Ser Asp Pro Val Asn Asp Asn
1               5                   10                  15

Asp Ile Leu Tyr Leu Arg Ile Pro Gln Asn Lys Leu Ile Thr Thr Pro
            20                  25                  30

Val Lys Ala Phe Met Ile Thr Gln Asn Ile Trp Val Ile Pro Glu Arg
        35                  40                  45

Phe Ser Ser Asp Thr Asn Pro Ser Leu Ser Lys Pro Pro Arg Pro Thr
    50                  55                  60

Ser Lys Tyr Gln Ser Tyr Tyr Asp Pro Ser Tyr Leu Ser Thr Asp Glu
65                  70                  75                  80

Gln Lys Asp Thr Phe Leu Lys Gly Ile Ile Lys Leu Phe Lys Arg Ile
                85                  90                  95

Asn Glu Arg Asp Ile Gly Lys Lys Leu Ile Asn Tyr Leu Val Val Gly
            100                 105                 110

Ser Pro Phe Met Gly Asp Ser Ser Thr Pro Glu Asp Thr Phe Asp Phe
        115                 120                 125

Thr Arg His Thr Thr Asn Ile Ala Val Glu Lys Phe Glu Asn Gly Ser
    130                 135                 140

Trp Lys Val Thr Asn Ile Ile Thr Pro Ser Val Leu Ile Phe Gly Pro
145                 150                 155                 160

Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Ser Ala Asn Ser Asn Pro Ala Met Ala Pro Arg Glu
450 455 460

Arg Lys Ala Gly Cys Lys Asn Phe Phe Trp Lys Thr Phe Thr Ser Cys
465 470 475 480

Ala Leu Ala Gly Gly Gly Gly Ser Gly Gly Gly Gly Ser Gly Gly Gly
485 490 495

Gly Ser Gly Gly Gly Gly Ser Gly Gly Gly Gly Ser Ala Leu Ala Leu
500 505 510

Gln Cys Ile Lys Val Lys Asn Asn Arg Leu Pro Tyr Val Ala Asp Lys
515 520 525

Asp Ser Ile Ser Gln Glu Ile Phe Glu Asn Lys Ile Ile Thr Asp Glu
530 535 540

Thr Asn Val Gln Asn Tyr Ser Asp Lys Phe Ser Leu Asp Glu Ser Ile
545 550 555 560

Leu Asp Gly Gln Val Pro Ile Asn Pro Glu Ile Val Asp Pro Leu Leu
565 570 575

Pro Asn Val Asn Met Glu Pro Leu Asn Leu Pro Gly Glu Glu Ile Val
580 585 590

Phe Tyr Asp Asp Ile Thr Lys Tyr Val Asp Tyr Leu Asn Ser Tyr Tyr
595 600 605

Tyr Leu Glu Ser Gln Lys Leu Ser Asn Asn Val Glu Asn Ile Thr Leu
610 615 620

Thr Thr Ser Val Glu Glu Ala Leu Gly Tyr Ser Asn Lys Ile Tyr Thr
625 630 635 640

Phe Leu Pro Ser Leu Ala Glu Lys Val Asn Lys Gly Val Gln Ala Gly
645 650 655

Leu Phe Leu Asn Trp Ala Asn Glu Val Val Glu Asp Phe Thr Thr Asn
660 665 670

Ile Met Lys Lys Asp Thr Leu Asp Lys Ile Ser Asp Val Ser Val Ile
675 680 685

Ile Pro Tyr Ile Gly Pro Ala Leu Asn Ile Gly Asn Ser Ala Leu Arg
690 695 700

Gly Asn Phe Asn Gln Ala Phe Ala Thr Ala Gly Val Ala Phe Leu Leu
705 710 715 720

Glu Gly Phe Pro Glu Phe Thr Ile Pro Ala Leu Gly Val Phe Thr Phe
725 730 735

Tyr Ser Ser Ile Gln Glu Arg Glu Lys Ile Ile Lys Thr Ile Glu Asn
740 745 750

Cys Leu Glu Gln Arg Val Lys Arg Trp Lys Asp Ser Tyr Gln Trp Met
755 760 765

Val Ser Asn Trp Leu Ser Arg Ile Thr Thr Gln Phe Asn His Ile Asn
770 775 780

Tyr Gln Met Tyr Asp Ser Leu Ser Tyr Gln Ala Asp Ala Ile Lys Ala
785 790 795 800

Lys Ile Asp Leu Glu Tyr Lys Lys Tyr Ser Gly Ser Asp Lys Glu Asn
805 810 815

Ile Lys Ser Gln Val Glu Asn Leu Lys Asn Ser Leu Asp Val Lys Ile
820 825 830

Ser Glu Ala Met Asn Asn Ile Asn Lys Phe Ile Arg Glu Cys Ser Val
835 840 845

Thr Tyr Leu Phe Lys Asn Met Leu Pro Lys Val Ile Asp Glu Leu Asn
850 855 860

Lys Phe Asp Leu Arg Thr Lys Thr Glu Leu Ile Asn Leu Ile Asp Ser
865 870 875 880

His Asn Ile Ile Leu Val Gly Glu Val Asp Arg Leu Lys Ala Lys Val
885 890 895

Asn Glu Ser Phe Glu Asn Thr Met Pro Phe Asn Ile Phe Ser Tyr Thr
900 905 910

Asn Asn Ser Leu Leu Lys Asp Ile Ile Asn Glu Tyr Phe Asn
915 920 925

<210> 15
<211> 905
<212> PRT
<213> Artificial Sequence

<220>
<221> source
<223> /note="Description of Artificial Sequence: Synthetic polypeptide"

<400> 15

```
Thr Trp Pro Val Lys Asp Phe Asn Tyr Ser Asp Pro Val Asn Asp Asn
1               5                   10                  15

Asp Ile Leu Tyr Leu Arg Ile Pro Gln Asn Lys Leu Ile Thr Thr Pro
            20                  25                  30

Val Lys Ala Phe Met Ile Thr Gln Asn Ile Trp Val Ile Pro Glu Arg
        35                  40                  45

Phe Ser Ser Asp Thr Asn Pro Ser Leu Ser Lys Pro Pro Arg Pro Thr
    50                  55                  60

Ser Lys Tyr Gln Ser Tyr Tyr Asp Pro Ser Tyr Leu Ser Thr Asp Glu
65                  70                  75                  80

Gln Lys Asp Thr Phe Leu Lys Gly Ile Ile Lys Leu Phe Lys Arg Ile
                85                  90                  95

Asn Glu Arg Asp Ile Gly Lys Lys Leu Ile Asn Tyr Leu Val Val Gly
            100                 105                 110

Ser Pro Phe Met Gly Asp Ser Ser Thr Pro Glu Asp Thr Phe Asp Phe
        115                 120                 125

Thr Arg His Thr Thr Asn Ile Ala Val Glu Lys Phe Glu Asn Gly Ser
    130                 135                 140

Trp Lys Val Thr Asn Ile Ile Thr Pro Ser Val Leu Ile Phe Gly Pro
145                 150                 155                 160

Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
```

225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Lys Ser Glu Glu Lys Leu Tyr Asp Asp Asp
435 440 445

Asp Lys Asp Arg Trp Gly Ser Ser Leu Gln Cys Ile Lys Val Lys Asn
450 455 460

Asn Arg Leu Pro Tyr Val Ala Asp Lys Asp Ser Ile Ser Gln Glu Ile
465 470 475 480

Phe Glu Asn Lys Ile Ile Thr Asp Glu Thr Asn Val Gln Asn Tyr Ser
485 490 495

Asp Lys Phe Ser Leu Asp Glu Ser Ile Leu Asp Gly Gln Val Pro Ile
500 505 510

Asn Pro Glu Ile Val Asp Pro Leu Leu Pro Asn Val Asn Met Glu Pro
515 520 525

Leu Asn Leu Pro Gly Glu Glu Ile Val Phe Tyr Asp Asp Ile Thr Lys
530 535 540

Tyr Val Asp Tyr Leu Asn Ser Tyr Tyr Tyr Leu Glu Ser Gln Lys Leu
545 550 555 560

Ser Asn Asn Val Glu Asn Ile Thr Leu Thr Thr Ser Val Glu Glu Ala
565 570 575

Leu Gly Tyr Ser Asn Lys Ile Tyr Thr Phe Leu Pro Ser Leu Ala Glu
580 585 590

Lys Val Asn Lys Gly Val Gln Ala Gly Leu Phe Leu Asn Trp Ala Asn
595 600 605

Glu Val Val Glu Asp Phe Thr Thr Asn Ile Met Lys Lys Asp Thr Leu
610 615 620

Asp Lys Ile Ser Asp Val Ser Val Ile Ile Pro Tyr Ile Gly Pro Ala
625 630 635 640

Leu Asn Ile Gly Asn Ser Ala Leu Arg Gly Asn Phe Asn Gln Ala Phe
645 650 655

Ala Thr Ala Gly Val Ala Phe Leu Leu Glu Gly Phe Pro Glu Phe Thr
660 665 670

Ile Pro Ala Leu Gly Val Phe Thr Phe Tyr Ser Ser Ile Gln Glu Arg
675 680 685

Glu Lys Ile Ile Lys Thr Ile Glu Asn Cys Leu Glu Gln Arg Val Lys
690 695 700

Arg Trp Lys Asp Ser Tyr Gln Trp Met Val Ser Asn Trp Leu Ser Arg
705 710 715 720

Ile Thr Thr Gln Phe Asn His Ile Asn Tyr Gln Met Tyr Asp Ser Leu
725 730 735

Ser Tyr Gln Ala Asp Ala Ile Lys Ala Lys Ile Asp Leu Glu Tyr Lys
740 745 750

Lys Tyr Ser Gly Ser Asp Lys Glu Asn Ile Lys Ser Gln Val Glu Asn
755 760 765

Leu Lys Asn Ser Leu Asp Val Lys Ile Ser Glu Ala Met Asn Asn Ile
770 775 780

Asn Lys Phe Ile Arg Glu Cys Ser Val Thr Tyr Leu Phe Lys Asn Met
785 790 795 800

Leu Pro Lys Val Ile Asp Glu Leu Asn Lys Phe Asp Leu Arg Thr Lys
805 810 815

Thr Glu Leu Ile Asn Leu Ile Asp Ser His Asn Ile Ile Leu Val Gly
820 825 830

Glu Val Asp Arg Leu Lys Ala Lys Val Asn Glu Ser Phe Glu Asn Thr
835 840 845

Met Pro Phe Asn Ile Phe Ser Tyr Thr Asn Asn Ser Leu Leu Lys Asp
850 855 860

Ile Ile Asn Glu Tyr Phe Asn Leu Glu Gly Gly Gly Gly Ser Gly Gly
865 870 875 880

Gly Gly Ser Gly Gly Gly Gly Ser Ala Leu Val Pro Cys Lys Asn Phe
885 890 895

Phe Trp Lys Thr Phe Ser Ser Cys Lys
900 905

<210> 16
<211> 915
<212> PRT
<213> Artificial Sequence

<220>
<221> source
<223> /note="Description of Artificial Sequence: Synthetic polypeptide"

<400> 16
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
    290                 295                 300

Asn Ile Asn Lys Thr Ile Pro Ser Ser Trp Ile Ser Asn Ile Asp Lys
305                 310                 315                 320

Tyr Lys Lys Ile Phe Ser Glu Lys Tyr Asn Phe Asp Lys Asp Asn Thr
                325                 330                 335

Gly Asn Phe Val Val Asn Ile Asp Lys Phe Asn Ser Leu Tyr Ser Asp
            340                 345                 350

Leu Thr Asn Val Met Ser Glu Val Val Tyr Ser Ser Gln Tyr Asn Val
        355                 360                 365

Lys Asn Arg Thr His Tyr Phe Ser Arg His Tyr Leu Pro Val Phe Ala
    370                 375                 380

Asn Ile Leu Asp Asp Asn Ile Tyr Thr Ile Arg Asp Gly Phe Asn Leu
385                 390                 395                 400

Thr Asn Lys Gly Phe Asn Ile Glu Asn Ser Gly Gln Asn Ile Glu Arg
                405                 410                 415

Asn Pro Ala Leu Gln Lys Leu Ser Ser Glu Ser Val Val Asp Leu Phe
            420                 425                 430

Thr Lys Val Cys Val Asp Lys Ser Glu Glu Lys Leu Tyr Asp Asp Asp
        435                 440                 445

Asp Lys Asp Arg Trp Gly Ser Ser Leu Gln Cys Ile Lys Val Lys Asn
    450                 455                 460

Asn Arg Leu Pro Tyr Val Ala Asp Lys Asp Ser Ile Ser Gln Glu Ile
465                 470                 475                 480

Phe Glu Asn Lys Ile Ile Thr Asp Glu Thr Asn Val Gln Asn Tyr Ser
                485                 490                 495

Asp Lys Phe Ser Leu Asp Glu Ser Ile Leu Asp Gly Gln Val Pro Ile
            500                 505                 510

Asn Pro Glu Ile Val Asp Pro Leu Leu Pro Asn Val Asn Met Glu Pro
        515                 520                 525

Leu Asn Leu Pro Gly Glu Glu Ile Val Phe Tyr Asp Asp Ile Thr Lys

```
    530                 535                 540

Tyr Val Asp Tyr Leu Asn Ser Tyr Tyr Tyr Leu Glu Ser Gln Lys Leu
545                 550                 555                 560

Ser Asn Asn Val Glu Asn Ile Thr Leu Thr Thr Ser Val Glu Glu Ala
                565                 570                 575

Leu Gly Tyr Ser Asn Lys Ile Tyr Thr Phe Leu Pro Ser Leu Ala Glu
            580                 585                 590

Lys Val Asn Lys Gly Val Gln Ala Gly Leu Phe Leu Asn Trp Ala Asn
        595                 600                 605

Glu Val Val Glu Asp Phe Thr Thr Asn Ile Met Lys Lys Asp Thr Leu
    610                 615                 620

Asp Lys Ile Ser Asp Val Ser Val Ile Ile Pro Tyr Ile Gly Pro Ala
625                 630                 635                 640

Leu Asn Ile Gly Asn Ser Ala Leu Arg Gly Asn Phe Asn Gln Ala Phe
                645                 650                 655

Ala Thr Ala Gly Val Ala Phe Leu Leu Glu Gly Phe Pro Glu Phe Thr
            660                 665                 670

Ile Pro Ala Leu Gly Val Phe Thr Phe Tyr Ser Ser Ile Gln Glu Arg
        675                 680                 685

Glu Lys Ile Ile Lys Thr Ile Glu Asn Cys Leu Glu Gln Arg Val Lys
    690                 695                 700

Arg Trp Lys Asp Ser Tyr Gln Trp Met Val Ser Asn Trp Leu Ser Arg
705                 710                 715                 720

Ile Thr Thr Gln Phe Asn His Ile Asn Tyr Gln Met Tyr Asp Ser Leu
                725                 730                 735

Ser Tyr Gln Ala Asp Ala Ile Lys Ala Lys Ile Asp Leu Glu Tyr Lys
            740                 745                 750

Lys Tyr Ser Gly Ser Asp Lys Glu Asn Ile Lys Ser Gln Val Glu Asn
        755                 760                 765

Leu Lys Asn Ser Leu Asp Val Lys Ile Ser Glu Ala Met Asn Asn Ile
    770                 775                 780
```

Asn Lys Phe Ile Arg Glu Cys Ser Val Thr Tyr Leu Phe Lys Asn Met
785 790 795 800

Leu Pro Lys Val Ile Asp Glu Leu Asn Lys Phe Asp Leu Arg Thr Lys
805 810 815

Thr Glu Leu Ile Asn Leu Ile Asp Ser His Asn Ile Ile Leu Val Gly
820 825 830

Glu Val Asp Arg Leu Lys Ala Lys Val Asn Glu Ser Phe Glu Asn Thr
835 840 845

Met Pro Phe Asn Ile Phe Ser Tyr Thr Asn Asn Ser Leu Leu Lys Asp
850 855 860

Ile Ile Asn Glu Tyr Phe Asn Leu Glu Gly Gly Gly Gly Ser Gly Gly
865 870 875 880

Gly Gly Ser Gly Gly Gly Gly Ser Gly Gly Gly Gly Ser Gly Gly Gly
885 890 895

Gly Ser Ala Leu Val Pro Cys Lys Asn Phe Phe Trp Lys Thr Phe Ser
900 905 910

Ser Cys Lys
915

<210> 17
<211> 2779
<212> DNA
<213> Artificial Sequence

<220>
<221> source
<223> /note="Description of Artificial Sequence: Synthetic polynucleotide"

<400> 17
ggatccatga cgtggccagt taaggatttc aactactcag atcctgtaaa tgacaacgat 60

attctgtacc ttcgcattcc acaaaataaa ctgatcacca caccagtcaa agcattcatg 120

attactcaaa acatttgggt cattccagaa cgcttttcta gtgacacaaa tccgagttta 180

tctaaacctc cgcgtccgac gtccaaatat cagagctatt acgatccctc atatctcagt 240

acggacgaac aaaaagatac tttccttaaa ggtatcatta aactgtttaa gcgtattaat 300

gagcgcgata tcgggaaaaa gttgattaat tatcttgttg tgggttcccc gttcatgggc 360

gatagctcta cccccgaaga cacttttgat tttacccgtc atacgacaaa catcgcggta 420

gagaagtttg agaacggatc gtggaaagtc acaaacatca ttacacctag cgtcttaatt 480

tttggtccgc tgccaaacat cttagattat acagccagcc tgactttgca ggggcaacag 540
tcgaatccga gtttcgaagg ttttggtacc ctgagcattc tgaaagttgc cccggaattt 600
ctgctcactt tttcagatgt caccagcaac cagagctcag cagtattagg aaagtcaatt 660
ttttgcatgg acccggttat tgcactgatg cacgaactga cgcactctct gcatcaactg 720
tatgggatca acatccccag tgacaaacgt attcgtcccc aggtgtctga aggatttttc 780
tcacaggatg ggccgaacgt ccagttcgaa gagttgtata ctttcggagg cctggacgta 840
gagatcattc cccagattga gcgcagtcag ctgcgtgaga aggcattggg ccattataag 900
gatattgcaa aacgcctgaa taacattaac aaaacgattc catcttcgtg gatctcgaat 960
attgataaat ataagaaaat ttttagcgag aaatataatt ttgataaaga taatacaggt 1020
aactttgtgg ttaacattga caaattcaac tccctttaca gtgatttgac gaatgtaatg 1080
agcgaagttg tgtatagttc ccaatacaac gttaagaatc gtacccatta cttctctcgt 1140
cactacctgc cggttttcgc gaacatcctt gacgataata tttacactat tcgtgacggc 1200
tttaacttga ccaacaaggg cttcaatatt gaaaattcag gccagaacat tgaacgcaac 1260
ccggccttgc agaaactgtc gagtgaatcc gtggttgacc tgtttaccaa agtctgcgtc 1320
gacaaaagcg aagagaagct gtacgatgac gatgacaaag atcgttgggg atcgtccctg 1380
cagtgtatta aagtgaaaaa caatcggctg ccttatgtag cagataaaga tagcattagt 1440
caggagattt tcgaaaataa aattatcact gacgaaacca atgttcagaa ttattcagat 1500
aaattttcac tggacgaaag catcttagat ggccaagttc cgattaaccc ggaaattgtt 1560
gatccgttac tgccgaacgt gaatatggaa ccgttaaacc tccctggcga agagatcgta 1620
ttttatgatg acattacgaa atatgtggac taccttaatt cttattacta tttggaaagc 1680
cagaaactgt ccaataacgt ggaaaacatt actctgacca caagcgtgga agaggcttta 1740
ggctactcaa ataagattta taccttcctc ccgtcgctgg cggaaaaagt aaataaaggt 1800
gtgcaggctg gtctgttcct caactgggcg aatgaagttg tcgaagactt taccacgaat 1860
attatgaaaa aggataccct ggataaaatc tccgacgtct cggttattat cccatatatt 1920
ggccctgcgt taaatatcgg taatagtgcg ctgcggggga attttaacca ggcctttgct 1980
accgcgggcg tcgcgttcct cctggagggc tttcctgaat ttactatccc ggcgctcggt 2040
gtttttacat tttactcttc catccaggag cgtgagaaaa ttatcaaaac catcgaaaac 2100
tgcctggagc agcgggtgaa acgctggaaa gattcttatc aatggatggt gtcaaactgg 2160
ttatctcgca tcacgaccca attcaaccat attaattacc agatgtatga tagtctgtcg 2220
taccaagctg acgccattaa agccaaaatt gatctggaat ataaaaagta ctctggtagc 2280
gataaggaga acatcaaaag ccaggtggag aaccttaaga atagtctgga tgtgaaaatc 2340
tctgaagcta tgaataacat taacaaattc attcgtgaat gttcggtgac gtacctgttc 2400

aagaatatgc tgccaaaagt tattgatgaa ctgaataaat ttgatctgcg taccaaaacc 2460

gaacttatca acctcatcga ctcccacaac attatccttg tgggcgaagt ggatcgtctg 2520

aaggccaaag taaacgagag ctttgaaaat acgatgccgt ttaatatttt ttcatatacc 2580

aataactcct tgctgaaaga tatcatcaat gaatatttca atctagaagg cggtggcggt 2640

agcggcggtg gcggtagcgg cggtggcggt agcgcactag tgcaggaaag acctccatta 2700

caacaacctc cacatcgcga taagaaacca tgtaagaatt tcttttggaa aacatttagc 2760

agttgcaaat aataagctt 2779

<210> 18
<211> 920
<212> PRT
<213> Artificial Sequence

<220>
<221> source
<223> /note="Description of Artificial Sequence: Synthetic
polypeptide"

<400> 18
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Lys Ser Glu Glu Lys Leu Tyr Asp Asp Asp
435 440 445

Asp Lys Asp Arg Trp Gly Ser Ser Leu Gln Cys Ile Lys Val Lys Asn
450 455 460

Asn Arg Leu Pro Tyr Val Ala Asp Lys Asp Ser Ile Ser Gln Glu Ile
465 470 475 480

Phe Glu Asn Lys Ile Ile Thr Asp Glu Thr Asn Val Gln Asn Tyr Ser
485 490 495

Asp Lys Phe Ser Leu Asp Glu Ser Ile Leu Asp Gly Gln Val Pro Ile
500 505 510

Asn Pro Glu Ile Val Asp Pro Leu Leu Pro Asn Val Asn Met Glu Pro
515 520 525

Leu Asn Leu Pro Gly Glu Glu Ile Val Phe Tyr Asp Asp Ile Thr Lys
530 535 540

Tyr Val Asp Tyr Leu Asn Ser Tyr Tyr Tyr Leu Glu Ser Gln Lys Leu
545 550 555 560

Ser Asn Asn Val Glu Asn Ile Thr Leu Thr Thr Ser Val Glu Glu Ala
565 570 575

Leu Gly Tyr Ser Asn Lys Ile Tyr Thr Phe Leu Pro Ser Leu Ala Glu
580 585 590

Lys Val Asn Lys Gly Val Gln Ala Gly Leu Phe Leu Asn Trp Ala Asn
595 600 605

Glu Val Val Glu Asp Phe Thr Thr Asn Ile Met Lys Lys Asp Thr Leu
610 615 620

Asp Lys Ile Ser Asp Val Ser Val Ile Ile Pro Tyr Ile Gly Pro Ala
625 630 635 640

Leu Asn Ile Gly Asn Ser Ala Leu Arg Gly Asn Phe Asn Gln Ala Phe
645 650 655

```
Ala Thr Ala Gly Val Ala Phe Leu Leu Glu Gly Phe Pro Glu Phe Thr
            660                 665                 670

Ile Pro Ala Leu Gly Val Phe Thr Phe Tyr Ser Ser Ile Gln Glu Arg
        675                 680                 685

Glu Lys Ile Ile Lys Thr Ile Glu Asn Cys Leu Glu Gln Arg Val Lys
    690                 695                 700

Arg Trp Lys Asp Ser Tyr Gln Trp Met Val Ser Asn Trp Leu Ser Arg
705                 710                 715                 720

Ile Thr Thr Gln Phe Asn His Ile Asn Tyr Gln Met Tyr Asp Ser Leu
                725                 730                 735

Ser Tyr Gln Ala Asp Ala Ile Lys Ala Lys Ile Asp Leu Glu Tyr Lys
            740                 745                 750

Lys Tyr Ser Gly Ser Asp Lys Glu Asn Ile Lys Ser Gln Val Glu Asn
        755                 760                 765

Leu Lys Asn Ser Leu Asp Val Lys Ile Ser Glu Ala Met Asn Asn Ile
    770                 775                 780

Asn Lys Phe Ile Arg Glu Cys Ser Val Thr Tyr Leu Phe Lys Asn Met
785                 790                 795                 800

Leu Pro Lys Val Ile Asp Glu Leu Asn Lys Phe Asp Leu Arg Thr Lys
                805                 810                 815

Thr Glu Leu Ile Asn Leu Ile Asp Ser His Asn Ile Ile Leu Val Gly
            820                 825                 830

Glu Val Asp Arg Leu Lys Ala Lys Val Asn Glu Ser Phe Glu Asn Thr
        835                 840                 845

Met Pro Phe Asn Ile Phe Ser Tyr Thr Asn Asn Ser Leu Leu Lys Asp
    850                 855                 860

Ile Ile Asn Glu Tyr Phe Asn Leu Glu Gly Gly Gly Gly Ser Gly Gly
865                 870                 875                 880

Gly Gly Ser Gly Gly Gly Gly Ser Ala Leu Val Gln Glu Arg Pro Pro
                885                 890                 895

Leu Gln Gln Pro Pro His Arg Asp Lys Lys Pro Cys Lys Asn Phe Phe
```

900 905 910

Trp Lys Thr Phe Ser Ser Cys Lys
915 920

<210> 19
<211> 930
<212> PRT
<213> Artificial Sequence

<220>
<221> source
<223> /note="Description of Artificial Sequence: Synthetic polypeptide"

<400> 19
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys

180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Lys Ser Glu Glu Lys Leu Tyr Asp Asp Asp
435 440 445

Asp Lys Asp Arg Trp Gly Ser Ser Leu Gln Cys Ile Lys Val Lys Asn
450 455 460

Asn Arg Leu Pro Tyr Val Ala Asp Lys Asp Ser Ile Ser Gln Glu Ile
465 470 475 480

Phe Glu Asn Lys Ile Ile Thr Asp Glu Thr Asn Val Gln Asn Tyr Ser
485 490 495

Asp Lys Phe Ser Leu Asp Glu Ser Ile Leu Asp Gly Gln Val Pro Ile
500 505 510

Asn Pro Glu Ile Val Asp Pro Leu Leu Pro Asn Val Asn Met Glu Pro
515 520 525

Leu Asn Leu Pro Gly Glu Glu Ile Val Phe Tyr Asp Asp Ile Thr Lys
530 535 540

Tyr Val Asp Tyr Leu Asn Ser Tyr Tyr Tyr Leu Glu Ser Gln Lys Leu
545 550 555 560

Ser Asn Asn Val Glu Asn Ile Thr Leu Thr Thr Ser Val Glu Glu Ala
565 570 575

Leu Gly Tyr Ser Asn Lys Ile Tyr Thr Phe Leu Pro Ser Leu Ala Glu
580 585 590

Lys Val Asn Lys Gly Val Gln Ala Gly Leu Phe Leu Asn Trp Ala Asn
595 600 605

Glu Val Val Glu Asp Phe Thr Thr Asn Ile Met Lys Lys Asp Thr Leu
610 615 620

Asp Lys Ile Ser Asp Val Ser Val Ile Ile Pro Tyr Ile Gly Pro Ala
625 630 635 640

Leu Asn Ile Gly Asn Ser Ala Leu Arg Gly Asn Phe Asn Gln Ala Phe
645 650 655

Ala Thr Ala Gly Val Ala Phe Leu Leu Glu Gly Phe Pro Glu Phe Thr
660 665 670

Ile Pro Ala Leu Gly Val Phe Thr Phe Tyr Ser Ser Ile Gln Glu Arg
675 680 685

Glu Lys Ile Ile Lys Thr Ile Glu Asn Cys Leu Glu Gln Arg Val Lys
690 695 700

Arg Trp Lys Asp Ser Tyr Gln Trp Met Val Ser Asn Trp Leu Ser Arg
705 710 715 720

Ile Thr Thr Gln Phe Asn His Ile Asn Tyr Gln Met Tyr Asp Ser Leu
725 730 735

Ser Tyr Gln Ala Asp Ala Ile Lys Ala Lys Ile Asp Leu Glu Tyr Lys
740 745 750

Lys Tyr Ser Gly Ser Asp Lys Glu Asn Ile Lys Ser Gln Val Glu Asn
755 760 765

Leu Lys Asn Ser Leu Asp Val Lys Ile Ser Glu Ala Met Asn Asn Ile
770 775 780

Asn Lys Phe Ile Arg Glu Cys Ser Val Thr Tyr Leu Phe Lys Asn Met
785 790 795 800

Leu Pro Lys Val Ile Asp Glu Leu Asn Lys Phe Asp Leu Arg Thr Lys
805 810 815

Thr Glu Leu Ile Asn Leu Ile Asp Ser His Asn Ile Ile Leu Val Gly
820 825 830

Glu Val Asp Arg Leu Lys Ala Lys Val Asn Glu Ser Phe Glu Asn Thr
835 840 845

Met Pro Phe Asn Ile Phe Ser Tyr Thr Asn Asn Ser Leu Leu Lys Asp
850 855 860

Ile Ile Asn Glu Tyr Phe Asn Leu Glu Gly Gly Gly Gly Ser Gly Gly
865 870 875 880

Gly Gly Ser Gly Gly Gly Gly Ser Gly Gly Gly Gly Ser Gly Gly Gly
885 890 895

Gly Ser Ala Leu Val Gln Glu Arg Pro Pro Leu Gln Gln Pro Pro His
900 905 910

Arg Asp Lys Lys Pro Cys Lys Asn Phe Phe Trp Lys Thr Phe Ser Ser
915 920 925

Cys Lys
930

<210> 20
<211> 902
<212> PRT
<213> Artificial Sequence

<220>
<221> source
<223> /note="Description of Artificial Sequence: Synthetic polypeptide"

<400> 20
Thr Trp Pro Val Lys Asp Phe Asn Tyr Ser Asp Pro Val Asn Asp Asn
1               5                   10                  15

Asp Ile Leu Tyr Leu Arg Ile Pro Gln Asn Lys Leu Ile Thr Thr Pro
            20                  25                  30

Val Lys Ala Phe Met Ile Thr Gln Asn Ile Trp Val Ile Pro Glu Arg
        35                  40                  45

Phe Ser Ser Asp Thr Asn Pro Ser Leu Ser Lys Pro Pro Arg Pro Thr
    50                  55                  60

Ser Lys Tyr Gln Ser Tyr Tyr Asp Pro Ser Tyr Leu Ser Thr Asp Glu
65                  70                  75                  80

Gln Lys Asp Thr Phe Leu Lys Gly Ile Ile Lys Leu Phe Lys Arg Ile
                85                  90                  95

Asn Glu Arg Asp Ile Gly Lys Lys Leu Ile Asn Tyr Leu Val Val Gly
            100                 105                 110

Ser Pro Phe Met Gly Asp Ser Ser Thr Pro Glu Asp Thr Phe Asp Phe
        115                 120                 125

Thr Arg His Thr Thr Asn Ile Ala Val Glu Lys Phe Glu Asn Gly Ser
    130                 135                 140

Trp Lys Val Thr Asn Ile Ile Thr Pro Ser Val Leu Ile Phe Gly Pro
145                 150                 155                 160

Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
225                 230                 235                 240

Asn Ile Pro Ser Asp Lys Arg Ile Arg Pro Gln Val Ser Glu Gly Phe
                245                 250                 255

Phe Ser Ile Asp Gly Arg Asn Val Gln Phe Glu Glu Leu Tyr Thr Phe
            260                 265                 270

Gly Gly Leu Asp Val Glu Ile Ile Pro Gln Ile Glu Arg Ser Gln Leu
        275                 280                 285

Arg Glu Lys Ala Leu Gly His Tyr Lys Asp Ile Ala Lys Arg Leu Asn
    290                 295                 300

Asn Ile Asn Lys Thr Ile Pro Ser Ser Trp Ile Ser Asn Ile Asp Lys
305                 310                 315                 320

Tyr Lys Lys Ile Phe Ser Glu Lys Tyr Asn Phe Asp Lys Asp Asn Thr
                325                 330                 335

Gly Asn Phe Val Val Asn Ile Asp Lys Phe Asn Ser Leu Tyr Ser Asp
            340                 345                 350

Leu Thr Asn Val Met Ser Glu Val Val Tyr Ser Ser Gln Tyr Asn Val
        355                 360                 365

Lys Asn Arg Thr His Tyr Phe Ser Arg His Tyr Leu Pro Val Phe Ala
    370                 375                 380

Asn Ile Leu Asp Asp Asn Ile Tyr Thr Ile Arg Asp Gly Phe Asn Leu
385                 390                 395                 400

Thr Asn Lys Gly Phe Asn Ile Glu Asn Ser Gly Gln Asn Ile Glu Arg
                405                 410                 415

Asn Pro Ala Leu Gln Lys Leu Ser Ser Glu Ser Val Val Asp Leu Phe
            420                 425                 430

Thr Lys Val Cys Val Asp Lys Ser Glu Glu Lys Leu Tyr Ile Asp Gly
        435                 440                 445

Arg Trp Gly Ser Ser Leu Gln Cys Ile Lys Val Lys Asn Asn Arg Leu

```
    450                 455                 460

Pro Tyr Val Ala Asp Lys Asp Ser Ile Ser Gln Glu Ile Phe Glu Asn
465                 470                 475                 480

Lys Ile Ile Thr Asp Glu Thr Asn Val Gln Asn Tyr Ser Asp Lys Phe
                485                 490                 495

Ser Leu Asp Glu Ser Ile Leu Asp Gly Gln Val Pro Ile Asn Pro Glu
            500                 505                 510

Ile Val Asp Pro Leu Leu Pro Asn Val Asn Met Glu Pro Leu Asn Leu
        515                 520                 525

Pro Gly Glu Glu Ile Val Phe Tyr Asp Asp Ile Thr Lys Tyr Val Asp
    530                 535                 540

Tyr Leu Asn Ser Tyr Tyr Tyr Leu Glu Ser Gln Lys Leu Ser Asn Asn
545                 550                 555                 560

Val Glu Asn Ile Thr Leu Thr Thr Ser Val Glu Glu Ala Leu Gly Tyr
                565                 570                 575

Ser Asn Lys Ile Tyr Thr Phe Leu Pro Ser Leu Ala Glu Lys Val Asn
            580                 585                 590

Lys Gly Val Gln Ala Gly Leu Phe Leu Asn Trp Ala Asn Glu Val Val
        595                 600                 605

Glu Asp Phe Thr Thr Asn Ile Met Lys Lys Asp Thr Leu Asp Lys Ile
    610                 615                 620

Ser Asp Val Ser Val Ile Ile Pro Tyr Ile Gly Pro Ala Leu Asn Ile
625                 630                 635                 640

Gly Asn Ser Ala Leu Arg Gly Asn Phe Asn Gln Ala Phe Ala Thr Ala
                645                 650                 655

Gly Val Ala Phe Leu Leu Glu Gly Phe Pro Glu Phe Thr Ile Pro Ala
            660                 665                 670

Leu Gly Val Phe Thr Phe Tyr Ser Ser Ile Gln Glu Arg Glu Lys Ile
        675                 680                 685

Ile Lys Thr Ile Glu Asn Cys Leu Glu Gln Arg Val Lys Arg Trp Lys
    690                 695                 700
```

Asp Ser Tyr Gln Trp Met Val Ser Asn Trp Leu Ser Arg Ile Thr Thr
705 710 715 720

Gln Phe Asn His Ile Asn Tyr Gln Met Tyr Asp Ser Leu Ser Tyr Gln
725 730 735

Ala Asp Ala Ile Lys Ala Lys Ile Asp Leu Glu Tyr Lys Lys Tyr Ser
740 745 750

Gly Ser Asp Lys Glu Asn Ile Lys Ser Gln Val Glu Asn Leu Lys Asn
755 760 765

Ser Leu Asp Val Lys Ile Ser Glu Ala Met Asn Asn Ile Asn Lys Phe
770 775 780

Ile Arg Glu Cys Ser Val Thr Tyr Leu Phe Lys Asn Met Leu Pro Lys
785 790 795 800

Val Ile Asp Glu Leu Asn Lys Phe Asp Leu Arg Thr Lys Thr Glu Leu
805 810 815

Ile Asn Leu Ile Asp Ser His Asn Ile Ile Leu Val Gly Glu Val Asp
820 825 830

Arg Leu Lys Ala Lys Val Asn Glu Ser Phe Glu Asn Thr Met Pro Phe
835 840 845

Asn Ile Phe Ser Tyr Thr Asn Asn Ser Leu Leu Lys Asp Ile Ile Asn
850 855 860

Glu Tyr Phe Asn Leu Glu Gly Gly Gly Gly Ser Gly Gly Gly Gly Ser
865 870 875 880

Gly Gly Gly Gly Ser Ala Leu Val Ala Gly Cys Lys Asn Phe Phe Trp
885 890 895

Lys Thr Phe Thr Ser Cys
900

<210> 21
<211> 915
<212> PRT
<213> Artificial Sequence

<220>
<221> source
<223> /note="Description of Artificial Sequence: Synthetic
polypeptide"

<400> 21

```
Thr Trp Pro Val Lys Asp Phe Asn Tyr Ser Asp Pro Val Asn Asp Asn
1               5                   10                  15

Asp Ile Leu Tyr Leu Arg Ile Pro Gln Asn Lys Leu Ile Thr Thr Pro
            20                  25                  30

Val Lys Ala Phe Met Ile Thr Gln Asn Ile Trp Val Ile Pro Glu Arg
        35                  40                  45

Phe Ser Ser Asp Thr Asn Pro Ser Leu Ser Lys Pro Pro Arg Pro Thr
    50                  55                  60

Ser Lys Tyr Gln Ser Tyr Tyr Asp Pro Ser Tyr Leu Ser Thr Asp Glu
65                  70                  75                  80

Gln Lys Asp Thr Phe Leu Lys Gly Ile Ile Lys Leu Phe Lys Arg Ile
                85                  90                  95

Asn Glu Arg Asp Ile Gly Lys Lys Leu Ile Asn Tyr Leu Val Val Gly
            100                 105                 110

Ser Pro Phe Met Gly Asp Ser Ser Thr Pro Glu Asp Thr Phe Asp Phe
        115                 120                 125

Thr Arg His Thr Thr Asn Ile Ala Val Glu Lys Phe Glu Asn Gly Ser
    130                 135                 140

Trp Lys Val Thr Asn Ile Ile Thr Pro Ser Val Leu Ile Phe Gly Pro
145                 150                 155                 160

Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
225                 230                 235                 240

Asn Ile Pro Ser Asp Lys Arg Ile Arg Pro Gln Val Ser Glu Gly Phe
                245                 250                 255
```

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Lys Ser Glu Glu Lys Leu Tyr Asp Asp Asp
435 440 445

Asp Lys Asp Arg Trp Gly Ser Ser Leu Gln Cys Ile Lys Val Lys Asn
450 455 460

Asn Arg Leu Pro Tyr Val Ala Asp Lys Asp Ser Ile Ser Gln Glu Ile
465 470 475 480

Phe Glu Asn Lys Ile Ile Thr Asp Glu Thr Asn Val Gln Asn Tyr Ser
485 490 495

Asp Lys Phe Ser Leu Asp Glu Ser Ile Leu Asp Gly Gln Val Pro Ile
500 505 510

Asn Pro Glu Ile Val Asp Pro Leu Leu Pro Asn Val Asn Met Glu Pro
515 520 525

Leu Asn Leu Pro Gly Glu Glu Ile Val Phe Tyr Asp Asp Ile Thr Lys
530 535 540

Tyr Val Asp Tyr Leu Asn Ser Tyr Tyr Tyr Leu Glu Ser Gln Lys Leu
545 550 555 560

Ser Asn Asn Val Glu Asn Ile Thr Leu Thr Thr Ser Val Glu Glu Ala
565 570 575

Leu Gly Tyr Ser Asn Lys Ile Tyr Thr Phe Leu Pro Ser Leu Ala Glu
580 585 590

Lys Val Asn Lys Gly Val Gln Ala Gly Leu Phe Leu Asn Trp Ala Asn
595 600 605

Glu Val Val Glu Asp Phe Thr Thr Asn Ile Met Lys Lys Asp Thr Leu
610 615 620

Asp Lys Ile Ser Asp Val Ser Val Ile Ile Pro Tyr Ile Gly Pro Ala
625 630 635 640

Leu Asn Ile Gly Asn Ser Ala Leu Arg Gly Asn Phe Asn Gln Ala Phe
645 650 655

Ala Thr Ala Gly Val Ala Phe Leu Leu Glu Gly Phe Pro Glu Phe Thr
660 665 670

Ile Pro Ala Leu Gly Val Phe Thr Phe Tyr Ser Ser Ile Gln Glu Arg
675 680 685

Glu Lys Ile Ile Lys Thr Ile Glu Asn Cys Leu Glu Gln Arg Val Lys
690 695 700

Arg Trp Lys Asp Ser Tyr Gln Trp Met Val Ser Asn Trp Leu Ser Arg
705 710 715 720

Ile Thr Thr Gln Phe Asn His Ile Asn Tyr Gln Met Tyr Asp Ser Leu
725 730 735

Ser Tyr Gln Ala Asp Ala Ile Lys Ala Lys Ile Asp Leu Glu Tyr Lys
740 745 750

Lys Tyr Ser Gly Ser Asp Lys Glu Asn Ile Lys Ser Gln Val Glu Asn

755 760 765

Leu Lys Asn Ser Leu Asp Val Lys Ile Ser Glu Ala Met Asn Asn Ile
770 775 780

Asn Lys Phe Ile Arg Glu Cys Ser Val Thr Tyr Leu Phe Lys Asn Met
785 790 795 800

Leu Pro Lys Val Ile Asp Glu Leu Asn Lys Phe Asp Leu Arg Thr Lys
805 810 815

Thr Glu Leu Ile Asn Leu Ile Asp Ser His Asn Ile Ile Leu Val Gly
820 825 830

Glu Val Asp Arg Leu Lys Ala Lys Val Asn Glu Ser Phe Glu Asn Thr
835 840 845

Met Pro Phe Asn Ile Phe Ser Tyr Thr Asn Asn Ser Leu Leu Lys Asp
850 855 860

Ile Ile Asn Glu Tyr Phe Asn Leu Glu Gly Gly Gly Gly Ser Gly Gly
865 870 875 880

Gly Gly Ser Gly Gly Gly Gly Ser Gly Gly Gly Gly Ser Gly Gly Gly
885 890 895

Gly Ser Ala Leu Val Ala Gly Cys Lys Asn Phe Phe Trp Lys Thr Phe
900 905 910

Thr Ser Cys
915

<210> 22
<211> 919
<212> PRT
<213> Artificial Sequence

<220>
<221> source
<223> /note="Description of Artificial Sequence: Synthetic polypeptide"

<400> 22
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg

35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Lys Ser Glu Glu Lys Leu Tyr Asp Asp Asp
435 440 445

Asp Lys Asp Arg Trp Gly Ser Ser Leu Gln Cys Ile Lys Val Lys Asn
450 455 460

Asn Arg Leu Pro Tyr Val Ala Asp Lys Asp Ser Ile Ser Gln Glu Ile
465 470 475 480

Phe Glu Asn Lys Ile Ile Thr Asp Glu Thr Asn Val Gln Asn Tyr Ser
485 490 495

Asp Lys Phe Ser Leu Asp Glu Ser Ile Leu Asp Gly Gln Val Pro Ile
500 505 510

Asn Pro Glu Ile Val Asp Pro Leu Leu Pro Asn Val Asn Met Glu Pro
515 520 525

Leu Asn Leu Pro Gly Glu Glu Ile Val Phe Tyr Asp Asp Ile Thr Lys
530 535 540

Tyr Val Asp Tyr Leu Asn Ser Tyr Tyr Tyr Leu Glu Ser Gln Lys Leu
545 550 555 560

Ser Asn Asn Val Glu Asn Ile Thr Leu Thr Thr Ser Val Glu Glu Ala
565 570 575

Leu Gly Tyr Ser Asn Lys Ile Tyr Thr Phe Leu Pro Ser Leu Ala Glu
580 585 590

Lys Val Asn Lys Gly Val Gln Ala Gly Leu Phe Leu Asn Trp Ala Asn
595 600 605

Glu Val Val Glu Asp Phe Thr Thr Asn Ile Met Lys Lys Asp Thr Leu
610 615 620

Asp Lys Ile Ser Asp Val Ser Val Ile Ile Pro Tyr Ile Gly Pro Ala
625 630 635 640

Leu Asn Ile Gly Asn Ser Ala Leu Arg Gly Asn Phe Asn Gln Ala Phe
645 650 655

Ala Thr Ala Gly Val Ala Phe Leu Leu Glu Gly Phe Pro Glu Phe Thr
660 665 670

Ile Pro Ala Leu Gly Val Phe Thr Phe Tyr Ser Ser Ile Gln Glu Arg
675 680 685

Glu Lys Ile Ile Lys Thr Ile Glu Asn Cys Leu Glu Gln Arg Val Lys
690 695 700

Arg Trp Lys Asp Ser Tyr Gln Trp Met Val Ser Asn Trp Leu Ser Arg
705 710 715 720

Ile Thr Thr Gln Phe Asn His Ile Asn Tyr Gln Met Tyr Asp Ser Leu
725 730 735

Ser Tyr Gln Ala Asp Ala Ile Lys Ala Lys Ile Asp Leu Glu Tyr Lys
740 745 750

Lys Tyr Ser Gly Ser Asp Lys Glu Asn Ile Lys Ser Gln Val Glu Asn
755 760 765

Leu Lys Asn Ser Leu Asp Val Lys Ile Ser Glu Ala Met Asn Asn Ile
770 775 780

Asn Lys Phe Ile Arg Glu Cys Ser Val Thr Tyr Leu Phe Lys Asn Met
785 790 795 800

Leu Pro Lys Val Ile Asp Glu Leu Asn Lys Phe Asp Leu Arg Thr Lys
805 810 815

Thr Glu Leu Ile Asn Leu Ile Asp Ser His Asn Ile Ile Leu Val Gly
820 825 830

Glu Val Asp Arg Leu Lys Ala Lys Val Asn Glu Ser Phe Glu Asn Thr
835 840 845

Met Pro Phe Asn Ile Phe Ser Tyr Thr Asn Asn Ser Leu Leu Lys Asp
850 855 860

Ile Ile Asn Glu Tyr Phe Asn Leu Glu Gly Gly Gly Gly Ser Gly Gly
865 870 875 880

Gly Gly Ser Gly Gly Gly Gly Ser Ala Leu Val Ser Ala Asn Ser Asn
885 890 895

Pro Ala Met Ala Pro Arg Glu Arg Lys Ala Gly Cys Lys Asn Phe Phe
900 905 910

Trp Lys Thr Phe Thr Ser Cys
915

<210> 23
<211> 929
<212> PRT
<213> Artificial Sequence

<220>
<221> source
<223> /note="Description of Artificial Sequence: Synthetic polypeptide"

<400> 23
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

```
Gln Lys Asp Thr Phe Leu Lys Gly Ile Ile Lys Leu Phe Lys Arg Ile
                85                  90                  95

Asn Glu Arg Asp Ile Gly Lys Lys Leu Ile Asn Tyr Leu Val Val Gly
            100                 105                 110

Ser Pro Phe Met Gly Asp Ser Ser Thr Pro Glu Asp Thr Phe Asp Phe
        115                 120                 125

Thr Arg His Thr Thr Asn Ile Ala Val Glu Lys Phe Glu Asn Gly Ser
    130                 135                 140

Trp Lys Val Thr Asn Ile Ile Thr Pro Ser Val Leu Ile Phe Gly Pro
145                 150                 155                 160

Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
225                 230                 235                 240

Asn Ile Pro Ser Asp Lys Arg Ile Arg Pro Gln Val Ser Glu Gly Phe
                245                 250                 255

Phe Ser Gln Asp Gly Pro Asn Val Gln Phe Glu Glu Leu Tyr Thr Phe
            260                 265                 270

Gly Gly Leu Asp Val Glu Ile Ile Pro Gln Ile Glu Arg Ser Gln Leu
        275                 280                 285

Arg Glu Lys Ala Leu Gly His Tyr Lys Asp Ile Ala Lys Arg Leu Asn
    290                 295                 300

Asn Ile Asn Lys Thr Ile Pro Ser Ser Trp Ile Ser Asn Ile Asp Lys
305                 310                 315                 320

Tyr Lys Lys Ile Phe Ser Glu Lys Tyr Asn Phe Asp Lys Asp Asn Thr
```

325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Lys Ser Glu Glu Lys Leu Tyr Asp Asp Asp
435 440 445

Asp Lys Asp Arg Trp Gly Ser Ser Leu Gln Cys Ile Lys Val Lys Asn
450 455 460

Asn Arg Leu Pro Tyr Val Ala Asp Lys Asp Ser Ile Ser Gln Glu Ile
465 470 475 480

Phe Glu Asn Lys Ile Ile Thr Asp Glu Thr Asn Val Gln Asn Tyr Ser
485 490 495

Asp Lys Phe Ser Leu Asp Glu Ser Ile Leu Asp Gly Gln Val Pro Ile
500 505 510

Asn Pro Glu Ile Val Asp Pro Leu Leu Pro Asn Val Asn Met Glu Pro
515 520 525

Leu Asn Leu Pro Gly Glu Glu Ile Val Phe Tyr Asp Asp Ile Thr Lys
530 535 540

Tyr Val Asp Tyr Leu Asn Ser Tyr Tyr Tyr Leu Glu Ser Gln Lys Leu
545 550 555 560

Ser Asn Asn Val Glu Asn Ile Thr Leu Thr Thr Ser Val Glu Glu Ala
565 570 575

```
Leu Gly Tyr Ser Asn Lys Ile Tyr Thr Phe Leu Pro Ser Leu Ala Glu
            580                 585                 590

Lys Val Asn Lys Gly Val Gln Ala Gly Leu Phe Leu Asn Trp Ala Asn
        595                 600                 605

Glu Val Val Glu Asp Phe Thr Thr Asn Ile Met Lys Lys Asp Thr Leu
    610                 615                 620

Asp Lys Ile Ser Asp Val Ser Val Ile Ile Pro Tyr Ile Gly Pro Ala
625                 630                 635                 640

Leu Asn Ile Gly Asn Ser Ala Leu Arg Gly Asn Phe Asn Gln Ala Phe
                645                 650                 655

Ala Thr Ala Gly Val Ala Phe Leu Leu Glu Gly Phe Pro Glu Phe Thr
            660                 665                 670

Ile Pro Ala Leu Gly Val Phe Thr Phe Tyr Ser Ser Ile Gln Glu Arg
        675                 680                 685

Glu Lys Ile Ile Lys Thr Ile Glu Asn Cys Leu Glu Gln Arg Val Lys
    690                 695                 700

Arg Trp Lys Asp Ser Tyr Gln Trp Met Val Ser Asn Trp Leu Ser Arg
705                 710                 715                 720

Ile Thr Thr Gln Phe Asn His Ile Asn Tyr Gln Met Tyr Asp Ser Leu
                725                 730                 735

Ser Tyr Gln Ala Asp Ala Ile Lys Ala Lys Ile Asp Leu Glu Tyr Lys
            740                 745                 750

Lys Tyr Ser Gly Ser Asp Lys Glu Asn Ile Lys Ser Gln Val Glu Asn
        755                 760                 765

Leu Lys Asn Ser Leu Asp Val Lys Ile Ser Glu Ala Met Asn Asn Ile
    770                 775                 780

Asn Lys Phe Ile Arg Glu Cys Ser Val Thr Tyr Leu Phe Lys Asn Met
785                 790                 795                 800

Leu Pro Lys Val Ile Asp Glu Leu Asn Lys Phe Asp Leu Arg Thr Lys
                805                 810                 815

Thr Glu Leu Ile Asn Leu Ile Asp Ser His Asn Ile Ile Leu Val Gly
            820                 825                 830
```

Glu Val Asp Arg Leu Lys Ala Lys Val Asn Glu Ser Phe Glu Asn Thr
835 840 845

Met Pro Phe Asn Ile Phe Ser Tyr Thr Asn Asn Ser Leu Leu Lys Asp
850 855 860

Ile Ile Asn Glu Tyr Phe Asn Leu Glu Gly Gly Gly Gly Ser Gly Gly
865 870 875 880

Gly Gly Ser Gly Gly Gly Gly Ser Gly Gly Gly Gly Ser Gly Gly Gly
885 890 895

Gly Ser Ala Leu Val Ser Ala Asn Ser Asn Pro Ala Met Ala Pro Arg
900 905 910

Glu Arg Lys Ala Gly Cys Lys Asn Phe Phe Trp Lys Thr Phe Thr Ser
915 920 925

Cys

<210> 24
<211> 919
<212> PRT
<213> Artificial Sequence

<220>
<221> source
<223> /note="Description of Artificial Sequence: Synthetic polypeptide"

<400> 24
Pro Ile Thr Ile Asn Asn Phe Asn Tyr Ser Asp Pro Val Asp Asn Lys
1 5 10 15

Asn Ile Leu Tyr Leu Asp Thr His Leu Asn Thr Leu Ala Asn Glu Pro
20 25 30

Glu Lys Ala Phe Arg Ile Thr Gly Asn Ile Trp Val Ile Pro Asp Arg
35 40 45

Phe Ser Arg Asn Ser Asn Pro Asn Leu Asn Lys Pro Pro Arg Val Thr
50 55 60

Ser Pro Lys Ser Gly Tyr Tyr Asp Pro Asn Tyr Leu Ser Thr Asp Ser
65 70 75 80

Asp Lys Asp Thr Phe Leu Lys Glu Ile Ile Lys Leu Phe Lys Arg Ile
85 90 95

Asn Ser Arg Glu Ile Gly Glu Glu Leu Ile Tyr Arg Leu Ser Thr Asp
100 105 110

Ile Pro Phe Pro Gly Asn Asn Asn Thr Pro Ile Asn Thr Phe Asp Phe
115 120 125

Asp Val Asp Phe Asn Ser Val Asp Val Lys Thr Arg Gln Gly Asn Asn
130 135 140

Trp Val Lys Thr Gly Ser Ile Asn Pro Ser Val Ile Ile Thr Gly Pro
145 150 155 160

Arg Glu Asn Ile Ile Asp Pro Glu Thr Ser Thr Phe Lys Leu Thr Asn
165 170 175

Asn Thr Phe Ala Ala Gln Glu Gly Phe Gly Ala Leu Ser Ile Ile Ser
180 185 190

Ile Ser Pro Arg Phe Met Leu Thr Tyr Ser Asn Ala Thr Asn Asp Val
195 200 205

Gly Glu Gly Arg Phe Ser Lys Ser Glu Phe Cys Met Asp Pro Ile Leu
210 215 220

Ile Leu Met His Glu Leu Asn His Ala Met His Asn Leu Tyr Gly Ile
225 230 235 240

Ala Ile Pro Asn Asp Gln Thr Ile Ser Ser Val Thr Ser Asn Ile Phe
245 250 255

Tyr Ser Gln Tyr Asn Val Lys Leu Glu Tyr Ala Glu Ile Tyr Ala Phe
260 265 270

Gly Gly Pro Thr Ile Asp Leu Ile Pro Lys Ser Ala Arg Lys Tyr Phe
275 280 285

Glu Glu Lys Ala Leu Asp Tyr Tyr Arg Ser Ile Ala Lys Arg Leu Asn
290 295 300

Ser Ile Thr Thr Ala Asn Pro Ser Ser Phe Asn Lys Tyr Ile Gly Glu
305 310 315 320

Tyr Lys Gln Lys Leu Ile Arg Lys Tyr Arg Phe Val Val Glu Ser Ser
325 330 335

Gly Glu Val Thr Val Asn Arg Asn Lys Phe Val Glu Leu Tyr Asn Glu
340 345 350

Leu Thr Gln Ile Phe Thr Glu Phe Asn Tyr Ala Lys Ile Tyr Asn Val
355 360 365

Gln Asn Arg Lys Ile Tyr Leu Ser Asn Val Tyr Thr Pro Val Thr Ala
370 375 380

Asn Ile Leu Asp Asp Asn Val Tyr Asp Ile Gln Asn Gly Phe Asn Ile
385 390 395 400

Pro Lys Ser Asn Leu Asn Val Leu Phe Met Gly Gln Asn Leu Ser Arg
405 410 415

Asn Pro Ala Leu Arg Lys Val Asn Pro Glu Asn Met Leu Tyr Leu Phe
420 425 430

Thr Lys Phe Cys Val Asp Ala Ile Asp Gly Arg Ser Leu Tyr Asn Lys
435 440 445

Thr Leu Gln Cys Arg Glu Leu Leu Val Lys Asn Thr Asp Leu Pro Phe
450 455 460

Ile Gly Asp Ile Ser Asp Val Lys Thr Asp Ile Phe Leu Arg Lys Asp
465 470 475 480

Ile Asn Glu Glu Thr Glu Val Ile Tyr Tyr Pro Asp Asn Val Ser Val
485 490 495

Asp Gln Val Ile Leu Ser Lys Asn Thr Ser Glu His Gly Gln Leu Asp
500 505 510

Leu Leu Tyr Pro Ser Ile Asp Ser Glu Ser Glu Ile Leu Pro Gly Glu
515 520 525

Asn Gln Val Phe Tyr Asp Asn Arg Thr Gln Asn Val Asp Tyr Leu Asn
530 535 540

Ser Tyr Tyr Tyr Leu Glu Ser Gln Lys Leu Ser Asp Asn Val Glu Asp
545 550 555 560

Phe Thr Phe Thr Arg Ser Ile Glu Glu Ala Leu Asp Asn Ser Ala Lys
565 570 575

Val Tyr Thr Tyr Phe Pro Thr Leu Ala Asn Lys Val Asn Ala Gly Val
580 585 590

Gln Gly Gly Leu Phe Leu Met Trp Ala Asn Asp Val Val Glu Asp Phe

595 600 605

Thr Thr Asn Ile Leu Arg Lys Asp Thr Leu Asp Lys Ile Ser Asp Val
610 615 620

Ser Ala Ile Ile Pro Tyr Ile Gly Pro Ala Leu Asn Ile Ser Asn Ser
625 630 635 640

Val Arg Arg Gly Asn Phe Thr Glu Ala Phe Ala Val Thr Gly Val Thr
645 650 655

Ile Leu Leu Glu Ala Phe Pro Glu Phe Thr Ile Pro Ala Leu Gly Ala
660 665 670

Phe Val Ile Tyr Ser Lys Val Gln Glu Arg Asn Glu Ile Ile Lys Thr
675 680 685

Ile Asp Asn Cys Leu Glu Gln Arg Ile Lys Arg Trp Lys Asp Ser Tyr
690 695 700

Glu Trp Met Met Gly Thr Trp Leu Ser Arg Ile Ile Thr Gln Phe Asn
705 710 715 720

Asn Ile Ser Tyr Gln Met Tyr Asp Ser Leu Asn Tyr Gln Ala Gly Ala
725 730 735

Ile Lys Ala Lys Ile Asp Leu Glu Tyr Lys Lys Tyr Ser Gly Ser Asp
740 745 750

Lys Glu Asn Ile Lys Ser Gln Val Glu Asn Leu Lys Asn Ser Leu Asp
755 760 765

Val Lys Ile Ser Glu Ala Met Asn Asn Ile Asn Lys Phe Ile Arg Glu
770 775 780

Cys Ser Val Thr Tyr Leu Phe Lys Asn Met Leu Pro Lys Val Ile Asp
785 790 795 800

Glu Leu Asn Glu Phe Asp Arg Asn Thr Lys Ala Lys Leu Ile Asn Leu
805 810 815

Ile Asp Ser His Asn Ile Ile Leu Val Gly Glu Val Asp Lys Leu Lys
820 825 830

Ala Lys Val Asn Asn Ser Phe Gln Asn Thr Ile Pro Phe Asn Ile Phe
835 840 845

Ser Tyr Thr Asn Asn Ser Leu Leu Lys Asp Ile Ile Asn Glu Tyr Phe
850 855 860

Asn Leu Glu Gly Gly Gly Gly Ser Gly Gly Gly Gly Ser Gly Gly Gly
865 870 875 880

Gly Ser Gly Gly Gly Gly Ser Gly Gly Gly Gly Ser Gly Gly Gly Gly
885 890 895

Ser Gly Gly Gly Gly Ser Ala Leu Val Ala Gly Cys Lys Asn Phe Phe
900 905 910

Trp Lys Thr Phe Thr Ser Cys
915

<210> 25
<211> 2743
<212> DNA
<213> Artificial Sequence

<220>
<221> source
<223> /note="Description of Artificial Sequence: Synthetic polynucleotide"

<400> 25
ggatccatgg agttcgttaa caaacagttc aactataaag acccagttaa cggtgttgac 60
attgcttaca tcaaaatccc gaacgctggc cagatgcagc cggtaaaggc attcaaaatc 120
cacaacaaaa tctgggttat cccggaacgt gataccttta ctaacccgga agaaggtgac 180
ctgaacccgc caccggaagc gaaacaggtg ccggtatctt actatgactc cacctacctg 240
tctaccgata acgaaaagga caactacctg aaaggtgtta ctaaactgtt cgagcgtatt 300
tactccaccg acctgggccg tatgctgctg actagcatcg ttcgcggtat cccgttctgg 360
ggcggttcta ccatcgatac cgaactgaaa gtaatcgaca ctaactgcat caacgttatt 420
cagccggacg gttcctatcg ttccgaagaa ctgaacctgg tgatcatcgg cccgtctgct 480
gatatcatcc agttcgagtg tctgagcttt ggtcacgaag ttctgaacct cacccgtaac 540
ggctacggtt ccactcagta catccgtttc tctccggact tcaccttcgg ttttgaagaa 600
tccctggaag tagacacgaa cccactgctg ggcgctggta aattcgcaac tgatcctgcg 660
gttaccctgg ctcacgaact gattcatgca ggccaccgcc tgtacggtat cgccatcaat 720
ccgaaccgtg tcttcaaagt taacaccaac gcgtattacg agatgtccgg tctggaagtt 780
agcttcgaag aactgcgtac ttttggcggt cacgacgcta aattcatcga ctctctgcaa 840
gaaaacgagt tccgtctgta ctactataac aagttcaaag atatcgcatc caccctgaac 900
aaagcgaaat ccatcgtggg taccactgct tctctccagt acatgaagaa cgtttttaaa 960

gaaaaatacc tgctcagcga agacacctcc ggcaaattct ctgtagacaa gttgaaattc 1020
gataaacttt acaaaatgct gactgaaatt tacaccgaag acaacttcgt taagttcttt 1080
aaagttctga accgcaaaac ctatctgaac ttcgacaagg cagtattcaa aatcaacatc 1140
gtgccgaaag ttaactacac tatctacgat ggtttcaacc tgcgtaacac caacctggct 1200
gctaatttta acggccagaa cacggaaatc aacaacatga acttcacaaa actgaaaaac 1260
ttcactggtc tgttcgagtt ttacaagctg ctgtgcgtcg acggcatcat tacctccaaa 1320
actaaatctg acgatgacga taaaagcgcc aattcaaatc ctgcaatggc gccacgcgaa 1380
cgcaaagctg gatgcaaaaa cttcttttgg aagacattta ctagttgtgc gctagcgggc 1440
ggtggcggta gcggcggtgg cggtagcggc ggtggcggta gcgcactagt gctgcagtgt 1500
atcaaggtta acaactggga tttattcttc agcccgagtg aagacaactt caccaacgac 1560
ctgaacaaag gtgaagaaat cacctcagat actaacatcg aagcagccga agaaaacatc 1620
tcgctggacc tgatccagca gtactacctg acctttaatt tcgacaacga gccggaaaac 1680
atttctatcg aaaacctgag ctctgatatc atcggccagc tggaactgat gccgaacatc 1740
gaacgtttcc caaacggtaa aaagtacgag ctggacaaat ataccatgtt ccactacctg 1800
cgcgcgcagg aatttgaaca cggcaaatcc cgtatcgcac tgactaactc cgttaacgaa 1860
gctctgctca acccgtcccg tgtatacacc ttcttctcta gcgactacgt gaaaaaggtc 1920
aacaaagcga ctgaagctgc aatgttcttg ggttgggttg aacagcttgt ttatgatttt 1980
accgacgaga cgtccgaagt atctactacc gacaaaattg cggatatcac tatcatcatc 2040
ccgtacatcg gtccggctct gaacattggc aacatgctgt acaaagacga cttcgttggc 2100
gcactgatct tctccggtgc ggtgatcctg ctggagttca tcccggaaat cgccatcccg 2160
gtactgggca cctttgctct ggtttcttac attgcaaaca aggttctgac tgtacaaacc 2220
atcgacaacg cgctgagcaa acgtaacgaa aaatgggatg aagtttacaa atatatcgtg 2280
accaactggc tggctaaggt taatactcag atcgacctca tccgcaaaaa aatgaaagaa 2340
gcactggaaa accaggcgga agctaccaag gcaatcatta actaccagta caaccagtac 2400
accgaggaag aaaaaaacaa catcaacttc aacatcgacg atctgtcctc taaactgaac 2460
gaatccatca acaaagctat gatcaacatc aacaagttcc tgaaccagtg ctctgtaagc 2520
tatctgatga actccatgat cccgtacggt gttaaacgtc tggaggactt cgatgcgtct 2580
ctgaaagacg ccctgctgaa atacatttac gacaaccgtg gcactctgat cggtcaggtt 2640
gatcgtctga aggacaaagt gaacaatacc ttatcgaccg acatcccttt tcagctcagt 2700
aaatatgtcg ataaccaacg ccttttgtcc acttaataag ctt 2743

<210> 26
<211> 908

<212> PRT
<213> Artificial Sequence

<220>
<221> source
<223> /note="Description of Artificial Sequence: Synthetic
polypeptide"

<400> 26
Glu Phe Val Asn Lys Gln Phe Asn Tyr Lys Asp Pro Val Asn Gly Val
1               5                   10                  15

Asp Ile Ala Tyr Ile Lys Ile Pro Asn Ala Gly Gln Met Gln Pro Val
            20                  25                  30

Lys Ala Phe Lys Ile His Asn Lys Ile Trp Val Ile Pro Glu Arg Asp
        35                  40                  45

Thr Phe Thr Asn Pro Glu Glu Gly Asp Leu Asn Pro Pro Pro Glu Ala
    50                  55                  60

Lys Gln Val Pro Val Ser Tyr Tyr Asp Ser Thr Tyr Leu Ser Thr Asp
65                  70                  75                  80

Asn Glu Lys Asp Asn Tyr Leu Lys Gly Val Thr Lys Leu Phe Glu Arg
                85                  90                  95

Ile Tyr Ser Thr Asp Leu Gly Arg Met Leu Leu Thr Ser Ile Val Arg
            100                 105                 110

Gly Ile Pro Phe Trp Gly Gly Ser Thr Ile Asp Thr Glu Leu Lys Val
        115                 120                 125

Ile Asp Thr Asn Cys Ile Asn Val Ile Gln Pro Asp Gly Ser Tyr Arg
    130                 135                 140

Ser Glu Glu Leu Asn Leu Val Ile Ile Gly Pro Ser Ala Asp Ile Ile
145                 150                 155                 160

Gln Phe Glu Cys Leu Ser Phe Gly His Glu Val Leu Asn Leu Thr Arg
                165                 170                 175

Asn Gly Tyr Gly Ser Thr Gln Tyr Ile Arg Phe Ser Pro Asp Phe Thr
            180                 185                 190

Phe Gly Phe Glu Glu Ser Leu Glu Val Asp Thr Asn Pro Leu Leu Gly
        195                 200                 205

Ala Gly Lys Phe Ala Thr Asp Pro Ala Val Thr Leu Ala His Glu Leu
    210                 215                 220

Ile His Ala Gly His Arg Leu Tyr Gly Ile Ala Ile Asn Pro Asn Arg
225 230 235 240

Val Phe Lys Val Asn Thr Asn Ala Tyr Tyr Glu Met Ser Gly Leu Glu
245 250 255

Val Ser Phe Glu Glu Leu Arg Thr Phe Gly Gly His Asp Ala Lys Phe
260 265 270

Ile Asp Ser Leu Gln Glu Asn Glu Phe Arg Leu Tyr Tyr Tyr Asn Lys
275 280 285

Phe Lys Asp Ile Ala Ser Thr Leu Asn Lys Ala Lys Ser Ile Val Gly
290 295 300

Thr Thr Ala Ser Leu Gln Tyr Met Lys Asn Val Phe Lys Glu Lys Tyr
305 310 315 320

Leu Leu Ser Glu Asp Thr Ser Gly Lys Phe Ser Val Asp Lys Leu Lys
325 330 335

Phe Asp Lys Leu Tyr Lys Met Leu Thr Glu Ile Tyr Thr Glu Asp Asn
340 345 350

Phe Val Lys Phe Phe Lys Val Leu Asn Arg Lys Thr Tyr Leu Asn Phe
355 360 365

Asp Lys Ala Val Phe Lys Ile Asn Ile Val Pro Lys Val Asn Tyr Thr
370 375 380

Ile Tyr Asp Gly Phe Asn Leu Arg Asn Thr Asn Leu Ala Ala Asn Phe
385 390 395 400

Asn Gly Gln Asn Thr Glu Ile Asn Asn Met Asn Phe Thr Lys Leu Lys
405 410 415

Asn Phe Thr Gly Leu Phe Glu Phe Tyr Lys Leu Leu Cys Val Asp Gly
420 425 430

Ile Ile Thr Ser Lys Thr Lys Ser Asp Asp Asp Asp Lys Ser Ala Asn
435 440 445

Ser Asn Pro Ala Met Ala Pro Arg Glu Arg Lys Ala Gly Cys Lys Asn
450 455 460

Phe Phe Trp Lys Thr Phe Thr Ser Cys Ala Leu Ala Gly Gly Gly Gly

465 470 475 480

Ser Gly Gly Gly Gly Ser Gly Gly Gly Gly Ser Ala Leu Val Leu Gln
485 490 495

Cys Ile Lys Val Asn Asn Trp Asp Leu Phe Phe Ser Pro Ser Glu Asp
500 505 510

Asn Phe Thr Asn Asp Leu Asn Lys Gly Glu Glu Ile Thr Ser Asp Thr
515 520 525

Asn Ile Glu Ala Ala Glu Glu Asn Ile Ser Leu Asp Leu Ile Gln Gln
530 535 540

Tyr Tyr Leu Thr Phe Asn Phe Asp Asn Glu Pro Glu Asn Ile Ser Ile
545 550 555 560

Glu Asn Leu Ser Ser Asp Ile Ile Gly Gln Leu Glu Leu Met Pro Asn
565 570 575

Ile Glu Arg Phe Pro Asn Gly Lys Lys Tyr Glu Leu Asp Lys Tyr Thr
580 585 590

Met Phe His Tyr Leu Arg Ala Gln Glu Phe Glu His Gly Lys Ser Arg
595 600 605

Ile Ala Leu Thr Asn Ser Val Asn Glu Ala Leu Leu Asn Pro Ser Arg
610 615 620

Val Tyr Thr Phe Phe Ser Ser Asp Tyr Val Lys Lys Val Asn Lys Ala
625 630 635 640

Thr Glu Ala Ala Met Phe Leu Gly Trp Val Glu Gln Leu Val Tyr Asp
645 650 655

Phe Thr Asp Glu Thr Ser Glu Val Ser Thr Thr Asp Lys Ile Ala Asp
660 665 670

Ile Thr Ile Ile Ile Pro Tyr Ile Gly Pro Ala Leu Asn Ile Gly Asn
675 680 685

Met Leu Tyr Lys Asp Asp Phe Val Gly Ala Leu Ile Phe Ser Gly Ala
690 695 700

Val Ile Leu Leu Glu Phe Ile Pro Glu Ile Ala Ile Pro Val Leu Gly
705 710 715 720

```
Thr Phe Ala Leu Val Ser Tyr Ile Ala Asn Lys Val Leu Thr Val Gln
                725                 730                 735

Thr Ile Asp Asn Ala Leu Ser Lys Arg Asn Glu Lys Trp Asp Glu Val
            740                 745                 750

Tyr Lys Tyr Ile Val Thr Asn Trp Leu Ala Lys Val Asn Thr Gln Ile
        755                 760                 765

Asp Leu Ile Arg Lys Lys Met Lys Glu Ala Leu Glu Asn Gln Ala Glu
    770                 775                 780

Ala Thr Lys Ala Ile Ile Asn Tyr Gln Tyr Asn Gln Tyr Thr Glu Glu
785                 790                 795                 800

Glu Lys Asn Asn Ile Asn Phe Asn Ile Asp Asp Leu Ser Ser Lys Leu
                805                 810                 815

Asn Glu Ser Ile Asn Lys Ala Met Ile Asn Ile Asn Lys Phe Leu Asn
            820                 825                 830

Gln Cys Ser Val Ser Tyr Leu Met Asn Ser Met Ile Pro Tyr Gly Val
        835                 840                 845

Lys Arg Leu Glu Asp Phe Asp Ala Ser Leu Lys Asp Ala Leu Leu Lys
    850                 855                 860

Tyr Ile Tyr Asp Asn Arg Gly Thr Leu Ile Gly Gln Val Asp Arg Leu
865                 870                 875                 880

Lys Asp Lys Val Asn Asn Thr Leu Ser Thr Asp Ile Pro Phe Gln Leu
                885                 890                 895

Ser Lys Tyr Val Asp Asn Gln Arg Leu Leu Ser Thr
            900                 905
```

<210> 27
<211> 920
<212> PRT
<213> Artificial Sequence

<220>
<221> source
<223> /note="Description of Artificial Sequence: Synthetic polypeptide"

<400> 27

```
Pro Val Thr Ile Asn Asn Phe Asn Tyr Asn Asp Pro Ile Asp Asn Asn
1               5                   10                  15
```

```
Asn Ile Ile Met Met Glu Pro Pro Phe Ala Arg Gly Thr Gly Arg Tyr
            20                  25                  30

Tyr Lys Ala Phe Lys Ile Thr Asp Arg Ile Trp Ile Ile Pro Glu Arg
        35                  40                  45

Tyr Thr Phe Gly Tyr Lys Pro Glu Asp Phe Asn Lys Ser Ser Gly Ile
    50                  55                  60

Phe Asn Arg Asp Val Cys Glu Tyr Tyr Asp Pro Asp Tyr Leu Asn Thr
65                  70                  75                  80

Asn Asp Lys Lys Asn Ile Phe Leu Gln Thr Met Ile Lys Leu Phe Asn
                85                  90                  95

Arg Ile Lys Ser Lys Pro Leu Gly Glu Lys Leu Leu Glu Met Ile Ile
            100                 105                 110

Asn Gly Ile Pro Tyr Leu Gly Asp Arg Arg Val Pro Leu Glu Glu Phe
        115                 120                 125

Asn Thr Asn Ile Ala Ser Val Thr Val Asn Lys Leu Ile Ser Asn Pro
    130                 135                 140

Gly Glu Val Glu Arg Lys Lys Gly Ile Phe Ala Asn Leu Ile Ile Phe
145                 150                 155                 160

Gly Pro Gly Pro Val Leu Asn Glu Asn Glu Thr Ile Asp Ile Gly Ile
                165                 170                 175

Gln Asn His Phe Ala Ser Arg Glu Gly Phe Gly Gly Ile Met Gln Met
            180                 185                 190

Lys Phe Cys Pro Glu Tyr Val Ser Val Phe Asn Asn Val Gln Glu Asn
        195                 200                 205

Lys Gly Ala Ser Ile Phe Asn Arg Arg Gly Tyr Phe Ser Asp Pro Ala
    210                 215                 220

Leu Ile Leu Met His Glu Leu Ile His Val Leu His Gly Leu Tyr Gly
225                 230                 235                 240

Ile Lys Val Asp Asp Leu Pro Ile Val Pro Asn Glu Lys Lys Phe Phe
                245                 250                 255

Met Gln Ser Thr Asp Ala Ile Gln Ala Glu Glu Leu Tyr Thr Phe Gly
            260                 265                 270
```

```
Gly Gln Asp Pro Ser Ile Ile Thr Pro Ser Thr Asp Lys Ser Ile Tyr
        275                 280                 285

Asp Lys Val Leu Gln Asn Phe Arg Gly Ile Val Asp Arg Leu Asn Lys
    290                 295                 300

Val Leu Val Cys Ile Ser Asp Pro Asn Ile Asn Ile Asn Ile Tyr Lys
305                 310                 315                 320

Asn Lys Phe Lys Asp Lys Tyr Lys Phe Val Glu Asp Ser Glu Gly Lys
                325                 330                 335

Tyr Ser Ile Asp Val Glu Ser Phe Asp Lys Leu Tyr Lys Ser Leu Met
            340                 345                 350

Phe Gly Phe Thr Glu Thr Asn Ile Ala Glu Asn Tyr Lys Ile Lys Thr
        355                 360                 365

Arg Ala Ser Tyr Phe Ser Asp Ser Leu Pro Pro Val Lys Ile Lys Asn
    370                 375                 380

Leu Leu Asp Asn Glu Ile Tyr Thr Ile Glu Glu Gly Phe Asn Ile Ser
385                 390                 395                 400

Asp Lys Asp Met Glu Lys Glu Tyr Arg Gly Gln Asn Lys Ala Ile Asn
                405                 410                 415

Lys Gln Ala Tyr Glu Glu Ile Ser Lys Glu His Leu Ala Val Tyr Lys
            420                 425                 430

Ile Gln Met Cys Val Asp Gly Ile Ile Thr Ser Lys Thr Lys Ser Asp
        435                 440                 445

Asp Asp Asp Lys Asn Lys Ala Leu Asn Leu Gln Cys Ile Asp Val Asp
    450                 455                 460

Asn Glu Asp Leu Phe Phe Ile Ala Asp Lys Asn Ser Phe Ser Asp Asp
465                 470                 475                 480

Leu Ser Lys Asn Glu Arg Ile Glu Tyr Asn Thr Gln Ser Asn Tyr Ile
                485                 490                 495

Glu Asn Asp Phe Pro Ile Asn Glu Leu Ile Leu Asp Thr Asp Leu Ile
            500                 505                 510

Ser Lys Ile Glu Leu Pro Ser Glu Asn Thr Glu Ser Leu Thr Asp Phe
        515                 520                 525
```

Asn Val Asp Val Pro Val Tyr Glu Lys Gln Pro Ala Ile Lys Lys Ile
530 535 540

Phe Thr Asp Glu Asn Thr Ile Phe Gln Tyr Leu Tyr Ser Gln Thr Phe
545 550 555 560

Pro Leu Asp Ile Arg Asp Ile Ser Leu Thr Ser Ser Phe Asp Asp Ala
565 570 575

Leu Leu Phe Ser Asn Lys Val Tyr Ser Phe Phe Ser Met Asp Tyr Ile
580 585 590

Lys Thr Ala Asn Lys Val Val Glu Ala Gly Leu Phe Ala Gly Trp Val
595 600 605

Lys Gln Ile Val Asn Asp Phe Val Ile Glu Ala Asn Lys Ser Asn Thr
610 615 620

Met Asp Lys Ile Ala Asp Ile Ser Leu Ile Val Pro Tyr Ile Gly Leu
625 630 635 640

Ala Leu Asn Val Gly Asn Glu Thr Ala Lys Gly Asn Phe Glu Asn Ala
645 650 655

Phe Glu Ile Ala Gly Ala Ser Ile Leu Leu Glu Phe Ile Pro Glu Leu
660 665 670

Leu Ile Pro Val Val Gly Ala Phe Leu Leu Glu Ser Tyr Ile Asp Asn
675 680 685

Lys Asn Lys Ile Ile Lys Thr Ile Asp Asn Ala Leu Thr Lys Arg Asn
690 695 700

Glu Lys Trp Ser Asp Met Tyr Gly Leu Ile Val Ala Gln Trp Leu Ser
705 710 715 720

Thr Val Asn Thr Gln Phe Tyr Thr Ile Lys Glu Gly Met Tyr Lys Ala
725 730 735

Leu Asn Tyr Gln Ala Gln Ala Leu Glu Glu Ile Ile Lys Tyr Arg Tyr
740 745 750

Asn Ile Tyr Ser Glu Lys Glu Lys Ser Asn Ile Asn Ile Asp Phe Asn
755 760 765

Asp Ile Asn Ser Lys Leu Asn Glu Gly Ile Asn Gln Ala Ile Asp Asn

770 775 780

Ile Asn Asn Phe Ile Asn Gly Cys Ser Val Ser Tyr Leu Met Lys Lys
785 790 795 800

Met Ile Pro Leu Ala Val Glu Lys Leu Leu Asp Phe Asp Asn Thr Leu
805 810 815

Lys Lys Asn Leu Leu Asn Tyr Ile Asp Glu Asn Lys Leu Tyr Leu Ile
820 825 830

Gly Ser Ala Glu Tyr Glu Lys Ser Lys Val Asn Lys Tyr Leu Lys Thr
835 840 845

Ile Met Pro Phe Asp Leu Ser Ile Tyr Thr Asn Asp Thr Ile Leu Ile
850 855 860

Glu Met Phe Asn Lys Tyr Asn Ser Leu Glu Gly Gly Gly Gly Ser Gly
865 870 875 880

Gly Gly Gly Ser Gly Gly Gly Gly Ser Ala Leu Asp Ser Ala Asn Ser
885 890 895

Asn Pro Ala Met Ala Pro Arg Glu Arg Lys Ala Gly Cys Lys Asn Phe
900 905 910

Phe Trp Lys Thr Phe Thr Ser Cys
915 920

<210> 28
<211> 904
<212> PRT
<213> Artificial Sequence

<220>
<221> source
<223> /note="Description of Artificial Sequence: Synthetic polypeptide"

<400> 28
Pro Ile Thr Ile Asn Asn Phe Asn Tyr Ser Asp Pro Val Asp Asn Lys
1 5 10 15

Asn Ile Leu Tyr Leu Asp Thr His Leu Asn Thr Leu Ala Asn Glu Pro
20 25 30

Glu Lys Ala Phe Arg Ile Thr Gly Asn Ile Trp Val Ile Pro Asp Arg
35 40 45

Phe Ser Arg Asn Ser Asn Pro Asn Leu Asn Lys Pro Pro Arg Val Thr

50 55 60

Ser Pro Lys Ser Gly Tyr Tyr Asp Pro Asn Tyr Leu Ser Thr Asp Ser
65 70 75 80

Asp Lys Asp Thr Phe Leu Lys Glu Ile Ile Lys Leu Phe Lys Arg Ile
85 90 95

Asn Ser Arg Glu Ile Gly Glu Glu Leu Ile Tyr Arg Leu Ser Thr Asp
100 105 110

Ile Pro Phe Pro Gly Asn Asn Asn Thr Pro Ile Asn Thr Phe Asp Phe
115 120 125

Asp Val Asp Phe Asn Ser Val Asp Val Lys Thr Arg Gln Gly Asn Asn
130 135 140

Trp Val Lys Thr Gly Ser Ile Asn Pro Ser Val Ile Ile Thr Gly Pro
145 150 155 160

Arg Glu Asn Ile Ile Asp Pro Glu Thr Ser Thr Phe Lys Leu Thr Asn
165 170 175

Asn Thr Phe Ala Ala Gln Glu Gly Phe Gly Ala Leu Ser Ile Ile Ser
180 185 190

Ile Ser Pro Arg Phe Met Leu Thr Tyr Ser Asn Ala Thr Asn Asp Val
195 200 205

Gly Glu Gly Arg Phe Ser Lys Ser Glu Phe Cys Met Asp Pro Ile Leu
210 215 220

Ile Leu Met His Glu Leu Asn His Ala Met His Asn Leu Tyr Gly Ile
225 230 235 240

Ala Ile Pro Asn Asp Gln Thr Ile Ser Ser Val Thr Ser Asn Ile Phe
245 250 255

Tyr Ser Gln Tyr Asn Val Lys Leu Glu Tyr Ala Glu Ile Tyr Ala Phe
260 265 270

Gly Gly Pro Thr Ile Asp Leu Ile Pro Lys Ser Ala Arg Lys Tyr Phe
275 280 285

Glu Glu Lys Ala Leu Asp Tyr Tyr Arg Ser Ile Ala Lys Arg Leu Asn
290 295 300

```
Ser Ile Thr Thr Ala Asn Pro Ser Ser Phe Asn Lys Tyr Ile Gly Glu
305                 310                 315                 320

Tyr Lys Gln Lys Leu Ile Arg Lys Tyr Arg Phe Val Val Glu Ser Ser
                325                 330                 335

Gly Glu Val Thr Val Asn Arg Asn Lys Phe Val Glu Leu Tyr Asn Glu
            340                 345                 350

Leu Thr Gln Ile Phe Thr Glu Phe Asn Tyr Ala Lys Ile Tyr Asn Val
        355                 360                 365

Gln Asn Arg Lys Ile Tyr Leu Ser Asn Val Tyr Thr Pro Val Thr Ala
    370                 375                 380

Asn Ile Leu Asp Asp Asn Val Tyr Asp Ile Gln Asn Gly Phe Asn Ile
385                 390                 395                 400

Pro Lys Ser Asn Leu Asn Val Leu Phe Met Gly Gln Asn Leu Ser Arg
                405                 410                 415

Asn Pro Ala Leu Arg Lys Val Asn Pro Glu Asn Met Leu Tyr Leu Phe
            420                 425                 430

Thr Lys Phe Cys Val Asp Ala Ile Asp Gly Arg Ser Leu Tyr Asn Lys
        435                 440                 445

Thr Leu Gln Cys Arg Glu Leu Leu Val Lys Asn Thr Asp Leu Pro Phe
    450                 455                 460

Ile Gly Asp Ile Ser Asp Val Lys Thr Asp Ile Phe Leu Arg Lys Asp
465                 470                 475                 480

Ile Asn Glu Glu Thr Glu Val Ile Tyr Tyr Pro Asp Asn Val Ser Val
                485                 490                 495

Asp Gln Val Ile Leu Ser Lys Asn Thr Ser Glu His Gly Gln Leu Asp
            500                 505                 510

Leu Leu Tyr Pro Ser Ile Asp Ser Glu Ser Glu Ile Leu Pro Gly Glu
        515                 520                 525

Asn Gln Val Phe Tyr Asp Asn Arg Thr Gln Asn Val Asp Tyr Leu Asn
    530                 535                 540

Ser Tyr Tyr Tyr Leu Glu Ser Gln Lys Leu Ser Asp Asn Val Glu Asp
545                 550                 555                 560
```

```
Phe Thr Phe Thr Arg Ser Ile Glu Glu Ala Leu Asp Asn Ser Ala Lys
                565                 570                 575

Val Tyr Thr Tyr Phe Pro Thr Leu Ala Asn Lys Val Asn Ala Gly Val
            580                 585                 590

Gln Gly Gly Leu Phe Leu Met Trp Ala Asn Asp Val Val Glu Asp Phe
        595                 600                 605

Thr Thr Asn Ile Leu Arg Lys Asp Thr Leu Asp Lys Ile Ser Asp Val
    610                 615                 620

Ser Ala Ile Ile Pro Tyr Ile Gly Pro Ala Leu Asn Ile Ser Asn Ser
625                 630                 635                 640

Val Arg Arg Gly Asn Phe Thr Glu Ala Phe Ala Val Thr Gly Val Thr
                645                 650                 655

Ile Leu Leu Glu Ala Phe Pro Glu Phe Thr Ile Pro Ala Leu Gly Ala
            660                 665                 670

Phe Val Ile Tyr Ser Lys Val Gln Glu Arg Asn Glu Ile Ile Lys Thr
        675                 680                 685

Ile Asp Asn Cys Leu Glu Gln Arg Ile Lys Arg Trp Lys Asp Ser Tyr
    690                 695                 700

Glu Trp Met Met Gly Thr Trp Leu Ser Arg Ile Ile Thr Gln Phe Asn
705                 710                 715                 720

Asn Ile Ser Tyr Gln Met Tyr Asp Ser Leu Asn Tyr Gln Ala Gly Ala
                725                 730                 735

Ile Lys Ala Lys Ile Asp Leu Glu Tyr Lys Lys Tyr Ser Gly Ser Asp
            740                 745                 750

Lys Glu Asn Ile Lys Ser Gln Val Glu Asn Leu Lys Asn Ser Leu Asp
        755                 760                 765

Val Lys Ile Ser Glu Ala Met Asn Asn Ile Asn Lys Phe Ile Arg Glu
    770                 775                 780

Cys Ser Val Thr Tyr Leu Phe Lys Asn Met Leu Pro Lys Val Ile Asp
785                 790                 795                 800

Glu Leu Asn Glu Phe Asp Arg Asn Thr Lys Ala Lys Leu Ile Asn Leu
                805                 810                 815
```

```
Ile Asp Ser His Asn Ile Ile Leu Val Gly Glu Val Asp Lys Leu Lys
            820                 825                 830

Ala Lys Val Asn Asn Ser Phe Gln Asn Thr Ile Pro Phe Asn Ile Phe
        835                 840                 845

Ser Tyr Thr Asn Asn Ser Leu Leu Lys Asp Ile Ile Asn Glu Tyr Phe
    850                 855                 860

Asn Leu Glu Gly Gly Gly Gly Ser Gly Gly Gly Gly Ser Gly Gly Gly
865                 870                 875                 880

Gly Ser Gly Gly Gly Gly Ser Ala Leu Val Ala Gly Cys Lys Asn Phe
                885                 890                 895

Phe Trp Lys Thr Phe Thr Ser Cys
            900


<210> 29
<211> 912
<212> PRT
<213> Artificial Sequence

<220>
<221> source
<223> /note="Description of Artificial Sequence: Synthetic
      polypeptide"

<400> 29
Pro Ile Thr Ile Asn Asn Phe Asn Tyr Ser Asp Pro Val Asp Asn Lys
1               5                   10                  15

Asn Ile Leu Tyr Leu Asp Thr His Leu Asn Thr Leu Ala Asn Glu Pro
            20                  25                  30

Glu Lys Ala Phe Arg Ile Thr Gly Asn Ile Trp Val Ile Pro Asp Arg
        35                  40                  45

Phe Ser Arg Asn Ser Asn Pro Asn Leu Asn Lys Pro Pro Arg Val Thr
    50                  55                  60

Ser Pro Lys Ser Gly Tyr Tyr Asp Pro Asn Tyr Leu Ser Thr Asp Ser
65                  70                  75                  80

Asp Lys Asp Thr Phe Leu Lys Glu Ile Ile Lys Leu Phe Lys Arg Ile
                85                  90                  95

Asn Ser Arg Glu Ile Gly Glu Glu Leu Ile Tyr Arg Leu Ser Thr Asp
            100                 105                 110
```

Ile Pro Phe Pro Gly Asn Asn Asn Thr Pro Ile Asn Thr Phe Asp Phe
115 120 125

Asp Val Asp Phe Asn Ser Val Asp Val Lys Thr Arg Gln Gly Asn Asn
130 135 140

Trp Val Lys Thr Gly Ser Ile Asn Pro Ser Val Ile Ile Thr Gly Pro
145 150 155 160

Arg Glu Asn Ile Ile Asp Pro Glu Thr Ser Thr Phe Lys Leu Thr Asn
165 170 175

Asn Thr Phe Ala Ala Gln Glu Gly Phe Gly Ala Leu Ser Ile Ile Ser
180 185 190

Ile Ser Pro Arg Phe Met Leu Thr Tyr Ser Asn Ala Thr Asn Asp Val
195 200 205

Gly Glu Gly Arg Phe Ser Lys Ser Glu Phe Cys Met Asp Pro Ile Leu
210 215 220

Ile Leu Met His Glu Leu Asn His Ala Met His Asn Leu Tyr Gly Ile
225 230 235 240

Ala Ile Pro Asn Asp Gln Thr Ile Ser Ser Val Thr Ser Asn Ile Phe
245 250 255

Tyr Ser Gln Tyr Asn Val Lys Leu Glu Tyr Ala Glu Ile Tyr Ala Phe
260 265 270

Gly Gly Pro Thr Ile Asp Leu Ile Pro Lys Ser Ala Arg Lys Tyr Phe
275 280 285

Glu Glu Lys Ala Leu Asp Tyr Tyr Arg Ser Ile Ala Lys Arg Leu Asn
290 295 300

Ser Ile Thr Thr Ala Asn Pro Ser Ser Phe Asn Lys Tyr Ile Gly Glu
305 310 315 320

Tyr Lys Gln Lys Leu Ile Arg Lys Tyr Arg Phe Val Val Glu Ser Ser
325 330 335

Gly Glu Val Thr Val Asn Arg Asn Lys Phe Val Glu Leu Tyr Asn Glu
340 345 350

Leu Thr Gln Ile Phe Thr Glu Phe Asn Tyr Ala Lys Ile Tyr Asn Val

355 360 365

Gln Asn Arg Lys Ile Tyr Leu Ser Asn Val Tyr Thr Pro Val Thr Ala
370 375 380

Asn Ile Leu Asp Asp Asn Val Tyr Asp Ile Gln Asn Gly Phe Asn Ile
385 390 395 400

Pro Lys Ser Asn Leu Asn Val Leu Phe Met Gly Gln Asn Leu Ser Arg
405 410 415

Asn Pro Ala Leu Arg Lys Val Asn Pro Glu Asn Met Leu Tyr Leu Phe
420 425 430

Thr Lys Phe Cys Val Asp Ala Ile Asp Gly Arg Ser Leu Tyr Asn Lys
435 440 445

Thr Leu Gln Cys Arg Glu Leu Leu Val Lys Asn Thr Asp Leu Pro Phe
450 455 460

Ile Gly Asp Ile Ser Asp Val Lys Thr Asp Ile Phe Leu Arg Lys Asp
465 470 475 480

Ile Asn Glu Glu Thr Glu Val Ile Tyr Tyr Pro Asp Asn Val Ser Val
485 490 495

Asp Gln Val Ile Leu Ser Lys Asn Thr Ser Glu His Gly Gln Leu Asp
500 505 510

Leu Leu Tyr Pro Ser Ile Asp Ser Glu Ser Glu Ile Leu Pro Gly Glu
515 520 525

Asn Gln Val Phe Tyr Asp Asn Arg Thr Gln Asn Val Asp Tyr Leu Asn
530 535 540

Ser Tyr Tyr Tyr Leu Glu Ser Gln Lys Leu Ser Asp Asn Val Glu Asp
545 550 555 560

Phe Thr Phe Thr Arg Ser Ile Glu Glu Ala Leu Asp Asn Ser Ala Lys
565 570 575

Val Tyr Thr Tyr Phe Pro Thr Leu Ala Asn Lys Val Asn Ala Gly Val
580 585 590

Gln Gly Gly Leu Phe Leu Met Trp Ala Asn Asp Val Val Glu Asp Phe
595 600 605

Thr Thr Asn Ile Leu Arg Lys Asp Thr Leu Asp Lys Ile Ser Asp Val
    610                 615                 620

Ser Ala Ile Ile Pro Tyr Ile Gly Pro Ala Leu Asn Ile Ser Asn Ser
625                 630                 635                 640

Val Arg Arg Gly Asn Phe Thr Glu Ala Phe Ala Val Thr Gly Val Thr
                645                 650                 655

Ile Leu Leu Glu Ala Phe Pro Glu Phe Thr Ile Pro Ala Leu Gly Ala
            660                 665                 670

Phe Val Ile Tyr Ser Lys Val Gln Glu Arg Asn Glu Ile Ile Lys Thr
        675                 680                 685

Ile Asp Asn Cys Leu Glu Gln Arg Ile Lys Arg Trp Lys Asp Ser Tyr
    690                 695                 700

Glu Trp Met Met Gly Thr Trp Leu Ser Arg Ile Ile Thr Gln Phe Asn
705                 710                 715                 720

Asn Ile Ser Tyr Gln Met Tyr Asp Ser Leu Asn Tyr Gln Ala Gly Ala
                725                 730                 735

Ile Lys Ala Lys Ile Asp Leu Glu Tyr Lys Lys Tyr Ser Gly Ser Asp
            740                 745                 750

Lys Glu Asn Ile Lys Ser Gln Val Glu Asn Leu Lys Asn Ser Leu Asp
        755                 760                 765

Val Lys Ile Ser Glu Ala Met Asn Asn Ile Asn Lys Phe Ile Arg Glu
    770                 775                 780

Cys Ser Val Thr Tyr Leu Phe Lys Asn Met Leu Pro Lys Val Ile Asp
785                 790                 795                 800

Glu Leu Asn Glu Phe Asp Arg Asn Thr Lys Ala Lys Leu Ile Asn Leu
                805                 810                 815

Ile Asp Ser His Asn Ile Ile Leu Val Gly Glu Val Asp Lys Leu Lys
            820                 825                 830

Ala Lys Val Asn Asn Ser Phe Gln Asn Thr Ile Pro Phe Asn Ile Phe
        835                 840                 845

Ser Tyr Thr Asn Asn Ser Leu Leu Lys Asp Ile Ile Asn Glu Tyr Phe
    850                 855                 860

Asn Leu Glu Gly Gly Gly Gly Ser Gly Gly Gly Gly Ser Gly Gly Gly
865 870 875 880

Gly Ser Gly Gly Gly Gly Ser Gly Gly Gly Gly Ser Ala Leu Val Asp
885 890 895

Arg Met Pro Cys Arg Asn Phe Phe Trp Lys Thr Phe Ser Ser Cys Lys
900 905 910

<210> 30
<211> 913
<212> PRT
<213> Artificial Sequence

<220>
<221> source
<223> /note="Description of Artificial Sequence: Synthetic polypeptide"

<400> 30
Glu Phe Val Asn Lys Gln Phe Asn Tyr Lys Asp Pro Val Asn Gly Val
1 5 10 15

Asp Ile Ala Tyr Ile Lys Ile Pro Asn Ala Gly Gln Met Gln Pro Val
20 25 30

Lys Ala Phe Lys Ile His Asn Lys Ile Trp Val Ile Pro Glu Arg Asp
35 40 45

Thr Phe Thr Asn Pro Glu Glu Gly Asp Leu Asn Pro Pro Pro Glu Ala
50 55 60

Lys Gln Val Pro Val Ser Tyr Tyr Asp Ser Thr Tyr Leu Ser Thr Asp
65 70 75 80

Asn Glu Lys Asp Asn Tyr Leu Lys Gly Val Thr Lys Leu Phe Glu Arg
85 90 95

Ile Tyr Ser Thr Asp Leu Gly Arg Met Leu Leu Thr Ser Ile Val Arg
100 105 110

Gly Ile Pro Phe Trp Gly Gly Ser Thr Ile Asp Thr Glu Leu Lys Val
115 120 125

Ile Asp Thr Asn Cys Ile Asn Val Ile Gln Pro Asp Gly Ser Tyr Arg
130 135 140

Ser Glu Glu Leu Asn Leu Val Ile Ile Gly Pro Ser Ala Asp Ile Ile
145 150 155 160

Gln Phe Glu Cys Leu Ser Phe Gly His Glu Val Leu Asn Leu Thr Arg
165 170 175

Asn Gly Tyr Gly Ser Thr Gln Tyr Ile Arg Phe Ser Pro Asp Phe Thr
180 185 190

Phe Gly Phe Glu Glu Ser Leu Glu Val Asp Thr Asn Pro Leu Leu Gly
195 200 205

Ala Gly Lys Phe Ala Thr Asp Pro Ala Val Thr Leu Ala His Glu Leu
210 215 220

Ile His Ala Gly His Arg Leu Tyr Gly Ile Ala Ile Asn Pro Asn Arg
225 230 235 240

Val Phe Lys Val Asn Thr Asn Ala Tyr Tyr Glu Met Ser Gly Leu Glu
245 250 255

Val Ser Phe Glu Glu Leu Arg Thr Phe Gly Gly His Asp Ala Lys Phe
260 265 270

Ile Asp Ser Leu Gln Glu Asn Glu Phe Arg Leu Tyr Tyr Tyr Asn Lys
275 280 285

Phe Lys Asp Ile Ala Ser Thr Leu Asn Lys Ala Lys Ser Ile Val Gly
290 295 300

Thr Thr Ala Ser Leu Gln Tyr Met Lys Asn Val Phe Lys Glu Lys Tyr
305 310 315 320

Leu Leu Ser Glu Asp Thr Ser Gly Lys Phe Ser Val Asp Lys Leu Lys
325 330 335

Phe Asp Lys Leu Tyr Lys Met Leu Thr Glu Ile Tyr Thr Glu Asp Asn
340 345 350

Phe Val Lys Phe Phe Lys Val Leu Asn Arg Lys Thr Tyr Leu Asn Phe
355 360 365

Asp Lys Ala Val Phe Lys Ile Asn Ile Val Pro Lys Val Asn Tyr Thr
370 375 380

Ile Tyr Asp Gly Phe Asn Leu Arg Asn Thr Asn Leu Ala Ala Asn Phe
385 390 395 400

Asn Gly Gln Asn Thr Glu Ile Asn Asn Met Asn Phe Thr Lys Leu Lys
405 410 415

```
Asn Phe Thr Gly Leu Phe Glu Phe Tyr Lys Leu Leu Cys Val Asp Gly
            420                 425                 430

Ile Ile Thr Ser Lys Thr Lys Ser Asp Asp Asp Asp Lys Asn Lys Ala
        435                 440                 445

Leu Asn Leu Gln Cys Ile Lys Val Asn Asn Trp Asp Leu Phe Phe Ser
    450                 455                 460

Pro Ser Glu Asp Asn Phe Thr Asn Asp Leu Asn Lys Gly Glu Glu Ile
465                 470                 475                 480

Thr Ser Asp Thr Asn Ile Glu Ala Ala Glu Glu Asn Ile Ser Leu Asp
                485                 490                 495

Leu Ile Gln Gln Tyr Tyr Leu Thr Phe Asn Phe Asp Asn Glu Pro Glu
            500                 505                 510

Asn Ile Ser Ile Glu Asn Leu Ser Ser Asp Ile Ile Gly Gln Leu Glu
        515                 520                 525

Leu Met Pro Asn Ile Glu Arg Phe Pro Asn Gly Lys Lys Tyr Glu Leu
    530                 535                 540

Asp Lys Tyr Thr Met Phe His Tyr Leu Arg Ala Gln Glu Phe Glu His
545                 550                 555                 560

Gly Lys Ser Arg Ile Ala Leu Thr Asn Ser Val Asn Glu Ala Leu Leu
                565                 570                 575

Asn Pro Ser Arg Val Tyr Thr Phe Phe Ser Ser Asp Tyr Val Lys Lys
            580                 585                 590

Val Asn Lys Ala Thr Glu Ala Ala Met Phe Leu Gly Trp Val Glu Gln
        595                 600                 605

Leu Val Tyr Asp Phe Thr Asp Glu Thr Ser Glu Val Ser Thr Thr Asp
    610                 615                 620

Lys Ile Ala Asp Ile Thr Ile Ile Ile Pro Tyr Ile Gly Pro Ala Leu
625                 630                 635                 640

Asn Ile Gly Asn Met Leu Tyr Lys Asp Asp Phe Val Gly Ala Leu Ile
                645                 650                 655

Phe Ser Gly Ala Val Ile Leu Leu Glu Phe Ile Pro Glu Ile Ala Ile
```

660 665 670

Pro Val Leu Gly Thr Phe Ala Leu Val Ser Tyr Ile Ala Asn Lys Val
675 680 685

Leu Thr Val Gln Thr Ile Asp Asn Ala Leu Ser Lys Arg Asn Glu Lys
690 695 700

Trp Asp Glu Val Tyr Lys Tyr Ile Val Thr Asn Trp Leu Ala Lys Val
705 710 715 720

Asn Thr Gln Ile Asp Leu Ile Arg Lys Lys Met Lys Glu Ala Leu Glu
725 730 735

Asn Gln Ala Glu Ala Thr Lys Ala Ile Ile Asn Tyr Gln Tyr Asn Gln
740 745 750

Tyr Thr Glu Glu Glu Lys Asn Asn Ile Asn Phe Asn Ile Asp Asp Leu
755 760 765

Ser Ser Lys Leu Asn Glu Ser Ile Asn Lys Ala Met Ile Asn Ile Asn
770 775 780

Lys Phe Leu Asn Gln Cys Ser Val Ser Tyr Leu Met Asn Ser Met Ile
785 790 795 800

Pro Tyr Gly Val Lys Arg Leu Glu Asp Phe Asp Ala Ser Leu Lys Asp
805 810 815

Ala Leu Leu Lys Tyr Ile Tyr Asp Asn Arg Gly Thr Leu Ile Gly Gln
820 825 830

Val Asp Arg Leu Lys Asp Lys Val Asn Asn Thr Leu Ser Thr Asp Ile
835 840 845

Pro Phe Gln Leu Ser Lys Tyr Val Asp Asn Gln Arg Leu Leu Ser Thr
850 855 860

Leu Glu Gly Gly Gly Gly Ser Gly Gly Gly Gly Ser Gly Gly Gly Gly
865 870 875 880

Ser Ala Leu Val Gln Glu Gly Ala Pro Pro Gln Gln Ser Ala Arg Arg
885 890 895

Asp Arg Met Pro Cys Arg Asn Phe Phe Trp Lys Thr Phe Ser Ser Cys
900 905 910

Lys

<210> 31
<211> 936
<212> PRT
<213> Artificial Sequence

<220>
<221> source
<223> /note="Description of Artificial Sequence: Synthetic
polypeptide"

<400> 31
Pro Val Thr Ile Asn Asn Phe Asn Tyr Asn Asp Pro Ile Asp Asn Asn
1               5                   10                  15

Asn Ile Ile Met Met Glu Pro Pro Phe Ala Arg Gly Thr Gly Arg Tyr
            20                  25                  30

Tyr Lys Ala Phe Lys Ile Thr Asp Arg Ile Trp Ile Ile Pro Glu Arg
        35                  40                  45

Tyr Thr Phe Gly Tyr Lys Pro Glu Asp Phe Asn Lys Ser Ser Gly Ile
    50                  55                  60

Phe Asn Arg Asp Val Cys Glu Tyr Tyr Asp Pro Asp Tyr Leu Asn Thr
65                  70                  75                  80

Asn Asp Lys Lys Asn Ile Phe Leu Gln Thr Met Ile Lys Leu Phe Asn
                85                  90                  95

Arg Ile Lys Ser Lys Pro Leu Gly Glu Lys Leu Leu Glu Met Ile Ile
            100                 105                 110

Asn Gly Ile Pro Tyr Leu Gly Asp Arg Arg Val Pro Leu Glu Glu Phe
        115                 120                 125

Asn Thr Asn Ile Ala Ser Val Thr Val Asn Lys Leu Ile Ser Asn Pro
    130                 135                 140

Gly Glu Val Glu Arg Lys Lys Gly Ile Phe Ala Asn Leu Ile Ile Phe
145                 150                 155                 160

Gly Pro Gly Pro Val Leu Asn Glu Asn Glu Thr Ile Asp Ile Gly Ile
                165                 170                 175

Gln Asn His Phe Ala Ser Arg Glu Gly Phe Gly Gly Ile Met Gln Met
            180                 185                 190

```
Lys Phe Cys Pro Glu Tyr Val Ser Val Phe Asn Asn Val Gln Glu Asn
        195                 200                 205

Lys Gly Ala Ser Ile Phe Asn Arg Arg Gly Tyr Phe Ser Asp Pro Ala
    210                 215                 220

Leu Ile Leu Met His Glu Leu Ile His Val Leu His Gly Leu Tyr Gly
225                 230                 235                 240

Ile Lys Val Asp Asp Leu Pro Ile Val Pro Asn Glu Lys Lys Phe Phe
                245                 250                 255

Met Gln Ser Thr Asp Ala Ile Gln Ala Glu Glu Leu Tyr Thr Phe Gly
            260                 265                 270

Gly Gln Asp Pro Ser Ile Ile Thr Pro Ser Thr Asp Lys Ser Ile Tyr
        275                 280                 285

Asp Lys Val Leu Gln Asn Phe Arg Gly Ile Val Asp Arg Leu Asn Lys
    290                 295                 300

Val Leu Val Cys Ile Ser Asp Pro Asn Ile Asn Ile Asn Ile Tyr Lys
305                 310                 315                 320

Asn Lys Phe Lys Asp Lys Tyr Lys Phe Val Glu Asp Ser Glu Gly Lys
                325                 330                 335

Tyr Ser Ile Asp Val Glu Ser Phe Asp Lys Leu Tyr Lys Ser Leu Met
            340                 345                 350

Phe Gly Phe Thr Glu Thr Asn Ile Ala Glu Asn Tyr Lys Ile Lys Thr
        355                 360                 365

Arg Ala Ser Tyr Phe Ser Asp Ser Leu Pro Pro Val Lys Ile Lys Asn
    370                 375                 380

Leu Leu Asp Asn Glu Ile Tyr Thr Ile Glu Glu Gly Phe Asn Ile Ser
385                 390                 395                 400

Asp Lys Asp Met Glu Lys Glu Tyr Arg Gly Gln Asn Lys Ala Ile Asn
                405                 410                 415

Lys Gln Ala Tyr Glu Glu Ile Ser Lys Glu His Leu Ala Val Tyr Lys
            420                 425                 430

Ile Gln Met Cys Val Asp Gly Ile Ile Thr Ser Lys Thr Lys Ser Asp
        435                 440                 445
```

Asp Asp Asp Lys Asn Lys Ala Leu Asn Leu Gln Cys Ile Asp Val Asp
    450                 455                 460

Asn Glu Asp Leu Phe Phe Ile Ala Asp Lys Asn Ser Phe Ser Asp Asp
465                 470                 475                 480

Leu Ser Lys Asn Glu Arg Ile Glu Tyr Asn Thr Gln Ser Asn Tyr Ile
                485                 490                 495

Glu Asn Asp Phe Pro Ile Asn Glu Leu Ile Leu Asp Thr Asp Leu Ile
            500                 505                 510

Ser Lys Ile Glu Leu Pro Ser Glu Asn Thr Glu Ser Leu Thr Asp Phe
        515                 520                 525

Asn Val Asp Val Pro Val Tyr Glu Lys Gln Pro Ala Ile Lys Lys Ile
    530                 535                 540

Phe Thr Asp Glu Asn Thr Ile Phe Gln Tyr Leu Tyr Ser Gln Thr Phe
545                 550                 555                 560

Pro Leu Asp Ile Arg Asp Ile Ser Leu Thr Ser Ser Phe Asp Asp Ala
                565                 570                 575

Leu Leu Phe Ser Asn Lys Val Tyr Ser Phe Phe Ser Met Asp Tyr Ile
            580                 585                 590

Lys Thr Ala Asn Lys Val Val Glu Ala Gly Leu Phe Ala Gly Trp Val
        595                 600                 605

Lys Gln Ile Val Asn Asp Phe Val Ile Glu Ala Asn Lys Ser Asn Thr
    610                 615                 620

Met Asp Lys Ile Ala Asp Ile Ser Leu Ile Val Pro Tyr Ile Gly Leu
625                 630                 635                 640

Ala Leu Asn Val Gly Asn Glu Thr Ala Lys Gly Asn Phe Glu Asn Ala
                645                 650                 655

Phe Glu Ile Ala Gly Ala Ser Ile Leu Leu Glu Phe Ile Pro Glu Leu
            660                 665                 670

Leu Ile Pro Val Val Gly Ala Phe Leu Leu Glu Ser Tyr Ile Asp Asn
        675                 680                 685

Lys Asn Lys Ile Ile Lys Thr Ile Asp Asn Ala Leu Thr Lys Arg Asn
    690                 695                 700

Glu Lys Trp Ser Asp Met Tyr Gly Leu Ile Val Ala Gln Trp Leu Ser
705                 710                 715                 720

Thr Val Asn Thr Gln Phe Tyr Thr Ile Lys Glu Gly Met Tyr Lys Ala
                725                 730                 735

Leu Asn Tyr Gln Ala Gln Ala Leu Glu Glu Ile Ile Lys Tyr Arg Tyr
            740                 745                 750

Asn Ile Tyr Ser Glu Lys Glu Lys Ser Asn Ile Asn Ile Asp Phe Asn
        755                 760                 765

Asp Ile Asn Ser Lys Leu Asn Glu Gly Ile Asn Gln Ala Ile Asp Asn
    770                 775                 780

Ile Asn Asn Phe Ile Asn Gly Cys Ser Val Ser Tyr Leu Met Lys Lys
785                 790                 795                 800

Met Ile Pro Leu Ala Val Glu Lys Leu Leu Asp Phe Asp Asn Thr Leu
                805                 810                 815

Lys Lys Asn Leu Leu Asn Tyr Ile Asp Glu Asn Lys Leu Tyr Leu Ile
            820                 825                 830

Gly Ser Ala Glu Tyr Glu Lys Ser Lys Val Asn Lys Tyr Leu Lys Thr
        835                 840                 845

Ile Met Pro Phe Asp Leu Ser Ile Tyr Thr Asn Asp Thr Ile Leu Ile
    850                 855                 860

Glu Met Phe Asn Lys Tyr Asn Ser Leu Glu Gly Gly Gly Gly Ser Gly
865                 870                 875                 880

Gly Gly Gly Ser Gly Gly Gly Gly Ser Gly Gly Gly Gly Ser Gly Gly
                885                 890                 895

Gly Gly Ser Gly Gly Gly Gly Ser Ala Leu Asp Gln Glu Gly Ala Pro
            900                 905                 910

Pro Gln Gln Ser Ala Arg Arg Asp Arg Met Pro Cys Arg Asn Phe Phe
        915                 920                 925

Trp Lys Thr Phe Ser Ser Cys Lys
    930                 935

<210> 32

<211> 2065
<212> DNA
<213> Artificial Sequence

<220>
<221> source
<223> /note="Description of Artificial Sequence: Synthetic polynucleotide"

<400> 32
ggatccatgg agtccaatca gccggaaaaa aatggaaccg cgactaaacc cgagaattcg 60

gggaacacta cgtcggaaaa cggccagacg gaacctgaga agaaactgga actacgaaat 120

gtgtccgata tcgagctata ctctcaaacc aatggaacct ataggcagca tgtttcattg 180

gacggaatcc cagaaaatac ggatacatat ttcgtcaaag tgaagtctag cgcattcaag 240

gatgtatata tccccgttgc gagtattaca gaagagaagc ggaacggtca aagcgtttat 300

aagattacag caaaggccga aaagttacaa caggagttag aaaacaaata cgttgacaat 360

ttcacttttt atctcgataa aaaggctaaa gaggaaaaca cgaacttcac gtcatttagt 420

aatctggtca aagccataaa tcaaaatcca tctggtacat accatctcgc ggcaagtcta 480

aacgcgaatg aagtagaact tggcccggac gagcgttcat acattaagga tacctttact 540

ggcagactca taggggaaaa agacggtaag aactatgcta tatacaattt gaaaaagcct 600

ttatttgaga acctgtcggg cgccaccgtc gagaaattgt cccttaaaaa cgtagctata 660

agcggaaaga atgacatcgg tagtcttgca aacgaggcta ctaacgggac aaagattaaa 720

caagtgcacg tagatgggtg tgtcgacggc atcattacct ccaaaactaa atctgacgat 780

gacgataaaa acaaagcgct gaacctgcag tgcattaaaa taaagaatga ggatttgaca 840

ttcatcgcag aaaaaaatag cttcagcgaa gagccgttcc aagatgagat agtaagctac 900

aacaccaaga acaagccgct taattttaat tactcgttag ataaaatcat agttgactac 960

aaccttcaat cgaagatcac gttaccgaat gacagaacaa ctcctgtcac aaaaggaatt 1020

ccctatgcac ctgagtataa gtcaaatgcc gcgtcaacaa tagagattca taatatagat 1080

gacaacacca tctatcaata tctgtacgct cagaaaagtc caacaactct tcagcgtata 1140

acaatgacca atagtgtcga tgacgcattg ataaattcta ccaagatata ctcttatttc 1200

ccgagcgtca tctccaaagt taatcaaggt gctcaaggca ttctattttt gcaatgggtc 1260

cgagacatca tagatgactt cactaatgag tcgtctcaga aaaccacgat tgataaaata 1320

tcagatgttt ccaccatcgt cccctacatc ggacctgcgc ttaacattgt gaagcagggg 1380

tatgagggga attttatcgg agcgttagaa actacggggg ttgtgctatt acttgaatac 1440

ataccagaga taacattgcc cgttatagcg gccctcagta tcgcagaatc aagtacacaa 1500

aaagaaaaga taatcaaaac aatcgacaac ttcctagaaa agaggtacga aaaatggata 1560

gaggtttata aactcgtgaa agcgaaatgg ttaggcactg ttaatacgca gttccaaaag 1620

agatcctatc aaatgtatag atcactggag taccaggtgg atgccataaa gaaaattatc 1680

gactatgaat ataaaatata ttcaggtcca gataaggagc agatagctga tgaaataaac 1740

aatttaaaaa acaaacttga agagaaggcg aataaggcca tgatcaatat caatattttt 1800

atgcgagaat cttcacgatc ttttttggta aatcagatga ttaacgaagc caaaaagcag 1860

ctgcttgagt tcgacacaca gtccaaaaac atactaatgc aatatatcaa agcaaactca 1920

aaattcattg gaattactga gctgaagaaa ctggaatcca aaataaataa agtattctct 1980

accccgatcc cgttctctta ctctaaaaac cttgactgct gggtagataa cgaagaagat 2040

attgacgttc tagagtaata agctt 2065

<210> 33
<211> 913
<212> PRT
<213> Artificial Sequence

<220>
<221> source
<223> /note="Description of Artificial Sequence: Synthetic polypeptide"

<400> 33
Pro Ile Thr Ile Asn Asn Phe Asn Tyr Ser Asp Pro Val Asp Asn Lys
1 5 10 15

Asn Ile Leu Tyr Leu Asp Thr His Leu Asn Thr Leu Ala Asn Glu Pro
20 25 30

Glu Lys Ala Phe Arg Ile Thr Gly Asn Ile Trp Val Ile Pro Asp Arg
35 40 45

Phe Ser Arg Asn Ser Asn Pro Asn Leu Asn Lys Pro Pro Arg Val Thr
50 55 60

Ser Pro Lys Ser Gly Tyr Tyr Asp Pro Asn Tyr Leu Ser Thr Asp Ser
65 70 75 80

Asp Lys Asp Thr Phe Leu Lys Glu Ile Ile Lys Leu Phe Lys Arg Ile
85 90 95

Asn Ser Arg Glu Ile Gly Glu Glu Leu Ile Tyr Arg Leu Ser Thr Asp
100 105 110

Ile Pro Phe Pro Gly Asn Asn Asn Thr Pro Ile Asn Thr Phe Asp Phe
115 120 125

Asp Val Asp Phe Asn Ser Val Asp Val Lys Thr Arg Gln Gly Asn Asn
130 135 140

Trp Val Lys Thr Gly Ser Ile Asn Pro Ser Val Ile Ile Thr Gly Pro
145 150 155 160

Arg Glu Asn Ile Ile Asp Pro Glu Thr Ser Thr Phe Lys Leu Thr Asn
165 170 175

Asn Thr Phe Ala Ala Gln Glu Gly Phe Gly Ala Leu Ser Ile Ile Ser
180 185 190

Ile Ser Pro Arg Phe Met Leu Thr Tyr Ser Asn Ala Thr Asn Asp Val
195 200 205

Gly Glu Gly Arg Phe Ser Lys Ser Glu Phe Cys Met Asp Pro Ile Leu
210 215 220

Ile Leu Met His Glu Leu Asn His Ala Met His Asn Leu Tyr Gly Ile
225 230 235 240

Ala Ile Pro Asn Asp Gln Thr Ile Ser Ser Val Thr Ser Asn Ile Phe
245 250 255

Tyr Ser Gln Tyr Asn Val Lys Leu Glu Tyr Ala Glu Ile Tyr Ala Phe
260 265 270

Gly Gly Pro Thr Ile Asp Leu Ile Pro Lys Ser Ala Arg Lys Tyr Phe
275 280 285

Glu Glu Lys Ala Leu Asp Tyr Tyr Arg Ser Ile Ala Lys Arg Leu Asn
290 295 300

Ser Ile Thr Thr Ala Asn Pro Ser Ser Phe Asn Lys Tyr Ile Gly Glu
305 310 315 320

Tyr Lys Gln Lys Leu Ile Arg Lys Tyr Arg Phe Val Val Glu Ser Ser
325 330 335

Gly Glu Val Thr Val Asn Arg Asn Lys Phe Val Glu Leu Tyr Asn Glu
340 345 350

Leu Thr Gln Ile Phe Thr Glu Phe Asn Tyr Ala Lys Ile Tyr Asn Val
355 360 365

Gln Asn Arg Lys Ile Tyr Leu Ser Asn Val Tyr Thr Pro Val Thr Ala
370 375 380

Asn Ile Leu Asp Asp Asn Val Tyr Asp Ile Gln Asn Gly Phe Asn Ile

385 390 395 400

Pro Lys Ser Asn Leu Asn Val Leu Phe Met Gly Gln Asn Leu Ser Arg
405 410 415

Asn Pro Ala Leu Arg Lys Val Asn Pro Glu Asn Met Leu Tyr Leu Phe
420 425 430

Thr Lys Phe Cys Val Asp Ala Ile Asp Gly Arg Ser Leu Tyr Asn Lys
435 440 445

Thr Leu Gln Cys Arg Glu Leu Leu Val Lys Asn Thr Asp Leu Pro Phe
450 455 460

Ile Gly Asp Ile Ser Asp Val Lys Thr Asp Ile Phe Leu Arg Lys Asp
465 470 475 480

Ile Asn Glu Glu Thr Glu Val Ile Tyr Tyr Pro Asp Asn Val Ser Val
485 490 495

Asp Gln Val Ile Leu Ser Lys Asn Thr Ser Glu His Gly Gln Leu Asp
500 505 510

Leu Leu Tyr Pro Ser Ile Asp Ser Glu Ser Glu Ile Leu Pro Gly Glu
515 520 525

Asn Gln Val Phe Tyr Asp Asn Arg Thr Gln Asn Val Asp Tyr Leu Asn
530 535 540

Ser Tyr Tyr Tyr Leu Glu Ser Gln Lys Leu Ser Asp Asn Val Glu Asp
545 550 555 560

Phe Thr Phe Thr Arg Ser Ile Glu Glu Ala Leu Asp Asn Ser Ala Lys
565 570 575

Val Tyr Thr Tyr Phe Pro Thr Leu Ala Asn Lys Val Asn Ala Gly Val
580 585 590

Gln Gly Gly Leu Phe Leu Met Trp Ala Asn Asp Val Val Glu Asp Phe
595 600 605

Thr Thr Asn Ile Leu Arg Lys Asp Thr Leu Asp Lys Ile Ser Asp Val
610 615 620

Ser Ala Ile Ile Pro Tyr Ile Gly Pro Ala Leu Asn Ile Ser Asn Ser
625 630 635 640

Val Arg Arg Gly Asn Phe Thr Glu Ala Phe Ala Val Thr Gly Val Thr
645 650 655

Ile Leu Leu Glu Ala Phe Pro Glu Phe Thr Ile Pro Ala Leu Gly Ala
660 665 670

Phe Val Ile Tyr Ser Lys Val Gln Glu Arg Asn Glu Ile Ile Lys Thr
675 680 685

Ile Asp Asn Cys Leu Glu Gln Arg Ile Lys Arg Trp Lys Asp Ser Tyr
690 695 700

Glu Trp Met Met Gly Thr Trp Leu Ser Arg Ile Ile Thr Gln Phe Asn
705 710 715 720

Asn Ile Ser Tyr Gln Met Tyr Asp Ser Leu Asn Tyr Gln Ala Gly Ala
725 730 735

Ile Lys Ala Lys Ile Asp Leu Glu Tyr Lys Lys Tyr Ser Gly Ser Asp
740 745 750

Lys Glu Asn Ile Lys Ser Gln Val Glu Asn Leu Lys Asn Ser Leu Asp
755 760 765

Val Lys Ile Ser Glu Ala Met Asn Asn Ile Asn Lys Phe Ile Arg Glu
770 775 780

Cys Ser Val Thr Tyr Leu Phe Lys Asn Met Leu Pro Lys Val Ile Asp
785 790 795 800

Glu Leu Asn Glu Phe Asp Arg Asn Thr Lys Ala Lys Leu Ile Asn Leu
805 810 815

Ile Asp Ser His Asn Ile Ile Leu Val Gly Glu Val Asp Lys Leu Lys
820 825 830

Ala Lys Val Asn Asn Ser Phe Gln Asn Thr Ile Pro Phe Asn Ile Phe
835 840 845

Ser Tyr Thr Asn Asn Ser Leu Leu Lys Asp Ile Ile Asn Glu Tyr Phe
850 855 860

Asn Leu Glu Gly Gly Gly Gly Ser Gly Gly Gly Gly Ser Gly Gly Gly
865 870 875 880

Gly Ser Ala Leu Val Gly Ser Ser Phe Leu Ser Pro Glu His Gln Arg
885 890 895

Val Gln Gln Arg Lys Glu Ser Lys Lys Pro Pro Ala Lys Leu Gln Pro
900 905 910

Arg

<210> 34
<211> 931
<212> PRT
<213> Artificial Sequence

<220>
<221> source
<223> /note="Description of Artificial Sequence: Synthetic polypeptide"

<400> 34
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

```
Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
225                 230                 235                 240

Asn Ile Pro Ser Asp Lys Arg Ile Arg Pro Gln Val Ser Glu Gly Phe
                245                 250                 255

Phe Ser Gln Asp Gly Pro Asn Val Gln Phe Glu Glu Leu Tyr Thr Phe
            260                 265                 270

Gly Gly Leu Asp Val Glu Ile Ile Pro Gln Ile Glu Arg Ser Gln Leu
        275                 280                 285

Arg Glu Lys Ala Leu Gly His Tyr Lys Asp Ile Ala Lys Arg Leu Asn
    290                 295                 300

Asn Ile Asn Lys Thr Ile Pro Ser Ser Trp Ile Ser Asn Ile Asp Lys
305                 310                 315                 320

Tyr Lys Lys Ile Phe Ser Glu Lys Tyr Asn Phe Asp Lys Asp Asn Thr
                325                 330                 335

Gly Asn Phe Val Val Asn Ile Asp Lys Phe Asn Ser Leu Tyr Ser Asp
            340                 345                 350

Leu Thr Asn Val Met Ser Glu Val Val Tyr Ser Ser Gln Tyr Asn Val
        355                 360                 365

Lys Asn Arg Thr His Tyr Phe Ser Arg His Tyr Leu Pro Val Phe Ala
    370                 375                 380

Asn Ile Leu Asp Asp Asn Ile Tyr Thr Ile Arg Asp Gly Phe Asn Leu
385                 390                 395                 400

Thr Asn Lys Gly Phe Asn Ile Glu Asn Ser Gly Gln Asn Ile Glu Arg
                405                 410                 415

Asn Pro Ala Leu Gln Lys Leu Ser Ser Glu Ser Val Val Asp Leu Phe
            420                 425                 430
```

Thr Lys Val Cys Val Asp Lys Ser Glu Glu Lys Leu Tyr Asp Asp Asp
435 440 445

Asp Lys Asp Arg Trp Gly Ser Ser Leu Gln Cys Ile Lys Val Lys Asn
450 455 460

Asn Arg Leu Pro Tyr Val Ala Asp Lys Asp Ser Ile Ser Gln Glu Ile
465 470 475 480

Phe Glu Asn Lys Ile Ile Thr Asp Glu Thr Asn Val Gln Asn Tyr Ser
485 490 495

Asp Lys Phe Ser Leu Asp Glu Ser Ile Leu Asp Gly Gln Val Pro Ile
500 505 510

Asn Pro Glu Ile Val Asp Pro Leu Leu Pro Asn Val Asn Met Glu Pro
515 520 525

Leu Asn Leu Pro Gly Glu Glu Ile Val Phe Tyr Asp Asp Ile Thr Lys
530 535 540

Tyr Val Asp Tyr Leu Asn Ser Tyr Tyr Tyr Leu Glu Ser Gln Lys Leu
545 550 555 560

Ser Asn Asn Val Glu Asn Ile Thr Leu Thr Thr Ser Val Glu Glu Ala
565 570 575

Leu Gly Tyr Ser Asn Lys Ile Tyr Thr Phe Leu Pro Ser Leu Ala Glu
580 585 590

Lys Val Asn Lys Gly Val Gln Ala Gly Leu Phe Leu Asn Trp Ala Asn
595 600 605

Glu Val Val Glu Asp Phe Thr Thr Asn Ile Met Lys Lys Asp Thr Leu
610 615 620

Asp Lys Ile Ser Asp Val Ser Val Ile Ile Pro Tyr Ile Gly Pro Ala
625 630 635 640

Leu Asn Ile Gly Asn Ser Ala Leu Arg Gly Asn Phe Asn Gln Ala Phe
645 650 655

Ala Thr Ala Gly Val Ala Phe Leu Leu Glu Gly Phe Pro Glu Phe Thr
660 665 670

Ile Pro Ala Leu Gly Val Phe Thr Phe Tyr Ser Ser Ile Gln Glu Arg

675 680 685

Glu Lys Ile Ile Lys Thr Ile Glu Asn Cys Leu Glu Gln Arg Val Lys
690 695 700

Arg Trp Lys Asp Ser Tyr Gln Trp Met Val Ser Asn Trp Leu Ser Arg
705 710 715 720

Ile Thr Thr Gln Phe Asn His Ile Asn Tyr Gln Met Tyr Asp Ser Leu
725 730 735

Ser Tyr Gln Ala Asp Ala Ile Lys Ala Lys Ile Asp Leu Glu Tyr Lys
740 745 750

Lys Tyr Ser Gly Ser Asp Lys Glu Asn Ile Lys Ser Gln Val Glu Asn
755 760 765

Leu Lys Asn Ser Leu Asp Val Lys Ile Ser Glu Ala Met Asn Asn Ile
770 775 780

Asn Lys Phe Ile Arg Glu Cys Ser Val Thr Tyr Leu Phe Lys Asn Met
785 790 795 800

Leu Pro Lys Val Ile Asp Glu Leu Asn Lys Phe Asp Leu Arg Thr Lys
805 810 815

Thr Glu Leu Ile Asn Leu Ile Asp Ser His Asn Ile Ile Leu Val Gly
820 825 830

Glu Val Asp Arg Leu Lys Ala Lys Val Asn Glu Ser Phe Glu Asn Thr
835 840 845

Met Pro Phe Asn Ile Phe Ser Tyr Thr Asn Asn Ser Leu Leu Lys Asp
850 855 860

Ile Ile Asn Glu Tyr Phe Asn Leu Glu Gly Gly Gly Gly Ser Gly Gly
865 870 875 880

Gly Gly Ser Gly Gly Gly Gly Ser Ala Leu Val Tyr Ala Asp Ala Ile
885 890 895

Phe Thr Asn Ser Tyr Arg Lys Val Leu Gly Gln Leu Ser Ala Arg Lys
900 905 910

Leu Leu Gln Asp Ile Met Ser Arg Gln Gln Gly Glu Ser Asn Gln Glu
915 920 925

```
Arg Gly Ala
    930


<210> 35
<211> 919
<212> PRT
<213> Artificial Sequence

<220>
<221> source
<223> /note="Description of Artificial Sequence: Synthetic
      polypeptide"

<400> 35
Thr Trp Pro Val Lys Asp Phe Asn Tyr Ser Asp Pro Val Asn Asp Asn
1               5                   10                  15


Asp Ile Leu Tyr Leu Arg Ile Pro Gln Asn Lys Leu Ile Thr Thr Pro
            20                  25                  30


Val Lys Ala Phe Met Ile Thr Gln Asn Ile Trp Val Ile Pro Glu Arg
        35                  40                  45


Phe Ser Ser Asp Thr Asn Pro Ser Leu Ser Lys Pro Pro Arg Pro Thr
    50                  55                  60


Ser Lys Tyr Gln Ser Tyr Tyr Asp Pro Ser Tyr Leu Ser Thr Asp Glu
65                  70                  75                  80


Gln Lys Asp Thr Phe Leu Lys Gly Ile Ile Lys Leu Phe Lys Arg Ile
                85                  90                  95


Asn Glu Arg Asp Ile Gly Lys Lys Leu Ile Asn Tyr Leu Val Val Gly
            100                 105                 110


Ser Pro Phe Met Gly Asp Ser Ser Thr Pro Glu Asp Thr Phe Asp Phe
        115                 120                 125


Thr Arg His Thr Thr Asn Ile Ala Val Glu Lys Phe Glu Asn Gly Ser
    130                 135                 140


Trp Lys Val Thr Asn Ile Ile Thr Pro Ser Val Leu Ile Phe Gly Pro
145                 150                 155                 160


Leu Pro Asn Ile Leu Asp Tyr Thr Ala Ser Leu Thr Leu Gln Gly Gln
                165                 170                 175


Gln Ser Asn Pro Ser Phe Glu Gly Phe Gly Thr Leu Ser Ile Leu Lys
            180                 185                 190
```

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
225                 230                 235                 240

Asn Ile Pro Ser Asp Lys Arg Ile Arg Pro Gln Val Ser Glu Gly Phe
                245                 250                 255

Phe Ser Gln Asp Gly Pro Asn Val Gln Phe Glu Glu Leu Tyr Thr Phe
            260                 265                 270

Gly Gly Leu Asp Val Glu Ile Ile Pro Gln Ile Glu Arg Ser Gln Leu
        275                 280                 285

Arg Glu Lys Ala Leu Gly His Tyr Lys Asp Ile Ala Lys Arg Leu Asn
    290                 295                 300

Asn Ile Asn Lys Thr Ile Pro Ser Ser Trp Ile Ser Asn Ile Asp Lys
305                 310                 315                 320

Tyr Lys Lys Ile Phe Ser Glu Lys Tyr Asn Phe Asp Lys Asp Asn Thr
                325                 330                 335

Gly Asn Phe Val Val Asn Ile Asp Lys Phe Asn Ser Leu Tyr Ser Asp
            340                 345                 350

Leu Thr Asn Val Met Ser Glu Val Val Tyr Ser Ser Gln Tyr Asn Val
        355                 360                 365

Lys Asn Arg Thr His Tyr Phe Ser Arg His Tyr Leu Pro Val Phe Ala
    370                 375                 380

Asn Ile Leu Asp Asp Asn Ile Tyr Thr Ile Arg Asp Gly Phe Asn Leu
385                 390                 395                 400

Thr Asn Lys Gly Phe Asn Ile Glu Asn Ser Gly Gln Asn Ile Glu Arg
                405                 410                 415

Asn Pro Ala Leu Gln Lys Leu Ser Ser Glu Ser Val Val Asp Leu Phe
            420                 425                 430

Thr Lys Val Cys Val Asp Lys Ser Glu Glu Lys Leu Tyr Asp Asp Asp
        435                 440                 445

```
Asp Lys Asp Arg Trp Gly Ser Ser Leu Gln Cys Ile Lys Val Lys Asn
    450                 455                 460

Asn Arg Leu Pro Tyr Val Ala Asp Lys Asp Ser Ile Ser Gln Glu Ile
465                 470                 475                 480

Phe Glu Asn Lys Ile Ile Thr Asp Glu Thr Asn Val Gln Asn Tyr Ser
                485                 490                 495

Asp Lys Phe Ser Leu Asp Glu Ser Ile Leu Asp Gly Gln Val Pro Ile
            500                 505                 510

Asn Pro Glu Ile Val Asp Pro Leu Leu Pro Asn Val Asn Met Glu Pro
        515                 520                 525

Leu Asn Leu Pro Gly Glu Glu Ile Val Phe Tyr Asp Asp Ile Thr Lys
    530                 535                 540

Tyr Val Asp Tyr Leu Asn Ser Tyr Tyr Tyr Leu Glu Ser Gln Lys Leu
545                 550                 555                 560

Ser Asn Asn Val Glu Asn Ile Thr Leu Thr Thr Ser Val Glu Glu Ala
                565                 570                 575

Leu Gly Tyr Ser Asn Lys Ile Tyr Thr Phe Leu Pro Ser Leu Ala Glu
            580                 585                 590

Lys Val Asn Lys Gly Val Gln Ala Gly Leu Phe Leu Asn Trp Ala Asn
        595                 600                 605

Glu Val Val Glu Asp Phe Thr Thr Asn Ile Met Lys Lys Asp Thr Leu
    610                 615                 620

Asp Lys Ile Ser Asp Val Ser Val Ile Ile Pro Tyr Ile Gly Pro Ala
625                 630                 635                 640

Leu Asn Ile Gly Asn Ser Ala Leu Arg Gly Asn Phe Asn Gln Ala Phe
                645                 650                 655

Ala Thr Ala Gly Val Ala Phe Leu Leu Glu Gly Phe Pro Glu Phe Thr
            660                 665                 670

Ile Pro Ala Leu Gly Val Phe Thr Phe Tyr Ser Ser Ile Gln Glu Arg
        675                 680                 685

Glu Lys Ile Ile Lys Thr Ile Glu Asn Cys Leu Glu Gln Arg Val Lys
    690                 695                 700
```

Arg Trp Lys Asp Ser Tyr Gln Trp Met Val Ser Asn Trp Leu Ser Arg
705                 710                 715                 720

Ile Thr Thr Gln Phe Asn His Ile Asn Tyr Gln Met Tyr Asp Ser Leu
                725                 730                 735

Ser Tyr Gln Ala Asp Ala Ile Lys Ala Lys Ile Asp Leu Glu Tyr Lys
            740                 745                 750

Lys Tyr Ser Gly Ser Asp Lys Glu Asn Ile Lys Ser Gln Val Glu Asn
        755                 760                 765

Leu Lys Asn Ser Leu Asp Val Lys Ile Ser Glu Ala Met Asn Asn Ile
    770                 775                 780

Asn Lys Phe Ile Arg Glu Cys Ser Val Thr Tyr Leu Phe Lys Asn Met
785                 790                 795                 800

Leu Pro Lys Val Ile Asp Glu Leu Asn Lys Phe Asp Leu Arg Thr Lys
                805                 810                 815

Thr Glu Leu Ile Asn Leu Ile Asp Ser His Asn Ile Ile Leu Val Gly
            820                 825                 830

Glu Val Asp Arg Leu Lys Ala Lys Val Asn Glu Ser Phe Glu Asn Thr
        835                 840                 845

Met Pro Phe Asn Ile Phe Ser Tyr Thr Asn Asn Ser Leu Leu Lys Asp
    850                 855                 860

Ile Ile Asn Glu Tyr Phe Asn Leu Glu Gly Gly Gly Gly Ser Gly Gly
865                 870                 875                 880

Gly Gly Ser Gly Gly Gly Gly Ser Ala Leu Val Gly Ser Ser Phe Leu
                885                 890                 895

Ser Pro Glu His Gln Arg Val Gln Gln Arg Lys Glu Ser Lys Lys Pro
            900                 905                 910

Pro Ala Lys Leu Gln Pro Arg
        915

<210> 36
<211> 912
<212> PRT
<213> Artificial Sequence

<220>
<221> source
<223> /note="Description of Artificial Sequence: Synthetic polypeptide"

<400> 36

```
Glu Phe Val Asn Lys Gln Phe Asn Tyr Lys Asp Pro Val Asn Gly Val
1               5                   10                  15

Asp Ile Ala Tyr Ile Lys Ile Pro Asn Ala Gly Gln Met Gln Pro Val
            20                  25                  30

Lys Ala Phe Lys Ile His Asn Lys Ile Trp Val Ile Pro Glu Arg Asp
        35                  40                  45

Thr Phe Thr Asn Pro Glu Glu Gly Asp Leu Asn Pro Pro Pro Glu Ala
    50                  55                  60

Lys Gln Val Pro Val Ser Tyr Tyr Asp Ser Thr Tyr Leu Ser Thr Asp
65                  70                  75                  80

Asn Glu Lys Asp Asn Tyr Leu Lys Gly Val Thr Lys Leu Phe Glu Arg
                85                  90                  95

Ile Tyr Ser Thr Asp Leu Gly Arg Met Leu Leu Thr Ser Ile Val Arg
            100                 105                 110

Gly Ile Pro Phe Trp Gly Gly Ser Thr Ile Asp Thr Glu Leu Lys Val
        115                 120                 125

Ile Asp Thr Asn Cys Ile Asn Val Ile Gln Pro Asp Gly Ser Tyr Arg
    130                 135                 140

Ser Glu Glu Leu Asn Leu Val Ile Ile Gly Pro Ser Ala Asp Ile Ile
145                 150                 155                 160

Gln Phe Glu Cys Lys Ser Phe Gly His Glu Val Leu Asn Leu Thr Arg
                165                 170                 175

Asn Gly Tyr Gly Ser Thr Gln Tyr Ile Arg Phe Ser Pro Asp Phe Thr
            180                 185                 190

Phe Gly Phe Glu Glu Ser Leu Glu Val Asp Thr Asn Pro Leu Leu Gly
        195                 200                 205

Ala Gly Lys Phe Ala Thr Asp Pro Ala Val Thr Leu Ala His Glu Leu
    210                 215                 220

Ile His Ala Gly His Arg Leu Tyr Gly Ile Ala Ile Asn Pro Asn Arg
```

225 230 235 240

Val Phe Lys Val Asn Thr Asn Ala Tyr Tyr Glu Met Ser Gly Leu Glu
245 250 255

Val Ser Phe Glu Glu Leu Arg Thr Phe Gly Gly His Asp Ala Lys Phe
260 265 270

Ile Asp Ser Leu Gln Glu Asn Glu Phe Arg Leu Tyr Tyr Tyr Asn Lys
275 280 285

Phe Lys Asp Ile Ala Ser Thr Leu Asn Lys Ala Lys Ser Ile Val Gly
290 295 300

Thr Thr Ala Ser Leu Gln Tyr Met Lys Asn Val Phe Lys Glu Lys Tyr
305 310 315 320

Leu Leu Ser Glu Asp Thr Ser Gly Lys Phe Ser Val Asp Lys Leu Lys
325 330 335

Phe Asp Lys Leu Tyr Lys Met Leu Thr Glu Ile Tyr Thr Glu Asp Asn
340 345 350

Phe Val Lys Phe Phe Lys Val Leu Asn Arg Lys Thr Tyr Leu Asn Phe
355 360 365

Asp Lys Ala Val Phe Lys Ile Asn Ile Val Pro Lys Val Asn Tyr Thr
370 375 380

Ile Tyr Asp Gly Phe Asn Leu Arg Asn Thr Asn Leu Ala Ala Asn Phe
385 390 395 400

Asn Gly Gln Asn Thr Glu Ile Asn Asn Met Asn Phe Thr Lys Leu Lys
405 410 415

Asn Phe Thr Gly Leu Phe Glu Phe Tyr Lys Leu Leu Cys Val Asp Gly
420 425 430

Ile Ile Thr Ser Lys Thr Lys Ser Asp Asp Asp Asp Lys Asn Lys Ala
435 440 445

Leu Asn Leu Gln Cys Ile Lys Val Asn Asn Trp Asp Leu Phe Phe Ser
450 455 460

Pro Ser Glu Asp Asn Phe Thr Asn Asp Leu Asn Lys Gly Glu Glu Ile
465 470 475 480

```
Thr Ser Asp Thr Asn Ile Glu Ala Ala Glu Glu Asn Ile Ser Leu Asp
                485                 490                 495

Leu Ile Gln Gln Tyr Tyr Leu Thr Phe Asn Phe Asp Asn Glu Pro Glu
            500                 505                 510

Asn Ile Ser Ile Glu Asn Leu Ser Ser Asp Ile Ile Gly Gln Leu Glu
        515                 520                 525

Leu Met Pro Asn Ile Glu Arg Phe Pro Asn Gly Lys Lys Tyr Glu Leu
    530                 535                 540

Asp Lys Tyr Thr Met Phe His Tyr Leu Arg Ala Gln Glu Phe Glu His
545                 550                 555                 560

Gly Lys Ser Arg Ile Ala Leu Thr Asn Ser Val Asn Glu Ala Leu Leu
                565                 570                 575

Asn Pro Ser Arg Val Tyr Thr Phe Phe Ser Ser Asp Tyr Val Lys Lys
            580                 585                 590

Val Asn Lys Ala Thr Glu Ala Ala Met Phe Leu Gly Trp Val Glu Gln
        595                 600                 605

Leu Val Tyr Asp Phe Thr Asp Glu Thr Ser Glu Val Ser Thr Thr Asp
    610                 615                 620

Lys Ile Ala Asp Ile Thr Ile Ile Ile Pro Tyr Ile Gly Pro Ala Leu
625                 630                 635                 640

Asn Ile Gly Asn Met Leu Tyr Lys Asp Asp Phe Val Gly Ala Leu Ile
                645                 650                 655

Phe Ser Gly Ala Val Ile Leu Leu Glu Phe Ile Pro Glu Ile Ala Ile
            660                 665                 670

Pro Val Leu Gly Thr Phe Ala Leu Val Ser Tyr Ile Ala Asn Lys Val
        675                 680                 685

Leu Thr Val Gln Thr Ile Asp Asn Ala Leu Ser Lys Arg Asn Glu Lys
    690                 695                 700

Trp Asp Glu Val Tyr Lys Tyr Ile Val Thr Asn Trp Leu Ala Lys Val
705                 710                 715                 720

Asn Thr Gln Ile Asp Leu Ile Arg Lys Lys Met Lys Glu Ala Leu Glu
                725                 730                 735
```

Asn Gln Ala Glu Ala Thr Lys Ala Ile Ile Asn Tyr Gln Tyr Asn Gln
740 745 750

Tyr Thr Glu Glu Glu Lys Asn Asn Ile Asn Phe Asn Ile Asp Asp Leu
755 760 765

Ser Ser Lys Leu Asn Glu Ser Ile Asn Lys Ala Met Ile Asn Ile Asn
770 775 780

Lys Phe Leu Asn Gln Cys Ser Val Ser Tyr Leu Met Asn Ser Met Ile
785 790 795 800

Pro Tyr Gly Val Lys Arg Leu Glu Asp Phe Asp Ala Ser Leu Lys Asp
805 810 815

Ala Leu Leu Lys Tyr Ile Tyr Asp Asn Arg Gly Thr Leu Ile Gly Gln
820 825 830

Val Asp Arg Leu Lys Asp Lys Val Asn Asn Thr Leu Ser Thr Asp Ile
835 840 845

Pro Phe Gln Leu Ser Lys Tyr Val Asp Asn Gln Arg Leu Leu Ser Thr
850 855 860

Leu Glu Gly Gly Gly Gly Ser Gly Gly Gly Gly Ser Gly Gly Gly Gly
865 870 875 880

Ser Ala Leu Val Gly Ser Ser Phe Leu Ser Pro Glu His Gln Arg Val
885 890 895

Gln Gln Arg Lys Glu Ser Lys Lys Pro Pro Ala Lys Leu Gln Pro Arg
900 905 910

<210> 37
<211> 714
<212> PRT
<213> Artificial Sequence

<220>
<221> source
<223> /note="Description of Artificial Sequence: Synthetic polypeptide"

<400> 37
Glu Ser Asn Gln Pro Glu Lys Asn Gly Thr Ala Thr Lys Pro Glu Asn
1 5 10 15

Ser Gly Asn Thr Thr Ser Glu Asn Gly Gln Thr Glu Pro Glu Lys Lys
20 25 30

```
Leu Glu Leu Arg Asn Val Ser Asp Ile Glu Leu Tyr Ser Gln Thr Asn
        35                  40                  45

Gly Thr Tyr Arg Gln His Val Ser Leu Asp Gly Ile Pro Glu Asn Thr
    50                  55                  60

Asp Thr Tyr Phe Val Lys Val Lys Ser Ser Ala Phe Lys Asp Val Tyr
65                  70                  75                  80

Ile Pro Val Ala Ser Ile Thr Glu Glu Lys Arg Asn Gly Gln Ser Val
                85                  90                  95

Tyr Lys Ile Thr Ala Lys Ala Glu Lys Leu Gln Gln Glu Leu Glu Asn
            100                 105                 110

Lys Tyr Val Asp Asn Phe Thr Phe Tyr Leu Asp Lys Lys Ala Lys Glu
        115                 120                 125

Glu Asn Thr Asn Phe Thr Ser Phe Ser Asn Leu Val Lys Ala Ile Asn
    130                 135                 140

Gln Asn Pro Ser Gly Thr Tyr His Leu Ala Ala Ser Leu Asn Ala Asn
145                 150                 155                 160

Glu Val Glu Leu Gly Pro Asp Glu Arg Ser Tyr Ile Lys Asp Thr Phe
                165                 170                 175

Thr Gly Arg Leu Ile Gly Glu Lys Asp Gly Lys Asn Tyr Ala Ile Tyr
            180                 185                 190

Asn Leu Lys Lys Pro Leu Phe Glu Asn Leu Ser Gly Ala Thr Val Glu
        195                 200                 205

Lys Leu Ser Leu Lys Asn Val Ala Ile Ser Gly Lys Asn Asp Ile Gly
    210                 215                 220

Ser Leu Ala Asn Glu Ala Thr Asn Gly Thr Lys Ile Lys Gln Val His
225                 230                 235                 240

Val Asp Gly Cys Val Asp Gly Ile Ile Thr Ser Lys Thr Lys Ser Asp
                245                 250                 255

Asp Asp Asp Lys Asn Lys Ala Leu Asn Leu Gln Cys Ile Lys Ile Lys
            260                 265                 270

Asn Glu Asp Leu Thr Phe Ile Ala Glu Lys Asn Ser Phe Ser Glu Glu
        275                 280                 285
```

Pro Phe Gln Asp Glu Ile Val Ser Tyr Asn Thr Lys Asn Lys Pro Leu
290 295 300

Asn Phe Asn Tyr Ser Leu Asp Lys Ile Ile Val Asp Tyr Asn Leu Gln
305 310 315 320

Ser Lys Ile Thr Leu Pro Asn Asp Arg Thr Thr Pro Val Thr Lys Gly
325 330 335

Ile Pro Tyr Ala Pro Glu Tyr Lys Ser Asn Ala Ala Ser Thr Ile Glu
340 345 350

Ile His Asn Ile Asp Asp Asn Thr Ile Tyr Gln Tyr Leu Tyr Ala Gln
355 360 365

Lys Ser Pro Thr Thr Leu Gln Arg Ile Thr Met Thr Asn Ser Val Asp
370 375 380

Asp Ala Leu Ile Asn Ser Thr Lys Ile Tyr Ser Tyr Phe Pro Ser Val
385 390 395 400

Ile Ser Lys Val Asn Gln Gly Ala Gln Gly Ile Leu Phe Leu Gln Trp
405 410 415

Val Arg Asp Ile Ile Asp Asp Phe Thr Asn Glu Ser Ser Gln Lys Thr
420 425 430

Thr Ile Asp Lys Ile Ser Asp Val Ser Thr Ile Val Pro Tyr Ile Gly
435 440 445

Pro Ala Leu Asn Ile Val Lys Gln Gly Tyr Glu Gly Asn Phe Ile Gly
450 455 460

Ala Leu Glu Thr Thr Gly Val Val Leu Leu Leu Glu Tyr Ile Pro Glu
465 470 475 480

Ile Thr Leu Pro Val Ile Ala Ala Leu Ser Ile Ala Glu Ser Ser Thr
485 490 495

Gln Lys Glu Lys Ile Ile Lys Thr Ile Asp Asn Phe Leu Glu Lys Arg
500 505 510

Tyr Glu Lys Trp Ile Glu Val Tyr Lys Leu Val Lys Ala Lys Trp Leu
515 520 525

Gly Thr Val Asn Thr Gln Phe Gln Lys Arg Ser Tyr Gln Met Tyr Arg

530 535 540

Ser Leu Glu Tyr Gln Val Asp Ala Ile Lys Lys Ile Ile Asp Tyr Glu
545 550 555 560

Tyr Lys Ile Tyr Ser Gly Pro Asp Lys Glu Gln Ile Ala Asp Glu Ile
565 570 575

Asn Asn Leu Lys Asn Lys Leu Glu Glu Lys Ala Asn Lys Ala Met Ile
580 585 590

Asn Ile Asn Ile Phe Met Arg Glu Ser Ser Arg Ser Phe Leu Val Asn
595 600 605

Gln Met Ile Asn Glu Ala Lys Lys Gln Leu Leu Glu Phe Asp Thr Gln
610 615 620

Ser Lys Asn Ile Leu Met Gln Tyr Ile Lys Ala Asn Ser Lys Phe Ile
625 630 635 640

Gly Ile Thr Glu Leu Lys Lys Leu Glu Ser Lys Ile Asn Lys Val Phe
645 650 655

Ser Thr Pro Ile Pro Phe Ser Tyr Ser Lys Asn Leu Asp Cys Trp Val
660 665 670

Asp Asn Glu Glu Asp Ile Asp Val Leu Glu Gly Gly Gly Gly Ser Gly
675 680 685

Gly Gly Gly Ser Gly Gly Gly Gly Ser Ala Leu Val Ala Gly Cys Lys
690 695 700

Asn Phe Phe Trp Lys Thr Phe Thr Ser Cys
705 710

<210> 38
<211> 728
<212> PRT
<213> Artificial Sequence

<220>
<221> source
<223> /note="Description of Artificial Sequence: Synthetic polypeptide"

<400> 38
Glu Ser Asn Gln Pro Glu Lys Asn Gly Thr Ala Thr Lys Pro Glu Asn
1 5 10 15

Ser Gly Asn Thr Thr Ser Glu Asn Gly Gln Thr Glu Pro Glu Lys Lys

20 25 30

Leu Glu Leu Arg Asn Val Ser Asp Ile Glu Leu Tyr Ser Gln Thr Asn
35 40 45

Gly Thr Tyr Arg Gln His Val Ser Leu Asp Gly Ile Pro Glu Asn Thr
50 55 60

Asp Thr Tyr Phe Val Lys Val Lys Ser Ser Ala Phe Lys Asp Val Tyr
65 70 75 80

Ile Pro Val Ala Ser Ile Thr Glu Glu Lys Arg Asn Gly Gln Ser Val
85 90 95

Tyr Lys Ile Thr Ala Lys Ala Glu Lys Leu Gln Gln Glu Leu Glu Asn
100 105 110

Lys Tyr Val Asp Asn Phe Thr Phe Tyr Leu Asp Lys Lys Ala Lys Glu
115 120 125

Glu Asn Thr Asn Phe Thr Ser Phe Ser Asn Leu Val Lys Ala Ile Asn
130 135 140

Gln Asn Pro Ser Gly Thr Tyr His Leu Ala Ala Ser Leu Asn Ala Asn
145 150 155 160

Glu Val Glu Leu Gly Pro Asp Glu Arg Ser Tyr Ile Lys Asp Thr Phe
165 170 175

Thr Gly Arg Leu Ile Gly Glu Lys Asp Gly Lys Asn Tyr Ala Ile Tyr
180 185 190

Asn Leu Lys Lys Pro Leu Phe Glu Asn Leu Ser Gly Ala Thr Val Glu
195 200 205

Lys Leu Ser Leu Lys Asn Val Ala Ile Ser Gly Lys Asn Asp Ile Gly
210 215 220

Ser Leu Ala Asn Glu Ala Thr Asn Gly Thr Lys Ile Lys Gln Val His
225 230 235 240

Val Asp Gly Cys Val Asp Gly Ile Ile Thr Ser Lys Thr Lys Ser Asp
245 250 255

Asp Asp Asp Lys Asn Lys Ala Leu Asn Leu Gln Cys Ile Lys Ile Lys
260 265 270

Asn Glu Asp Leu Thr Phe Ile Ala Glu Lys Asn Ser Phe Ser Glu Glu
275 280 285

Pro Phe Gln Asp Glu Ile Val Ser Tyr Asn Thr Lys Asn Lys Pro Leu
290 295 300

Asn Phe Asn Tyr Ser Leu Asp Lys Ile Ile Val Asp Tyr Asn Leu Gln
305 310 315 320

Ser Lys Ile Thr Leu Pro Asn Asp Arg Thr Thr Pro Val Thr Lys Gly
325 330 335

Ile Pro Tyr Ala Pro Glu Tyr Lys Ser Asn Ala Ala Ser Thr Ile Glu
340 345 350

Ile His Asn Ile Asp Asp Asn Thr Ile Tyr Gln Tyr Leu Tyr Ala Gln
355 360 365

Lys Ser Pro Thr Thr Leu Gln Arg Ile Thr Met Thr Asn Ser Val Asp
370 375 380

Asp Ala Leu Ile Asn Ser Thr Lys Ile Tyr Ser Tyr Phe Pro Ser Val
385 390 395 400

Ile Ser Lys Val Asn Gln Gly Ala Gln Gly Ile Leu Phe Leu Gln Trp
405 410 415

Val Arg Asp Ile Ile Asp Asp Phe Thr Asn Glu Ser Ser Gln Lys Thr
420 425 430

Thr Ile Asp Lys Ile Ser Asp Val Ser Thr Ile Val Pro Tyr Ile Gly
435 440 445

Pro Ala Leu Asn Ile Val Lys Gln Gly Tyr Glu Gly Asn Phe Ile Gly
450 455 460

Ala Leu Glu Thr Thr Gly Val Val Leu Leu Leu Glu Tyr Ile Pro Glu
465 470 475 480

Ile Thr Leu Pro Val Ile Ala Ala Leu Ser Ile Ala Glu Ser Ser Thr
485 490 495

Gln Lys Glu Lys Ile Ile Lys Thr Ile Asp Asn Phe Leu Glu Lys Arg
500 505 510

Tyr Glu Lys Trp Ile Glu Val Tyr Lys Leu Val Lys Ala Lys Trp Leu
515 520 525

Gly Thr Val Asn Thr Gln Phe Gln Lys Arg Ser Tyr Gln Met Tyr Arg
530 535 540

Ser Leu Glu Tyr Gln Val Asp Ala Ile Lys Lys Ile Ile Asp Tyr Glu
545 550 555 560

Tyr Lys Ile Tyr Ser Gly Pro Asp Lys Glu Gln Ile Ala Asp Glu Ile
565 570 575

Asn Asn Leu Lys Asn Lys Leu Glu Glu Lys Ala Asn Lys Ala Met Ile
580 585 590

Asn Ile Asn Ile Phe Met Arg Glu Ser Ser Arg Ser Phe Leu Val Asn
595 600 605

Gln Met Ile Asn Glu Ala Lys Lys Gln Leu Leu Glu Phe Asp Thr Gln
610 615 620

Ser Lys Asn Ile Leu Met Gln Tyr Ile Lys Ala Asn Ser Lys Phe Ile
625 630 635 640

Gly Ile Thr Glu Leu Lys Lys Leu Glu Ser Lys Ile Asn Lys Val Phe
645 650 655

Ser Thr Pro Ile Pro Phe Ser Tyr Ser Lys Asn Leu Asp Cys Trp Val
660 665 670

Asp Asn Glu Glu Asp Ile Asp Val Leu Glu Gly Gly Gly Gly Ser Gly
675 680 685

Gly Gly Gly Ser Gly Gly Gly Gly Ser Ala Leu Val Gly Ser Ser Phe
690 695 700

Leu Ser Pro Glu His Gln Arg Val Gln Gln Arg Lys Glu Ser Lys Lys
705 710 715 720

Pro Pro Ala Lys Leu Gln Pro Arg
725

<210> 39
<211> 899
<212> PRT
<213> Artificial Sequence

<220>
<221> source
<223> /note="Description of Artificial Sequence: Synthetic polypeptide"

<400> 39

```
Glu Phe Val Asn Lys Gln Phe Asn Tyr Lys Asp Pro Val Asn Gly Val
1               5                   10                  15

Asp Ile Ala Tyr Ile Lys Ile Pro Asn Ala Gly Gln Met Gln Pro Val
            20                  25                  30

Lys Ala Phe Lys Ile His Asn Lys Ile Trp Val Ile Pro Glu Arg Asp
        35                  40                  45

Thr Phe Thr Asn Pro Glu Glu Gly Asp Leu Asn Pro Pro Pro Glu Ala
    50                  55                  60

Lys Gln Val Pro Val Ser Tyr Tyr Asp Ser Thr Tyr Leu Ser Thr Asp
65                  70                  75                  80

Asn Glu Lys Asp Asn Tyr Leu Lys Gly Val Thr Lys Leu Phe Glu Arg
                85                  90                  95

Ile Tyr Ser Thr Asp Leu Gly Arg Met Leu Leu Thr Ser Ile Val Arg
            100                 105                 110

Gly Ile Pro Phe Trp Gly Gly Ser Thr Ile Asp Thr Glu Leu Lys Val
        115                 120                 125

Ile Asp Thr Asn Cys Ile Asn Val Ile Gln Pro Asp Gly Ser Tyr Arg
    130                 135                 140

Ser Glu Glu Leu Asn Leu Val Ile Ile Gly Pro Ser Ala Asp Ile Ile
145                 150                 155                 160

Gln Phe Glu Cys Lys Ser Phe Gly His Glu Val Leu Asn Leu Thr Arg
                165                 170                 175

Asn Gly Tyr Gly Ser Thr Gln Tyr Ile Arg Phe Ser Pro Asp Phe Thr
            180                 185                 190

Phe Gly Phe Glu Glu Ser Leu Glu Val Asp Thr Asn Pro Leu Leu Gly
        195                 200                 205

Ala Gly Lys Phe Ala Thr Asp Pro Ala Val Thr Leu Ala His Glu Leu
    210                 215                 220

Ile His Ala Gly His Arg Leu Tyr Gly Ile Ala Ile Asn Pro Asn Arg
225                 230                 235                 240

Val Phe Lys Val Asn Thr Asn Ala Tyr Tyr Glu Met Ser Gly Leu Glu
                245                 250                 255
```

Val Ser Phe Glu Glu Leu Arg Thr Phe Gly Gly His Asp Ala Lys Phe
            260                 265                 270

Ile Asp Ser Leu Gln Glu Asn Glu Phe Arg Leu Tyr Tyr Tyr Asn Lys
        275                 280                 285

Phe Lys Asp Ile Ala Ser Thr Leu Asn Lys Ala Lys Ser Ile Val Gly
    290                 295                 300

Thr Thr Ala Ser Leu Gln Tyr Met Lys Asn Val Phe Lys Glu Lys Tyr
305                 310                 315                 320

Leu Leu Ser Glu Asp Thr Ser Gly Lys Phe Ser Val Asp Lys Leu Lys
                325                 330                 335

Phe Asp Lys Leu Tyr Lys Met Leu Thr Glu Ile Tyr Thr Glu Asp Asn
            340                 345                 350

Phe Val Lys Phe Phe Lys Val Leu Asn Arg Lys Thr Tyr Leu Asn Phe
        355                 360                 365

Asp Lys Ala Val Phe Lys Ile Asn Ile Val Pro Lys Val Asn Tyr Thr
    370                 375                 380

Ile Tyr Asp Gly Phe Asn Leu Arg Asn Thr Asn Leu Ala Ala Asn Phe
385                 390                 395                 400

Asn Gly Gln Asn Thr Glu Ile Asn Asn Met Asn Phe Thr Lys Leu Lys
                405                 410                 415

Asn Phe Thr Gly Leu Phe Glu Phe Tyr Lys Leu Leu Cys Val Asp Gly
            420                 425                 430

Ile Ile Thr Ser Lys Thr Lys Ser Asp Asp Asp Asp Lys Asn Lys Ala
        435                 440                 445

Leu Asn Leu Gln Cys Ile Lys Val Asn Asn Trp Asp Leu Phe Phe Ser
    450                 455                 460

Pro Ser Glu Asp Asn Phe Thr Asn Asp Leu Asn Lys Gly Glu Glu Ile
465                 470                 475                 480

Thr Ser Asp Thr Asn Ile Glu Ala Ala Glu Glu Asn Ile Ser Leu Asp
                485                 490                 495

Leu Ile Gln Gln Tyr Tyr Leu Thr Phe Asn Phe Asp Asn Glu Pro Glu

500 505 510

Asn Ile Ser Ile Glu Asn Leu Ser Ser Asp Ile Ile Gly Gln Leu Glu
515 520 525

Leu Met Pro Asn Ile Glu Arg Phe Pro Asn Gly Lys Lys Tyr Glu Leu
530 535 540

Asp Lys Tyr Thr Met Phe His Tyr Leu Arg Ala Gln Glu Phe Glu His
545 550 555 560

Gly Lys Ser Arg Ile Ala Leu Thr Asn Ser Val Asn Glu Ala Leu Leu
565 570 575

Asn Pro Ser Arg Val Tyr Thr Phe Phe Ser Ser Asp Tyr Val Lys Lys
580 585 590

Val Asn Lys Ala Thr Glu Ala Ala Met Phe Leu Gly Trp Val Glu Gln
595 600 605

Leu Val Tyr Asp Phe Thr Asp Glu Thr Ser Glu Val Ser Thr Thr Asp
610 615 620

Lys Ile Ala Asp Ile Thr Ile Ile Ile Pro Tyr Ile Gly Pro Ala Leu
625 630 635 640

Asn Ile Gly Asn Met Leu Tyr Lys Asp Asp Phe Val Gly Ala Leu Ile
645 650 655

Phe Ser Gly Ala Val Ile Leu Leu Glu Phe Ile Pro Glu Ile Ala Ile
660 665 670

Pro Val Leu Gly Thr Phe Ala Leu Val Ser Tyr Ile Ala Asn Lys Val
675 680 685

Leu Thr Val Gln Thr Ile Asp Asn Ala Leu Ser Lys Arg Asn Glu Lys
690 695 700

Trp Asp Glu Val Tyr Lys Tyr Ile Val Thr Asn Trp Leu Ala Lys Val
705 710 715 720

Asn Thr Gln Ile Asp Leu Ile Arg Lys Lys Met Lys Glu Ala Leu Glu
725 730 735

Asn Gln Ala Glu Ala Thr Lys Ala Ile Ile Asn Tyr Gln Tyr Asn Gln
740 745 750

Tyr Thr Glu Glu Glu Lys Asn Asn Ile Asn Phe Asn Ile Asp Asp Leu
755 760 765

Ser Ser Lys Leu Asn Glu Ser Ile Asn Lys Ala Met Ile Asn Ile Asn
770 775 780

Lys Phe Leu Asn Gln Cys Ser Val Ser Tyr Leu Met Asn Ser Met Ile
785 790 795 800

Pro Tyr Gly Val Lys Arg Leu Glu Asp Phe Asp Ala Ser Leu Lys Asp
805 810 815

Ala Leu Leu Lys Tyr Ile Tyr Asp Asn Arg Gly Thr Leu Ile Gly Gln
820 825 830

Val Asp Arg Leu Lys Asp Lys Val Asn Asn Thr Leu Ser Thr Asp Ile
835 840 845

Pro Phe Gln Leu Ser Lys Tyr Val Asp Asn Gln Arg Leu Leu Ser Thr
850 855 860

Leu Glu Ile Tyr Ala Leu Val Gly Ser Trp Phe Leu Ser Pro Glu His
865 870 875 880

Gln Arg Val Gln Gln Arg Lys Glu Ser Lys Lys Pro Pro Ala Lys Leu
885 890 895

Gln Pro Arg

<210> 40
<211> 901
<212> PRT
<213> Artificial Sequence

<220>
<221> source
<223> /note="Description of Artificial Sequence: Synthetic polypeptide"

<400> 40
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

```
Phe Ser Ser Asp Thr Asn Pro Ser Leu Ser Lys Pro Pro Arg Pro Thr
    50                  55                  60

Ser Lys Tyr Gln Ser Tyr Tyr Asp Pro Ser Tyr Leu Ser Thr Asp Glu
65                  70                  75                  80

Gln Lys Asp Thr Phe Leu Lys Gly Ile Ile Lys Leu Phe Lys Arg Ile
                85                  90                  95

Asn Glu Arg Asp Ile Gly Lys Lys Leu Ile Asn Tyr Leu Val Val Gly
            100                 105                 110

Ser Pro Phe Met Gly Asp Ser Ser Thr Pro Glu Asp Thr Phe Asp Phe
        115                 120                 125

Thr Arg His Thr Thr Asn Ile Ala Val Glu Lys Phe Glu Asn Gly Ser
    130                 135                 140

Trp Lys Val Thr Asn Ile Ile Thr Pro Ser Val Leu Ile Phe Gly Pro
145                 150                 155                 160

Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
225                 230                 235                 240

Asn Ile Pro Ser Asp Lys Arg Ile Arg Pro Gln Val Ser Glu Gly Phe
                245                 250                 255

Phe Ser Gln Asp Gly Pro Asn Val Gln Phe Glu Glu Leu Tyr Thr Phe
            260                 265                 270

Gly Gly Leu Asp Val Glu Ile Ile Pro Gln Ile Glu Arg Ser Gln Leu
        275                 280                 285

Arg Glu Lys Ala Leu Gly His Tyr Lys Asp Ile Ala Lys Arg Leu Asn
    290                 295                 300
```

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Lys Ser Glu Glu Lys Leu Tyr Asp Asp Asp
435 440 445

Asp Lys Asp Arg Trp Gly Ser Ser Leu Gln Cys Ile Lys Val Lys Asn
450 455 460

Asn Arg Leu Pro Tyr Val Ala Asp Lys Asp Ser Ile Ser Gln Glu Ile
465 470 475 480

Phe Glu Asn Lys Ile Ile Thr Asp Glu Thr Asn Val Gln Asn Tyr Ser
485 490 495

Asp Lys Phe Ser Leu Asp Glu Ser Ile Leu Asp Gly Gln Val Pro Ile
500 505 510

Asn Pro Glu Ile Val Asp Pro Leu Leu Pro Asn Val Asn Met Glu Pro
515 520 525

Leu Asn Leu Pro Gly Glu Glu Ile Val Phe Tyr Asp Asp Ile Thr Lys
530 535 540

Tyr Val Asp Tyr Leu Asn Ser Tyr Tyr Tyr Leu Glu Ser Gln Lys Leu
545 550 555 560

Ser Asn Asn Val Glu Asn Ile Thr Leu Thr Thr Ser Val Glu Glu Ala
565 570 575

Leu Gly Tyr Ser Asn Lys Ile Tyr Thr Phe Leu Pro Ser Leu Ala Glu
580 585 590

Lys Val Asn Lys Gly Val Gln Ala Gly Leu Phe Leu Asn Trp Ala Asn
595 600 605

Glu Val Val Glu Asp Phe Thr Thr Asn Ile Met Lys Lys Asp Thr Leu
610 615 620

Asp Lys Ile Ser Asp Val Ser Val Ile Ile Pro Tyr Ile Gly Pro Ala
625 630 635 640

Leu Asn Ile Gly Asn Ser Ala Leu Arg Gly Asn Phe Asn Gln Ala Phe
645 650 655

Ala Thr Ala Gly Val Ala Phe Leu Leu Glu Gly Phe Pro Glu Phe Thr
660 665 670

Ile Pro Ala Leu Gly Val Phe Thr Phe Tyr Ser Ser Ile Gln Glu Arg
675 680 685

Glu Lys Ile Ile Lys Thr Ile Glu Asn Cys Leu Glu Gln Arg Val Lys
690 695 700

Arg Trp Lys Asp Ser Tyr Gln Trp Met Val Ser Asn Trp Leu Ser Arg
705 710 715 720

Ile Thr Thr Gln Phe Asn His Ile Asn Tyr Gln Met Tyr Asp Ser Leu
725 730 735

Ser Tyr Gln Ala Asp Ala Ile Lys Ala Lys Ile Asp Leu Glu Tyr Lys
740 745 750

Lys Tyr Ser Gly Ser Asp Lys Glu Asn Ile Lys Ser Gln Val Glu Asn
755 760 765

Leu Lys Asn Ser Leu Asp Val Lys Ile Ser Glu Ala Met Asn Asn Ile
770 775 780

Asn Lys Phe Ile Arg Glu Cys Ser Val Thr Tyr Leu Phe Lys Asn Met
785 790 795 800

Leu Pro Lys Val Ile Asp Glu Leu Asn Lys Phe Asp Leu Arg Thr Lys

805 810 815

Thr Glu Leu Ile Asn Leu Ile Asp Ser His Asn Ile Ile Leu Val Gly
820 825 830

Glu Val Asp Arg Leu Lys Ala Lys Val Asn Glu Ser Phe Glu Asn Thr
835 840 845

Met Pro Phe Asn Ile Phe Ser Tyr Thr Asn Asn Ser Leu Leu Lys Asp
850 855 860

Ile Ile Asn Glu Tyr Phe Asn Leu Glu Gly Gly Gly Gly Ser Gly Gly
865 870 875 880

Gly Gly Ser Gly Gly Gly Gly Ser Ala Leu Val Gly Asn His Trp Ala
885 890 895

Val Gly His Leu Met
900

<210> 41
<211> 899
<212> PRT
<213> Artificial Sequence

<220>
<221> source
<223> /note="Description of Artificial Sequence: Synthetic polypeptide"

<400> 41
Pro Val Thr Ile Asn Asn Phe Asn Tyr Asn Asp Pro Ile Asp Asn Asn
1 5 10 15

Asn Ile Ile Met Met Glu Pro Pro Phe Ala Arg Gly Thr Gly Arg Tyr
20 25 30

Tyr Lys Ala Phe Lys Ile Thr Asp Arg Ile Trp Ile Ile Pro Glu Arg
35 40 45

Tyr Thr Phe Gly Tyr Lys Pro Glu Asp Phe Asn Lys Ser Ser Gly Ile
50 55 60

Phe Asn Arg Asp Val Cys Glu Tyr Tyr Asp Pro Asp Tyr Leu Asn Thr
65 70 75 80

Asn Asp Lys Lys Asn Ile Phe Leu Gln Thr Met Ile Lys Leu Phe Asn
85 90 95

Arg Ile Lys Ser Lys Pro Leu Gly Glu Lys Leu Leu Glu Met Ile Ile

100 105 110

Asn Gly Ile Pro Tyr Leu Gly Asp Arg Arg Val Pro Leu Glu Glu Phe
115 120 125

Asn Thr Asn Ile Ala Ser Val Thr Val Asn Lys Leu Ile Ser Asn Pro
130 135 140

Gly Glu Val Glu Arg Lys Lys Gly Ile Phe Ala Asn Leu Ile Ile Phe
145 150 155 160

Gly Pro Gly Pro Val Leu Asn Glu Asn Glu Thr Ile Asp Ile Gly Ile
165 170 175

Gln Asn His Phe Ala Ser Arg Glu Gly Phe Gly Gly Ile Met Gln Met
180 185 190

Lys Phe Cys Pro Glu Tyr Val Ser Val Phe Asn Asn Val Gln Glu Asn
195 200 205

Lys Gly Ala Ser Ile Phe Asn Arg Arg Gly Tyr Phe Ser Asp Pro Ala
210 215 220

Leu Ile Leu Met His Glu Leu Ile His Val Leu His Gly Leu Tyr Gly
225 230 235 240

Ile Lys Val Asp Asp Leu Pro Ile Val Pro Asn Glu Lys Lys Phe Phe
245 250 255

Met Gln Ser Thr Asp Ala Ile Gln Ala Glu Glu Leu Tyr Thr Phe Gly
260 265 270

Gly Gln Asp Pro Ser Ile Ile Thr Pro Ser Thr Asp Lys Ser Ile Tyr
275 280 285

Asp Lys Val Leu Gln Asn Phe Arg Gly Ile Val Asp Arg Leu Asn Lys
290 295 300

Val Leu Val Cys Ile Ser Asp Pro Asn Ile Asn Ile Asn Ile Tyr Lys
305 310 315 320

Asn Lys Phe Lys Asp Lys Tyr Lys Phe Val Glu Asp Ser Glu Gly Lys
325 330 335

Tyr Ser Ile Asp Val Glu Ser Phe Asp Lys Leu Tyr Lys Ser Leu Met
340 345 350

Phe Gly Phe Thr Glu Thr Asn Ile Ala Glu Asn Tyr Lys Ile Lys Thr
355 360 365

Arg Ala Ser Tyr Phe Ser Asp Ser Leu Pro Pro Val Lys Ile Lys Asn
370 375 380

Leu Leu Asp Asn Glu Ile Tyr Thr Ile Glu Glu Gly Phe Asn Ile Ser
385 390 395 400

Asp Lys Asp Met Glu Lys Glu Tyr Arg Gly Gln Asn Lys Ala Ile Asn
405 410 415

Lys Gln Ala Tyr Glu Glu Ile Ser Lys Glu His Leu Ala Val Tyr Lys
420 425 430

Ile Gln Met Cys Val Asp Glu Glu Lys Leu Tyr Asp Asp Asp Asp Lys
435 440 445

Asp Arg Trp Gly Ser Ser Leu Gln Cys Ile Asp Val Asp Asn Glu Asp
450 455 460

Leu Phe Phe Ile Ala Asp Lys Asn Ser Phe Ser Asp Asp Leu Ser Lys
465 470 475 480

Asn Glu Arg Ile Glu Tyr Asn Thr Gln Ser Asn Tyr Ile Glu Asn Asp
485 490 495

Phe Pro Ile Asn Glu Leu Ile Leu Asp Thr Asp Leu Ile Ser Lys Ile
500 505 510

Glu Leu Pro Ser Glu Asn Thr Glu Ser Leu Thr Asp Phe Asn Val Asp
515 520 525

Val Pro Val Tyr Glu Lys Gln Pro Ala Ile Lys Lys Ile Phe Thr Asp
530 535 540

Glu Asn Thr Ile Phe Gln Tyr Leu Tyr Ser Gln Thr Phe Pro Leu Asp
545 550 555 560

Ile Arg Asp Ile Ser Leu Thr Ser Ser Phe Asp Asp Ala Leu Leu Phe
565 570 575

Ser Asn Lys Val Tyr Ser Phe Phe Ser Met Asp Tyr Ile Lys Thr Ala
580 585 590

Asn Lys Val Val Glu Ala Gly Leu Phe Ala Gly Trp Val Lys Gln Ile
595 600 605

Val Asn Asp Phe Val Ile Glu Ala Asn Lys Ser Asn Thr Met Asp Ala
610 615 620

Ile Ala Asp Ile Ser Leu Ile Val Pro Tyr Ile Gly Leu Ala Leu Asn
625 630 635 640

Val Gly Asn Glu Thr Ala Lys Gly Asn Phe Glu Asn Ala Phe Glu Ile
645 650 655

Ala Gly Ala Ser Ile Leu Leu Glu Phe Ile Pro Glu Leu Leu Ile Pro
660 665 670

Val Val Gly Ala Phe Leu Leu Glu Ser Tyr Ile Asp Asn Lys Asn Lys
675 680 685

Ile Ile Lys Thr Ile Asp Asn Ala Leu Thr Lys Arg Asn Glu Lys Trp
690 695 700

Ser Asp Met Tyr Gly Leu Ile Val Ala Gln Trp Leu Ser Thr Val Asn
705 710 715 720

Thr Gln Phe Tyr Thr Ile Lys Glu Gly Met Tyr Lys Ala Leu Asn Tyr
725 730 735

Gln Ala Gln Ala Leu Glu Glu Ile Ile Lys Tyr Arg Tyr Asn Ile Tyr
740 745 750

Ser Glu Lys Glu Lys Ser Asn Ile Asn Ile Asp Phe Asn Asp Ile Asn
755 760 765

Ser Lys Leu Asn Glu Gly Ile Asn Gln Ala Ile Asp Asn Ile Asn Asn
770 775 780

Phe Ile Asn Gly Cys Ser Val Ser Tyr Leu Met Lys Lys Met Ile Pro
785 790 795 800

Leu Ala Val Glu Lys Leu Leu Asp Phe Asp Asn Thr Leu Lys Lys Asn
805 810 815

Leu Leu Asn Tyr Ile Asp Glu Asn Lys Leu Tyr Leu Ile Gly Ser Ala
820 825 830

Glu Tyr Glu Lys Ser Lys Val Asn Lys Tyr Leu Lys Thr Ile Met Pro
835 840 845

Phe Asp Leu Ser Ile Tyr Thr Asn Asp Thr Ile Leu Ile Glu Met Phe
850 855 860

Asn Lys Tyr Asn Ser Leu Glu Gly Gly Gly Gly Ser Gly Gly Gly Gly
865 870 875 880

Ser Gly Gly Gly Gly Ser Ala Leu Val Gly Asn His Trp Ala Val Gly
885 890 895

His Leu Met

<210> 42
<211> 909
<212> PRT
<213> Artificial Sequence

<220>
<221> source
<223> /note="Description of Artificial Sequence: Synthetic polypeptide"

<400> 42
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg

405 410 415

Asn Pro Ala Leu Gln Lys Leu Ser Ser Glu Ser Val Val Asp Leu Phe
420 425 430

Thr Lys Val Cys Val Asp Asn Asn Asn Asn Asn Asn Asn Asn Asn Asn
435 440 445

Asp Asp Asp Asp Lys His Val Asp Ala Ile Phe Thr Gln Ser Tyr Arg
450 455 460

Lys Val Leu Ala Gln Leu Ser Ala Arg Lys Leu Leu Gln Asp Ile Leu
465 470 475 480

Asn Arg Ala Glu Ala Ala Ala Lys Glu Ala Ala Ala Lys Ala Leu Gln
485 490 495

Cys Ile Lys Val Lys Asn Asn Arg Leu Pro Tyr Val Ala Asp Lys Asp
500 505 510

Ser Ile Ser Gln Glu Ile Phe Glu Asn Lys Ile Ile Thr Asp Glu Thr
515 520 525

Asn Val Gln Asn Tyr Ser Asp Lys Phe Ser Leu Asp Glu Ser Ile Leu
530 535 540

Asp Gly Gln Val Pro Ile Asn Pro Glu Ile Val Asp Pro Leu Leu Pro
545 550 555 560

Asn Val Asn Met Glu Pro Leu Asn Leu Pro Gly Glu Glu Ile Val Phe
565 570 575

Tyr Asp Asp Ile Thr Lys Tyr Val Asp Tyr Leu Asn Ser Tyr Tyr Tyr
580 585 590

Leu Glu Ser Gln Lys Leu Ser Asn Asn Val Glu Asn Ile Thr Leu Thr
595 600 605

Thr Ser Val Glu Glu Ala Leu Gly Tyr Ser Asn Lys Ile Tyr Thr Phe
610 615 620

Leu Pro Ser Leu Ala Glu Lys Val Asn Lys Gly Val Gln Ala Gly Leu
625 630 635 640

Phe Leu Asn Trp Ala Asn Glu Val Val Glu Asp Phe Thr Thr Asn Ile
645 650 655

Met Lys Lys Asp Thr Leu Asp Lys Ile Ser Asp Val Ser Val Ile Ile
660 665 670

Pro Tyr Ile Gly Pro Ala Leu Asn Ile Gly Asn Ser Ala Leu Arg Gly
675 680 685

Asn Phe Asn Gln Ala Phe Ala Thr Ala Gly Val Ala Phe Leu Leu Glu
690 695 700

Gly Phe Pro Glu Phe Thr Ile Pro Ala Leu Gly Val Phe Thr Phe Tyr
705 710 715 720

Ser Ser Ile Gln Glu Arg Glu Lys Ile Ile Lys Thr Ile Glu Asn Cys
725 730 735

Leu Glu Gln Arg Val Lys Arg Trp Lys Asp Ser Tyr Gln Trp Met Val
740 745 750

Ser Asn Trp Leu Ser Arg Ile Thr Thr Gln Phe Asn His Ile Asn Tyr
755 760 765

Gln Met Tyr Asp Ser Leu Ser Tyr Gln Ala Asp Ala Ile Lys Ala Lys
770 775 780

Ile Asp Leu Glu Tyr Lys Lys Tyr Ser Gly Ser Asp Lys Glu Asn Ile
785 790 795 800

Lys Ser Gln Val Glu Asn Leu Lys Asn Ser Leu Asp Val Lys Ile Ser
805 810 815

Glu Ala Met Asn Asn Ile Asn Lys Phe Ile Arg Glu Cys Ser Val Thr
820 825 830

Tyr Leu Phe Lys Asn Met Leu Pro Lys Val Ile Asp Glu Leu Asn Lys
835 840 845

Phe Asp Leu Arg Thr Lys Thr Glu Leu Ile Asn Leu Ile Asp Ser His
850 855 860

Asn Ile Ile Leu Val Gly Glu Val Asp Arg Leu Lys Ala Lys Val Asn
865 870 875 880

Glu Ser Phe Glu Asn Thr Met Pro Phe Asn Ile Phe Ser Tyr Thr Asn
885 890 895

Asn Ser Leu Leu Lys Asp Ile Ile Asn Glu Tyr Phe Asn
900 905

<210> 43
<211> 919
<212> PRT
<213> Artificial Sequence

<220>
<221> source
<223> /note="Description of Artificial Sequence: Synthetic polypeptide"

<400> 43
Glu Phe Val Asn Lys Gln Phe Asn Tyr Lys Asp Pro Val Asn Gly Val
1               5                   10                  15

Asp Ile Ala Tyr Ile Lys Ile Pro Asn Ala Gly Gln Met Gln Pro Val
            20                  25                  30

Lys Ala Phe Lys Ile His Asn Lys Ile Trp Val Ile Pro Glu Arg Asp
        35                  40                  45

Thr Phe Thr Asn Pro Glu Glu Gly Asp Leu Asn Pro Pro Pro Glu Ala
    50                  55                  60

Lys Gln Val Pro Val Ser Tyr Tyr Asp Ser Thr Tyr Leu Ser Thr Asp
65                  70                  75                  80

Asn Glu Lys Asp Asn Tyr Leu Lys Gly Val Thr Lys Leu Phe Glu Arg
                85                  90                  95

Ile Tyr Ser Thr Asp Leu Gly Arg Met Leu Leu Thr Ser Ile Val Arg
            100                 105                 110

Gly Ile Pro Phe Trp Gly Gly Ser Thr Ile Asp Thr Glu Leu Lys Val
        115                 120                 125

Ile Asp Thr Asn Cys Ile Asn Val Ile Gln Pro Asp Gly Ser Tyr Arg
    130                 135                 140

Ser Glu Glu Leu Asn Leu Val Ile Ile Gly Pro Ser Ala Asp Ile Ile
145                 150                 155                 160

Gln Phe Glu Cys Lys Ser Phe Gly His Glu Val Leu Asn Leu Thr Arg
                165                 170                 175

Asn Gly Tyr Gly Ser Thr Gln Tyr Ile Arg Phe Ser Pro Asp Phe Thr
            180                 185                 190

Phe Gly Phe Glu Glu Ser Leu Glu Val Asp Thr Asn Pro Leu Leu Gly
        195                 200                 205

```
Ala Gly Lys Phe Ala Thr Asp Pro Ala Val Thr Leu Ala His Glu Leu
    210                 215                 220

Ile His Ala Gly His Arg Leu Tyr Gly Ile Ala Ile Asn Pro Asn Arg
225                 230                 235                 240

Val Phe Lys Val Asn Thr Asn Ala Tyr Tyr Glu Met Ser Gly Leu Glu
                245                 250                 255

Val Ser Phe Glu Glu Leu Arg Thr Phe Gly Gly His Asp Ala Lys Phe
            260                 265                 270

Ile Asp Ser Leu Gln Glu Asn Glu Phe Arg Leu Tyr Tyr Tyr Asn Lys
        275                 280                 285

Phe Lys Asp Ile Ala Ser Thr Leu Asn Lys Ala Lys Ser Ile Val Gly
    290                 295                 300

Thr Thr Ala Ser Leu Gln Tyr Met Lys Asn Val Phe Lys Glu Lys Tyr
305                 310                 315                 320

Leu Leu Ser Glu Asp Thr Ser Gly Lys Phe Ser Val Asp Lys Leu Lys
                325                 330                 335

Phe Asp Lys Leu Tyr Lys Met Leu Thr Glu Ile Tyr Thr Glu Asp Asn
            340                 345                 350

Phe Val Lys Phe Phe Lys Val Leu Asn Arg Lys Thr Tyr Leu Asn Phe
        355                 360                 365

Asp Lys Ala Val Phe Lys Ile Asn Ile Val Pro Lys Val Asn Tyr Thr
    370                 375                 380

Ile Tyr Asp Gly Phe Asn Leu Arg Asn Thr Asn Leu Ala Ala Asn Phe
385                 390                 395                 400

Asn Gly Gln Asn Thr Glu Ile Asn Asn Met Asn Phe Thr Lys Leu Lys
                405                 410                 415

Asn Phe Thr Gly Leu Phe Glu Phe Tyr Lys Leu Leu Cys Val Asp Gly
            420                 425                 430

Ile Ile Thr Ser Lys Thr Lys Ser Leu Ile Glu Gly Arg His Val Asp
        435                 440                 445

Ala Ile Phe Thr Gln Ser Tyr Arg Lys Val Leu Ala Gln Leu Ser Ala
    450                 455                 460
```

```
Arg Lys Leu Leu Gln Asp Ile Leu Asn Arg Gln Gln Gly Glu Arg Asn
465                 470                 475                 480

Gln Glu Gln Gly Ala Leu Ala Gly Gly Gly Gly Ser Gly Gly Gly Gly
                485                 490                 495

Ser Gly Gly Gly Gly Ser Ala Leu Val Leu Gln Cys Ile Lys Val Asn
            500                 505                 510

Asn Trp Asp Leu Phe Phe Ser Pro Ser Glu Asp Asn Phe Thr Asn Asp
        515                 520                 525

Leu Asn Lys Gly Glu Glu Ile Thr Ser Asp Thr Asn Ile Glu Ala Ala
    530                 535                 540

Glu Glu Asn Ile Ser Leu Asp Leu Ile Gln Gln Tyr Tyr Leu Thr Phe
545                 550                 555                 560

Asn Phe Asp Asn Glu Pro Glu Asn Ile Ser Ile Glu Asn Leu Ser Ser
                565                 570                 575

Asp Ile Ile Gly Gln Leu Glu Leu Met Pro Asn Ile Glu Arg Phe Pro
            580                 585                 590

Asn Gly Lys Lys Tyr Glu Leu Asp Lys Tyr Thr Met Phe His Tyr Leu
        595                 600                 605

Arg Ala Gln Glu Phe Glu His Gly Lys Ser Arg Ile Ala Leu Thr Asn
    610                 615                 620

Ser Val Asn Glu Ala Leu Leu Asn Pro Ser Arg Val Tyr Thr Phe Phe
625                 630                 635                 640

Ser Ser Asp Tyr Val Lys Lys Val Asn Lys Ala Thr Glu Ala Ala Met
                645                 650                 655

Phe Leu Gly Trp Val Glu Gln Leu Val Tyr Asp Phe Thr Asp Glu Thr
            660                 665                 670

Ser Glu Val Ser Thr Thr Asp Lys Ile Ala Asp Ile Thr Ile Ile Ile
        675                 680                 685

Pro Tyr Ile Gly Pro Ala Leu Asn Ile Gly Asn Met Leu Tyr Lys Asp
    690                 695                 700

Asp Phe Val Gly Ala Leu Ile Phe Ser Gly Ala Val Ile Leu Leu Glu
```

```
705                 710                 715                 720

Phe Ile Pro Glu Ile Ala Ile Pro Val Leu Gly Thr Phe Ala Leu Val
                725                 730                 735

Ser Tyr Ile Ala Asn Lys Val Leu Thr Val Gln Thr Ile Asp Asn Ala
            740                 745                 750

Leu Ser Lys Arg Asn Glu Lys Trp Asp Glu Val Tyr Lys Tyr Ile Val
        755                 760                 765

Thr Asn Trp Leu Ala Lys Val Asn Thr Gln Ile Asp Leu Ile Arg Lys
    770                 775                 780

Lys Met Lys Glu Ala Leu Glu Asn Gln Ala Glu Ala Thr Lys Ala Ile
785                 790                 795                 800

Ile Asn Tyr Gln Tyr Asn Gln Tyr Thr Glu Glu Glu Lys Asn Asn Ile
                805                 810                 815

Asn Phe Asn Ile Asp Asp Leu Ser Ser Lys Leu Asn Glu Ser Ile Asn
            820                 825                 830

Lys Ala Met Ile Asn Ile Asn Lys Phe Leu Asn Gln Cys Ser Val Ser
        835                 840                 845

Tyr Leu Met Asn Ser Met Ile Pro Tyr Gly Val Lys Arg Leu Glu Asp
    850                 855                 860

Phe Asp Ala Ser Leu Lys Asp Ala Leu Leu Lys Tyr Ile Tyr Asp Asn
865                 870                 875                 880

Arg Gly Thr Leu Ile Gly Gln Val Asp Arg Leu Lys Asp Lys Val Asn
                885                 890                 895

Asn Thr Leu Ser Thr Asp Ile Pro Phe Gln Leu Ser Lys Tyr Val Asp
            900                 905                 910

Asn Gln Arg Leu Leu Ser Thr
        915
```

<210> 44
<211> 919
<212> PRT
<213> Artificial Sequence

<220>
<221> source
<223> /note="Description of Artificial Sequence: Synthetic

polypeptide"

<400> 44

```
Pro Ile Thr Ile Asn Asn Phe Asn Tyr Ser Asp Pro Val Asp Asn Lys
1               5                   10                  15

Asn Ile Leu Tyr Leu Asp Thr His Leu Asn Thr Leu Ala Asn Glu Pro
            20                  25                  30

Glu Lys Ala Phe Arg Ile Thr Gly Asn Ile Trp Val Ile Pro Asp Arg
        35                  40                  45

Phe Ser Arg Asn Ser Asn Pro Asn Leu Asn Lys Pro Pro Arg Val Thr
    50                  55                  60

Ser Pro Lys Ser Gly Tyr Tyr Asp Pro Asn Tyr Leu Ser Thr Asp Ser
65                  70                  75                  80

Asp Lys Asp Thr Phe Leu Lys Glu Ile Ile Lys Leu Phe Lys Arg Ile
                85                  90                  95

Asn Ser Arg Glu Ile Gly Glu Glu Leu Ile Tyr Arg Leu Ser Thr Asp
            100                 105                 110

Ile Pro Phe Pro Gly Asn Asn Asn Thr Pro Ile Asn Thr Phe Asp Phe
        115                 120                 125

Asp Val Asp Phe Asn Ser Val Asp Val Lys Thr Arg Gln Gly Asn Asn
    130                 135                 140

Trp Val Lys Thr Gly Ser Ile Asn Pro Ser Val Ile Ile Thr Gly Pro
145                 150                 155                 160

Arg Glu Asn Ile Ile Asp Pro Glu Thr Ser Thr Phe Lys Leu Thr Asn
                165                 170                 175

Asn Thr Phe Ala Ala Gln Glu Gly Phe Gly Ala Leu Ser Ile Ile Ser
            180                 185                 190

Ile Ser Pro Arg Phe Met Leu Thr Tyr Ser Asn Ala Thr Asn Asp Val
        195                 200                 205

Gly Glu Gly Arg Phe Ser Lys Ser Glu Phe Cys Met Asp Pro Ile Leu
    210                 215                 220

Ile Leu Met His Glu Leu Asn His Ala Met His Asn Leu Tyr Gly Ile
225                 230                 235                 240
```

Ala Ile Pro Asn Asp Gln Thr Ile Ser Ser Val Thr Ser Asn Ile Phe
245 250 255

Tyr Ser Gln Tyr Asn Val Lys Leu Glu Tyr Ala Glu Ile Tyr Ala Phe
260 265 270

Gly Gly Pro Thr Ile Asp Leu Ile Pro Lys Ser Ala Arg Lys Tyr Phe
275 280 285

Glu Glu Lys Ala Leu Asp Tyr Tyr Arg Ser Ile Ala Lys Arg Leu Asn
290 295 300

Ser Ile Thr Thr Ala Asn Pro Ser Ser Phe Asn Lys Tyr Ile Gly Glu
305 310 315 320

Tyr Lys Gln Lys Leu Ile Arg Lys Tyr Arg Phe Val Val Glu Ser Ser
325 330 335

Gly Glu Val Thr Val Asn Arg Asn Lys Phe Val Glu Leu Tyr Asn Glu
340 345 350

Leu Thr Gln Ile Phe Thr Glu Phe Asn Tyr Ala Lys Ile Tyr Asn Val
355 360 365

Gln Asn Arg Lys Ile Tyr Leu Ser Asn Val Tyr Thr Pro Val Thr Ala
370 375 380

Asn Ile Leu Asp Asp Asn Val Tyr Asp Ile Gln Asn Gly Phe Asn Ile
385 390 395 400

Pro Lys Ser Asn Leu Asn Val Leu Phe Met Gly Gln Asn Leu Ser Arg
405 410 415

Asn Pro Ala Leu Arg Lys Val Asn Pro Glu Asn Met Leu Tyr Leu Phe
420 425 430

Thr Lys Phe Cys Val Asp Ala Ile Asp Gly Arg His Val Asp Ala Ile
435 440 445

Phe Thr Gln Ser Tyr Arg Lys Val Leu Ala Gln Leu Ser Ala Arg Lys
450 455 460

Leu Leu Gln Asp Ile Leu Asn Arg Gln Gln Gly Glu Arg Asn Gln Glu
465 470 475 480

Gln Gly Ala Leu Ala Gly Gly Gly Gly Ser Gly Gly Gly Gly Ser Gly
485 490 495

Gly Gly Gly Ser Ala Leu Val Leu Gln Cys Arg Glu Leu Leu Val Lys
500 505 510

Asn Thr Asp Leu Pro Phe Ile Gly Asp Ile Ser Asp Val Lys Thr Asp
515 520 525

Ile Phe Leu Arg Lys Asp Ile Asn Glu Glu Thr Glu Val Ile Tyr Tyr
530 535 540

Pro Asp Asn Val Ser Val Asp Gln Val Ile Leu Ser Lys Asn Thr Ser
545 550 555 560

Glu His Gly Gln Leu Asp Leu Leu Tyr Pro Ser Ile Asp Ser Glu Ser
565 570 575

Glu Ile Leu Pro Gly Glu Asn Gln Val Phe Tyr Asp Asn Arg Thr Gln
580 585 590

Asn Val Asp Tyr Leu Asn Ser Tyr Tyr Tyr Leu Glu Ser Gln Lys Leu
595 600 605

Ser Asp Asn Val Glu Asp Phe Thr Phe Thr Arg Ser Ile Glu Glu Ala
610 615 620

Leu Asp Asn Ser Ala Lys Val Tyr Thr Tyr Phe Pro Thr Leu Ala Asn
625 630 635 640

Lys Val Asn Ala Gly Val Gln Gly Gly Leu Phe Leu Met Trp Ala Asn
645 650 655

Asp Val Val Glu Asp Phe Thr Thr Asn Ile Leu Arg Lys Asp Thr Leu
660 665 670

Asp Lys Ile Ser Asp Val Ser Ala Ile Ile Pro Tyr Ile Gly Pro Ala
675 680 685

Leu Asn Ile Ser Asn Ser Val Arg Arg Gly Asn Phe Thr Glu Ala Phe
690 695 700

Ala Val Thr Gly Val Thr Ile Leu Leu Glu Ala Phe Pro Glu Phe Thr
705 710 715 720

Ile Pro Ala Leu Gly Ala Phe Val Ile Tyr Ser Lys Val Gln Glu Arg
725 730 735

Asn Glu Ile Ile Lys Thr Ile Asp Asn Cys Leu Glu Gln Arg Ile Lys
740 745 750

Arg Trp Lys Asp Ser Tyr Glu Trp Met Met Gly Thr Trp Leu Ser Arg
755 760 765

Ile Ile Thr Gln Phe Asn Asn Ile Ser Tyr Gln Met Tyr Asp Ser Leu
770 775 780

Asn Tyr Gln Ala Gly Ala Ile Lys Ala Lys Ile Asp Leu Glu Tyr Lys
785 790 795 800

Lys Tyr Ser Gly Ser Asp Lys Glu Asn Ile Lys Ser Gln Val Glu Asn
805 810 815

Leu Lys Asn Ser Leu Asp Val Lys Ile Ser Glu Ala Met Asn Asn Ile
820 825 830

Asn Lys Phe Ile Arg Glu Cys Ser Val Thr Tyr Leu Phe Lys Asn Met
835 840 845

Leu Pro Lys Val Ile Asp Glu Leu Asn Glu Phe Asp Arg Asn Thr Lys
850 855 860

Ala Lys Leu Ile Asn Leu Ile Asp Ser His Asn Ile Ile Leu Val Gly
865 870 875 880

Glu Val Asp Lys Leu Lys Ala Lys Val Asn Asn Ser Phe Gln Asn Thr
885 890 895

Ile Pro Phe Asn Ile Phe Ser Tyr Thr Asn Asn Ser Leu Leu Lys Asp
900 905 910

Ile Ile Asn Glu Tyr Phe Asn
915

<210> 45
<211> 928
<212> PRT
<213> Artificial Sequence

<220>
<221> source
<223> /note="Description of Artificial Sequence: Synthetic polypeptide"

<400> 45
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu

275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys His Val Asp Ala Ile Phe Thr Gln Ser Tyr Arg Lys
450 455 460

Val Leu Ala Gln Leu Ser Ala Arg Lys Leu Leu Gln Asp Ile Leu Asn
465 470 475 480

Arg Gln Gln Gly Glu Arg Asn Gln Glu Gln Gly Ala Ala Leu Ala Gly
485 490 495

Gly Gly Gly Ser Gly Gly Gly Gly Ser Gly Gly Gly Gly Ser Ala Leu
500 505 510

Ala Leu Gln Cys Ile Lys Val Lys Asn Asn Arg Leu Pro Tyr Val Ala
515 520 525

Asp Lys Asp Ser Ile Ser Gln Glu Ile Phe Glu Asn Lys Ile Ile Thr
530 535 540

Asp Glu Thr Asn Val Gln Asn Tyr Ser Asp Lys Phe Ser Leu Asp Glu
545 550 555 560

Ser Ile Leu Asp Gly Gln Val Pro Ile Asn Pro Glu Ile Val Asp Pro
565 570 575

Leu Leu Pro Asn Val Asn Met Glu Pro Leu Asn Leu Pro Gly Glu Glu
580 585 590

Ile Val Phe Tyr Asp Asp Ile Thr Lys Tyr Val Asp Tyr Leu Asn Ser
595 600 605

Tyr Tyr Tyr Leu Glu Ser Gln Lys Leu Ser Asn Asn Val Glu Asn Ile
610 615 620

Thr Leu Thr Thr Ser Val Glu Glu Ala Leu Gly Tyr Ser Asn Lys Ile
625 630 635 640

Tyr Thr Phe Leu Pro Ser Leu Ala Glu Lys Val Asn Lys Gly Val Gln
645 650 655

Ala Gly Leu Phe Leu Asn Trp Ala Asn Glu Val Val Glu Asp Phe Thr
660 665 670

Thr Asn Ile Met Lys Lys Asp Thr Leu Asp Lys Ile Ser Asp Val Ser
675 680 685

Val Ile Ile Pro Tyr Ile Gly Pro Ala Leu Asn Ile Gly Asn Ser Ala
690 695 700

Leu Arg Gly Asn Phe Asn Gln Ala Phe Ala Thr Ala Gly Val Ala Phe
705 710 715 720

Leu Leu Glu Gly Phe Pro Glu Phe Thr Ile Pro Ala Leu Gly Val Phe
725 730 735

Thr Phe Tyr Ser Ser Ile Gln Glu Arg Glu Lys Ile Ile Lys Thr Ile
740 745 750

Glu Asn Cys Leu Glu Gln Arg Val Lys Arg Trp Lys Asp Ser Tyr Gln
755 760 765

Trp Met Val Ser Asn Trp Leu Ser Arg Ile Thr Thr Gln Phe Asn His
770 775 780

Ile Asn Tyr Gln Met Tyr Asp Ser Leu Ser Tyr Gln Ala Asp Ala Ile
785 790 795 800

Lys Ala Lys Ile Asp Leu Glu Tyr Lys Lys Tyr Ser Gly Ser Asp Lys
805 810 815

Glu Asn Ile Lys Ser Gln Val Glu Asn Leu Lys Asn Ser Leu Asp Val
820 825 830

Lys Ile Ser Glu Ala Met Asn Asn Ile Asn Lys Phe Ile Arg Glu Cys
835 840 845

Ser Val Thr Tyr Leu Phe Lys Asn Met Leu Pro Lys Val Ile Asp Glu
850 855 860

Leu Asn Lys Phe Asp Leu Arg Thr Lys Thr Glu Leu Ile Asn Leu Ile
865 870 875 880

Asp Ser His Asn Ile Ile Leu Val Gly Glu Val Asp Arg Leu Lys Ala
885 890 895

Lys Val Asn Glu Ser Phe Glu Asn Thr Met Pro Phe Asn Ile Phe Ser
900 905 910

Tyr Thr Asn Asn Ser Leu Leu Lys Asp Ile Ile Asn Glu Tyr Phe Asn
915 920 925

<210> 46
<211> 913
<212> PRT
<213> Artificial Sequence

<220>
<221> source
<223> /note="Description of Artificial Sequence: Synthetic polypeptide"

<400> 46
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65                  70                  75                  80

Gln Lys Asp Thr Phe Leu Lys Gly Ile Ile Lys Leu Phe Lys Arg Ile
                85                  90                  95

Asn Glu Arg Asp Ile Gly Lys Lys Leu Ile Asn Tyr Leu Val Val Gly
            100                 105                 110

Ser Pro Phe Met Gly Asp Ser Ser Thr Pro Glu Asp Thr Phe Asp Phe
        115                 120                 125

Thr Arg His Thr Thr Asn Ile Ala Val Glu Lys Phe Glu Asn Gly Ser
    130                 135                 140

Trp Lys Val Thr Asn Ile Ile Thr Pro Ser Val Leu Ile Phe Gly Pro
145                 150                 155                 160

Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
225                 230                 235                 240

Asn Ile Pro Ser Asp Lys Arg Ile Arg Pro Gln Val Ser Glu Gly Phe
                245                 250                 255

Phe Ser Gln Asp Gly Pro Asn Val Gln Phe Glu Glu Leu Tyr Thr Phe
            260                 265                 270

Gly Gly Leu Asp Val Glu Ile Ile Pro Gln Ile Glu Arg Ser Gln Leu
        275                 280                 285

Arg Glu Lys Ala Leu Gly His Tyr Lys Asp Ile Ala Lys Arg Leu Asn
    290                 295                 300

Asn Ile Asn Lys Thr Ile Pro Ser Ser Trp Ile Ser Asn Ile Asp Lys
305                 310                 315                 320

```
Tyr Lys Lys Ile Phe Ser Glu Lys Tyr Asn Phe Asp Lys Asp Asn Thr
                325                 330                 335

Gly Asn Phe Val Val Asn Ile Asp Lys Phe Asn Ser Leu Tyr Ser Asp
            340                 345                 350

Leu Thr Asn Val Met Ser Glu Val Val Tyr Ser Ser Gln Tyr Asn Val
        355                 360                 365

Lys Asn Arg Thr His Tyr Phe Ser Arg His Tyr Leu Pro Val Phe Ala
    370                 375                 380

Asn Ile Leu Asp Asp Asn Ile Tyr Thr Ile Arg Asp Gly Phe Asn Leu
385                 390                 395                 400

Thr Asn Lys Gly Phe Asn Ile Glu Asn Ser Gly Gln Asn Ile Glu Arg
                405                 410                 415

Asn Pro Ala Leu Gln Lys Leu Ser Ser Glu Ser Val Val Asp Leu Phe
            420                 425                 430

Thr Lys Val Cys Val Asp Asn Asn Asn Asn Asn Asn Asn Asn Asn Asn
        435                 440                 445

Asp Asp Asp Asp Lys His Val Asp Ala Ile Phe Thr Gln Ser Tyr Arg
    450                 455                 460

Lys Val Leu Ala Gln Leu Ser Ala Arg Lys Leu Leu Gln Asp Ile Leu
465                 470                 475                 480

Asn Arg Gln Gln Gly Glu Arg Asn Gln Glu Gln Gly Ala Pro Ala Pro
                485                 490                 495

Ala Pro Leu Gln Cys Ile Lys Val Lys Asn Asn Arg Leu Pro Tyr Val
            500                 505                 510

Ala Asp Lys Asp Ser Ile Ser Gln Glu Ile Phe Glu Asn Lys Ile Ile
        515                 520                 525

Thr Asp Glu Thr Asn Val Gln Asn Tyr Ser Asp Lys Phe Ser Leu Asp
    530                 535                 540

Glu Ser Ile Leu Asp Gly Gln Val Pro Ile Asn Pro Glu Ile Val Asp
545                 550                 555                 560

Pro Leu Leu Pro Asn Val Asn Met Glu Pro Leu Asn Leu Pro Gly Glu
```

565 570 575

Glu Ile Val Phe Tyr Asp Asp Ile Thr Lys Tyr Val Asp Tyr Leu Asn
580 585 590

Ser Tyr Tyr Tyr Leu Glu Ser Gln Lys Leu Ser Asn Asn Val Glu Asn
595 600 605

Ile Thr Leu Thr Thr Ser Val Glu Glu Ala Leu Gly Tyr Ser Asn Lys
610 615 620

Ile Tyr Thr Phe Leu Pro Ser Leu Ala Glu Lys Val Asn Lys Gly Val
625 630 635 640

Gln Ala Gly Leu Phe Leu Asn Trp Ala Asn Glu Val Val Glu Asp Phe
645 650 655

Thr Thr Asn Ile Met Lys Lys Asp Thr Leu Asp Lys Ile Ser Asp Val
660 665 670

Ser Val Ile Ile Pro Tyr Ile Gly Pro Ala Leu Asn Ile Gly Asn Ser
675 680 685

Ala Leu Arg Gly Asn Phe Asn Gln Ala Phe Ala Thr Ala Gly Val Ala
690 695 700

Phe Leu Leu Glu Gly Phe Pro Glu Phe Thr Ile Pro Ala Leu Gly Val
705 710 715 720

Phe Thr Phe Tyr Ser Ser Ile Gln Glu Arg Glu Lys Ile Ile Lys Thr
725 730 735

Ile Glu Asn Cys Leu Glu Gln Arg Val Lys Arg Trp Lys Asp Ser Tyr
740 745 750

Gln Trp Met Val Ser Asn Trp Leu Ser Arg Ile Thr Thr Gln Phe Asn
755 760 765

His Ile Asn Tyr Gln Met Tyr Asp Ser Leu Ser Tyr Gln Ala Asp Ala
770 775 780

Ile Lys Ala Lys Ile Asp Leu Glu Tyr Lys Lys Tyr Ser Gly Ser Asp
785 790 795 800

Lys Glu Asn Ile Lys Ser Gln Val Glu Asn Leu Lys Asn Ser Leu Asp
805 810 815

Val Lys Ile Ser Glu Ala Met Asn Asn Ile Asn Lys Phe Ile Arg Glu
820 825 830

Cys Ser Val Thr Tyr Leu Phe Lys Asn Met Leu Pro Lys Val Ile Asp
835 840 845

Glu Leu Asn Lys Phe Asp Leu Arg Thr Lys Thr Glu Leu Ile Asn Leu
850 855 860

Ile Asp Ser His Asn Ile Ile Leu Val Gly Glu Val Asp Arg Leu Lys
865 870 875 880

Ala Lys Val Asn Glu Ser Phe Glu Asn Thr Met Pro Phe Asn Ile Phe
885 890 895

Ser Tyr Thr Asn Asn Ser Leu Leu Lys Asp Ile Ile Asn Glu Tyr Phe
900 905 910

Asn

<210> 47
<211> 919
<212> PRT
<213> Artificial Sequence

<220>
<221> source
<223> /note="Description of Artificial Sequence: Synthetic polypeptide"

<400> 47
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Asn Asn Asn Asn Asn Asn Asn Asn Asn Asn
435 440 445

Asp Asp Asp Asp Lys His Val Asp Ala Ile Phe Thr Gln Ser Tyr Arg
450 455 460

Lys Val Leu Ala Gln Leu Ser Ala Arg Lys Leu Leu Gln Asp Ile Leu
465 470 475 480

Asn Arg Gln Gln Gly Glu Arg Asn Gln Glu Gln Gly Ala Glu Ala Ala
485 490 495

Ala Lys Glu Ala Ala Ala Lys Ala Leu Gln Cys Ile Lys Val Lys Asn
500 505 510

Asn Arg Leu Pro Tyr Val Ala Asp Lys Asp Ser Ile Ser Gln Glu Ile
515 520 525

Phe Glu Asn Lys Ile Ile Thr Asp Glu Thr Asn Val Gln Asn Tyr Ser
530 535 540

Asp Lys Phe Ser Leu Asp Glu Ser Ile Leu Asp Gly Gln Val Pro Ile
545 550 555 560

Asn Pro Glu Ile Val Asp Pro Leu Leu Pro Asn Val Asn Met Glu Pro
565 570 575

Leu Asn Leu Pro Gly Glu Glu Ile Val Phe Tyr Asp Asp Ile Thr Lys
580 585 590

Tyr Val Asp Tyr Leu Asn Ser Tyr Tyr Tyr Leu Glu Ser Gln Lys Leu
595 600 605

Ser Asn Asn Val Glu Asn Ile Thr Leu Thr Thr Ser Val Glu Glu Ala
    610                 615                 620

Leu Gly Tyr Ser Asn Lys Ile Tyr Thr Phe Leu Pro Ser Leu Ala Glu
625                 630                 635                 640

Lys Val Asn Lys Gly Val Gln Ala Gly Leu Phe Leu Asn Trp Ala Asn
                645                 650                 655

Glu Val Val Glu Asp Phe Thr Thr Asn Ile Met Lys Lys Asp Thr Leu
            660                 665                 670

Asp Lys Ile Ser Asp Val Ser Val Ile Ile Pro Tyr Ile Gly Pro Ala
        675                 680                 685

Leu Asn Ile Gly Asn Ser Ala Leu Arg Gly Asn Phe Asn Gln Ala Phe
    690                 695                 700

Ala Thr Ala Gly Val Ala Phe Leu Leu Glu Gly Phe Pro Glu Phe Thr
705                 710                 715                 720

Ile Pro Ala Leu Gly Val Phe Thr Phe Tyr Ser Ser Ile Gln Glu Arg
                725                 730                 735

Glu Lys Ile Ile Lys Thr Ile Glu Asn Cys Leu Glu Gln Arg Val Lys
            740                 745                 750

Arg Trp Lys Asp Ser Tyr Gln Trp Met Val Ser Asn Trp Leu Ser Arg
        755                 760                 765

Ile Thr Thr Gln Phe Asn His Ile Asn Tyr Gln Met Tyr Asp Ser Leu
    770                 775                 780

Ser Tyr Gln Ala Asp Ala Ile Lys Ala Lys Ile Asp Leu Glu Tyr Lys
785                 790                 795                 800

Lys Tyr Ser Gly Ser Asp Lys Glu Asn Ile Lys Ser Gln Val Glu Asn
                805                 810                 815

Leu Lys Asn Ser Leu Asp Val Lys Ile Ser Glu Ala Met Asn Asn Ile
            820                 825                 830

Asn Lys Phe Ile Arg Glu Cys Ser Val Thr Tyr Leu Phe Lys Asn Met
        835                 840                 845

Leu Pro Lys Val Ile Asp Glu Leu Asn Lys Phe Asp Leu Arg Thr Lys

850 855 860

Thr Glu Leu Ile Asn Leu Ile Asp Ser His Asn Ile Ile Leu Val Gly
865 870 875 880

Glu Val Asp Arg Leu Lys Ala Lys Val Asn Glu Ser Phe Glu Asn Thr
885 890 895

Met Pro Phe Asn Ile Phe Ser Tyr Thr Asn Asn Ser Leu Leu Lys Asp
900 905 910

Ile Ile Asn Glu Tyr Phe Asn
915

<210> 48
<211> 918
<212> PRT
<213> Artificial Sequence

<220>
<221> source
<223> /note="Description of Artificial Sequence: Synthetic polypeptide"

<400> 48
Glu Phe Val Asn Lys Gln Phe Asn Tyr Lys Asp Pro Val Asn Gly Val
1 5 10 15

Asp Ile Ala Tyr Ile Lys Ile Pro Asn Ala Gly Gln Met Gln Pro Val
20 25 30

Lys Ala Phe Lys Ile His Asn Lys Ile Trp Val Ile Pro Glu Arg Asp
35 40 45

Thr Phe Thr Asn Pro Glu Glu Gly Asp Leu Asn Pro Pro Pro Glu Ala
50 55 60

Lys Gln Val Pro Val Ser Tyr Tyr Asp Ser Thr Tyr Leu Ser Thr Asp
65 70 75 80

Asn Glu Lys Asp Asn Tyr Leu Lys Gly Val Thr Lys Leu Phe Glu Arg
85 90 95

Ile Tyr Ser Thr Asp Leu Gly Arg Met Leu Leu Thr Ser Ile Val Arg
100 105 110

Gly Ile Pro Phe Trp Gly Gly Ser Thr Ile Asp Thr Glu Leu Lys Val
115 120 125

Ile Asp Thr Asn Cys Ile Asn Val Ile Gln Pro Asp Gly Ser Tyr Arg

130 135 140

Ser Glu Glu Leu Asn Leu Val Ile Ile Gly Pro Ser Ala Asp Ile Ile
145 150 155 160

Gln Phe Glu Cys Lys Ser Phe Gly His Glu Val Leu Asn Leu Thr Arg
165 170 175

Asn Gly Tyr Gly Ser Thr Gln Tyr Ile Arg Phe Ser Pro Asp Phe Thr
180 185 190

Phe Gly Phe Glu Glu Ser Leu Glu Val Asp Thr Asn Pro Leu Leu Gly
195 200 205

Ala Gly Lys Phe Ala Thr Asp Pro Ala Val Thr Leu Ala His Glu Leu
210 215 220

Ile His Ala Gly His Arg Leu Tyr Gly Ile Ala Ile Asn Pro Asn Arg
225 230 235 240

Val Phe Lys Val Asn Thr Asn Ala Tyr Tyr Glu Met Ser Gly Leu Glu
245 250 255

Val Ser Phe Glu Glu Leu Arg Thr Phe Gly Gly His Asp Ala Lys Phe
260 265 270

Ile Asp Ser Leu Gln Glu Asn Glu Phe Arg Leu Tyr Tyr Tyr Asn Lys
275 280 285

Phe Lys Asp Ile Ala Ser Thr Leu Asn Lys Ala Lys Ser Ile Val Gly
290 295 300

Thr Thr Ala Ser Leu Gln Tyr Met Lys Asn Val Phe Lys Glu Lys Tyr
305 310 315 320

Leu Leu Ser Glu Asp Thr Ser Gly Lys Phe Ser Val Asp Lys Leu Lys
325 330 335

Phe Asp Lys Leu Tyr Lys Met Leu Thr Glu Ile Tyr Thr Glu Asp Asn
340 345 350

Phe Val Lys Phe Phe Lys Val Leu Asn Arg Lys Thr Tyr Leu Asn Phe
355 360 365

Asp Lys Ala Val Phe Lys Ile Asn Ile Val Pro Lys Val Asn Tyr Thr
370 375 380

Ile Tyr Asp Gly Phe Asn Leu Arg Asn Thr Asn Leu Ala Ala Asn Phe
385 390 395 400

Asn Gly Gln Asn Thr Glu Ile Asn Asn Met Asn Phe Thr Lys Leu Lys
405 410 415

Asn Phe Thr Gly Leu Phe Glu Phe Tyr Lys Leu Leu Cys Val Asp Gly
420 425 430

Gly Gly Gly Ser Ala Asp Asp Asp Asp Lys Asn Asp Asp Pro Pro Ile
435 440 445

Ser Ile Asp Leu Thr Phe His Leu Leu Arg Asn Met Ile Glu Met Ala
450 455 460

Arg Ile Glu Asn Glu Arg Glu Gln Ala Gly Leu Asn Arg Lys Tyr Leu
465 470 475 480

Asp Glu Val Ala Leu Ala Gly Gly Gly Gly Ser Gly Gly Gly Gly Ser
485 490 495

Gly Gly Gly Gly Ser Ala Leu Val Leu Gln Cys Ile Lys Val Asn Asn
500 505 510

Trp Asp Leu Phe Phe Ser Pro Ser Glu Asp Asn Phe Thr Asn Asp Leu
515 520 525

Asn Lys Gly Glu Glu Ile Thr Ser Asp Thr Asn Ile Glu Ala Ala Glu
530 535 540

Glu Asn Ile Ser Leu Asp Leu Ile Gln Gln Tyr Tyr Leu Thr Phe Asn
545 550 555 560

Phe Asp Asn Glu Pro Glu Asn Ile Ser Ile Glu Asn Leu Ser Ser Asp
565 570 575

Ile Ile Gly Gln Leu Glu Leu Met Pro Asn Ile Glu Arg Phe Pro Asn
580 585 590

Gly Lys Lys Tyr Glu Leu Asp Lys Tyr Thr Met Phe His Tyr Leu Arg
595 600 605

Ala Gln Glu Phe Glu His Gly Lys Ser Arg Ile Ala Leu Thr Asn Ser
610 615 620

Val Asn Glu Ala Leu Leu Asn Pro Ser Arg Val Tyr Thr Phe Phe Ser
625 630 635 640

Ser Asp Tyr Val Lys Lys Val Asn Lys Ala Thr Glu Ala Ala Met Phe
645 650 655

Leu Gly Trp Val Glu Gln Leu Val Tyr Asp Phe Thr Asp Glu Thr Ser
660 665 670

Glu Val Ser Thr Thr Asp Lys Ile Ala Asp Ile Thr Ile Ile Ile Pro
675 680 685

Tyr Ile Gly Pro Ala Leu Asn Ile Gly Asn Met Leu Tyr Lys Asp Asp
690 695 700

Phe Val Gly Ala Leu Ile Phe Ser Gly Ala Val Ile Leu Leu Glu Phe
705 710 715 720

Ile Pro Glu Ile Ala Ile Pro Val Leu Gly Thr Phe Ala Leu Val Ser
725 730 735

Tyr Ile Ala Asn Lys Val Leu Thr Val Gln Thr Ile Asp Asn Ala Leu
740 745 750

Ser Lys Arg Asn Glu Lys Trp Asp Glu Val Tyr Lys Tyr Ile Val Thr
755 760 765

Asn Trp Leu Ala Lys Val Asn Thr Gln Ile Asp Leu Ile Arg Lys Lys
770 775 780

Met Lys Glu Ala Leu Glu Asn Gln Ala Glu Ala Thr Lys Ala Ile Ile
785 790 795 800

Asn Tyr Gln Tyr Asn Gln Tyr Thr Glu Glu Glu Lys Asn Asn Ile Asn
805 810 815

Phe Asn Ile Asp Asp Leu Ser Ser Lys Leu Asn Glu Ser Ile Asn Lys
820 825 830

Ala Met Ile Asn Ile Asn Lys Phe Leu Asn Gln Cys Ser Val Ser Tyr
835 840 845

Leu Met Asn Ser Met Ile Pro Tyr Gly Val Lys Arg Leu Glu Asp Phe
850 855 860

Asp Ala Ser Leu Lys Asp Ala Leu Leu Lys Tyr Ile Tyr Asp Asn Arg
865 870 875 880

Gly Thr Leu Ile Gly Gln Val Asp Arg Leu Lys Asp Lys Val Asn Asn
885 890 895

Thr Leu Ser Thr Asp Ile Pro Phe Gln Leu Ser Lys Tyr Val Asp Asn
900 905 910

Gln Arg Leu Leu Ser Thr
915

<210> 49
<211> 864
<212> PRT
<213> Artificial Sequence

<220>
<221> source
<223> /note="Description of Artificial Sequence: Synthetic polypeptide"

<400> 49
Glu Phe Val Asn Lys Gln Phe Asn Tyr Lys Asp Pro Val Asn Gly Val
1 5 10 15

Asp Ile Ala Tyr Ile Lys Ile Pro Asn Ala Gly Gln Met Gln Pro Val
20 25 30

Lys Ala Phe Lys Ile His Asn Lys Ile Trp Val Ile Pro Glu Arg Asp
35 40 45

Thr Phe Thr Asn Pro Glu Glu Gly Asp Leu Asn Pro Pro Pro Glu Ala
50 55 60

Lys Gln Val Pro Val Ser Tyr Tyr Asp Ser Thr Tyr Leu Ser Thr Asp
65 70 75 80

Asn Glu Lys Asp Asn Tyr Leu Lys Gly Val Thr Lys Leu Phe Glu Arg
85 90 95

Ile Tyr Ser Thr Asp Leu Gly Arg Met Leu Leu Thr Ser Ile Val Arg
100 105 110

Gly Ile Pro Phe Trp Gly Gly Ser Thr Ile Asp Thr Glu Leu Lys Val
115 120 125

Ile Asp Thr Asn Cys Ile Asn Val Ile Gln Pro Asp Gly Ser Tyr Arg
130 135 140

Ser Glu Glu Leu Asn Leu Val Ile Ile Gly Pro Ser Ala Asp Ile Ile
145 150 155 160

Gln Phe Glu Cys Lys Ser Phe Gly His Glu Val Leu Asn Leu Thr Arg
165 170 175

Asn Gly Tyr Gly Ser Thr Gln Tyr Ile Arg Phe Ser Pro Asp Phe Thr
180 185 190

Phe Gly Phe Glu Glu Ser Leu Glu Val Asp Thr Asn Pro Leu Leu Gly
195 200 205

Ala Gly Lys Phe Ala Thr Asp Pro Ala Val Thr Leu Ala His Glu Leu
210 215 220

Ile His Ala Gly His Arg Leu Tyr Gly Ile Ala Ile Asn Pro Asn Arg
225 230 235 240

Val Phe Lys Val Asn Thr Asn Ala Tyr Tyr Glu Met Ser Gly Leu Glu
245 250 255

Val Ser Phe Glu Glu Leu Arg Thr Phe Gly Gly His Asp Ala Lys Phe
260 265 270

Ile Asp Ser Leu Gln Glu Asn Glu Phe Arg Leu Tyr Tyr Tyr Asn Lys
275 280 285

Phe Lys Asp Ile Ala Ser Thr Leu Asn Lys Ala Lys Ser Ile Val Gly
290 295 300

Thr Thr Ala Ser Leu Gln Tyr Met Lys Asn Val Phe Lys Glu Lys Tyr
305 310 315 320

Leu Leu Ser Glu Asp Thr Ser Gly Lys Phe Ser Val Asp Lys Leu Lys
325 330 335

Phe Asp Lys Leu Tyr Lys Met Leu Thr Glu Ile Tyr Thr Glu Asp Asn
340 345 350

Phe Val Lys Phe Phe Lys Val Leu Asn Arg Lys Thr Tyr Leu Asn Phe
355 360 365

Asp Lys Ala Val Phe Lys Ile Asn Ile Val Pro Lys Val Asn Tyr Thr
370 375 380

Ile Tyr Asp Gly Phe Asn Leu Arg Asn Thr Asn Leu Ala Ala Asn Phe
385 390 395 400

Asn Gly Gln Asn Thr Glu Ile Asn Asn Met Asn Phe Thr Lys Leu Lys
405 410 415

Asn Phe Thr Gly Leu Phe Glu Phe Tyr Lys Leu Leu Cys Val Asp Gly

420 425 430

Ile Ile Thr Ser Lys Thr Lys Ser Asp Asp Asp Asp Lys Asn Lys Ala
435 440 445

Leu Asn Leu Gln Cys Ile Lys Val Asn Asn Trp Asp Leu Phe Phe Ser
450 455 460

Pro Ser Glu Asp Asn Phe Thr Asn Asp Leu Asn Lys Gly Glu Glu Ile
465 470 475 480

Thr Ser Asp Thr Asn Ile Glu Ala Ala Glu Glu Asn Ile Ser Leu Asp
485 490 495

Leu Ile Gln Gln Tyr Tyr Leu Thr Phe Asn Phe Asp Asn Glu Pro Glu
500 505 510

Asn Ile Ser Ile Glu Asn Leu Ser Ser Asp Ile Ile Gly Gln Leu Glu
515 520 525

Leu Met Pro Asn Ile Glu Arg Phe Pro Asn Gly Lys Lys Tyr Glu Leu
530 535 540

Asp Lys Tyr Thr Met Phe His Tyr Leu Arg Ala Gln Glu Phe Glu His
545 550 555 560

Gly Lys Ser Arg Ile Ala Leu Thr Asn Ser Val Asn Glu Ala Leu Leu
565 570 575

Asn Pro Ser Arg Val Tyr Thr Phe Phe Ser Ser Asp Tyr Val Lys Lys
580 585 590

Val Asn Lys Ala Thr Glu Ala Ala Met Phe Leu Gly Trp Val Glu Gln
595 600 605

Leu Val Tyr Asp Phe Thr Asp Glu Thr Ser Glu Val Ser Thr Thr Asp
610 615 620

Lys Ile Ala Asp Ile Thr Ile Ile Ile Pro Tyr Ile Gly Pro Ala Leu
625 630 635 640

Asn Ile Gly Asn Met Leu Tyr Lys Asp Asp Phe Val Gly Ala Leu Ile
645 650 655

Phe Ser Gly Ala Val Ile Leu Leu Glu Phe Ile Pro Glu Ile Ala Ile
660 665 670

Pro Val Leu Gly Thr Phe Ala Leu Val Ser Tyr Ile Ala Asn Lys Val
675 680 685

Leu Thr Val Gln Thr Ile Asp Asn Ala Leu Ser Lys Arg Asn Glu Lys
690 695 700

Trp Asp Glu Val Tyr Lys Tyr Ile Val Thr Asn Trp Leu Ala Lys Val
705 710 715 720

Asn Thr Gln Ile Asp Leu Ile Arg Lys Lys Met Lys Glu Ala Leu Glu
725 730 735

Asn Gln Ala Glu Ala Thr Lys Ala Ile Ile Asn Tyr Gln Tyr Asn Gln
740 745 750

Tyr Thr Glu Glu Glu Lys Asn Asn Ile Asn Phe Asn Ile Asp Asp Leu
755 760 765

Ser Ser Lys Leu Asn Glu Ser Ile Asn Lys Ala Met Ile Asn Ile Asn
770 775 780

Lys Phe Leu Asn Gln Cys Ser Val Ser Tyr Leu Met Asn Ser Met Ile
785 790 795 800

Pro Tyr Gly Val Lys Arg Leu Glu Asp Phe Asp Ala Ser Leu Lys Asp
805 810 815

Ala Leu Leu Lys Tyr Ile Tyr Asp Asn Arg Gly Thr Leu Ile Gly Gln
820 825 830

Val Asp Arg Leu Lys Asp Lys Val Asn Asn Thr Leu Ser Thr Asp Ile
835 840 845

Pro Phe Gln Leu Ser Lys Tyr Val Asp Asn Gln Arg Leu Leu Ser Thr
850 855 860

<210> 50
<211> 869
<212> PRT
<213> Artificial Sequence

<220>
<221> source
<223> /note="Description of Artificial Sequence: Synthetic polypeptide"

<400> 50
Pro Val Thr Ile Asn Asn Phe Asn Tyr Asn Asp Pro Ile Asp Asn Asn
1 5 10 15

```
Asn Ile Ile Met Met Glu Pro Pro Phe Ala Arg Gly Thr Gly Arg Tyr
            20                  25                  30

Tyr Lys Ala Phe Lys Ile Thr Asp Arg Ile Trp Ile Ile Pro Glu Arg
        35                  40                  45

Tyr Thr Phe Gly Tyr Lys Pro Glu Asp Phe Asn Lys Ser Ser Gly Ile
    50                  55                  60

Phe Asn Arg Asp Val Cys Glu Tyr Tyr Asp Pro Asp Tyr Leu Asn Thr
65                  70                  75                  80

Asn Asp Lys Lys Asn Ile Phe Leu Gln Thr Met Ile Lys Leu Phe Asn
                85                  90                  95

Arg Ile Lys Ser Lys Pro Leu Gly Glu Lys Leu Leu Glu Met Ile Ile
            100                 105                 110

Asn Gly Ile Pro Tyr Leu Gly Asp Arg Arg Val Pro Leu Glu Glu Phe
        115                 120                 125

Asn Thr Asn Ile Ala Ser Val Thr Val Asn Lys Leu Ile Ser Asn Pro
    130                 135                 140

Gly Glu Val Glu Arg Lys Lys Gly Ile Phe Ala Asn Leu Ile Ile Phe
145                 150                 155                 160

Gly Pro Gly Pro Val Leu Asn Glu Asn Glu Thr Ile Asp Ile Gly Ile
                165                 170                 175

Gln Asn His Phe Ala Ser Arg Glu Gly Phe Gly Gly Ile Met Gln Met
            180                 185                 190

Lys Phe Cys Pro Glu Tyr Val Ser Val Phe Asn Asn Val Gln Glu Asn
        195                 200                 205

Lys Gly Ala Ser Ile Phe Asn Arg Arg Gly Tyr Phe Ser Asp Pro Ala
    210                 215                 220

Leu Ile Leu Met His Glu Leu Ile His Val Leu His Gly Leu Tyr Gly
225                 230                 235                 240

Ile Lys Val Asp Asp Leu Pro Ile Val Pro Asn Glu Lys Lys Phe Phe
                245                 250                 255

Met Gln Ser Thr Asp Ala Ile Gln Ala Glu Glu Leu Tyr Thr Phe Gly
            260                 265                 270
```

Gly Gln Asp Pro Ser Ile Ile Thr Pro Ser Thr Asp Lys Ser Ile Tyr
275 280 285

Asp Lys Val Leu Gln Asn Phe Arg Gly Ile Val Asp Arg Leu Asn Lys
290 295 300

Val Leu Val Cys Ile Ser Asp Pro Asn Ile Asn Ile Asn Ile Tyr Lys
305 310 315 320

Asn Lys Phe Lys Asp Lys Tyr Lys Phe Val Glu Asp Ser Glu Gly Lys
325 330 335

Tyr Ser Ile Asp Val Glu Ser Phe Asp Lys Leu Tyr Lys Ser Leu Met
340 345 350

Phe Gly Phe Thr Glu Thr Asn Ile Ala Glu Asn Tyr Lys Ile Lys Thr
355 360 365

Arg Ala Ser Tyr Phe Ser Asp Ser Leu Pro Pro Val Lys Ile Lys Asn
370 375 380

Leu Leu Asp Asn Glu Ile Tyr Thr Ile Glu Glu Gly Phe Asn Ile Ser
385 390 395 400

Asp Lys Asp Met Glu Lys Glu Tyr Arg Gly Gln Asn Lys Ala Ile Asn
405 410 415

Lys Gln Ala Tyr Glu Glu Ile Ser Lys Glu His Leu Ala Val Tyr Lys
420 425 430

Ile Gln Met Cys Val Asp Glu Glu Lys Leu Tyr Asp Asp Asp Asp Lys
435 440 445

Asp Arg Trp Gly Ser Ser Leu Gln Cys Ile Asp Val Asp Asn Glu Asp
450 455 460

Leu Phe Phe Ile Ala Asp Lys Asn Ser Phe Ser Asp Asp Leu Ser Lys
465 470 475 480

Asn Glu Arg Ile Glu Tyr Asn Thr Gln Ser Asn Tyr Ile Glu Asn Asp
485 490 495

Phe Pro Ile Asn Glu Leu Ile Leu Asp Thr Asp Leu Ile Ser Lys Ile
500 505 510

Glu Leu Pro Ser Glu Asn Thr Glu Ser Leu Thr Asp Phe Asn Val Asp
515 520 525

Val Pro Val Tyr Glu Lys Gln Pro Ala Ile Lys Lys Ile Phe Thr Asp
530 535 540

Glu Asn Thr Ile Phe Gln Tyr Leu Tyr Ser Gln Thr Phe Pro Leu Asp
545 550 555 560

Ile Arg Asp Ile Ser Leu Thr Ser Ser Phe Asp Asp Ala Leu Leu Phe
565 570 575

Ser Asn Lys Val Tyr Ser Phe Phe Ser Met Asp Tyr Ile Lys Thr Ala
580 585 590

Asn Lys Val Val Glu Ala Gly Leu Phe Ala Gly Trp Val Lys Gln Ile
595 600 605

Val Asn Asp Phe Val Ile Glu Ala Asn Lys Ser Asn Thr Met Asp Ala
610 615 620

Ile Ala Asp Ile Ser Leu Ile Val Pro Tyr Ile Gly Leu Ala Leu Asn
625 630 635 640

Val Gly Asn Glu Thr Ala Lys Gly Asn Phe Glu Asn Ala Phe Glu Ile
645 650 655

Ala Gly Ala Ser Ile Leu Leu Glu Phe Ile Pro Glu Leu Leu Ile Pro
660 665 670

Val Val Gly Ala Phe Leu Leu Glu Ser Tyr Ile Asp Asn Lys Asn Lys
675 680 685

Ile Ile Lys Thr Ile Asp Asn Ala Leu Thr Lys Arg Asn Glu Lys Trp
690 695 700

Ser Asp Met Tyr Gly Leu Ile Val Ala Gln Trp Leu Ser Thr Val Asn
705 710 715 720

Thr Gln Phe Tyr Thr Ile Lys Glu Gly Met Tyr Lys Ala Leu Asn Tyr
725 730 735

Gln Ala Gln Ala Leu Glu Glu Ile Ile Lys Tyr Arg Tyr Asn Ile Tyr
740 745 750

Ser Glu Lys Glu Lys Ser Asn Ile Asn Ile Asp Phe Asn Asp Ile Asn
755 760 765

Ser Lys Leu Asn Glu Gly Ile Asn Gln Ala Ile Asp Asn Ile Asn Asn

770 775 780

Phe Ile Asn Gly Cys Ser Val Ser Tyr Leu Met Lys Lys Met Ile Pro
785 790 795 800

Leu Ala Val Glu Lys Leu Leu Asp Phe Asp Asn Thr Leu Lys Lys Asn
805 810 815

Leu Leu Asn Tyr Ile Asp Glu Asn Lys Leu Tyr Leu Ile Gly Ser Ala
820 825 830

Glu Tyr Glu Lys Ser Lys Val Asn Lys Tyr Leu Lys Thr Ile Met Pro
835 840 845

Phe Asp Leu Ser Ile Tyr Thr Asn Asp Thr Ile Leu Ile Glu Met Phe
850 855 860

Asn Lys Tyr Asn Ser
865

<210> 51
<211> 865
<212> PRT
<213> Artificial Sequence

<220>
<221> source
<223> /note="Description of Artificial Sequence: Synthetic polypeptide"

<400> 51
Pro Ile Thr Ile Asn Asn Phe Asn Tyr Ser Asp Pro Val Asp Asn Lys
1 5 10 15

Asn Ile Leu Tyr Leu Asp Thr His Leu Asn Thr Leu Ala Asn Glu Pro
20 25 30

Glu Lys Ala Phe Arg Ile Thr Gly Asn Ile Trp Val Ile Pro Asp Arg
35 40 45

Phe Ser Arg Asn Ser Asn Pro Asn Leu Asn Lys Pro Pro Arg Val Thr
50 55 60

Ser Pro Lys Ser Gly Tyr Tyr Asp Pro Asn Tyr Leu Ser Thr Asp Ser
65 70 75 80

Asp Lys Asp Thr Phe Leu Lys Glu Ile Ile Lys Leu Phe Lys Arg Ile
85 90 95

Asn Ser Arg Glu Ile Gly Glu Glu Leu Ile Tyr Arg Leu Ser Thr Asp

100 105 110

Ile Pro Phe Pro Gly Asn Asn Asn Thr Pro Ile Asn Thr Phe Asp Phe
115 120 125

Asp Val Asp Phe Asn Ser Val Asp Val Lys Thr Arg Gln Gly Asn Asn
130 135 140

Trp Val Lys Thr Gly Ser Ile Asn Pro Ser Val Ile Ile Thr Gly Pro
145 150 155 160

Arg Glu Asn Ile Ile Asp Pro Glu Thr Ser Thr Phe Lys Leu Thr Asn
165 170 175

Asn Thr Phe Ala Ala Gln Glu Gly Phe Gly Ala Leu Ser Ile Ile Ser
180 185 190

Ile Ser Pro Arg Phe Met Leu Thr Tyr Ser Asn Ala Thr Asn Asp Val
195 200 205

Gly Glu Gly Arg Phe Ser Lys Ser Glu Phe Cys Met Asp Pro Ile Leu
210 215 220

Ile Leu Met His Glu Leu Asn His Ala Met His Asn Leu Tyr Gly Ile
225 230 235 240

Ala Ile Pro Asn Asp Gln Thr Ile Ser Ser Val Thr Ser Asn Ile Phe
245 250 255

Tyr Ser Gln Tyr Asn Val Lys Leu Glu Tyr Ala Glu Ile Tyr Ala Phe
260 265 270

Gly Gly Pro Thr Ile Asp Leu Ile Pro Lys Ser Ala Arg Lys Tyr Phe
275 280 285

Glu Glu Lys Ala Leu Asp Tyr Tyr Arg Ser Ile Ala Lys Arg Leu Asn
290 295 300

Ser Ile Thr Thr Ala Asn Pro Ser Ser Phe Asn Lys Tyr Ile Gly Glu
305 310 315 320

Tyr Lys Gln Lys Leu Ile Arg Lys Tyr Arg Phe Val Val Glu Ser Ser
325 330 335

Gly Glu Val Thr Val Asn Arg Asn Lys Phe Val Glu Leu Tyr Asn Glu
340 345 350

```
Leu Thr Gln Ile Phe Thr Glu Phe Asn Tyr Ala Lys Ile Tyr Asn Val
        355                 360                 365

Gln Asn Arg Lys Ile Tyr Leu Ser Asn Val Tyr Thr Pro Val Thr Ala
    370                 375                 380

Asn Ile Leu Asp Asp Asn Val Tyr Asp Ile Gln Asn Gly Phe Asn Ile
385                 390                 395                 400

Pro Lys Ser Asn Leu Asn Val Leu Phe Met Gly Gln Asn Leu Ser Arg
                405                 410                 415

Asn Pro Ala Leu Arg Lys Val Asn Pro Glu Asn Met Leu Tyr Leu Phe
            420                 425                 430

Thr Lys Phe Cys Val Asp Ala Ile Asp Gly Arg Ser Leu Tyr Asn Lys
        435                 440                 445

Thr Leu Gln Cys Arg Glu Leu Leu Val Lys Asn Thr Asp Leu Pro Phe
    450                 455                 460

Ile Gly Asp Ile Ser Asp Val Lys Thr Asp Ile Phe Leu Arg Lys Asp
465                 470                 475                 480

Ile Asn Glu Glu Thr Glu Val Ile Tyr Tyr Pro Asp Asn Val Ser Val
                485                 490                 495

Asp Gln Val Ile Leu Ser Lys Asn Thr Ser Glu His Gly Gln Leu Asp
            500                 505                 510

Leu Leu Tyr Pro Ser Ile Asp Ser Glu Ser Glu Ile Leu Pro Gly Glu
        515                 520                 525

Asn Gln Val Phe Tyr Asp Asn Arg Thr Gln Asn Val Asp Tyr Leu Asn
    530                 535                 540

Ser Tyr Tyr Tyr Leu Glu Ser Gln Lys Leu Ser Asp Asn Val Glu Asp
545                 550                 555                 560

Phe Thr Phe Thr Arg Ser Ile Glu Glu Ala Leu Asp Asn Ser Ala Lys
                565                 570                 575

Val Tyr Thr Tyr Phe Pro Thr Leu Ala Asn Lys Val Asn Ala Gly Val
            580                 585                 590

Gln Gly Gly Leu Phe Leu Met Trp Ala Asn Asp Val Val Glu Asp Phe
        595                 600                 605
```

Thr Thr Asn Ile Leu Arg Lys Asp Thr Leu Asp Lys Ile Ser Asp Val
    610                 615                 620

Ser Ala Ile Ile Pro Tyr Ile Gly Pro Ala Leu Asn Ile Ser Asn Ser
625                 630                 635                 640

Val Arg Arg Gly Asn Phe Thr Glu Ala Phe Ala Val Thr Gly Val Thr
                645                 650                 655

Ile Leu Leu Glu Ala Phe Pro Glu Phe Thr Ile Pro Ala Leu Gly Ala
            660                 665                 670

Phe Val Ile Tyr Ser Lys Val Gln Glu Arg Asn Glu Ile Ile Lys Thr
        675                 680                 685

Ile Asp Asn Cys Leu Glu Gln Arg Ile Lys Arg Trp Lys Asp Ser Tyr
    690                 695                 700

Glu Trp Met Met Gly Thr Trp Leu Ser Arg Ile Ile Thr Gln Phe Asn
705                 710                 715                 720

Asn Ile Ser Tyr Gln Met Tyr Asp Ser Leu Asn Tyr Gln Ala Gly Ala
                725                 730                 735

Ile Lys Ala Lys Ile Asp Leu Glu Tyr Lys Lys Tyr Ser Gly Ser Asp
            740                 745                 750

Lys Glu Asn Ile Lys Ser Gln Val Glu Asn Leu Lys Asn Ser Leu Asp
        755                 760                 765

Val Lys Ile Ser Glu Ala Met Asn Asn Ile Asn Lys Phe Ile Arg Glu
    770                 775                 780

Cys Ser Val Thr Tyr Leu Phe Lys Asn Met Leu Pro Lys Val Ile Asp
785                 790                 795                 800

Glu Leu Asn Glu Phe Asp Arg Asn Thr Lys Ala Lys Leu Ile Asn Leu
                805                 810                 815

Ile Asp Ser His Asn Ile Ile Leu Val Gly Glu Val Asp Lys Leu Lys
            820                 825                 830

Ala Lys Val Asn Asn Ser Phe Gln Asn Thr Ile Pro Phe Asn Ile Phe
        835                 840                 845

Ser Tyr Thr Asn Asn Ser Leu Leu Lys Asp Ile Ile Asn Glu Tyr Phe
    850                 855                 860

Asn
865

<210> 52
<211> 871
<212> PRT
<213> Artificial Sequence

<220>
<221> source
<223> /note="Description of Artificial Sequence: Synthetic polypeptide"

<400> 52
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Lys Ser Glu Glu Lys Leu Tyr Asp Asp Asp

435 440 445

Asp Lys Asp Arg Trp Gly Ser Ser Leu Gln Cys Ile Lys Val Lys Asn
450 455 460

Asn Arg Leu Pro Tyr Val Ala Asp Lys Asp Ser Ile Ser Gln Glu Ile
465 470 475 480

Phe Glu Asn Lys Ile Ile Thr Asp Glu Thr Asn Val Gln Asn Tyr Ser
485 490 495

Asp Lys Phe Ser Leu Asp Glu Ser Ile Leu Asp Gly Gln Val Pro Ile
500 505 510

Asn Pro Glu Ile Val Asp Pro Leu Leu Pro Asn Val Asn Met Glu Pro
515 520 525

Leu Asn Leu Pro Gly Glu Glu Ile Val Phe Tyr Asp Asp Ile Thr Lys
530 535 540

Tyr Val Asp Tyr Leu Asn Ser Tyr Tyr Tyr Leu Glu Ser Gln Lys Leu
545 550 555 560

Ser Asn Asn Val Glu Asn Ile Thr Leu Thr Thr Ser Val Glu Glu Ala
565 570 575

Leu Gly Tyr Ser Asn Lys Ile Tyr Thr Phe Leu Pro Ser Leu Ala Glu
580 585 590

Lys Val Asn Lys Gly Val Gln Ala Gly Leu Phe Leu Asn Trp Ala Asn
595 600 605

Glu Val Val Glu Asp Phe Thr Thr Asn Ile Met Lys Lys Asp Thr Leu
610 615 620

Asp Lys Ile Ser Asp Val Ser Val Ile Ile Pro Tyr Ile Gly Pro Ala
625 630 635 640

Leu Asn Ile Gly Asn Ser Ala Leu Arg Gly Asn Phe Asn Gln Ala Phe
645 650 655

Ala Thr Ala Gly Val Ala Phe Leu Leu Glu Gly Phe Pro Glu Phe Thr
660 665 670

Ile Pro Ala Leu Gly Val Phe Thr Phe Tyr Ser Ser Ile Gln Glu Arg
675 680 685

Glu Lys Ile Ile Lys Thr Ile Glu Asn Cys Leu Glu Gln Arg Val Lys
    690                 695                 700

Arg Trp Lys Asp Ser Tyr Gln Trp Met Val Ser Asn Trp Leu Ser Arg
705                 710                 715                 720

Ile Thr Thr Gln Phe Asn His Ile Asn Tyr Gln Met Tyr Asp Ser Leu
                725                 730                 735

Ser Tyr Gln Ala Asp Ala Ile Lys Ala Lys Ile Asp Leu Glu Tyr Lys
            740                 745                 750

Lys Tyr Ser Gly Ser Asp Lys Glu Asn Ile Lys Ser Gln Val Glu Asn
        755                 760                 765

Leu Lys Asn Ser Leu Asp Val Lys Ile Ser Glu Ala Met Asn Asn Ile
    770                 775                 780

Asn Lys Phe Ile Arg Glu Cys Ser Val Thr Tyr Leu Phe Lys Asn Met
785                 790                 795                 800

Leu Pro Lys Val Ile Asp Glu Leu Asn Lys Phe Asp Leu Arg Thr Lys
                805                 810                 815

Thr Glu Leu Ile Asn Leu Ile Asp Ser His Asn Ile Ile Leu Val Gly
            820                 825                 830

Glu Val Asp Arg Leu Lys Ala Lys Val Asn Glu Ser Phe Glu Asn Thr
        835                 840                 845

Met Pro Phe Asn Ile Phe Ser Tyr Thr Asn Asn Ser Leu Leu Lys Asp
    850                 855                 860

Ile Ile Asn Glu Tyr Phe Asn
865                 870

<210> 53
<211> 680
<212> PRT
<213> Artificial Sequence

<220>
<221> source
<223> /note="Description of Artificial Sequence: Synthetic polypeptide"

<400> 53
Glu Ser Asn Gln Pro Glu Lys Asn Gly Thr Ala Thr Lys Pro Glu Asn
1               5                   10                  15

Ser Gly Asn Thr Thr Ser Glu Asn Gly Gln Thr Glu Pro Glu Lys Lys
20 25 30

Leu Glu Leu Arg Asn Val Ser Asp Ile Glu Leu Tyr Ser Gln Thr Asn
35 40 45

Gly Thr Tyr Arg Gln His Val Ser Leu Asp Gly Ile Pro Glu Asn Thr
50 55 60

Asp Thr Tyr Phe Val Lys Val Lys Ser Ser Ala Phe Lys Asp Val Tyr
65 70 75 80

Ile Pro Val Ala Ser Ile Thr Glu Glu Lys Arg Asn Gly Gln Ser Val
85 90 95

Tyr Lys Ile Thr Ala Lys Ala Glu Lys Leu Gln Gln Glu Leu Glu Asn
100 105 110

Lys Tyr Val Asp Asn Phe Thr Phe Tyr Leu Asp Lys Lys Ala Lys Glu
115 120 125

Glu Asn Thr Asn Phe Thr Ser Phe Ser Asn Leu Val Lys Ala Ile Asn
130 135 140

Gln Asn Pro Ser Gly Thr Tyr His Leu Ala Ala Ser Leu Asn Ala Asn
145 150 155 160

Glu Val Glu Leu Gly Pro Asp Glu Arg Ser Tyr Ile Lys Asp Thr Phe
165 170 175

Thr Gly Arg Leu Ile Gly Glu Lys Asp Gly Lys Asn Tyr Ala Ile Tyr
180 185 190

Asn Leu Lys Lys Pro Leu Phe Glu Asn Leu Ser Gly Ala Thr Val Glu
195 200 205

Lys Leu Ser Leu Lys Asn Val Ala Ile Ser Gly Lys Asn Asp Ile Gly
210 215 220

Ser Leu Ala Asn Glu Ala Thr Asn Gly Thr Lys Ile Lys Gln Val His
225 230 235 240

Val Asp Gly Cys Val Asp Gly Ile Ile Thr Ser Lys Thr Lys Ser Asp
245 250 255

Asp Asp Asp Lys Asn Lys Ala Leu Asn Leu Gln Cys Ile Lys Ile Lys
260 265 270

Asn Glu Asp Leu Thr Phe Ile Ala Glu Lys Asn Ser Phe Ser Glu Glu
275 280 285

Pro Phe Gln Asp Glu Ile Val Ser Tyr Asn Thr Lys Asn Lys Pro Leu
290 295 300

Asn Phe Asn Tyr Ser Leu Asp Lys Ile Ile Val Asp Tyr Asn Leu Gln
305 310 315 320

Ser Lys Ile Thr Leu Pro Asn Asp Arg Thr Thr Pro Val Thr Lys Gly
325 330 335

Ile Pro Tyr Ala Pro Glu Tyr Lys Ser Asn Ala Ala Ser Thr Ile Glu
340 345 350

Ile His Asn Ile Asp Asp Asn Thr Ile Tyr Gln Tyr Leu Tyr Ala Gln
355 360 365

Lys Ser Pro Thr Thr Leu Gln Arg Ile Thr Met Thr Asn Ser Val Asp
370 375 380

Asp Ala Leu Ile Asn Ser Thr Lys Ile Tyr Ser Tyr Phe Pro Ser Val
385 390 395 400

Ile Ser Lys Val Asn Gln Gly Ala Gln Gly Ile Leu Phe Leu Gln Trp
405 410 415

Val Arg Asp Ile Ile Asp Asp Phe Thr Asn Glu Ser Ser Gln Lys Thr
420 425 430

Thr Ile Asp Lys Ile Ser Asp Val Ser Thr Ile Val Pro Tyr Ile Gly
435 440 445

Pro Ala Leu Asn Ile Val Lys Gln Gly Tyr Glu Gly Asn Phe Ile Gly
450 455 460

Ala Leu Glu Thr Thr Gly Val Val Leu Leu Leu Glu Tyr Ile Pro Glu
465 470 475 480

Ile Thr Leu Pro Val Ile Ala Ala Leu Ser Ile Ala Glu Ser Ser Thr
485 490 495

Gln Lys Glu Lys Ile Ile Lys Thr Ile Asp Asn Phe Leu Glu Lys Arg
500 505 510

Tyr Glu Lys Trp Ile Glu Val Tyr Lys Leu Val Lys Ala Lys Trp Leu
515 520 525

Gly Thr Val Asn Thr Gln Phe Gln Lys Arg Ser Tyr Gln Met Tyr Arg
    530                 535                 540

Ser Leu Glu Tyr Gln Val Asp Ala Ile Lys Lys Ile Ile Asp Tyr Glu
545                 550                 555                 560

Tyr Lys Ile Tyr Ser Gly Pro Asp Lys Glu Gln Ile Ala Asp Glu Ile
                565                 570                 575

Asn Asn Leu Lys Asn Lys Leu Glu Glu Lys Ala Asn Lys Ala Met Ile
            580                 585                 590

Asn Ile Asn Ile Phe Met Arg Glu Ser Ser Arg Ser Phe Leu Val Asn
        595                 600                 605

Gln Met Ile Asn Glu Ala Lys Lys Gln Leu Leu Glu Phe Asp Thr Gln
    610                 615                 620

Ser Lys Asn Ile Leu Met Gln Tyr Ile Lys Ala Asn Ser Lys Phe Ile
625                 630                 635                 640

Gly Ile Thr Glu Leu Lys Lys Leu Glu Ser Lys Ile Asn Lys Val Phe
                645                 650                 655

Ser Thr Pro Ile Pro Phe Ser Tyr Ser Lys Asn Leu Asp Cys Trp Val
            660                 665                 670

Asp Asn Glu Glu Asp Ile Asp Val
        675                 680

<210> 54
<211> 9
<212> PRT
<213> Artificial Sequence

<220>
<221> source
<223> /note="Description of Artificial Sequence: Synthetic peptide"

<220>
<221> MOD_RES
<222> (2)..(2)
<223> D-Phe

<220>
<221> MOD_RES
<222> (5)..(5)
<223> D-Trp

<220>
<221> misc_feature
<222> (9)..(9)
<223> /note="C-terminal-alcohol"

<400> 54
Cys Phe Cys Phe Trp Lys Thr Cys Thr
1               5

<210> 55
<211> 30
<212> PRT
<213> Artificial Sequence

<220>
<221> source
<223> /note="Description of Artificial Sequence: Synthetic
      polypeptide"

<220>
<221> MOD_RES
<222> (27)..(27)
<223> Norleucine

<400> 55
His Ala Asp Ala Ile Phe Thr Asn Ser Tyr Arg Lys Val Leu Gly Gln
1               5                   10                  15

Leu Ser Ala Arg Lys Leu Leu Gln Asp Ile Leu Ser Arg Cys
            20                  25                  30

<210> 56
<211> 30
<212> PRT
<213> Artificial Sequence

<220>
<221> source
<223> /note="Description of Artificial Sequence: Synthetic
      polypeptide"

<220>
<221> MOD_RES
<222> (1)..(1)
<223> PhAc-Tyr

<220>
<221> MOD_RES
<222> (2)..(2)
<223> D-Arg

<220>
<221> MOD_RES
<222> (6)..(6)
<223> Phe(4-Cl)

<220>
<221> MOD_RES

<222> (9)..(9)
<223> Homoarginine

<220>
<221> MOD_RES
<222> (10)..(10)
<223> Tyr(Me)

<220>
<221> MOD_RES
<222> (15)..(15)
<223> Alpha-aminobutyric acid

<220>
<221> MOD_RES
<222> (27)..(27)
<223> Norleucine

<220>
<221> MOD_RES
<222> (28)..(28)
<223> D-Arg

<220>
<221> MOD_RES
<222> (29)..(29)
<223> Homoarginine

<400> 56
Tyr Arg Asp Ala Ile Phe Thr Ala Arg Tyr His Lys Val Leu Xaa Gln
1               5                   10                  15

Leu Ser Ala His Lys Leu Leu Gln Asp Ile Leu Arg Arg Cys
            20                  25                  30

<210> 57
<211> 893
<212> PRT
<213> Artificial Sequence

<220>
<221> source
<223> /note="Description of Artificial Sequence: Synthetic
      polypeptide"

<400> 57
Thr Trp Pro Val Lys Asp Phe Asn Tyr Ser Asp Pro Val Asn Asp Asn
1               5                   10                  15

Asp Ile Leu Tyr Leu Arg Ile Pro Gln Asn Lys Leu Ile Thr Thr Pro
            20                  25                  30

Val Lys Ala Phe Met Ile Thr Gln Asn Ile Trp Val Ile Pro Glu Arg
        35                  40                  45

Phe Ser Ser Asp Thr Asn Pro Ser Leu Ser Lys Pro Pro Arg Pro Thr
    50                  55                  60

```
Ser Lys Tyr Gln Ser Tyr Tyr Asp Pro Ser Tyr Leu Ser Thr Asp Glu
65                  70                  75                  80

Gln Lys Asp Thr Phe Leu Lys Gly Ile Ile Lys Leu Phe Lys Arg Ile
                85                  90                  95

Asn Glu Arg Asp Ile Gly Lys Lys Leu Ile Asn Tyr Leu Val Val Gly
            100                 105                 110

Ser Pro Phe Met Gly Asp Ser Ser Thr Pro Glu Asp Thr Phe Asp Phe
        115                 120                 125

Thr Arg His Thr Thr Asn Ile Ala Val Glu Lys Phe Glu Asn Gly Ser
    130                 135                 140

Trp Lys Val Thr Asn Ile Ile Thr Pro Ser Val Leu Ile Phe Gly Pro
145                 150                 155                 160

Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
225                 230                 235                 240

Asn Ile Pro Ser Asp Lys Arg Ile Arg Pro Gln Val Ser Glu Gly Phe
                245                 250                 255

Phe Ser Gln Asp Gly Pro Asn Val Gln Phe Glu Glu Leu Tyr Thr Phe
            260                 265                 270

Gly Gly Leu Asp Val Glu Ile Ile Pro Gln Ile Glu Arg Ser Gln Leu
        275                 280                 285

Arg Glu Lys Ala Leu Gly His Tyr Lys Asp Ile Ala Lys Arg Leu Asn
    290                 295                 300

Asn Ile Asn Lys Thr Ile Pro Ser Ser Trp Ile Ser Asn Ile Asp Lys
305                 310                 315                 320
```

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Asp Phe Asp Met Leu Arg Cys Met Leu Gly Arg Val
450 455 460

Tyr Arg Pro Cys Trp Gln Val Ala Leu Ala Lys Arg Leu Val Leu Gln
465 470 475 480

Cys Ile Lys Val Lys Asn Asn Arg Leu Pro Tyr Val Ala Asp Lys Asp
485 490 495

Ser Ile Ser Gln Glu Ile Phe Glu Asn Lys Ile Ile Thr Asp Glu Thr
500 505 510

Asn Val Gln Asn Tyr Ser Asp Lys Phe Ser Leu Asp Glu Ser Ile Leu
515 520 525

Asp Gly Gln Val Pro Ile Asn Pro Glu Ile Val Asp Pro Leu Leu Pro
530 535 540

Asn Val Asn Met Glu Pro Leu Asn Leu Pro Gly Glu Glu Ile Val Phe
545 550 555 560

Tyr Asp Asp Ile Thr Lys Tyr Val Asp Tyr Leu Asn Ser Tyr Tyr Tyr

565 570 575

Leu Glu Ser Gln Lys Leu Ser Asn Asn Val Glu Asn Ile Thr Leu Thr
580 585 590

Thr Ser Val Glu Glu Ala Leu Gly Tyr Ser Asn Lys Ile Tyr Thr Phe
595 600 605

Leu Pro Ser Leu Ala Glu Lys Val Asn Lys Gly Val Gln Ala Gly Leu
610 615 620

Phe Leu Asn Trp Ala Asn Glu Val Val Glu Asp Phe Thr Thr Asn Ile
625 630 635 640

Met Lys Lys Asp Thr Leu Asp Lys Ile Ser Asp Val Ser Val Ile Ile
645 650 655

Pro Tyr Ile Gly Pro Ala Leu Asn Ile Gly Asn Ser Ala Leu Arg Gly
660 665 670

Asn Phe Asn Gln Ala Phe Ala Thr Ala Gly Val Ala Phe Leu Leu Glu
675 680 685

Gly Phe Pro Glu Phe Thr Ile Pro Ala Leu Gly Val Phe Thr Phe Tyr
690 695 700

Ser Ser Ile Gln Glu Arg Glu Lys Ile Ile Lys Thr Ile Glu Asn Cys
705 710 715 720

Leu Glu Gln Arg Val Lys Arg Trp Lys Asp Ser Tyr Gln Trp Met Val
725 730 735

Ser Asn Trp Leu Ser Arg Ile Thr Thr Gln Phe Asn His Ile Asn Tyr
740 745 750

Gln Met Tyr Asp Ser Leu Ser Tyr Gln Ala Asp Ala Ile Lys Ala Lys
755 760 765

Ile Asp Leu Glu Tyr Lys Lys Tyr Ser Gly Ser Asp Lys Glu Asn Ile
770 775 780

Lys Ser Gln Val Glu Asn Leu Lys Asn Ser Leu Asp Val Lys Ile Ser
785 790 795 800

Glu Ala Met Asn Asn Ile Asn Lys Phe Ile Arg Glu Cys Ser Val Thr
805 810 815

```
Tyr Leu Phe Lys Asn Met Leu Pro Lys Val Ile Asp Glu Leu Asn Lys
            820                 825                 830

Phe Asp Leu Arg Thr Lys Thr Glu Leu Ile Asn Leu Ile Asp Ser His
        835                 840                 845

Asn Ile Ile Leu Val Gly Glu Val Asp Arg Leu Lys Ala Lys Val Asn
    850                 855                 860

Glu Ser Phe Glu Asn Thr Met Pro Phe Asn Ile Phe Ser Tyr Thr Asn
865                 870                 875                 880

Asn Ser Leu Leu Lys Asp Ile Ile Asn Glu Tyr Phe Asn
                885                 890


<210> 58
<211> 902
<212> PRT
<213> Artificial Sequence

<220>
<221> source
<223> /note="Description of Artificial Sequence: Synthetic
      polypeptide"

<400> 58
Thr Trp Pro Val Lys Asp Phe Asn Tyr Ser Asp Pro Val Asn Asp Asn
1               5                   10                  15

Asp Ile Leu Tyr Leu Arg Ile Pro Gln Asn Lys Leu Ile Thr Thr Pro
            20                  25                  30

Val Lys Ala Phe Met Ile Thr Gln Asn Ile Trp Val Ile Pro Glu Arg
        35                  40                  45

Phe Ser Ser Asp Thr Asn Pro Ser Leu Ser Lys Pro Pro Arg Pro Thr
    50                  55                  60

Ser Lys Tyr Gln Ser Tyr Tyr Asp Pro Ser Tyr Leu Ser Thr Asp Glu
65                  70                  75                  80

Gln Lys Asp Thr Phe Leu Lys Gly Ile Ile Lys Leu Phe Lys Arg Ile
                85                  90                  95

Asn Glu Arg Asp Ile Gly Lys Lys Leu Ile Asn Tyr Leu Val Val Gly
            100                 105                 110

Ser Pro Phe Met Gly Asp Ser Ser Thr Pro Glu Asp Thr Phe Asp Phe
        115                 120                 125
```

```
Thr Arg His Thr Thr Asn Ile Ala Val Glu Lys Phe Glu Asn Gly Ser
    130                 135                 140

Trp Lys Val Thr Asn Ile Ile Thr Pro Ser Val Leu Ile Phe Gly Pro
145                 150                 155                 160

Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
225                 230                 235                 240

Asn Ile Pro Ser Asp Lys Arg Ile Arg Pro Gln Val Ser Glu Gly Phe
                245                 250                 255

Phe Ser Gln Asp Gly Pro Asn Val Gln Phe Glu Glu Leu Tyr Thr Phe
            260                 265                 270

Gly Gly Leu Asp Val Glu Ile Ile Pro Gln Ile Glu Arg Ser Gln Leu
        275                 280                 285

Arg Glu Lys Ala Leu Gly His Tyr Lys Asp Ile Ala Lys Arg Leu Asn
    290                 295                 300

Asn Ile Asn Lys Thr Ile Pro Ser Ser Trp Ile Ser Asn Ile Asp Lys
305                 310                 315                 320

Tyr Lys Lys Ile Phe Ser Glu Lys Tyr Asn Phe Asp Lys Asp Asn Thr
                325                 330                 335

Gly Asn Phe Val Val Asn Ile Asp Lys Phe Asn Ser Leu Tyr Ser Asp
            340                 345                 350

Leu Thr Asn Val Met Ser Glu Val Val Tyr Ser Ser Gln Tyr Asn Val
        355                 360                 365

Lys Asn Arg Thr His Tyr Phe Ser Arg His Tyr Leu Pro Val Phe Ala
    370                 375                 380
```

```
Asn Ile Leu Asp Asp Asn Ile Tyr Thr Ile Arg Asp Gly Phe Asn Leu
385                 390                 395                 400

Thr Asn Lys Gly Phe Asn Ile Glu Asn Ser Gly Gln Asn Ile Glu Arg
                405                 410                 415

Asn Pro Ala Leu Gln Lys Leu Ser Ser Glu Ser Val Val Asp Leu Phe
            420                 425                 430

Thr Lys Val Cys Val Asp Lys Ser Glu Glu Lys Leu Tyr Asp Asp Asp
        435                 440                 445

Asp Lys Asp Arg Trp Gly Ser Ser Leu Gln Cys Ile Lys Val Lys Asn
    450                 455                 460

Asn Arg Leu Pro Tyr Val Ala Asp Lys Asp Ser Ile Ser Gln Glu Ile
465                 470                 475                 480

Phe Glu Asn Lys Ile Ile Thr Asp Glu Thr Asn Val Gln Asn Tyr Ser
                485                 490                 495

Asp Lys Phe Ser Leu Asp Glu Ser Ile Leu Asp Gly Gln Val Pro Ile
            500                 505                 510

Asn Pro Glu Ile Val Asp Pro Leu Leu Pro Asn Val Asn Met Glu Pro
        515                 520                 525

Leu Asn Leu Pro Gly Glu Glu Ile Val Phe Tyr Asp Asp Ile Thr Lys
    530                 535                 540

Tyr Val Asp Tyr Leu Asn Ser Tyr Tyr Tyr Leu Glu Ser Gln Lys Leu
545                 550                 555                 560

Ser Asn Asn Val Glu Asn Ile Thr Leu Thr Thr Ser Val Glu Glu Ala
                565                 570                 575

Leu Gly Tyr Ser Asn Lys Ile Tyr Thr Phe Leu Pro Ser Leu Ala Glu
            580                 585                 590

Lys Val Asn Lys Gly Val Gln Ala Gly Leu Phe Leu Asn Trp Ala Asn
        595                 600                 605

Glu Val Val Glu Asp Phe Thr Thr Asn Ile Met Lys Lys Asp Thr Leu
    610                 615                 620

Asp Lys Ile Ser Asp Val Ser Val Ile Ile Pro Tyr Ile Gly Pro Ala
625                 630                 635                 640
```

Leu Asn Ile Gly Asn Ser Ala Leu Arg Gly Asn Phe Asn Gln Ala Phe
645 650 655

Ala Thr Ala Gly Val Ala Phe Leu Leu Glu Gly Phe Pro Glu Phe Thr
660 665 670

Ile Pro Ala Leu Gly Val Phe Thr Phe Tyr Ser Ser Ile Gln Glu Arg
675 680 685

Glu Lys Ile Ile Lys Thr Ile Glu Asn Cys Leu Glu Gln Arg Val Lys
690 695 700

Arg Trp Lys Asp Ser Tyr Gln Trp Met Val Ser Asn Trp Leu Ser Arg
705 710 715 720

Ile Thr Thr Gln Phe Asn His Ile Asn Tyr Gln Met Tyr Asp Ser Leu
725 730 735

Ser Tyr Gln Ala Asp Ala Ile Lys Ala Lys Ile Asp Leu Glu Tyr Lys
740 745 750

Lys Tyr Ser Gly Ser Asp Lys Glu Asn Ile Lys Ser Gln Val Glu Asn
755 760 765

Leu Lys Asn Ser Leu Asp Val Lys Ile Ser Glu Ala Met Asn Asn Ile
770 775 780

Asn Lys Phe Ile Arg Glu Cys Ser Val Thr Tyr Leu Phe Lys Asn Met
785 790 795 800

Leu Pro Lys Val Ile Asp Glu Leu Asn Lys Phe Asp Leu Arg Thr Lys
805 810 815

Thr Glu Leu Ile Asn Leu Ile Asp Ser His Asn Ile Ile Leu Val Gly
820 825 830

Glu Val Asp Arg Leu Lys Ala Lys Val Asn Glu Ser Phe Glu Asn Thr
835 840 845

Met Pro Phe Asn Ile Phe Ser Tyr Thr Asn Asn Ser Leu Leu Lys Asp
850 855 860

Ile Ile Asn Glu Tyr Phe Asn Leu Glu Gly Gly Gly Gly Ser Gly Gly
865 870 875 880

Gly Gly Ser Gly Gly Gly Gly Ser Ala Leu Val Tyr Asn Trp Asn Ser

885 890 895

Phe Gly Leu Arg Phe Gly
900

<210> 59
<211> 905
<212> PRT
<213> Artificial Sequence

<220>
<221> source
<223> /note="Description of Artificial Sequence: Synthetic polypeptide"

<400> 59
Glu Phe Val Asn Lys Gln Phe Asn Tyr Lys Asp Pro Val Asn Gly Val
1 5 10 15

Asp Ile Ala Tyr Ile Lys Ile Pro Asn Ala Gly Gln Met Gln Pro Val
20 25 30

Lys Ala Phe Lys Ile His Asn Lys Ile Trp Val Ile Pro Glu Arg Asp
35 40 45

Thr Phe Thr Asn Pro Glu Glu Gly Asp Leu Asn Pro Pro Pro Glu Ala
50 55 60

Lys Gln Val Pro Val Ser Tyr Tyr Asp Ser Thr Tyr Leu Ser Thr Asp
65 70 75 80

Asn Glu Lys Asp Asn Tyr Leu Lys Gly Val Thr Lys Leu Phe Glu Arg
85 90 95

Ile Tyr Ser Thr Asp Leu Gly Arg Met Leu Leu Thr Ser Ile Val Arg
100 105 110

Gly Ile Pro Phe Trp Gly Gly Ser Thr Ile Asp Thr Glu Leu Lys Val
115 120 125

Ile Asp Thr Asn Cys Ile Asn Val Ile Gln Pro Asp Gly Ser Tyr Arg
130 135 140

Ser Glu Glu Leu Asn Leu Val Ile Ile Gly Pro Ser Ala Asp Ile Ile
145 150 155 160

Gln Phe Glu Cys Lys Ser Phe Gly His Glu Val Leu Asn Leu Thr Arg
165 170 175

Asn Gly Tyr Gly Ser Thr Gln Tyr Ile Arg Phe Ser Pro Asp Phe Thr

180 185 190

Phe Gly Phe Glu Glu Ser Leu Glu Val Asp Thr Asn Pro Leu Leu Gly
195 200 205

Ala Gly Lys Phe Ala Thr Asp Pro Ala Val Thr Leu Ala His Glu Leu
210 215 220

Ile His Ala Gly His Arg Leu Tyr Gly Ile Ala Ile Asn Pro Asn Arg
225 230 235 240

Val Phe Lys Val Asn Thr Asn Ala Tyr Tyr Glu Met Ser Gly Leu Glu
245 250 255

Val Ser Phe Glu Glu Leu Arg Thr Phe Gly Gly His Asp Ala Lys Phe
260 265 270

Ile Asp Ser Leu Gln Glu Asn Glu Phe Arg Leu Tyr Tyr Tyr Asn Lys
275 280 285

Phe Lys Asp Ile Ala Ser Thr Leu Asn Lys Ala Lys Ser Ile Val Gly
290 295 300

Thr Thr Ala Ser Leu Gln Tyr Met Lys Asn Val Phe Lys Glu Lys Tyr
305 310 315 320

Leu Leu Ser Glu Asp Thr Ser Gly Lys Phe Ser Val Asp Lys Leu Lys
325 330 335

Phe Asp Lys Leu Tyr Lys Met Leu Thr Glu Ile Tyr Thr Glu Asp Asn
340 345 350

Phe Val Lys Phe Phe Lys Val Leu Asn Arg Lys Thr Tyr Leu Asn Phe
355 360 365

Asp Lys Ala Val Phe Lys Ile Asn Ile Val Pro Lys Val Asn Tyr Thr
370 375 380

Ile Tyr Asp Gly Phe Asn Leu Arg Asn Thr Asn Leu Ala Ala Asn Phe
385 390 395 400

Asn Gly Gln Asn Thr Glu Ile Asn Asn Met Asn Phe Thr Lys Leu Lys
405 410 415

Asn Phe Thr Gly Leu Phe Glu Phe Tyr Lys Leu Leu Cys Val Asp Gly
420 425 430

Ile Ile Thr Ser Lys Thr Lys Ser Asp Asp Asp Asp Lys Asn Lys Ala
435 440 445

Leu Asn Leu Gln Cys Ile Lys Val Asn Asn Trp Asp Leu Phe Phe Ser
450 455 460

Pro Ser Glu Asp Asn Phe Thr Asn Asp Leu Asn Lys Gly Glu Glu Ile
465 470 475 480

Thr Ser Asp Thr Asn Ile Glu Ala Ala Glu Glu Asn Ile Ser Leu Asp
485 490 495

Leu Ile Gln Gln Tyr Tyr Leu Thr Phe Asn Phe Asp Asn Glu Pro Glu
500 505 510

Asn Ile Ser Ile Glu Asn Leu Ser Ser Asp Ile Ile Gly Gln Leu Glu
515 520 525

Leu Met Pro Asn Ile Glu Arg Phe Pro Asn Gly Lys Lys Tyr Glu Leu
530 535 540

Asp Lys Tyr Thr Met Phe His Tyr Leu Arg Ala Gln Glu Phe Glu His
545 550 555 560

Gly Lys Ser Arg Ile Ala Leu Thr Asn Ser Val Asn Glu Ala Leu Leu
565 570 575

Asn Pro Ser Arg Val Tyr Thr Phe Phe Ser Ser Asp Tyr Val Lys Lys
580 585 590

Val Asn Lys Ala Thr Glu Ala Ala Met Phe Leu Gly Trp Val Glu Gln
595 600 605

Leu Val Tyr Asp Phe Thr Asp Glu Thr Ser Glu Val Ser Thr Thr Asp
610 615 620

Lys Ile Ala Asp Ile Thr Ile Ile Ile Pro Tyr Ile Gly Pro Ala Leu
625 630 635 640

Asn Ile Gly Asn Met Leu Tyr Lys Asp Asp Phe Val Gly Ala Leu Ile
645 650 655

Phe Ser Gly Ala Val Ile Leu Leu Glu Phe Ile Pro Glu Ile Ala Ile
660 665 670

Pro Val Leu Gly Thr Phe Ala Leu Val Ser Tyr Ile Ala Asn Lys Val
675 680 685

Leu Thr Val Gln Thr Ile Asp Asn Ala Leu Ser Lys Arg Asn Glu Lys
    690                 695                 700

Trp Asp Glu Val Tyr Lys Tyr Ile Val Thr Asn Trp Leu Ala Lys Val
705                 710                 715                 720

Asn Thr Gln Ile Asp Leu Ile Arg Lys Lys Met Lys Glu Ala Leu Glu
                725                 730                 735

Asn Gln Ala Glu Ala Thr Lys Ala Ile Ile Asn Tyr Gln Tyr Asn Gln
            740                 745                 750

Tyr Thr Glu Glu Glu Lys Asn Asn Ile Asn Phe Asn Ile Asp Asp Leu
        755                 760                 765

Ser Ser Lys Leu Asn Glu Ser Ile Asn Lys Ala Met Ile Asn Ile Asn
    770                 775                 780

Lys Phe Leu Asn Gln Cys Ser Val Ser Tyr Leu Met Asn Ser Met Ile
785                 790                 795                 800

Pro Tyr Gly Val Lys Arg Leu Glu Asp Phe Asp Ala Ser Leu Lys Asp
                805                 810                 815

Ala Leu Leu Lys Tyr Ile Tyr Asp Asn Arg Gly Thr Leu Ile Gly Gln
            820                 825                 830

Val Asp Arg Leu Lys Asp Lys Val Asn Asn Thr Leu Ser Thr Asp Ile
        835                 840                 845

Pro Phe Gln Leu Ser Lys Tyr Val Asp Asn Gln Arg Leu Leu Ser Thr
    850                 855                 860

Leu Glu Gly Gly Gly Gly Ser Gly Gly Gly Gly Ser Gly Gly Gly Gly
865                 870                 875                 880

Ser Ala Leu Val Thr Pro Asp Ile Asn Pro Ala Trp Tyr Ala Ser Arg
                885                 890                 895

Gly Ile Arg Pro Val Gly Arg Phe Gly
            900                 905

<210> 60
<211> 915
<212> PRT
<213> Artificial Sequence

<220>

<221> source
<223> /note="Description of Artificial Sequence: Synthetic polypeptide"

<400> 60
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

```
Asn Ile Pro Ser Asp Lys Arg Ile Arg Pro Gln Val Ser Glu Gly Phe
                245                 250                 255

Phe Ser Gln Asp Gly Pro Asn Val Gln Phe Glu Glu Leu Tyr Thr Phe
            260                 265                 270

Gly Gly Leu Asp Val Glu Ile Ile Pro Gln Ile Glu Arg Ser Gln Leu
        275                 280                 285

Arg Glu Lys Ala Leu Gly His Tyr Lys Asp Ile Ala Lys Arg Leu Asn
    290                 295                 300

Asn Ile Asn Lys Thr Ile Pro Ser Ser Trp Ile Ser Asn Ile Asp Lys
305                 310                 315                 320

Tyr Lys Lys Ile Phe Ser Glu Lys Tyr Asn Phe Asp Lys Asp Asn Thr
                325                 330                 335

Gly Asn Phe Val Val Asn Ile Asp Lys Phe Asn Ser Leu Tyr Ser Asp
            340                 345                 350

Leu Thr Asn Val Met Ser Glu Val Val Tyr Ser Ser Gln Tyr Asn Val
        355                 360                 365

Lys Asn Arg Thr His Tyr Phe Ser Arg His Tyr Leu Pro Val Phe Ala
    370                 375                 380

Asn Ile Leu Asp Asp Asn Ile Tyr Thr Ile Arg Asp Gly Phe Asn Leu
385                 390                 395                 400

Thr Asn Lys Gly Phe Asn Ile Glu Asn Ser Gly Gln Asn Ile Glu Arg
                405                 410                 415

Asn Pro Ala Leu Gln Lys Leu Ser Ser Glu Ser Val Val Asp Leu Phe
            420                 425                 430

Thr Lys Val Cys Val Asp Gly Ile Ile Thr Ser Lys Thr Lys Ser Asp
        435                 440                 445

Asp Asp Asp Lys Tyr Ala Asp Ala Ile Phe Thr Asn Ser Tyr Arg Lys
    450                 455                 460

Val Leu Gly Gln Leu Ser Ala Arg Lys Leu Leu Gln Asp Ile Met Ala
465                 470                 475                 480

Leu Ala Gly Gly Gly Gly Ser Gly Gly Gly Gly Ser Gly Gly Gly Gly
```

485 490 495

Ser Ala Leu Ala Leu Gln Cys Ile Lys Val Lys Asn Asn Arg Leu Pro
500 505 510

Tyr Val Ala Asp Lys Asp Ser Ile Ser Gln Glu Ile Phe Glu Asn Lys
515 520 525

Ile Ile Thr Asp Glu Thr Asn Val Gln Asn Tyr Ser Asp Lys Phe Ser
530 535 540

Leu Asp Glu Ser Ile Leu Asp Gly Gln Val Pro Ile Asn Pro Glu Ile
545 550 555 560

Val Asp Pro Leu Leu Pro Asn Val Asn Met Glu Pro Leu Asn Leu Pro
565 570 575

Gly Glu Glu Ile Val Phe Tyr Asp Asp Ile Thr Lys Tyr Val Asp Tyr
580 585 590

Leu Asn Ser Tyr Tyr Tyr Leu Glu Ser Gln Lys Leu Ser Asn Asn Val
595 600 605

Glu Asn Ile Thr Leu Thr Thr Ser Val Glu Glu Ala Leu Gly Tyr Ser
610 615 620

Asn Lys Ile Tyr Thr Phe Leu Pro Ser Leu Ala Glu Lys Val Asn Lys
625 630 635 640

Gly Val Gln Ala Gly Leu Phe Leu Asn Trp Ala Asn Glu Val Val Glu
645 650 655

Asp Phe Thr Thr Asn Ile Met Lys Lys Asp Thr Leu Asp Lys Ile Ser
660 665 670

Asp Val Ser Val Ile Ile Pro Tyr Ile Gly Pro Ala Leu Asn Ile Gly
675 680 685

Asn Ser Ala Leu Arg Gly Asn Phe Asn Gln Ala Phe Ala Thr Ala Gly
690 695 700

Val Ala Phe Leu Leu Glu Gly Phe Pro Glu Phe Thr Ile Pro Ala Leu
705 710 715 720

Gly Val Phe Thr Phe Tyr Ser Ser Ile Gln Glu Arg Glu Lys Ile Ile
725 730 735

Lys Thr Ile Glu Asn Cys Leu Glu Gln Arg Val Lys Arg Trp Lys Asp
            740                 745                 750

Ser Tyr Gln Trp Met Val Ser Asn Trp Leu Ser Arg Ile Thr Thr Gln
        755                 760                 765

Phe Asn His Ile Asn Tyr Gln Met Tyr Asp Ser Leu Ser Tyr Gln Ala
    770                 775                 780

Asp Ala Ile Lys Ala Lys Ile Asp Leu Glu Tyr Lys Lys Tyr Ser Gly
785                 790                 795                 800

Ser Asp Lys Glu Asn Ile Lys Ser Gln Val Glu Asn Leu Lys Asn Ser
                805                 810                 815

Leu Asp Val Lys Ile Ser Glu Ala Met Asn Asn Ile Asn Lys Phe Ile
            820                 825                 830

Arg Glu Cys Ser Val Thr Tyr Leu Phe Lys Asn Met Leu Pro Lys Val
        835                 840                 845

Ile Asp Glu Leu Asn Lys Phe Asp Leu Arg Thr Lys Thr Glu Leu Ile
    850                 855                 860

Asn Leu Ile Asp Ser His Asn Ile Ile Leu Val Gly Glu Val Asp Arg
865                 870                 875                 880

Leu Lys Ala Lys Val Asn Glu Ser Phe Glu Asn Thr Met Pro Phe Asn
                885                 890                 895

Ile Phe Ser Tyr Thr Asn Asn Ser Leu Leu Lys Asp Ile Ile Asn Glu
            900                 905                 910

Tyr Phe Asn
        915

<210> 61
<211> 916
<212> PRT
<213> Artificial Sequence

<220>
<221> source
<223> /note="Description of Artificial Sequence: Synthetic
      polypeptide"

<400> 61
Thr Trp Pro Val Lys Asp Phe Asn Tyr Ser Asp Pro Val Asn Asp Asn
1               5                   10                  15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Tyr Ala Asp Ala Ile Phe Thr Asn Ser Tyr Arg Lys
450 455 460

Val Leu Gly Gln Leu Ser Ala Arg Lys Leu Leu Gln Asp Ile Met Ser
465 470 475 480

Ala Leu Ala Gly Gly Gly Gly Ser Gly Gly Gly Gly Ser Gly Gly Gly
485 490 495

Gly Ser Ala Leu Ala Leu Gln Cys Ile Lys Val Lys Asn Asn Arg Leu
500 505 510

Pro Tyr Val Ala Asp Lys Asp Ser Ile Ser Gln Glu Ile Phe Glu Asn
515 520 525

Lys Ile Ile Thr Asp Glu Thr Asn Val Gln Asn Tyr Ser Asp Lys Phe
530 535 540

Ser Leu Asp Glu Ser Ile Leu Asp Gly Gln Val Pro Ile Asn Pro Glu
545 550 555 560

Ile Val Asp Pro Leu Leu Pro Asn Val Asn Met Glu Pro Leu Asn Leu
565 570 575

Pro Gly Glu Glu Ile Val Phe Tyr Asp Asp Ile Thr Lys Tyr Val Asp
580 585 590

Tyr Leu Asn Ser Tyr Tyr Tyr Leu Glu Ser Gln Lys Leu Ser Asn Asn
595 600 605

Val Glu Asn Ile Thr Leu Thr Thr Ser Val Glu Glu Ala Leu Gly Tyr
610 615 620

Ser Asn Lys Ile Tyr Thr Phe Leu Pro Ser Leu Ala Glu Lys Val Asn
625 630 635 640

Lys Gly Val Gln Ala Gly Leu Phe Leu Asn Trp Ala Asn Glu Val Val
645 650 655

Glu Asp Phe Thr Thr Asn Ile Met Lys Lys Asp Thr Leu Asp Lys Ile
660 665 670

Ser Asp Val Ser Val Ile Ile Pro Tyr Ile Gly Pro Ala Leu Asn Ile
675 680 685

Gly Asn Ser Ala Leu Arg Gly Asn Phe Asn Gln Ala Phe Ala Thr Ala
690 695 700

Gly Val Ala Phe Leu Leu Glu Gly Phe Pro Glu Phe Thr Ile Pro Ala
705 710 715 720

Leu Gly Val Phe Thr Phe Tyr Ser Ser Ile Gln Glu Arg Glu Lys Ile
725 730 735

Ile Lys Thr Ile Glu Asn Cys Leu Glu Gln Arg Val Lys Arg Trp Lys
740 745 750

Asp Ser Tyr Gln Trp Met Val Ser Asn Trp Leu Ser Arg Ile Thr Thr
755 760 765

Gln Phe Asn His Ile Asn Tyr Gln Met Tyr Asp Ser Leu Ser Tyr Gln

770 775 780

Ala Asp Ala Ile Lys Ala Lys Ile Asp Leu Glu Tyr Lys Lys Tyr Ser
785 790 795 800

Gly Ser Asp Lys Glu Asn Ile Lys Ser Gln Val Glu Asn Leu Lys Asn
805 810 815

Ser Leu Asp Val Lys Ile Ser Glu Ala Met Asn Asn Ile Asn Lys Phe
820 825 830

Ile Arg Glu Cys Ser Val Thr Tyr Leu Phe Lys Asn Met Leu Pro Lys
835 840 845

Val Ile Asp Glu Leu Asn Lys Phe Asp Leu Arg Thr Lys Thr Glu Leu
850 855 860

Ile Asn Leu Ile Asp Ser His Asn Ile Ile Leu Val Gly Glu Val Asp
865 870 875 880

Arg Leu Lys Ala Lys Val Asn Glu Ser Phe Glu Asn Thr Met Pro Phe
885 890 895

Asn Ile Phe Ser Tyr Thr Asn Asn Ser Leu Leu Lys Asp Ile Ile Asn
900 905 910

Glu Tyr Phe Asn
915

<210> 62
<211> 917
<212> PRT
<213> Artificial Sequence

<220>
<221> source
<223> /note="Description of Artificial Sequence: Synthetic polypeptide"

<400> 62
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr

50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Tyr Ala Asp Ala Ile Phe Thr Asn Ser Tyr Arg Lys
450 455 460

Val Leu Gly Gln Leu Ser Ala Arg Lys Leu Leu Gln Asp Ile Met Ser
465 470 475 480

Arg Ala Leu Ala Gly Gly Gly Gly Ser Gly Gly Gly Gly Ser Gly Gly
485 490 495

Gly Gly Ser Ala Leu Ala Leu Gln Cys Ile Lys Val Lys Asn Asn Arg
500 505 510

Leu Pro Tyr Val Ala Asp Lys Asp Ser Ile Ser Gln Glu Ile Phe Glu
515 520 525

Asn Lys Ile Ile Thr Asp Glu Thr Asn Val Gln Asn Tyr Ser Asp Lys
530 535 540

Phe Ser Leu Asp Glu Ser Ile Leu Asp Gly Gln Val Pro Ile Asn Pro
545 550 555 560

Glu Ile Val Asp Pro Leu Leu Pro Asn Val Asn Met Glu Pro Leu Asn
565 570 575

Leu Pro Gly Glu Glu Ile Val Phe Tyr Asp Asp Ile Thr Lys Tyr Val
580 585 590

Asp Tyr Leu Asn Ser Tyr Tyr Tyr Leu Glu Ser Gln Lys Leu Ser Asn
595 600 605

Asn Val Glu Asn Ile Thr Leu Thr Thr Ser Val Glu Glu Ala Leu Gly
610 615 620

Tyr Ser Asn Lys Ile Tyr Thr Phe Leu Pro Ser Leu Ala Glu Lys Val
625 630 635 640

Asn Lys Gly Val Gln Ala Gly Leu Phe Leu Asn Trp Ala Asn Glu Val
645 650 655

Val Glu Asp Phe Thr Thr Asn Ile Met Lys Lys Asp Thr Leu Asp Lys
660 665 670

Ile Ser Asp Val Ser Val Ile Ile Pro Tyr Ile Gly Pro Ala Leu Asn
675 680 685

Ile Gly Asn Ser Ala Leu Arg Gly Asn Phe Asn Gln Ala Phe Ala Thr
690 695 700

Ala Gly Val Ala Phe Leu Leu Glu Gly Phe Pro Glu Phe Thr Ile Pro
705 710 715 720

Ala Leu Gly Val Phe Thr Phe Tyr Ser Ser Ile Gln Glu Arg Glu Lys
725 730 735

Ile Ile Lys Thr Ile Glu Asn Cys Leu Glu Gln Arg Val Lys Arg Trp
740 745 750

Lys Asp Ser Tyr Gln Trp Met Val Ser Asn Trp Leu Ser Arg Ile Thr
755 760 765

Thr Gln Phe Asn His Ile Asn Tyr Gln Met Tyr Asp Ser Leu Ser Tyr
770 775 780

Gln Ala Asp Ala Ile Lys Ala Lys Ile Asp Leu Glu Tyr Lys Lys Tyr
785 790 795 800

Ser Gly Ser Asp Lys Glu Asn Ile Lys Ser Gln Val Glu Asn Leu Lys
805 810 815

Asn Ser Leu Asp Val Lys Ile Ser Glu Ala Met Asn Asn Ile Asn Lys
            820                 825                 830

Phe Ile Arg Glu Cys Ser Val Thr Tyr Leu Phe Lys Asn Met Leu Pro
        835                 840                 845

Lys Val Ile Asp Glu Leu Asn Lys Phe Asp Leu Arg Thr Lys Thr Glu
    850                 855                 860

Leu Ile Asn Leu Ile Asp Ser His Asn Ile Ile Leu Val Gly Glu Val
865                 870                 875                 880

Asp Arg Leu Lys Ala Lys Val Asn Glu Ser Phe Glu Asn Thr Met Pro
                885                 890                 895

Phe Asn Ile Phe Ser Tyr Thr Asn Asn Ser Leu Leu Lys Asp Ile Ile
            900                 905                 910

Asn Glu Tyr Phe Asn
        915

<210> 63
<211> 932
<212> PRT
<213> Artificial Sequence

<220>
<221> source
<223> /note="Description of Artificial Sequence: Synthetic
      polypeptide"

<400> 63
Thr Trp Pro Val Lys Asp Phe Asn Tyr Ser Asp Pro Val Asn Asp Asn
1               5                   10                  15

Asp Ile Leu Tyr Leu Arg Ile Pro Gln Asn Lys Leu Ile Thr Thr Pro
            20                  25                  30

Val Lys Ala Phe Met Ile Thr Gln Asn Ile Trp Val Ile Pro Glu Arg
        35                  40                  45

Phe Ser Ser Asp Thr Asn Pro Ser Leu Ser Lys Pro Pro Arg Pro Thr
    50                  55                  60

Ser Lys Tyr Gln Ser Tyr Tyr Asp Pro Ser Tyr Leu Ser Thr Asp Glu
65                  70                  75                  80

Gln Lys Asp Thr Phe Leu Lys Gly Ile Ile Lys Leu Phe Lys Arg Ile
                85                  90                  95

Asn Glu Arg Asp Ile Gly Lys Lys Leu Ile Asn Tyr Leu Val Val Gly
            100                 105                 110

Ser Pro Phe Met Gly Asp Ser Ser Thr Pro Glu Asp Thr Phe Asp Phe
        115                 120                 125

Thr Arg His Thr Thr Asn Ile Ala Val Glu Lys Phe Glu Asn Gly Ser
    130                 135                 140

Trp Lys Val Thr Asn Ile Ile Thr Pro Ser Val Leu Ile Phe Gly Pro
145                 150                 155                 160

Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
225                 230                 235                 240

Asn Ile Pro Ser Asp Lys Arg Ile Arg Pro Gln Val Ser Glu Gly Phe
                245                 250                 255

Phe Ser Gln Asp Gly Pro Asn Val Gln Phe Glu Glu Leu Tyr Thr Phe
            260                 265                 270

Gly Gly Leu Asp Val Glu Ile Ile Pro Gln Ile Glu Arg Ser Gln Leu
        275                 280                 285

Arg Glu Lys Ala Leu Gly His Tyr Lys Asp Ile Ala Lys Arg Leu Asn
    290                 295                 300

Asn Ile Asn Lys Thr Ile Pro Ser Ser Trp Ile Ser Asn Ile Asp Lys
305                 310                 315                 320

Tyr Lys Lys Ile Phe Ser Glu Lys Tyr Asn Phe Asp Lys Asp Asn Thr
                325                 330                 335

Gly Asn Phe Val Val Asn Ile Asp Lys Phe Asn Ser Leu Tyr Ser Asp

340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Tyr Ala Asp Ala Ile Phe Thr Asn Ser Tyr Arg Lys
450 455 460

Val Leu Gly Gln Leu Ser Ala Arg Lys Leu Leu Gln Asp Ile Met Ser
465 470 475 480

Arg Gln Gln Gly Glu Ser Asn Gln Glu Arg Gly Ala Arg Ala Arg Leu
485 490 495

Ala Leu Ala Gly Gly Gly Gly Ser Gly Gly Gly Gly Ser Gly Gly Gly
500 505 510

Gly Ser Ala Leu Ala Leu Gln Cys Ile Lys Val Lys Asn Asn Arg Leu
515 520 525

Pro Tyr Val Ala Asp Lys Asp Ser Ile Ser Gln Glu Ile Phe Glu Asn
530 535 540

Lys Ile Ile Thr Asp Glu Thr Asn Val Gln Asn Tyr Ser Asp Lys Phe
545 550 555 560

Ser Leu Asp Glu Ser Ile Leu Asp Gly Gln Val Pro Ile Asn Pro Glu
565 570 575

Ile Val Asp Pro Leu Leu Pro Asn Val Asn Met Glu Pro Leu Asn Leu
580 585 590

```
Pro Gly Glu Glu Ile Val Phe Tyr Asp Asp Ile Thr Lys Tyr Val Asp
        595                 600                 605

Tyr Leu Asn Ser Tyr Tyr Tyr Leu Glu Ser Gln Lys Leu Ser Asn Asn
    610                 615                 620

Val Glu Asn Ile Thr Leu Thr Thr Ser Val Glu Glu Ala Leu Gly Tyr
625                 630                 635                 640

Ser Asn Lys Ile Tyr Thr Phe Leu Pro Ser Leu Ala Glu Lys Val Asn
                645                 650                 655

Lys Gly Val Gln Ala Gly Leu Phe Leu Asn Trp Ala Asn Glu Val Val
            660                 665                 670

Glu Asp Phe Thr Thr Asn Ile Met Lys Lys Asp Thr Leu Asp Lys Ile
        675                 680                 685

Ser Asp Val Ser Val Ile Ile Pro Tyr Ile Gly Pro Ala Leu Asn Ile
    690                 695                 700

Gly Asn Ser Ala Leu Arg Gly Asn Phe Asn Gln Ala Phe Ala Thr Ala
705                 710                 715                 720

Gly Val Ala Phe Leu Leu Glu Gly Phe Pro Glu Phe Thr Ile Pro Ala
                725                 730                 735

Leu Gly Val Phe Thr Phe Tyr Ser Ser Ile Gln Glu Arg Glu Lys Ile
            740                 745                 750

Ile Lys Thr Ile Glu Asn Cys Leu Glu Gln Arg Val Lys Arg Trp Lys
        755                 760                 765

Asp Ser Tyr Gln Trp Met Val Ser Asn Trp Leu Ser Arg Ile Thr Thr
    770                 775                 780

Gln Phe Asn His Ile Asn Tyr Gln Met Tyr Asp Ser Leu Ser Tyr Gln
785                 790                 795                 800

Ala Asp Ala Ile Lys Ala Lys Ile Asp Leu Glu Tyr Lys Lys Tyr Ser
                805                 810                 815

Gly Ser Asp Lys Glu Asn Ile Lys Ser Gln Val Glu Asn Leu Lys Asn
            820                 825                 830

Ser Leu Asp Val Lys Ile Ser Glu Ala Met Asn Asn Ile Asn Lys Phe
        835                 840                 845
```

Ile Arg Glu Cys Ser Val Thr Tyr Leu Phe Lys Asn Met Leu Pro Lys
850 855 860

Val Ile Asp Glu Leu Asn Lys Phe Asp Leu Arg Thr Lys Thr Glu Leu
865 870 875 880

Ile Asn Leu Ile Asp Ser His Asn Ile Ile Leu Val Gly Glu Val Asp
885 890 895

Arg Leu Lys Ala Lys Val Asn Glu Ser Phe Glu Asn Thr Met Pro Phe
900 905 910

Asn Ile Phe Ser Tyr Thr Asn Asn Ser Leu Leu Lys Asp Ile Ile Asn
915 920 925

Glu Tyr Phe Asn
930

<210> 64
<211> 927
<212> PRT
<213> Artificial Sequence

<220>
<221> source
<223> /note="Description of Artificial Sequence: Synthetic polypeptide"

<400> 64
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

```
Lys Asn Arg Thr His Tyr Phe Ser Arg His Tyr Leu Pro Val Phe Ala
    370                 375                 380

Asn Ile Leu Asp Asp Asn Ile Tyr Thr Ile Arg Asp Gly Phe Asn Leu
385                 390                 395                 400

Thr Asn Lys Gly Phe Asn Ile Glu Asn Ser Gly Gln Asn Ile Glu Arg
                405                 410                 415

Asn Pro Ala Leu Gln Lys Leu Ser Ser Glu Ser Val Val Asp Leu Phe
            420                 425                 430

Thr Lys Val Cys Val Asp Gly Ile Ile Thr Ser Lys Thr Lys Ser Asp
        435                 440                 445

Asp Asp Asp Lys Tyr Ala Asp Ala Ile Phe Thr Asn Ser Tyr Arg Lys
    450                 455                 460

Val Leu Gly Gln Leu Ser Ala Arg Lys Leu Leu Gln Asp Ile Met Ser
465                 470                 475                 480

Arg Gln Gln Gly Glu Ser Asn Gln Glu Arg Gly Ala Leu Ala Gly Gly
                485                 490                 495

Gly Gly Ser Gly Gly Gly Gly Ser Gly Gly Gly Gly Ser Ala Leu Ala
            500                 505                 510

Leu Gln Cys Ile Lys Val Lys Asn Asn Arg Leu Pro Tyr Val Ala Asp
        515                 520                 525

Lys Asp Ser Ile Ser Gln Glu Ile Phe Glu Asn Lys Ile Ile Thr Asp
    530                 535                 540

Glu Thr Asn Val Gln Asn Tyr Ser Asp Lys Phe Ser Leu Asp Glu Ser
545                 550                 555                 560

Ile Leu Asp Gly Gln Val Pro Ile Asn Pro Glu Ile Val Asp Pro Leu
                565                 570                 575

Leu Pro Asn Val Asn Met Glu Pro Leu Asn Leu Pro Gly Glu Glu Ile
            580                 585                 590

Val Phe Tyr Asp Asp Ile Thr Lys Tyr Val Asp Tyr Leu Asn Ser Tyr
        595                 600                 605

Tyr Tyr Leu Glu Ser Gln Lys Leu Ser Asn Asn Val Glu Asn Ile Thr
```

610 615 620

Leu Thr Thr Ser Val Glu Glu Ala Leu Gly Tyr Ser Asn Lys Ile Tyr
625 630 635 640

Thr Phe Leu Pro Ser Leu Ala Glu Lys Val Asn Lys Gly Val Gln Ala
645 650 655

Gly Leu Phe Leu Asn Trp Ala Asn Glu Val Val Glu Asp Phe Thr Thr
660 665 670

Asn Ile Met Lys Lys Asp Thr Leu Asp Lys Ile Ser Asp Val Ser Val
675 680 685

Ile Ile Pro Tyr Ile Gly Pro Ala Leu Asn Ile Gly Asn Ser Ala Leu
690 695 700

Arg Gly Asn Phe Asn Gln Ala Phe Ala Thr Ala Gly Val Ala Phe Leu
705 710 715 720

Leu Glu Gly Phe Pro Glu Phe Thr Ile Pro Ala Leu Gly Val Phe Thr
725 730 735

Phe Tyr Ser Ser Ile Gln Glu Arg Glu Lys Ile Ile Lys Thr Ile Glu
740 745 750

Asn Cys Leu Glu Gln Arg Val Lys Arg Trp Lys Asp Ser Tyr Gln Trp
755 760 765

Met Val Ser Asn Trp Leu Ser Arg Ile Thr Thr Gln Phe Asn His Ile
770 775 780

Asn Tyr Gln Met Tyr Asp Ser Leu Ser Tyr Gln Ala Asp Ala Ile Lys
785 790 795 800

Ala Lys Ile Asp Leu Glu Tyr Lys Lys Tyr Ser Gly Ser Asp Lys Glu
805 810 815

Asn Ile Lys Ser Gln Val Glu Asn Leu Lys Asn Ser Leu Asp Val Lys
820 825 830

Ile Ser Glu Ala Met Asn Asn Ile Asn Lys Phe Ile Arg Glu Cys Ser
835 840 845

Val Thr Tyr Leu Phe Lys Asn Met Leu Pro Lys Val Ile Asp Glu Leu
850 855 860

Asn Lys Phe Asp Leu Arg Thr Lys Thr Glu Leu Ile Asn Leu Ile Asp
865 870 875 880

Ser His Asn Ile Ile Leu Val Gly Glu Val Asp Arg Leu Lys Ala Lys
885 890 895

Val Asn Glu Ser Phe Glu Asn Thr Met Pro Phe Asn Ile Phe Ser Tyr
900 905 910

Thr Asn Asn Ser Leu Leu Lys Asp Ile Ile Asn Glu Tyr Phe Asn
915 920 925

<210> 65
<211> 917
<212> PRT
<213> Artificial Sequence

<220>
<221> source
<223> /note="Description of Artificial Sequence: Synthetic polypeptide"

<400> 65
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Tyr Ala Asp Ala Ile Phe Thr Asn Ala Tyr Arg Lys
450 455 460

Val Leu Gly Gln Leu Ser Ala Arg Lys Leu Leu Gln Asp Ile Met Ser
465 470 475 480

Arg Ala Leu Ala Gly Gly Gly Gly Ser Gly Gly Gly Gly Ser Gly Gly
485 490 495

Gly Gly Ser Ala Leu Ala Leu Gln Cys Ile Lys Val Lys Asn Asn Arg
500 505 510

Leu Pro Tyr Val Ala Asp Lys Asp Ser Ile Ser Gln Glu Ile Phe Glu
515 520 525

Asn Lys Ile Ile Thr Asp Glu Thr Asn Val Gln Asn Tyr Ser Asp Lys
530 535 540

Phe Ser Leu Asp Glu Ser Ile Leu Asp Gly Gln Val Pro Ile Asn Pro
545 550 555 560

Glu Ile Val Asp Pro Leu Leu Pro Asn Val Asn Met Glu Pro Leu Asn
565 570 575

Leu Pro Gly Glu Glu Ile Val Phe Tyr Asp Asp Ile Thr Lys Tyr Val
580 585 590

Asp Tyr Leu Asn Ser Tyr Tyr Tyr Leu Glu Ser Gln Lys Leu Ser Asn
595 600 605

Asn Val Glu Asn Ile Thr Leu Thr Thr Ser Val Glu Glu Ala Leu Gly
610 615 620

Tyr Ser Asn Lys Ile Tyr Thr Phe Leu Pro Ser Leu Ala Glu Lys Val
625 630 635 640

Asn Lys Gly Val Gln Ala Gly Leu Phe Leu Asn Trp Ala Asn Glu Val
645 650 655

Val Glu Asp Phe Thr Thr Asn Ile Met Lys Lys Asp Thr Leu Asp Lys
660 665 670

Ile Ser Asp Val Ser Val Ile Ile Pro Tyr Ile Gly Pro Ala Leu Asn
675 680 685

Ile Gly Asn Ser Ala Leu Arg Gly Asn Phe Asn Gln Ala Phe Ala Thr
690 695 700

Ala Gly Val Ala Phe Leu Leu Glu Gly Phe Pro Glu Phe Thr Ile Pro
705 710 715 720

Ala Leu Gly Val Phe Thr Phe Tyr Ser Ser Ile Gln Glu Arg Glu Lys
725 730 735

Ile Ile Lys Thr Ile Glu Asn Cys Leu Glu Gln Arg Val Lys Arg Trp
740 745 750

Lys Asp Ser Tyr Gln Trp Met Val Ser Asn Trp Leu Ser Arg Ile Thr
755 760 765

Thr Gln Phe Asn His Ile Asn Tyr Gln Met Tyr Asp Ser Leu Ser Tyr
770 775 780

Gln Ala Asp Ala Ile Lys Ala Lys Ile Asp Leu Glu Tyr Lys Lys Tyr
785 790 795 800

Ser Gly Ser Asp Lys Glu Asn Ile Lys Ser Gln Val Glu Asn Leu Lys
805 810 815

Asn Ser Leu Asp Val Lys Ile Ser Glu Ala Met Asn Asn Ile Asn Lys
820 825 830

Phe Ile Arg Glu Cys Ser Val Thr Tyr Leu Phe Lys Asn Met Leu Pro
835 840 845

Lys Val Ile Asp Glu Leu Asn Lys Phe Asp Leu Arg Thr Lys Thr Glu
850 855 860

Leu Ile Asn Leu Ile Asp Ser His Asn Ile Ile Leu Val Gly Glu Val
865 870 875 880

Asp Arg Leu Lys Ala Lys Val Asn Glu Ser Phe Glu Asn Thr Met Pro
885 890 895

Phe Asn Ile Phe Ser Tyr Thr Asn Asn Ser Leu Leu Lys Asp Ile Ile

900 905 910

Asn Glu Tyr Phe Asn
915

<210> 66
<211> 917
<212> PRT
<213> Artificial Sequence

<220>
<221> source
<223> /note="Description of Artificial Sequence: Synthetic polypeptide"

<400> 66
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys

180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Tyr Ala Asp Ala Ile Phe Thr Asn Ser Tyr Arg Lys
450 455 460

Val Leu Gly Gln Leu Ser Ala Arg Lys Ala Leu Gln Asp Ile Met Ser
465 470 475 480

Arg Ala Leu Ala Gly Gly Gly Gly Ser Gly Gly Gly Gly Ser Gly Gly
485 490 495

Gly Gly Ser Ala Leu Ala Leu Gln Cys Ile Lys Val Lys Asn Asn Arg
500 505 510

Leu Pro Tyr Val Ala Asp Lys Asp Ser Ile Ser Gln Glu Ile Phe Glu
515 520 525

Asn Lys Ile Ile Thr Asp Glu Thr Asn Val Gln Asn Tyr Ser Asp Lys
530 535 540

Phe Ser Leu Asp Glu Ser Ile Leu Asp Gly Gln Val Pro Ile Asn Pro
545 550 555 560

Glu Ile Val Asp Pro Leu Leu Pro Asn Val Asn Met Glu Pro Leu Asn
565 570 575

Leu Pro Gly Glu Glu Ile Val Phe Tyr Asp Asp Ile Thr Lys Tyr Val
580 585 590

Asp Tyr Leu Asn Ser Tyr Tyr Tyr Leu Glu Ser Gln Lys Leu Ser Asn
595 600 605

Asn Val Glu Asn Ile Thr Leu Thr Thr Ser Val Glu Glu Ala Leu Gly
610 615 620

Tyr Ser Asn Lys Ile Tyr Thr Phe Leu Pro Ser Leu Ala Glu Lys Val
625 630 635 640

Asn Lys Gly Val Gln Ala Gly Leu Phe Leu Asn Trp Ala Asn Glu Val
645 650 655

Val Glu Asp Phe Thr Thr Asn Ile Met Lys Lys Asp Thr Leu Asp Lys
660 665 670

Ile Ser Asp Val Ser Val Ile Ile Pro Tyr Ile Gly Pro Ala Leu Asn
675 680 685

```
Ile Gly Asn Ser Ala Leu Arg Gly Asn Phe Asn Gln Ala Phe Ala Thr
    690                 695                 700

Ala Gly Val Ala Phe Leu Leu Glu Gly Phe Pro Glu Phe Thr Ile Pro
705                 710                 715                 720

Ala Leu Gly Val Phe Thr Phe Tyr Ser Ser Ile Gln Glu Arg Glu Lys
                725                 730                 735

Ile Ile Lys Thr Ile Glu Asn Cys Leu Glu Gln Arg Val Lys Arg Trp
            740                 745                 750

Lys Asp Ser Tyr Gln Trp Met Val Ser Asn Trp Leu Ser Arg Ile Thr
        755                 760                 765

Thr Gln Phe Asn His Ile Asn Tyr Gln Met Tyr Asp Ser Leu Ser Tyr
    770                 775                 780

Gln Ala Asp Ala Ile Lys Ala Lys Ile Asp Leu Glu Tyr Lys Lys Tyr
785                 790                 795                 800

Ser Gly Ser Asp Lys Glu Asn Ile Lys Ser Gln Val Glu Asn Leu Lys
                805                 810                 815

Asn Ser Leu Asp Val Lys Ile Ser Glu Ala Met Asn Asn Ile Asn Lys
            820                 825                 830

Phe Ile Arg Glu Cys Ser Val Thr Tyr Leu Phe Lys Asn Met Leu Pro
        835                 840                 845

Lys Val Ile Asp Glu Leu Asn Lys Phe Asp Leu Arg Thr Lys Thr Glu
    850                 855                 860

Leu Ile Asn Leu Ile Asp Ser His Asn Ile Ile Leu Val Gly Glu Val
865                 870                 875                 880

Asp Arg Leu Lys Ala Lys Val Asn Glu Ser Phe Glu Asn Thr Met Pro
                885                 890                 895

Phe Asn Ile Phe Ser Tyr Thr Asn Asn Ser Leu Leu Lys Asp Ile Ile
            900                 905                 910

Asn Glu Tyr Phe Asn
        915

<210> 67
<211> 917
```

<212> PRT
<213> Artificial Sequence

<220>
<221> source
<223> /note="Description of Artificial Sequence: Synthetic polypeptide"

<400> 67
Thr Trp Pro Val Lys Asp Phe Asn Tyr Ser Asp Pro Val Asn Asp Asn
1               5                   10                  15

Asp Ile Leu Tyr Leu Arg Ile Pro Gln Asn Lys Leu Ile Thr Thr Pro
            20                  25                  30

Val Lys Ala Phe Met Ile Thr Gln Asn Ile Trp Val Ile Pro Glu Arg
        35                  40                  45

Phe Ser Ser Asp Thr Asn Pro Ser Leu Ser Lys Pro Pro Arg Pro Thr
    50                  55                  60

Ser Lys Tyr Gln Ser Tyr Tyr Asp Pro Ser Tyr Leu Ser Thr Asp Glu
65                  70                  75                  80

Gln Lys Asp Thr Phe Leu Lys Gly Ile Ile Lys Leu Phe Lys Arg Ile
                85                  90                  95

Asn Glu Arg Asp Ile Gly Lys Lys Leu Ile Asn Tyr Leu Val Val Gly
            100                 105                 110

Ser Pro Phe Met Gly Asp Ser Ser Thr Pro Glu Asp Thr Phe Asp Phe
        115                 120                 125

Thr Arg His Thr Thr Asn Ile Ala Val Glu Lys Phe Glu Asn Gly Ser
    130                 135                 140

Trp Lys Val Thr Asn Ile Ile Thr Pro Ser Val Leu Ile Phe Gly Pro
145                 150                 155                 160

Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Tyr Ala Asp Ala Ile Phe Thr Ala Ser Tyr Lys Lys
450 455 460

Val Leu Gly Gln Leu Ser Ala Arg Lys Leu Leu Gln Asp Ile Met Ser

465 470 475 480

Arg Ala Leu Ala Gly Gly Gly Gly Ser Gly Gly Gly Gly Ser Gly Gly
485 490 495

Gly Gly Ser Ala Leu Ala Leu Gln Cys Ile Lys Val Lys Asn Asn Arg
500 505 510

Leu Pro Tyr Val Ala Asp Lys Asp Ser Ile Ser Gln Glu Ile Phe Glu
515 520 525

Asn Lys Ile Ile Thr Asp Glu Thr Asn Val Gln Asn Tyr Ser Asp Lys
530 535 540

Phe Ser Leu Asp Glu Ser Ile Leu Asp Gly Gln Val Pro Ile Asn Pro
545 550 555 560

Glu Ile Val Asp Pro Leu Leu Pro Asn Val Asn Met Glu Pro Leu Asn
565 570 575

Leu Pro Gly Glu Glu Ile Val Phe Tyr Asp Asp Ile Thr Lys Tyr Val
580 585 590

Asp Tyr Leu Asn Ser Tyr Tyr Tyr Leu Glu Ser Gln Lys Leu Ser Asn
595 600 605

Asn Val Glu Asn Ile Thr Leu Thr Thr Ser Val Glu Glu Ala Leu Gly
610 615 620

Tyr Ser Asn Lys Ile Tyr Thr Phe Leu Pro Ser Leu Ala Glu Lys Val
625 630 635 640

Asn Lys Gly Val Gln Ala Gly Leu Phe Leu Asn Trp Ala Asn Glu Val
645 650 655

Val Glu Asp Phe Thr Thr Asn Ile Met Lys Lys Asp Thr Leu Asp Lys
660 665 670

Ile Ser Asp Val Ser Val Ile Ile Pro Tyr Ile Gly Pro Ala Leu Asn
675 680 685

Ile Gly Asn Ser Ala Leu Arg Gly Asn Phe Asn Gln Ala Phe Ala Thr
690 695 700

Ala Gly Val Ala Phe Leu Leu Glu Gly Phe Pro Glu Phe Thr Ile Pro
705 710 715 720

Ala Leu Gly Val Phe Thr Phe Tyr Ser Ser Ile Gln Glu Arg Glu Lys
725 730 735

Ile Ile Lys Thr Ile Glu Asn Cys Leu Glu Gln Arg Val Lys Arg Trp
740 745 750

Lys Asp Ser Tyr Gln Trp Met Val Ser Asn Trp Leu Ser Arg Ile Thr
755 760 765

Thr Gln Phe Asn His Ile Asn Tyr Gln Met Tyr Asp Ser Leu Ser Tyr
770 775 780

Gln Ala Asp Ala Ile Lys Ala Lys Ile Asp Leu Glu Tyr Lys Lys Tyr
785 790 795 800

Ser Gly Ser Asp Lys Glu Asn Ile Lys Ser Gln Val Glu Asn Leu Lys
805 810 815

Asn Ser Leu Asp Val Lys Ile Ser Glu Ala Met Asn Asn Ile Asn Lys
820 825 830

Phe Ile Arg Glu Cys Ser Val Thr Tyr Leu Phe Lys Asn Met Leu Pro
835 840 845

Lys Val Ile Asp Glu Leu Asn Lys Phe Asp Leu Arg Thr Lys Thr Glu
850 855 860

Leu Ile Asn Leu Ile Asp Ser His Asn Ile Ile Leu Val Gly Glu Val
865 870 875 880

Asp Arg Leu Lys Ala Lys Val Asn Glu Ser Phe Glu Asn Thr Met Pro
885 890 895

Phe Asn Ile Phe Ser Tyr Thr Asn Asn Ser Leu Leu Lys Asp Ile Ile
900 905 910

Asn Glu Tyr Phe Asn
915

<210> 68
<211> 917
<212> PRT
<213> Artificial Sequence

<220>
<221> source
<223> /note="Description of Artificial Sequence: Synthetic polypeptide"

<400> 68

```
Thr Trp Pro Val Lys Asp Phe Asn Tyr Ser Asp Pro Val Asn Asp Asn
1               5                   10                  15

Asp Ile Leu Tyr Leu Arg Ile Pro Gln Asn Lys Leu Ile Thr Thr Pro
            20                  25                  30

Val Lys Ala Phe Met Ile Thr Gln Asn Ile Trp Val Ile Pro Glu Arg
        35                  40                  45

Phe Ser Ser Asp Thr Asn Pro Ser Leu Ser Lys Pro Pro Arg Pro Thr
    50                  55                  60

Ser Lys Tyr Gln Ser Tyr Tyr Asp Pro Ser Tyr Leu Ser Thr Asp Glu
65                  70                  75                  80

Gln Lys Asp Thr Phe Leu Lys Gly Ile Ile Lys Leu Phe Lys Arg Ile
                85                  90                  95

Asn Glu Arg Asp Ile Gly Lys Lys Leu Ile Asn Tyr Leu Val Val Gly
            100                 105                 110

Ser Pro Phe Met Gly Asp Ser Ser Thr Pro Glu Asp Thr Phe Asp Phe
        115                 120                 125

Thr Arg His Thr Thr Asn Ile Ala Val Glu Lys Phe Glu Asn Gly Ser
    130                 135                 140

Trp Lys Val Thr Asn Ile Ile Thr Pro Ser Val Leu Ile Phe Gly Pro
145                 150                 155                 160

Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
225                 230                 235                 240

Asn Ile Pro Ser Asp Lys Arg Ile Arg Pro Gln Val Ser Glu Gly Phe
                245                 250                 255
```

```
Phe Ser Gln Asp Gly Pro Asn Val Gln Phe Glu Glu Leu Tyr Thr Phe
            260                 265                 270

Gly Gly Leu Asp Val Glu Ile Ile Pro Gln Ile Glu Arg Ser Gln Leu
        275                 280                 285

Arg Glu Lys Ala Leu Gly His Tyr Lys Asp Ile Ala Lys Arg Leu Asn
    290                 295                 300

Asn Ile Asn Lys Thr Ile Pro Ser Ser Trp Ile Ser Asn Ile Asp Lys
305                 310                 315                 320

Tyr Lys Lys Ile Phe Ser Glu Lys Tyr Asn Phe Asp Lys Asp Asn Thr
                325                 330                 335

Gly Asn Phe Val Val Asn Ile Asp Lys Phe Asn Ser Leu Tyr Ser Asp
            340                 345                 350

Leu Thr Asn Val Met Ser Glu Val Val Tyr Ser Ser Gln Tyr Asn Val
        355                 360                 365

Lys Asn Arg Thr His Tyr Phe Ser Arg His Tyr Leu Pro Val Phe Ala
    370                 375                 380

Asn Ile Leu Asp Asp Asn Ile Tyr Thr Ile Arg Asp Gly Phe Asn Leu
385                 390                 395                 400

Thr Asn Lys Gly Phe Asn Ile Glu Asn Ser Gly Gln Asn Ile Glu Arg
                405                 410                 415

Asn Pro Ala Leu Gln Lys Leu Ser Ser Glu Ser Val Val Asp Leu Phe
            420                 425                 430

Thr Lys Val Cys Val Asp Gly Ile Ile Thr Ser Lys Thr Lys Ser Asp
        435                 440                 445

Asp Asp Asp Lys Tyr Ala Asp Ala Ile Phe Thr Ala Ser Tyr Lys Arg
    450                 455                 460

Val Leu Gly Gln Leu Ser Ala Arg Lys Leu Leu Gln Asp Ile Met Ser
465                 470                 475                 480

Arg Ala Leu Ala Gly Gly Gly Gly Ser Gly Gly Gly Gly Ser Gly Gly
                485                 490                 495

Gly Gly Ser Ala Leu Ala Leu Gln Cys Ile Lys Val Lys Asn Asn Arg
            500                 505                 510
```

```
Leu Pro Tyr Val Ala Asp Lys Asp Ser Ile Ser Gln Glu Ile Phe Glu
        515                 520                 525

Asn Lys Ile Ile Thr Asp Glu Thr Asn Val Gln Asn Tyr Ser Asp Lys
    530                 535                 540

Phe Ser Leu Asp Glu Ser Ile Leu Asp Gly Gln Val Pro Ile Asn Pro
545                 550                 555                 560

Glu Ile Val Asp Pro Leu Leu Pro Asn Val Asn Met Glu Pro Leu Asn
                565                 570                 575

Leu Pro Gly Glu Glu Ile Val Phe Tyr Asp Asp Ile Thr Lys Tyr Val
            580                 585                 590

Asp Tyr Leu Asn Ser Tyr Tyr Tyr Leu Glu Ser Gln Lys Leu Ser Asn
        595                 600                 605

Asn Val Glu Asn Ile Thr Leu Thr Thr Ser Val Glu Glu Ala Leu Gly
    610                 615                 620

Tyr Ser Asn Lys Ile Tyr Thr Phe Leu Pro Ser Leu Ala Glu Lys Val
625                 630                 635                 640

Asn Lys Gly Val Gln Ala Gly Leu Phe Leu Asn Trp Ala Asn Glu Val
                645                 650                 655

Val Glu Asp Phe Thr Thr Asn Ile Met Lys Lys Asp Thr Leu Asp Lys
            660                 665                 670

Ile Ser Asp Val Ser Val Ile Ile Pro Tyr Ile Gly Pro Ala Leu Asn
        675                 680                 685

Ile Gly Asn Ser Ala Leu Arg Gly Asn Phe Asn Gln Ala Phe Ala Thr
    690                 695                 700

Ala Gly Val Ala Phe Leu Leu Glu Gly Phe Pro Glu Phe Thr Ile Pro
705                 710                 715                 720

Ala Leu Gly Val Phe Thr Phe Tyr Ser Ser Ile Gln Glu Arg Glu Lys
                725                 730                 735

Ile Ile Lys Thr Ile Glu Asn Cys Leu Glu Gln Arg Val Lys Arg Trp
            740                 745                 750

Lys Asp Ser Tyr Gln Trp Met Val Ser Asn Trp Leu Ser Arg Ile Thr
```

755 760 765

Thr Gln Phe Asn His Ile Asn Tyr Gln Met Tyr Asp Ser Leu Ser Tyr
770 775 780

Gln Ala Asp Ala Ile Lys Ala Lys Ile Asp Leu Glu Tyr Lys Lys Tyr
785 790 795 800

Ser Gly Ser Asp Lys Glu Asn Ile Lys Ser Gln Val Glu Asn Leu Lys
805 810 815

Asn Ser Leu Asp Val Lys Ile Ser Glu Ala Met Asn Asn Ile Asn Lys
820 825 830

Phe Ile Arg Glu Cys Ser Val Thr Tyr Leu Phe Lys Asn Met Leu Pro
835 840 845

Lys Val Ile Asp Glu Leu Asn Lys Phe Asp Leu Arg Thr Lys Thr Glu
850 855 860

Leu Ile Asn Leu Ile Asp Ser His Asn Ile Ile Leu Val Gly Glu Val
865 870 875 880

Asp Arg Leu Lys Ala Lys Val Asn Glu Ser Phe Glu Asn Thr Met Pro
885 890 895

Phe Asn Ile Phe Ser Tyr Thr Asn Asn Ser Leu Leu Lys Asp Ile Ile
900 905 910

Asn Glu Tyr Phe Asn
915

<210> 69
<211> 917
<212> PRT
<213> Artificial Sequence

<220>
<221> source
<223> /note="Description of Artificial Sequence: Synthetic polypeptide"

<400> 69
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg

35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Tyr Ala Asp Ala Ile Phe Thr Ala Ser Tyr Asn Lys
450 455 460

Val Leu Gly Gln Leu Ser Ala Arg Lys Leu Leu Gln Asp Ile Met Ser
465 470 475 480

Arg Ala Leu Ala Gly Gly Gly Gly Ser Gly Gly Gly Gly Ser Gly Gly
485 490 495

Gly Gly Ser Ala Leu Ala Leu Gln Cys Ile Lys Val Lys Asn Asn Arg
500 505 510

Leu Pro Tyr Val Ala Asp Lys Asp Ser Ile Ser Gln Glu Ile Phe Glu
515 520 525

Asn Lys Ile Ile Thr Asp Glu Thr Asn Val Gln Asn Tyr Ser Asp Lys
530 535 540

Phe Ser Leu Asp Glu Ser Ile Leu Asp Gly Gln Val Pro Ile Asn Pro
545 550 555 560

Glu Ile Val Asp Pro Leu Leu Pro Asn Val Asn Met Glu Pro Leu Asn
565 570 575

Leu Pro Gly Glu Glu Ile Val Phe Tyr Asp Asp Ile Thr Lys Tyr Val
580 585 590

Asp Tyr Leu Asn Ser Tyr Tyr Tyr Leu Glu Ser Gln Lys Leu Ser Asn
595 600 605

Asn Val Glu Asn Ile Thr Leu Thr Thr Ser Val Glu Glu Ala Leu Gly
610 615 620

Tyr Ser Asn Lys Ile Tyr Thr Phe Leu Pro Ser Leu Ala Glu Lys Val
625 630 635 640

Asn Lys Gly Val Gln Ala Gly Leu Phe Leu Asn Trp Ala Asn Glu Val
645 650 655

Val Glu Asp Phe Thr Thr Asn Ile Met Lys Lys Asp Thr Leu Asp Lys
660 665 670

Ile Ser Asp Val Ser Val Ile Ile Pro Tyr Ile Gly Pro Ala Leu Asn
675 680 685

Ile Gly Asn Ser Ala Leu Arg Gly Asn Phe Asn Gln Ala Phe Ala Thr
690 695 700

Ala Gly Val Ala Phe Leu Leu Glu Gly Phe Pro Glu Phe Thr Ile Pro
705 710 715 720

Ala Leu Gly Val Phe Thr Phe Tyr Ser Ser Ile Gln Glu Arg Glu Lys
725 730 735

Ile Ile Lys Thr Ile Glu Asn Cys Leu Glu Gln Arg Val Lys Arg Trp
740 745 750

Lys Asp Ser Tyr Gln Trp Met Val Ser Asn Trp Leu Ser Arg Ile Thr
755 760 765

Thr Gln Phe Asn His Ile Asn Tyr Gln Met Tyr Asp Ser Leu Ser Tyr
770 775 780

Gln Ala Asp Ala Ile Lys Ala Lys Ile Asp Leu Glu Tyr Lys Lys Tyr
785 790 795 800

Ser Gly Ser Asp Lys Glu Asn Ile Lys Ser Gln Val Glu Asn Leu Lys
805 810 815

Asn Ser Leu Asp Val Lys Ile Ser Glu Ala Met Asn Asn Ile Asn Lys
820 825 830

Phe Ile Arg Glu Cys Ser Val Thr Tyr Leu Phe Lys Asn Met Leu Pro
835 840 845

Lys Val Ile Asp Glu Leu Asn Lys Phe Asp Leu Arg Thr Lys Thr Glu
850 855 860

Leu Ile Asn Leu Ile Asp Ser His Asn Ile Ile Leu Val Gly Glu Val
865 870 875 880

Asp Arg Leu Lys Ala Lys Val Asn Glu Ser Phe Glu Asn Thr Met Pro
885 890 895

Phe Asn Ile Phe Ser Tyr Thr Asn Asn Ser Leu Leu Lys Asp Ile Ile
900 905 910

Asn Glu Tyr Phe Asn
915

<210> 70
<211> 917
<212> PRT
<213> Artificial Sequence

<220>
<221> source
<223> /note="Description of Artificial Sequence: Synthetic polypeptide"

<400> 70
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr

325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Tyr Ala Asp Ala Ile Phe Thr Ala Ser Tyr Arg Lys
450 455 460

Val Leu Gly Gln Leu Ser Ala Lys Lys Leu Leu Gln Asp Ile Met Ser
465 470 475 480

Arg Ala Leu Ala Gly Gly Gly Gly Ser Gly Gly Gly Gly Ser Gly Gly
485 490 495

Gly Gly Ser Ala Leu Ala Leu Gln Cys Ile Lys Val Lys Asn Asn Arg
500 505 510

Leu Pro Tyr Val Ala Asp Lys Asp Ser Ile Ser Gln Glu Ile Phe Glu
515 520 525

Asn Lys Ile Ile Thr Asp Glu Thr Asn Val Gln Asn Tyr Ser Asp Lys
530 535 540

Phe Ser Leu Asp Glu Ser Ile Leu Asp Gly Gln Val Pro Ile Asn Pro
545 550 555 560

Glu Ile Val Asp Pro Leu Leu Pro Asn Val Asn Met Glu Pro Leu Asn
565 570 575

Leu Pro Gly Glu Glu Ile Val Phe Tyr Asp Asp Ile Thr Lys Tyr Val
580 585 590

Asp Tyr Leu Asn Ser Tyr Tyr Tyr Leu Glu Ser Gln Lys Leu Ser Asn
595 600 605

Asn Val Glu Asn Ile Thr Leu Thr Thr Ser Val Glu Glu Ala Leu Gly
610 615 620

Tyr Ser Asn Lys Ile Tyr Thr Phe Leu Pro Ser Leu Ala Glu Lys Val
625 630 635 640

Asn Lys Gly Val Gln Ala Gly Leu Phe Leu Asn Trp Ala Asn Glu Val
645 650 655

Val Glu Asp Phe Thr Thr Asn Ile Met Lys Lys Asp Thr Leu Asp Lys
660 665 670

Ile Ser Asp Val Ser Val Ile Ile Pro Tyr Ile Gly Pro Ala Leu Asn
675 680 685

Ile Gly Asn Ser Ala Leu Arg Gly Asn Phe Asn Gln Ala Phe Ala Thr
690 695 700

Ala Gly Val Ala Phe Leu Leu Glu Gly Phe Pro Glu Phe Thr Ile Pro
705 710 715 720

Ala Leu Gly Val Phe Thr Phe Tyr Ser Ser Ile Gln Glu Arg Glu Lys
725 730 735

Ile Ile Lys Thr Ile Glu Asn Cys Leu Glu Gln Arg Val Lys Arg Trp
740 745 750

Lys Asp Ser Tyr Gln Trp Met Val Ser Asn Trp Leu Ser Arg Ile Thr
755 760 765

Thr Gln Phe Asn His Ile Asn Tyr Gln Met Tyr Asp Ser Leu Ser Tyr
770 775 780

Gln Ala Asp Ala Ile Lys Ala Lys Ile Asp Leu Glu Tyr Lys Lys Tyr
785 790 795 800

Ser Gly Ser Asp Lys Glu Asn Ile Lys Ser Gln Val Glu Asn Leu Lys
805 810 815

Asn Ser Leu Asp Val Lys Ile Ser Glu Ala Met Asn Asn Ile Asn Lys
820 825 830

Phe Ile Arg Glu Cys Ser Val Thr Tyr Leu Phe Lys Asn Met Leu Pro
        835                 840                 845

Lys Val Ile Asp Glu Leu Asn Lys Phe Asp Leu Arg Thr Lys Thr Glu
    850                 855                 860

Leu Ile Asn Leu Ile Asp Ser His Asn Ile Ile Leu Val Gly Glu Val
865                 870                 875                 880

Asp Arg Leu Lys Ala Lys Val Asn Glu Ser Phe Glu Asn Thr Met Pro
                885                 890                 895

Phe Asn Ile Phe Ser Tyr Thr Asn Asn Ser Leu Leu Lys Asp Ile Ile
            900                 905                 910

Asn Glu Tyr Phe Asn
        915

<210> 71
<211> 917
<212> PRT
<213> Artificial Sequence

<220>
<221> source
<223> /note="Description of Artificial Sequence: Synthetic polypeptide"

<400> 71
Thr Trp Pro Val Lys Asp Phe Asn Tyr Ser Asp Pro Val Asn Asp Asn
1               5                   10                  15

Asp Ile Leu Tyr Leu Arg Ile Pro Gln Asn Lys Leu Ile Thr Thr Pro
            20                  25                  30

Val Lys Ala Phe Met Ile Thr Gln Asn Ile Trp Val Ile Pro Glu Arg
        35                  40                  45

Phe Ser Ser Asp Thr Asn Pro Ser Leu Ser Lys Pro Pro Arg Pro Thr
    50                  55                  60

Ser Lys Tyr Gln Ser Tyr Tyr Asp Pro Ser Tyr Leu Ser Thr Asp Glu
65                  70                  75                  80

Gln Lys Asp Thr Phe Leu Lys Gly Ile Ile Lys Leu Phe Lys Arg Ile
                85                  90                  95

Asn Glu Arg Asp Ile Gly Lys Lys Leu Ile Asn Tyr Leu Val Val Gly
            100                 105                 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Tyr Ala Asp Ala Ile Phe Thr Ala Ser Tyr Lys Lys
450 455 460

Val Leu Gly Gln Leu Ser Ala Lys Lys Leu Leu Gln Asp Ile Met Ser
465 470 475 480

Arg Ala Leu Ala Gly Gly Gly Gly Ser Gly Gly Gly Gly Ser Gly Gly
485 490 495

Gly Gly Ser Ala Leu Ala Leu Gln Cys Ile Lys Val Lys Asn Asn Arg
500 505 510

Leu Pro Tyr Val Ala Asp Lys Asp Ser Ile Ser Gln Glu Ile Phe Glu
515 520 525

Asn Lys Ile Ile Thr Asp Glu Thr Asn Val Gln Asn Tyr Ser Asp Lys
530 535 540

Phe Ser Leu Asp Glu Ser Ile Leu Asp Gly Gln Val Pro Ile Asn Pro
545 550 555 560

Glu Ile Val Asp Pro Leu Leu Pro Asn Val Asn Met Glu Pro Leu Asn
565 570 575

Leu Pro Gly Glu Glu Ile Val Phe Tyr Asp Asp Ile Thr Lys Tyr Val
580 585 590

Asp Tyr Leu Asn Ser Tyr Tyr Tyr Leu Glu Ser Gln Lys Leu Ser Asn
595 600 605

Asn Val Glu Asn Ile Thr Leu Thr Thr Ser Val Glu Glu Ala Leu Gly

610 615 620

Tyr Ser Asn Lys Ile Tyr Thr Phe Leu Pro Ser Leu Ala Glu Lys Val
625 630 635 640

Asn Lys Gly Val Gln Ala Gly Leu Phe Leu Asn Trp Ala Asn Glu Val
645 650 655

Val Glu Asp Phe Thr Thr Asn Ile Met Lys Lys Asp Thr Leu Asp Lys
660 665 670

Ile Ser Asp Val Ser Val Ile Ile Pro Tyr Ile Gly Pro Ala Leu Asn
675 680 685

Ile Gly Asn Ser Ala Leu Arg Gly Asn Phe Asn Gln Ala Phe Ala Thr
690 695 700

Ala Gly Val Ala Phe Leu Leu Glu Gly Phe Pro Glu Phe Thr Ile Pro
705 710 715 720

Ala Leu Gly Val Phe Thr Phe Tyr Ser Ser Ile Gln Glu Arg Glu Lys
725 730 735

Ile Ile Lys Thr Ile Glu Asn Cys Leu Glu Gln Arg Val Lys Arg Trp
740 745 750

Lys Asp Ser Tyr Gln Trp Met Val Ser Asn Trp Leu Ser Arg Ile Thr
755 760 765

Thr Gln Phe Asn His Ile Asn Tyr Gln Met Tyr Asp Ser Leu Ser Tyr
770 775 780

Gln Ala Asp Ala Ile Lys Ala Lys Ile Asp Leu Glu Tyr Lys Lys Tyr
785 790 795 800

Ser Gly Ser Asp Lys Glu Asn Ile Lys Ser Gln Val Glu Asn Leu Lys
805 810 815

Asn Ser Leu Asp Val Lys Ile Ser Glu Ala Met Asn Asn Ile Asn Lys
820 825 830

Phe Ile Arg Glu Cys Ser Val Thr Tyr Leu Phe Lys Asn Met Leu Pro
835 840 845

Lys Val Ile Asp Glu Leu Asn Lys Phe Asp Leu Arg Thr Lys Thr Glu
850 855 860

Leu Ile Asn Leu Ile Asp Ser His Asn Ile Ile Leu Val Gly Glu Val
865                 870                 875                 880

Asp Arg Leu Lys Ala Lys Val Asn Glu Ser Phe Glu Asn Thr Met Pro
                885                 890                 895

Phe Asn Ile Phe Ser Tyr Thr Asn Asn Ser Leu Leu Lys Asp Ile Ile
            900                 905                 910

Asn Glu Tyr Phe Asn
        915

<210> 72
<211> 917
<212> PRT
<213> Artificial Sequence

<220>
<221> source
<223> /note="Description of Artificial Sequence: Synthetic polypeptide"

<400> 72
Thr Trp Pro Val Lys Asp Phe Asn Tyr Ser Asp Pro Val Asn Asp Asn
1               5                   10                  15

Asp Ile Leu Tyr Leu Arg Ile Pro Gln Asn Lys Leu Ile Thr Thr Pro
            20                  25                  30

Val Lys Ala Phe Met Ile Thr Gln Asn Ile Trp Val Ile Pro Glu Arg
        35                  40                  45

Phe Ser Ser Asp Thr Asn Pro Ser Leu Ser Lys Pro Pro Arg Pro Thr
    50                  55                  60

Ser Lys Tyr Gln Ser Tyr Tyr Asp Pro Ser Tyr Leu Ser Thr Asp Glu
65                  70                  75                  80

Gln Lys Asp Thr Phe Leu Lys Gly Ile Ile Lys Leu Phe Lys Arg Ile
                85                  90                  95

Asn Glu Arg Asp Ile Gly Lys Lys Leu Ile Asn Tyr Leu Val Val Gly
            100                 105                 110

Ser Pro Phe Met Gly Asp Ser Ser Thr Pro Glu Asp Thr Phe Asp Phe
        115                 120                 125

Thr Arg His Thr Thr Asn Ile Ala Val Glu Lys Phe Glu Asn Gly Ser
    130                 135                 140

```
Trp Lys Val Thr Asn Ile Ile Thr Pro Ser Val Leu Ile Phe Gly Pro
145                 150                 155                 160

Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
225                 230                 235                 240

Asn Ile Pro Ser Asp Lys Arg Ile Arg Pro Gln Val Ser Glu Gly Phe
                245                 250                 255

Phe Ser Gln Asp Gly Pro Asn Val Gln Phe Glu Glu Leu Tyr Thr Phe
            260                 265                 270

Gly Gly Leu Asp Val Glu Ile Ile Pro Gln Ile Glu Arg Ser Gln Leu
        275                 280                 285

Arg Glu Lys Ala Leu Gly His Tyr Lys Asp Ile Ala Lys Arg Leu Asn
    290                 295                 300

Asn Ile Asn Lys Thr Ile Pro Ser Ser Trp Ile Ser Asn Ile Asp Lys
305                 310                 315                 320

Tyr Lys Lys Ile Phe Ser Glu Lys Tyr Asn Phe Asp Lys Asp Asn Thr
                325                 330                 335

Gly Asn Phe Val Val Asn Ile Asp Lys Phe Asn Ser Leu Tyr Ser Asp
            340                 345                 350

Leu Thr Asn Val Met Ser Glu Val Val Tyr Ser Ser Gln Tyr Asn Val
        355                 360                 365

Lys Asn Arg Thr His Tyr Phe Ser Arg His Tyr Leu Pro Val Phe Ala
    370                 375                 380

Asn Ile Leu Asp Asp Asn Ile Tyr Thr Ile Arg Asp Gly Phe Asn Leu
385                 390                 395                 400
```

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Tyr Ala Asp Ala Ile Phe Thr Ala Ser Tyr Arg Lys
450 455 460

Val Leu Gly Gln Leu Ser Ala Asn Lys Leu Leu Gln Asp Ile Met Ser
465 470 475 480

Arg Ala Leu Ala Gly Gly Gly Gly Ser Gly Gly Gly Gly Ser Gly Gly
485 490 495

Gly Gly Ser Ala Leu Ala Leu Gln Cys Ile Lys Val Lys Asn Asn Arg
500 505 510

Leu Pro Tyr Val Ala Asp Lys Asp Ser Ile Ser Gln Glu Ile Phe Glu
515 520 525

Asn Lys Ile Ile Thr Asp Glu Thr Asn Val Gln Asn Tyr Ser Asp Lys
530 535 540

Phe Ser Leu Asp Glu Ser Ile Leu Asp Gly Gln Val Pro Ile Asn Pro
545 550 555 560

Glu Ile Val Asp Pro Leu Leu Pro Asn Val Asn Met Glu Pro Leu Asn
565 570 575

Leu Pro Gly Glu Glu Ile Val Phe Tyr Asp Asp Ile Thr Lys Tyr Val
580 585 590

Asp Tyr Leu Asn Ser Tyr Tyr Tyr Leu Glu Ser Gln Lys Leu Ser Asn
595 600 605

Asn Val Glu Asn Ile Thr Leu Thr Thr Ser Val Glu Glu Ala Leu Gly
610 615 620

Tyr Ser Asn Lys Ile Tyr Thr Phe Leu Pro Ser Leu Ala Glu Lys Val
625 630 635 640

Asn Lys Gly Val Gln Ala Gly Leu Phe Leu Asn Trp Ala Asn Glu Val
645 650 655

Val Glu Asp Phe Thr Thr Asn Ile Met Lys Lys Asp Thr Leu Asp Lys
660 665 670

Ile Ser Asp Val Ser Val Ile Ile Pro Tyr Ile Gly Pro Ala Leu Asn
675 680 685

Ile Gly Asn Ser Ala Leu Arg Gly Asn Phe Asn Gln Ala Phe Ala Thr
690 695 700

Ala Gly Val Ala Phe Leu Leu Glu Gly Phe Pro Glu Phe Thr Ile Pro
705 710 715 720

Ala Leu Gly Val Phe Thr Phe Tyr Ser Ser Ile Gln Glu Arg Glu Lys
725 730 735

Ile Ile Lys Thr Ile Glu Asn Cys Leu Glu Gln Arg Val Lys Arg Trp
740 745 750

Lys Asp Ser Tyr Gln Trp Met Val Ser Asn Trp Leu Ser Arg Ile Thr
755 760 765

Thr Gln Phe Asn His Ile Asn Tyr Gln Met Tyr Asp Ser Leu Ser Tyr
770 775 780

Gln Ala Asp Ala Ile Lys Ala Lys Ile Asp Leu Glu Tyr Lys Lys Tyr
785 790 795 800

Ser Gly Ser Asp Lys Glu Asn Ile Lys Ser Gln Val Glu Asn Leu Lys
805 810 815

Asn Ser Leu Asp Val Lys Ile Ser Glu Ala Met Asn Asn Ile Asn Lys
820 825 830

Phe Ile Arg Glu Cys Ser Val Thr Tyr Leu Phe Lys Asn Met Leu Pro
835 840 845

Lys Val Ile Asp Glu Leu Asn Lys Phe Asp Leu Arg Thr Lys Thr Glu
850 855 860

Leu Ile Asn Leu Ile Asp Ser His Asn Ile Ile Leu Val Gly Glu Val
865 870 875 880

Asp Arg Leu Lys Ala Lys Val Asn Glu Ser Phe Glu Asn Thr Met Pro
885 890 895

Phe Asn Ile Phe Ser Tyr Thr Asn Asn Ser Leu Leu Lys Asp Ile Ile

900 905 910

Asn Glu Tyr Phe Asn
915

<210> 73
<211> 917
<212> PRT
<213> Artificial Sequence

<220>
<221> source
<223> /note="Description of Artificial Sequence: Synthetic polypeptide"

<400> 73
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys

180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
        435                 440                 445

Asp Asp Asp Lys Tyr Ala Asp Ala Ile Phe Thr Ala Ser Tyr Arg Asn
    450                 455                 460

Val Leu Gly Gln Leu Ser Ala Arg Lys Leu Leu Gln Asp Ile Met Ser
465                 470                 475                 480

Arg Ala Leu Ala Gly Gly Gly Gly Ser Gly Gly Gly Gly Ser Gly Gly
                485                 490                 495

Gly Gly Ser Ala Leu Ala Leu Gln Cys Ile Lys Val Lys Asn Asn Arg
            500                 505                 510

Leu Pro Tyr Val Ala Asp Lys Asp Ser Ile Ser Gln Glu Ile Phe Glu
        515                 520                 525

Asn Lys Ile Ile Thr Asp Glu Thr Asn Val Gln Asn Tyr Ser Asp Lys
    530                 535                 540

Phe Ser Leu Asp Glu Ser Ile Leu Asp Gly Gln Val Pro Ile Asn Pro
545                 550                 555                 560

Glu Ile Val Asp Pro Leu Leu Pro Asn Val Asn Met Glu Pro Leu Asn
                565                 570                 575

Leu Pro Gly Glu Glu Ile Val Phe Tyr Asp Asp Ile Thr Lys Tyr Val
            580                 585                 590

Asp Tyr Leu Asn Ser Tyr Tyr Tyr Leu Glu Ser Gln Lys Leu Ser Asn
        595                 600                 605

Asn Val Glu Asn Ile Thr Leu Thr Thr Ser Val Glu Glu Ala Leu Gly
    610                 615                 620

Tyr Ser Asn Lys Ile Tyr Thr Phe Leu Pro Ser Leu Ala Glu Lys Val
625                 630                 635                 640

Asn Lys Gly Val Gln Ala Gly Leu Phe Leu Asn Trp Ala Asn Glu Val
                645                 650                 655

Val Glu Asp Phe Thr Thr Asn Ile Met Lys Lys Asp Thr Leu Asp Lys
            660                 665                 670

Ile Ser Asp Val Ser Val Ile Ile Pro Tyr Ile Gly Pro Ala Leu Asn
        675                 680                 685

```
Ile Gly Asn Ser Ala Leu Arg Gly Asn Phe Asn Gln Ala Phe Ala Thr
    690                 695                 700

Ala Gly Val Ala Phe Leu Leu Glu Gly Phe Pro Glu Phe Thr Ile Pro
705                 710                 715                 720

Ala Leu Gly Val Phe Thr Phe Tyr Ser Ser Ile Gln Glu Arg Glu Lys
                725                 730                 735

Ile Ile Lys Thr Ile Glu Asn Cys Leu Glu Gln Arg Val Lys Arg Trp
            740                 745                 750

Lys Asp Ser Tyr Gln Trp Met Val Ser Asn Trp Leu Ser Arg Ile Thr
        755                 760                 765

Thr Gln Phe Asn His Ile Asn Tyr Gln Met Tyr Asp Ser Leu Ser Tyr
    770                 775                 780

Gln Ala Asp Ala Ile Lys Ala Lys Ile Asp Leu Glu Tyr Lys Lys Tyr
785                 790                 795                 800

Ser Gly Ser Asp Lys Glu Asn Ile Lys Ser Gln Val Glu Asn Leu Lys
                805                 810                 815

Asn Ser Leu Asp Val Lys Ile Ser Glu Ala Met Asn Asn Ile Asn Lys
            820                 825                 830

Phe Ile Arg Glu Cys Ser Val Thr Tyr Leu Phe Lys Asn Met Leu Pro
        835                 840                 845

Lys Val Ile Asp Glu Leu Asn Lys Phe Asp Leu Arg Thr Lys Thr Glu
    850                 855                 860

Leu Ile Asn Leu Ile Asp Ser His Asn Ile Ile Leu Val Gly Glu Val
865                 870                 875                 880

Asp Arg Leu Lys Ala Lys Val Asn Glu Ser Phe Glu Asn Thr Met Pro
                885                 890                 895

Phe Asn Ile Phe Ser Tyr Thr Asn Asn Ser Leu Leu Lys Asp Ile Ile
            900                 905                 910

Asn Glu Tyr Phe Asn
        915
```

<210> 74
<211> 917

<212> PRT
<213> Artificial Sequence

<220>
<221> source
<223> /note="Description of Artificial Sequence: Synthetic polypeptide"

<400> 74
Thr Trp Pro Val Lys Asp Phe Asn Tyr Ser Asp Pro Val Asn Asp Asn
1               5                   10                  15

Asp Ile Leu Tyr Leu Arg Ile Pro Gln Asn Lys Leu Ile Thr Thr Pro
            20                  25                  30

Val Lys Ala Phe Met Ile Thr Gln Asn Ile Trp Val Ile Pro Glu Arg
        35                  40                  45

Phe Ser Ser Asp Thr Asn Pro Ser Leu Ser Lys Pro Pro Arg Pro Thr
    50                  55                  60

Ser Lys Tyr Gln Ser Tyr Tyr Asp Pro Ser Tyr Leu Ser Thr Asp Glu
65                  70                  75                  80

Gln Lys Asp Thr Phe Leu Lys Gly Ile Ile Lys Leu Phe Lys Arg Ile
                85                  90                  95

Asn Glu Arg Asp Ile Gly Lys Lys Leu Ile Asn Tyr Leu Val Val Gly
            100                 105                 110

Ser Pro Phe Met Gly Asp Ser Ser Thr Pro Glu Asp Thr Phe Asp Phe
        115                 120                 125

Thr Arg His Thr Thr Asn Ile Ala Val Glu Lys Phe Glu Asn Gly Ser
    130                 135                 140

Trp Lys Val Thr Asn Ile Ile Thr Pro Ser Val Leu Ile Phe Gly Pro
145                 150                 155                 160

Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
225                 230                 235                 240

Asn Ile Pro Ser Asp Lys Arg Ile Arg Pro Gln Val Ser Glu Gly Phe
                245                 250                 255

Phe Ser Gln Asp Gly Pro Asn Val Gln Phe Glu Glu Leu Tyr Thr Phe
            260                 265                 270

Gly Gly Leu Asp Val Glu Ile Ile Pro Gln Ile Glu Arg Ser Gln Leu
        275                 280                 285

Arg Glu Lys Ala Leu Gly His Tyr Lys Asp Ile Ala Lys Arg Leu Asn
    290                 295                 300

Asn Ile Asn Lys Thr Ile Pro Ser Ser Trp Ile Ser Asn Ile Asp Lys
305                 310                 315                 320

Tyr Lys Lys Ile Phe Ser Glu Lys Tyr Asn Phe Asp Lys Asp Asn Thr
                325                 330                 335

Gly Asn Phe Val Val Asn Ile Asp Lys Phe Asn Ser Leu Tyr Ser Asp
            340                 345                 350

Leu Thr Asn Val Met Ser Glu Val Val Tyr Ser Ser Gln Tyr Asn Val
        355                 360                 365

Lys Asn Arg Thr His Tyr Phe Ser Arg His Tyr Leu Pro Val Phe Ala
    370                 375                 380

Asn Ile Leu Asp Asp Asn Ile Tyr Thr Ile Arg Asp Gly Phe Asn Leu
385                 390                 395                 400

Thr Asn Lys Gly Phe Asn Ile Glu Asn Ser Gly Gln Asn Ile Glu Arg
                405                 410                 415

Asn Pro Ala Leu Gln Lys Leu Ser Ser Glu Ser Val Val Asp Leu Phe
            420                 425                 430

Thr Lys Val Cys Val Asp Gly Ile Ile Thr Ser Lys Thr Lys Ser Asp
        435                 440                 445

Asp Asp Asp Lys Tyr Ala Asp Ala Ile Phe Thr Ala Ser Tyr Arg Lys
    450                 455                 460

Val Leu Gly Gln Leu Ser Ala Arg Asn Leu Leu Gln Asp Ile Met Ser

465 470 475 480

Arg Ala Leu Ala Gly Gly Gly Gly Ser Gly Gly Gly Gly Ser Gly Gly
485 490 495

Gly Gly Ser Ala Leu Ala Leu Gln Cys Ile Lys Val Lys Asn Asn Arg
500 505 510

Leu Pro Tyr Val Ala Asp Lys Asp Ser Ile Ser Gln Glu Ile Phe Glu
515 520 525

Asn Lys Ile Ile Thr Asp Glu Thr Asn Val Gln Asn Tyr Ser Asp Lys
530 535 540

Phe Ser Leu Asp Glu Ser Ile Leu Asp Gly Gln Val Pro Ile Asn Pro
545 550 555 560

Glu Ile Val Asp Pro Leu Leu Pro Asn Val Asn Met Glu Pro Leu Asn
565 570 575

Leu Pro Gly Glu Glu Ile Val Phe Tyr Asp Asp Ile Thr Lys Tyr Val
580 585 590

Asp Tyr Leu Asn Ser Tyr Tyr Tyr Leu Glu Ser Gln Lys Leu Ser Asn
595 600 605

Asn Val Glu Asn Ile Thr Leu Thr Thr Ser Val Glu Glu Ala Leu Gly
610 615 620

Tyr Ser Asn Lys Ile Tyr Thr Phe Leu Pro Ser Leu Ala Glu Lys Val
625 630 635 640

Asn Lys Gly Val Gln Ala Gly Leu Phe Leu Asn Trp Ala Asn Glu Val
645 650 655

Val Glu Asp Phe Thr Thr Asn Ile Met Lys Lys Asp Thr Leu Asp Lys
660 665 670

Ile Ser Asp Val Ser Val Ile Ile Pro Tyr Ile Gly Pro Ala Leu Asn
675 680 685

Ile Gly Asn Ser Ala Leu Arg Gly Asn Phe Asn Gln Ala Phe Ala Thr
690 695 700

Ala Gly Val Ala Phe Leu Leu Glu Gly Phe Pro Glu Phe Thr Ile Pro
705 710 715 720

Ala Leu Gly Val Phe Thr Phe Tyr Ser Ser Ile Gln Glu Arg Glu Lys
725 730 735

Ile Ile Lys Thr Ile Glu Asn Cys Leu Glu Gln Arg Val Lys Arg Trp
740 745 750

Lys Asp Ser Tyr Gln Trp Met Val Ser Asn Trp Leu Ser Arg Ile Thr
755 760 765

Thr Gln Phe Asn His Ile Asn Tyr Gln Met Tyr Asp Ser Leu Ser Tyr
770 775 780

Gln Ala Asp Ala Ile Lys Ala Lys Ile Asp Leu Glu Tyr Lys Lys Tyr
785 790 795 800

Ser Gly Ser Asp Lys Glu Asn Ile Lys Ser Gln Val Glu Asn Leu Lys
805 810 815

Asn Ser Leu Asp Val Lys Ile Ser Glu Ala Met Asn Asn Ile Asn Lys
820 825 830

Phe Ile Arg Glu Cys Ser Val Thr Tyr Leu Phe Lys Asn Met Leu Pro
835 840 845

Lys Val Ile Asp Glu Leu Asn Lys Phe Asp Leu Arg Thr Lys Thr Glu
850 855 860

Leu Ile Asn Leu Ile Asp Ser His Asn Ile Ile Leu Val Gly Glu Val
865 870 875 880

Asp Arg Leu Lys Ala Lys Val Asn Glu Ser Phe Glu Asn Thr Met Pro
885 890 895

Phe Asn Ile Phe Ser Tyr Thr Asn Asn Ser Leu Leu Lys Asp Ile Ile
900 905 910

Asn Glu Tyr Phe Asn
915

<210> 75
<211> 917
<212> PRT
<213> Artificial Sequence

<220>
<221> source
<223> /note="Description of Artificial Sequence: Synthetic polypeptide"

<400> 75

Thr Trp Pro Val Lys Asp Phe Asn Tyr Ser Asp Pro Val Asn Asp Asn
1               5                   10                  15

Asp Ile Leu Tyr Leu Arg Ile Pro Gln Asn Lys Leu Ile Thr Thr Pro
            20                  25                  30

Val Lys Ala Phe Met Ile Thr Gln Asn Ile Trp Val Ile Pro Glu Arg
        35                  40                  45

Phe Ser Ser Asp Thr Asn Pro Ser Leu Ser Lys Pro Pro Arg Pro Thr
    50                  55                  60

Ser Lys Tyr Gln Ser Tyr Tyr Asp Pro Ser Tyr Leu Ser Thr Asp Glu
65                  70                  75                  80

Gln Lys Asp Thr Phe Leu Lys Gly Ile Ile Lys Leu Phe Lys Arg Ile
                85                  90                  95

Asn Glu Arg Asp Ile Gly Lys Lys Leu Ile Asn Tyr Leu Val Val Gly
            100                 105                 110

Ser Pro Phe Met Gly Asp Ser Ser Thr Pro Glu Asp Thr Phe Asp Phe
        115                 120                 125

Thr Arg His Thr Thr Asn Ile Ala Val Glu Lys Phe Glu Asn Gly Ser
    130                 135                 140

Trp Lys Val Thr Asn Ile Ile Thr Pro Ser Val Leu Ile Phe Gly Pro
145                 150                 155                 160

Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
225                 230                 235                 240

Asn Ile Pro Ser Asp Lys Arg Ile Arg Pro Gln Val Ser Glu Gly Phe
                245                 250                 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Tyr Ala Asp Ala Ile Phe Glu Ala Ser Tyr Arg Lys
450 455 460

Val Leu Gly Gln Leu Ser Ala Arg Lys Leu Leu Gln Asp Ile Met Ser
465 470 475 480

Arg Ala Leu Ala Gly Gly Gly Gly Ser Gly Gly Gly Gly Ser Gly Gly
485 490 495

Gly Gly Ser Ala Leu Ala Leu Gln Cys Ile Lys Val Lys Asn Asn Arg
500 505 510

Leu Pro Tyr Val Ala Asp Lys Asp Ser Ile Ser Gln Glu Ile Phe Glu
515 520 525

Asn Lys Ile Ile Thr Asp Glu Thr Asn Val Gln Asn Tyr Ser Asp Lys
530 535 540

Phe Ser Leu Asp Glu Ser Ile Leu Asp Gly Gln Val Pro Ile Asn Pro
545 550 555 560

Glu Ile Val Asp Pro Leu Leu Pro Asn Val Asn Met Glu Pro Leu Asn
565 570 575

Leu Pro Gly Glu Glu Ile Val Phe Tyr Asp Asp Ile Thr Lys Tyr Val
580 585 590

Asp Tyr Leu Asn Ser Tyr Tyr Tyr Leu Glu Ser Gln Lys Leu Ser Asn
595 600 605

Asn Val Glu Asn Ile Thr Leu Thr Thr Ser Val Glu Glu Ala Leu Gly
610 615 620

Tyr Ser Asn Lys Ile Tyr Thr Phe Leu Pro Ser Leu Ala Glu Lys Val
625 630 635 640

Asn Lys Gly Val Gln Ala Gly Leu Phe Leu Asn Trp Ala Asn Glu Val
645 650 655

Val Glu Asp Phe Thr Thr Asn Ile Met Lys Lys Asp Thr Leu Asp Lys
660 665 670

Ile Ser Asp Val Ser Val Ile Ile Pro Tyr Ile Gly Pro Ala Leu Asn
675 680 685

Ile Gly Asn Ser Ala Leu Arg Gly Asn Phe Asn Gln Ala Phe Ala Thr
690 695 700

Ala Gly Val Ala Phe Leu Leu Glu Gly Phe Pro Glu Phe Thr Ile Pro
705 710 715 720

Ala Leu Gly Val Phe Thr Phe Tyr Ser Ser Ile Gln Glu Arg Glu Lys
725 730 735

Ile Ile Lys Thr Ile Glu Asn Cys Leu Glu Gln Arg Val Lys Arg Trp
740 745 750

Lys Asp Ser Tyr Gln Trp Met Val Ser Asn Trp Leu Ser Arg Ile Thr

755 760 765

Thr Gln Phe Asn His Ile Asn Tyr Gln Met Tyr Asp Ser Leu Ser Tyr
770 775 780

Gln Ala Asp Ala Ile Lys Ala Lys Ile Asp Leu Glu Tyr Lys Lys Tyr
785 790 795 800

Ser Gly Ser Asp Lys Glu Asn Ile Lys Ser Gln Val Glu Asn Leu Lys
805 810 815

Asn Ser Leu Asp Val Lys Ile Ser Glu Ala Met Asn Asn Ile Asn Lys
820 825 830

Phe Ile Arg Glu Cys Ser Val Thr Tyr Leu Phe Lys Asn Met Leu Pro
835 840 845

Lys Val Ile Asp Glu Leu Asn Lys Phe Asp Leu Arg Thr Lys Thr Glu
850 855 860

Leu Ile Asn Leu Ile Asp Ser His Asn Ile Ile Leu Val Gly Glu Val
865 870 875 880

Asp Arg Leu Lys Ala Lys Val Asn Glu Ser Phe Glu Asn Thr Met Pro
885 890 895

Phe Asn Ile Phe Ser Tyr Thr Asn Asn Ser Leu Leu Lys Asp Ile Ile
900 905 910

Asn Glu Tyr Phe Asn
915

<210> 76
<211> 917
<212> PRT
<213> Artificial Sequence

<220>
<221> source
<223> /note="Description of Artificial Sequence: Synthetic polypeptide"

<400> 76
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg

35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
    290                 295                 300

Asn Ile Asn Lys Thr Ile Pro Ser Ser Trp Ile Ser Asn Ile Asp Lys
305                 310                 315                 320

Tyr Lys Lys Ile Phe Ser Glu Lys Tyr Asn Phe Asp Lys Asp Asn Thr
                325                 330                 335

Gly Asn Phe Val Val Asn Ile Asp Lys Phe Asn Ser Leu Tyr Ser Asp
            340                 345                 350

Leu Thr Asn Val Met Ser Glu Val Val Tyr Ser Ser Gln Tyr Asn Val
        355                 360                 365

Lys Asn Arg Thr His Tyr Phe Ser Arg His Tyr Leu Pro Val Phe Ala
    370                 375                 380

Asn Ile Leu Asp Asp Asn Ile Tyr Thr Ile Arg Asp Gly Phe Asn Leu
385                 390                 395                 400

Thr Asn Lys Gly Phe Asn Ile Glu Asn Ser Gly Gln Asn Ile Glu Arg
                405                 410                 415

Asn Pro Ala Leu Gln Lys Leu Ser Ser Glu Ser Val Val Asp Leu Phe
            420                 425                 430

Thr Lys Val Cys Val Asp Gly Ile Ile Thr Ser Lys Thr Lys Ser Asp
        435                 440                 445

Asp Asp Asp Lys Tyr Ala Asp Ala Ile Phe Thr Ala Ser Glu Arg Lys
    450                 455                 460

Val Leu Gly Gln Leu Ser Ala Arg Lys Leu Leu Gln Asp Ile Met Ser
465                 470                 475                 480

Arg Ala Leu Ala Gly Gly Gly Gly Ser Gly Gly Gly Gly Ser Gly Gly
                485                 490                 495

Gly Gly Ser Ala Leu Ala Leu Gln Cys Ile Lys Val Lys Asn Asn Arg
            500                 505                 510

Leu Pro Tyr Val Ala Asp Lys Asp Ser Ile Ser Gln Glu Ile Phe Glu
        515                 520                 525

Asn Lys Ile Ile Thr Asp Glu Thr Asn Val Gln Asn Tyr Ser Asp Lys
    530                 535                 540

Phe Ser Leu Asp Glu Ser Ile Leu Asp Gly Gln Val Pro Ile Asn Pro
545                 550                 555                 560

Glu Ile Val Asp Pro Leu Leu Pro Asn Val Asn Met Glu Pro Leu Asn
                565                 570                 575

Leu Pro Gly Glu Glu Ile Val Phe Tyr Asp Asp Ile Thr Lys Tyr Val
            580                 585                 590

Asp Tyr Leu Asn Ser Tyr Tyr Tyr Leu Glu Ser Gln Lys Leu Ser Asn
        595                 600                 605

Asn Val Glu Asn Ile Thr Leu Thr Thr Ser Val Glu Glu Ala Leu Gly
    610                 615                 620

Tyr Ser Asn Lys Ile Tyr Thr Phe Leu Pro Ser Leu Ala Glu Lys Val
625                 630                 635                 640

Asn Lys Gly Val Gln Ala Gly Leu Phe Leu Asn Trp Ala Asn Glu Val
                645                 650                 655

Val Glu Asp Phe Thr Thr Asn Ile Met Lys Lys Asp Thr Leu Asp Lys
            660                 665                 670

Ile Ser Asp Val Ser Val Ile Ile Pro Tyr Ile Gly Pro Ala Leu Asn
        675                 680                 685

Ile Gly Asn Ser Ala Leu Arg Gly Asn Phe Asn Gln Ala Phe Ala Thr
    690                 695                 700

Ala Gly Val Ala Phe Leu Leu Glu Gly Phe Pro Glu Phe Thr Ile Pro
705                 710                 715                 720

Ala Leu Gly Val Phe Thr Phe Tyr Ser Ser Ile Gln Glu Arg Glu Lys
                725                 730                 735

Ile Ile Lys Thr Ile Glu Asn Cys Leu Glu Gln Arg Val Lys Arg Trp
            740                 745                 750

Lys Asp Ser Tyr Gln Trp Met Val Ser Asn Trp Leu Ser Arg Ile Thr
        755                 760                 765

Thr Gln Phe Asn His Ile Asn Tyr Gln Met Tyr Asp Ser Leu Ser Tyr
    770                 775                 780

Gln Ala Asp Ala Ile Lys Ala Lys Ile Asp Leu Glu Tyr Lys Lys Tyr
785                 790                 795                 800

Ser Gly Ser Asp Lys Glu Asn Ile Lys Ser Gln Val Glu Asn Leu Lys
805 810 815

Asn Ser Leu Asp Val Lys Ile Ser Glu Ala Met Asn Asn Ile Asn Lys
820 825 830

Phe Ile Arg Glu Cys Ser Val Thr Tyr Leu Phe Lys Asn Met Leu Pro
835 840 845

Lys Val Ile Asp Glu Leu Asn Lys Phe Asp Leu Arg Thr Lys Thr Glu
850 855 860

Leu Ile Asn Leu Ile Asp Ser His Asn Ile Ile Leu Val Gly Glu Val
865 870 875 880

Asp Arg Leu Lys Ala Lys Val Asn Glu Ser Phe Glu Asn Thr Met Pro
885 890 895

Phe Asn Ile Phe Ser Tyr Thr Asn Asn Ser Leu Leu Lys Asp Ile Ile
900 905 910

Asn Glu Tyr Phe Asn
915

<210> 77
<211> 917
<212> PRT
<213> Artificial Sequence

<220>
<221> source
<223> /note="Description of Artificial Sequence: Synthetic polypeptide"

<400> 77
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr

325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Tyr Ala Asp Ala Ile Phe Thr Ala Ser Tyr Arg Lys
450 455 460

Glu Leu Gly Gln Leu Ser Ala Arg Lys Leu Leu Gln Asp Ile Met Ser
465 470 475 480

Arg Ala Leu Ala Gly Gly Gly Gly Ser Gly Gly Gly Gly Ser Gly Gly
485 490 495

Gly Gly Ser Ala Leu Ala Leu Gln Cys Ile Lys Val Lys Asn Asn Arg
500 505 510

Leu Pro Tyr Val Ala Asp Lys Asp Ser Ile Ser Gln Glu Ile Phe Glu
515 520 525

Asn Lys Ile Ile Thr Asp Glu Thr Asn Val Gln Asn Tyr Ser Asp Lys
530 535 540

Phe Ser Leu Asp Glu Ser Ile Leu Asp Gly Gln Val Pro Ile Asn Pro
545 550 555 560

Glu Ile Val Asp Pro Leu Leu Pro Asn Val Asn Met Glu Pro Leu Asn
565 570 575

```
Leu Pro Gly Glu Glu Ile Val Phe Tyr Asp Asp Ile Thr Lys Tyr Val
            580                 585                 590

Asp Tyr Leu Asn Ser Tyr Tyr Tyr Leu Glu Ser Gln Lys Leu Ser Asn
        595                 600                 605

Asn Val Glu Asn Ile Thr Leu Thr Thr Ser Val Glu Glu Ala Leu Gly
    610                 615                 620

Tyr Ser Asn Lys Ile Tyr Thr Phe Leu Pro Ser Leu Ala Glu Lys Val
625                 630                 635                 640

Asn Lys Gly Val Gln Ala Gly Leu Phe Leu Asn Trp Ala Asn Glu Val
                645                 650                 655

Val Glu Asp Phe Thr Thr Asn Ile Met Lys Lys Asp Thr Leu Asp Lys
            660                 665                 670

Ile Ser Asp Val Ser Val Ile Ile Pro Tyr Ile Gly Pro Ala Leu Asn
        675                 680                 685

Ile Gly Asn Ser Ala Leu Arg Gly Asn Phe Asn Gln Ala Phe Ala Thr
    690                 695                 700

Ala Gly Val Ala Phe Leu Leu Glu Gly Phe Pro Glu Phe Thr Ile Pro
705                 710                 715                 720

Ala Leu Gly Val Phe Thr Phe Tyr Ser Ser Ile Gln Glu Arg Glu Lys
                725                 730                 735

Ile Ile Lys Thr Ile Glu Asn Cys Leu Glu Gln Arg Val Lys Arg Trp
            740                 745                 750

Lys Asp Ser Tyr Gln Trp Met Val Ser Asn Trp Leu Ser Arg Ile Thr
        755                 760                 765

Thr Gln Phe Asn His Ile Asn Tyr Gln Met Tyr Asp Ser Leu Ser Tyr
    770                 775                 780

Gln Ala Asp Ala Ile Lys Ala Lys Ile Asp Leu Glu Tyr Lys Lys Tyr
785                 790                 795                 800

Ser Gly Ser Asp Lys Glu Asn Ile Lys Ser Gln Val Glu Asn Leu Lys
                805                 810                 815

Asn Ser Leu Asp Val Lys Ile Ser Glu Ala Met Asn Asn Ile Asn Lys
            820                 825                 830
```

Phe Ile Arg Glu Cys Ser Val Thr Tyr Leu Phe Lys Asn Met Leu Pro
835 840 845

Lys Val Ile Asp Glu Leu Asn Lys Phe Asp Leu Arg Thr Lys Thr Glu
850 855 860

Leu Ile Asn Leu Ile Asp Ser His Asn Ile Ile Leu Val Gly Glu Val
865 870 875 880

Asp Arg Leu Lys Ala Lys Val Asn Glu Ser Phe Glu Asn Thr Met Pro
885 890 895

Phe Asn Ile Phe Ser Tyr Thr Asn Asn Ser Leu Leu Lys Asp Ile Ile
900 905 910

Asn Glu Tyr Phe Asn
915

<210> 78
<211> 917
<212> PRT
<213> Artificial Sequence

<220>
<221> source
<223> /note="Description of Artificial Sequence: Synthetic polypeptide"

<400> 78
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

```
Ser Pro Phe Met Gly Asp Ser Ser Thr Pro Glu Asp Thr Phe Asp Phe
        115                 120                 125

Thr Arg His Thr Thr Asn Ile Ala Val Glu Lys Phe Glu Asn Gly Ser
    130                 135                 140

Trp Lys Val Thr Asn Ile Ile Thr Pro Ser Val Leu Ile Phe Gly Pro
145                 150                 155                 160

Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
225                 230                 235                 240

Asn Ile Pro Ser Asp Lys Arg Ile Arg Pro Gln Val Ser Glu Gly Phe
                245                 250                 255

Phe Ser Gln Asp Gly Pro Asn Val Gln Phe Glu Glu Leu Tyr Thr Phe
            260                 265                 270

Gly Gly Leu Asp Val Glu Ile Ile Pro Gln Ile Glu Arg Ser Gln Leu
        275                 280                 285

Arg Glu Lys Ala Leu Gly His Tyr Lys Asp Ile Ala Lys Arg Leu Asn
    290                 295                 300

Asn Ile Asn Lys Thr Ile Pro Ser Ser Trp Ile Ser Asn Ile Asp Lys
305                 310                 315                 320

Tyr Lys Lys Ile Phe Ser Glu Lys Tyr Asn Phe Asp Lys Asp Asn Thr
                325                 330                 335

Gly Asn Phe Val Val Asn Ile Asp Lys Phe Asn Ser Leu Tyr Ser Asp
            340                 345                 350

Leu Thr Asn Val Met Ser Glu Val Val Tyr Ser Ser Gln Tyr Asn Val
        355                 360                 365
```

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Tyr Ala Asp Ala Ile Phe Thr Ala Ser Tyr Arg Lys
450 455 460

Val Leu Gly Gln Leu Ser Ala Arg Lys Leu Leu Gln Asp Ile Met Ser
465 470 475 480

Arg Ala Leu Ala Gly Gly Gly Gly Ser Gly Gly Gly Gly Ser Gly Gly
485 490 495

Gly Gly Ser Ala Leu Ala Leu Gln Cys Ile Lys Val Lys Asn Asn Arg
500 505 510

Leu Pro Tyr Val Ala Asp Lys Asp Ser Ile Ser Gln Glu Ile Phe Glu
515 520 525

Asn Lys Ile Ile Thr Asp Glu Thr Asn Val Gln Asn Tyr Ser Asp Lys
530 535 540

Phe Ser Leu Asp Glu Ser Ile Leu Asp Gly Gln Val Pro Ile Asn Pro
545 550 555 560

Glu Ile Val Asp Pro Leu Leu Pro Asn Val Asn Met Glu Pro Leu Asn
565 570 575

Leu Pro Gly Glu Glu Ile Val Phe Tyr Asp Asp Ile Thr Lys Tyr Val
580 585 590

Asp Tyr Leu Asn Ser Tyr Tyr Tyr Leu Glu Ser Gln Lys Leu Ser Asn
595 600 605

Asn Val Glu Asn Ile Thr Leu Thr Thr Ser Val Glu Glu Ala Leu Gly

610 615 620

Tyr Ser Asn Lys Ile Tyr Thr Phe Leu Pro Ser Leu Ala Glu Lys Val
625 630 635 640

Asn Lys Gly Val Gln Ala Gly Leu Phe Leu Asn Trp Ala Asn Glu Val
645 650 655

Val Glu Asp Phe Thr Thr Asn Ile Met Lys Lys Asp Thr Leu Asp Lys
660 665 670

Ile Ser Asp Val Ser Val Ile Ile Pro Tyr Ile Gly Pro Ala Leu Asn
675 680 685

Ile Gly Asn Ser Ala Leu Arg Gly Asn Phe Asn Gln Ala Phe Ala Thr
690 695 700

Ala Gly Val Ala Phe Leu Leu Glu Gly Phe Pro Glu Phe Thr Ile Pro
705 710 715 720

Ala Leu Gly Val Phe Thr Phe Tyr Ser Ser Ile Gln Glu Arg Glu Lys
725 730 735

Ile Ile Lys Thr Ile Glu Asn Cys Leu Glu Gln Arg Val Lys Arg Trp
740 745 750

Lys Asp Ser Tyr Gln Trp Met Val Ser Asn Trp Leu Ser Arg Ile Thr
755 760 765

Thr Gln Phe Asn His Ile Asn Tyr Gln Met Tyr Asp Ser Leu Ser Tyr
770 775 780

Gln Ala Asp Ala Ile Lys Ala Lys Ile Asp Leu Glu Tyr Lys Lys Tyr
785 790 795 800

Ser Gly Ser Asp Lys Glu Asn Ile Lys Ser Gln Val Glu Asn Leu Lys
805 810 815

Asn Ser Leu Asp Val Lys Ile Ser Glu Ala Met Asn Asn Ile Asn Lys
820 825 830

Phe Ile Arg Glu Cys Ser Val Thr Tyr Leu Phe Lys Asn Met Leu Pro
835 840 845

Lys Val Ile Asp Glu Leu Asn Lys Phe Asp Leu Arg Thr Lys Thr Glu
850 855 860

Leu Ile Asn Leu Ile Asp Ser His Asn Ile Ile Leu Val Gly Glu Val
865                 870                 875                 880

Asp Arg Leu Lys Ala Lys Val Asn Glu Ser Phe Glu Asn Thr Met Pro
                885                 890                 895

Phe Asn Ile Phe Ser Tyr Thr Asn Asn Ser Leu Leu Lys Asp Ile Ile
            900                 905                 910

Asn Glu Tyr Phe Asn
        915

<210> 79
<211> 917
<212> PRT
<213> Artificial Sequence

<220>
<221> source
<223> /note="Description of Artificial Sequence: Synthetic polypeptide"

<400> 79
Thr Trp Pro Val Lys Asp Phe Asn Tyr Ser Asp Pro Val Asn Asp Asn
1               5                   10                  15

Asp Ile Leu Tyr Leu Arg Ile Pro Gln Asn Lys Leu Ile Thr Thr Pro
            20                  25                  30

Val Lys Ala Phe Met Ile Thr Gln Asn Ile Trp Val Ile Pro Glu Arg
        35                  40                  45

Phe Ser Ser Asp Thr Asn Pro Ser Leu Ser Lys Pro Pro Arg Pro Thr
    50                  55                  60

Ser Lys Tyr Gln Ser Tyr Tyr Asp Pro Ser Tyr Leu Ser Thr Asp Glu
65                  70                  75                  80

Gln Lys Asp Thr Phe Leu Lys Gly Ile Ile Lys Leu Phe Lys Arg Ile
                85                  90                  95

Asn Glu Arg Asp Ile Gly Lys Lys Leu Ile Asn Tyr Leu Val Val Gly
            100                 105                 110

Ser Pro Phe Met Gly Asp Ser Ser Thr Pro Glu Asp Thr Phe Asp Phe
        115                 120                 125

Thr Arg His Thr Thr Asn Ile Ala Val Glu Lys Phe Glu Asn Gly Ser
    130                 135                 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Tyr Ala Asp Ala Ile Phe Thr Glu Ser Tyr Arg Lys
450 455 460

Val Leu Gly Gln Leu Ser Ala Arg Lys Leu Leu Gln Asp Ile Met Ser
465 470 475 480

Arg Ala Leu Ala Gly Gly Gly Gly Ser Gly Gly Gly Gly Ser Gly Gly
485 490 495

Gly Gly Ser Ala Leu Ala Leu Gln Cys Ile Lys Val Lys Asn Asn Arg
500 505 510

Leu Pro Tyr Val Ala Asp Lys Asp Ser Ile Ser Gln Glu Ile Phe Glu
515 520 525

Asn Lys Ile Ile Thr Asp Glu Thr Asn Val Gln Asn Tyr Ser Asp Lys
530 535 540

Phe Ser Leu Asp Glu Ser Ile Leu Asp Gly Gln Val Pro Ile Asn Pro
545 550 555 560

Glu Ile Val Asp Pro Leu Leu Pro Asn Val Asn Met Glu Pro Leu Asn
565 570 575

Leu Pro Gly Glu Glu Ile Val Phe Tyr Asp Asp Ile Thr Lys Tyr Val
580 585 590

Asp Tyr Leu Asn Ser Tyr Tyr Tyr Leu Glu Ser Gln Lys Leu Ser Asn
595 600 605

Asn Val Glu Asn Ile Thr Leu Thr Thr Ser Val Glu Glu Ala Leu Gly
610 615 620

Tyr Ser Asn Lys Ile Tyr Thr Phe Leu Pro Ser Leu Ala Glu Lys Val
625 630 635 640

Asn Lys Gly Val Gln Ala Gly Leu Phe Leu Asn Trp Ala Asn Glu Val
645 650 655

Val Glu Asp Phe Thr Thr Asn Ile Met Lys Lys Asp Thr Leu Asp Lys
660 665 670

Ile Ser Asp Val Ser Val Ile Ile Pro Tyr Ile Gly Pro Ala Leu Asn
675 680 685

Ile Gly Asn Ser Ala Leu Arg Gly Asn Phe Asn Gln Ala Phe Ala Thr
690 695 700

Ala Gly Val Ala Phe Leu Leu Glu Gly Phe Pro Glu Phe Thr Ile Pro
705 710 715 720

Ala Leu Gly Val Phe Thr Phe Tyr Ser Ser Ile Gln Glu Arg Glu Lys
725 730 735

Ile Ile Lys Thr Ile Glu Asn Cys Leu Glu Gln Arg Val Lys Arg Trp
740 745 750

Lys Asp Ser Tyr Gln Trp Met Val Ser Asn Trp Leu Ser Arg Ile Thr
755 760 765

Thr Gln Phe Asn His Ile Asn Tyr Gln Met Tyr Asp Ser Leu Ser Tyr
770 775 780

Gln Ala Asp Ala Ile Lys Ala Lys Ile Asp Leu Glu Tyr Lys Lys Tyr
785 790 795 800

Ser Gly Ser Asp Lys Glu Asn Ile Lys Ser Gln Val Glu Asn Leu Lys
805 810 815

Asn Ser Leu Asp Val Lys Ile Ser Glu Ala Met Asn Asn Ile Asn Lys
820 825 830

Phe Ile Arg Glu Cys Ser Val Thr Tyr Leu Phe Lys Asn Met Leu Pro
835 840 845

Lys Val Ile Asp Glu Leu Asn Lys Phe Asp Leu Arg Thr Lys Thr Glu
850 855 860

Leu Ile Asn Leu Ile Asp Ser His Asn Ile Ile Leu Val Gly Glu Val
865 870 875 880

Asp Arg Leu Lys Ala Lys Val Asn Glu Ser Phe Glu Asn Thr Met Pro
885 890 895

Phe Asn Ile Phe Ser Tyr Thr Asn Asn Ser Leu Leu Lys Asp Ile Ile

900 905 910

Asn Glu Tyr Phe Asn
915

<210> 80
<211> 915
<212> PRT
<213> Artificial Sequence

<220>
<221> source
<223> /note="Description of Artificial Sequence: Synthetic polypeptide"

<400> 80
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys

180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

```
Thr Lys Val Cys Val Asp Gly Ile Ile Thr Ser Lys Thr Lys Ser Asp
        435                 440                 445

Asp Asp Asp Lys Tyr Ala Asp Ala Ile Phe Thr Asn Ser Tyr Arg Lys
    450                 455                 460

Val Leu Ala Gln Leu Ser Ala Arg Lys Leu Leu Gln Asp Ile Met Ala
465                 470                 475                 480

Leu Ala Gly Gly Gly Gly Ser Gly Gly Gly Gly Ser Gly Gly Gly Gly
                485                 490                 495

Ser Ala Leu Ala Leu Gln Cys Ile Lys Val Lys Asn Asn Arg Leu Pro
            500                 505                 510

Tyr Val Ala Asp Lys Asp Ser Ile Ser Gln Glu Ile Phe Glu Asn Lys
        515                 520                 525

Ile Ile Thr Asp Glu Thr Asn Val Gln Asn Tyr Ser Asp Lys Phe Ser
    530                 535                 540

Leu Asp Glu Ser Ile Leu Asp Gly Gln Val Pro Ile Asn Pro Glu Ile
545                 550                 555                 560

Val Asp Pro Leu Leu Pro Asn Val Asn Met Glu Pro Leu Asn Leu Pro
                565                 570                 575

Gly Glu Glu Ile Val Phe Tyr Asp Asp Ile Thr Lys Tyr Val Asp Tyr
            580                 585                 590

Leu Asn Ser Tyr Tyr Tyr Leu Glu Ser Gln Lys Leu Ser Asn Asn Val
        595                 600                 605

Glu Asn Ile Thr Leu Thr Thr Ser Val Glu Glu Ala Leu Gly Tyr Ser
    610                 615                 620

Asn Lys Ile Tyr Thr Phe Leu Pro Ser Leu Ala Glu Lys Val Asn Lys
625                 630                 635                 640

Gly Val Gln Ala Gly Leu Phe Leu Asn Trp Ala Asn Glu Val Val Glu
                645                 650                 655

Asp Phe Thr Thr Asn Ile Met Lys Lys Asp Thr Leu Asp Lys Ile Ser
            660                 665                 670

Asp Val Ser Val Ile Ile Pro Tyr Ile Gly Pro Ala Leu Asn Ile Gly
        675                 680                 685
```

```
Asn Ser Ala Leu Arg Gly Asn Phe Asn Gln Ala Phe Ala Thr Ala Gly
    690                 695                 700

Val Ala Phe Leu Leu Glu Gly Phe Pro Glu Phe Thr Ile Pro Ala Leu
705                 710                 715                 720

Gly Val Phe Thr Phe Tyr Ser Ser Ile Gln Glu Arg Glu Lys Ile Ile
                725                 730                 735

Lys Thr Ile Glu Asn Cys Leu Glu Gln Arg Val Lys Arg Trp Lys Asp
            740                 745                 750

Ser Tyr Gln Trp Met Val Ser Asn Trp Leu Ser Arg Ile Thr Thr Gln
        755                 760                 765

Phe Asn His Ile Asn Tyr Gln Met Tyr Asp Ser Leu Ser Tyr Gln Ala
    770                 775                 780

Asp Ala Ile Lys Ala Lys Ile Asp Leu Glu Tyr Lys Lys Tyr Ser Gly
785                 790                 795                 800

Ser Asp Lys Glu Asn Ile Lys Ser Gln Val Glu Asn Leu Lys Asn Ser
                805                 810                 815

Leu Asp Val Lys Ile Ser Glu Ala Met Asn Asn Ile Asn Lys Phe Ile
            820                 825                 830

Arg Glu Cys Ser Val Thr Tyr Leu Phe Lys Asn Met Leu Pro Lys Val
        835                 840                 845

Ile Asp Glu Leu Asn Lys Phe Asp Leu Arg Thr Lys Thr Glu Leu Ile
    850                 855                 860

Asn Leu Ile Asp Ser His Asn Ile Ile Leu Val Gly Glu Val Asp Arg
865                 870                 875                 880

Leu Lys Ala Lys Val Asn Glu Ser Phe Glu Asn Thr Met Pro Phe Asn
                885                 890                 895

Ile Phe Ser Tyr Thr Asn Asn Ser Leu Leu Lys Asp Ile Ile Asn Glu
            900                 905                 910

Tyr Phe Asn
        915

<210> 81
<211> 917
```

<212> PRT
<213> Artificial Sequence

<220>
<221> source
<223> /note="Description of Artificial Sequence: Synthetic polypeptide"

<400> 81

Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Tyr Ala Asp Ala Ile Phe Thr Asn Ser Tyr Arg Lys
450 455 460

Val Leu Ala Gln Leu Ser Ala Arg Lys Leu Leu Gln Asp Ile Met Ser

465 470 475 480

Arg Ala Leu Ala Gly Gly Gly Gly Ser Gly Gly Gly Gly Ser Gly Gly
485 490 495

Gly Gly Ser Ala Leu Ala Leu Gln Cys Ile Lys Val Lys Asn Asn Arg
500 505 510

Leu Pro Tyr Val Ala Asp Lys Asp Ser Ile Ser Gln Glu Ile Phe Glu
515 520 525

Asn Lys Ile Ile Thr Asp Glu Thr Asn Val Gln Asn Tyr Ser Asp Lys
530 535 540

Phe Ser Leu Asp Glu Ser Ile Leu Asp Gly Gln Val Pro Ile Asn Pro
545 550 555 560

Glu Ile Val Asp Pro Leu Leu Pro Asn Val Asn Met Glu Pro Leu Asn
565 570 575

Leu Pro Gly Glu Glu Ile Val Phe Tyr Asp Asp Ile Thr Lys Tyr Val
580 585 590

Asp Tyr Leu Asn Ser Tyr Tyr Tyr Leu Glu Ser Gln Lys Leu Ser Asn
595 600 605

Asn Val Glu Asn Ile Thr Leu Thr Thr Ser Val Glu Glu Ala Leu Gly
610 615 620

Tyr Ser Asn Lys Ile Tyr Thr Phe Leu Pro Ser Leu Ala Glu Lys Val
625 630 635 640

Asn Lys Gly Val Gln Ala Gly Leu Phe Leu Asn Trp Ala Asn Glu Val
645 650 655

Val Glu Asp Phe Thr Thr Asn Ile Met Lys Lys Asp Thr Leu Asp Lys
660 665 670

Ile Ser Asp Val Ser Val Ile Ile Pro Tyr Ile Gly Pro Ala Leu Asn
675 680 685

Ile Gly Asn Ser Ala Leu Arg Gly Asn Phe Asn Gln Ala Phe Ala Thr
690 695 700

Ala Gly Val Ala Phe Leu Leu Glu Gly Phe Pro Glu Phe Thr Ile Pro
705 710 715 720

Ala Leu Gly Val Phe Thr Phe Tyr Ser Ser Ile Gln Glu Arg Glu Lys
725 730 735

Ile Ile Lys Thr Ile Glu Asn Cys Leu Glu Gln Arg Val Lys Arg Trp
740 745 750

Lys Asp Ser Tyr Gln Trp Met Val Ser Asn Trp Leu Ser Arg Ile Thr
755 760 765

Thr Gln Phe Asn His Ile Asn Tyr Gln Met Tyr Asp Ser Leu Ser Tyr
770 775 780

Gln Ala Asp Ala Ile Lys Ala Lys Ile Asp Leu Glu Tyr Lys Lys Tyr
785 790 795 800

Ser Gly Ser Asp Lys Glu Asn Ile Lys Ser Gln Val Glu Asn Leu Lys
805 810 815

Asn Ser Leu Asp Val Lys Ile Ser Glu Ala Met Asn Asn Ile Asn Lys
820 825 830

Phe Ile Arg Glu Cys Ser Val Thr Tyr Leu Phe Lys Asn Met Leu Pro
835 840 845

Lys Val Ile Asp Glu Leu Asn Lys Phe Asp Leu Arg Thr Lys Thr Glu
850 855 860

Leu Ile Asn Leu Ile Asp Ser His Asn Ile Ile Leu Val Gly Glu Val
865 870 875 880

Asp Arg Leu Lys Ala Lys Val Asn Glu Ser Phe Glu Asn Thr Met Pro
885 890 895

Phe Asn Ile Phe Ser Tyr Thr Asn Asn Ser Leu Leu Lys Asp Ile Ile
900 905 910

Asn Glu Tyr Phe Asn
915

<210> 82
<211> 917
<212> PRT
<213> Artificial Sequence

<220>
<221> source
<223> /note="Description of Artificial Sequence: Synthetic polypeptide"

<400> 82

Thr Trp Pro Val Lys Asp Phe Asn Tyr Ser Asp Pro Val Asn Asp Asn
1               5                   10                  15

Asp Ile Leu Tyr Leu Arg Ile Pro Gln Asn Lys Leu Ile Thr Thr Pro
            20                  25                  30

Val Lys Ala Phe Met Ile Thr Gln Asn Ile Trp Val Ile Pro Glu Arg
        35                  40                  45

Phe Ser Ser Asp Thr Asn Pro Ser Leu Ser Lys Pro Pro Arg Pro Thr
    50                  55                  60

Ser Lys Tyr Gln Ser Tyr Tyr Asp Pro Ser Tyr Leu Ser Thr Asp Glu
65                  70                  75                  80

Gln Lys Asp Thr Phe Leu Lys Gly Ile Ile Lys Leu Phe Lys Arg Ile
                85                  90                  95

Asn Glu Arg Asp Ile Gly Lys Lys Leu Ile Asn Tyr Leu Val Val Gly
            100                 105                 110

Ser Pro Phe Met Gly Asp Ser Ser Thr Pro Glu Asp Thr Phe Asp Phe
        115                 120                 125

Thr Arg His Thr Thr Asn Ile Ala Val Glu Lys Phe Glu Asn Gly Ser
    130                 135                 140

Trp Lys Val Thr Asn Ile Ile Thr Pro Ser Val Leu Ile Phe Gly Pro
145                 150                 155                 160

Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
225                 230                 235                 240

Asn Ile Pro Ser Asp Lys Arg Ile Arg Pro Gln Val Ser Glu Gly Phe
                245                 250                 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Tyr Ala Asp Ala Ile Phe Thr Ala Ser Tyr Arg Lys
450 455 460

Val Leu Ala Gln Leu Ser Ala Arg Lys Leu Leu Gln Asp Ile Met Ser
465 470 475 480

Arg Ala Leu Ala Gly Gly Gly Gly Ser Gly Gly Gly Gly Ser Gly Gly
485 490 495

Gly Gly Ser Ala Leu Ala Leu Gln Cys Ile Lys Val Lys Asn Asn Arg
500 505 510

Leu Pro Tyr Val Ala Asp Lys Asp Ser Ile Ser Gln Glu Ile Phe Glu
515 520 525

Asn Lys Ile Ile Thr Asp Glu Thr Asn Val Gln Asn Tyr Ser Asp Lys
530 535 540

Phe Ser Leu Asp Glu Ser Ile Leu Asp Gly Gln Val Pro Ile Asn Pro
545 550 555 560

Glu Ile Val Asp Pro Leu Leu Pro Asn Val Asn Met Glu Pro Leu Asn
565 570 575

Leu Pro Gly Glu Glu Ile Val Phe Tyr Asp Asp Ile Thr Lys Tyr Val
580 585 590

Asp Tyr Leu Asn Ser Tyr Tyr Tyr Leu Glu Ser Gln Lys Leu Ser Asn
595 600 605

Asn Val Glu Asn Ile Thr Leu Thr Thr Ser Val Glu Glu Ala Leu Gly
610 615 620

Tyr Ser Asn Lys Ile Tyr Thr Phe Leu Pro Ser Leu Ala Glu Lys Val
625 630 635 640

Asn Lys Gly Val Gln Ala Gly Leu Phe Leu Asn Trp Ala Asn Glu Val
645 650 655

Val Glu Asp Phe Thr Thr Asn Ile Met Lys Lys Asp Thr Leu Asp Lys
660 665 670

Ile Ser Asp Val Ser Val Ile Ile Pro Tyr Ile Gly Pro Ala Leu Asn
675 680 685

Ile Gly Asn Ser Ala Leu Arg Gly Asn Phe Asn Gln Ala Phe Ala Thr
690 695 700

Ala Gly Val Ala Phe Leu Leu Glu Gly Phe Pro Glu Phe Thr Ile Pro
705 710 715 720

Ala Leu Gly Val Phe Thr Phe Tyr Ser Ser Ile Gln Glu Arg Glu Lys
725 730 735

Ile Ile Lys Thr Ile Glu Asn Cys Leu Glu Gln Arg Val Lys Arg Trp
740 745 750

Lys Asp Ser Tyr Gln Trp Met Val Ser Asn Trp Leu Ser Arg Ile Thr

755 760 765

Thr Gln Phe Asn His Ile Asn Tyr Gln Met Tyr Asp Ser Leu Ser Tyr
770 775 780

Gln Ala Asp Ala Ile Lys Ala Lys Ile Asp Leu Glu Tyr Lys Lys Tyr
785 790 795 800

Ser Gly Ser Asp Lys Glu Asn Ile Lys Ser Gln Val Glu Asn Leu Lys
805 810 815

Asn Ser Leu Asp Val Lys Ile Ser Glu Ala Met Asn Asn Ile Asn Lys
820 825 830

Phe Ile Arg Glu Cys Ser Val Thr Tyr Leu Phe Lys Asn Met Leu Pro
835 840 845

Lys Val Ile Asp Glu Leu Asn Lys Phe Asp Leu Arg Thr Lys Thr Glu
850 855 860

Leu Ile Asn Leu Ile Asp Ser His Asn Ile Ile Leu Val Gly Glu Val
865 870 875 880

Asp Arg Leu Lys Ala Lys Val Asn Glu Ser Phe Glu Asn Thr Met Pro
885 890 895

Phe Asn Ile Phe Ser Tyr Thr Asn Asn Ser Leu Leu Lys Asp Ile Ile
900 905 910

Asn Glu Tyr Phe Asn
915

<210> 83
<211> 917
<212> PRT
<213> Artificial Sequence

<220>
<221> source
<223> /note="Description of Artificial Sequence: Synthetic polypeptide"

<400> 83
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg

35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
    290                 295                 300

Asn Ile Asn Lys Thr Ile Pro Ser Ser Trp Ile Ser Asn Ile Asp Lys
305                 310                 315                 320

Tyr Lys Lys Ile Phe Ser Glu Lys Tyr Asn Phe Asp Lys Asp Asn Thr
                325                 330                 335

Gly Asn Phe Val Val Asn Ile Asp Lys Phe Asn Ser Leu Tyr Ser Asp
            340                 345                 350

Leu Thr Asn Val Met Ser Glu Val Val Tyr Ser Ser Gln Tyr Asn Val
        355                 360                 365

Lys Asn Arg Thr His Tyr Phe Ser Arg His Tyr Leu Pro Val Phe Ala
    370                 375                 380

Asn Ile Leu Asp Asp Asn Ile Tyr Thr Ile Arg Asp Gly Phe Asn Leu
385                 390                 395                 400

Thr Asn Lys Gly Phe Asn Ile Glu Asn Ser Gly Gln Asn Ile Glu Arg
                405                 410                 415

Asn Pro Ala Leu Gln Lys Leu Ser Ser Glu Ser Val Val Asp Leu Phe
            420                 425                 430

Thr Lys Val Cys Val Asp Gly Ile Ile Thr Ser Lys Thr Lys Ser Asp
        435                 440                 445

Asp Asp Asp Lys Tyr Ala Asp Ala Ile Phe Thr Ala Ala Tyr Arg Lys
    450                 455                 460

Val Leu Ala Gln Leu Ser Ala Arg Lys Ala Leu Gln Asp Ile Ala Ser
465                 470                 475                 480

Arg Ala Leu Ala Gly Gly Gly Gly Ser Gly Gly Gly Gly Ser Gly Gly
                485                 490                 495

Gly Gly Ser Ala Leu Ala Leu Gln Cys Ile Lys Val Lys Asn Asn Arg
            500                 505                 510

Leu Pro Tyr Val Ala Asp Lys Asp Ser Ile Ser Gln Glu Ile Phe Glu
        515                 520                 525

Asn Lys Ile Ile Thr Asp Glu Thr Asn Val Gln Asn Tyr Ser Asp Lys
    530                 535                 540

Phe Ser Leu Asp Glu Ser Ile Leu Asp Gly Gln Val Pro Ile Asn Pro
545 550 555 560

Glu Ile Val Asp Pro Leu Leu Pro Asn Val Asn Met Glu Pro Leu Asn
565 570 575

Leu Pro Gly Glu Glu Ile Val Phe Tyr Asp Asp Ile Thr Lys Tyr Val
580 585 590

Asp Tyr Leu Asn Ser Tyr Tyr Tyr Leu Glu Ser Gln Lys Leu Ser Asn
595 600 605

Asn Val Glu Asn Ile Thr Leu Thr Thr Ser Val Glu Glu Ala Leu Gly
610 615 620

Tyr Ser Asn Lys Ile Tyr Thr Phe Leu Pro Ser Leu Ala Glu Lys Val
625 630 635 640

Asn Lys Gly Val Gln Ala Gly Leu Phe Leu Asn Trp Ala Asn Glu Val
645 650 655

Val Glu Asp Phe Thr Thr Asn Ile Met Lys Lys Asp Thr Leu Asp Lys
660 665 670

Ile Ser Asp Val Ser Val Ile Ile Pro Tyr Ile Gly Pro Ala Leu Asn
675 680 685

Ile Gly Asn Ser Ala Leu Arg Gly Asn Phe Asn Gln Ala Phe Ala Thr
690 695 700

Ala Gly Val Ala Phe Leu Leu Glu Gly Phe Pro Glu Phe Thr Ile Pro
705 710 715 720

Ala Leu Gly Val Phe Thr Phe Tyr Ser Ser Ile Gln Glu Arg Glu Lys
725 730 735

Ile Ile Lys Thr Ile Glu Asn Cys Leu Glu Gln Arg Val Lys Arg Trp
740 745 750

Lys Asp Ser Tyr Gln Trp Met Val Ser Asn Trp Leu Ser Arg Ile Thr
755 760 765

Thr Gln Phe Asn His Ile Asn Tyr Gln Met Tyr Asp Ser Leu Ser Tyr
770 775 780

Gln Ala Asp Ala Ile Lys Ala Lys Ile Asp Leu Glu Tyr Lys Lys Tyr
785 790 795 800

Ser Gly Ser Asp Lys Glu Asn Ile Lys Ser Gln Val Glu Asn Leu Lys
805 810 815

Asn Ser Leu Asp Val Lys Ile Ser Glu Ala Met Asn Asn Ile Asn Lys
820 825 830

Phe Ile Arg Glu Cys Ser Val Thr Tyr Leu Phe Lys Asn Met Leu Pro
835 840 845

Lys Val Ile Asp Glu Leu Asn Lys Phe Asp Leu Arg Thr Lys Thr Glu
850 855 860

Leu Ile Asn Leu Ile Asp Ser His Asn Ile Ile Leu Val Gly Glu Val
865 870 875 880

Asp Arg Leu Lys Ala Lys Val Asn Glu Ser Phe Glu Asn Thr Met Pro
885 890 895

Phe Asn Ile Phe Ser Tyr Thr Asn Asn Ser Leu Leu Lys Asp Ile Ile
900 905 910

Asn Glu Tyr Phe Asn
915

<210> 84
<211> 917
<212> PRT
<213> Artificial Sequence

<220>
<221> source
<223> /note="Description of Artificial Sequence: Synthetic polypeptide"

<400> 84
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr

325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Tyr Ala Asp Ala Ile Phe Thr Ala Ala Tyr Arg Lys
450 455 460

Val Leu Ala Gln Leu Ser Ala Arg Lys Ala Leu Gln Asp Ile Met Ser
465 470 475 480

Arg Ala Leu Ala Gly Gly Gly Gly Ser Gly Gly Gly Gly Ser Gly Gly
485 490 495

Gly Gly Ser Ala Leu Ala Leu Gln Cys Ile Lys Val Lys Asn Asn Arg
500 505 510

Leu Pro Tyr Val Ala Asp Lys Asp Ser Ile Ser Gln Glu Ile Phe Glu
515 520 525

Asn Lys Ile Ile Thr Asp Glu Thr Asn Val Gln Asn Tyr Ser Asp Lys
530 535 540

Phe Ser Leu Asp Glu Ser Ile Leu Asp Gly Gln Val Pro Ile Asn Pro
545 550 555 560

Glu Ile Val Asp Pro Leu Leu Pro Asn Val Asn Met Glu Pro Leu Asn
565 570 575

```
Leu Pro Gly Glu Glu Ile Val Phe Tyr Asp Asp Ile Thr Lys Tyr Val
            580                 585                 590

Asp Tyr Leu Asn Ser Tyr Tyr Tyr Leu Glu Ser Gln Lys Leu Ser Asn
        595                 600                 605

Asn Val Glu Asn Ile Thr Leu Thr Thr Ser Val Glu Glu Ala Leu Gly
    610                 615                 620

Tyr Ser Asn Lys Ile Tyr Thr Phe Leu Pro Ser Leu Ala Glu Lys Val
625                 630                 635                 640

Asn Lys Gly Val Gln Ala Gly Leu Phe Leu Asn Trp Ala Asn Glu Val
                645                 650                 655

Val Glu Asp Phe Thr Thr Asn Ile Met Lys Lys Asp Thr Leu Asp Lys
            660                 665                 670

Ile Ser Asp Val Ser Val Ile Ile Pro Tyr Ile Gly Pro Ala Leu Asn
        675                 680                 685

Ile Gly Asn Ser Ala Leu Arg Gly Asn Phe Asn Gln Ala Phe Ala Thr
    690                 695                 700

Ala Gly Val Ala Phe Leu Leu Glu Gly Phe Pro Glu Phe Thr Ile Pro
705                 710                 715                 720

Ala Leu Gly Val Phe Thr Phe Tyr Ser Ser Ile Gln Glu Arg Glu Lys
                725                 730                 735

Ile Ile Lys Thr Ile Glu Asn Cys Leu Glu Gln Arg Val Lys Arg Trp
            740                 745                 750

Lys Asp Ser Tyr Gln Trp Met Val Ser Asn Trp Leu Ser Arg Ile Thr
        755                 760                 765

Thr Gln Phe Asn His Ile Asn Tyr Gln Met Tyr Asp Ser Leu Ser Tyr
    770                 775                 780

Gln Ala Asp Ala Ile Lys Ala Lys Ile Asp Leu Glu Tyr Lys Lys Tyr
785                 790                 795                 800

Ser Gly Ser Asp Lys Glu Asn Ile Lys Ser Gln Val Glu Asn Leu Lys
                805                 810                 815

Asn Ser Leu Asp Val Lys Ile Ser Glu Ala Met Asn Asn Ile Asn Lys
            820                 825                 830
```

Phe Ile Arg Glu Cys Ser Val Thr Tyr Leu Phe Lys Asn Met Leu Pro
835 840 845

Lys Val Ile Asp Glu Leu Asn Lys Phe Asp Leu Arg Thr Lys Thr Glu
850 855 860

Leu Ile Asn Leu Ile Asp Ser His Asn Ile Ile Leu Val Gly Glu Val
865 870 875 880

Asp Arg Leu Lys Ala Lys Val Asn Glu Ser Phe Glu Asn Thr Met Pro
885 890 895

Phe Asn Ile Phe Ser Tyr Thr Asn Asn Ser Leu Leu Lys Asp Ile Ile
900 905 910

Asn Glu Tyr Phe Asn
915

<210> 85
<211> 920
<212> PRT
<213> Artificial Sequence

<220>
<221> source
<223> /note="Description of Artificial Sequence: Synthetic polypeptide"

<400> 85
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys His Val Asp Ala Ile Phe Thr Gln Ser Tyr Arg Lys
450 455 460

Val Leu Ala Gln Leu Ser Ala Arg Lys Ala Leu Gln Asp Ile Leu Ser
465 470 475 480

Arg Gln Gln Gly Ala Leu Ala Gly Gly Gly Gly Ser Gly Gly Gly Gly
485 490 495

Ser Gly Gly Gly Gly Ser Ala Leu Ala Leu Gln Cys Ile Lys Val Lys
500 505 510

Asn Asn Arg Leu Pro Tyr Val Ala Asp Lys Asp Ser Ile Ser Gln Glu
515 520 525

Ile Phe Glu Asn Lys Ile Ile Thr Asp Glu Thr Asn Val Gln Asn Tyr
530 535 540

Ser Asp Lys Phe Ser Leu Asp Glu Ser Ile Leu Asp Gly Gln Val Pro
545 550 555 560

Ile Asn Pro Glu Ile Val Asp Pro Leu Leu Pro Asn Val Asn Met Glu
565 570 575

Pro Leu Asn Leu Pro Gly Glu Glu Ile Val Phe Tyr Asp Asp Ile Thr
580 585 590

Lys Tyr Val Asp Tyr Leu Asn Ser Tyr Tyr Tyr Leu Glu Ser Gln Lys
595 600 605

Leu Ser Asn Asn Val Glu Asn Ile Thr Leu Thr Thr Ser Val Glu Glu

610 615 620

Ala Leu Gly Tyr Ser Asn Lys Ile Tyr Thr Phe Leu Pro Ser Leu Ala
625 630 635 640

Glu Lys Val Asn Lys Gly Val Gln Ala Gly Leu Phe Leu Asn Trp Ala
645 650 655

Asn Glu Val Val Glu Asp Phe Thr Thr Asn Ile Met Lys Lys Asp Thr
660 665 670

Leu Asp Lys Ile Ser Asp Val Ser Val Ile Ile Pro Tyr Ile Gly Pro
675 680 685

Ala Leu Asn Ile Gly Asn Ser Ala Leu Arg Gly Asn Phe Asn Gln Ala
690 695 700

Phe Ala Thr Ala Gly Val Ala Phe Leu Leu Glu Gly Phe Pro Glu Phe
705 710 715 720

Thr Ile Pro Ala Leu Gly Val Phe Thr Phe Tyr Ser Ser Ile Gln Glu
725 730 735

Arg Glu Lys Ile Ile Lys Thr Ile Glu Asn Cys Leu Glu Gln Arg Val
740 745 750

Lys Arg Trp Lys Asp Ser Tyr Gln Trp Met Val Ser Asn Trp Leu Ser
755 760 765

Arg Ile Thr Thr Gln Phe Asn His Ile Asn Tyr Gln Met Tyr Asp Ser
770 775 780

Leu Ser Tyr Gln Ala Asp Ala Ile Lys Ala Lys Ile Asp Leu Glu Tyr
785 790 795 800

Lys Lys Tyr Ser Gly Ser Asp Lys Glu Asn Ile Lys Ser Gln Val Glu
805 810 815

Asn Leu Lys Asn Ser Leu Asp Val Lys Ile Ser Glu Ala Met Asn Asn
820 825 830

Ile Asn Lys Phe Ile Arg Glu Cys Ser Val Thr Tyr Leu Phe Lys Asn
835 840 845

Met Leu Pro Lys Val Ile Asp Glu Leu Asn Lys Phe Asp Leu Arg Thr
850 855 860

Lys Thr Glu Leu Ile Asn Leu Ile Asp Ser His Asn Ile Ile Leu Val
865 870 875 880

Gly Glu Val Asp Arg Leu Lys Ala Lys Val Asn Glu Ser Phe Glu Asn
885 890 895

Thr Met Pro Phe Asn Ile Phe Ser Tyr Thr Asn Asn Ser Leu Leu Lys
900 905 910

Asp Ile Ile Asn Glu Tyr Phe Asn
915 920

<210> 86
<211> 917
<212> PRT
<213> Artificial Sequence

<220>
<221> source
<223> /note="Description of Artificial Sequence: Synthetic polypeptide"

<400> 86
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

```
Trp Lys Val Thr Asn Ile Ile Thr Pro Ser Val Leu Ile Phe Gly Pro
145                 150                 155                 160

Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
225                 230                 235                 240

Asn Ile Pro Ser Asp Lys Arg Ile Arg Pro Gln Val Ser Glu Gly Phe
                245                 250                 255

Phe Ser Gln Asp Gly Pro Asn Val Gln Phe Glu Glu Leu Tyr Thr Phe
            260                 265                 270

Gly Gly Leu Asp Val Glu Ile Ile Pro Gln Ile Glu Arg Ser Gln Leu
        275                 280                 285

Arg Glu Lys Ala Leu Gly His Tyr Lys Asp Ile Ala Lys Arg Leu Asn
    290                 295                 300

Asn Ile Asn Lys Thr Ile Pro Ser Ser Trp Ile Ser Asn Ile Asp Lys
305                 310                 315                 320

Tyr Lys Lys Ile Phe Ser Glu Lys Tyr Asn Phe Asp Lys Asp Asn Thr
                325                 330                 335

Gly Asn Phe Val Val Asn Ile Asp Lys Phe Asn Ser Leu Tyr Ser Asp
            340                 345                 350

Leu Thr Asn Val Met Ser Glu Val Val Tyr Ser Ser Gln Tyr Asn Val
        355                 360                 365

Lys Asn Arg Thr His Tyr Phe Ser Arg His Tyr Leu Pro Val Phe Ala
    370                 375                 380

Asn Ile Leu Asp Asp Asn Ile Tyr Thr Ile Arg Asp Gly Phe Asn Leu
385                 390                 395                 400
```

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys His Val Asp Ala Ile Phe Thr Ser Ser Tyr Arg Lys
450 455 460

Val Leu Ala Gln Leu Ser Ala Arg Lys Leu Leu Gln Asp Ile Leu Ser
465 470 475 480

Arg Ala Leu Ala Gly Gly Gly Gly Ser Gly Gly Gly Gly Ser Gly Gly
485 490 495

Gly Gly Ser Ala Leu Ala Leu Gln Cys Ile Lys Val Lys Asn Asn Arg
500 505 510

Leu Pro Tyr Val Ala Asp Lys Asp Ser Ile Ser Gln Glu Ile Phe Glu
515 520 525

Asn Lys Ile Ile Thr Asp Glu Thr Asn Val Gln Asn Tyr Ser Asp Lys
530 535 540

Phe Ser Leu Asp Glu Ser Ile Leu Asp Gly Gln Val Pro Ile Asn Pro
545 550 555 560

Glu Ile Val Asp Pro Leu Leu Pro Asn Val Asn Met Glu Pro Leu Asn
565 570 575

Leu Pro Gly Glu Glu Ile Val Phe Tyr Asp Asp Ile Thr Lys Tyr Val
580 585 590

Asp Tyr Leu Asn Ser Tyr Tyr Tyr Leu Glu Ser Gln Lys Leu Ser Asn
595 600 605

Asn Val Glu Asn Ile Thr Leu Thr Thr Ser Val Glu Glu Ala Leu Gly
610 615 620

Tyr Ser Asn Lys Ile Tyr Thr Phe Leu Pro Ser Leu Ala Glu Lys Val
625 630 635 640

Asn Lys Gly Val Gln Ala Gly Leu Phe Leu Asn Trp Ala Asn Glu Val
645 650 655

Val Glu Asp Phe Thr Thr Asn Ile Met Lys Lys Asp Thr Leu Asp Lys
660 665 670

Ile Ser Asp Val Ser Val Ile Ile Pro Tyr Ile Gly Pro Ala Leu Asn
675 680 685

Ile Gly Asn Ser Ala Leu Arg Gly Asn Phe Asn Gln Ala Phe Ala Thr
690 695 700

Ala Gly Val Ala Phe Leu Leu Glu Gly Phe Pro Glu Phe Thr Ile Pro
705 710 715 720

Ala Leu Gly Val Phe Thr Phe Tyr Ser Ser Ile Gln Glu Arg Glu Lys
725 730 735

Ile Ile Lys Thr Ile Glu Asn Cys Leu Glu Gln Arg Val Lys Arg Trp
740 745 750

Lys Asp Ser Tyr Gln Trp Met Val Ser Asn Trp Leu Ser Arg Ile Thr
755 760 765

Thr Gln Phe Asn His Ile Asn Tyr Gln Met Tyr Asp Ser Leu Ser Tyr
770 775 780

Gln Ala Asp Ala Ile Lys Ala Lys Ile Asp Leu Glu Tyr Lys Lys Tyr
785 790 795 800

Ser Gly Ser Asp Lys Glu Asn Ile Lys Ser Gln Val Glu Asn Leu Lys
805 810 815

Asn Ser Leu Asp Val Lys Ile Ser Glu Ala Met Asn Asn Ile Asn Lys
820 825 830

Phe Ile Arg Glu Cys Ser Val Thr Tyr Leu Phe Lys Asn Met Leu Pro
835 840 845

Lys Val Ile Asp Glu Leu Asn Lys Phe Asp Leu Arg Thr Lys Thr Glu
850 855 860

Leu Ile Asn Leu Ile Asp Ser His Asn Ile Ile Leu Val Gly Glu Val
865 870 875 880

Asp Arg Leu Lys Ala Lys Val Asn Glu Ser Phe Glu Asn Thr Met Pro
885 890 895

Phe Asn Ile Phe Ser Tyr Thr Asn Asn Ser Leu Leu Lys Asp Ile Ile

900 905 910

Asn Glu Tyr Phe Asn
915

<210> 87
<211> 917
<212> PRT
<213> Artificial Sequence

<220>
<221> source
<223> /note="Description of Artificial Sequence: Synthetic polypeptide"

<400> 87
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys

180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys His Val Asp Ala Ile Phe Thr Thr Ser Tyr Arg Lys
450 455 460

Val Leu Ala Gln Leu Ser Ala Arg Lys Leu Leu Gln Asp Ile Leu Ser
465 470 475 480

Arg Ala Leu Ala Gly Gly Gly Gly Ser Gly Gly Gly Gly Ser Gly Gly
485 490 495

Gly Gly Ser Ala Leu Ala Leu Gln Cys Ile Lys Val Lys Asn Asn Arg
500 505 510

Leu Pro Tyr Val Ala Asp Lys Asp Ser Ile Ser Gln Glu Ile Phe Glu
515 520 525

Asn Lys Ile Ile Thr Asp Glu Thr Asn Val Gln Asn Tyr Ser Asp Lys
530 535 540

Phe Ser Leu Asp Glu Ser Ile Leu Asp Gly Gln Val Pro Ile Asn Pro
545 550 555 560

Glu Ile Val Asp Pro Leu Leu Pro Asn Val Asn Met Glu Pro Leu Asn
565 570 575

Leu Pro Gly Glu Glu Ile Val Phe Tyr Asp Asp Ile Thr Lys Tyr Val
580 585 590

Asp Tyr Leu Asn Ser Tyr Tyr Tyr Leu Glu Ser Gln Lys Leu Ser Asn
595 600 605

Asn Val Glu Asn Ile Thr Leu Thr Thr Ser Val Glu Glu Ala Leu Gly
610 615 620

Tyr Ser Asn Lys Ile Tyr Thr Phe Leu Pro Ser Leu Ala Glu Lys Val
625 630 635 640

Asn Lys Gly Val Gln Ala Gly Leu Phe Leu Asn Trp Ala Asn Glu Val
645 650 655

Val Glu Asp Phe Thr Thr Asn Ile Met Lys Lys Asp Thr Leu Asp Lys
660 665 670

Ile Ser Asp Val Ser Val Ile Ile Pro Tyr Ile Gly Pro Ala Leu Asn
675 680 685

Ile Gly Asn Ser Ala Leu Arg Gly Asn Phe Asn Gln Ala Phe Ala Thr
690 695 700

Ala Gly Val Ala Phe Leu Leu Glu Gly Phe Pro Glu Phe Thr Ile Pro
705 710 715 720

Ala Leu Gly Val Phe Thr Phe Tyr Ser Ser Ile Gln Glu Arg Glu Lys
725 730 735

Ile Ile Lys Thr Ile Glu Asn Cys Leu Glu Gln Arg Val Lys Arg Trp
740 745 750

Lys Asp Ser Tyr Gln Trp Met Val Ser Asn Trp Leu Ser Arg Ile Thr
755 760 765

Thr Gln Phe Asn His Ile Asn Tyr Gln Met Tyr Asp Ser Leu Ser Tyr
770 775 780

Gln Ala Asp Ala Ile Lys Ala Lys Ile Asp Leu Glu Tyr Lys Lys Tyr
785 790 795 800

Ser Gly Ser Asp Lys Glu Asn Ile Lys Ser Gln Val Glu Asn Leu Lys
805 810 815

Asn Ser Leu Asp Val Lys Ile Ser Glu Ala Met Asn Asn Ile Asn Lys
820 825 830

Phe Ile Arg Glu Cys Ser Val Thr Tyr Leu Phe Lys Asn Met Leu Pro
835 840 845

Lys Val Ile Asp Glu Leu Asn Lys Phe Asp Leu Arg Thr Lys Thr Glu
850 855 860

Leu Ile Asn Leu Ile Asp Ser His Asn Ile Ile Leu Val Gly Glu Val
865 870 875 880

Asp Arg Leu Lys Ala Lys Val Asn Glu Ser Phe Glu Asn Thr Met Pro
885 890 895

Phe Asn Ile Phe Ser Tyr Thr Asn Asn Ser Leu Leu Lys Asp Ile Ile
900 905 910

Asn Glu Tyr Phe Asn
915

<210> 88
<211> 917

<212> PRT
<213> Artificial Sequence

<220>
<221> source
<223> /note="Description of Artificial Sequence: Synthetic
polypeptide"

<400> 88
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Tyr Ala Asp Ala Ile Phe Thr Gln Ser Tyr Arg Lys
450 455 460

Val Leu Ala Gln Leu Ser Ala Arg Lys Ala Leu Gln Asp Ile Leu Asn

465 470 475 480

Arg Ala Leu Ala Gly Gly Gly Gly Ser Gly Gly Gly Gly Ser Gly Gly
485 490 495

Gly Gly Ser Ala Leu Ala Leu Gln Cys Ile Lys Val Lys Asn Asn Arg
500 505 510

Leu Pro Tyr Val Ala Asp Lys Asp Ser Ile Ser Gln Glu Ile Phe Glu
515 520 525

Asn Lys Ile Ile Thr Asp Glu Thr Asn Val Gln Asn Tyr Ser Asp Lys
530 535 540

Phe Ser Leu Asp Glu Ser Ile Leu Asp Gly Gln Val Pro Ile Asn Pro
545 550 555 560

Glu Ile Val Asp Pro Leu Leu Pro Asn Val Asn Met Glu Pro Leu Asn
565 570 575

Leu Pro Gly Glu Glu Ile Val Phe Tyr Asp Asp Ile Thr Lys Tyr Val
580 585 590

Asp Tyr Leu Asn Ser Tyr Tyr Tyr Leu Glu Ser Gln Lys Leu Ser Asn
595 600 605

Asn Val Glu Asn Ile Thr Leu Thr Thr Ser Val Glu Glu Ala Leu Gly
610 615 620

Tyr Ser Asn Lys Ile Tyr Thr Phe Leu Pro Ser Leu Ala Glu Lys Val
625 630 635 640

Asn Lys Gly Val Gln Ala Gly Leu Phe Leu Asn Trp Ala Asn Glu Val
645 650 655

Val Glu Asp Phe Thr Thr Asn Ile Met Lys Lys Asp Thr Leu Asp Lys
660 665 670

Ile Ser Asp Val Ser Val Ile Ile Pro Tyr Ile Gly Pro Ala Leu Asn
675 680 685

Ile Gly Asn Ser Ala Leu Arg Gly Asn Phe Asn Gln Ala Phe Ala Thr
690 695 700

Ala Gly Val Ala Phe Leu Leu Glu Gly Phe Pro Glu Phe Thr Ile Pro
705 710 715 720

```
Ala Leu Gly Val Phe Thr Phe Tyr Ser Ser Ile Gln Glu Arg Glu Lys
                725                 730                 735

Ile Ile Lys Thr Ile Glu Asn Cys Leu Glu Gln Arg Val Lys Arg Trp
            740                 745                 750

Lys Asp Ser Tyr Gln Trp Met Val Ser Asn Trp Leu Ser Arg Ile Thr
        755                 760                 765

Thr Gln Phe Asn His Ile Asn Tyr Gln Met Tyr Asp Ser Leu Ser Tyr
    770                 775                 780

Gln Ala Asp Ala Ile Lys Ala Lys Ile Asp Leu Glu Tyr Lys Lys Tyr
785                 790                 795                 800

Ser Gly Ser Asp Lys Glu Asn Ile Lys Ser Gln Val Glu Asn Leu Lys
                805                 810                 815

Asn Ser Leu Asp Val Lys Ile Ser Glu Ala Met Asn Asn Ile Asn Lys
            820                 825                 830

Phe Ile Arg Glu Cys Ser Val Thr Tyr Leu Phe Lys Asn Met Leu Pro
        835                 840                 845

Lys Val Ile Asp Glu Leu Asn Lys Phe Asp Leu Arg Thr Lys Thr Glu
    850                 855                 860

Leu Ile Asn Leu Ile Asp Ser His Asn Ile Ile Leu Val Gly Glu Val
865                 870                 875                 880

Asp Arg Leu Lys Ala Lys Val Asn Glu Ser Phe Glu Asn Thr Met Pro
                885                 890                 895

Phe Asn Ile Phe Ser Tyr Thr Asn Asn Ser Leu Leu Lys Asp Ile Ile
            900                 905                 910

Asn Glu Tyr Phe Asn
        915


<210> 89
<211> 917
<212> PRT
<213> Artificial Sequence

<220>
<221> source
<223> /note="Description of Artificial Sequence: Synthetic
      polypeptide"

<400> 89
```

Thr Trp Pro Val Lys Asp Phe Asn Tyr Ser Asp Pro Val Asn Asp Asn
1               5                   10                  15

Asp Ile Leu Tyr Leu Arg Ile Pro Gln Asn Lys Leu Ile Thr Thr Pro
            20                  25                  30

Val Lys Ala Phe Met Ile Thr Gln Asn Ile Trp Val Ile Pro Glu Arg
        35                  40                  45

Phe Ser Ser Asp Thr Asn Pro Ser Leu Ser Lys Pro Pro Arg Pro Thr
    50                  55                  60

Ser Lys Tyr Gln Ser Tyr Tyr Asp Pro Ser Tyr Leu Ser Thr Asp Glu
65                  70                  75                  80

Gln Lys Asp Thr Phe Leu Lys Gly Ile Ile Lys Leu Phe Lys Arg Ile
                85                  90                  95

Asn Glu Arg Asp Ile Gly Lys Lys Leu Ile Asn Tyr Leu Val Val Gly
            100                 105                 110

Ser Pro Phe Met Gly Asp Ser Ser Thr Pro Glu Asp Thr Phe Asp Phe
        115                 120                 125

Thr Arg His Thr Thr Asn Ile Ala Val Glu Lys Phe Glu Asn Gly Ser
    130                 135                 140

Trp Lys Val Thr Asn Ile Ile Thr Pro Ser Val Leu Ile Phe Gly Pro
145                 150                 155                 160

Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
225                 230                 235                 240

Asn Ile Pro Ser Asp Lys Arg Ile Arg Pro Gln Val Ser Glu Gly Phe
                245                 250                 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Asp
435 440 445

Asp Asp Asp Lys Tyr Ala Asp Ala Ile Phe Thr Gln Ser Tyr Arg Lys
450 455 460

Val Leu Ala Gln Leu Ser Ala Arg Lys Ala Leu Gln Asp Ile Leu Ser
465 470 475 480

Arg Ala Leu Ala Gly Gly Gly Gly Ser Gly Gly Gly Gly Ser Gly Gly
485 490 495

Gly Gly Ser Ala Leu Ala Leu Gln Cys Ile Lys Val Lys Asn Asn Arg
500 505 510

Leu Pro Tyr Val Ala Asp Lys Asp Ser Ile Ser Gln Glu Ile Phe Glu
515 520 525

Asn Lys Ile Ile Thr Asp Glu Thr Asn Val Gln Asn Tyr Ser Asp Lys
530 535 540

Phe Ser Leu Asp Glu Ser Ile Leu Asp Gly Gln Val Pro Ile Asn Pro
545 550 555 560

Glu Ile Val Asp Pro Leu Leu Pro Asn Val Asn Met Glu Pro Leu Asn
565 570 575

Leu Pro Gly Glu Glu Ile Val Phe Tyr Asp Asp Ile Thr Lys Tyr Val
580 585 590

Asp Tyr Leu Asn Ser Tyr Tyr Tyr Leu Glu Ser Gln Lys Leu Ser Asn
595 600 605

Asn Val Glu Asn Ile Thr Leu Thr Thr Ser Val Glu Glu Ala Leu Gly
610 615 620

Tyr Ser Asn Lys Ile Tyr Thr Phe Leu Pro Ser Leu Ala Glu Lys Val
625 630 635 640

Asn Lys Gly Val Gln Ala Gly Leu Phe Leu Asn Trp Ala Asn Glu Val
645 650 655

Val Glu Asp Phe Thr Thr Asn Ile Met Lys Lys Asp Thr Leu Asp Lys
660 665 670

Ile Ser Asp Val Ser Val Ile Ile Pro Tyr Ile Gly Pro Ala Leu Asn
675 680 685

Ile Gly Asn Ser Ala Leu Arg Gly Asn Phe Asn Gln Ala Phe Ala Thr
690 695 700

Ala Gly Val Ala Phe Leu Leu Glu Gly Phe Pro Glu Phe Thr Ile Pro
705 710 715 720

Ala Leu Gly Val Phe Thr Phe Tyr Ser Ser Ile Gln Glu Arg Glu Lys
725 730 735

Ile Ile Lys Thr Ile Glu Asn Cys Leu Glu Gln Arg Val Lys Arg Trp
740 745 750

Lys Asp Ser Tyr Gln Trp Met Val Ser Asn Trp Leu Ser Arg Ile Thr

755 760 765

Thr Gln Phe Asn His Ile Asn Tyr Gln Met Tyr Asp Ser Leu Ser Tyr
770 775 780

Gln Ala Asp Ala Ile Lys Ala Lys Ile Asp Leu Glu Tyr Lys Lys Tyr
785 790 795 800

Ser Gly Ser Asp Lys Glu Asn Ile Lys Ser Gln Val Glu Asn Leu Lys
805 810 815

Asn Ser Leu Asp Val Lys Ile Ser Glu Ala Met Asn Asn Ile Asn Lys
820 825 830

Phe Ile Arg Glu Cys Ser Val Thr Tyr Leu Phe Lys Asn Met Leu Pro
835 840 845

Lys Val Ile Asp Glu Leu Asn Lys Phe Asp Leu Arg Thr Lys Thr Glu
850 855 860

Leu Ile Asn Leu Ile Asp Ser His Asn Ile Ile Leu Val Gly Glu Val
865 870 875 880

Asp Arg Leu Lys Ala Lys Val Asn Glu Ser Phe Glu Asn Thr Met Pro
885 890 895

Phe Asn Ile Phe Ser Tyr Thr Asn Asn Ser Leu Leu Lys Asp Ile Ile
900 905 910

Asn Glu Tyr Phe Asn
915

<210> 90
<211> 916
<212> PRT
<213> Artificial Sequence

<220>
<221> source
<223> /note="Description of Artificial Sequence: Synthetic polypeptide"

<400> 90
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg

```
        35                  40                  45

Phe Ser Ser Asp Thr Asn Pro Ser Leu Ser Lys Pro Pro Arg Pro Thr
    50                  55                  60

Ser Lys Tyr Gln Ser Tyr Tyr Asp Pro Ser Tyr Leu Ser Thr Asp Glu
65                  70                  75                  80

Gln Lys Asp Thr Phe Leu Lys Gly Ile Ile Lys Leu Phe Lys Arg Ile
                85                  90                  95

Asn Glu Arg Asp Ile Gly Lys Lys Leu Ile Asn Tyr Leu Val Val Gly
            100                 105                 110

Ser Pro Phe Met Gly Asp Ser Ser Thr Pro Glu Asp Thr Phe Asp Phe
        115                 120                 125

Thr Arg His Thr Thr Asn Ile Ala Val Glu Lys Phe Glu Asn Gly Ser
    130                 135                 140

Trp Lys Val Thr Asn Ile Ile Thr Pro Ser Val Leu Ile Phe Gly Pro
145                 150                 155                 160

Leu Pro Asn Ile Leu Asp Tyr Thr Ala Ser Leu Thr Leu Gln Gly Gln
                165                 170                 175

Gln Ser Asn Pro Ser Phe Glu Gly Phe Gly Thr Leu Ser Ile Leu Lys
            180                 185                 190

Val Ala Pro Glu Phe Leu Leu Thr Phe Ser Asp Val Thr Ser Asn Gln
        195                 200                 205

Ser Ser Ala Val Leu Gly Lys Ser Ile Phe Cys Met Asp Pro Val Ile
    210                 215                 220

Ala Leu Met His Glu Leu Thr His Ser Leu His Gln Leu Tyr Gly Ile
225                 230                 235                 240

Asn Ile Pro Ser Asp Lys Arg Ile Arg Pro Gln Val Ser Glu Gly Phe
                245                 250                 255

Phe Ser Gln Asp Gly Pro Asn Val Gln Phe Glu Glu Leu Tyr Thr Phe
            260                 265                 270

Gly Gly Leu Asp Val Glu Ile Ile Pro Gln Ile Glu Arg Ser Gln Leu
        275                 280                 285
```

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Ile
435 440 445

Glu Gly Arg Tyr Ala Asp Ala Ile Phe Thr Ala Ser Tyr Arg Lys Val
450 455 460

Leu Gly Gln Leu Ser Ala Arg Lys Leu Leu Gln Asp Ile Leu Ser Arg
465 470 475 480

Ala Leu Ala Gly Gly Gly Gly Ser Gly Gly Gly Gly Ser Gly Gly Gly
485 490 495

Gly Ser Ala Leu Ala Leu Gln Cys Ile Lys Val Lys Asn Asn Arg Leu
500 505 510

Pro Tyr Val Ala Asp Lys Asp Ser Ile Ser Gln Glu Ile Phe Glu Asn
515 520 525

Lys Ile Ile Thr Asp Glu Thr Asn Val Gln Asn Tyr Ser Asp Lys Phe
530 535 540

Ser Leu Asp Glu Ser Ile Leu Asp Gly Gln Val Pro Ile Asn Pro Glu
545 550 555 560

Ile Val Asp Pro Leu Leu Pro Asn Val Asn Met Glu Pro Leu Asn Leu
565 570 575

Pro Gly Glu Glu Ile Val Phe Tyr Asp Asp Ile Thr Lys Tyr Val Asp
580 585 590

Tyr Leu Asn Ser Tyr Tyr Tyr Leu Glu Ser Gln Lys Leu Ser Asn Asn
595 600 605

Val Glu Asn Ile Thr Leu Thr Thr Ser Val Glu Glu Ala Leu Gly Tyr
610 615 620

Ser Asn Lys Ile Tyr Thr Phe Leu Pro Ser Leu Ala Glu Lys Val Asn
625 630 635 640

Lys Gly Val Gln Ala Gly Leu Phe Leu Asn Trp Ala Asn Glu Val Val
645 650 655

Glu Asp Phe Thr Thr Asn Ile Met Lys Lys Asp Thr Leu Asp Lys Ile
660 665 670

Ser Asp Val Ser Val Ile Ile Pro Tyr Ile Gly Pro Ala Leu Asn Ile
675 680 685

Gly Asn Ser Ala Leu Arg Gly Asn Phe Asn Gln Ala Phe Ala Thr Ala
690 695 700

Gly Val Ala Phe Leu Leu Glu Gly Phe Pro Glu Phe Thr Ile Pro Ala
705 710 715 720

Leu Gly Val Phe Thr Phe Tyr Ser Ser Ile Gln Glu Arg Glu Lys Ile
725 730 735

Ile Lys Thr Ile Glu Asn Cys Leu Glu Gln Arg Val Lys Arg Trp Lys
740 745 750

Asp Ser Tyr Gln Trp Met Val Ser Asn Trp Leu Ser Arg Ile Thr Thr
755 760 765

Gln Phe Asn His Ile Asn Tyr Gln Met Tyr Asp Ser Leu Ser Tyr Gln
770 775 780

Ala Asp Ala Ile Lys Ala Lys Ile Asp Leu Glu Tyr Lys Lys Tyr Ser
785 790 795 800

Gly Ser Asp Lys Glu Asn Ile Lys Ser Gln Val Glu Asn Leu Lys Asn
                805                 810                 815

Ser Leu Asp Val Lys Ile Ser Glu Ala Met Asn Asn Ile Asn Lys Phe
            820                 825                 830

Ile Arg Glu Cys Ser Val Thr Tyr Leu Phe Lys Asn Met Leu Pro Lys
        835                 840                 845

Val Ile Asp Glu Leu Asn Lys Phe Asp Leu Arg Thr Lys Thr Glu Leu
    850                 855                 860

Ile Asn Leu Ile Asp Ser His Asn Ile Ile Leu Val Gly Glu Val Asp
865                 870                 875                 880

Arg Leu Lys Ala Lys Val Asn Glu Ser Phe Glu Asn Thr Met Pro Phe
                885                 890                 895

Asn Ile Phe Ser Tyr Thr Asn Asn Ser Leu Leu Lys Asp Ile Ile Asn
            900                 905                 910

Glu Tyr Phe Asn
        915

<210> 91
<211> 916
<212> PRT
<213> Artificial Sequence

<220>
<221> source
<223> /note="Description of Artificial Sequence: Synthetic
      polypeptide"

<400> 91
Thr Trp Pro Val Lys Asp Phe Asn Tyr Ser Asp Pro Val Asn Asp Asn
1               5                   10                  15

Asp Ile Leu Tyr Leu Arg Ile Pro Gln Asn Lys Leu Ile Thr Thr Pro
            20                  25                  30

Val Lys Ala Phe Met Ile Thr Gln Asn Ile Trp Val Ile Pro Glu Arg
        35                  40                  45

Phe Ser Ser Asp Thr Asn Pro Ser Leu Ser Lys Pro Pro Arg Pro Thr
    50                  55                  60

Ser Lys Tyr Gln Ser Tyr Tyr Asp Pro Ser Tyr Leu Ser Thr Asp Glu
65                  70                  75                  80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser
130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr

325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Gly Ile Ile Thr Ser Lys Thr Lys Ser Ile
435 440 445

Glu Gly Arg Tyr Ala Asp Ala Ile Phe Thr Ala Ser Tyr Arg Asn Val
450 455 460

Leu Gly Gln Leu Ser Ala Arg Lys Leu Leu Gln Asp Ile Leu Ser Arg
465 470 475 480

Ala Leu Ala Gly Gly Gly Gly Ser Gly Gly Gly Gly Ser Gly Gly Gly
485 490 495

Gly Ser Ala Leu Ala Leu Gln Cys Ile Lys Val Lys Asn Asn Arg Leu
500 505 510

Pro Tyr Val Ala Asp Lys Asp Ser Ile Ser Gln Glu Ile Phe Glu Asn
515 520 525

Lys Ile Ile Thr Asp Glu Thr Asn Val Gln Asn Tyr Ser Asp Lys Phe
530 535 540

Ser Leu Asp Glu Ser Ile Leu Asp Gly Gln Val Pro Ile Asn Pro Glu
545 550 555 560

Ile Val Asp Pro Leu Leu Pro Asn Val Asn Met Glu Pro Leu Asn Leu
565 570 575

Pro Gly Glu Glu Ile Val Phe Tyr Asp Asp Ile Thr Lys Tyr Val Asp
580 585 590

Tyr Leu Asn Ser Tyr Tyr Tyr Leu Glu Ser Gln Lys Leu Ser Asn Asn
595 600 605

Val Glu Asn Ile Thr Leu Thr Thr Ser Val Glu Glu Ala Leu Gly Tyr
610 615 620

Ser Asn Lys Ile Tyr Thr Phe Leu Pro Ser Leu Ala Glu Lys Val Asn
625 630 635 640

Lys Gly Val Gln Ala Gly Leu Phe Leu Asn Trp Ala Asn Glu Val Val
645 650 655

Glu Asp Phe Thr Thr Asn Ile Met Lys Lys Asp Thr Leu Asp Lys Ile
660 665 670

Ser Asp Val Ser Val Ile Ile Pro Tyr Ile Gly Pro Ala Leu Asn Ile
675 680 685

Gly Asn Ser Ala Leu Arg Gly Asn Phe Asn Gln Ala Phe Ala Thr Ala
690 695 700

Gly Val Ala Phe Leu Leu Glu Gly Phe Pro Glu Phe Thr Ile Pro Ala
705 710 715 720

Leu Gly Val Phe Thr Phe Tyr Ser Ser Ile Gln Glu Arg Glu Lys Ile
725 730 735

Ile Lys Thr Ile Glu Asn Cys Leu Glu Gln Arg Val Lys Arg Trp Lys
740 745 750

Asp Ser Tyr Gln Trp Met Val Ser Asn Trp Leu Ser Arg Ile Thr Thr
755 760 765

Gln Phe Asn His Ile Asn Tyr Gln Met Tyr Asp Ser Leu Ser Tyr Gln
770 775 780

Ala Asp Ala Ile Lys Ala Lys Ile Asp Leu Glu Tyr Lys Lys Tyr Ser
785 790 795 800

Gly Ser Asp Lys Glu Asn Ile Lys Ser Gln Val Glu Asn Leu Lys Asn
805 810 815

Ser Leu Asp Val Lys Ile Ser Glu Ala Met Asn Asn Ile Asn Lys Phe
820 825 830

Ile Arg Glu Cys Ser Val Thr Tyr Leu Phe Lys Asn Met Leu Pro Lys
835 840 845

Val Ile Asp Glu Leu Asn Lys Phe Asp Leu Arg Thr Lys Thr Glu Leu
850 855 860

Ile Asn Leu Ile Asp Ser His Asn Ile Ile Leu Val Gly Glu Val Asp
865 870 875 880

Arg Leu Lys Ala Lys Val Asn Glu Ser Phe Glu Asn Thr Met Pro Phe
885 890 895

Asn Ile Phe Ser Tyr Thr Asn Asn Ser Leu Leu Lys Asp Ile Ile Asn
900 905 910

Glu Tyr Phe Asn
915

<210> 92
<211> 910
<212> PRT
<213> Artificial Sequence

<220>
<221> source
<223> /note="Description of Artificial Sequence: Synthetic polypeptide"

<400> 92
His Val Asp Ala Ile Phe Thr Gln Ser Tyr Arg Lys Val Leu Ala Gln
1 5 10 15

Leu Ser Ala Arg Lys Leu Leu Gln Asp Ile Leu Asn Arg Asn Asn Asn
20 25 30

Asn Asn Asn Asn Asn Asn Asn Thr Trp Pro Val Lys Asp Phe Asn Tyr
35 40 45

Ser Asp Pro Val Asn Asp Asn Asp Ile Leu Tyr Leu Arg Ile Pro Gln
50 55 60

Asn Lys Leu Ile Thr Thr Pro Val Lys Ala Phe Met Ile Thr Gln Asn
65 70 75 80

Ile Trp Val Ile Pro Glu Arg Phe Ser Ser Asp Thr Asn Pro Ser Leu
85 90 95

Ser Lys Pro Pro Arg Pro Thr Ser Lys Tyr Gln Ser Tyr Tyr Asp Pro
100 105 110

Ser Tyr Leu Ser Thr Asp Glu Gln Lys Asp Thr Phe Leu Lys Gly Ile
115 120 125

Ile Lys Leu Phe Lys Arg Ile Asn Glu Arg Asp Ile Gly Lys Lys Leu
130 135 140

Ile Asn Tyr Leu Val Val Gly Ser Pro Phe Met Gly Asp Ser Ser Thr
145 150 155 160

Pro Glu Asp Thr Phe Asp Phe Thr Arg His Thr Thr Asn Ile Ala Val
165 170 175

Glu Lys Phe Glu Asn Gly Ser Trp Lys Val Thr Asn Ile Ile Thr Pro
180 185 190

Ser Val Leu Ile Phe Gly Pro Leu Pro Asn Ile Leu Asp Tyr Thr Ala
195 200 205

Ser Leu Thr Leu Gln Gly Gln Gln Ser Asn Pro Ser Phe Glu Gly Phe
210 215 220

Gly Thr Leu Ser Ile Leu Lys Val Ala Pro Glu Phe Leu Leu Thr Phe
225 230 235 240

Ser Asp Val Thr Ser Asn Gln Ser Ser Ala Val Leu Gly Lys Ser Ile
245 250 255

Phe Cys Met Asp Pro Val Ile Ala Leu Met His Glu Leu Thr His Ser
260 265 270

Leu His Gln Leu Tyr Gly Ile Asn Ile Pro Ser Asp Lys Arg Ile Arg
275 280 285

Pro Gln Val Ser Glu Gly Phe Phe Ser Gln Asp Gly Pro Asn Val Gln
290 295 300

Phe Glu Glu Leu Tyr Thr Phe Gly Gly Leu Asp Val Glu Ile Ile Pro
305 310 315 320

Gln Ile Glu Arg Ser Gln Leu Arg Glu Lys Ala Leu Gly His Tyr Lys
325 330 335

Asp Ile Ala Lys Arg Leu Asn Asn Ile Asn Lys Thr Ile Pro Ser Ser
340 345 350

Trp Ile Ser Asn Ile Asp Lys Tyr Lys Lys Ile Phe Ser Glu Lys Tyr
355 360 365

```
Asn Phe Asp Lys Asp Asn Thr Gly Asn Phe Val Val Asn Ile Asp Lys
    370                 375                 380

Phe Asn Ser Leu Tyr Ser Asp Leu Thr Asn Val Met Ser Glu Val Val
385                 390                 395                 400

Tyr Ser Ser Gln Tyr Asn Val Lys Asn Arg Thr His Tyr Phe Ser Arg
                405                 410                 415

His Tyr Leu Pro Val Phe Ala Asn Ile Leu Asp Asp Asn Ile Tyr Thr
            420                 425                 430

Ile Arg Asp Gly Phe Asn Leu Thr Asn Lys Gly Phe Asn Ile Glu Asn
        435                 440                 445

Ser Gly Gln Asn Ile Glu Arg Asn Pro Ala Leu Gln Lys Leu Ser Ser
    450                 455                 460

Glu Ser Val Val Asp Leu Phe Thr Lys Val Cys Val Asp Lys Ser Glu
465                 470                 475                 480

Glu Lys Leu Tyr Asp Asp Asp Asp Lys Asp Arg Trp Gly Ser Ser Leu
                485                 490                 495

Gln Cys Ile Lys Val Lys Asn Asn Arg Leu Pro Tyr Val Ala Asp Lys
            500                 505                 510

Asp Ser Ile Ser Gln Glu Ile Phe Glu Asn Lys Ile Ile Thr Asp Glu
        515                 520                 525

Thr Asn Val Gln Asn Tyr Ser Asp Lys Phe Ser Leu Asp Glu Ser Ile
    530                 535                 540

Leu Asp Gly Gln Val Pro Ile Asn Pro Glu Ile Val Asp Pro Leu Leu
545                 550                 555                 560

Pro Asn Val Asn Met Glu Pro Leu Asn Leu Pro Gly Glu Glu Ile Val
                565                 570                 575

Phe Tyr Asp Asp Ile Thr Lys Tyr Val Asp Tyr Leu Asn Ser Tyr Tyr
            580                 585                 590

Tyr Leu Glu Ser Gln Lys Leu Ser Asn Asn Val Glu Asn Ile Thr Leu
        595                 600                 605

Thr Thr Ser Val Glu Glu Ala Leu Gly Tyr Ser Asn Lys Ile Tyr Thr
```

610 615 620

Phe Leu Pro Ser Leu Ala Glu Lys Val Asn Lys Gly Val Gln Ala Gly
625 630 635 640

Leu Phe Leu Asn Trp Ala Asn Glu Val Val Glu Asp Phe Thr Thr Asn
645 650 655

Ile Met Lys Lys Asp Thr Leu Asp Lys Ile Ser Asp Val Ser Val Ile
660 665 670

Ile Pro Tyr Ile Gly Pro Ala Leu Asn Ile Gly Asn Ser Ala Leu Arg
675 680 685

Gly Asn Phe Asn Gln Ala Phe Ala Thr Ala Gly Val Ala Phe Leu Leu
690 695 700

Glu Gly Phe Pro Glu Phe Thr Ile Pro Ala Leu Gly Val Phe Thr Phe
705 710 715 720

Tyr Ser Ser Ile Gln Glu Arg Glu Lys Ile Ile Lys Thr Ile Glu Asn
725 730 735

Cys Leu Glu Gln Arg Val Lys Arg Trp Lys Asp Ser Tyr Gln Trp Met
740 745 750

Val Ser Asn Trp Leu Ser Arg Ile Thr Thr Gln Phe Asn His Ile Asn
755 760 765

Tyr Gln Met Tyr Asp Ser Leu Ser Tyr Gln Ala Asp Ala Ile Lys Ala
770 775 780

Lys Ile Asp Leu Glu Tyr Lys Lys Tyr Ser Gly Ser Asp Lys Glu Asn
785 790 795 800

Ile Lys Ser Gln Val Glu Asn Leu Lys Asn Ser Leu Asp Val Lys Ile
805 810 815

Ser Glu Ala Met Asn Asn Ile Asn Lys Phe Ile Arg Glu Cys Ser Val
820 825 830

Thr Tyr Leu Phe Lys Asn Met Leu Pro Lys Val Ile Asp Glu Leu Asn
835 840 845

Lys Phe Asp Leu Arg Thr Lys Thr Glu Leu Ile Asn Leu Ile Asp Ser
850 855 860

His Asn Ile Ile Leu Val Gly Glu Val Asp Arg Leu Lys Ala Lys Val
865 870 875 880

Asn Glu Ser Phe Glu Asn Thr Met Pro Phe Asn Ile Phe Ser Tyr Thr
885 890 895

Asn Asn Ser Leu Leu Lys Asp Ile Ile Asn Glu Tyr Phe Asn
900 905 910

<210> 93
<211> 977
<212> PRT
<213> Artificial Sequence

<220>
<221> source
<223> /note="Description of Artificial Sequence: Synthetic polypeptide"

<400> 93
Pro Ile Thr Ile Asn Asn Phe Asn Tyr Ser Asp Pro Val Asp Asn Lys
1 5 10 15

Asn Ile Leu Tyr Leu Asp Thr His Leu Asn Thr Leu Ala Asn Glu Pro
20 25 30

Glu Lys Ala Phe Arg Ile Thr Gly Asn Ile Trp Val Ile Pro Asp Arg
35 40 45

Phe Ser Arg Asn Ser Asn Pro Asn Leu Asn Lys Pro Pro Arg Val Thr
50 55 60

Ser Pro Lys Ser Gly Tyr Tyr Asp Pro Asn Tyr Leu Ser Thr Asp Ser
65 70 75 80

Asp Lys Asp Thr Phe Leu Lys Glu Ile Ile Lys Leu Phe Lys Arg Ile
85 90 95

Asn Ser Arg Glu Ile Gly Glu Glu Leu Ile Tyr Arg Leu Ser Thr Asp
100 105 110

Ile Pro Phe Pro Gly Asn Asn Asn Thr Pro Ile Asn Thr Phe Asp Phe
115 120 125

Asp Val Asp Phe Asn Ser Val Asp Val Lys Thr Arg Gln Gly Asn Asn
130 135 140

Trp Val Lys Thr Gly Ser Ile Asn Pro Ser Val Ile Ile Thr Gly Pro
145 150 155 160

```
Arg Glu Asn Ile Ile Asp Pro Glu Thr Ser Thr Phe Lys Leu Thr Asn
                165                 170                 175

Asn Thr Phe Ala Ala Gln Glu Gly Phe Gly Ala Leu Ser Ile Ile Ser
            180                 185                 190

Ile Ser Pro Arg Phe Met Leu Thr Tyr Ser Asn Ala Thr Asn Asp Val
        195                 200                 205

Gly Glu Gly Arg Phe Ser Lys Ser Glu Phe Cys Met Asp Pro Ile Leu
    210                 215                 220

Ile Leu Met His Glu Leu Asn His Ala Met His Asn Leu Tyr Gly Ile
225                 230                 235                 240

Ala Ile Pro Asn Asp Gln Thr Ile Ser Ser Val Thr Ser Asn Ile Phe
                245                 250                 255

Tyr Ser Gln Tyr Asn Val Lys Leu Glu Tyr Ala Glu Ile Tyr Ala Phe
            260                 265                 270

Gly Gly Pro Thr Ile Asp Leu Ile Pro Lys Ser Ala Arg Lys Tyr Phe
        275                 280                 285

Glu Glu Lys Ala Leu Asp Tyr Tyr Arg Ser Ile Ala Lys Arg Leu Asn
    290                 295                 300

Ser Ile Thr Thr Ala Asn Pro Ser Ser Phe Asn Lys Tyr Ile Gly Glu
305                 310                 315                 320

Tyr Lys Gln Lys Leu Ile Arg Lys Tyr Arg Phe Val Val Glu Ser Ser
                325                 330                 335

Gly Glu Val Thr Val Asn Arg Asn Lys Phe Val Glu Leu Tyr Asn Glu
            340                 345                 350

Leu Thr Gln Ile Phe Thr Glu Phe Asn Tyr Ala Lys Ile Tyr Asn Val
        355                 360                 365

Gln Asn Arg Lys Ile Tyr Leu Ser Asn Val Tyr Thr Pro Val Thr Ala
    370                 375                 380

Asn Ile Leu Asp Asp Asn Val Tyr Asp Ile Gln Asn Gly Phe Asn Ile
385                 390                 395                 400

Pro Lys Ser Asn Leu Asn Val Leu Phe Met Gly Gln Asn Leu Ser Arg
                405                 410                 415
```

Asn Pro Ala Leu Arg Lys Val Asn Pro Glu Asn Met Leu Tyr Leu Phe
420 425 430

Thr Lys Phe Cys Val Asp Ala Ile Asp Gly Arg Ser Leu Tyr Asn Lys
435 440 445

Thr Leu Gln Cys Arg Glu Leu Leu Val Lys Asn Thr Asp Leu Pro Phe
450 455 460

Ile Gly Asp Ile Ser Asp Val Lys Thr Asp Ile Phe Leu Arg Lys Asp
465 470 475 480

Ile Asn Glu Glu Thr Glu Val Ile Tyr Tyr Pro Asp Asn Val Ser Val
485 490 495

Asp Gln Val Ile Leu Ser Lys Asn Thr Ser Glu His Gly Gln Leu Asp
500 505 510

Leu Leu Tyr Pro Ser Ile Asp Ser Glu Ser Glu Ile Leu Pro Gly Glu
515 520 525

Asn Gln Val Phe Tyr Asp Asn Arg Thr Gln Asn Val Asp Tyr Leu Asn
530 535 540

Ser Tyr Tyr Tyr Leu Glu Ser Gln Lys Leu Ser Asp Asn Val Glu Asp
545 550 555 560

Phe Thr Phe Thr Arg Ser Ile Glu Glu Ala Leu Asp Asn Ser Ala Lys
565 570 575

Val Tyr Thr Tyr Phe Pro Thr Leu Ala Asn Lys Val Asn Ala Gly Val
580 585 590

Gln Gly Gly Leu Phe Leu Met Trp Ala Asn Asp Val Val Glu Asp Phe
595 600 605

Thr Thr Asn Ile Leu Arg Lys Asp Thr Leu Asp Lys Ile Ser Asp Val
610 615 620

Ser Ala Ile Ile Pro Tyr Ile Gly Pro Ala Leu Asn Ile Ser Asn Ser
625 630 635 640

Val Arg Arg Gly Asn Phe Thr Glu Ala Phe Ala Val Thr Gly Val Thr
645 650 655

Ile Leu Leu Glu Ala Phe Pro Glu Phe Thr Ile Pro Ala Leu Gly Ala
660 665 670

Phe Val Ile Tyr Ser Lys Val Gln Glu Arg Asn Glu Ile Ile Lys Thr
675 680 685

Ile Asp Asn Cys Leu Glu Gln Arg Ile Lys Arg Trp Lys Asp Ser Tyr
690 695 700

Glu Trp Met Met Gly Thr Trp Leu Ser Arg Ile Ile Thr Gln Phe Asn
705 710 715 720

Asn Ile Ser Tyr Gln Met Tyr Asp Ser Leu Asn Tyr Gln Ala Gly Ala
725 730 735

Ile Lys Ala Lys Ile Asp Leu Glu Tyr Lys Lys Tyr Ser Gly Ser Asp
740 745 750

Lys Glu Asn Ile Lys Ser Gln Val Glu Asn Leu Lys Asn Ser Leu Asp
755 760 765

Val Lys Ile Ser Glu Ala Met Asn Asn Ile Asn Lys Phe Ile Arg Glu
770 775 780

Cys Ser Val Thr Tyr Leu Phe Lys Asn Met Leu Pro Lys Val Ile Asp
785 790 795 800

Glu Leu Asn Glu Phe Asp Arg Asn Thr Lys Ala Lys Leu Ile Asn Leu
805 810 815

Ile Asp Ser His Asn Ile Ile Leu Val Gly Glu Val Asp Lys Leu Lys
820 825 830

Ala Lys Val Asn Asn Ser Phe Gln Asn Thr Ile Pro Phe Asn Ile Phe
835 840 845

Ser Tyr Thr Asn Asn Ser Leu Leu Lys Asp Ile Ile Asn Glu Tyr Phe
850 855 860

Asn Leu Glu Gly Gly Gly Gly Ser Gly Gly Gly Gly Ser Gly Gly Gly
865 870 875 880

Gly Ser Ala Leu Val Met Lys Pro Ile Gln Lys Leu Leu Ala Gly Leu
885 890 895

Ile Leu Leu Thr Trp Cys Val Glu Gly Cys Ser Ser Gln His Trp Ser
900 905 910

Tyr Gly Leu Arg Pro Gly Gly Lys Arg Asp Ala Glu Asn Leu Ile Asp

915 920 925

Ser Phe Gln Glu Ile Val Lys Glu Val Gly Gln Leu Ala Glu Thr Gln
930 935 940

Arg Phe Glu Cys Thr Thr His Gln Pro Arg Ser Pro Leu Arg Asp Leu
945 950 955 960

Lys Gly Ala Leu Glu Ser Leu Ile Glu Glu Glu Thr Gly Gln Lys Lys
965 970 975

Ile

<210> 94
<211> 988
<212> PRT
<213> Artificial Sequence

<220>
<221> source
<223> /note="Description of Artificial Sequence: Synthetic polypeptide"

<400> 94
Thr Trp Pro Val Lys Asp Phe Asn Tyr Ser Asp Pro Val Asn Asp Asn
1 5 10 15

Asp Ile Leu Tyr Leu Arg Ile Pro Gln Asn Lys Leu Ile Thr Thr Pro
20 25 30

Val Lys Ala Phe Met Ile Thr Gln Asn Ile Trp Val Ile Pro Glu Arg
35 40 45

Phe Ser Ser Asp Thr Asn Pro Ser Leu Ser Lys Pro Pro Arg Pro Thr
50 55 60

Ser Lys Tyr Gln Ser Tyr Tyr Asp Pro Ser Tyr Leu Ser Thr Asp Glu
65 70 75 80

Gln Lys Asp Thr Phe Leu Lys Gly Ile Ile Lys Leu Phe Lys Arg Ile
85 90 95

Asn Glu Arg Asp Ile Gly Lys Lys Leu Ile Asn Tyr Leu Val Val Gly
100 105 110

Ser Pro Phe Met Gly Asp Ser Ser Thr Pro Glu Asp Thr Phe Asp Phe
115 120 125

Thr Arg His Thr Thr Asn Ile Ala Val Glu Lys Phe Glu Asn Gly Ser

130 135 140

Trp Lys Val Thr Asn Ile Ile Thr Pro Ser Val Leu Ile Phe Gly Pro
145 150 155 160

Leu Pro Asn Ile Leu Asp Tyr Thr Ala Ser Leu Thr Leu Gln Gly Gln
165 170 175

Gln Ser Asn Pro Ser Phe Glu Gly Phe Gly Thr Leu Ser Ile Leu Lys
180 185 190

Val Ala Pro Glu Phe Leu Leu Thr Phe Ser Asp Val Thr Ser Asn Gln
195 200 205

Ser Ser Ala Val Leu Gly Lys Ser Ile Phe Cys Met Asp Pro Val Ile
210 215 220

Ala Leu Met His Glu Leu Thr His Ser Leu His Gln Leu Tyr Gly Ile
225 230 235 240

Asn Ile Pro Ser Asp Lys Arg Ile Arg Pro Gln Val Ser Glu Gly Phe
245 250 255

Phe Ser Gln Asp Gly Pro Asn Val Gln Phe Glu Glu Leu Tyr Thr Phe
260 265 270

Gly Gly Leu Asp Val Glu Ile Ile Pro Gln Ile Glu Arg Ser Gln Leu
275 280 285

Arg Glu Lys Ala Leu Gly His Tyr Lys Asp Ile Ala Lys Arg Leu Asn
290 295 300

Asn Ile Asn Lys Thr Ile Pro Ser Ser Trp Ile Ser Asn Ile Asp Lys
305 310 315 320

Tyr Lys Lys Ile Phe Ser Glu Lys Tyr Asn Phe Asp Lys Asp Asn Thr
325 330 335

Gly Asn Phe Val Val Asn Ile Asp Lys Phe Asn Ser Leu Tyr Ser Asp
340 345 350

Leu Thr Asn Val Met Ser Glu Val Val Tyr Ser Ser Gln Tyr Asn Val
355 360 365

Lys Asn Arg Thr His Tyr Phe Ser Arg His Tyr Leu Pro Val Phe Ala
370 375 380

Asn Ile Leu Asp Asp Asn Ile Tyr Thr Ile Arg Asp Gly Phe Asn Leu
385 390 395 400

Thr Asn Lys Gly Phe Asn Ile Glu Asn Ser Gly Gln Asn Ile Glu Arg
405 410 415

Asn Pro Ala Leu Gln Lys Leu Ser Ser Glu Ser Val Val Asp Leu Phe
420 425 430

Thr Lys Val Cys Val Asp Lys Ser Glu Glu Lys Leu Tyr Asp Asp Asp
435 440 445

Asp Lys Asp Arg Trp Gly Ser Ser Leu Gln Cys Ile Lys Val Lys Asn
450 455 460

Asn Arg Leu Pro Tyr Val Ala Asp Lys Asp Ser Ile Ser Gln Glu Ile
465 470 475 480

Phe Glu Asn Lys Ile Ile Thr Asp Glu Thr Asn Val Gln Asn Tyr Ser
485 490 495

Asp Lys Phe Ser Leu Asp Glu Ser Ile Leu Asp Gly Gln Val Pro Ile
500 505 510

Asn Pro Glu Ile Val Asp Pro Leu Leu Pro Asn Val Asn Met Glu Pro
515 520 525

Leu Asn Leu Pro Gly Glu Glu Ile Val Phe Tyr Asp Asp Ile Thr Lys
530 535 540

Tyr Val Asp Tyr Leu Asn Ser Tyr Tyr Tyr Leu Glu Ser Gln Lys Leu
545 550 555 560

Ser Asn Asn Val Glu Asn Ile Thr Leu Thr Thr Ser Val Glu Glu Ala
565 570 575

Leu Gly Tyr Ser Asn Lys Ile Tyr Thr Phe Leu Pro Ser Leu Ala Glu
580 585 590

Lys Val Asn Lys Gly Val Gln Ala Gly Leu Phe Leu Asn Trp Ala Asn
595 600 605

Glu Val Val Glu Asp Phe Thr Thr Asn Ile Met Lys Lys Asp Thr Leu
610 615 620

Asp Lys Ile Ser Asp Val Ser Val Ile Ile Pro Tyr Ile Gly Pro Ala
625 630 635 640

Leu Asn Ile Gly Asn Ser Ala Leu Arg Gly Asn Phe Asn Gln Ala Phe
645 650 655

Ala Thr Ala Gly Val Ala Phe Leu Leu Glu Gly Phe Pro Glu Phe Thr
660 665 670

Ile Pro Ala Leu Gly Val Phe Thr Phe Tyr Ser Ser Ile Gln Glu Arg
675 680 685

Glu Lys Ile Ile Lys Thr Ile Glu Asn Cys Leu Glu Gln Arg Val Lys
690 695 700

Arg Trp Lys Asp Ser Tyr Gln Trp Met Val Ser Asn Trp Leu Ser Arg
705 710 715 720

Ile Thr Thr Gln Phe Asn His Ile Asn Tyr Gln Met Tyr Asp Ser Leu
725 730 735

Ser Tyr Gln Ala Asp Ala Ile Lys Ala Lys Ile Asp Leu Glu Tyr Lys
740 745 750

Lys Tyr Ser Gly Ser Asp Lys Glu Asn Ile Lys Ser Gln Val Glu Asn
755 760 765

Leu Lys Asn Ser Leu Asp Val Lys Ile Ser Glu Ala Met Asn Asn Ile
770 775 780

Asn Lys Phe Ile Arg Glu Cys Ser Val Thr Tyr Leu Phe Lys Asn Met
785 790 795 800

Leu Pro Lys Val Ile Asp Glu Leu Asn Lys Phe Asp Leu Arg Thr Lys
805 810 815

Thr Glu Leu Ile Asn Leu Ile Asp Ser His Asn Ile Ile Leu Val Gly
820 825 830

Glu Val Asp Arg Leu Lys Ala Lys Val Asn Glu Ser Phe Glu Asn Thr
835 840 845

Met Pro Phe Asn Ile Phe Ser Tyr Thr Asn Asn Ser Leu Leu Lys Asp
850 855 860

Ile Ile Asn Glu Tyr Phe Asn Leu Glu Gly Gly Gly Gly Ser Gly Gly
865 870 875 880

Gly Gly Ser Gly Gly Gly Gly Ser Gly Gly Gly Gly Ser Ala Leu Val
885 890 895

Met Lys Pro Ile Gln Lys Leu Leu Ala Gly Leu Ile Leu Leu Thr Trp
900 905 910

Cys Val Glu Gly Cys Ser Ser Gln His Trp Ser Tyr Gly Leu Arg Pro
915 920 925

Gly Gly Lys Arg Asp Ala Glu Asn Leu Ile Asp Ser Phe Gln Glu Ile
930 935 940

Val Lys Glu Val Gly Gln Leu Ala Glu Thr Gln Arg Phe Glu Cys Thr
945 950 955 960

Thr His Gln Pro Arg Ser Pro Leu Arg Asp Leu Lys Gly Ala Leu Glu
965 970 975

Ser Leu Ile Glu Glu Glu Thr Gly Gln Lys Lys Ile
980 985

<210> 95
<211> 28
<212> PRT
<213> Homo sapiens

<400> 95
Ser Ala Asn Ser Asn Pro Ala Met Ala Pro Arg Glu Arg Lys Ala Gly
1 5 10 15

Cys Lys Asn Phe Phe Trp Lys Thr Phe Thr Ser Cys
20 25

<210> 96
<211> 14
<212> PRT
<213> Homo sapiens

<400> 96
Ala Gly Cys Lys Asn Phe Phe Trp Lys Thr Phe Thr Ser Cys
1 5 10

<210> 97
<211> 29
<212> PRT
<213> Homo sapiens

<400> 97
Gln Glu Gly Ala Pro Pro Gln Gln Ser Ala Arg Arg Asp Arg Met Pro
1 5 10 15

Cys Arg Asn Phe Phe Trp Lys Thr Phe Ser Ser Cys Lys
20 25

<210> 98
<211> 29
<212> PRT
<213> Homo sapiens

<400> 98
Gln Glu Arg Pro Pro Leu Gln Gln Pro Pro His Arg Asp Lys Lys Pro
1 5 10 15

Cys Lys Asn Phe Phe Trp Lys Thr Phe Ser Ser Cys Lys
20 25

<210> 99
<211> 29
<212> PRT
<213> Homo sapiens

<400> 99
Gln Glu Arg Pro Pro Pro Gln Gln Pro Pro His Leu Asp Lys Lys Pro
1 5 10 15

Cys Lys Asn Phe Phe Trp Lys Thr Phe Ser Ser Cys Lys
20 25

<210> 100
<211> 17
<212> PRT
<213> Homo sapiens

<400> 100
Asp Arg Met Pro Cys Arg Asn Phe Phe Trp Lys Thr Phe Ser Ser Cys
1 5 10 15

Lys

<210> 101
<211> 14
<212> PRT
<213> Homo sapiens

<400> 101
Pro Cys Arg Asn Phe Phe Trp Lys Thr Phe Ser Ser Cys Lys
1 5 10

<210> 102
<211> 14
<212> PRT
<213> Homo sapiens

<400> 102
Pro Cys Lys Asn Phe Phe Trp Lys Thr Phe Ser Ser Cys Lys
1 5 10

<210> 103
<211> 44
<212> PRT
<213> Artificial Sequence

<220>
<221> source
<223> /note="Description of Artificial Sequence: Synthetic polypeptide"

<220>
<221> MOD_RES
<222> (1)..(1)
<223> H-Tyr

<220>
<221> misc_feature
<222> (44)..(44)
<223> /note="C-terminus amidated"

<400> 103
```
Tyr Ala Asp Ala Ile Phe Thr Asn Ser Tyr Arg Lys Val Leu Gly Gln
1               5                   10                  15

Leu Ser Ala Arg Lys Leu Leu Gln Asp Ile Met Ser Arg Gln Gln Gly
            20                  25                  30

Glu Ser Asn Gln Glu Arg Gly Ala Arg Ala Arg Leu
        35                  40
```

<210> 104
<211> 29
<212> PRT
<213> Artificial Sequence

<220>
<221> source
<223> /note="Description of Artificial Sequence: Synthetic peptide"

<220>
<221> MOD_RES
<222> (1)..(1)
<223> H-Tyr

<220>
<221> misc_feature
<222> (29)..(29)
<223> /note="C-terminus amidated"

<400> 104
```
Tyr Ala Asp Ala Ile Phe Thr Asn Ser Tyr Arg Lys Val Leu Gly Gln
1               5                   10                  15

Leu Ser Ala Arg Lys Leu Leu Gln Asp Ile Met Ser Arg
            20                  25
```

<210> 105
<211> 44
<212> PRT
<213> Artificial Sequence

<220>
<221> source
<223> /note="Description of Artificial Sequence: Synthetic polypeptide"

<220>
<221> MOD_RES
<222> (1)..(1)
<223> H-Tyr

<220>
<221> misc_feature
<222> (44)..(44)
<223> /note="C-terminus amidated"

<400> 105
Tyr Ala Asp Ala Ile Phe Thr Asn Ser Tyr Arg Lys Ile Leu Gly Gln
1               5                   10                  15

Leu Ser Ala Arg Lys Leu Leu Gln Asp Ile Met Asn Arg Gln Gln Gly
            20                  25                  30

Glu Arg Asn Gln Glu Gln Gly Ala Lys Val Arg Leu
        35                  40

<210> 106
<211> 44
<212> PRT
<213> Artificial Sequence

<220>
<221> source
<223> /note="Description of Artificial Sequence: Synthetic polypeptide"

<220>
<221> MOD_RES
<222> (1)..(1)
<223> H-Tyr

<220>
<221> misc_feature
<222> (44)..(44)
<223> /note="C-terminus amidated"

<400> 106
Tyr Ala Asp Ala Ile Phe Thr Asn Ser Tyr Arg Lys Val Leu Gly Gln
1               5                   10                  15

Leu Ser Ala Arg Lys Leu Leu Gln Asp Ile Met Asn Arg Gln Gln Gly
            20                  25                  30

Glu Arg Asn Gln Glu Gln Gly Ala Lys Val Arg Leu
        35                  40

<210> 107
<211> 29
<212> PRT
<213> Artificial Sequence

<220>
<221> source
<223> /note="Description of Artificial Sequence: Synthetic polypeptide"

<400> 107
His Val Asp Ala Ile Phe Thr Gln Ser Tyr Arg Lys Val Leu Ala Gln
1               5                   10                  15

Leu Ser Ala Arg Lys Leu Leu Gln Asp Ile Leu Asn Arg
            20                  25

<210> 108
<211> 40
<212> PRT
<213> Artificial Sequence

<220>
<221> source
<223> /note="Description of Artificial Sequence: Synthetic polypeptide"

<400> 108
His Val Asp Ala Ile Phe Thr Gln Ser Tyr Arg Lys Val Leu Ala Gln
1               5                   10                  15

Leu Ser Ala Arg Lys Leu Leu Gln Asp Ile Leu Asn Arg Gln Gln Gly
            20                  25                  30

Glu Arg Asn Gln Glu Gln Gly Ala
        35                  40

<210> 109
<211> 14
<212> PRT
<213> Unknown

<220>
<221> source
<223> /note="Description of Unknown: Bombesin peptide"

<220>
<221> MOD_RES
<222> (1)..(1)
<223> pGlu

```
<220>
<221> misc_feature
<222> (14)..(14)
<223> /note="C-terminus amidated"

<400> 109
Glu Gln Arg Leu Gly Asn Gln Trp Ala Val Gly His Leu Met
1               5                   10


<210> 110
<211> 27
<212> PRT
<213> Sus sp.


<220>
<221> misc_feature
<222> (27)..(27)
<223> /note="C-terminus amidated"

<400> 110
Ala Pro Val Ser Val Gly Gly Gly Thr Val Leu Ala Lys Met Tyr Pro
1               5                   10                  15


Arg Gly Asn His Trp Ala Val Gly His Leu Met
            20                  25


<210> 111
<211> 27
<212> PRT
<213> Homo sapiens


<220>
<221> MOD_RES
<222> (27)..(27)
<223> /note="C-terminus amidated"

<400> 111
Val Pro Leu Pro Ala Gly Gly Gly Thr Val Leu Thr Lys Met Tyr Pro
1               5                   10                  15


Arg Gly Asn His Trp Ala Val Gly His Leu Met
            20                  25


<210> 112
<211> 6
<212> PRT
<213> Homo sapiens

<400> 112
Cys Phe Trp Lys Tyr Cys
1               5


<210> 113
<211> 4
<212> PRT
```

<213> Homo sapiens

<400> 113
Phe Trp Lys Thr
1

<210> 114
<211> 10
<212> PRT
<213> Artificial Sequence

<220>
<221> source
<223> /note="Description of Artificial Sequence: Synthetic peptide"

<220>
<221> MOD_RES
<222> (1)..(1)
<223> pyroGlu

<220>
<221> MOD_RES
<222> (10)..(10)
<223> Gly-CONH2

<400> 114
Glu His Trp Ser Tyr Gly Leu Arg Pro Gly
1               5                   10

<210> 115
<211> 5
<212> PRT
<213> Unknown

<220>
<221> source
<223> /note="Description of Unknown: Enterokinase peptide"

<400> 115
Asp Asp Asp Asp Lys
1               5

<210> 116
<211> 4
<212> PRT
<213> Unknown

<220>
<221> source
<223> /note="Description of Unknown: Factor Xa peptide"

<400> 116
Ile Glu Gly Arg
1

<210> 117
<211> 4

<212> PRT
<213> Unknown

<220>
<221> source
<223> /note="Description of Unknown: Factor Xa peptide"

<400> 117
Ile Asp Gly Arg
1

<210> 118
<211> 7
<212> PRT
<213> Tobacco etch virus

<400> 118
Glu Asn Leu Tyr Phe Gln Gly
1               5

<210> 119
<211> 6
<212> PRT
<213> Unknown

<220>
<221> source
<223> /note="Description of Unknown: Thrombin peptide"

<400> 119
Leu Val Pro Arg Gly Ser
1               5

<210> 120
<211> 8
<212> PRT
<213> Artificial Sequence

<220>
<221> source
<223> /note="Description of Artificial Sequence: Synthetic peptide"

<400> 120
Leu Glu Val Leu Phe Gln Gly Pro
1               5

<210> 121
<211> 6
<212> PRT
<213> Artificial Sequence

<220>
<221> source
<223> /note="Description of Artificial Sequence: Synthetic 6xHis tag"

<400> 121
His His His His His His

1 5

<210> 122
<211> 4
<212> PRT
<213> Unknown

<220>
<221> source
<223> /note="Description of Unknown: Caspase 3 peptide"

<400> 122
Asp Met Gln Asp
1

<210> 123
<211> 6
<212> PRT
<213> Unknown

<220>
<221> source
<223> /note="Description of Unknown: Thrombin peptide"

<400> 123
Leu Val Pro Arg Gly Ser
1 5

<210> 124
<211> 10
<212> PRT
<213> Unknown

<220>
<221> source
<223> /note="Description of Unknown: ADAM17 peptide"

<400> 124
Pro Leu Ala Gln Ala Val Arg Ser Ser Ser
1 5 10

<210> 125
<211> 10
<212> PRT
<213> Homo sapiens

<400> 125
Ser Lys Gly Arg Ser Leu Ile Gly Arg Val
1 5 10

<210> 126
<211> 6
<212> PRT
<213> Unknown

<220>
<221> source
<223> /note="Description of Unknown: Elastase peptide"

<400> 126
Met Glu Ala Val Thr Tyr
1               5

<210> 127
<211> 4
<212> PRT
<213> Unknown

<220>
<221> source
<223> /note="Description of Unknown: Granzyme peptide"

<400> 127
Ile Glu Pro Asp
1

<210> 128
<211> 4
<212> PRT
<213> Unknown

<220>
<221> source
<223> /note="Description of Unknown: Caspase 2 peptide"

<400> 128
Asp Val Ala Asp
1

<210> 129
<211> 4
<212> PRT
<213> Unknown

<220>
<221> source
<223> /note="Description of Unknown: Caspase 4 peptide"

<400> 129
Leu Glu Val Asp
1

<210> 130
<211> 4
<212> PRT
<213> Unknown

<220>
<221> source
<223> /note="Description of Unknown: Caspase 7 peptide"

<400> 130
Asp Glu Val Asp
1

<210> 131

<211> 4
<212> PRT
<213> Unknown

<220>
<221> source
<223> /note="Description of Unknown: Caspase 9 peptide"

<400> 131
Leu Glu His Asp
1

<210> 132
<211> 4
<212> PRT
<213> Unknown

<220>
<221> source
<223> /note="Description of Unknown: Caspase 10 peptide"

<400> 132
Ile Glu His Asp
1

<210> 133
<211> 116
<212> PRT
<213> Homo sapiens

<400> 133
```
Met Leu Ser Cys Arg Leu Gln Cys Ala Leu Ala Ala Leu Ser Ile Val
1               5                   10                  15

Leu Ala Leu Gly Cys Val Thr Gly Ala Pro Ser Asp Pro Arg Leu Arg
            20                  25                  30

Gln Phe Leu Gln Lys Ser Leu Ala Ala Ala Ala Gly Lys Gln Glu Leu
        35                  40                  45

Ala Lys Tyr Phe Leu Ala Glu Leu Leu Ser Glu Pro Asn Gln Thr Glu
    50                  55                  60

Asn Asp Ala Leu Glu Pro Glu Asp Leu Ser Gln Ala Ala Glu Gln Asp
65                  70                  75                  80

Glu Met Arg Leu Glu Leu Gln Arg Ser Ala Asn Ser Asn Pro Ala Met
                85                  90                  95

Ala Pro Arg Glu Arg Lys Ala Gly Cys Lys Asn Phe Phe Trp Lys Thr
            100                 105                 110

Phe Thr Ser Cys
        115
```

<210> 134
<211> 155
<212> PRT
<213> Homo sapiens

<400> 134
Met Tyr Arg His Lys Asn Ser Trp Arg Leu Gly Leu Lys Tyr Pro Pro
1               5                   10                  15

Ser Ser Lys Glu Glu Thr Gln Val Pro Lys Thr Leu Ile Ser Gly Leu
            20                  25                  30

Pro Gly Arg Lys Ser Ser Ser Arg Val Gly Glu Lys Leu Gln Ser Ala
        35                  40                  45

His Lys Met Pro Leu Ser Pro Gly Leu Leu Leu Leu Leu Leu Ser Gly
    50                  55                  60

Ala Thr Ala Thr Ala Ala Leu Pro Leu Glu Gly Gly Pro Thr Gly Arg
65                  70                  75                  80

Asp Ser Glu His Met Gln Glu Ala Ala Gly Ile Arg Lys Ser Ser Leu
                85                  90                  95

Leu Thr Phe Leu Ala Trp Trp Phe Glu Trp Thr Ser Gln Ala Ser Ala
            100                 105                 110

Gly Pro Leu Ile Gly Glu Glu Ala Arg Glu Val Ala Arg Arg Gln Glu
        115                 120                 125

Gly Ala Pro Pro Gln Gln Ser Ala Arg Arg Asp Arg Met Pro Cys Arg
    130                 135                 140

Asn Phe Phe Trp Lys Thr Phe Ser Ser Cys Lys
145                 150                 155

<210> 135
<211> 7
<212> PRT
<213> Homo sapiens

<400> 135
Asn Phe Phe Trp Lys Thr Phe
1               5

<210> 136
<211> 8
<212> PRT
<213> Homo sapiens

<220>
<221> VARIANT
<222> (1)..(1)
<223> /replace="Lys"

<220>
<221> misc_feature
<222> (1)..(1)
<223> /note="Residue given in the sequence has no preference with respect to the residue in the annotation for said position"

<400> 136
Arg Asn Phe Phe Trp Lys Thr Phe
1               5

<210> 137
<211> 9
<212> PRT
<213> Homo sapiens

<220>
<221> VARIANT
<222> (2)..(2)
<223> /replace="Lys"

<220>
<221> misc_feature
<222> (2)..(2)
<223> /note="Residue given in the sequence has no preference with respect to the residue in the annotation for said position"

<400> 137
Cys Arg Asn Phe Phe Trp Lys Thr Phe
1               5

<210> 138
<211> 10
<212> PRT
<213> Homo sapiens

<220>
<221> VARIANT
<222> (1)..(1)
<223> /replace="Gly"

<220>
<221> misc_feature
<222> (1)..(1)
<223> /note="Residue given in the sequence has no preference with respect to the residue in the annotation for said position"

<220>
<221> VARIANT
<222> (3)..(3)
<223> /replace="Lys"

<220>
<221> misc_feature

<222> (3)..(3)
<223> /note="Residue given in the sequence has no preference with respect to the residue in the annotation for said position"

<400> 138
Pro Cys Arg Asn Phe Phe Trp Lys Thr Phe
1 5 10

<210> 139
<211> 8
<212> PRT
<213> Homo sapiens

<220>
<221> VARIANT
<222> (8)..(8)
<223> /replace="Thr"

<220>
<221> misc_feature
<222> (8)..(8)
<223> /note="Residue given in the sequence has no preference with respect to the residue in the annotation for said position"

<400> 139
Asn Phe Phe Trp Lys Thr Phe Ser
1 5

<210> 140
<211> 9
<212> PRT
<213> Homo sapiens

<220>
<221> VARIANT
<222> (8)..(8)
<223> /replace="Thr"

<220>
<221> misc_feature
<222> (8)..(8)
<223> /note="Residue given in the sequence has no preference with respect to the residue in the annotation for said position"

<400> 140
Asn Phe Phe Trp Lys Thr Phe Ser Ser
1 5

<210> 141
<211> 10
<212> PRT
<213> Homo sapiens

<220>
<221> VARIANT
<222> (8)..(8)
<223> /replace="Thr"

<220>
<221> misc_feature
<222> (8)..(8)
<223> /note="Residue given in the sequence has no preference with respect to the residue in the annotation for said position"

<400> 141
Asn Phe Phe Trp Lys Thr Phe Ser Ser Cys
1 5 10

<210> 142
<211> 9
<212> PRT
<213> Homo sapiens

<220>
<221> VARIANT
<222> (1)..(1)
<223> /replace="Lys"

<220>
<221> misc_feature
<222> (1)..(1)
<223> /note="Residue given in the sequence has no preference with respect to the residue in the annotation for said position"

<220>
<221> VARIANT
<222> (9)..(9)
<223> /replace="Thr"

<220>
<221> misc_feature
<222> (9)..(9)
<223> /note="Residue given in the sequence has no preference with respect to the residue in the annotation for said position"

<400> 142
Arg Asn Phe Phe Trp Lys Thr Phe Ser
1 5

<210> 143
<211> 10
<212> PRT
<213> Homo sapiens

<220>
<221> VARIANT
<222> (1)..(1)
<223> /replace="Lys"

<220>
<221> misc_feature
<222> (1)..(1)
<223> /note="Residue given in the sequence has no preference with respect to the residue in the annotation for said position"

<220>

<221> VARIANT
<222> (9)..(9)
<223> /replace="Thr"

<220>
<221> misc_feature
<222> (9)..(9)
<223> /note="Residue given in the sequence has no preference with respect to the residue in the annotation for said position"

<400> 143
Arg Asn Phe Phe Trp Lys Thr Phe Ser Ser
1               5                   10

<210> 144
<211> 11
<212> PRT
<213> Homo sapiens

<220>
<221> VARIANT
<222> (1)..(1)
<223> /replace="Lys"

<220>
<221> misc_feature
<222> (1)..(1)
<223> /note="Residue given in the sequence has no preference with respect to the residue in the annotation for said position"

<220>
<221> VARIANT
<222> (9)..(9)
<223> /replace="Thr"

<220>
<221> misc_feature
<222> (9)..(9)
<223> /note="Residue given in the sequence has no preference with respect to the residue in the annotation for said position"

<400> 144
Arg Asn Phe Phe Trp Lys Thr Phe Ser Ser Cys
1               5                   10

<210> 145
<211> 10
<212> PRT
<213> Homo sapiens

<220>
<221> VARIANT
<222> (2)..(2)
<223> /replace="Lys"

<220>
<221> misc_feature
<222> (2)..(2)
<223> /note="Residue given in the sequence has no preference with

respect to the residue in the annotation for said position"

<220>
<221> VARIANT
<222> (10)..(10)
<223> /replace="Thr"

<220>
<221> misc_feature
<222> (10)..(10)
<223> /note="Residue given in the sequence has no preference with respect to the residue in the annotation for said position"

<400> 145
Cys Arg Asn Phe Phe Trp Lys Thr Phe Ser
1 5 10

<210> 146
<211> 11
<212> PRT
<213> Homo sapiens

<220>
<221> VARIANT
<222> (2)..(2)
<223> /replace="Lys"

<220>
<221> misc_feature
<222> (2)..(2)
<223> /note="Residue given in the sequence has no preference with respect to the residue in the annotation for said position"

<220>
<221> VARIANT
<222> (10)..(10)
<223> /replace="Thr"

<220>
<221> misc_feature
<222> (10)..(10)
<223> /note="Residue given in the sequence has no preference with respect to the residue in the annotation for said position"

<400> 146
Cys Arg Asn Phe Phe Trp Lys Thr Phe Ser Ser
1 5 10

<210> 147
<211> 12
<212> PRT
<213> Homo sapiens

<220>
<221> VARIANT
<222> (2)..(2)
<223> /replace="Lys"

<220>

<221> misc_feature
<222> (2)..(2)
<223> /note="Residue given in the sequence has no preference with respect to the residue in the annotation for said position"

<220>
<221> VARIANT
<222> (10)..(10)
<223> /replace="Thr"

<220>
<221> misc_feature
<222> (10)..(10)
<223> /note="Residue given in the sequence has no preference with respect to the residue in the annotation for said position"

<400> 147
Cys Arg Asn Phe Phe Trp Lys Thr Phe Ser Ser Cys
1 5 10

<210> 148
<211> 11
<212> PRT
<213> Homo sapiens

<220>
<221> VARIANT
<222> (1)..(1)
<223> /replace="Gly"

<220>
<221> misc_feature
<222> (1)..(1)
<223> /note="Residue given in the sequence has no preference with respect to the residue in the annotation for said position"

<220>
<221> VARIANT
<222> (3)..(3)
<223> /replace="Lys"

<220>
<221> misc_feature
<222> (3)..(3)
<223> /note="Residue given in the sequence has no preference with respect to the residue in the annotation for said position"

<220>
<221> VARIANT
<222> (11)..(11)
<223> /replace="Thr"

<220>
<221> misc_feature
<222> (11)..(11)
<223> /note="Residue given in the sequence has no preference with respect to the residue in the annotation for said position"

<400> 148
Pro Cys Arg Asn Phe Phe Trp Lys Thr Phe Ser
1 5 10

<210> 149
<211> 12
<212> PRT
<213> Homo sapiens

<220>
<221> VARIANT
<222> (1)..(1)
<223> /replace="Gly"

<220>
<221> misc_feature
<222> (1)..(1)
<223> /note="Residue given in the sequence has no preference with respect to the residue in the annotation for said position"

<220>
<221> VARIANT
<222> (3)..(3)
<223> /replace="Lys"

<220>
<221> misc_feature
<222> (3)..(3)
<223> /note="Residue given in the sequence has no preference with respect to the residue in the annotation for said position"

<220>
<221> VARIANT
<222> (11)..(11)
<223> /replace="Thr"

<220>
<221> misc_feature
<222> (11)..(11)
<223> /note="Residue given in the sequence has no preference with respect to the residue in the annotation for said position"

<400> 149
```
Pro Cys Arg Asn Phe Phe Trp Lys Thr Phe Ser Ser
1               5                   10
```

<210> 150
<211> 12
<212> PRT
<213> Homo sapiens

<220>
<221> VARIANT
<222> (1)..(1)
<223> /replace="Gly"

<220>
<221> misc_feature
<222> (1)..(1)
<223> /note="Residue given in the sequence has no preference with respect to the residue in the annotation for said position"

<220>
<221> VARIANT
<222> (3)..(3)
<223> /replace="Lys"

<220>
<221> misc_feature
<222> (3)..(3)
<223> /note="Residue given in the sequence has no preference with respect to the residue in the annotation for said position"

<220>
<221> VARIANT
<222> (11)..(11)
<223> /replace="Thr"

<220>
<221> misc_feature
<222> (11)..(11)
<223> /note="Residue given in the sequence has no preference with respect to the residue in the annotation for said position"

<400> 150
Pro Cys Arg Asn Phe Phe Trp Lys Thr Phe Ser Cys
1 5 10

<210> 151
<211> 29
<212> PRT
<213> Artificial Sequence

<220>
<221> source
<223> /note="Description of Artificial Sequence: Synthetic peptide"

<400> 151
Tyr Ala Asp Ala Ile Phe Thr Ala Ser Tyr Arg Lys Val Leu Gly Gln
1 5 10 15

Leu Ser Ala Arg Lys Leu Leu Gln Asp Ile Leu Ser Arg
20 25

<210> 152
<211> 29
<212> PRT
<213> Artificial Sequence

<220>
<221> source
<223> /note="Description of Artificial Sequence: Synthetic peptide"

<400> 152
Tyr Ala Asp Ala Ile Phe Thr Ala Ser Tyr Arg Asn Val Leu Gly Gln
1 5 10 15

Leu Ser Ala Arg Lys Leu Leu Gln Asp Ile Leu Ser Arg
20 25

<210> 153
<211> 27
<212> PRT
<213> Artificial Sequence

<220>
<221> source
<223> /note="Description of Artificial Sequence: Synthetic peptide"

<400> 153
Tyr Ala Asp Ala Ile Phe Thr Asn Ser Tyr Arg Lys Val Leu Gly Gln
1               5                   10                  15

Leu Ser Ala Arg Lys Leu Leu Gln Asp Ile Met
            20                  25

<210> 154
<211> 28
<212> PRT
<213> Artificial Sequence

<220>
<221> source
<223> /note="Description of Artificial Sequence: Synthetic peptide"

<400> 154
Tyr Ala Asp Ala Ile Phe Thr Asn Ser Tyr Arg Lys Val Leu Gly Gln
1               5                   10                  15

Leu Ser Ala Arg Lys Leu Leu Gln Asp Ile Met Ser
            20                  25

<210> 155
<211> 28
<212> PRT
<213> Artificial Sequence

<220>
<221> source
<223> /note="Description of Artificial Sequence: Synthetic peptide"

<400> 155
Ala Asp Ala Ile Phe Thr Asn Ser Tyr Arg Lys Val Leu Gly Gln Leu
1               5                   10                  15

Ser Ala Arg Lys Leu Leu Gln Asp Ile Met Ser Arg
            20                  25

<210> 156
<211> 44
<212> PRT
<213> Artificial Sequence

<220>
<221> source
<223> /note="Description of Artificial Sequence: Synthetic polypeptide"

<400> 156
Tyr Ala Asp Ala Ile Phe Thr Asn Ser Tyr Arg Lys Val Leu Gly Gln
1 5 10 15

Leu Ser Ala Arg Lys Leu Leu Gln Asp Ile Met Ser Arg Gln Gln Gly
20 25 30

Glu Ser Asn Gln Glu Arg Gly Ala Arg Ala Arg Leu
35 40

<210> 157
<211> 40
<212> PRT
<213> Artificial Sequence

<220>
<221> source
<223> /note="Description of Artificial Sequence: Synthetic polypeptide"

<400> 157
Tyr Ala Asp Ala Ile Phe Thr Asn Ser Tyr Arg Lys Val Leu Gly Gln
1 5 10 15

Leu Ser Ala Arg Lys Leu Leu Gln Asp Ile Met Ser Arg Gln Gln Gly
20 25 30

Glu Ser Asn Gln Glu Arg Gly Ala
35 40

<210> 158
<211> 29
<212> PRT
<213> Artificial Sequence

<220>
<221> source
<223> /note="Description of Artificial Sequence: Synthetic peptide"

<400> 158
Tyr Ala Asp Ala Ile Phe Thr Asn Ala Tyr Arg Lys Val Leu Gly Gln
1 5 10 15

Leu Ser Ala Arg Lys Leu Leu Gln Asp Ile Met Ser Arg
20 25

<210> 159
<211> 29

<212> PRT
<213> Artificial Sequence

<220>
<221> source
<223> /note="Description of Artificial Sequence: Synthetic peptide"

<400> 159
Tyr Ala Asp Ala Ile Phe Thr Asn Ser Tyr Arg Lys Val Leu Gly Gln
1 5 10 15

Leu Ser Ala Arg Lys Ala Leu Gln Asp Ile Met Ser Arg
20 25

<210> 160
<211> 29
<212> PRT
<213> Artificial Sequence

<220>
<221> source
<223> /note="Description of Artificial Sequence: Synthetic peptide"

<400> 160
Tyr Ala Asp Ala Ile Phe Thr Ala Ser Tyr Lys Lys Val Leu Gly Gln
1 5 10 15

Leu Ser Ala Arg Lys Leu Leu Gln Asp Ile Met Ser Arg
20 25

<210> 161
<211> 29
<212> PRT
<213> Artificial Sequence

<220>
<221> source
<223> /note="Description of Artificial Sequence: Synthetic peptide"

<400> 161
Tyr Ala Asp Ala Ile Phe Thr Ala Ser Tyr Lys Arg Val Leu Gly Gln
1 5 10 15

Leu Ser Ala Arg Lys Leu Leu Gln Asp Ile Met Ser Arg
20 25

<210> 162
<211> 29
<212> PRT
<213> Artificial Sequence

<220>
<221> source
<223> /note="Description of Artificial Sequence: Synthetic

peptide"

<400> 162
Tyr Ala Asp Ala Ile Phe Thr Ala Ser Tyr Asn Lys Val Leu Gly Gln
1 5 10 15

Leu Ser Ala Arg Lys Leu Leu Gln Asp Ile Met Ser Arg
20 25

<210> 163
<211> 29
<212> PRT
<213> Artificial Sequence

<220>
<221> source
<223> /note="Description of Artificial Sequence: Synthetic peptide"

<400> 163
Tyr Ala Asp Ala Ile Phe Thr Ala Ser Tyr Arg Lys Val Leu Gly Gln
1 5 10 15

Leu Ser Ala Lys Lys Leu Leu Gln Asp Ile Met Ser Arg
20 25

<210> 164
<211> 29
<212> PRT
<213> Artificial Sequence

<220>
<221> source
<223> /note="Description of Artificial Sequence: Synthetic peptide"

<400> 164
Tyr Ala Asp Ala Ile Phe Thr Ala Ser Tyr Lys Lys Val Leu Gly Gln
1 5 10 15

Leu Ser Ala Lys Lys Leu Leu Gln Asp Ile Met Ser Arg
20 25

<210> 165
<211> 29
<212> PRT
<213> Artificial Sequence

<220>
<221> source
<223> /note="Description of Artificial Sequence: Synthetic peptide"

<400> 165
Tyr Ala Asp Ala Ile Phe Thr Ala Ser Tyr Arg Lys Val Leu Gly Gln
1 5 10 15

Leu Ser Ala Asn Lys Leu Leu Gln Asp Ile Met Ser Arg
20 25

<210> 166
<211> 29
<212> PRT
<213> Artificial Sequence

<220>
<221> source
<223> /note="Description of Artificial Sequence: Synthetic peptide"

<400> 166
Tyr Ala Asp Ala Ile Phe Thr Ala Ser Tyr Arg Asn Val Leu Gly Gln
1 5 10 15

Leu Ser Ala Arg Lys Leu Leu Gln Asp Ile Met Ser Arg
20 25

<210> 167
<211> 29
<212> PRT
<213> Artificial Sequence

<220>
<221> source
<223> /note="Description of Artificial Sequence: Synthetic peptide"

<400> 167
Tyr Ala Asp Ala Ile Phe Thr Ala Ser Tyr Arg Lys Val Leu Gly Gln
1 5 10 15

Leu Ser Ala Arg Asn Leu Leu Gln Asp Ile Met Ser Arg
20 25

<210> 168
<211> 29
<212> PRT
<213> Artificial Sequence

<220>
<221> source
<223> /note="Description of Artificial Sequence: Synthetic peptide"

<400> 168
Tyr Ala Asp Ala Ile Phe Glu Ala Ser Tyr Arg Lys Val Leu Gly Gln
1 5 10 15

Leu Ser Ala Arg Lys Leu Leu Gln Asp Ile Met Ser Arg
20 25

<210> 169

<211> 29
<212> PRT
<213> Artificial Sequence

<220>
<221> source
<223> /note="Description of Artificial Sequence: Synthetic peptide"

<400> 169
Tyr Ala Asp Ala Ile Phe Thr Ala Ser Glu Arg Lys Val Leu Gly Gln
1 5 10 15

Leu Ser Ala Arg Lys Leu Leu Gln Asp Ile Met Ser Arg
20 25

<210> 170
<211> 29
<212> PRT
<213> Artificial Sequence

<220>
<221> source
<223> /note="Description of Artificial Sequence: Synthetic peptide"

<400> 170
Tyr Ala Asp Ala Ile Phe Thr Ala Ser Tyr Arg Lys Glu Leu Gly Gln
1 5 10 15

Leu Ser Ala Arg Lys Leu Leu Gln Asp Ile Met Ser Arg
20 25

<210> 171
<211> 29
<212> PRT
<213> Artificial Sequence

<220>
<221> source
<223> /note="Description of Artificial Sequence: Synthetic peptide"

<400> 171
Tyr Ala Asp Ala Ile Phe Thr Ala Ser Tyr Arg Lys Val Leu Gly Gln
1 5 10 15

Leu Ser Ala Arg Lys Leu Leu Gln Asp Ile Met Ser Arg
20 25

<210> 172
<211> 29
<212> PRT
<213> Artificial Sequence

<220>
<221> source

<223> /note="Description of Artificial Sequence: Synthetic peptide"

<400> 172
Tyr Ala Asp Ala Ile Phe Thr Glu Ser Tyr Arg Lys Val Leu Gly Gln
1               5                   10                  15

Leu Ser Ala Arg Lys Leu Leu Gln Asp Ile Met Ser Arg
            20                  25

<210> 173
<211> 27
<212> PRT
<213> Artificial Sequence

<220>
<221> source
<223> /note="Description of Artificial Sequence: Synthetic peptide"

<400> 173
Tyr Ala Asp Ala Ile Phe Thr Asn Ser Tyr Arg Lys Val Leu Ala Gln
1               5                   10                  15

Leu Ser Ala Arg Lys Leu Leu Gln Asp Ile Met
            20                  25

<210> 174
<211> 29
<212> PRT
<213> Artificial Sequence

<220>
<221> source
<223> /note="Description of Artificial Sequence: Synthetic peptide"

<400> 174
Tyr Ala Asp Ala Ile Phe Thr Asn Ser Tyr Arg Lys Val Leu Ala Gln
1               5                   10                  15

Leu Ser Ala Arg Lys Leu Leu Gln Asp Ile Met Ser Arg
            20                  25

<210> 175
<211> 29
<212> PRT
<213> Artificial Sequence

<220>
<221> source
<223> /note="Description of Artificial Sequence: Synthetic peptide"

<400> 175
Tyr Ala Asp Ala Ile Phe Thr Ala Ser Tyr Arg Lys Val Leu Ala Gln
1               5                   10                  15

Leu Ser Ala Arg Lys Leu Leu Gln Asp Ile Met Ser Arg
20 25

<210> 176
<211> 29
<212> PRT
<213> Artificial Sequence

<220>
<221> source
<223> /note="Description of Artificial Sequence: Synthetic peptide"

<400> 176
Tyr Ala Asp Ala Ile Phe Thr Ala Ala Tyr Arg Lys Val Leu Ala Gln
1 5 10 15

Leu Ser Ala Arg Lys Ala Leu Gln Asp Ile Ala Ser Arg
20 25

<210> 177
<211> 29
<212> PRT
<213> Artificial Sequence

<220>
<221> source
<223> /note="Description of Artificial Sequence: Synthetic peptide"

<400> 177
Tyr Ala Asp Ala Ile Phe Thr Ala Ala Tyr Arg Lys Val Leu Ala Gln
1 5 10 15

Leu Ser Ala Arg Lys Ala Leu Gln Asp Ile Met Ser Arg
20 25

<210> 178
<211> 40
<212> PRT
<213> Artificial Sequence

<220>
<221> source
<223> /note="Description of Artificial Sequence: Synthetic polypeptide"

<400> 178
His Val Asp Ala Ile Phe Thr Gln Ser Tyr Arg Lys Val Leu Ala Gln
1 5 10 15

Leu Ser Ala Arg Lys Leu Leu Gln Asp Ile Leu Asn Arg Gln Gln Gly
20 25 30

```
Glu Arg Asn Gln Glu Gln Gly Ala
        35                  40


<210> 179
<211> 32
<212> PRT
<213> Artificial Sequence

<220>
<221> source
<223> /note="Description of Artificial Sequence: Synthetic
      polypeptide"

<400> 179
His Val Asp Ala Ile Phe Thr Gln Ser Tyr Arg Lys Val Leu Ala Gln
1               5                   10                  15


Leu Ser Ala Arg Lys Ala Leu Gln Asp Ile Leu Ser Arg Gln Gln Gly
            20                  25                  30


<210> 180
<211> 29
<212> PRT
<213> Artificial Sequence

<220>
<221> source
<223> /note="Description of Artificial Sequence: Synthetic
      peptide"

<400> 180
His Val Asp Ala Ile Phe Thr Ser Ser Tyr Arg Lys Val Leu Ala Gln
1               5                   10                  15


Leu Ser Ala Arg Lys Leu Leu Gln Asp Ile Leu Ser Arg
            20                  25


<210> 181
<211> 29
<212> PRT
<213> Artificial Sequence

<220>
<221> source
<223> /note="Description of Artificial Sequence: Synthetic
      peptide"

<400> 181
His Val Asp Ala Ile Phe Thr Thr Ser Tyr Arg Lys Val Leu Ala Gln
1               5                   10                  15


Leu Ser Ala Arg Lys Leu Leu Gln Asp Ile Leu Ser Arg
            20                  25


<210> 182
<211> 29
```

<212> PRT
<213> Artificial Sequence

<220>
<221> source
<223> /note="Description of Artificial Sequence: Synthetic peptide"

<400> 182
Tyr Ala Asp Ala Ile Phe Thr Gln Ser Tyr Arg Lys Val Leu Ala Gln
1 5 10 15

Leu Ser Ala Arg Lys Ala Leu Gln Asp Ile Leu Asn Arg
20 25

<210> 183
<211> 29
<212> PRT
<213> Artificial Sequence

<220>
<221> source
<223> /note="Description of Artificial Sequence: Synthetic peptide"

<400> 183
Tyr Ala Asp Ala Ile Phe Thr Gln Ser Tyr Arg Lys Val Leu Ala Gln
1 5 10 15

Leu Ser Ala Arg Lys Ala Leu Gln Asp Ile Leu Ser Arg
20 25

<210> 184
<211> 6
<212> PRT
<213> Artificial Sequence

<220>
<221> source
<223> /note="Description of Artificial Sequence: Synthetic peptide"

<220>
<221> misc_feature
<222> (1)..(6)
<223> /note="cylic"

<400> 184
Pro Phe Trp Lys Thr Phe
1 5

<210> 185
<211> 6
<212> PRT
<213> Artificial Sequence

<220>

<221> source
<223> /note="Description of Artificial Sequence: Synthetic peptide"

<220>
<221> misc_feature
<222> (1)..(6)
<223> /note="cylic"

<220>
<221> MOD_RES
<222> (3)..(3)
<223> 5-fluoro-Trp

<400> 185
Pro Phe Trp Lys Thr Phe
1               5

<210> 186
<211> 8
<212> PRT
<213> Artificial Sequence

<220>
<221> source
<223> /note="Description of Artificial Sequence: Synthetic peptide"

<220>
<221> misc_feature
<222> (1)..(8)
<223> /note="cylic"

<220>
<221> MOD_RES
<222> (4)..(4)
<223> 5-bromo-Trp

<220>
<221> MOD_RES
<222> (8)..(8)
<223> Sigma-aminobutyric acid

<400> 186
Asn Phe Phe Trp Lys Thr Phe Xaa
1               5

<210> 187
<211> 10
<212> PRT
<213> Unknown

<220>
<221> source
<223> /note="Description of Unknown: Bombesin peptide"

<220>
<221> misc_feature

<222> (10)..(10)
<223> /note="C-terminus amidated"

<400> 187
Gly Asn Gln Trp Ala Val Gly His Leu Met
1 5 10

<210> 188
<211> 10
<212> PRT
<213> Unknown

<220>
<221> source
<223> /note="Description of Unknown: Neuromedin B peptide"

<220>
<221> misc_feature
<222> (10)..(10)
<223> /note="C-terminus amidated"

<400> 188
Gly Asn Leu Trp Ala Thr Gly His Phe Met
1 5 10

<210> 189
<211> 10
<212> PRT
<213> Unknown

<220>
<221> source
<223> /note="Description of Unknown: Neuromedin C peptide"

<220>
<221> misc_feature
<222> (10)..(10)
<223> /note="C-terminus amidated"

<400> 189
Gly Asn His Trp Ala Val Gly His Leu Met
1 5 10

<210> 190
<211> 9
<212> PRT
<213> Unknown

<220>
<221> source
<223> /note="Description of Unknown: Litorin peptide"

<220>
<221> MOD_RES
<222> (1)..(1)
<223> pGlu

<220>
<221> misc_feature
<222> (9)..(9)
<223> /note="C-terminus amidated"

<400> 190
Glu Gln Trp Ala Val Gly His Phe Met
1               5

<210> 191
<211> 10
<212> PRT
<213> Homo sapiens

<220>
<221> misc_feature
<222> (10)..(10)
<223> /note="C-terminus amidated"

<400> 191
Gly Asn His Trp Ala Val Gly His Leu Met
1               5                   10

<210> 192
<211> 8
<212> PRT
<213> Artificial Sequence

<220>
<221> source
<223> /note="Description of Artificial Sequence: Synthetic peptide"

<220>
<221> MOD_RES
<222> (1)..(1)
<223> Desamino-Phe

<220>
<221> MOD_RES
<222> (8)..(8)
<223> Leu-CH2N-thiazolidine-4-carboxylic acid

<220>
<221> misc_feature
<222> (8)..(8)
<223> /note="C-terminus amidated"

<400> 192
Phe Gln Trp Ala Val Gly His Leu
1               5

<210> 193
<211> 7
<212> PRT
<213> Homo sapiens

<220>
<221> MOD_RES
<222> (1)..(1)
<223> 2,3,4,9 tetrahydro-1 H-pyrido-[3,4-b] indole-3-carboxylic acid-Gln

<220>
<221> MOD_RES
<222> (7)..(7)
<223> Leu-CH2N-2,3,4,9 tetrahydro-1 H-pyrido-[3,4-b] indole-3-carboxylic acid

<220>
<221> misc_feature
<222> (7)..(7)
<223> /note="C-terminus amidated"

<400> 193
Gln Trp Ala Val Gly His Leu
1               5

<210> 194
<211> 7
<212> PRT
<213> Artificial Sequence

<220>
<221> source
<223> /note="Description of Artificial Sequence: Synthetic peptide"

<220>
<221> MOD_RES
<222> (1)..(1)
<223> NH2CO-2,3,4,9 tetrahydro-1 H-pyrido-[3,4-b] indole-3-carboxylic acid-Gln

<220>
<221> MOD_RES
<222> (7)..(7)
<223> Leu-CH2N-2,3,4,9 tetrahydro-1 H-pyrido-[3,4-b] indole-3-carboxylic acid

<220>
<221> misc_feature
<222> (7)..(7)
<223> /note="C-terminus amidated"

<400> 194
Gln Trp Ala Val Gly His Leu
1               5

<210> 195
<211> 7
<212> PRT
<213> Artificial Sequence

<220>
<221> source
<223> /note="Description of Artificial Sequence: Synthetic

peptide"

<220>
<221> MOD_RES
<222> (1)..(1)
<223> Acetyl-2,3,4,9 tetrahydro-1 H-pyrido-[3,4-b] indole-3-carboxylic acid-Gln, Octanoyl-2,3,4,9 tetrahydro-1 H-pyrido-[3,4-b] indole-3-carboxylic acid-Gln or 3-hydroxy-2-naphthoyl-2,3,4,9 tetrahydro-1 H-pyrido-[3,4-b] indole-3-carboxylic acid-Gln

<220>
<221> MOD_RES
<222> (7)..(7)
<223> Leu-CH2N-2,3,4,9 tetrahydro-1 H-pyrido-[3,4-b] indole-3-carboxylic acid

<220>
<221> misc_feature
<222> (7)..(7)
<223> /note="C-terminus amidated"

<400> 195
Gln Trp Ala Val Gly His Leu
1               5

<210> 196
<211> 9
<212> PRT
<213> Artificial Sequence

<220>
<221> source
<223> /note="Description of Artificial Sequence: Synthetic peptide"

<220>
<221> MOD_RES
<222> (1)..(1)
<223> pGlu

<220>
<221> misc_feature
<222> (9)..(9)
<223> /note="C-terminus amidated"

<400> 196
Glu Gln Trp Ala Val Gly His Leu Leu
1               5

<210> 197
<211> 9
<212> PRT
<213> Artificial Sequence

<220>
<221> source
<223> /note="Description of Artificial Sequence: Synthetic peptide"

<220>
<221> MOD_RES
<222> (1)..(1)
<223> pGlu

<220>
<221> misc_feature
<222> (9)..(9)
<223> /note="C-terminus amidated"

<400> 197
Glu Gln Trp Ala Val Gly His Phe Leu
1               5

<210> 198
<211> 8
<212> PRT
<213> Artificial Sequence

<220>
<221> source
<223> /note="Description of Artificial Sequence: Synthetic
      peptide"

<220>
<221> misc_feature
<222> (1)..(8)
<223> /note="cyclic"

<400> 198
His Trp Ala Val Gly His Leu Met
1               5

<210> 199
<211> 8
<212> PRT
<213> Artificial Sequence

<220>
<221> source
<223> /note="Description of Artificial Sequence: Synthetic
      peptide"

<220>
<221> misc_feature
<222> (8)..(8)
<223> /note="C-terminus amidated"

<400> 199
Cys Trp Ala Val Gly His Leu Cys
1               5

<210> 200
<211> 7
<212> PRT
<213> Artificial Sequence

<220>
<221> source
<223> /note="Description of Artificial Sequence: Synthetic peptide"

<220>
<221> misc_feature
<222> (1)..(7)
<223> /note="cyclic"

<400> 200
Trp Ala Val Gly His Leu Met
1               5

<210> 201
<211> 8
<212> PRT
<213> Artificial Sequence

<220>
<221> source
<223> /note="Description of Artificial Sequence: Synthetic peptide"

<220>
<221> misc_feature
<222> (2)..(7)
<223> /note="cyclic"

<400> 201
Asp Cys Phe Trp Lys Tyr Cys Val
1               5

<210> 202
<211> 23
<212> PRT
<213> Influenza virus

<400> 202
Gly Leu Phe Gly Ala Ile Ala Gly Phe Ile Glu Asn Gly Trp Glu Gly
1               5                   10                  15

Met Ile Asp Gly Trp Tyr Gly
            20

<210> 203
<211> 23
<212> PRT
<213> Artificial Sequence

<220>
<221> source
<223> /note="Description of Artificial Sequence: Synthetic peptide"

<400> 203
Val Arg Gly Ile Ile Pro Phe Lys Thr Lys Ser Leu Asp Glu Gly Tyr

```
1               5                   10                  15

Asn Lys Ala Leu Asn Asp Leu
            20


<210> 204
<211> 16
<212> PRT
<213> Artificial Sequence

<220>
<221> source
<223> /note="Description of Artificial Sequence: Synthetic
      peptide"

<400> 204
Val Asp Gly Ile Ile Thr Ser Lys Thr Lys Ser Leu Ile Glu Gly Arg
1               5                   10                  15


<210> 205
<211> 23
<212> PRT
<213> Artificial Sequence

<220>
<221> source
<223> /note="Description of Artificial Sequence: Synthetic
      peptide"

<400> 205
Val Asp Gly Ile Ile Thr Ser Lys Thr Lys Ser Asp Asp Asp Asp Lys
1               5                   10                  15

Asn Lys Ala Leu Asn Leu Gln
            20


<210> 206
<211> 15
<212> PRT
<213> Artificial Sequence

<220>
<221> source
<223> /note="Description of Artificial Sequence: Synthetic
      peptide"

<400> 206
Gly Gly Gly Gly Ser Gly Gly Gly Gly Ser Gly Gly Gly Gly Ser
1               5                   10                  15
```

## Description

The present invention relates to therapeutics and corresponding therapies for the treatment of neuroendocrine diseases and conditions.

The neuroendocrine system is formed from cells derived from the embryonic neural crest, neuroectoderm and endoderm. It can be divided into cell types that form glands and others that are diffusely distributed, i.e. the disseminated or diffuse neuroendocrine system. The first group include those cells forming the pituitary, the parathyroid glands and the adrenal medulla. The second group include cells in the skin, lung, thymus thyroid, pancreas, and the GI, biliary and urogenital tracts. Neuroendocrine tumours can arise in all these locations and can cause pathophysiology by either their physical size causing localised pressure or constrictions on surrounding organs, or by abnormal secretions of a variety of hormones and other bioactive molecules. These molecules are normally secreted by non-tumour cells in physiologically appropriate amounts and under tight physiological control. When these cells form tumours, however, the secretions can be excessive leading to disease.

Current therapies for these hypersecretion diseases can include surgical removal of the tumour(s), generic anti-tumour chemotherapy, interferon therapy, radiotherapy and more specific treatment with, for example, somatostatin analogues. The preference for initial treatment mode varies according to the consultant physician and, while each of these approaches can be successful, they are not always appropriate. Depending on the size and location of the tumour surgical intervention may be considered too risky and the tumour may not be completely removed. Anti-tumour chemotherapy, interferon therapy and radiotherapy are sometimes poorly tolerated by the patient or may be contra-indicated for other reasons.

Furthermore, therapies resulting in tumour cell death also introduce the prospect of tumour lysis syndrome (TLS) occurring. TLS is a very serious and sometimes life-threatening complication of tumour therapy. It can be defined as a constellation of metabolic abnormalities resulting from spontaneous or treatment-related tumour necrosis or fulminant apoptosis. The metabolic abnormalities observed in patients with TLS include: hyperkalaemia, hyperuricaemia, and hyperphosphataemia with secondary hypocalcaemia. TLS can also lead to acute renal failure (ARF).

In the majority of patients with metastatic carcinoids and pancreatic endocrine tumours, treatment with current medicaments such as octreotide may induce a rapid improvement in clinical symptoms, such as diarrhoea, dehydration, flushing attacks, hypokalaemia, peptic ulceration, hypoglycaemic attacks and necrotic skin lesions *(Kvols et al. 1986, 1987, Ruszniewski et al.1996, Caplin et al. 1998, Kulke & Mayer 1999, Wymenga et al. 1999).* However, the majority of patients show desensitisation of the inhibition of hormone secretion by octreotide and lanreotide within weeks to months. These limitations on current therapies represent a major problem.

Neuroendocrine tumours, including gastroenteropancreatic endocrine tumours and pituitary adenomas are rare and heterogeneous diseases (table 1). As a result their prognosis and long-term survival are not well known. Regardless of survival prospects, the excessive secretions from such tumours can markedly affect quality of life for the affected individuals and so effective treatment of this aberrant function is a requirement to maintain quality of life in sufferers.

**Table 1 Incidence/prevalence of major neuroendocrine tumours (U.S. unless otherwise stated)**

| Tumour type | Incidence |
|---|---|
| carcinoid tumours | Approximately 5,000 carcinoid tumours per annum are diagnosed. According to the National Cancer Institute (NCI), approximately 74% of these tumours originate in the GI tract and 25% occur in the respiratory tract.<br>Carcinoids are rare in children and are more common in patients older than the age of 50. They are twice as common in men. Carcinoid tumours of the appendix usually are benign and often occur between the ages of 20 and 40. |
| Insulinomas | The incidence is approximately 4 cases per million per year and the prevalence is approximately 4 per million population per year |
| Gastrinomas | The incidence of gastrinomas occurring sporadically or in association with multiple endocrine neoplasia type 1 (MEN-1) is 0.1-3 per million. The prevalence of MEN-1 is 0.2-2 per 100,000. MEN-1 is diagnosed in 30-38% of patients with gastrinomas, whereas 20-61% of patients diagnosed with MEN-1 are found to have gastrinomas associated with ZES (Zollinger-Ellison Syndrome) |
| VIPomas | Prevalence = 1.12 per million of the population |
| Glucagonomas | Glucagonoma is listed as a "rare disease" by the Office of Rare Diseases (ORD) of the National Institutes of Health (NIH). Prevalence = approx 1 in 2,720,000 people in USA |

(continued)

| Tumour type | Incidence |
|---|---|
| Prolactinoma | Incidence: 6-10 per million per year. Prevalence 60-100 per million |
| somatotrophinoma | Prevalance of Acromegaly: 40-60 per million affected people at any time; Incidence (annual) of Acromegaly: 3 per million annual cases |
| corticotrophinoma | Incidence: 2-3 per million per year. Prevalence 20-30 per million |
| phaeochromocytoma | In Western countries the prevalence of phaeochromocytoma can be estimated to lie between 1:6,500 to 1:2,500 with an annual incidence in the United States of 500 to 1,100 cases per year |
| Thyrotroph inoma | Very rare |

Generally the symptoms of these tumours vary depending on the tumour type as they each secrete different hormones causing different symptoms (table 2).

**Table 2 Symptoms or diseases caused by hypersecretion from neuroendocrine tumours**

| Tumour type | Pathophysiology and symptoms (caused by hypersecretion rather than tumour mass) |
|---|---|
| carcinoid tumours | A combination of symptoms that result from secretion of hormone or hormone-like substances (e.g. serotonin, gastrin, ACTH, histamine) that are produced by some carcinoid tumours. These symptoms include flushing, diarrhoea, cramp-like abdominal pain, swelling of skin or face and neck, wheezing, weight gain, increased body and facial hair, diabetes, headaches, oedema, lacrimation, weakness, pulmonary hypertension, symptoms of heart failure including shortness of breath |
| Insulinomas | Blurred vision, diplopia, weakness, palpitations, confusion and bizarre behaviour. Hypoglycaemia tends to occur 5 hours or so after a meal and the associated symptoms may be affected by diet, ingestion of ethanol and exercise |
| Gastrinomas | Diarrhoea, gastritis, recurrent gastric ulcers |
| VIPomas | Watery diarrhoea (3-20 litres per day), hypokalaemia, hypomagnesaemia, hypercalcaemia, acidosis, flushing, flaccid distended bladder, ileus/subileus. Diabetes or glucose intolerance are also common. |
| Glucagonomas | Necrolytic erythematous rash (often on the face, extremities and intertrigenous areas), anaemia, weight loss, impaired glucose tolerance, thrombosis and diarrhoea. |
| corticotrophinoma | Cushing’s disease resulting from ACTH inducing excess circulating cortisol |
| somatotrophinoma | Acromegaly |
| prolactinoma | oligomenorrhea/amenorrhea, galactorrhea, vaginal dryness, loss of libido in females; sexual dysfunction (impotence), galactorrhea and gynaecomastia in males |
| phaeochromocytoma | A wide range of symptoms resulting from metabolic and hemodynamic actions of circulating catecholamines. Sustained or paroxysmal hypertension is the most common clinical sign found in more than 90% of patients; with decreasing frequency:- headache, palpitations, pallor, nausea, flushing, weight loss, tiredness. Anxiety/panic, orthostatic hypotension, hyperglycaemia |
| Thyrotrophinoma | Thyrotoxicosis (overactivity of the thyroid gland), symptoms of which include weight loss in spite of increased appetite, rapid heart rate, a fine tremor, increased nervousness and emotional instability, intolerance of heat, and excessive sweating staring, bulging eyes, enlargement of the thyroid gland; in about a third of cases, the tumour also produces excess growth hormone resulting in mild acromegaly |

Current therapies are highly individualised as the symptoms experienced by each patient are often different and may also be changing over time. The three potential aims of treating a patient are (1) to remove the tumour, (2) to slow down or stop the growth of the tumour or (3) to ameliorate the symptoms caused by hypersecretion from the tumour - all three may be sought in combination. The most common current therapies are described below.

**Carcinoid tumours/carcinoid syndrome**

A 2-pronged approach is often used in the treatment of carcinoid syndrome, beginning with surgery to remove the tumour or reduce its size, followed by treatment with chemotherapy or interferons. A procedure known as hepatic embolisation may be used to control cancer that has spread from a carcinoid tumour into the liver; it helps reduce symptoms by decreasing blood supply to the liver and starving tumour cells.

A second approach involves treating symptoms with different medications: diuretics for heart disease, bronchodilators for wheezing, somatostatin analogues for wheezing, diarrhoea and flushing.

**Insulinomas**

The symptoms from insulinomas can sometimes be treated through diet regulation (e.g. by frequent, slow-release complex carbohydrate intake; guar gum). With malignant insulinoma, metastases may be found in the surrounding lymph nodes and liver. If the tumour cannot be localised before or during surgery (intra-operatively), it may be removed through distal pancreatectomy.

**Gastrinomas**

In patients with gastrinomas, antisecretory medication such as a proton pump inhibitor is used to control gastric acid hypersecretion. If a patient cannot take this medication, a total gastrectomy is recommended. Surgery has been shown to yield a 30% 5-year cure rate, and is recommended in patients without liver metastases, MEN 1, or complicating medical conditions that may limit life expectancy. (Ninety-five percent of patients with gastrinomas have tumours). Patients with metastatic disease may benefit from chemotherapy or octreotide, if chemotherapy fails.

**VIPomas**

First-line therapy for VIPomas aims to correct the profound hypokalaemia, dehydration and metabolic acidosis by replenishing fluids and electrolytes. Patients are typically given up to 5 L of fluid and 350 mEq of potassium daily. The optimal treatment for VIPomas is surgical removal of the primary tumour.

**Glucagonomas**

Surgery is used to relieve the effects of glucagonomas or to reduce the size of the tumours, though about two-third of patients are not cured by surgery even after successful tumour localisation and assessment of metastatic disease. Currently, active drugs used to treat glucagonoma do not exist

**Prolactinomas**

Medical treatment is usually with the dopamine agonists bromocriptine or cabergoline. These drugs shrink the tumour and return prolactin levels to normal in approximately 80 percent of patients. However, use of these agonists is associated with side effects such as nausea and dizziness. Surgery is an option where medical therapy cannot be tolerated or if it fails to reduce prolactin levels, restore normal reproduction and pituitary function, and reduce tumour size. However, the results of surgery depend a great deal on tumour size and prolactin level as well as the skill and experience of the neurosurgeon. Depending on the size of the tumour and how much of it is removed, studies show that 20 to 50 percent will recur, usually within five years

**Somatotrophinomas (e.g. causing acromegaly)**

Current treatment for patients with acromegaly include surgical, radiation, and medical therapies. Treatment depends on the size and extent of the tumour and the need for rapid cessation of hormone function that results in serious clinical sequelae. The standard treatments include surgery (usually a transsphenoidal approach) with or without postoperative radiation therapy, bromocriptine treatment, octreotide treatment and, more recently, pegvisomant treatment. The above-described therapies have variable success.

**Corticotrophinomas**

For patients with corticotroph adenomas, transsphenoidal microsurgery is the treatment of choice. However, remission rates reported in most series are approximately 70% to 90%. Drug therapy is considered to be an adjunct to transsphenoidal microsurgery in cases with a residual tumour and in cases in which one is awaiting the effects of the radiation therapy. Steroidogenesis inhibitors, including mitotane, metyrapone, ketoconazole, and aminoglutethimide are used. Ketoconazole is the best tolerated of these agents, though only in about 70% of patients. Radiation therapy has been used in patients who are deemed to be poor surgical candidates and has also been used as adjunctive therapy in patients with residual or recurrent active tumour.

**Phaeochromocytoma**

Laparoscopic tumour removal is the preferred procedure. However, complications during surgery need to be kept to a minimum by appropriate preoperative medical treatment to prevent catecholamine-induced, serious, and potentially life-threatening complications during surgery, including hypertensive crises, cardiac arrhythmias, pulmonary oedema, and cardiac ischaemia. Traditional regimens include $\alpha$-adrenoceptor blockers, combined $\alpha/\beta$-adrenoceptor blockers and, calcium-channel blockers, all of which can have undesired effects both before and after surgery.

**Thyrotroph inomas**

Transsphenoidal surgery is the treatment of choice for patients with thyrotrophic adenomas. Adjuvant radiation therapy may be employed when surgery is known to be non-curative even if the patient is still euthyroid because relapse is inevitable, and the full effect of radiation therapy requires months or years. Medical therapy may be required for patients who still have hyperthyroid symptoms despite surgery and external radiation.

As well as representing rare, but life-affecting, human conditions neuroendocrine tumours continue to pose a major problem for animal healthcare on a global scale. Accordingly, there is a need in the art for alternative and/ or improved therapeutics and therapies that address one or more of the above problems.

In all cases, surgery can be of limited success as well as carrying inherent risks to the patient. In addition, current drug treatments also are no guarantee of success in alleviating the symptoms in al patients.

The present invention solves one or more of the above problems or risks associated with surgery or existing medical therapies, by providing a new category of non-cytotoxic agent designed to suppress undesirable (e.g. abnormally elevated) tumour secretions and thus minimising or reversing the resultant disease.

In more detail, a first aspect of the present invention provides a polypeptide for use in suppressing secretion(s) from a neuroendocrine tumour, said polypeptide comprising:

a a non-cytotoxic protease, which protease is capable of cleaving a protein of the exocytic fusion apparatus in a neuroendocrine tumour cell;

b. a Targeting Moiety (TM) that is capable of binding to a Binding Site on a neuroendocrine tumour cell, which Binding Site is capable of undergoing endocytosis to be incorporated into an endosome within the neuroendocrine tumour cell; and

c. a translocation domain that is capable of translocating the protease from within an endosome, across the endosomal membrane and into the cytosol of the neuroendocrine tumour cell.

In use, a polypeptide of the invention binds to a neuroendocrine tumour cell. Thereafter, the translocation component effects transport of the protease component into the cytosol of the tumour cell. Finally, once inside, the protease inhibits the exocytic fusion process of the neuroendocrine tumour cell. Thus, by inactivating the exocytic fusion apparatus of the neuroendocrine tumour cell, the polypeptide of the invention inhibits secretion therefrom. Accordingly, the polypeptides of the present invention suppress/ treat one or more of the various pathophysiological conditions or symptoms listed in Table 2 above.

The principal target cells of the present invention are tumour cells of neuroendocrine origin that secrete one or more hormones (or other bioactive molecules) leading to the development of a pathophysiological condition.

The present invention provides polypeptides that are capable of (and for use in) suppression of the secretion of hormones and/or other bioactive molecules from neuroendocrine tumours.

In a related aspect of the present invention, there is provided a method for treating a neuroendocrine tumour in a patient, said method comprising administering to the patient a therapeutically effective amount of a polypeptide of the present invention.

Without wishing to be bound by any theory, the present inventors believe that undesirable (e.g. unusual levels of) secretion of physiologically active molecules from neuroendocrine tumours cause and maintain pathological conditions in a patient. Thus, by inhibiting said secretions, the progression of the disease state can be halted and the symptoms reversed.

The polypeptides of the present invention are particularly suited for use in treating a range of neuroendocrine tumours, including their hormone-secreting metastases, precancerous conditions and symptoms thereof. In this regard, 'treating' includes reducing or eliminating excessive secretions from such cells.

By way of example, important neuroendocrine tumour target cells of the present invention include: pituitary adenomas and/ or gastroenteropancreatic neuroendocrine tumours (GEP-NETs). GEP-NETs are located mainly in the stomach, intestine or pancreas and secrete excessive amounts of hormones and other bioactive molecules that are normally secreted at lower levels under physiological regulation. These secretions contribute to the symptoms experienced by the patients. GEP-NETs can be divided into carcinoid and non-carcinoid subtypes.

Carcinoid GEP-NETs (55% of all GEP-NETs) tend to be classified according to their tissue location and include, in order of prevalence, those arising from cells in the appendix (38%), ileum (23%), rectum (13%) and bronchus (11.5%).

Non-carcinoid GEP-NETs include insulinomas of the pancreatic islets secreting excess insulin (17%), tumours of unknown type (15%), gastrinomas of the pancreas or duodenum secreting excess gastrin (9%), VIPomas of the pancreas, lung or ganglioneuromas, secreting excess vasoactive intestinal polypeptide, and glucagonomas, tumours of the pancreatic islets secreting excess glucagon.

The pituitary tumours, which tend to be classified according to their secretion type or cellular identity, include: prolactinomas secreting prolactin (the most common), somatotrophinomas (growth hormone, corticotrophinomas (adrenocorticotrophic hormone), thyrotrophinomas (thyroid stimulating hormone), gonadotrophinomas (FSH, LH), and non-functioning pituitary adenomas.

Other secretory tumours include thyroid medullary tumours, small and non-small cell lung tumours, Merkel cell tumours, and phaeochromocytomas. The latter can be deadly if excessive secreted adrenaline leads to severe hypertension. Such hypersecretion can make the individual unsuitable for surgery to remove tumour mass and so a reinforcing deleterious cycle can emerge and treatment of the tumour to minimise secretion is desirable.

A particularly preferred sub-set of neuroendocrine tumour cells addressed by the present invention is: insulinomas, gastrinomas, VIPomas, glucagonomas, prolactinomas, somatotrophinomas, corticotrophinomas, thyrotrophinomas and phaeochromocytomas.

By suppressing the secretory functions of neuroendocrine tumour cells (such as the above sub-set of tumour cells), the present invention provides a therapy for the treatment of, amongst others, conditions such as Cushing's disease, acromegaly, carcinoid syndrome, hypoglycaemic syndrome, necrolytic migratory erythema, Zollinger-Ellison syndrome and Verner-Morrison syndrome. Also provided are therapies for treatment of the symptoms ensuing from undesirable neuroendocrine tumour secretions (see Table 2).

The 'bioactive' component of the polypeptides of the present invention is provided by a non-cytotoxic protease. This distinct group of proteases act by proteolytically-cleaving intracellular transport proteins known as SNARE proteins (e.g. SNAP-25, VAMP, or Syntaxin) - see Gerald K (2002) "Cell and Molecular Biology" (4th edition) John Wiley & Sons, Inc. The acronym SNARE derives from the term **S**oluble **N**SF **A**ttachment **R**eceptor, where NSF means **N**-ethylmaleimide-**S**ensitive **F**actor. SNARE proteins are integral to intracellular vesicle formation, and thus to secretion of molecules via vesicle transport from a cell. Accordingly, once delivered to a desired target cell, the non-cytotoxic protease is capable of inhibiting cellular secretion from the target cell.

Non-cytotoxic proteases are a discrete class of molecules that do not kill cells; instead, they act by inhibiting cellular processes other than protein synthesis. Non-cytotoxic proteases are produced as part of a larger toxin molecule by a variety of plants, and by a variety of microorganisms such as Clostridium sp. and Neisseria sp.

Clostridial neurotoxins represent a major group of non-cytotoxic toxin molecules, and comprise two polypeptide chains joined together by a disulphide bond. The two chains are termed the heavy chain (H-chain), which has a molecular mass of approximately 100 kDa, and the light chain (L-chain), which has a molecular mass of approximately 50 kDa. It is the L-chain, which possesses a protease function and exhibits a high substrate specificity for vesicle and/or plasma membrane associated (SNARE) proteins involved in the exocytic process (eg. synaptobrevin, syntaxin or SNAP-25). These substrates are important components of the neurosecretory machinery.

Neisseria sp., most importantly from the species *N. gonorrhoeae,* and Streptococcus sp., most importantly from the species *S. pneumoniae,* produce functionally similar non-cytotoxic toxin molecules. An example of such a non-cytotoxic protease is IgA protease (see WO99/58571, which is hereby incorporated in its entirety by reference thereto). Thus, the non-cytotoxic protease of the present invention is preferably a clostridial neurotoxin protease or an IgA protease.

Turning now to the Targeting Moiety (TM) component of the present invention, it is this component that binds the polypeptide of the present invention to a neuroendocrine tumour cell.

Thus, a TM of the present invention binds to a receptor on a neuroendocrine tumour cell. By way of example, a TM of the present invention may bind to a receptor selected from the group comprising: a somatostatin (sst) receptor, including splice variants thereof (e.g. $sst_1$, $sst_2$, $sst_3$, $sst_4$ and $sst_5$); a growth hormone-releasing hormone (GHRH) receptor - also known a GRF receptor; a ghrelin receptor; a bombesin receptor (eg. BRS-1, BRS-2, or BRS-3); a urotensin receptor (eg. a urotensin II receptor); a melanin-concentrating hormone receptor 1; a prolactin releasing hormone receptor; a gonadotropin-releasing hormone receptor (GnRHR) such as a Type 1 GnRHR and/ or a Type 2 GnRHR receptor; and/ or a KiSS-1 receptor.

In one embodiment, a TM of the present invention binds to a somatostatin (SST) receptor. Examples of suitable SST peptide TMs include full-length SST and cortistatin (CST), as well as truncations and peptide analogues thereof such as: SANSNPAMAPRERKAGCKNFFWKTFTSC (SST-28); AGCKNFFWKTFTSC (SST-14); QEGAPPQQSARRDRMPCRNFFWKTFSSCK (CST-29); QERPPLQQPPHRDKKPCKNFFWKTFSSCK (CST-29); QERPPPQQPPHLDKKPCKNFFWKTFSSCK (CST-29); DRMPCRNFFWKTFSSCK (CST-17); PCRNFFWKTFSSCK (CST-14); and PCKNFFWKTFSSCK (CST-14); D-Phe-Phe-Phe-D-Trp-Lys-Thr-Phe-Thr-NH2 (BIM 23052), D-Phe-Phe-Tyr-D-Trp-Lys-Val-Phe-D-Nal-NH2 (BIM 23056) or c[Cys-Phe-Phe-D-Trp-Lys-Thr-Phe-Cys]-$NH_2$ (BIM23268); octreotide peptides, lanreotide peptides, BIM23027, CYN154806, BIM23027, vapreotide peptides, seglitide peptides, and SOM230. These TMs bind to sst receptors, such as $sst_1$, $sst_2$, $sst_3$, $sst_4$ and $sst_5$ receptors, which are present on neuroendocrine tumour cells relevant to the present invention - see Table 3. SST and CST have high structural homology, and bind to all known sst receptors.

**Table 3**

**Expression of somatostatin receptor subtypes in gastroenteropancreatic neuroendocrine tumours (%)**

| | sst1 | sst2 | sst3 | sst4 | sst5 |
|---|---|---|---|---|---|
| All tumours | 68 | 86 | 46 | 93 | 57 |
| Insulinoma | 33 | 100 | 33 | 100 | 67 |
| Gastrinoma | 33 | 50 | 17 | 83 | 50 |
| Glucagonoma | 67 | 100 | 67 | 67 | 67 |
| VIPoma | 100 | 100 | 100 | 100 | 100 |
| Non-functioning | 80 | 100 | 40 | 100 | 60 |
| mid-gut NETs | 80 | 95 | 65 | 35 | 75 |

In another embodiment, a TM of the present invention binds to a growth hormone releasing hormone (GHRH) receptor. GHRH is also known as growth-hormone-releasing factor (GRF or GHRF) or somatocrinin. Suitable GHRH peptides include full-length GHRH (1-44) peptide, and truncations thereof such as GHRH(1-27, 1-28, 1-29), GHRH(1-37), and GHRH(1-40, 1-43)-OH, as well as peptide analogues such as: BIM 28011 or NC-9-96; [MeTyr1,Ala15,22,Nle27]-hGHRH(1-29)-NH2; MeTyr1,Ala8,9,15,22,28,Nle27]-hGHRH(1-29)-NH2; cyclo(25-29)[MeTyr1,Ala15,DAsp25,Nle27,Orn29+ ++]-hGHRH(1-29)-NH2; (D-Tyr1)-GHRH (1-29)-NH2; (D-Ala2)-GHRH (1-29)-NH2; (D-Asp3)-GHRH (1-29)-NH2; (D-Ala4)-GHRH (1-29)-NH2; (D-Thr7)-GHRH (1-29)-NH2; (D-Asn8)-GHRH (1-29)-NH2; (D-Ser9)-GHRH (1-29)-NH2; (D-Tyr10)-GHRH (1-29)-NH2; (Phe4)-GHRH (1-29)-NH2; (pCl-Phe6)-GHRH (1-29)-NH2; (N-Ac-Tyr1)-GHRH (1-29)-NH2; (N-Ac-Tyr1, D-Ala2)-GHRH (1-29)-NH2; (N-Ac-D-Tyr1, D-Ala2)-GHRH (1-29)-NH2; (N-Ac-D-Tyr1, D-Ala 2, D-Asp3)-GHRH (1-29)-NH2; (D-Ala2, NLeu27)-GHRH (1-29)-NH2; (His1, D-Ala2, NLeu27)-GHRH (1-29)-NH2; (N-Ac-His1, D-Ala2, N-Leu27)-GHRH (1-29)-NH2; (His1, D-Ala 2, D-Ala 4, Nleu27)-GHRH (1-29)-NH2; (D-Ala2, D-Asp3, D-Asn8, NLeu27)-GHRH (1-29)-NH2; (D-Asp3, D-Asn8, NLeu27)-GHRH (1-29)-NH2; [His1, NLeu27]-hGHRH(1-29)-NH2; [NLeu27]-hGHRH(1-29)-NH2; H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-Arg-Gly-Ala-Arg-Ala-Arg-Leu-NH2; H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH2; H-Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH2; H-Tyr-Ala-Asp-Ala-Ile-Phe-Thr-Asn-Ser-Tyr-Arg-Lys-Ile-Leu-Gly-Gln-Leu-Ser-Ala-Arg-Lys-Leu-Leu-Gln-Asp-Ile-Met-Asn-Arg-Gln-Gln-Gly-Glu-Arg-Asn-Gln-Glu-Gln-Gly-Ala-Lys-Val-Arg-Leu-NH2; H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Asn-Arg-Gln-Gln-Gly-Glu-Arg-Asn-Gln-Glu-Gln-Gly-Ala-Lys-Val-Arg-Leu-NH2; His-Val-Asp-Ala-Ile-Phe-Thr-Gln-Ser-Tyr-Arg-Lys-Val-Leu-Ala-Gln-Leu-Ser-Ala-Arg-Lys-Leu-Leu-Gln-Asp-Ile-Leu-Asn-Arg; His-Val-Asp-Ala-Ile-Phe-Thr-Gln-Ser-Tyr-Arg-Lys-Val-Leu-Ala-Gln-Leu-Ser-Ala-Arg-Lys-Leu-Leu-Gln-Asp-Ile-Leu-Asn-Arg-Gln-Gln-Gly-Glu-Arg-Asn-Gln-Glu-Gln-Gly-Ala.

In another embodiment, a TM of the present invention binds to a ghrelin receptor. Examples of suitable TMs in this regard include: ghrelin peptides such as full-length ghrelin (eg. $ghrelin_{117}$) and truncations and peptide analogues thereof such as $ghrelin_{24-117}$, $ghrelin_{52-117}$, [Trp3, Arg5]-ghrelin (1-5), des-Gln-Ghrelin, cortistatin-8, His-D-Trp-Ala-Trp-D-Phe-Lys-$NH_2$, growth hormone releasing peptide (e.g. GHRP-6), or hexarelin.

In a further embodiment, the TM binds to a bombesin receptor (eg. BRS-1, BRS-2, or BRS-3). Examples of suitable bombesin peptides include full-length: bombesin - a 14 amino acid peptide originally isolated from the skin of a frog (pGlu-Gln-Arg-Leu-Gly-Asn-Gln-Trp-Ala-Val-Gly-His-Leu-Met-$NH_2$); and the two known homologs in mammals, namely neuromedin B, and gastrin releasing peptide (GRP) such as: porcine GRP - Ala-Pro-Val-Ser-Val-Gly-Gly-Gly-Thr-Val-Leu-Ala-Lys-Met-Tyr-Pro-Arg-Gly-Asn-His-Trp-Ala-Val-Gly-His-Leu-Met-$NH_2$, and human GRP - Val-Pro-Leu-Pro-Ala-Gly-Gly-Gly-Thr-Val-Leu-Thr-Lys-Met-Tyr-Pro-Arg-Gly-Asn-His-Trp-Ala-Val-Gly-His-Leu-Met-$NH_2$. Reference to bombesin peptides embraces homologs thereof such as neuromedin B and GRP, and includes truncations and peptide analogues thereof.

In another embodiment, a TM of the present invention binds to a urotensin receptor. Suitable TMs in this regard include urotensin peptides such as Urotensin-II (U-II), which is a cyclic neuropeptide. The C-terminal cyclic region of U-II is strongly conserved across different species, and includes the six amino acid residues (-Cys Ple-Trp-Lys-Tyr-Cys-), which is structurally similar to the central region of somatostatin-14 (-Phe-Trp-Lys-Thr-). Urotensin peptides of the present invention include the U-II precursor peptides, such as prepro-urotensin-II (including the two human 124 and 139 isoforms thereof) as well as other truncations such as the eleven residue mature peptide form and peptide analogues thereof.

In a further embodiment, a TM of the present invention binds to a melanin-concentrating hormone receptor 1. Examples of suitable TMs in this regard include: melanin-concentrating hormone (MCH) peptides such as full-length MCH, truncations and analogues thereof.

In another embodiment, a TM of the present invention binds to a prolactin releasing hormone receptor. An example of a suitable TM in this regard includes prolactin releasing peptide, truncations and analogues thereof.

In another embodiment, a TM of the present invention binds to a gonadotropin-releasing hormone (GnRH) receptor. GnRH is also known as Luteinizing-Hormone Releasing Hormone (LHRH). Examples of suitable GnRH receptor TMs include: GnRHI peptides, GnRHII peptides and GnRHIII peptides, for example the full-length 92 amino acid GnRH precursor polypeptide and truncations thereof such as the decapeptide: pyroGlu-His-Trp-Ser-Tyr-Gly-Leu-Arg-Pro-Gly CONH2.

In a further embodiment, a TM of the present invention binds to a KiSS-1 receptor. Examples of suitable TMs in this regard include Kisspeptin-10, Kisspeptin-54 peptides, truncations and analogues thereof.

According to a second aspect of the present invention, there is provided a composition of matter, namely a polypeptide comprising:

a a non-cytotoxic protease, which protease is capable of cleaving a protein of the exocytic fusion apparatus in a neuroendocrine tumour cell;

b. a Targeting Moiety (TM) that is capable of binding to a Binding Site on a neuroendocrine tumour cell, which Binding Site is capable of undergoing endocytosis to be incorporated into an endosome within the neuroendocrine tumour cell; and

d. a translocation domain that is capable of translocating the protease from within an endosome, across the endosomal membrane and into the cytosol of the neuroendocrine tumour cell.

All of the features of the first aspect of the present invention apply equally to the above-described second aspect.

In a preferred embodiment of the first and/ or second aspects of the present invention, the TM has a human peptide amino acid sequence. Thus, a highly preferred TM is a human SST peptide, a human CST peptide or a human GHRH peptide.

**Polypeptide preparation**

The polypeptides of the present invention comprise 3 principal components: a 'bioactive' (ie. a non-cytotoxic protease); a TM; and a translocation domain. The general technology associated with the preparation of such fusion proteins is often referred to as re-targeted toxin technology. By way of exemplification, we refer to: WO94/21300; WO96/33273; WO98/07864; WO00/10598; WO01/21213; WO06/059093; WO00/62814; WO00/04926; WO93/15766; WO00/61192; and WO99/58571. All of these publications are herein incorporated by reference thereto.

In more detail, the TM component of the present invention may be fused to either the protease component or the translocation component of the present invention. Said fusion is preferably by way of a covalent bond, for example either a direct covalent bond or via a spacer/ linker molecule. The protease component and the translocation component are preferably linked together via a covalent bond, for example either a direct covalent bond or via a spacer/ linker molecule. Suitable spacer/ linked molecules are well known in the art, and typically comprise an amino acid-based sequence of between 5 and 40, preferably between 10 and 30 amino acid residues in length.

In use, the polypeptides have a di-chain conformation, wherein the protease component and the translocation component are linked together, preferably via a disulphide bond.

The polypeptides of the present invention may be prepared by conventional chemical conjugation techniques, which are well known to a skilled person. By way of example, reference is made to Hermanson, G.T. (1996), Bioconjugate techniques, Academic Press, and to Wong, S.S. (1991), Chemistry of protein conjugation and cross-linking, CRC Press, Nagy et al., PNAS 95 p1794-99 (1998). Further detailed methodologies for attaching synthetic TMs to a polypeptide of the present invention are provided in, for example, EP0257742. The above-mentioned conjugation publications are herein incorporated by reference thereto.

Alternatively, the polypeptides may be prepared by recombinant preparation of a single polypeptide fusion protein (see, for example, WO98/07864). This technique is based on the *in vivo* bacterial mechanism by which native clostridial neurotoxin (i.e. holotoxin) is prepared, and results in a fusion protein having the following 'simplified' structural arrangement:

$NH_2$ - [protease component] - [translocation component] - [TM] - COOH

According to WO98/07864, the TM is placed towards the C-terminal end of the fusion protein. The fusion protein is then activated by treatment with a protease, which cleaves at a site between the protease component and the translocation component. A di-chain protein is thus produced, comprising the protease component as a single polypeptide chain covalently attached (via a disulphide bridge) to another single polypeptide chain containing the translocation component plus TM.

Alternatively, according to WO06/059093, the TM component of the fusion protein is located towards the middle of the linear fusion protein sequence, between the protease cleavage site and the translocation component. This ensures that the TM is attached to the translocation domain (ie. as occurs with native clostridial holotoxin), though in this case the two components are reversed in order *vis-à-vis* native holotoxin. Subsequent cleavage at the protease cleavage site exposes the N-terminal portion of the TM, and provides the di-chain polypeptide fusion protein.

The above-mentioned protease cleavage sequence(s) may be introduced (and/ or any inherent cleavage sequence removed) at the DNA level by conventional means, such as by site-directed mutagenesis. Screening to confirm the presence of cleavage sequences may be performed manually or with the assistance of computer software (e.g. the MapDraw program by DNASTAR, Inc.). Whilst any protease cleavage site may be employed (ie. clostridial, or non-clostridial), the following are preferred:

| | |
|---|---|
| Enterokinase | (DDDDK↓) |
| Factor Xa | (IEGR↓ / IDGR↓) |
| TEV(Tobacco Etch virus) | (ENLYFQ↓G) |
| Thrombin | (LVPR↓GS) |
| PreScission | (LEVLFQ↓GP). |

Additional protease cleavage sites include recognition sequences that are cleaved by a non-cytotoxic protease, for example by a clostridial neurotoxin. These include the SNARE (eg. SNAP-25, syntaxin, VAMP) protein recognition sequences that are cleaved by non-cytotoxic proteases such as clostridial neurotoxins. Particular examples are provided in US2007/0166332, which is hereby incorporated in its entirety by reference thereto.

Also embraced by the term protease cleavage site is an intein, which is a self-cleaving sequence. The self-splicing reaction is controllable, for example by varying the concentration of reducing agent present. The above-mentioned 'activation' cleavage sites may also be employed as a 'destructive' cleavage site (discussed below) should one be incorporated into a polypeptide of the present invention.

In a preferred embodiment, the fusion protein of the present invention may comprise one or more N-terminal and/ or C-terminal located purification tags. Whilst any purification tag may be employed, the following are preferred:

His-tag (e.g. 6 × histidine), preferably as a C-terminal and/ or N-terminal tag
MBP-tag (maltose binding protein), preferably as an N-terminal tag
GST-tag (glutathione-S-transferase), preferably as an N-terminal tag
His-MBP-tag, preferably as an N-terminal tag
GST-MBP-tag, preferably as an N-terminal tag
Thioredoxin-tag, preferably as an N-terminal tag
CBD-tag (Chitin Binding Domain), preferably as an N-terminal tag.

One or more peptide spacer/ linker molecules may be included in the fusion protein. For example, a peptide spacer may be employed between a purification tag and the rest of the fusion protein molecule.

Thus, a third aspect of the present invention provides a nucleic acid (e.g. DNA) sequence encoding a polypeptide as described above (i.e. the second aspect of the present invention).

Said nucleic acid may be included in the form of a vector, such as a plasmid, which may optionally include one or more of an origin of replication, a nucleic acid integration site, a promoter, a terminator, and a ribosome binding site.

The present invention also includes a method for expressing the above-described nucleic acid sequence (i.e. the third aspect of the present invention) in a host cell, in particular in *E. coli* or via a baculovirus expression system.

The present invention also includes a method for activating a polypeptide of the present invention, said method comprising contacting the polypeptide with a protease that cleaves the polypeptide at a recognition site (cleavage site) located between the non-cytotoxic protease component and the translocation component, thereby converting the polypeptide into a di-chain polypeptide wherein the non-cytotoxic protease and translocation components are joined together by a disulphide bond. In a preferred embodiment, the recognition site is not native to a naturally-occurring clostridial neurotoxin and/ or to a naturally-occurring igA protease.

The polypeptides of the present invention may be further modified to reduce or prevent unwanted side-effects associated with dispersal into non-targeted areas. According to this embodiment, the polypeptide comprises a destructive cleavage site. The destructive cleavage site is distinct from the 'activation' site (i.e. di-chain formation), and is cleavable by a second protease and not by the non-cytotoxic protease. Moreover, when so cleaved at the destructive cleavage site by the second protease, the polypeptide has reduced potency (e.g. reduced binding ability to the intended target cell, reduced translocation activity and/ or reduced non-cytotoxic protease activity). For completeness, any of the 'destructive' cleavage sites of the present invention may be separately employed as an 'activation' site in a polypeptide of the present invention.

Thus, according to this embodiment, the present invention provides a polypeptide that can be controllably inactivated and/ or destroyed at an off-site location.

In a preferred embodiment, the destructive cleavage site is recognised and cleaved by a second protease (i.e. a destructive protease) selected from a circulating protease (e.g. an extracellular protease, such as a serum protease or a protease of the blood clotting cascade), a tissue-associated protease (e.g. a matrix metalloprotease (MMP), such as an MMP of muscle), and an intracellular protease (preferably a protease that is absent from the target cell).

Thus, in use, should a polypeptide of the present invention become dispersed away from its intended target cell and/ or be taken up by a non-target cell, the polypeptide will become inactivated by cleavage of the destructive cleavage site (by the second protease).

In one embodiment, the destructive cleavage site is recognised and cleaved by a second protease that is present within an off-site cell-type. In this embodiment, the off-site cell and the target cell are preferably different cell types. Alternatively (or in addition), the destructive cleavage site is recognised and cleaved by a second protease that is present at an off-site location (e.g. distal to the target cell). Accordingly, when destructive cleavage occurs extracellularly, the target cell and the off-site cell may be either the same or different cell-types. In this regard, the target cell and the off-site cell may each possess a receptor to which the same polypeptide of the invention binds.

The destructive cleavage site of the present invention provides for inactivation/ destruction of the polypeptide when the polypeptide is in or at an off-site location. In this regard, cleavage at the destructive cleavage site minimises the potency of the polypeptide (when compared with an identical polypeptide lacking the same destructive cleavage site, or possessing the same destructive site but in an uncleaved form). By way of example, reduced potency includes: reduced binding (to a mammalian cell receptor) and/ or reduced translocation (across the endosomal membrane of a mammalian cell in the direction of the cytosol), and/ or reduced SNARE protein cleavage.

When selecting destructive cleavage site(s) in the context of the present invention, it is preferred that the destructive cleavage site(s) are not substrates for any proteases that may be separately used for post-translational modification of the polypeptide of the present invention as part of its manufacturing process. In this regard, the non-cytotoxic proteases of the present invention typically employ a protease activation event (via a separate 'activation' protease cleavage site, which is structurally distinct from the destructive cleavage site of the present invention). The purpose of the activation cleavage site is to cleave a peptide bond between the non-cytotoxic protease and the translocation or the binding components of the polypeptide of the present invention, thereby providing an 'activated' di-chain polypeptide wherein said two components are linked together via a di-sulfide bond.

Thus, to help ensure that the destructive cleavage site(s) of the polypeptides of the present invention do not adversely affect the 'activation' cleavage site and subsequent di-sulfide bond formation, the former are preferably introduced into polypeptide of the present invention at a position of at least 20, at least 30, at least 40, at least 50, and more preferably at least 60, at least 70, at least 80 (contiguous) amino acid residues away from the 'activation' cleavage site.

The destructive cleavage site(s) and the activation cleavage site are preferably exogenous (i.e. engineered/ artificial) with regard to the native components of the polypeptide. In other words, said cleavage sites are preferably not inherent to the corresponding native components of the polypeptide. By way of example, a protease or translocation component based on BoNT/A L-chain or H-chain (respectively) may be engineered according to the present invention to include a cleavage site. Said cleavage site would not, however, be present in the corresponding BoNT native L-chain or H-chain. Similarly, when the Targeting Moiety component of the polypeptide is engineered to include a protease cleavage site, said cleavage site would not be present in the corresponding native sequence of the corresponding Targeting Moiety.

In a preferred embodiment of the present invention, the destructive cleavage site(s) and the 'activation' cleavage site are not cleaved by the same protease. In one embodiment, the two cleavage sites differ from one another in that at least one, more preferably at least two, particularly preferably at least three, and most preferably at least four of the tolerated amino acids within the respective recognition sequences is/ are different.

By way of example, in the case of a polypeptide chimera containing a Factor Xa 'activation' site between clostridial L-chain and $H_N$ components, it is preferred to employ a destructive cleavage site that is a site other than a Factor Xa site, which may be inserted elsewhere in the L-chain and/ or $H_N$ and/ or TM component(s). In this scenario, the polypeptide may be modified to accommodate an alternative 'activation' site between the L-chain and $H_N$ components (for example, an enterokinase cleavage site), in which case a separate Factor Xa cleavage site may be incorporated elsewhere into the polypeptide as the destructive cleavage site. Alternatively, the existing Factor Xa 'activation' site between the L-chain and $H_N$ components may be retained, and an alternative cleavage site such as a thrombin cleavage site incorporated as the destructive cleavage site.

When identifying suitable sites within the primary sequence of any of the components of the present invention for inclusion of cleavage site(s), it is preferable to select a primary sequence that closely matches with the proposed cleavage site that is to be inserted. By doing so, minimal structural changes are introduced into the polypeptide. By way of example, cleavage sites typically comprise at least 3 contiguous amino acid residues. Thus, in a preferred embodiment, a cleavage site is selected that already possesses (in the correct position(s)) at least one, preferably at least two of the amino acid residues that are required in order to introduce the new cleavage site. By way of example, in one embodiment, the Caspase 3 cleavage site (DMQD) may be introduced. In this regard, a preferred insertion position is identified that already includes a primary sequence selected from, for example, Dxxx, xMxx, xxQx, xxxD, DMxx, DxQx, DxxD, xMQx, xMxD, xxQD, DMQx, xMQD, DxQD, and DMxD.

Similarly, it is preferred to introduce the cleavage sites into surface exposed regions. Within surface exposed regions, existing loop regions are preferred.

In a preferred embodiment of the present invention, the destructive cleavage site(s) are introduced at one or more of the following position(s), which are based on the primary amino acid sequence of BoNT/A. Whilst the insertion positions are identified (for convenience) by reference to BoNT/A, the primary amino acid sequences of alternative protease domains and/ or translocation domains may be readily aligned with said BoNT/A positions.

For the protease component, one or more of the following positions is preferred: 27-31, 56-63, 73-75, 78-81, 99-105, 120-124, 137-144, 161-165, 169-173, 187-194, 202-214, 237-241, 243-250, 300-304, 323-335, 375-382, 391-400, and 413-423. The above numbering preferably starts from the N-terminus of the protease component of the present invention.

In a preferred embodiment, the destructive cleavage site(s) are located at a position greater than 8 amino acid residues, preferably greater than 10 amino acid residues, more preferably greater than 25 amino acid residues, particularly preferably greater than 50 amino acid residues from the N-terminus of the protease component. Similarly, in a preferred embodiment, the destructive cleavage site(s) are located at a position greater than 20 amino acid residues, preferably greater than 30 amino acid residues, more preferably greater than 40 amino acid residues, particularly preferably greater than 50 amino acid residues from the C-terminus of the protease component.

For the translocation component, one or more of the following positions is preferred: 474-479, 483-495, 507-543, 557-567, 576-580, 618-631, 643-650, 669-677, 751-767, 823-834, 845-859. The above numbering preferably acknowledges a starting position of 449 for the N-terminus of the translocation domain component of the present invention, and an ending position of 871 for the C-terminus of the translocation domain component.

In a preferred embodiment, the destructive cleavage site(s) are located at a position greater than 10 amino acid residues, preferably greater than 25 amino acid residues, more preferably greater than 40 amino acid residues, particularly preferably greater than 50 amino acid residues from the N-terminus of the translocation component. Similarly, in a preferred embodiment, the destructive cleavage site(s) are located at a position greater than 10 amino acid residues, preferably greater than 25 amino acid residues, more preferably greater than 40 amino acid residues, particularly preferably greater than 50 amino acid residues from the C-terminus of the translocation component.

In a preferred embodiment, the destructive cleavage site(s) are located at a position greater than 10 amino acid residues, preferably greater than 25 amino acid residues, more preferably greater than 40 amino acid residues, particularly preferably greater than 50 amino acid residues from the N-terminus of the TM component. Similarly, in a preferred embodiment, the destructive cleavage site(s) are located at a position greater than 10 amino acid residues, preferably greater than 25 amino acid residues, more preferably greater than 40 amino acid residues, particularly preferably greater than 50 amino acid residues from the C-terminus of the TM component.

The polypeptide of the present invention may include one or more (e.g. two, three, four, five or more) destructive protease cleavage sites. Where more than one destructive cleavage site is included, each cleavage site may be the same or different. In this regard, use of more than one destructive cleavage site provides improved off-site inactivation. Similarly, use of two or more different destructive cleavage sites provides additional design flexibility.

The destructive cleavage site(s) may be engineered into any of the following component(s) of the polypeptide:

the non-cytotoxic protease component; the translocation component; the Targeting Moiety; or the spacer peptide (if present). In this regard, the destructive cleavage site(s) are chosen to ensure minimal adverse effect on the potency of the polypeptide (for example by having minimal effect on the targeting/ binding regions and/ or translocation domain, and/ or on the non-cytotoxic protease domain) whilst ensuring that the polypeptide is labile away from its target site/ target cell.

Preferred destructive cleavage sites (plus the corresponding second proteases) are listed in the Table immediately below. The listed cleavage sites are purely illustrative and are not intended to be limiting to the present invention.

| **Second protease** | **Destructive cleavage site recognition sequence** | **Tolerated recognition sequence variance P4-P3-P2-P1-▼-P1'-P2'-P3'** | | | | | | |
|---|---|---|---|---|---|---|---|---|
| | | **P4** | **P3** | **P2** | **P1** | **P1'** | **P2'** | **P 3'** |
| Thrombin | LVPR▼GS | A,F,G, I, L,T, V or M | A,F,G, I, L,T, V, W or A | P | R | Not D or E | Not D or E | --- |
| Thrombin | GR▼G | | | G | R | G | | |
| Factor Xa | IEGR▼ | A,F,G, I, L,T, V or M | D or E | G | R | --- | --- | --- |
| ADAM 17 | PLAQA▼VRSSS | | | | | | | |
| Human airway trypsin-like protease (HAT) | SKGR▼SLIGRV | | | | | | | |
| ACE (peptidyl-dipeptidase A) | | --- | --- | --- | --- | Not P | Not D or E | N /A |
| Elastase (leukocyte) | MEA▼VTY | M, R | E | A, H | V, T | V, T, H | Y | --- |
| Furin | RXR/KR▼ | R | X | R or K | R | | | |
| Granzyme | IEPD▼ | I | E | P | D | --- | --- | -- |
| Caspase 1 | | F,W,Y, L | --- | H, A,T | D | Not P, E.D. Q.K or R | --- | --- |
| Caspase 2 | DVAD▼ | D | V | A | D | Not P, E.D. Q.K or R | --- | --- |
| Caspase 3 | DMQD▼ | D | M | Q | D | Not P, E.D. Q.K or R | --- | --- |
| Caspase 4 | LEVD▼ | L | E | V | D | Not P, E.D. Q.K or R | --- | --- |
| Caspase 5 | | L or W | E | H | D | --- | --- | -- |
| Caspase 6 | | V | E | H or I | D | Not P, E.D. Q.K or R | --- | --- |
| Caspase 7 | DEVD▼ | D | E | V | D | Not P, E.D. Q.K or R | --- | --- |

(continued)

| Second protease | Destructive cleavage site recognition sequence | Tolerated recognition sequence variance P4-P3-P2-P1-▼-P1'-P2'-P3' | | | | | | |
|---|---|---|---|---|---|---|---|---|
| | | P4 | P3 | P2 | P1 | P1' | P2' | P3' |
| Caspase 8 | | I or L | E | T | D | Not P, E.D. Q.K or R | --- | --- |
| Caspase 9 | LEHD▼ | L | E | H | D | --- | --- | --- |
| Caspase 10 | IEHD▼ | I | E | H | D | --- | --- | --- |

Matrix metalloproteases (MMPs) are a preferred group of destructive proteases in the context of the present invention. Within this group, ADAM17 (EC 3.4.24.86, also known as TACE), is preferred and cleaves a variety of membrane-anchored, cell-surface proteins to "shed" the extracellular domains. Additional, preferred MMPs include adamalysins, serralysins, and astacins.

Another group of preferred destructive proteases is a mammalian blood protease, such as Thrombin, Coagulation Factor VIIa, Coagulation Factor IXa, Coagulation Factor Xa, Coagulation Factor XIa, Coagulation Factor XIIa, Kallikrein, Protein C, and MBP-associated serine protease.

In one embodiment of the present invention, said destructive cleavage site comprises a recognition sequence having at least 3 or 4, preferably 5 or 6, more preferably 6 or 7, and particularly preferably at least 8 contiguous amino acid residues. In this regard, the longer (in terms of contiguous amino acid residues) the recognition sequence, the less likely non-specific cleavage of the destructive site will occur via an unintended second protease.

It is preferred that the destructive cleavage site of the present invention is introduced into the protease component and/ or the Targeting Moiety and/ or into the translocation component and/ or into the spacer peptide. Of these four components, the protease component is preferred. Accordingly, the polypeptide may be rapidly inactivated by direct destruction of the non-cytotoxic protease and/ or binding and/ or translocation components.

**Polypeptide delivery**

In use, the present invention employs a pharmaceutical composition, comprising a polypeptide, together with at least one component selected from a pharmaceutically acceptable carrier, excipient, adjuvant, propellant and/ or salt. The polypeptides of the present invention may be formulated for oral, parenteral, continuous infusion, implant, inhalation or topical application. Compositions suitable for injection may be in the form of solutions, suspensions or emulsions, or dry powders which are dissolved or suspended in a suitable vehicle prior to use.

Local delivery means may include an aerosol, or other spray (eg. a nebuliser). In this regard, an aerosol formulation of a polypeptide enables delivery to the lungs and/or other nasal and/or bronchial or airway passages.

The preferred route of administration is selected from: systemic (eg. iv), laparoscopic and/ or localised injection (for example, transsphenoidal injection directly into the tumour).

In the case of formulations for injection, it is optional to include a pharmaceutically active substance to assist retention at or reduce removal of the polypeptide from the site of administration. One example of such a pharmaceutically active substance is a vasoconstrictor such as adrenaline. Such a formulation confers the advantage of increasing the residence time of polypeptide following administration and thus increasing and/or enhancing its effect.

The dosage ranges for administration of the polypeptides of the present invention are those to produce the desired therapeutic effect. It will be appreciated that the dosage range required depends on the precise nature of the polypeptide or composition, the route of administration, the nature of the formulation, the age of the patient, the nature, extent or severity of the patient's condition, contraindications, if any, and the judgement of the attending physician. Variations in these dosage levels can be adjusted using standard empirical routines for optimisation.

Suitable daily dosages (per kg weight of patient) are in the range 0.0001-1 mg/kg, preferably 0.0001-0.5 mg/kg, more preferably 0.002-0.5 mg/kg, and particularly preferably 0.004-0.5 mg/kg. The unit dosage can vary from less that 1 microgram to 30mg, but typically will be in the region of 0.01 to 1 mg per dose, which may be administered daily or preferably less frequently, such as weekly or six monthly.

A particularly preferred dosing regimen is based on 2.5 ng of polypeptide as the 1X dose. In this regard, preferred dosages are in the range 1X-100X (i.e. 2.5-250 ng).

Fluid dosage forms are typically prepared utilising the polypeptide and a pyrogen-free sterile vehicle. The polypeptide, depending on the vehicle and concentration used, can be either dissolved or suspended in the vehicle. In preparing solutions the polypeptide can be dissolved in the vehicle, the solution being made isotonic if necessary by addition of sodium chloride and sterilised by filtration through a sterile filter using aseptic techniques before filling into suitable sterile vials or ampoules and sealing. Alternatively, if solution stability is adequate, the solution in its sealed containers may be sterilised by autoclaving. Advantageously additives such as buffering, solubilising, stabilising, preservative or bactericidal, suspending or emulsifying agents and or local anaesthetic agents may be dissolved in the vehicle.

Dry powders, which are dissolved or suspended in a suitable vehicle prior to use, may be prepared by filling pre-sterilised ingredients into a sterile container using aseptic technique in a sterile area. Alternatively the ingredients may be dissolved into suitable containers using aseptic technique in a sterile area. The product is then freeze dried and the containers are sealed aseptically.

Parenteral suspensions, suitable for intramuscular, subcutaneous or intradermal injection, are prepared in substantially the same manner, except that the sterile components are suspended in the sterile vehicle, instead of being dissolved and sterilisation cannot be accomplished by filtration. The components may be isolated in a sterile state or alternatively it may be sterilised after isolation, e.g. by gamma irradiation.

Advantageously, a suspending agent for example polyvinylpyrrolidone is included in the composition/s to facilitate uniform distribution of the components.

**Definitions Section**

Targeting Moiety (TM) means any chemical structure that functionally interacts with a Binding Site to cause a physical association between the polypeptide of the invention and the surface of a target cell (typically a mammalian cell, especially a human cell). The term TM embraces any molecule (ie. a naturally occurring molecule, or a chemically/physically modified variant thereof) that is capable of binding to a Binding Site on the target cell, which Binding Site is capable of internalisation (eg. endosome formation) - also referred to as receptor-mediated endocytosis. The TM may possess an endosomal membrane translocation function, in which case separate TM and Translocation Domain components need not be present in an agent of the present invention. Throughout the preceding description, specific TMs have been described. Reference to said TMs is merely exemplary, and the present invention embraces all variants and derivatives thereof, which possess a basic binding (i.e. targeting) ability to a Binding Site on the neuroendocrine tumour cell, wherein the Binding Site is capable of internalisation.

The TM of the present invention binds (preferably specifically binds) to the target cell in question. The term "specifically binds" preferably means that a given TM binds to the target cell (e.g. to an SST receptor) with a binding affinity (Ka) of $10^6$ $M^{-1}$ or greater, preferably $10^7$ $M^{-1}$ or greater, or $10^8$ $M^{-1}$ or greater, or $10^9$ $M^{-1}$ or greater. The TMs of the present invention (when in a free form, namely when separate from any protease and/ or translocation component), preferably demonstrate a binding affinity ($IC_{50}$) for the target receptor in question (eg. an SST receptor) in the region of 0.05-18nM.

The TM of the present invention is preferably not wheat germ agglutinin (WGA).

Reference to TM in the present specification embraces fragments and variants thereof, which retain the ability to bind to the target cell in question. By way of example, a variant may have at least 80%, preferably at least 90%, more preferably at least 95%, and most preferably at least 97 or at least 99% amino acid sequence homology with the reference TM - the latter is any TM sequence recited in the present application. Thus, a variant may include one or more analogues of an amino acid (e.g. an unnatural amino acid), or a substituted linkage. Also, by way of example, the term fragment, when used in relation to a TM, means a peptide having at least five, preferably at least ten, more preferably at least twenty, and most preferably at least twenty five amino acid residues of the reference TM. The term fragment also relates to the above-mentioned variants. Thus, by way of example, a fragment of the present invention may comprise a peptide sequence having at least 7, 10, 14, 17, 20, 25, 28, 29, or 30 amino acids, wherein the peptide sequence has at least 80% sequence homology over a corresponding peptide sequence (of contiguous) amino acids of the reference peptide.

By way of example, somatostatin (SST) and cortistatin (CST) have high structural homology, and bind to all known SST receptors. Full-length SST has the amino acid sequence:

MLSCRLQCALAALSIVLALGCVTGAPSDPRLRQFLQKSLAAAGKQELAKYF
LAELLSEPNQTENDALEPEDLSQAAEQDEMRLELQRSANSNPAMAPRERKA
GCKNFFWKTFTSC

Full-length CST has the amino acid sequence:

MYRHKNSWRLGLKYPPSSKEETQVPKTLISGLPGRKSSSRVGEKLQSAHKM
PLSPGLLLLLLSGATATAALPLEGGPTGRDSEHMQEAAGIRKSSLLTFLAWW
FEWTSQASAGPLIGEEAREVARRQEGAPPQQSARRDRMPCRNFFWKTFSS
CK

Reference to these TMs includes the following fragments (and corresponding variants) thereof:

NFFWKTF;
(R or K)NFFWKTF;
C(R or K)NFFWKTF;
(P or G)C(R or K)NFFWKTF;
NFFWKTF(S or T);
NFFWKTF(S or T)S;
NFFWKTF(S or T)SC;
(R or K)NFFWKTF(S or T);
(R or K)NFFWKTF(S or T)S;
(R or K)NFFWKTF(S or T)SC;
C(R or K)NFFWKTF(S or T);
C(R or K)NFFWKTF(S or T)S;
C(R or K)NFFWKTF(S or T)SC;
(P or G)C(R or K)NFFWKTF(S or T);
(P or G)C(R or K)NFFWKTF(S or T)S; or
(P or G)C(R or K)NFFWKTF(S or T)C.

With regard to the above sequences, where a (P or G) alternative is given, a P is preferred in the case of a CST TM, whereas a G is preferred in the case of an SST TM. Where an (R or K) alternative is given, an R is preferred in the case of a CST TM, whereas a K is preferred in the case of an SST TM. Where an (S or T) alternative is given, an S is preferred in the case of a CST TM, whereas a T is preferred in the case of an SST TM.

Preferred fragments comprise at least 7 or at least 10 amino acid residues, preferably at least 14 or at least 17 amino acid residues, and more preferably at least 28 or 29 amino acid residues. By way of example, preferred sequences include: SANSNPAMAPRERKAGCKNFFWKTFTSC (SST-28); AGCKNFFWKTFTSC (SST-14); QEGAPPQQSARRDRMPCRNFFWKTFSSCK (CST-29); QERPPLQQPPHRDKKPCKNFFWKTFSSCK (CST-29); QERPPPQQPPHLDKKPCKNFFWKTFSSCK (CST-29); DRMPCRNFFWKTFSSCK (CST-17); PCRNFFWKTFSSCK (CST-14); and PCKNFFWKTFSSCK (CST-14).

The TM may comprise a longer amino acid sequence, for example, at least 30 or 35 amino acid residues, or at least 40 or 45 amino acid residues, so long as the TM is able to bind to a neuroendocrine tumour cell, preferably to an SST or to a CST receptor on a neuroendocrine tumour cell. In this regard, the TM is preferably a fragment of full-length SST or CST, though including at least the core sequence "NFFWKTF" or one of the above-defined primary amino acid sequences.

By way of further example, GHRH peptides of the present invention include: YADAIFTASYRKVLGQLSARKLLQDILSR; YADAIFTASYRNVLGQLSARKLLQDILSR; YADAIFTNSYRKVLGQLSARKLLQDIM; YADAIFTNSYRKVLGQLSARKLLQDIMS; ADAIFTNSYRKVLGQLSARKLLQDIMSR; YADAIFTNSYRKVLGQLSARKLLQDIMSRQQGESNQERGARARL; YADAIFTNSYRKVLGQLSARKLLQDIMSRQQGESNQERGA; YADAIFTNAYRKVLGQLSARKLLQDIMSR; YADAIFTNSYRKVLGQLSARKALQDIMSR; YADAIFTASYKKVLGQLSARKLLQDIMSR; YADAIFTASYKRVLGQLSARKLLQDIMSR; YADAIFTASYNKVLGQLSARKLLQDIMSR; YADAIFTASYRKVLGQLSAKKLLQDIMSR; YADAIFTASYKKVLGQLSAKKLLQDIMSR; YADAIFTASYRKVLGQLSANKLLQDIMSR; YADAIFTASYRNVLGQLSARKLLQDIMSR; YADAIFTASYRKVLGQLSARNLLQDIMSR; YADAIFEASYRKVLGQLSARKLLQDIMSR; YADAIFTASERKVLGQLSARKLLQDIMSR; YADAIFTASYRKELGQLSARKLLQDIMSR; YADAIFTASYRKVLGQLSARKLLQDIMSR; YADAIFTESYRKVLGQLSARKLLQDIMSR; YADAIFTNSYRKVLAQLSARKLLQDIM; YADAIFTNSYRKVLAQLSARKLLQDIMSR; YADAIFTASYRKVLAQLSARKLLQDIMSR; YADAIFTAAYRKVLAQLSARKALQDIASR; YADAIFTAAYRKVLAQLSARKALQDIMSR; HVDAIFTQSYRKVLAQLSARKLLQDILNRQQGERNQEQGA; HVDAIFTQSYRKVLAQLSARKALQDILSRQQG; HVDAIFTSSYRKVLAQLSARKLLQDILSR; HVDAIFTTSYRKVLAQLSARKLLQDILSR; YADAIFTQSYRKVLAQLSARKALQDILNR; YADAIFTQSYRKVLAQLSARKALQDILSR.

It is routine to confirm that a TM binds to the selected target cell. For example, a simple radioactive displacement experiment may be employed in which tissue or cells representative of a neuroendocrine tumour cell are exposed to labelled (eg. tritiated) TM in the presence of an excess of unlabelled TM. In such an experiment, the relative proportions of non-specific and specific binding may be assessed, thereby allowing confirmation that the TM binds to the target cell. Optionally, the assay may include one or more binding antagonists, and the assay may further comprise observing a loss of TM binding. Examples of this type of experiment can be found in Hulme, E.C. (1990), Receptor-binding studies, a brief outline, pp. 303-311, In Receptor biochemistry, A Practical Approach, Ed. E.C. Hulme, Oxford University Press. In the context of the present invention, reference to a peptide TM (e.g. SST peptide, CST peptide, or GHRH peptide, etc) embraces peptide analogues thereof, so long as the analogue TM binds to the same receptor as the corresponding 'reference' TM. Said analogues may include synthetic residues such as: β-Nal = β-naphthylalanine; β-Pal = β-pyridylalanine; hArg(Bu) = N-guanidino-(butyl)-homoarginine; $hArg(Et)_2$ = N, N'-guanidino-(dimethyl)-homoarginine; $hArg(CH_2CF_3)_2$ = N, N-guanidino-bis-(2,2,2,-trifluoroethyl)-homoarginine; $hArg(CH_3$, hexyl) = N, N-guanidino-(methyl, hexyl)-homoarginine; Lys(Me) = $N^e$-methyllysine; Lys(iPr) = $N^e$-isopropyllysine; AmPhe = aminomethylphenylalanine; AChxAla = aminocyclohexylalanine; Abu = α-aminobutyric acid; Tpo = 4-thiaproline; MeLeu = N-methylleucine; Orn = ornithine; Nle - norleucine; Nva = norvaline; Trp(Br) = 5-bromo-tryptophan; Trp(F) = 5-fluoro-tryptophan; $Trp(N0_2)$ = 5-nitro-tryptophan; Gaba = γ-aminobutyric acid; Bmp = J-mercaptopropionyl; Ac = acetyl; and Pen = pencillamine

By way of example, the above peptide analogue aspect is described in more detail with reference to specific peptide TMs, such as SST peptides, GHRH peptides, bombesin peptides, ghrelin peptides, GnRH (aka LHRH peptides), and urotensin peptides, though the same principle applies to all TMs of the present invention.

Somatostatin analogues, which can be used to practice the present invention include, but are not limited to, those described in the following publications, which are hereby incorporated by reference: Van Binst, G. et al. Peptide Research 5: 8 (1992); Horvath, A. et al. Abstract, "Conformations of Somatostatin Analogs Having Antitumor Activity", 22nd European peptide Symposium, September 13-19,1992, Interlaken, Switzerland; US5,506,339; EP0363589; US4,904,642; US4,871,717; US4,725,577; US4,684,620; US4,650,787; US4,585,755; US4,725,577; US4,522,813; US4,369,179; US4,360,516; US4,328,214; US4,316,890; US4,310,518; US4,291,022; US4,238,481; US4,235,886; US4,211,693; US4,190,648; US4,146,612; US4,133,782; US5,506,339; US4,261,885; US4,282,143; US4,190,575; US5,552,520; EP0389180; EP0505680; US4,603,120; EP0030920; US4,853,371; WO90/12811; WO97/01579; WO91/18016; WO98/08529 and WO98/08528; WO/0075186 and WO00/06185; WO99/56769; and FR 2,522,655.

Preferred analogues include: cyclo(N-Me-Ala-Tyr-D-Trp-Lys-Val-Phe) or H-D-β-Nal-Cys-Tyr-D-Trp-Lys-Thr-Cys-Thr-NH2; H-Cys-Phe-Phe-D-Trp-Lys-Thr-Phe-Cys-NH2; H-Cys-Phe-Tyr-D-Trp-Lys-Thr-Phe-Cys-NH2; H-Cys-Phe-Phe-D-Trp-Lys-Ser-Phe-Cys-NH2; H-Cys-Phe-Tyr-D-Trp-Lys-Thr-Phe-Cys-NH2; H-Cys-Phe-Phe-D-Trp-Lys-Thr-NH2; H-Cys-Phe-Phe-D-Trp-Lys-Thr-Phe-Cys-NH2; H-Phe-Phe-Phe-D-Trp-Lys-Thr-NH2; H-D-Phe-Phe-Phe-D-Trp-Lys-Thr-Phe-THr-NH2; H-Cys-Phe-Tyr(I)-D-Trp-Lys-Thr-Phe-Cys-NH2; H-D-Phe-p-chloro-Phe-Tyr-D-Trp-Lys-Thr-Phe-Thr-NH2, H-D-Phe-p-NO2-Phe-Tyr-D-Trp-Lys-Val-Phe-Thr-NH2, H-D-β-Nal-p-chloro-Phe-Tyr-D-Trp-Lys-Val-Phe-Thr-NH2, H-D-Phe-Phe-Tyr-D-Trp-Lys-Val-Phe-Thr-NH2, H-D-Phe-p-chloro-Phe-Tyr-D-Trp-Lys-Val-Phe-Thr-NH2, H-D-Phe-Ala-Tyr-D-Trp-Lys-Val-Ala-D-β-Nal-NH2; H-D-β-Nal-Cys-Tyr-D-Trp-Lys-Thr-Cys-Thr-NH2; H-D-Phe-Cys-Phe-D-Trp-Lys-Thr-Cys-β-Nal-NH2; H-D-Phe-Cys-Tyr-D-Trp-Lys-Thr-Cys-β-Nal-NH2; H-D-β-Nal-Cys-Phe-D-Trp-Lys-Thr-Cys-Thr-NH2; H-D-Phe-Cys-Tyr-D-Trp-Lys-Thr-Pen-Thr-NH2; H-D-Phe-Cys-Phe-D-Trp-Lys-Thr-Pen-Thr-NH2; H-D-Phe-Cys-Tyr-D-Trp-Lys-Thr-Pen-Thr-OH; H-D-Phe-Cys-Phe-D-Trp-Lys-Thr-Pen-Thr-OH; H-Gly-Pen-Phe-D-Trp-Lys-Thr-Cys-Thr-OH; H-Phe-Pen-Tyr-D-Trp-Lys-Thr-Cys-Thr-OH; H-Phe-Pen-Phe-D-Trp-Lys-Thr-Pen-Thr-OH; H-D-Phe-Cys-Phe-D-Trp-Lys-Thr-Cys-Thr-ol; H-D-Phe-Cys-Phe-D-Trp-Lys-Thr-Cys-Thr-NH2; H-D-Trp-Cys-Tyr-D-Trp-Lys-Val-Cys-Thr-NH2; H-D-Trp-Cys-Phe-D-Trp-Lys-Thr-Cys-Thr-NH2; H-D-Phe-Cys-Tyr-D-Trp-Lys-Val-Cys-Thr-NH2; H-D-Phe-Cys-Tyr-D-Trp-Lys-Val-Cys-Trp-NH2; H-D-Phe-Cys-Tyr-D-Trp-Lys-Val-Cys-Thr-NH2; Ac-D-Phe-Lys*-Tyr-D-Trp-Lys-Val-Asp*-Thr-NH2 (an amide bridge formed between Lys* and Asp*); Ac-hArg (Et) 2-Gly-Cys-Phe-D-Trp-Lys-Thr-Cys-Thr-NH2; Ac-D-hArg (Et) 2-Gly-Cys-Phe-D-Trp-Lys-Thr-Cys-Thr-NH2; Ac-D-hArg (Bu)-Gly-Cys-Phe-D-Trp-Lys-Thr-Cys-Thr-NH2; Ac-D-hArg (Et) 2-Cys-Phe-D-Trp-Lys-Thr-Cys-Thr-NH2; Ac-L-hArg (Et) 2-Cys-Phe-D-Trp-Lys-Thr-Cys-Thr-NH2; Ac-D-hArg (CH2CF3) 2-Cys-Phe-D-Trp-Lys-Thr-Cys-Thr-NH2; Ac-D-hArg (CH2CF3) 2-Gly-Cys-Phe-D-Trp-Lys-Thr-Cys-Thr-NH2; Ac-D-hArg (CH2CF3) 2-Gly-Cys-Phe-D-Trp-Lys-Thr-Cys-Phe-NH2; Ac-D-hArg (CH2CF3) 2-Gly-Cys-Phe-D-Trp-Lys-Thr-Cys-Thr-NHEt; Ac-L-hArg (CH2-CF3) 2-Gly-Cys-Phe-D-Trp-Lys-Thr-Cys-Thr-NH2; Ac-D-hArg (CH2CF3) 2-Gly-Cys-Phe-D-Trp-Lys (Me)-Thr-Cys-Thr-NH2; Ac-D-hArg (CH2CF3) 2-Gly-Cys-Phe-D-Trp-Lys (Me)-Thr-Cys-Thr-NHEt; Ac-hArg (CH3, hexyl)-Gly-Cys-Phe-D-Trp-Lys-Thr-Cys-Thr-NH2; H-hArg (hexyl2)-Gly-Cys-Phe-D-Trp-Lys-Thr-Cys-Thr-NH2; Ac-D-hArg (Et) 2-Gly-Cys-Phe-D-Trp-Lys-Thr-Cys-Thr-NHEt; Ac-D-hArg (Et) 2-Gly-Cys-Phe-D-Trp-Lys-Thr-Cys-Phe-NH2; Propionyl-D-hArg (Et) 2-Gly-Cys-Phe-D-Trp-Lys (iPr)-Thr-Cys-Thr-NH2; Ac-D-β-Nal-Gly-Cys-Phe-D-Trp-Lys-Thr-Cys-Gly-hArg (Et) 2-NH2; Ac-D-Lys (iPr)-Gly-Cys-Phe-D-Trp-Lys-Thr-Cys-Thr-NH2; Ac-D-hArg (CH2CF3) 2-D-hArg (CH2CF3) 2-Gly-Cys-Phe-D-Trp-Lys-Thr- Cys- Thr-NH2; Ac-D-hArg (CH2CF3) 2-D-hArg (CH2CF3) 2-Gly-Cys-Phe-D-Trp-Lys-Thr- Cys- Phe-NH2; Ac-D-hArg (Et) 2-D-hArg (Et) 2-Gly-Cys-Phe-D-Trp-Lys-Thr-Cys-Thr-NH2; c-Cys-Lys-Asn-4-Cl-Phe-Phe-D-Trp-Lys-Thr-Phe-Thr-Ser-D-Cys-NH2; H-Bmp-Tyr-D-Trp-Lys-Val-Cys-Thr-NH2; H-Bmp-Tyr-D-Trp-Lys-Val-Cys-Phe-NH2; H-Bmp-Tyr-D-Trp-Lys-Val-Cys-p-Cl-Phe-NH2; H-Bmp-Tyr-D-Trp-Lys-Val-Cys-p-Nal-NH2; H-D-β-Nal-Cys-Tyr-D-Trp-Lys-Val-Cys-Thr-NH2; H-D-Phe-Cys-Tyr-D-Trp-Lys-Abu-Cys-Thr-NH2; H-D-Phe-Cys-Tyr-D-Trp-Lys-Abu-Cys-beta-Nal-NH2; H-pentafluoro-D-Phe-Cys-Tyr-D-Trp-Lys-Val-Cys-Thr-NH2; Ac-D-β-Nal-Cys-pentafluoro-Phe-D-Trp-Lys-Val-Cys-Thr-NH2; H-D-i-Nal-Cys-Tyr-D-Trp-Lys-Val-Cys-p-Nal-NH2; H-D-Phe-Cys-Tyr-D-Trp-Lys-Val-Cys-β-Nal-NH2; H-D-, SNal-Cys-Tyr-D-Trp-Lys-Abu-Cys-Thr-NH2; H-D-p-Cl-Phe-Cys-Tyr-D-Trp-Lys-Abu-Cys-Thr-NH2; Ac-D-p-Cl-Phe-Cys-Tyr-D-Trp-Lys-Abu-Cys-Thr-NH2; H-D-Phe-Cys-p-Nal-D-Trp-Lys-Val-Cys-Thr-NH2; H-D-Phe-Cys-Tyr-D-Trp-Lys-Cys-Thr-NH2; cyclo (Pro-Phe-D-Trp-N-Me-Lys-Thr-Phe); cyclo (Pro-Phe-D-Trp-N-Me-Lys-Thr-Phe); cyclo (Pro-Phe-D-Trp-Lys-Thr-N-Me-Phe); cyclo (N-Me-Ala-Tyr-D-Trp-Lys-Thr-Phe); cyclo (Pro-Tyr-D-Trp-Lys-Thr-Phe); cyclo (Pro-Phe-D-Trp-Lys-Thr-Phe); cyclo (Pro-Phe-L-Trp-Lys-Thr-Phe); cyclo (Pro-Phe-D-Trp (F)-Lys-Thr-Phe); cyclo (Pro-Phe-Trp (F)-Lys-Thr-Phe); cyclo (Pro-Phe-D-Trp-Lys-Ser-Phe); cyclo (Pro-Phe-D-Trp-Lys-Thr-p-Cl-Phe); cyclo (D-Ala-N-Me-D-Phe-D-Thr-D-Lys-Trp-D-Phe); cyclo (D-Ala-N-Me-D-Phe-D-Val-Lys-D-Trp-D-Phe); cyclo (D-Ala-N-Me-D-Phe-D-Thr-Lys-D-Trp-D-Phe); cyclo (D-Abu-N-Me-D-Phe-D-Val-Lys-D-Trp-D-Tyr); cyclo (Pro-Tyr-D-Trp-t-4-AchxAla-Thr-Phe); cyclo (Pro-Phe-D-Trp-t-4-AchxAla-Thr-Phe); cyclo (N-Me-Ala-Tyr-D-Trp-Lys-Val-Phe); cyclo (N-Me-Ala-Tyr-D-Trp-t-4-AchxAla-Thr-Phe); cyclo (Pro-Tyr-D-Trp-4-Amphe-Thr-Phe); cyclo (Pro-Phe-D-Trp-4-Amphe-Thr-Phe); cyclo (N-Me-Ala-Tyr-D-Trp-4-Amphe-Thr-Phe); cyclo (Asn-Phe-Phe-D-Trp-Lys-Thr-Phe-Gaba); cyclo (Asn-Phe-Phe-D-Trp-Lys-Thr-Phe-Gaba-Gaba); cyclo (Asn-Phe-D-Trp-Lys-Thr-Phe); cyclo (Asn-Phe-Phe-D-Trp-Lys-Thr-Phe-NH (CH2) 4CO); cyclo (Asn-Phe-Phe-D-Trp-Lys-Thr-Phe->Ala); cyclo (Asn-Phe-Phe-D-Trp-Lys-Thr-Phe-D-Glu)-OH; cyclo (Phe-Phe-D-Trp-Lys-Thr-Phe); cyclo (Phe-Phe-D-Trp-Lys-Thr-Phe-Gly); cyclo (Phe-Phe-D-Trp-Lys-Thr-Phe-Gaba); cyclo (Asn-Phe-Phe-D-Trp-Lys-Thr-Phe-Gly); cyclo (Asn-Phe-Phe-D-Trp (F)-Lys-Thr-Phe-Gaba); cyclo (Asn-Phe-Phe-D-Trp (NO2)-Lys-Thr-Phe-Gaba); cyclo (Asn-Phe-Phe-Trp (Br)-Lys-Thr-Phe-Gaba); cyclo (Asn-Phe-Phe-D-Trp-Lys-Thr-Phe (I)-Gaba); cyclo (Asn-Phe-Phe-D-Trp-Lys-Thr-Tyr (But)-Gaba); cyclo (Bmp-Lys-Asn-Phe-Phe-D-Trp-Lys-Thr-Phe-Thr-Pro-Cys)-OH; cyclo (Bmp-Lys-Asn-Phe-Phe-D-Trp-Lys-Thr-Phe-Thr-Pro-Cys)-OH; cyclo (Bmp-Lys-Asn-Phe-Phe-D-Trp-Lys-Thr-Phe-Thr-Tpo-Cys)-OH; cyclo (Bmp-Lys-Asn-Phe-Phe-D-Trp-Lys-Thr-Phe-Thr-MeLeu-Cys)-OH; cyclo (Phe-Phe-D-Trp-Lys-Thr-Phe-Phe-Gaba); cyclo (Phe-Phe-D-Trp-Lys-Thr-Phe-D-Phe-Gaba); cyclo (Phe-Phe-D-Trp (5F)-Lys-Thr-Phe-Phe-Gaba); cyclo (Asn-Phe-Phe-D-Trp-Lys (Ac)-Thr-Phe-NH- (CH2) 3-CO); cyclo (Lys-Phe-Phe-D-Trp-Lys-Thr-Phe-Gaba); cyclo (Lys-Phe-Phe-D-Trp-Lys-Thr-Phe-Gaba); cyclo (Orn-Phe-Phe-D-Trp-Lys-Thr-Phe-Gaba); H-Cys-Phe-Phe-D-Trp-Lys-Thr-Phe-Cys-NH2; H-Cys-Phe-Phe-D-Trp-Lys-Ser-Phe-Cys-NH2; H-Cys-Phe-Tyr-D-Trp-Lys-Thr-Phe-Cys-NH2; H-Cys-Phe-Tyr (I)-D-Trp-Lys-Thr-Phe-Cys-NH2.

Methods for synthesizing analogues are well documented, as illustrated, for example, by the patents cited above. For example, synthesis of H-D-Phe-Phe-Phe-D-Trp-Lys-Thr-Phe-Thr-NH2, can be achieved by following the protocol set forth in Example 1 of EP0395417A1. Similarly, synthesis analogues with a substituted N-terminus can be achieved, for example, by following the protocol set forth in WO88/02756, EP0329295, and US5,240,561.

Preferred examples of linear analogues include: H-D-Phe-p-chloro-Phe-Tyr-D-Trp-Lys-Thr-Phe-Thr-NH2; H-D-Phe-p-N02-Phe-Tyr-D-Trp-Lys-Val-Phe-Thr-NH2; H-D-*Nal-p-chloro-Phe-Tyr-D-Trp-Lys-Val-Phe-Thr-NH2; H-D-Phe-Phe-Phe-D-Trp-Lys-Thr-Phe-Thr-NH2; H-D-Phe-Phe-Tyr-D-Trp-Lys-Val-Phe-Thr-NH2; H-D-Phe-p-chloro-Phe-Tyr-D-Trp-Lys-Val-Phe-Thr-NH2; and H-D-Phe-Ala-Tyr-D-Trp-Lys-Val-Ala-D-beta-Nal-NH2.

One or more chemical moieties, eg. a sugar derivative, mono or poly-hydroxy (C2-12) alkyl, mono or poly-hydroxy (C2-12) acyl groups, or a piperazine derivative, can be attached to a SST analogue, e g. to the N-terminus amino acid - see WO88/02756, EP0329295, and US5,240,561.

Further examples of SST analogues that can be used as a TM in the present invention include the following: D-Cpa-cyclo[Cys-Tyr-D-Trp-Lys-Val-Cys]-Thr-NH2; D-Phe-cyclo[Cys-Tyr-D-Trp-Lys-Val-Cys]-Thr-NH2; D-Phe-cyclo[Cys-p-NH2-Phe-D-Trp-Lys-Val-Cys]-Thr-NH2; N-Me-D-Phe-cyclo[Cys-Tyr-D-Trp-Lys-Val-Cys]-Thr-NH2; D-Phe-cyclo[Cys-Tyr-D-Pal-Lys-Val-Cys]-Thr-NH2; Ac-D-Nal-cyclo[Cys-Tyr-D-Trp-Lys-Val-Cys]-Thr-NH2; D-Phe-cyclo [Cys-Tyr-D-Trp-Lys-Val-Cys]-Nal-NH2; D-Nal-cyclo[Cys-Tyr-D-Trp-Lys-Val-Cys]-Nal-NH2; D-Nal-cyclo[Cys-Tyr-D-Trp-Lys-Val-Cys]-Thr-OH; ED-Phe-cyclo[Cys-Nal-D-Trp-Lys-Val-Cys]-Thr-NH2; D-Nal-cyclo[Cys-Tyr-D-Nal-Lys-Val-Cys]-Nal-NH2; D-Nal-cyclo[Cys-Tyr-D-Trp-Lys-Val-D-Cys]-Nal-NH2; D-Trp-cyclo[Cys-Tyr-D-Trp-Lys-Val-Cys]-Nal-NH2; D-Nal-cyclo[Cys-Tyr-D-Trp-Lys-Val-Cys]-D-Nal-NH2; Nal-cyclo[Cys-Tyr-D-Trp-Lys-Val-Cys]-D-Nal-NH2; (AcO-CH2)3-C-NH-CO-(CH2)2-CO-D-Nal-cyclo(Cys-Tyr-D-Trp-Lys-Val-Cys]Thr-NH2; [3-O-(2,5,6-triacetyl ascorbic)acetyl-D-Nal-cyclo[Cys-Tyr-D-Trp-Lys-Val-Cys]-Thr-NH2; D-Nal-cyclo[Cys-Tyr-D-Trp-Lys-Val-Cys]-Trp-NH2; Phe-cyclo[Cys-Tyr-D-Trp-Lys-Val-Cys]-Trp-NH2; 3-0-(ascorbic)-butyrl-D-Nal-cyclo[Cys-Tyr-D-Trp-Lys-Val-Cys]-Thr-NH2; 3-O-(ascorbic acid)Ac-D-Nal-cyclo[Cys-Tyr-D-Trp-Lys-Val-Cys]-Thr-NH2; D-Bpa-cyclo[Cys-Tyr-D-Trp-Lys-Val-Cys]-Nal-NH2; D-Nal-cyclo[Cys-Tyr-D-Trp-Lys-Val-Cys]-Bpa-NH2; Tris-Suc-D-Nal-cyclo[Cys-Tyr-D-Trp-Lys-Val-Cys]-Thr-NH2; D-Dpa-cyclo[Cys-Tyr-D-Trp-Lys-Val-Cys]-Nal-NH2; D-Nal-cyclo[Cys-Tyr-D-Trp-Lys-Val-Cys]-Dpa-NH2; Ac-D-Nal-cyclo[Cys-Tyr-D-Trp-Lys-Val-Cys]-Thr-NH2; cyclo-[Cys-Tyr-D-Trp-Lys-Val-Cys]-Thr-NH2; NmeCpa-cyclo (DCys-3-Pal-DTrp-Lys-Thr-Cys)-2-Nal-NH2; Cpa-cyclo(NMeDCys-3-Pal-DTrp-Lys-Thr-Cys)-2-Nal-NHMe; Cpa-cyclo (DCys-NMe3-Pal-DTrp-Lys-Thr-Cys)-2-Nal-NH2; Cpa-cyclo(DCys-3-Pal-NMeDTrp-Lys-Thr-Cys)-2-Nal-NH2; Cpa-cyclo(DCys-3-Pal-DTrp-NMe-Lys-Thr-Cys)-2-Nal-NH2; Cpa-cyclo(DCys-3-Pal-DTrp-Lys-NMeThr-Cys)-2-Nal-NH2; Cpa-cyclo (DCys-3-Pal-DTrp-Lys-Thr-NMeCys)-2-Nal-NH2; Cpa-cyclo (DCys-3-Pal-DTrp-Lys-Thr-Cys)-Nme2-Nal-NH2; Cpa-cyclo(NMeDCys-3-Pal-DTrp-Lys-Thr-Cys)-Dip-NHMe; Cpa-cyclo (DCys-3-Pal-NMeDTrp-NMeLys-Thr-Cys)-2-Nal-NH2; Cpa-cyclo(DCys-Tyr-DTrp-NMeLys-Thr-Cys)-2-Nal-NH2; Tfm-cyclo (DCys-3-Pal-DTrp-NMeLys-Thr-Cys)-2-Nal-NH2; Cpa-cyclo(DCys-3-Pal-DTrp-NMeLys-Thr-Cys)-DTrp-NH2; Nal-cyclo (DCys-3-Pal-DTrp-NMeLys-Thr-Cys)-DTrp-NH2; 3-Pal-cyclo (DCys-3-Pal-DTrp-NMeLys-Thr-Cys)-DTrp-NH2; NmeCpa-cyclo (DCys-3-Pal-DTrp-Lys-Thr-Cys)-2-Nal-NH2; Cpa-cyclo(DCys-3-Pal-DTrp-NMeLys-Thr-Cys)-2-Nal-NH2; Cpa-cyclo(DCys-3-Pal-NMeDTrp-NMeLys-Thr-Cys)-2-Nal-NH2; Cpa-cyclo (DCys-Tyr-DTrp-NMeLys-Thr-Cys)-2-Nal-NH2; Cpa-cyclo(DCys-3-Pal-DTrp-NMeLys-Thr-Cys)-DTrp-NH2; Nal-cyclo (DCys-Pal-DTrp-NMeLys-Thr-Cys)-DTrp-NH2; or 3-Pal-cyclo(DCys-3-Pal-DTrp-NMeLys-Thr-Cys)-DTrp-NH2; NmeCpa-cyclo (DCys-3-Pal-DTrp-Lys-Thr-Cys)-2-Nal-NH2; Cpa-cyclo(DCys-3-Pal-DTrp-NMeLys-Thr-Cys)-2-Nal-NH2; Cpa-cyclo (DCys-3-Pal-NMeDTrp-NMeLys-Thr-Cys)-2-Nal-NH2; Cpa-cyclo (DCys-Tyr-DTrp-NMeLys-Thr-Cys)-2-Nal-NH2; or Cpa-cyclo(DCys-3-Pal-DTrp-NMeLys-Thr-Cys)-DTrp-NH2; Cpa-cyclo (DCys-3-Pal-DTrp-NMeLys-Thr-Cys)-2-Nal-NH2; Cpa-cyclo(DCys-Tyr-DTrp-NMeLys-Thr-Cys)-2-Nal-NH2; methylpropionic acid-Tyr-D-Trp- ys-Val-Cys-Thr-$NH_2$; methylpropionic acid-Tyr-D-Trp- ys-Val-Cys-Phe-$NH_2$; methylpropionic acid-Tyr-D-Trp-Lys-Val-Cys-p-Cl-Phe-NH2; methylpropionic acid-Tyr-D-Trp-Lys-Val-Cys-β-Nal-$NH_2$; D-Phe-Phe-Phe-D-Trp-Lys-Thr-Phe-Thr-NH2; D-Phe-Phe-Tyr-D-Trp-Lys-val-Phe-Thr-NH2; D-Phe-p-chloro-Phe-Tyr-D-Trp-Lys-Val-Phe-Thr-$NH_2$; or D-Phe-Ala-Tyr-D-Trp-Lys-Val-Ala-β-D-Nal-$NH_2$; $H_2$-c[Cys-Phe-Phe-D-Trp-Lys-Thr-Phe-Cys]-$NH_2$, $H_2$-c[D-Cys-Phe-Phe-D-Trp-Lys-Thr-Phe-Cys]-$NH_2$, $H_2$-c[Cys-Phe-Trp-D-Trp-Lys-Ser-Phe-Cys]-$NH_2$, $H_2$-c[Cys-Phe-Phe-D-Trp-Lys-Ser-Phe-Cys]-$NH_2$, or $H_2$-c[Cys-Phe-Tyr(I)-D-Trp-Lys-Thr-Phe-Cys]-$NH_2$, $H_2$-c[Cys-Phe-Trp-D-Trp-Lys-Thr-Phe-Cys]-$NH_2$, $H_2$-c[D-Cys-Phe-Trp-D-Trp-Lys-Thr-Phe-Cys]-$NH_2$, $H_2$-c[Cys-Phe-His-D-Trp-Lys-Thr-Phe-Cys]-$NH_2$, $H_2$-c[D-Cys-Phe-His-D-Trp-Lys-Thr-Phe-Cys]-$NH_2$, $H_2$-c[D-Cys-Phe-Phe-D-Trp-Lys-Ser-Phe-Cys]-$NH_2$, $H_2$-c[D-Cys-Phe-Trp-D-Trp-Lys-Ser-Phe-Cys]-$NH_2$, $H_2$-c[Cys-Phe-His-D-Trp-Lys-Ser-Phe-Cys]-$NH_2$, $H_2$-c[D-Cys-Phe-His-D-Trp-Lys-Ser-Phe-Cys]-$NH_2$, $H_2$-c[D-Cys-Phe-Tyr(I)-D-Trp-Lys-Thr-Phe-Cys]-$NH_2$, $H_2$-c[Cys-Phe-Tyr(I)-D-Trp-Lys-Ser-Phe-Cys]-$NH_2$, or $H_2$-c[D-Cys-Phe-Tyr(I)-D-Trp-Lys-Ser-Phe-Cys]-$NH_2$, $H_2$-c[D-Cys-Asn-Phe-Phe-D-Trp-Lys-Thr-Phe-Cys]-$NH_2$, $H_2$-c[Cys-Asn-Phe-Trp-D-Trp-Lys-Thr-Phe-Cys]-$NH_2$, $H_2$-c [D-Cys-Asn-Phe-Trp-D-Trp-Lys-Thr-Phe-Cys]-$NH_2$, $H_2$-c [Cys-Asn-Phe-His-D-Trp-Lys-Thr-Phe-Cys]-$NH_2$, $H_2$-c[D-Cys-Asn-Phe-His-D-Trp-Lys-Thr-Phe-Cys]-$NH_2$, $H_2$-c[Cys-Asn-Phe-Phe-D-Trp-Lys-Ser-Phe-Cys]-$NH_2$, $H_2$-c[D-Cys-Asn-Phe-Phe-D-Trp-Lys-Ser-Phe-Cys]-$NH_2$, $H_2$-c[Cys-Asn-Phe-Trp-D-Trp-Lys-Ser-Phe-Cys]-$NH_2$, $H_2$-c[D-Cys-Asn-Phe-Trp-D-Trp-Lys-Ser-Phe-Cys]-$NH_2$, $H_2$-c[Cys-Asn-Phe-His-D-Trp-Lys-Ser-Phe-Cys]-$NH_2$, $H_2$-c[D-Cys-Asn-Phe-His-D-Trp-Lys-Ser-Phe-Cys]-$NH_2$, $H_2$-c[Cys-Asn-Phe-Tyr(I)-D-Trp-Lys-Thr-Phe-Cys]-$NH_2$, $H_2$-c[D-Cys-Asn-Phe-Tyr(I)-D-Trp-Lys-Thr-Phe-Cys]-$NH_2$, $H_2$-c[Cys-Asn-Phe-Tyr(I)-D-rp-Lys-Ser-Phe-Cys]-$NH_2$, $H_2$-c[D-Cys-Asn-Phe-Tyr(I)-D-Trp-Lys-Ser-Phe-Cys]-$NH_2$, $H_2$-c[Cys-Phe-Phe-D-Trp-Lys-Thr-Phe-Cys]-$NH_2$; Ac-D-Phe-Tyr-cyclo (D-Cys-D-Trp-Lys-Cys)-Abu-Thr-NH2; Nal-Tyr-cyclo(Cys-D-Trp-Lys-D-Cys)-Val-Nal-NH2; Nal-Tyr-cyclo(Cys-D-Trp-Lys-D-Cys)-Abu-Nal-NH2; D-Dip-Tyr-cyclo(Cys-D-Trp-Lys-D-Cys)-Abu-Nal-NH2; Dip-Tyr-cyclo (D-Cys-D-Trp-Lys-D-Cys)-Abu-Nal-NH2; Nal-Tyr-cyclo(D-Cys-D-Trp-Lys-D-Cys)-Abu-Nal-NH2; Dip-Tyr-cyclo(D-Cys-D-Trp-Lys-D-Cys)-Val-Nal-NH2; Nal-Tyr-cyclo(D-Cys-D-Trp-Lys-D-Cys)-Val-Nal-NH2; cyclo(D-Phe-Tyr-cyclo(D-Cys-D-Trp-Lys-Cys)-Abu-Thr); Cpa-Pal-cyclo(D-Cys-D-Trp-Lys-D-Cys)-A3c-Nal-NH2; Cpa-Pal-cyclo(D-Cys-D-Trp-Lys-D-Cys)-A5c-Nal-NH2; Cpa-Pal-cyclo(D-Cys-D-Trp-Lys-D-Cys)-A6c-Nal-NH2; (G(z))aeg-cyclo(D-Cys-D-Trp-Lys-D-Cys)-A5c-Nal-NH2; Pal-cyclo(D-Cys-D-Trp-Lys-D-Cys)-A5c-Nal-NH2; Cpa-Pal-cyclo(D-Cys-D-Trp-Lys-D-Cys)-β-Ala-Nal-NH2; Cpa-Pal-cyclo(D-Cys-D-Trp-Lys-D-Cys)-Sar-Nal-NH2; Cpa-Pal-cyclo(D-Cys-D-Trp-Lys-D-Cys)-Gaba-Nal-NH2; Cpa-Pal-cyclo(D-Cys-D-Trp-Lys-D-Cys)-Pro-Nal-NH2; Pro-Phe-c(D-Cys-D-Trp-Lys-D-Cys)-Nle-Phe-NH2; Pro-Phe-c(D-Cys-D-Trp-Lys-D-Cys)-Thr-Nle-NH2; Pro-Phe-c (D-Cys-D-Trp-Lys-D-Cys)-Thr-Phe-NH2; Cpa-Phe-c (D-Cys-D-Trp-Lys-D-Cys)-Gaba-NH2 ; Cpa-Phe-c(D-Cys-D-Trp-Lys-D-Cys)-Gaba-Tyr-NH2; Pip-Phe-c (D-Cys-D-Trp-Lys-D-Cys)-NH2; Pip-Phe-c (Cys-D-Trp-Lys-Cys)-Gaba-NH2; or Pro-Phe-c(D-Cys-D-Trp-Lys-D-Cys)-Thr-NH2; Phe-cyclo(Cys-D-Trp-Lys-Cys)-Thr-NH2; Phe-Tyr-cyclo(D-Cys-D-Trp-Lys-Cys)-Abu-Thr-NH2; Ac-D-Phe-Tyr-cyclo(D-Cys-D-Trp-Lys-Cys)-Abu-Thr-NH2; Nal-Tyr-cyclo(Cys-D-Trp-Lys-D-Cys)-Val-Nal-NH2; Nal-Tyr-cyclo(Cys-D-Trp-Lys-D-Cys)-Abu-Nal-NH2; Dip-Tyr-cyclo(D-Cys-D-Trp-Lys-D-Cys)-Abu-Nal-NH2; Nal-Tyr-cyclo(D-Cys-D-Trp-Lys-D-Cys)-Abu-Nal-NH2; Dip-Tyr-cyclo (D-Cys-D-Trp-Lys-D-Cys)-Val-Nal-NH2; Nal-Tyr-cyclo(D-Cys-D-Trp-Lys-D-Cys)-Val-Nal-NH2, Cpa-Pal-cyclo(D-Cys-D-Trp-Lys-D-Cys)-A3c-Nal-NH2; Cpa-Pal-cyclo(D-Cys-D-Trp-Lys-D-Cys)-A5c-Nal-NH2; Cpa-Pal-cyclo(D-Cys-D-Trp-Lys-D-Cys)-A6c-Nal-NH2; (G(z))aeg-cyclo(D-Cys-D-Trp-Lys-D-Cys)-A5c-Nal-NH2; D-Cpa-cyclo(Cys-D-Trp-Lys-D-Cys)-A5c-Nal-NH2; Pal-cyclo (D-Cys-D-Trp-Lys-D-Cys)-A5c-Nal-NH2; Cpa-cyclo(D-Cys-D-Trp-Lys-D-Cys)-A5c-Nal-NH2; Cpa-Pal-cyclo(D-Cys-D-Trp-Lys-D-Cys)-β-Ala-Nal-NH2; Cpa-Pal-cyclo(D-Cys-D-Trp-Lys-D-Cys)-Sar-Nal-NH2; Cpa-Pal-cyclo(D-Cys-D-Trp-Lys-D-Cys)-Aic-Nal-NH2; Cpa-Pal-cyclo(D-Cys-D-Trp-Lys-D-Cys)-Gaba-Nal-NH2; Cpa-Pal-cyclo(D-Cys-D-Trp-Lys-D-Cys)-Pro-Nal-NH2; (T)aeg-cyclo(D-Cys-D-Trp-Lys-D-Cys)-(A)aeg-NH2; Cpa-Pal-cyclo(D-Cys-D-Trp-Lys-D-Cys)-A4c-Nal-NH2; Cpa-Pal-cyclo(D-Cys-D-Trp-Lys-D-Cys)-Nal-NH2; Pal-cyclo(D-Cys-D-Trp-Lys-D-Cys)-Nal-NH2; Pro-Phe-cyclo(Cys-D-Trp-Lys-D-Cys)-Val-NH2; Pro-Phe-cyclo(D-Cys-D-Trp-Lys-Cys)-Val-NH2; Pip-4-NO2-Phe-cyclo(D-cys-D-Trp-Lys-D-Cys)-Nle-NH2; (G)aeg-Pal-cyclo(D-Cys-D-Trp-Lys-D-Cys)-Thr(Bzl)-(C)aeg-NH2; or (C)aeg-Pal-cyclo(D-Cys-D-Trp-Lys-D-Cys)-Thr (Bzl)-(G)aeg-NH2; Nal-cyclo(D-Cys-Tyr-D-Trp-Lys-Cys)-Nal-NH2; D-Nal-cyclo(D-Cys-Tyr-D-Trp-Lys-Cys)-Nal-NH2; D-Phe-cyclo(Cys-Tyr-D-Trp-Lys-Cys)-Thr-NH2, D-4-NO2-Phe-cyclo(D-Cys-Tyr-D-Trp-Lys-Cys)-Nal-NH2; Ac-D-4-NO2-Phe-cyclo(D-Cys-Tyr-D-Trp-Lys-Cys)-Nal-NH2; D-4-NO2-Phe-Pal-cyclo(D-Cys-Phe (4-O-Bzl)-D-Trp-Lys-Cys)-Tyr-NH2; Cpa-cyclo(D-Cys-Pal-D-Trp-Lys-Cys)-Thr(Bzl)-Tyr-NH2; D-4-NO2-Phe-cyclo(D-Cys-Pal-D-Trp-Lys-Cys)-Thr-Tyr-NH2; D-4-NO2-Phe-cyclo(D-Cys-Pal-D-Trp-Lys-Cys)-Thr(Bzl)-NH2; D-4-NO2-Phe-cyclo (D-Cys-Pal-D-Trp-Lys-D-Cys)-Thr(Bzl)-Tyr-NH2; D-4-NO2-Phe-cyclo(D-Cys-Tyr-D-Trp-Lys-Cys)-Thr(Bzl)-Tyr-NH2; 4-N02-Phe-cyclo(D-Cys-Pal-D-Trp-Lys-Cys)-Thr(Bzl)-Tyr-NH2; D-Nal-cyclo(D-Cys-Pal-D-Trp-Lys-Cys)-Thr(Bzl)-Tyr-NH2; Pro-cyclo(D-Cys-Pal-D-Trp-Lys-Cys)-Thr(Bzl)-Tyr-NH2; Cpa-cyclo(D-Cys-Pal-D-Trp-Lys-Cys)-Thr(Bzl)-Nal-NH2; Ser(Bzl)-cyclo(D-Cys-Pal-D-Trp-Lys-Cys)-Thr-Tyr-NH2; (T)aeg-cyclo(D-Cys-Pal-D-Trp-Lys-D-Cys)-Thr(Bzl)-Tyr-NH2; (A)aeg-cyclo(D-Cys-Pal-D-Trp-Lys-Cys)-Thr(Bzl)-Tyr-NH2; (G)aeg-cyclo(D-Cys-Pal-D-Trp-Lys-Cys)-Thr(Bzl)-Tyr-NH2; (T)aeg-cyclo(D-Cys-4-Pal-D-Trp-Lys-Cys)-Thr(Bzl)-Tyr-NH2; (T)aeg-cyclo(D-Cys-Tyr-D-Trp-Lys-Cys)-Thr(Bzl)-Tyr-NH2; (T)aeg-cyclo(D-Cys-Phe-D-Trp-Lys-Cys)-Thr(Bzl)-Tyr-NH2; (T)aeg-cyclo(D-Cys-(T)aeg-D-Trp-Lys-Cys)-Thr (Bzl)-Tyr-NH2; (T)aeg-cyclo(D-Cys-Pal-D-Trp-Lys-Cys)-Ser(Bzl)-Tyr-NH2; (T)aeg-cyclo(D-Cys-Pal-D-Trp-Lys-Cys)-Phe(4-O-Bzl)-Tyr-NH2; (T)aeg-cyclo(D-Cys-Pal-D-Trp-Lys-Cys)-A5c-Tyr-NH2; (T)aeg-cyclo(D-Cys-Pal-D-Trp-Lys-Cys)-Abu-Tyr-NH2; D-Cpa-cyclo(D-Cys-(T)aeg-D-Trp-Lys-Cys)-Thr(Bzl)-Tyr-NH2; (C)aeg-cyclo(D-Cys-Pal-D-Trp-Lys-D-Cys)-Thr(Bzl)-Tyr-NH2; D-Cpa-c(D-Cys-Pal-D-Trp-Lys-D-Cys)Thr(Bzl)-Tyr-NH2; (T)aeg-c(Pen-Pal-D-Trp-Lys-D-Cys)Thr(Bzl)-Tyr-NH2; (T)aeg-c(D-Cys-Trp-D-Trp-Lys-D-Cys)Thr(Bzl)-Tyr-NH2; (T)aeg-c(D-Cys-Phe-D-Trp-Lys-D-Cys)Thr(Bzl)-Tyr-NH2; (T)aeg-c(D-Cys-Pal-D-Trp-Orn-D-Cys)Thr(Bzl)-Tyr-NH2; (T)aeg-c(D-Cys-Pal-D-Trp-hLys-D-Cys)Thr(Bzl)-Tyr-NH2; (T)aeg-c(D-Cys-Pal-D-Trp-lamp-D-Cys)Thr(Bzl)-Tyr-NH2; (T)aeg-c(D-Cys-Pal-D-Trp-Cha(4-am)-D-Cys) Thr (Bzl)-Tyr-NH2; (T)aeg-c(D-Cys-Pal-D-Trp-Lys-D-Cys)-Ser(Bzl)-Tyr-NH2; (T)aeg-c(D-Cys-Pal-D-Trp-Lys-D-Cys)Thr(Bzl)-D-Tyr-NH2; (T)aeg-c(D-Cys-Pal-D-Trp-Lys-D-Cys) Thr (Bzl)-Trp-NH2; (T) aeg-c (D-Cys-Pal-D-Trp-Lys-D-Pen)Thr(Bzl)-Tyr-NH2; (C)aeg-c(D-Cys-Phe-D-Trp-Lys-D-Cys)Thr(Bzl)-Tyr-NH2; Ina-c(D-Cys-Phe-D-Trp-Lys-D-Cys)-Thr(Bzl)-Tyr-NH2; Mnf-c(D-Cys-Phe-D-Trp-Lys-D-Cys)-Thr(Bzl)-Tyr-NH2; Inp-c(D-Cys-Phe-D-Trp-Lys-D-Cys)-thr(Bzl)-Tyr-NH2; Nua-c(D-Cys-Phe-D-Trp-Lys-D-Cys)-Thr(Bzl)-Tyr-NH2; (T)aeg-Pal-c(D-Cys-D-Trp-Lys-D-Cys)Thr(Bzl)-Tyr-NH2; (T)aeg-Pal-c(D-Cys-D-Trp-Lys-D-Cys)Tyr(Bzl)-Thr-NH2; (C)aeg-Phe-c(D-Cys-D-Trp-Lys-D-Cys) Thr(Bzl)-Tyr-NH2; or (T)aeg-D-Trp-c(D-Cys-Pal-Lys-D-Cys)Thr(Bzl)-Leu-NH2; Hca-cyclo(D-Cys-Tyr-D-Trp-Lys-Cys)-Nal-NH2; Ac-Nal-cyclo(D-Cys-Tyr-D-Trp-Lys-Cys)-Nal-NH2; Ac-D-Phe-cyclo(D-Cys-Tyr-D-Trp-Lys-Cys)-Nal-NH2; Ac-D-Nal-cyclo(D-Cys-Tyr-D-Trp-Lys-Cys)-Nal-NH2; D-Phe-cyclo(D-Cys-Tyr-D-Trp-Lys-Cys)-Nal-NH2; Nal-cyclo(D-Cys-Tyr-D-Trp-Lys-Cys)-Nal-NH2; D-Nal-cyclo(D-Cys-Tyr-D-Trp-Lys-Cys)-Nal-NH2; D-Phe-cyclo(Cys-Tyr-D-Trp-Lys-Cys)-Thr-NH2; D-4-NO2-Phe-cyclo(D-Cys-Tyr-D-Trp-Lys-Cys)-Nal-NH2; Ac-D-4-NO2-Phe-cyclo(D-Cys-Tyr-D-Trp-Lys-Cys)-Nal-NH2; D-4-NO2-Phe-Pal-cyclo(D-Cys-Phe(4-O-Bzl)-D-Trp-Lys-Cys)-Tyr-NH2; D-4-NO2-Phe-cyclo(D-Cys-Pal-D-Trp-Lys-Cys)-Thr(Bzl)-Tyr-NH2; Cpa-cyclo(D-Cys-Pal-D-Trp-Lys-Cys)-Thr(Bzl)-Tyr-NH2; D-4-NO2-Phe-cyclo(D-Cys-Pal-D-Trp-Lys-Cys)-Thr(Bzl)-NH2; D-4-NO2-Phe-cyclo(D-Cys-Pal-D-Trp-Lys-D-Cys)-Thr (Bzl)-Tyr-NH2; D-4-NO2-Phe-cyclo(D-Cys-Tyr-D-Trp-Lys-Cys)-Thr(Bzl)-Tyr-NH2; 4-N02-Phe-cyclo(D-Cys-Pal-D-Trp-Lys-Cys)-Thr(Bzl)-Tyr-NH2; D-Nal-cyclo(D-Cys-Pal-D-Trp-Lys-Cys)-Thr (Bzl)-Tyr-NH2; Pro-cyclo(D-Cys-Pal-D-Trp-Lys-Cys)-Thr(Bzl)-Tyr-NH2; Cpa-cyclo(D-Cys-Pal-D-Trp-Lys-Cys)-Thr(Bzl)-Nal-NH2; Ser(Bzl)-cyclo(D-Cys-Pal-D-Trp-Lys-Cys)-Thr-Tyr-NH2; (T)aeg-cyclo(D-Cys-Pal-D-Trp-Lys-Cys)-Thr(Bzl)-Tyr-NH2; (C)aeg-cyclo(D-Cys-Pal-D-Trp-Lys-Cys)-Thr(Bzl)-Tyr-NH2; Aic-cyclo(D-Cys-Pal-D-Trp-Lys-Cys)-Thr(Bzl)-Tyr-NH2; (C(z))aeg-cyclo(D-Cys-Pal-D-Trp-Lys-Cys)-Thr(Bzl)-Tyr-NH2; (A(z))aeg-cyclo(D-Cys-Pal-D-Trp-Lys-Cys)-Thr(Bzl)-Tyr-NH2; (T)aeg-cyclo(D-Cys-Pal-D-Trp-Lys-D-Cys)-Thr(Bzl)-Tyr-NH2; (A)aeg-cyclo(D-Cys-Pal-D-Trp-Lys-Cys)-Thr(Bzl)-Tyr-NH2; (G)aeg-cyclo(D-Cys-Pal-D-Trp-Lys-Cys)-Thr(Bzl)-Tyr-NH2; (T)aeg-cyclo(D-Cys-4-Pal-D-Trp-Lys-Cys)-Thr(Bzl)-Tyr-NH2; (T)aeg-cyclo(D-Cys-Tyr-D-Trp-Lys-Cys)-Thr (Bzl)-Tyr-NH2; (T)aeg-cyclo(D-Cys-Phe-D-Trp-Lys-Cys)-Thr(Bzl)-Tyr-NH2; (T)aeg-cyclo(D-Cys-(T)aeg-D-Trp-Lys-Cys)-Thr(Bzl)-Tyr-NH2; (T)aeg-cyclo(D-Cys-Pal-D-Trp-Lys-Cys)-Ser(Bzl)-Tyr-NH2; (T)aeg-cyclo(D-Cys-Pal-D-Trp-Lys-Cys)-Phe(4-O-Bzl)-Tyr-NH2; (T)aeg-cyclo(D-Cys-Pal-D-Trp-Lys-Cys)-A5c-Tyr-NH2; (T)aeg-cyclo(D-Cys-Pal-D-Trp-Lys-Cys)-Abu-Tyr-NH2; D-Cpa-cyclo(D-Cys-(T)aeg-D-Trp-Lys-Cys)-Thr(Bzl)-Tyr-NH2; (T)aeg-cyclo(D-Cys-Pal-D-Trp-Lys-D-Cys)-Thr(Bzl)-p-Me-Phe-NH2; Ac-(T)aeg-cyclo(D-Cys-Pal-D-Trp-Lys-D-Cys)-Thr(Bzl)-Tyr-NH2; (T)aeg-cyclo(D-Cys-Pal-D-Trp-Lys-D-Cys)-Nal-NH2; D-Cpa-cyclo(D-Cys-Pal-D-Trp-Lys-D-Cys)-Nal-NH2; (A)aeg-cyclo(D-Cys-Pal-D-Trp-Lys-D-Cys)-Thr(Bzl)-Tyr-NH2; (C)aeg-cyclo(D-Cys-Pal-D-Trp-Lys-D-Cys)-Thr(Bzl)-Tyr-NH2; (C)aeg-c(D-Cys-Pal-D-Trp-Lys-D-Cys)-Thr(Bzl)-Tyr-NH2; D-Cpa-c(D-Cys-Pal-D-Trp-Lys-D-Cys)Thr(Bzl)-Tyr-NH2; (T)aeg-c(Pen-Pal-D-Trp-Lys-D-Cys)Thr(Bzl)-Tyr-NH2; (T)aeg-c(D-Cys-Trp-D-Trp-Lys-D-Cys)Thr(Bzl)-Tyr-NH2; (T)aeg-c(D-Cys-Phe-D-Trp-Lys-D-Cys)Thr(Bzl)-Tyr-NH2; (T)aeg-c(D-Cys-Pal-D-Trp-Orn-D-Cys)Thr(Bzl)-Tyr-NH2; (T)aeg-c(D-Cys-Pal-D-Trp-hLys-D-Cys)Thr(Bzl)-Tyr-NH2, (T)aeg-c(D-Cys-Pal-D-Trp-lamp-D-Cys)Thr(Bzl)-Tyr-NH2; (T)aeg-c(D-Cys-Pal-D-Trp-Cha(4-am)-D-Cys)Thr(Bzl)-Tyr-NH2; (T)aeg-c(D-Cys-Pal-D-Trp-Lys-D-Cys)-Ser(Bzl)-Tyr-NH2; (T)aeg-c (D-Cys-Pal-D-Trp-Lys-D-Cys)Thr (Bzl)-D-Tyr-NH2; (T)aeg-c (D-Cys-Pal-D-Trp-Lys-D-Cys)Thr(Bzl)-Trp-NH2; (T)aeg-c(D-Cys-Pal-D-Trp-Lys-D-Pen)Thr(Bzl)-Tyr-NH2; (C)aeg-c(D-Cys-Phe-D-Trp-Lys-D-Cys)Thr(Bzl)-Tyr-NH2; Ina-c(D-Cys-Phe-D-Trp-Lys-D-Cys)-Thr(Bzl)-Tyr-NH2; Mnf-c(D-Cys-Phe-D-Trp-Lys-D-Cys)-Thr(Bzl)-Tyr-NH2; Inp-c(D-Cys-Phe-D-Trp-Lys-D-Cys)-Thr(Bzl)-Tyr-NH2; Nua-c(D-Cys-Phe-D-Trp-Lys-D-Cys)-Thr(Bzl)-Tyr-NH2; (T)aeg-Pal-c(D-Cys-D-Trp-Lys-D-Cys)Thr(Bzl)-Tyr-NH2; (T)aeg-Pal-c(D-Cys-D-Trp-Lys-D-Cys)Tyr(Bzl)-Thr-NH2; (C)aeg-Phe-c(D-Cys-D-Trp-Lys-D-Cys)Thr(Bzl)-Tyr-NH2; or (T)aeg-D-Trp-c(D-Cys-Pal-Lys-D-Cys)Thr(Bzl)-Leu-NH2; cyclo(Trp-D-Trp-Lys-Phe(4-O-Bzl)-Phe-(T)aeg); cyclo(Trp-D-Trp-Lys-Pal-Phe-(T)aeg); cyclo(Phe-Phe-D-Trp-Lys-Thr-(T)aeg); or H-β-D-Nal-Cys-Tyr-D-Trp-Lys-Val-Cys-Thr-NH2 (also known as lanreotide) cyclo(Pro-Phe-D-Trp-Lys-Thr-Phe), cyclo(N-Me-Ala-Tyr-D-Trp-Lys-Val- Phe); D-beta-Nal-Cys-Tyr-D-Trp-Lys-Val-Cys-Thr-$NH_2$; D-Phe-Cys-Tyr-D-Trp-Lys-Val-Cysbeta-Nal-$NH_2$; D-Phe-Cys-Tyr-D-Trp-Lys-α-Aminobutyric acid-Cys-Thr-$NH_2$; pentafluoro-D-Phe-Cys-Tyr-D-Trp-Lys-Val-Cys-Thr-$NH_2$; N-Ac-D-beta-Nal-Cys-Tyr-D-Trp-Lys-Val-Cys-Thr-$NH_2$; D-beta-Nal~Cys-pentafluoro-Phe-D-Trp-Lys-Val-Cys-Thr-

$NH_2$ ; D-/3-Nal-Cys-Tyr-D-Trp-Lys-Val-Cys-beta-Nal-$NH_2$; D-Phe-Cys-/3-Nal-D-Trp-Lys-Val-Cys-Thr-$NH_2$; D-beta-Nal-Cys-Tyr-D-Trp-Lys-α-aminobutyric acid-Cys-Thr-$NH_2$; D-p-Cl-Phe-Cys-Tyr-D-Trp-Lys-α-aminobutyric acid-Cys-Thr-$NH_2$; acetyl-D-p-Cl-Phe-Cys-Tyr-D-Trp-Lys-α-aminobutyric acid-Cys-Thr-$NH_2$; cyclo(Pro-Phe-D-Trp- Lys-Thr-Phe); cyclo(N-Me-Ala-Tyr-D-Trp- Lys-Val-Phe); D-beta-Nal-Cys-Tyr-D-Trp-Lys-Val-Cys-Thr-$NH_2$; D-Phe-Cys-Tyr-D-Trp-Lys-Val-Cys-Trp-$NH_2$; D-Phe-Cys-Phe-D-Trp-Lys-Thr-Cys-Thr(ol); D-p-Cl-Phe-Cys-Tyr-D-Trp-Lys-Val-Cys-Thr-$NH_2$; D-Phe-Cys-Tyr-D-Trp-Lys-Val-Cys-beta-Nal; H2-beta-Nal-D-Cys-Tyr-D-Trp-Lys-Val-Cys-beta-Nal-NH2; (H)(CH3CO)-beta-Nal-D-Cys-Tyr-D-Trp-Lys-Val-Cys-beta-Nal-NH2; (H)-(4-(2-hydroxyethyl)-1-piperazinylacetyl)-beta-Nal-D-Cys-Tyr-D-Trp-Lys-Val-Cys-beta-Nal-NH2; (H)-(4-(2-hydroxyethyl)-1-piperizineethanesulfonyl)-beta-Nal-D-Cys-Tyr-D-Trp-Lys-Val-Cys-beta-Nal-NH2; H2-beta-Nal-D-Cys-Pal-D-Trp-Lys-Val-Cys-beta-Nal-NH2; (H)(CH3CO)-beta-Nal-D-Cys-Pal-D-Trp-Lys-Val-Cys-beta-Nal-NH2; (H)-(4-(2-hydroxyethyl)-1-piperazinylacetyl)-beta-Nal-D-Cys-Pal-D-Trp-Lys-Val-Cys-beta-Nal-NH2; (H)-(4-(2-hydroxyethyl)-1-piperizineethanesulfonyl)-beta-Nal-D-Cys-Pal-D-Trp-Lys-Val-Cys-beta-Nal-NH2; H2-beta-Nal-D-Cys-Tyr-D-Trp-Lys-Val-Cys-Thr-NH2; (H)(CH3CO)-beta-Nal-D-Cys-Tyr-D-Trp-Lys-Val-Cys-Thr-NH2; (H)(4-(2-hydroxyethyl)-1-piperazinylacetyl)-beta-Nal-D-Cys-Tyr-D-Trp-Lys-Val-Cys-Thr-NH2; (H)(4-(2-hydroxyethyi)-1-piperizineethanesulfonyl)-beta-Nal-D-Cys-Tyr-D-Trp-Lys-Val-Cys-Thr-NH2; H2-beta-Nal-D-Cys-Pal-D-Trp-Lys-Val-Cys-Thr-NH2; (H)(CH3CO)-beta-Nal-D-Cys-Pal-D-Trp-Lys-Val-Cys-Thr-NH2; (H)(4-(2-hydroxyethyl)-1-piperazinylacetyl)-beta-Nal-D-Cys-Pal-D-Trp-Lys-Val-Cys-Thr-NH2; (H)(4-(2-hydroxyethyl)-1-piperizineethanesulfonyl)-0-Nal-D-Cys-Pal-D-Trp-Lys-Val-Lys-Thr-NH2; H2-Phe-D-Cys-Tyr-D-Trp-Lys-Val-Cys-beta-Nal-NH2; (H)(CH3CO)Phe-D-Cys-Tyr-D-Trp-Lys-Val-Cys-beta-Nal-NH2; (H)(4-(2-hydroxyethyl)-1-piperazinylacetyl)-Phe-D-Cys-Tyr-D-Trp-Lys-Val-Cys-beta-Nal-NH2; (H)(4-(2-hydroxyethyi)-1-piperizineethanesuifonyl)-Phe-D-Cys-Tyr-D-Trp-Lys-Val-Cys-beta-Nal-NH2; H2-Phe-D-Cys-Pal-D-Trp-Lys-Val-Cys-beta-Nal-NH2; (H)(CH3CO)Phe-D-Cys-Pal-D-Trp-Lys-Val-Cys-beta-Nal-NH2; (H)(4-(2-hydroxyethyl)-1-piperazinylacetyl)-Phe-D-Cys-Pal-D-Trp-Lys-Val-Cys-beta-Nal-NH2; (H)(4-(2-hydroxyethyl)-1-piperizineethanesuifonyi)-Phe-D-Cys-Pal-D-Trp-Lys-Val-Cys-beta-Nal-NH2; H2-Phe-D-Cys-Pal-D-Trp-Lys-Val-Cys-Thr-NH2; (H)(CH3CO)-Phe-D-Cys-Pal-D-Trp-Lys-Val-Cys-Thr-NH2; (H)(4-(2-hydroxyethyl)-1-piperazinylacetyl)-Phe-D-Cys-Pal-D-Trp-Lys-Val-Cys-Thr-NH2; (H)(4-(2-hydroxyethyl)-1-piperizineethanesulfonyl)-Phe-D-Cys-Pal-D-Trp-Lys-Val-Cys-Thr-NH2; H2-beta-Nal-D-Cys-Tyr-D-Trp-Lys-Thr-Cys-beta-Nal-NH2; (H)(CH3CO)-beta-Nal-D-Cys-Pal-D-Trp-Lys-Thr-Cys-beta-Nal-NH2; (H)(4-(2-hydroxyethyl)-1-piperazinylacetyl)-beta-Nal-D-Cys-Tyr-D-Trp-Lys-Thr-Cys-beta-Nal-NH2; (H)(4-(2-hydroxyethyl)-1-piperizineethanesulfonyl)-beta-Nal-D-Cys-Tyr-D-Trp-Lys-Thr-Cys-beta-Nal-NH2; H2-beta-Nal-D-Cys-Pal-D-Trp-Lys-Thr-Cys-beta-Nal-NH2; (H)(CH3C0)-beta-Nal-D-Cys-Pal-D-Trp-Lys-Thr-Cys-beta-Nal-NH2; (H)(4-(2-hydroxyethyl)-1-piperazinylacetyl)-beta-Nal-D-Cys-Pal-D-Trp-Lys-Thr-Cys-beta-Nal-NH2; (H)(4-(2-hydroxyethyl)-1-piperizineethanesulfonyl)-beta-Nal-D-Cys-Pal-D-Trp-Lys-Thr-Cys-beta-Nal-NH2; H2-beta-Nal-D-Cys-Tyr-D-Trp-Lys-Thr-Cys-Thr-NH2; H(CH3CO)-beta-Nal-D-Cys-Tyr-D-Trp-Lys-Thr-Cys-Thr-NH2; (H)(4-(2-hydroxyethyl)-1-piperazinylacetyl)-beta-Nal-D-Cys-Tyr-D-Trp-Lys-Thr-Cys-Thr-NH2; (H)(4-(2-hydroxyethyl)-1-piperizineethanesulfonyl)-beta-Nal-D-Cys-Tyr-D-Trp-Lys-Thr-Cys-Thr-NH2; H2-beta-Nal-D-Cys-Pal-D-Trp-Lys-Thr-Cys-Thr-NH2; (H)(CH3CO)-beta-Nal-D-Cys-Pal-D-Trp-Lys-Thr-Cys-Thr-NH2; (H)(4-(2-hydroxyethyl)-1-piperazinylacetyl)-beta-Nal-D-Cys-Pal-D-Trp-Lys-Thr-Cys-Thr-NH2; (H)(4-(2-hydroxyethyl)-1-piperizineethanesulfonyl)-beta- Nal-D-Cys-Pal-D-Trp-Lys-Thr-Cys-Thr-NH2; H2-Phe-D-Cys-Tyr-D-Trp-Lys-Thr-Cys-beta-Nal-NH2; (H)(CH3CO)Phe-D-Cys-Tyr-D-Trp-Lys-Thr-Cys-beta-Nal-NH2; (H)(4-(2-hydroxyethyl)-1-piperazinylacetyl)Phe-D-Cys-Tyr-D-Trp-Lys-Thr-Cys-beta-Nal-NH2; (H)(4-(2-hydroxyethyl)-1-piperizineethanesulfonyl)Phe-D-Cys-Tyr-D-Trp-Lys-Thr-Cys-beta-Nal-NH2; H2-Phe-D-Cys-Pal-D-Trp-Lys-Thr-Cys-beta-Nal-NH2; (H)(CH3CO)Phe-D-Cys-Pal-D-Trp-Lys-Thr-Cys-beta-Nal-NH2; (H)(4-(2-hydroxyethyl)-1-piperazinylacetyl)Phe-D-Cys-Pal-D-Trp-Lys-Thr-Cys-beta-Nal-NH2; (H)(4-(2-hydroxyethyi)-1-piperizineethanesulfonyl)Phe-D-Cys-Pal-D-Trp-Lys-Thr-Cys-beta-Nal-NH2; H2-Phe-D-Cys-Tyr-D-Trp-Lys-Thr-Cys-Thr-NH2; (H)(CH3CO)Phe-D-Cys-Tyr-D-Trp-Lys-Thr-Cys-Thr-NH2; (H)(4-(2-hydroxyethyl)-1-piperazinylacetyl)Phe-D-Cys-Tyr-D-Trp-Lys-Thr-Cys-Thr-NH2; (H)(4-(2-hydroxyethyl)-1-piperizineethanesuifonyl)Phe-D-Cys-Tyr-D-Trp-Lys-Thr-Cys-Thr-NH2; H2-Phe-D-Cys-Pal-D-Trp-Lys-Thr-Cys-Thr-NH2; (H)(CH3CO)-Phe-D-Cys-Pal-D-Trp-Lys-Thr-Cys-Thr-NH2; (H)(4-(2-hydroxyethyl)-1-piperazinylacetyl)Phe-D-Cys-Pal-D-Trp-Lys-Thr-Cys-Thr-NH2; (H)(4-(2-hydroxyethyl)-1-piperizineethanesulfonyl)Phe-D-Cys-Pal-D-Trp-Lys-Thr-Cys-Thr-NH2; H2-beta-Nal-D-Cys-Tyr-D-Trp-Lys-Abu-Cys-beta-Nal-NH2; H2-Phe-D-Cys-Tyr-D-Trp-Lys-Abu-Cys-beta-Nal-NH2; H2-beta-Nal-D-Cys-Pal-D-Trp-Lys-Abu-Cys-beta-Nal-NH2; H2-Phe-D-Cys-Pal-D-Trp-Lys-Abu-Cys-beta-Nal-NH2; H2-beta-Nal-D-Cys-Tyr-D-Trp-Lys-Abu-Cys-Thr-NH2; H2-Phe-D-Pen-Tyr-D-Trp-Lys-Val-Pen-beta-Nal-NH2; H2-Phe-D-Pen-Pal-D-Trp-Lys-Thr-Pen-Thr-NH2; H2-Dip-D-Cys-Pal-D-Trp-Lys-Val-Cys-Dip-NH2; H2-F5-Phe-D-Cys-His-D-Trp-Lys-Val-Cys-F5-Phe-NH2; H2-Dip-D-Cys-Pal-D-Trp-Lys-Val-Cys-beta-Nal-NH2; H2-m-F-Phe-D-Cys-Pal-D-Trp-Lys-Val-Cys-m-F-Phe-NH2 H2-o-F-Phe-D-Cys-Pal-D-Trp-Lys-Val-Cys-o-F-Phe-NH2; H2-p-F-Phe-D-Cys-Pal-D-Trp-Lys-Val-Cys-p-F-Phe-NH2; H2-F5-Phe-D-Cys-Pal-D-Trp-Lys-Val-Cys-F5-Phe-NH2; H2-F5-Phe-D-Cys-2-Pal-D-Trp-Lys-Val-Cys-F5-Phe-NH2; H2-beta-Nal-D-Cys-His-D-Trp-Lys-Val-Cys-D-Dip-NH2; H2-Dip-D-Cys-His-D-Trp-Lys-Val-Cys-beta-Nal-NH2; H2-Dip-D-Cys-His-D-Trp-Lys-Val-Cys-Dip-NH2; H2-beta-Nal-D-Cys-His-D-Trp-Lys-Val-Cys-beta-Nal-NH2; H2-Trp-D-Cys-Tyr-D-Trp-Lys-Val-Cys-D-beta-Nal-NH2; H2-beta-Nal-D-Cys-Tyr-D-Trp-Lys-Val-Cys-D-beta-Nal-NH2; H2-beta-Nal-D-Cys-Pal-D-Trp-Lys-Val-Cys-D-p-F-Phe-NH2; H2-beta-Nal-D-Cys-Pal-D-Trp-Lys-Tle-Cys-beta-Nal-NH2; H2-p-F-Phe-D-Cys-Pal-D-Trp-Lys-Val-Cys-beta-Nal-NH2; H2-beta-Nal-D-Cys-Pal-D-Trp-Lys-Nle-Cys-beta-Nal-NH2; H2-beta-Nal-D-Cys-Pal-D-Trp-Lys-Ile-Cys-beta-Nal-NH2; H2-beta-Nal-D-Cys-Pal-D-Trp-Lys-Gly-Cys-beta-Nal-NH2; H2-beta-Nal-D-Cys-Pal-D-Trp-Lys-Ala-Cys-beta-Nal-NH2; H2-beta-Nal-D-Cys-Pal-D-Trp-Lys-Leu-Cys-beta-Nal-NH2; H2-Bip-D-Cys-Tyr-D-Trp-Lys-Ile-Cys-Bip-NH2; H2-p-F-Phe-D-Cys-His-D-Trp Lys-Val-Cys-p-F-Phe-NH2; H2-Npa-D-Cys-Pal-D-Trp-Lys-Val-Cys-Tyr-NH2; H2-m-F-Phe-D-Cys-His-D-Trp-Lys-Val-Cys-m-F-Phe-NH2; H2-o-F-Phe-D-Cys-His-D-Trp-Lys-Val-Cys-o-F-Phe-NH2; H2-beta-Nal-D-Cys-Pal-D-Trp-Lys-Val-Cys-Dip-NH2; H2-Cpa-D-Cys-Pal-D-Trp-Lys-Val-Cys-Cpa-NH2; H2-Igl-D-Cys-Pal-D-Trp-Lys-Val-Cys-Igl-NH2; H2-beta-Nal-D-Cys-Pal-D-Trp-Lys-Val-Cys-D-Dip-NH2; H2-beta-Nal-D-Cys-3-I-Tyr-D-Trp-Lys-Val-Cys-beta-Nal-NH2; H2-p-CN-Phe-D-Cys-Pal-D-Trp-Lys-Val-Cys-p-CN-Phe-NH2; H2-beta-Nal-D-Cys-Tyr-D-Trp-Lys-Val-Cys-D-Dip-NH2; H2-beta-Nal-D-Cys-Bta-D-Trp-Lys-Val-Cys-beta-Nal-NH2; H2-p-F-Phe-D-Cys-Pal-D-Trp-Lys-Tle-Cys-beta-Nal-NH2; H2-Bpa-D-Cys-Pal-D-Trp-Lys-Val-Cys-Bpa-NH2; H2-Iph-D-Cys-Pal-D-Trp-Lys-Val-Cys-Iph-NH2; H2-Trp-D-Cys-Pal-D-Trp-Lys-Tle-Cys-beta-Nal-NH2; H2-p-Cl-Phe-D-Cys-Pal-D-Trp-Lys-Val-Cys-beta-Nal-NH2; H2-p-Cl-Phe-D-Cys-Pal-D-Trp-Lys-Tle-Cys-beta-Nal-NH2; H2-p-Cl-Phe-D-Cys-Pal-D-Trp-Lys-Tle-Cys-p-Cl-Phe-NH2; H2-p-Cl-Phe-D-Cys-Pal-D-Trp-Lys-Cha-Cys-p-Cl-Phe-NH2; H2-p-Cl-Phe-D-Cys-Tr(I)-D-Trp-Lys-Val-Cys-p-Cl-Phe-NH2; H2-p-Cl-Phe-D-Cys-Tyr(I)-D-Trp-Lys-Val-Cys-beta-Nal-NH2; H2-p-Cl-Phe-D-Cys-Tyr(I)-D-Trp-Lys-Tle-Cys-beta-Nal-NH2; H2-p-F-Phe-D-Cys-Tyr(I)-D-Trp-Lys-Val-Cys-beta-Nal-NH2; H2-p-F-Phe-D-Cys-Tyr(I)-D-Trp-Lys-Tle-Cys-beta-Nal-NH2; H2-beta-Nal-D-Cys-Tyr-D-Trp-Lys-Abu-Cys-beta-Nal-NH2; (H)(CH3CO)-beta-Nal-D-Cys-Tyr-D-Trp-Lys-Abu-Cys-beta-Nal-NH2; H2-p-N02-Phe-D-Cys-Tyr-D-Trp-Lys-Abu-Cys-beta-Nal-NH2; (H)(CH3CO)-beta-Nal-D-Cys-Tyr-D-Trp-Lys-Abu-Cys-beta-Nal-NH2; H2-p-N02-Phe-D-Cys-Tyr(Bzl)-D-Trp-Lys-Thr(Bzl)-Cys-Nal-NH2; (H)(4-(2-hydroxyethyl)-1-piperazinylacetyl)-p-NO2-Phe-D-Cys-Tyr(Bzl)-D-Trp-Lys-Thr(Bzl)-Cys-beta-Nal-NH2; (H)(4-(2-hydroxyethyl)-1-piperazinylacetyl)-p-NO2-Phe-D-Cys-Tyr-D-Trp-Lys-Thr-Cys-Tyr-NH2; H2-p-NO2-Phe-D-Cys-Tyr-D-Trp-Lys-Val-Cys-beta-Nal-NH2; (H)(4-(2-hydroxyethyl)-1-piperazinylacetyl)-p-NO2-Phe-D-Cys-Tyr-D-Trp-Lys-Val-Cys-P-Nal-NH2; (H)(4-(2-hydroxyethyl)-1-piperazinylacetyl)-beta-Nal-Phe-D-Cys-Tyr-D-Trp-Lys-Val-Cys-beta-Nal-NH2; H2-beta-Nal-D-Cys-Tyr(Bzl)-D-Trp-Lys-Thr(Bzl)-Cys-beta-Nal-NH2; (H)(4-(2-hydroxyethyi)-1-piperazinylacetyl)-beta-Nal-D-Cys-Tyr(Bzl)-D-Trp-Lys-Thr(Bzl)-Cys-Tyr(Bzl)-NH2; H2-D-Phe-D-Pen-Tyr-D-Trp-Lys-Val-Cys-Thr-NH2; H2-D-beta-Nal-D-Cys-Tyr-D-Trp-Lys-Val-Cys-Thr-NH2; H2-D-beta-Nal-D-Cys-Tyr-D-Trp-Lys-Val-Cys-beta-Nal-NH2; H2-D-beta-Nal-D-Cys-Tyr-D-Trp-Lys-Thr-Cys-beta-Nal-NH2; H2-D-Phe-D-Cys-Pal-D-Trp-Lys-Thr-Cys-Thr-NH2; H2-D-Phe-D-Cys-Tyr-D-Trp-Lys-Abu-Cys-Thr-NH2; H2-D-beta-Nal-D-Cys-Tyr-D-Trp-Lys-Abu-Cys-Thr-NH2; H2-D-beta-Nal-D-Cys-Tyr-D-Trp-Lys-Val-Cys-D-beta-Nal-NH2; H2-D-p-F-Phe-D-Cys-Pal-D-Trp-Lys-Val-Cys-D-p-F-Phe-NH2; H2-D-Bip-D-Cys-Tyr-D-Trp-Lys-Val-Cys-beta-Nal-NH2; H2-D-Dip-D-Cys-Pal-D-Trp-Lys-Val-Cys-beta-Nal-NH2; H2-D-p-F-Phe-D-Cys-Pal-D-Trp-Lys-Tle-Cys-beta-Nal-NH2; H2-D-p-Cl-Phe-D-Cys-Pal-D-Trp-Lys-Tle-Cys-p-Cl-Phe-NH2; p-N02-D-Phe-D-Cys-Pal-D-Trp-Lys-Thr(Bzl)-Cys-Tyr(Bzl)-NH2; p-N02-D-Phe-D-Cys-Tyr(Bzl)-D-Trp-Lys-Val-Cys-Tyr(Bzl)-NH2; (H)(4-(2-hydroxyethyi)-1-piperazinylacetyl)-p-NO2-P-Phe-D-Cys-Pal-D-Trp-Lys-Thr(Bzl)-Cys-Tyr(Bzl)-NH2; (H)(4-(2-hydroxyethyl)-1-piperazinylacetyl)-p-NO2-P-Phe-D-Cys-Tyr(Bzl)-D-Trp-Lys-Val-Cys-Tyr(Bzl)-NH2; (H) (5-phenylpropionyl)-D-Cys-Tyr-D-Trp-Lys-Val-Cys-beta-Nal-NH2; (H)(3-phenylpropionyl)-D-Cys-Pal-D-Trp-Lys-Val-Cys-beta-Nal-NH2; (H)(3-phenylpropionyl)-D-Cys-Tyr-D-Trp-Lys-Thr-Cys-beta-Nal-NH2; (H)(3-phenylpropionyl)-D-Cys-Pal-D-Trp-Lys-Thr-Cys-beta-Nal-NH2; (H)(3-phenylpropionyl)-D-Cys-Tyr-D-Trp-Lys-Val-Cys-Thr-NH2; (H)(3-phenylpropionyl)-D-Cys-Pal-D-Trp-Lys-Val-Cys-Thr-NH2; (H)(3-phenylpropionyl)-D-Cys-Tyr-D-Trp-Lys-Thr-Cys-Thr-NH2; (H)(3-phenylpropionyl)-D-Cys-Pal-D-Trp-Lys-Thr-Cys-Thr-NH2; (H)(3-[2-naphthyl]propionyl)-D-Cys-Tyr-D-Trp-Lys-Val-Cys-beta-Nal-NH2; (H)(3-[2-naphthyl]propionyl)-D-Cys-Pal-D-Trp-Lys-Val-Cys-beta-Nal-NH2; (H)(3-[2-naphthyl]propionyl)-D-Cys-Tyr-D-Trp-Lys-Thr-Cys-beta-Nal-NH2; (H)(3-[2-naphthyl]propionyl)-D-Cys-Pal-D-Trp-Lys-Thr-Cys-beta-Nal-NH2; (H)(3-[2-naphthyl]propionyl)-D-Cys-Tyr-D-Trp-Lys-Val-Cys-Thr-NH2; (H)(3-[2-naphthyl]propionyl)-D-Cys-Pal-D-Trp-Lys-Val-Cys-Thr-NH2; (H)(3-[2-naphthyl]propionyl)-D-Cys-Tyr-D-Trp-Lys-Thr-Cys-Thr-NH2; (H)(3-[2-naphthyl]propionyl)-D-Cys-Pal-D-Trp-Lys-Thr-Cys-Thr-NH2; (H)(3-[p-hydroxyphenyl])-D-Cys-Tyr-D-Trp-Lys-Val-Cys-beta-Nal-NH2; (H)(3-naphthyl]propionyl)-D-Cys-Tyr-D-Trp-Lys-Abu-Cys-beta-Nal-NH2; (H)(3-naphthyl]propionyl)-D-Cys-Tyr-D-Trp-Lys-Abu-Cys-Thr-NH2; (H)(3-phenylylpropionyl)-D-Cys-Tyr-D-Trp-Lys-Abu-Cys-beta-Nal-NH2; or (H)(3-phenylylpropionyl)-D-Cys-Tyr-D-Trp-Lys-Abu-Cys-Thr-NH2; H2-beta-Nal-D-Cys-Tyr-D-Trp-Lys-Val-Cys-2R,3R-(2-hydroxymethyl)-3-hydroxy)propylamide; (H)(CH3CO)-beta-Nal-D-Cys-Tyr-D-Trp-Lys-Val-Cys-2R,3R-(2-hydroxymethyl)-3-hydroxy)propylamide; (H)(4-(2-hydroxyethyl)-1-piperazinylacetyl)-beta-Nal-D-Cys-Tyr-D-Trp-Lys-Val-Cys-2R,3R-(2-hydroxymethyl)-3-hydroxy)propylamide; (H)(4-(2-hydroxyethyl)-1-piperizineethanesuifonyi)-beta-Nal-D-Cys-Tyr-D-Trp-Lys-Val-Cys-2R,3R-(2-hydroxymethyl)-3-hydroxy)propylamide; H2-beta-Nal-D-Cys-Pal-D-Trp-Lys-Val-Cys-2R,3R-(2-hydroxymethyl)-3-hydroxy)propylamide; (H)(CH3CO)-beta-Nal-D-Cys-Pal-D-Trp-Lys-Val-Cys-2R,3R-(2-hydroxymethyl)-3-hydroxy)propylamide; (H)(4-(2-hydroxyethyl)-1-piperazinylacetyl)-beta-Nal-D-Cys-Pal-D-Trp-Lys-Val-Cys-2R,3R-(2-hydroxymethyl)-3-hydroxy)propylamide; (H)(4-(2-hydroxyethyi)-1-piperizineethanesuifonyi)-beta-Nal-D-Cys-Pal-D-Trp-Lys-Val-Cys-2R,3R-(2-hydroxymethyl)-3-hydroxy)propylamide; H2-beta-Nal-D-Cys-Tyr-D-Trp-Lys-Thr-Cys-2R,3R-(2-hydroxymethyl)-3-hydroxy)propylamide; (H)(CH3CO)-beta-Nal-D-Cys-Tyr-D-Trp-Lys-Thr-Cys-2R,3R-(2-hydroxymethyl)-3-hydroxy)propylamide; (H)(4-(2-hydroxyethyl)-1-piperazinylacetyl)-beta-Nal-D-Cys-Tyr-D-Trp-Lys-Thr-Cys-2R,3R-(2-hydroxymethyl)-3-hydroxy)propylamide; (H)(4-(2-hydroxyethyl)-1-piperizineethanesuifonyi)-beta-Nal-D-Cys-Tyr-D-Trp-Lys-Thr-Cys-2R,3R-(2-hydroxymethyl)-3-hydroxy)propylamide; H2-beta-Nal-D-Cys-Pal-D-Trp-Lys-Thr-Cys-2R,3R-(2-hydroxymethyl)-3-hydroxy)propylamide; (H)(CH3CO)-beta-Nal-D-Cys-Pal-D-Trp-Lys-Thr-Cys-2R,3R-(2-hydroxymethyl)-3-hydroxy)propylamide; (H)(4-(2-hydroxyethyl)-1-piperazinylacetyl)-beta-Nal-D-Cys-Pal-D-Trp-Lys-Thr-Cys-2R,3R-(2-hydroxymethyl)-3-hydroxy)propylamide; (H)(4-(2-hydroxyethyl)-1-piperizineethanesuifonyl)-beta-Nal-D-Cys-Pal-D-Trp-Lys-Thr-Cys-2R,3R-(2-hydroxymethyl)-3-hydroxy)propylamide; H2-Phe-D-Cys-Tyr-D-Trp-Lys-Val-Cys-2R,3R-(2-hydroxymethyl)-3-hydroxy)propylamide; (H)(CH3CO)Phe-D-Cys-Tyr-D-Trp-Lys-Val-Cys-2R,3R-(2-hydroxymethyl)-3-hydroxy)propylamide; (H)(4-(2-hydroxyethyl)-1-piperazinylacetyl)Phe-D-Cys-Tyr-D-Trp-Lys-Val-Cys-2R,3R-(2-hydroxymethyl)-3-hydroxy)propylamide; (H)(4-(2-hydroxyethyl)-1-piperizineethanesuifonyl) Phe-D-Cys-Tyr-D-Trp-Lys-Val-Cys-2R,3R-(2-hydroxymethyl)-3-hydroxy)propylamide; H2-Phe-D-Cys-Pal-D-Trp-Lys-Val-Cys-2R,3R-(2-hydroxymethyl)-3-hydroxy)propylamide; H(CH3CO)Phe-D-Cys-Pal-D-Trp-Lys-Val-Cys-2R,3R-(2-hydroxymethyl)-3-hydroxy)propylamide; (H)(4-(2-hydroxyethyi)-1-piperazinylacetyl)Phe-D-Cys-Pal-D-Trp-Lys-Val-Cys-2R,3R-(2-hydroxymethyl)-3-hydroxy)propylamide; (H)(4-(2-hydroxyethyl)-1-piperizineethanesuifonyi) Phe-D-Cys-Pal-D-Trp-Lys-Val-Cys-2R,3R-(2-hydroxymethyl)-3-hydroxy)propylamide; H2-Phe-D-Cys-Tyr-D-Trp-Lys-Thr-Cys-2R,3R-(2-hydroxymethyl)-3-hydroxy)propylamide; (H)(CH3CO)Phe-D-Cys-Tyr-D-Trp-Lys-Thr-Cys-2R, 3R-(2-hydroxymethyl)-3-hydroxy)propylamide; (H)(4-(2-hydroxyethyl)-1-piperazinylacetyl)Phe-D-Cys-Tyr-D-Trp-Lys-Thr-Cys-2R,3R-(2-hydroxymethyl)-3-hydroxy)propylamide; (H)(4-(2-hydroxyethyl)-1-piperizineethanesulfonyl)Phe-D-Cys-Tyr-D-Trp-Lys-Thr-Cys-2R,3R-(2-hydroxymethyl)-3-hydroxy)propylamide; H2-Phe-D-Cys-Pal-D-Trp-Lys-Thr-Cys-2R,3R-(2-hydroxymethyl)-3-hydroxy)propylamide; (H)(CH3CO)Phe-D-Cys-Pal-D-Trp-Lys-Thr-Cys-2R,3R-(2-hydroxymethyl)-3-hydroxy)propylamide; (H)(4-(2-hydroxyethyl)-1-piperazinylacetyl)Phe-D-Cys-Pal-D-Trp-Lys-Thr-Cys-2R,3R-(2-hydroxymethyl)-3-hydroxy)propylamide; (H)(4-(2-hydroxyethyl)-1-piperizineethanesuifonyl)Phe-D-Cys-Pal-D-Trp-Lys-Thr-Cys-2R,3R-(2-hydroxymethyl)-3-hydroxy)propylamide; H2-beta-Nal-D-ys-Tyr-D-Trp-Lys-Val-Cys-2R-(2-naphthyl)ethylamide; (H)(CH3CO)-beta-Nal-D-Cys-Tyr-P-Trp-Lys-Vai-Cys-2R-(2-naphthyl)ethylamide; (H)(4-(2-hydroxyethyl) -1-piperazinylacetyl)-beta-Nal-D-Cys-Tyr-D-Trp-Lys-Val-Cys-2R-(2-naphthyl)ethylamide; (H)(4-(2-hydroxyethyl)-1-piperizineethanesulfonyl)-D-Nal-D-Cys-Tyr-D-Trp-Lys-Val-Cys-2R-(2-naphthyl)ethylamide; H2-beta-Nal-D-Cys-Pal-D-Trp-Lys-Val-Cys-2R-(2-naphthyl)ethylamide; (H)(CH,CO)-beta-Nal-D-Cys-Pal-D-Trp-Lys-Val-Cys-2R-(2-naphthyl)ethylamide; (H)(4-(2-hydroxyethyl)-1-piperazinylacetyl)-beta-Nal-D-Cys-Pal-D-Trp-Lys-Val-Cys-2R-(2-naphthyl)ethylamide; (H)(4-(2-hydroxyethyl)-1-piperizineethanesuifonyl)-beta-Nal-D-Cys-Pal-D-Trp-Lys-Val-Cys-2R-(2-naphthyl)ethylamide; H2-beta-Nal-D-Cys-Tyr-D-Trp-Lys-Thr-Cys-2R-(2-naphthyl)ethylamide; (H)(CH,CO)-beta-Nal-D-Cys-Tyr-D-Trp-Lys-Thr-Cys-2R-(2-naphthyl)ethylamide; (H)(4-(2-hydroxyethyl)-1-piperazinylacetyl)-beta-Nal-D-Cys-Tyr-D-Trp-Lys-Thr-Cys-2R-(2-naphthyl)ethylamide; (H)(4-(2-hydroxyethyl)-1-piperizineethanesulfonyl)-beta-Nal-D-Cys-Tyr-D-Trp-Lys-Thr-Cys-2R-(2-naphthyl)ethylamide; H2-beta-Nal-D-Cys-Pal-D-Trp-Lys-Thr-Cys-2R-(2-naphthyl)ethylamide; (H)(CH3CO)-beta-Nal-D-Cys-Pal-D-Trp-Lys-Thr-Cys-2R-(2-naphthyl)ethylamide; (H)(4-(2-hydroxyethyi)-1-piperazinylacetyl)-beta-Nal-D-Cys-Pal-D-Trp-Lys-Thr-Cys-2R-(2-naphthyl)ethylamide; (H)(4-(2-hydroxyethyl)-1-piperizineethanesuifonyl)-beta-Nal-D-Cys-Pal-D-Trp-Lys-Thr-Cys-2R-(2-naphthyl)ethylamide; H2-Phe-D-Cys-Tyr-D-Trp-Lys-Val-Cys-2R-(2-naphthyl)ethylamide; (H)(CH3CO)Phe-D-Cys-Tyr-D-Trp-Lys-Val-Cys-2R-(2-naphthyl)ethylamide; (H)(4-(2-hydroxyethyl) -1-piperazinylacetyl)Phe-P-Cys-Tyr-D-Trp-Lys-Val-Cys-2R-(2-naphthyl)ethylamide; (H)(4-(2-hydroxyethyl)-1-piperizineethanesuifonyl)Phe-D-Cys-Tyr-D-Trp-Lys-Val-Cys-2R-(2-naphthyl)ethylamide; H2-Phe-D-Cys-Pal-D-Trp-Lys-Val-Cys-2R-(2-naphthyl)ethylamide; (H)(CH3CO)Phe-Cys-Pal-D-Trp-Lys-Val-Cys-2R-(2-naphthyl)ethylamide; (H)(4-(2-hydroxyethyl)-1-piperaziyiacetyl)Phe-D-Cys-Pal-D-Trp-Lys-Val-Cys-2R-(2-naphthyl)ethylamide; (H)(4-(2-hydroxyethyl)-1I-piperizineethanesuifonyl)Phe-D-Cys-Pal-D-Trp-Lys-Val-Cys-2R-(2-naphthyl)ethylamide; H2-Phe-D-Cys-Tyr-D-Trp-Lys-Thr-Cys-2R-(2-naphthyl)ethylamide; (H)(CH3CO)Phe-D-Cys-Tyr-D-Trp-Lys-Thr-Cys-2R-(2-naphthyl)ethylamide; (H)(4-(2-hydroxyethyl)-1-piperazinylacetyl)Phe-P-Cys-Tyr-D-Trp-Lys-Thr-Cys-2R-(2-naphthyl)ethylamide; (H)(4-(2-hydroxyethyl)-1-piperizineethanesuifonyi)Phe-D-Cys-Tyr-D-Trp-Lys-Thr-Cys-2R-(2-naphthyl)ethylamide; H2-Phe-D-Cys-Pal-D-Trp-Lys-Thr-Cys-2R-(2-naphthyl)ethylamide; (H)(CH3CO)Phe-Cys-Pal-D-Trp-Lys-Thr-Cys-2R-(2-naphthyl)ethylamide; (H)(4-(2-hydroxyethyl)-1-piperazinylacetyl)Phe-P-Cys-Pal-D-Trp-Lys-Thr-Cys-2R-(2-naphthyl)ethylamide; (H)(4-(2-hydroxyethyl)-1-piperizineethanesuifonyi)Phe-D-Cys-Pal-D-Trp-Lys-Thr-Cys-2R-(2-naphthyl)ethylamide; H2-beta-Nal-D-Cys-Tyr-D-Trp-Lys-Abu-Cys-2R-(2-naphthyl)ethylamide; H2-Phe-D-Cys-Tyr-D-Trp-Lys-Abu-Cys-2R-(2-naphthyl)ethylamide; H2-beta-Nal-D-Cys-Tyr-D-Trp-Lys-Abu-Cys-2R,3R-(2-hydroxymethyl)-3-hydroxy)propylamide; or H2-Phe-D-Cys-Tyr-D-Trp-Lys-Abu-Cys-2R,3R-(2-hydroxymethyl)-3-hydroxy)propylamide; H2-Phe-D-Phe-Tyr-D-Trp-Lys-Thr-Phe-Thr-NH2; H2-Phe-D-Phe-Tyr-D-Trp-Lys-Val-Phe-Thr-NH2; H2-Phe-D-Cpa-Tyr-D-Trp-Lys-Val-Phe-Thr-NH2; H2-beta-Nal-D-Cpa-Tyr-D-Trp-Lys-Val-Phe-Thr-NH2; (H)(CH3CO)-beta-Nal-D-Cpa-Tyr-D-Trp-Lys-Val-Phe-Thr-NH2; (H)(4-(2-hydroxyethyl)-1-piperazinylacetyl) -beta-Nal-D-Cpa-Tyr-D-Trp-Lys-Val-Phe-Thr-NH2; (H)(4-(2-hydroxyethyl)-1-piperizineethanesuifonyl)-beta-Nal-D-Cpa-Tyr-D-Trp-Lys-Val-Phe-Thr-NH2; H2-beta-Nal-D-Cpa-Pal-D-Trp-Lys-Val-Phe-Thr-NH2; (H)(CH3CO)-beta-Nal-D-Cpa-Pal-D-Trp-Lys-Val-Phe-Thr-NH2; (H)(4-(2-hydroxyethyl)-1-piperazinylacetyl)-beta-Nal-D-Cpa-Pal-D-Trp-Lys-Val-Phe-Thr-NH2; (H)(4-(2-hydroxyethyl)-1-piperizineethanesuifonyl)-beta-Nal-D-Cpa-Pal-D-Trp-Lys-Val-Phe-Thr-NH2; H2-beta-Nal-D-Cpa-Tyr-D-Trp-Lys-Thr-Phe-Thr-NH2; (H)(CH3CO)-beta-Nal-D-Cpa-Tyr-D-Trp-Lys-Thr-Phe-Thr-NH2; (H)(4-(2-hydroxyethyl)-1-piperazinylacetyl)-beta-Nal-D-Cpa-Tyr-D-Trp-Lys-Thr-Phe-Thr-NH2; (H)(4-(2-hydroxyethyi)-1-piperizineethanesuifonyl)-beta-Nal-D-Cpa-Tyr-D-Trp-Lys-Thr-Phe-Thr-NH2; H2-beta-Nal-D-Cpa-Pal-D-Trp-Lys-Thr-Phe-Thr-NH2; (H)(CH3CO)-beta-Nal-D-Cpa-Pal-D-Trp-Lys-Thr-Phe-Thr-NH2; (H)(4-(2-hydroxyethyl)-1-piperazinylacetyl)-beta-Nal-D-Cpa-Pal-D-Trp-Lys-Thr-Phe-Thr-NH2; (H)(4-(2-hydroxyethyi)-1-piperizineethanesuifonyl) -beta-Nal-D-Cpa-Pal-D-Trp-Lys-Thr-Phe-Thr-NH2; H2-beta-Nal-D-Cpa-Tyr-D-Trp-Lys-Val-Phe-beta-Nal-NH2; (H)(CH3CO)-beta-Nal-D-Cpa-Tyr-D-Trp-Lys-Val-Phe-beta-Nal-NH2; (H)(4-(2-hydroxyethyl)-1-piperazinylacetyl)-beta-Nal-D-Cpa-Tyr-D-Trp-Lys-Val-Phe-beta-Nal-NH2; (H)(4-(2-hydroxyethyl)-1-piperizineethanesuifonyl)-beta-Nal-D-Cpa-Tyr-D-Trp-Lys-Val-Phe-beta-Nal-NH2; H2-beta-Nal-D-Cpa-Tyr-D-Trp-Lys-Val-Phe-beta-Nal-NH2-; or H2-beta-Nal-D-Cpa-Tyr-D-Trp-Lys-Val-Phe-Thr-NH2; H2-D-beta-Nal-D-Cpa-Phe-D-Trp-Lys-Val-Phe-Thr-NH2; H2-D-beta-Nal-D-Phe-Tyr-D-Trp-Lys-Thr-Phe-Thr-NH2; H2-D-Phe-D-Phe-Tyr-D-Trp-Lys-Val-Phe-Thr-NH2; H2-D-beta-Nal-D-Cpa-Tyr-D-Trp-Lys-Val-Phe-Thr-NH2; or H2-D-beta-Nal-D-Cpa-Tyr-D-Trp-Lys-Val-Phe-beta-Nal-NH2.

GHRH peptide analogues date back to the 1990s, and include the ’standard antogonist’ [Ac-Tyr’, D-Arg2jhGH-RH (1-29) Nha. US Patent 4, 659,693 (hereby incorporated in its entirety by reference thereto) discloses GH-RH antagonistic analogs which contain certain N, N’- dialkyl-omega-guanidino alpha-amino acyl residues in position 2 of the GH-RH (1-29) sequence. The following publications are of note, all of which are hereby incorporated by reference thereto. WO91/16923 describes hGH-RH modifications including: replacing Tyr1, Ala2, Asp3 or Asn8 with their D-isomers; replacing Asn8 with L-or D-Ser, D-Arg, Asn, Thr, Gln or D-Lys; replacing Ser9 with Ala to enhance amphiphilicity of the region; and replacing Goy’S with Ala or Aib. US5,084,555 describes an analogue [Se-psi [CH2-NH]-Tyrl°lhGH-RH (1-29) that includes a pseudopeptide bond (ie. a peptide bond reduced to a [CH2-NH] linkage) between the R9 and R10 residues. US 5,550,212, US5,942,489, and US6,057,422 disclose analogs of hGH-RH (1-29) NH2 produced by replacement of various amino acids and acylation with aromatic or nonpotar acids at the N-terminus of GH-RH (1-29) NH2. The tumor inhibitory properties of antagonists featured in US Patent 5, 942,489 and US Patent 6,057, 422 have been demonstrated by using nude mice bearing xenografts of experimental human cancer models. Specific examples include: [PhAc-Tyr’, D-Arg2, Phe (pCl) 6, Amp9, Tyr (Me010, Abu15, Nle27, D-Arg28, Har29]hGH-RH (1-29) NH2; [PhAc-Tyr’, D-Arg2, Phe (pCl)6, Amp9, Abu15, Nle27, D-Arg28, Har29]hGH-RH(1-29)NH2; [PhAc-Tyr’, D-Arg2, Phe (pCl) 6, His9, Tyr (Me)10, Abu15, Nle27, D-Arg28, Har29] hGH-RH (1-29) NH2; [CH3 (CH2) 6CO-Tyr1, D-Arg2, Phe (pCl)6, Amp9, Tyr (Me)10, Abu15, Nle27, D-Arg28, Har29]hGH-RH(1- 29) NH2; [HOOC (CH2) 8CO-Tyr1, D-Arg2, Phe (pCl) 6, Amp9, Tyr (Me)10, Abu15, Nle27, D-Arg28, Har29]hGH-RH (1-29) NH2; [HOOC (CH2) 2CO-Tyr1, D-Arg2, Phe (pCi) 6, Amp9, Tyr (Me)10, Abu15, Nle27, D-Arg28, Har29]hGH-RH (1-29) NH2; [PhAc-Tyr’, D-Arg2, Phe (pCl)6, Amp9, Tyr (Me)10, His11, Abu15, Nle27, D-Arg28, Har29]hGH-RH(1- 29) NH2; [PhAc-Tyr’, D-Arg2, Phe (pCl) 6, Cit8, Amp9, Tyr (Me)10, His", Abu’5, Nle27, D-Arg28, Har29] hGH-RH (1- 29) NH2; [1-nac-Tyr1, D-Arg2, Phe (pCl) 6, Cit8, Amp9, Tyr (Me)10, His11, Abu15, Nle27, D-Arg28, Har29]hGH- RH (1-29) NH2; [CH3 (CH2) 6CO-Tyr’, D-Arg2, Phe (pCl) 6, Cit8, Amp9, Tyr (Me)10, His", Abu15, Nle27, D-Arg28 Har’] hGH-RH (1-29) NH2; [HOOC (CH2) 12CO-Tyr’, D-Arg2, Phe (pCl) 6, Cit8, Amp9, Tyr (Me)10, His", Abu’5, Nle27, D-Arg28, Har29] hGH-RH (1-29)NH2; [CH3 (CH2) 6CO -Tyr1, D-Arg2, Phe (pCl)6, Cit8, Amp9, Tyr (Et)’°, His", Abu’5, Nle27, D-Arg28, Har29] hGH-RH (1-29) NH2; [CH3 (CH2) 6CO-Tyr’, D-Arg2, Phe (pCl) 6, Cit8, His9, Tyr (Et010, His11, Abu15, Nle27, D-Arg28, Har29]hGH-RH(1-29)NH2; [CH3 (CH2) 6CO -Tyr1, D-Arg2, Phe (pCl) 6, Alpe, His9, Tyr (Et)10, His11, Abu15, Nle27, D-Arg28, i Har29] hGH-RH (1-29) NH2; [HOOC (CH2) 8CO -Tyr1, D-Arg2, Phe(pCl)6, Ala8, His9, Tyr(Et)10, His11, Abu15, Nle27, D-Arg28, Har29] hGH-RH (1-29) NH2; [HOOC(CH2)12CO -Tyr1, D-Arg2, Phe(pCl)6, Ala8, His9, Tyr(Et)10, His11, Abu15, Nle27, D-Arg28, Har29] hGH-RH (1-29) NH2; [CH3 (CH2) 6CO-Tyr’, D-Arg2, Phe (pCl)6, Ala8, His9, Tyr (Et)10, His11, Abu15, His20, Nle27, D-Arg28, Har29] hGH-RH (1-29) NH2; [CH3 (CH2) 6CO-Tyr1, D-Arg2, Phe (pCl)6, Ala8, Amp9, Tyr (Et)10, His11, Abu15, His20, Nle27, D-Arg28, Har29]hGH-RH(1-29)NH2; [HOOC (CH2)1 2CO-Tyr1, D-Arg2, Phe (pCl) 6, Ala8, His9, Tyr (Et)10, His11, Abu15, His20, Nle27, D-Arg28, Har2lhGH-RH’ (1-29) NH2; [HOOC(CH2)12CO-Tyr1, D-Arg2, Phe(pCl)6, Ala8, Amp9, Tyr(Et)10, His11, Abu15, His20, Nle27, D-Arg28, Har29]hGH-RH(1-29)NH2; [1-Nac-Tyr1, D-Arg2, Phe (pCl) 6, Ala8, His9, Tyr (Et)10, His11, Abu15, Nle27, D-Arg28, Har29]hGH-RH(1-29) NH2; [CH3 (CH2) 6CO -Tyr1, D-Arg2, Phe (pCl) 6, His9, Tyr (Et)’°, His", Abu’5, Nle27, D-Arg28, Har29]hGH- RH (1-29) NH2; [CH3 (CH2) 6CO-Tyr’, D-Arg2, Phe (pCl)6, Ala8, His9, Cit15, Nle27, D-Arg28, har29]hGH-RH (1-29) NH2; [CH3 (CH2) 6CO-Tyr1, D-Arg2, Phe(pCl)6, Ala8, His9, tyr(Et)10, His11, His15, His20, Nle27, D-Arg28 Har29]hGH-RH (1-29) NH2; [CH3 (CH2) 6CO-Tyr’, D-Arg2, Phe (pCl) 6, Ala8, His9, Tyr (Et)10, His11, Orn12, Abu15, Orn21, Nle27, D- Arg28, Har29] hGH-RH (1-29) NH2; [CH3 (CH2) 6CO-Tyr’, D-Arg2, Phe (pCl) 6, Alas, His9, Tyr (Et)10, His11, Orn12, Abu15, His20, Orn21, Nle27, D-Arg28, Har29] hGH-RH (1-29) NH2; [CH3 (CH2) 6CO -Tyr1, D-Arg2, Phe (pCl)6, Ala8, His9, Tyr (Et)", His", Abu", Nie 2’, D-Arg2, Har29]hGH-RH (1-29) NHEt; [CH3 (CH2) 8CO -Tyr1, D-Arg2, Phe (pCl) 6, Ala8, His9, Tyr (Et)10, His11, Abu15, Nle27, D-Arg28, Har29] hGH-RH (1-29) NHEt; [CH3 (CH2) 10CO -Tyr1, D-Arg2 Phe (pCl) 6 Ala8, His9, Tyr(Et)10, His11, Abu15, Nle27, D-Arg28 Har29] hGH-RH (1-29) NHEt; [Hca -Tyr1, D-Arg2, Phe(pCl)6, Ala8, His9, Tyr(Et)10, His11, Abu15, Nle27, D-Arg28, Har29]hGH-RH(1- 29) NHEt; [CH3 (CH2) 6CO-Tyr’, D-Arg2, Phe (pCl) 6, Ala8, His9, Tyr (Et)10, His11, Abu15, nle27, D-Arg28, Har29] hGH-RH (1-29)NHMe; [HOOC (CH2) 12CO-Tyr’, D-Arg2, Phe (pCl) 6, Alas, His9, Tyr (Et)10, His11, Orn12, Abu15, His20, Orn21, Nle27 D-Arg28, Har29] hGH-RH (1-29) NH2; [CH3 (CH2) 6CO-Tyr’, D-Arg2, Phe Cl)6, Ala8, Amp9, Tyr(Et)10, His11, Orn12, Abu15, His20, Orn21 Nle27, D-Arg28, Har29] hGH-RH (1-29) NH2; [CH3(CH2)6CO -Tyr1, D-Arg2, Phe (pCl) 6, Ala8, His9, Dip10, His11, Orn12, Abu15, His20, Orn21, Nle27, D-Arg28, Har29]hGH-RH(1-29)NH2; [CH3 (CH2) 6CO-Tyr’, D-Arg2, Phe (pCl) 6, Ala8, His9, Phe (pNO2)10, His11, Orn12, Abu15, His20, Orn21, Nle27, D-Arg28, Har29]hGH-RH(1-29)NH2; [CH3(CH2)6CO -Tyr1, D-Arg2, Phe(pCl)6, Ala8, His9, Tyr(Et)10, His11, Orn12, Abu15, His20, Orn21, Nle27, D-Arg28, Har29] hGH-RH (1-29) NHEt; [HOOC 9CH2)12CO -Tyr1, D-Arg2, Phe (pCl) 6, Alas, Amp9, Tyr (Et)10, His", Orn, Abu15, His20, Orn21, Nle27, D-Arg28, Har29] hGH-RH (1-29) NH2; [HOOC (CH2) 2CO -Tyr1, D-Arg2 Phe (pCl) 6, Ala8, His9, Dip'°, His", Orn12, Abu'5, His, Orn21, Nie D-Arg28, Har29]hGH-RH (1-29) NH2; [HOOC(CH2)12CO -Tyr1, D-Arg2, Phe (pCl) 6, Ala8, His9, Phe (pNO2)10, His11, Orn12, Abu15, His20, Orn21, Nle27, D-Arg28, Har29] hGH-RH (1-29) NH2; [HOOC (CH2) 12CO-Tyr', D-Arg2, Phe (pCl) 6, Alas, His9, Tyr (Et)10, His11, Orn12, Abu15, His20, Orn21, Nle27, D-Arg28, Har29] hGH-RH (1-29) NHEt; [CH3(CH2)6CO -Tyr1, D-Arg2, Phe (pCl) 6, Ala8, Amp9, Dip10, His11, Orn12, Abu15, His20, Orn21, Nle27, d-Arg28, Har29]hGH-RH (1-29) NH2; [CH3(CH2)6CO -Tyr1, D-Arg2, Phe(pCl)6, Ala8, Amp9, Phe(pNO2)10, His11, Orn12, Abu15, His20, Orn21, Nle27, D-Arg28, har29]hGH-RH(1-29)NH2; [CH3 (CH2) 6CO -Tyr1, D-Arg2, Phe(pCl)6, Ala8, Amp9, Tyr(Et)10, His11, Orn12, Abu15, His20, Orn21, Nle27, D-Arg28, Har29]hGH-RH(1-29) NHEt; [CH3 (CH2) 6CO -Tyr1, D-Arg2, Phe (pCl) 6, Ala8, His9, Dip10, His11, Orn12, Abu15, His20, Orn21, Nle27, D- Arg28, Har29]hGH-RH (1-29) NHEt; [CH3 (CH2) 6CO -Tyr1, D-Arg2, Phe (pCl) 6, Aia8, His9, Phe (pN02)'°, His", Orn'2, Abu'5, His20, Orn21, Nle27, D-Arg28, Har29] hGH-RH (1-29) NHEt; [HOOC (CH2) 12CO-Tyr', D-Arg2, Phe (pCl)6, Ala8, Amp9, Dip10, His", Orn12, Abu15, His20, Orn21, Nie27 D-Arg Har29] hGH-RH (1-29) NH2; [HOOC(CH2)12CO -Tyr1, D-Arg2, Phe (pCl) 6, Ala8, Amp9, Phe (pN02) 10, His11, Orn12, Abu15, His20, Orn21, Nle27, D-Arg28, Har29] hGH-RH (1-29) NH2; [CH3 (CH2) 6CO-Tyr1, D-Arg2, Phe (pCl) 6, Ala', Amp9 Dip'°, His", Orn12, Abu15, His20, Orn21, Nle27, D-Arg28, Har29]hGH-RH (1-29) NHEt; [CH3 (CH2) 6CO-Tyr', D-Arg2, Phe (pCl) 6, Ala8, Amp9, Phe (pNO2)10, His11, orn12, Abu15, His20, Orn21, Nle27, D-Arg28, Har29] hGH-RH (1-29) NHEt; [HOOC (CH2) 12CO -Tyr1, D-Arg2, Phe (pCl) 6, Ala8, Amp9, Dip10, His11, Orn12, Abu15, his20, Orn21, Nle27, D-Arg28, Har29] hGH-RH (1-29) NHEt; [HOOC (CH2)1 2CO -Tyr1, D-Arg2, Phe (pCl) 6, Alas, Amp9, Phe (pNO2)10, His", Orn12, Abu15, His20 Orn21, Nle27, D-Arg28, Har29] hGH-RH (1-29) NHEt; [CH3 (CH2) 4CO-Tyr1, D-Arg2, Phe (pCl)6, Arg9, Abu15, Nle27, D-Arg28, Har29]hGH-RH(1-29)NH2; [HOOC (CH2) 4CO-Tyr', D-Arg2, Phe (pCl)6, Arg9, Abu15, Nle27, D-Arg28, Har29] hGH-RH (1-29) NH2; [CH3 (CH2) 6CO-Tyr1, D-Arg2, Phe (pCl) 6, Arg9, Abu15, Nle27, D-Arg28, Har29]hGH-RH (1-29) NH2; [HOOC(CH2)6CO-Tyr1, D-Arg2, Phe(pCl)6, Arg9, Abu15, Nle27, D-Arg28, Har29]hGH-RH(1-29)NH2; [CH3(CH2)8CO-Tyr1, D-Arg2, Phe (pCl) 6, Arg9, Abu'5, Nle27, D-Arg28, Har29] hGH-RH (1-29) NH2; [HOOC(CH2)8CO-Tyr1, D-Arg2, Phe(pCl)6, Arg9, Abu15, Nle27, D-Arg28, Har29]hGH-RH(1-29)NH2; [CH3 (CH2)1 0CO-Tyr1, D-Arg2 Phe Cl)6, Arg9, Abu15, Nle27, D-Arg26, Har29]hGH-RH(1-29)NH2; [HOOC (CH2) 0CO-Tyr1, D-Arg2, Phe (pCl)6, Arg9, Abu15, Nle27, D-Arg28, Har29]hGH-RH(1-29)NH2; [CH3 (CH2) 12CO-Tyr', D-Arg2, Phe (pCl)6, Arg9, Abu15, Nle27, D-Arg28, Har29]hGH-RH (1-29) NH2; [HOOC (CH2) i2CO-Tyr\ D-Arg2, Phe (pCl)6, Arg9, Abu15, Nle27, D-Arg28, Har29]hGH-RH (1-29) NH2; [CH3 (CH2) 4CO-Tyr1 D-Arg2, Phe (pCl) 6, Arg9, Abu15, Nle27, D-Arg28, Har29]hGH-RH (1-29) NH2; [HOOC (CH2) 4CO-Tyr1, D-Arg2, Phe (pCl)6, Arg9, Abu15, Nle27, D-Arg28, Har29]hGH-RH (1-29) NH2; [CH3 (CH2) CO-Tyr1, D-Arg2, Phe(pCl)6, Arg9, Abu15, Nle27, Har28, D-Arg29]hGH-RH(1-29)NH2; [PhAc-Tyr', D-Arg2, Phe (pCl) 6, Arg9, Abu'5, Nle27, Har28, D-Arg29] hGH-RH (1-29) NH2; [CH3 (CH2) 4CO-Phe0, D-Arg2, Phe (pCl) 6, Arg9, Abu15, Nle27, D-Arg28, har29] hGH-RH (1-29) NH2; [CH3 (CH2) 14CO-D-Phe0, D-Arg2, Phe (pCl)6, Arg9, Abu15, Nle27, D-Arg28, Har29]hGH-RH (1-29) NH2; [PhAc-Arg°, D-Arg2, Phe (pCi) 6, Arg9, Abu'5, NLe27, D-Arg28, Har29] hGH-RH (1-29) NH2; [PhAc-D-Arg°, D-Arg2, Phe (pCl) 6, Arg9, Abu'5, Nle27, D-Arg28, Har29] hGH-RH (1-29) NH2; [PhAc-Tyrl, D-Arg2, Phe (pCl) 6, Cite, Arg9 Abut5 Nie27, D-Arg28, Har29] hGH-RH (1-29) NH2; [PhAc-Tyr', D-Arg2, Phe (pCl) 6, Cite, Cit9, Abu15, Nle27, D-Arg28, har29]hGH-RH(1-29)NH2; [PhAc-Tyr', D-Arg2, Phe (pCl) 6, Cit8, Arg9, Abu'5, Nle27, Har28, D-Arg29]hGH-RH (1-29) NH2; [PhAc-Tyr1, D-Arg2, Phe (pCl)6, Cit8, Cit9, Abu15, Nle27, Har28, D-Arg29]hGH-RH (1-29) NH2; [HOOC (CH2) i2CO-Tyr\ D-Arg2, Phe (pCl)6, Cit8, Cit9, Abu15, Nle27, D-Arg28, Har29]hGH-RH(1- 29) NH2; [PhAc-Tyr1, D-Arg2, Phe (pCl) 6, D-Ala8, Arg9, Abu15, Nle27, D-Arg28, Har29] hGH-RH (1-29) NH2; [PhAc-Tyr1, D-Arg2, Phe (pCl) r3, Abu3, Arg9, Abu'5, Nle27, D-Arg28, Har29] hGH-RH (1-29) NH2; [PhAc-Tyr', D-Arg2, Phe (pCl)6, Cit9, Abu15, Nle27, Har28, D-Arg29]hGH-RH (1-29) NH2; [PhAc-Tyr', D-Arg2, Phe (pCl) 6, Arg9, Amp'°, Abu'5, Nle27, D-Arg28, Har29]hGH-RH (1-29) NH2; [PhAc-Tyr1, D-Arg2, Phe (pCl) 6, Har9, Amp10 Abu5, Nle27, D-Arg28, Har29 hGH-RH (1-29) NH2; PhAc-Tyr1, D-Arg2, Phe (pCl) 6 Arg9, His'o, Abu'5, Nle 27, D-Arg28, Ha) hGH-RH (1-29) NH2; [PhAc-Tyr', D-Arg2, Phe (pCl) 6, Arg9, Cha10, Abu15, Nle27, D-Arg28, Har29]hGH-RH(1-29)NH2; [PhAc-Tyr', D-Arg2, Phe (pCl)6, Har9, Tpi10, Abu15, Nle27, D-Arg28, har29]hGH-RH (1-29) NH2; PhAc-Tyr1, D-Arg2, Phe(pCl)6, Har9, 2-Nal10, Abu15, Nle27, D-Arg28, Har29]hGH-RH(1-29)NH2; [PhAc-Tyr1, D-Arg2, Phe (pCl) 6, Har9, Dip10, Abu15, Nle27, D-Arg28, Har29]hGH-RH (1-29) NH2; [PhAc-Tyr1, D-Arg2, Phe(pCl)6, Har9, Phe (pNH2)10, Abu15, Nle27, D-Arg28, Har29]hGH-RH (1-29) NH2; [PhAc-Tyr1, D-Arg2 Phe (pCl) zu Har9, Trpt°, Abu15 Nle27, D-Arg28, Har29]hGH-RH(1-29)NH2; [PhAc-Tyr1, D-Arg2, Phe(pCl)6, Har9, Phe(pNO2)10, Abu15, Nle27, D-Arg28, Har29]hGH-RH(1-29)NH2; [PhAc-Tyr1, D-Arg2, Phe (pCl)6, Har9, 3-Pal10, Abu15, Nle27, D-Arg28, Har29] hGH-RH (1-29) NH2; [PhAc-Tyrl, D-Arg2, Phe (pCl) 6, Har9, Tyr (Et)'°, Abu15, Nle27, D-Arg28, Har29] hGH-RH (1-29) NH2; [PhAc-His', D-Arg2, Tyr6, Har9, Bpa10, Abu'5, Nle27, D-Arg28, Har29] hGH-RH (1-29) NH2; [PhAc-Tyr', D-Arg2, Phe (pCl) 6, Arg9, Har12, Abu15, Nle27, D-Arg28, Har29] hGH-RH (1-29) NH2; [Hca-Tyr', D-Arg2, Phe (pCl) 6, Har9, Tyr (Me)10, Abu15, Nle27, D-Arg28, Har29]hGH-RH (1-29) NHEt; [PhAc-Tyr'D-Arg2, Phe (pCl) 6 Har9, Tyr(Me)10, Abu15, Nle27, D-Arg28, Har29] hGH-RH (1-29) NHEt; [Hca-Tyr1, D-Arg2, Phe (pCl)6, Arg9, Abu15, Nle27, D-Arg28, Har29[hGH-RH(1-29) NHEt; PhAc-Tyr1, D-Arg2 Phe Cl)6, Arg9, Abu15, Nle27, D-Arg28, Har29]hGH-RH(1-29 NHEt; [PhAc-Tyr1, D-Arg2, Phe (pCl) 6, Har9, Tyr (Me)10, Aib15, Nle27, D-Arg28, Har29] hGH-RH (1-29) NHEt; [PhAc-Tyr', D-Arg2, Phe (pCl) 6, Har9, Tyr (Me)10, Orn12, Abu15, Nle27, D-Arg28, Har29] hGH-RH (1- 29) NHEt; [Hca-Tyr1, D-Arg2, Phe (pCl) 6, Har9, Tyr (Me)10, Abu15, Nle27, D-Arg28, Agm29]hGH-RH (1-29); [PhAc-Tyr1, D-Arg2, Phe (pCl) 6, Har9, Tyr (Me)'°, Abu15, Nle27, D-Arg28, Agm29]hGH-RH(1-29); [Hca-Tyr1, D-Arg2, Phe (pCl) 6, Har9, Tyr (Me)10, Abu15, Nle27, D-Arg28, Har29, Har30]hGH-RH(1- 30) NH2; [Dat-Tyr1, D-Arg2, Phe (pCl)6, Har9, Tyr (Me)10, Abu15, Nle27, D-Arg28, Har29, Har30]hGH-RH (1-30) NH2; [Ipa-Tyr1, D-Arg2, Phe (pCl) 6, Har9, Tyr (Me)10, Abu15, Nle27, D-Arg28, Har29, Har30]hGH-RH(1-30)NH2; [Hca-Tyr', D-Arg2, Phe (pCl) 6, Har9, Tyr (Me)10, Abu15, Nle27, D-Arg28, Har29, Har30] hGH-RH (1-30) NHEt; [Hca-Tyr', D-Arg2, Phe (pCl) 6, Har9, Tyr (Me10), Abu15, Nle27, D-Arg28, D-Arg29, Har30]hGH-RH(1- 30) NH2; [Hca-Tyr', D-Arg2, Phe (pCl) 6, Har9, Tyr (Me)10, Abu15, Nle27, D-Arg28, Har29, D-Arg30]hGH-RH(1- 30) NH2; [Hca-Tyr', D-Arg2, Phe (pCl) 6, Har9, Tyr (Me)10, Abu15, Nle27, D-Arg28, Har29, Agm30] hGH-RH (1-30); [PhAc-Tyr', D-Arg2, Phe (pCl) 6, Har9, Tyr (Me)10, Abu15, Nle27, D-Arg28, Har29, Agm30] hGH-RH (1-30); [PhAc-Tyr', D-Arg2, Phe (pCl) 6, Har9, Tyr (Me)10, His11, Abu15, Nle27, D-Arg28, Har29]hGH-RH(1-29)NH2; [PhAc-Tyr1, D-Arg2, Phe(pCl)6, Har9, Tyr(Me)10, Har11, Abu15, Nle27, D-Arg28, Har29]hGH-RH(1-29) NH2 [PhAc-Tyr1, D-Arg2, Phe(pCl)6, Har9, Tyr (Me)10, Amp11, Abu15, Nle27, D-Arg28, Har29] hGH-RH (1-29) NH2; [PhAc-Tyr1, D-Arg2, Phe (pCl)6, Har9, Tyr (Me)10, Cit", Abu15, Nle27, D-Arg28, Har29]hGH-RH(1-29) NH2; [PhAc-Tyr1, D-Arg2, Phe (pCl)6, Har9, Tyr (Me)'°, Abu15, His20, Nle, D-Arg28, Har29]hGH-RH(1-29) NH2; [PhAc-Tyr', D-Arg2, Phe(pCl)6, Har9, Tyr (Me)10, His", Abu15, His20, Nle27, D-Arg28, Har29]hGH- RH (1-29) NH2; [PhAc-Tyr1, D-Arg2, Phe (pCl) 6, Arg9, Cit15, Nle27, D-Arg28, Har29]hGH-RH(1-29)NH2; [PhAc°, D-Arg2, Phe (pCl)6, Arg9, Abu15, Nle27, D-Arg28, Har29] hGH-RH (1-29) NH2; [IndAc0, D-Arg2, Phe(pCl)6, Arg9, Abu15, Nle27, D-Arg28, Har29]hGH-RH(1- 29) NH2; [PhAc°, D-Arg2, Phe pCl) r, Har9, Tyr(Me)10, Abu15, Nle27, D-Arg28, Har29]hGH-RH (1-29) NH2; [PhAc°, D-Arg2, Phe(pCl)6, Arg9, Tyr(Me)10, Abu15, Nle27, D-Arg28, Har29] hGH- RH (1-29) NH2; [PhAc°, His', D-Arg2, Phe (pCl)6, Arg9, Abu15, Nle27, D-Arg28, Har29]hGH-RH(1- 29) NH2; [Nac°, His', D-Arg2, Phe (pCl) 6, Arg9, Abu15, Nle27, D-Arg28, Har29] hGH-RH (1-29) NH2; [PhAc°, D-Arg2, Phe (pCl) 6 Arg9, Abu'5, Nle27, D-Arg28, Har29] hGH-RH (1-29) NH2; [IndAc°, D-Arg2, Phe (pCl)6, Arg9, Abu15, Nle27, D-Arg28, Har29] hGH-RH (1-29) NH2; [PhAc°, D-Arg2, Phe (pCl) 6, Har9, Tyr (Me)10, Abu15, Nle27, D-Arg28, Har29] hGH-RH (1-29) NH2; [PhAc°, D-Arg2, Phe (pCl) 6, Arg9, Tyr (Me)10, Abu15, Nle27, D-Arg28, Har29] hGH-RH (1-29) NH2 ; [PhAc°, His', D- Arg2, Phe (pCl)6, Arg9, Abu15, Nle27, D-Arg28, Har29]hGH-RH(1-29)NH2; [Nac°, His', D-Arg2, Phe(pCl)6, Arg9, Abu15, Nle27, D-Arg28, Har29] hGH-RH (1-29) NH2; [PhAc$^0$, D-Arg$^2$, Phe(pCl)$^{fi}$, Ala$^{15}$, Nle$^{27}$, Asp$^{28}$]hGH-RH(1-28)Agm; [Ibu°,D-Arg$^2$, Phe(pCl)$^{8\ 10}$, Abu$^{15}$ , Nle$^{27}$]hGH-RH(1-28)Agm; [PhAc°,D-Arg$^2$, Phe(pCl)$^6$, Abu$^{15}$, NÏe $^7$]hGH-RH(1-28)Agm; [PhAc°,D-Arg$^2$, Phe(pCl)$^6$, Ala$^{15}$, Nle$^{27}$]hGH-RH(1-29)-$NH_2$; [PhAc°, D-Arg$^2$,Phe(pCl)$^6$,Abu$^8$,Ala$^{15}$,Nle$^{27}$]hGH-RH(1-29)$NH_2$; [PhAc$^0$, D-Arg$^2$, Phe(pCl)$^6$, Abu$^{8,28}$, Ala$^{15}$ , Nle$^{27}$]hGH-RH(1-29)-$NH_2$; cyclo$^{8,12}$[PhAc°,D-Arg$^2$,Phe(pCl)$^6$,Glu$^{\beta}$,Ala$^{15}$,Nle$^{27}$]hGH-RH(1-29)-$NH_2$; cyclo$^{17,\ 21}$ [PhAc°,D-Arg$^2$, Phe(pCl)$^6$, Ser$^8$Ala$^{15}$ Glu$^{17}$ Nle$^{27}$]hGH-RH(1-29)-$NH_2$; cyclo$^{8,12;21,25}$[PhAc°,D-Arg$^2$,Phe(pCl)$^{\theta}$,Glu$^{8,25}$ ,Abu$^{15}$,Nle$^{27}$]hGH-RH(1-28)Agm; cyclo$^{8,12;21,25}$[PhAc° ,D-Arg$^2$,D-Asp$^3$,Phe(pCl)$^8$,Glu$^{8,25}$,D-Lys$^{12}$,Ala$^{16}$,Nle$^{27}$]hGH-RH(1-29)-$NH_2$; cyclo$^{8,12;21,25}$[[PhAc°,D-Arg$^2$, Phe(pCl)$^6$, Glu$^{8,25}$, D-Lys$^{12}$, Ala$^{15}$, Nle$^{27}$] hGH-RH(1-29)-$NH_2$. Additional GHRH analogue examples are provided in WO96/032126, WO96/022782, WO96/016707, WO94/011397, WO94/011396, each of which is herein incorporated by reference thereto.

Examples of bombesin analogues suitable for use in the present invention include TMs comprising: D-Phe-Gln-Trp-Ala-Val-Gly-His-Leu-Met-$NH_2$ (code named BIM-26218), D-Phe-Gln-Trp-Ala-Val-Gly-His-Leu-Leu-$NH_2$ (code named BIM-26187); D-Cpa-Gln-Trp-Ala-Val-Gly-His-Leu-$\varphi$ [$CH_2NH$]-Phe-$NH_2$ (code named BIM-26159), and D-Phe-Gln-Trp-Ala-Val-Gly-His-Leu-$\varphi$ [$CH_2NH$]-Cpa-$NH_2$ (code named BIM-26189); D-Phe-Gln-Trp-Ala-Val-N-methyl-D-Ala-His-Leu- methylester, and D-$F_g$-Phe-Gln-Trp-Ala-Val-D-Ala-His-Leu- methylester.

Bombesin analogues include peptides derived from the naturally-occurring, structurally-related peptides, namely, bombesin, neuromedin B, neuromedin C, litorin, and GRP. The relevant amino acid sequences of these naturally occurring peptides are: Bombesin (last 10 amino acids): Gly-Asn-Gln-Trp-Ala-Val-Gly-His-Leu-Met-$NH_2$; Neuromedin B: Gly-Asn-Leu-Trp-Ala-Thr-Gly-His-Phe-Met-$NH_2$; Neuromedin C: Gly-Asn-His-Trp-Ala-Val-Gly-His-Leu-Met-$NH_2$; Litorin: pGlu-Gln-Trp-Ala-Val-Gly-His-Phe-Met-$NH_2$; Human GRP (last 10 amino acids): Gly-Asn-His-Trp-Ala-Val-Gly-His-Leu-Met-$NH_2$.

Analogs suitable for use in the present invention include those described in U.S. Serial Number 502,438, filed March 30, 1990, U.S. Serial No. 397,169, filed August 21, 1989, U.S. Serial No. 376,555, filed July 7, 1989, U.S. Serial Number 394,727, filed August 16, 1989, U.S. Serial No. 317,941, filed March 2, 1989, U.S. Serial Number 282,328, filed December 9, 1988, U.S. Serial No. 257,998, filed October 14, 1988, U.S. Serial No. 248,771, filed September 23, 1988, U.S. Serial No. 207,759, filed June 16, 1988, U.S. Serial No. 204,171, filed June 8, 1988, U.S. Serial No. 173,311, filed March 25, 1988, U.S. Serial No. 100,571, filed September 24, 1987; and U.S. Serial No. 520,225, filed May 9, 1990, U.S. Serial No. 440,039, filed November 21, 1989. All these applications are hereby incorporated by reference. Bombesin analogs are also described in Zachary et al., Proc. Nat. Aca. Sci. 82:7616 (1985); Heimbrook et al., "Synthetic Peptides: Approaches to Biological Problems", UCLA Symposium on Mol. and Cell. Biol. New Series, Vol. 86, ed. Tarn and Kaiser; Heinz-Erian et al. , Am. J. Physiol. G439 (1986); Martinez et al., J. Med. Chem. 28:1874 (1985); Gargosky et al., Biochem.

J. 247:427 (1987); Dubreuil et al. , Drug Design and Delivery, Vol 2:49, Harwood Academic Publishers, GB (1987); Heikkila et al., J. Biol. Chem. 262:16456 (1987); Caranikas et al. , J. Med. Chem. 25:1313 (1982); Saeed et al., Peptides 10:597 (1989); Rosell et al., Trends in Pharmacological Sciences 3:211 (1982); Lundberg et al., Proc. Nat. Aca. Sci. 80:1120, (1983); Engberg et al., Nature 293:222 (1984); Mizrahi et al., Euro. J. Pharma. 82:101 (1982); Leander et al., Nature 294:467 (1981); Woll et al., Biochem. Biophys. Res. Comm. 155:359 (1988); Rivier et al., Biochem. 17:1766 (1978); Cuttitta et al., Cancer Surveys 4:707 (1985); Aumelas et al., Int. J. Peptide Res. 30:596 (1987); all of which are also hereby incorporated by reference.

The analogs can be prepared by conventional techniques, such as those described in WO92/20363 and EP0737691.

Additional bombesin analogues suitable for use in the present invention comprise: D-Phe-Gln-Trp-Ala-Val-Gly-His-Leu-jjsi-Tac-NH2; D-Tpi-Gln-Trp-Ala-Val-Gly-His-Leu-£si-Tac-$NH_2$; D-Phe-Gln-Trp-Ala-Val-Gly-His-Leu-£si-DMTac-$NH_2$; Hca-Gln-Trp-Ala-Val-Gly-His-Leu-jβsi-Tac-$NH_2$; D-Trp-Gln-Trp-Ala-Val-Gly-His-Leu-psi-Leu-$NH_2$; D-Trp-Gln-Trp-Ala-Val-Gly-His-Leu-psi-Phe-$NH_2$; D-Trp-Glu(MeNH)-Trp-Ala-Val-Gly-His-Leu-psi-Phe-$NH_2$; D-Trp-Gin-Trp-Ala-Val-Gly-His-Leu-psi-Trp-$NH_2$; D-Tpi-Gln-Trp-Ala-Val-Gly- His-Leu-psi-Leu-$NH_2$; D-Tpi-Gln-Trp-Ala-Val-Gly- His-Leu-psi-Phe-$NH_2$; D-Tpi-Gln-Trp-Ala-Val-Gly- His-Leu-psi-Trp-$NH_2$; D-pGlu-Gln-Trp-Ala-Val- Gly-His-Leu-psi-Tpi-$NH_2$.; D-Phe-Gln-Trp-Ala-Val-Gly-His-Leu-psi-Tpi-$NH_2$; D-Trp-Gln-Trp-Ala-Val-Gly-His-Leu-psi-Tpi-$NH_2$; Tpi-Gln-Trp-Ala-Val-Gly-His-Leu-psi-Tpi-NH2; $NH_2$CO-Tpi-Gln-Trp-Ala-Val-Gly-His-Leu-psi-Tpi-$NH_2$ and ACY-Tpi-Gln-Trp-Ala-Val-Gly-His-Leu-psi-Tpi-$NH_2$ wherein ACY is acetyl, octanoyl or 3-hydroxy-2-naphthoyl; D-Tpi-Gln-Trp-Ala-Val-Gly His-Leu-psi-Tpi-$NH_2$; D-Trp-Glu(MeO)-Trp-Ala-Val-Gly-His-Leu-psi-Tpi-$NH_2$; D-Trp-Glu(MeNH)-Trp-Ala-Val-Gly-His-Leu-psi-Tpi-$NH_2$; D-Trp-His(Bz)-Trp-Ala-Val Gly-His-Leu-psi-Tpi-$NH_2$; Phe-Glu-Trp-Ala-Val-Gly His-Leu-psi-Tpi-$NH_2$; $H_2$-D-Nal-Cys-Tyr-D-Trp-Lys-Val-Cys-Nal-$NH_2$; $H_2$-D-Nal-Cys-Tyr-D-Trp-Lys-Nal-Cys-Thr-$NH_2$; $H_2$-D-Nal-Cys-Tyr-D-Trp-Lys-Nal-Cys-Nal-$NH_2$; $H_2$-D-Phe-Cys-Tyr-D-Trp-Lys-Val-Cys-Nal-$NH_2$; $H_2$-D-Nal-Cys-Tyr-D-Trp-Lys-Val-Cys-D-Nal-$NH_2$; $H_2$-Nal-Cys-Tyr-D-Trp-Lys-Val-Cys-D-Nal-$NH_2$; $H_2$-D-Nal-D-Cys-Tyr-D-Trp-Lys-Val-Cys-Nal-$NH_2$; $H_2$-D-Nal-Cys-Tyr-D-Trp-Lys-Val-D-Cys-Nal-$NH_2$; $H_2$-D-Trp-Cys-Tyr-D-Trp-Lys-Val-Cys-Nal-$NH_2$; $H_2$-D-Nal-Cys-Tyr-D-Trp-Lys-Phe-Cys-Nal-$NH_2$; $H_2$-D-Nal-Cys-Tyr-D-Nal-Lys-Val-Cys-Nal-$NH_2$; $H_2$-D-Phe-Cys-Tyr-D-Trp-Lys-Nal-Cys-Thr-$NH_2$; $H_2$-D-Nal-Cys-Tyr-D-Trp-Orn-Val-Cys-Nal-$NH_2$; $H_2$-D-Phe-Cys-Tyr-D-Trp-Lys-Thr-Cys-Nal-$NH_2$; $H_2$-D-Phe-Cys-Tyr-D-Trp-Lys(iPr)-Thr-Cys-Nal-$NH_2$; $H_2$-D-Phe-Cys-Tyr-D-Trp-Lys(diEt)-Thr-Cys-Nal-$NH_2$ $H_2$-D-Phe-Cys-Tyr-D-Trp-Lys-Ser-Cys-Thr-$NH_2$; $H_2$-D-Nal-Cys-Tyr-D-Trp-Lys-Thr-Cys-Nal-$NH_2$; $H_2$-D-Nal-D-Cys-Tyr-D-Trp-Lys-Thr-Cys-Nal-$NH_2$; or $H_2$-D-Nal-Cys-Phe-D-Trp-Lys-Thr-Cys-Nal-$NH_2$; $H_2$-D-Nal-Cys-Tyr-D-Trp-Dab-Val-Cys-Nal-$NH_2$, $H_2$-D-Nal-Cys-Tyr-D-Trp-Orn-Val-Cys-Nal-$NH_2$, $H_2$-D-Nal-Cys-Tyr-D-Trp-Arg-Val-Cys-Nal-$NH_2$; pGlu-Gln-Trp-Ala-Val-Gly-His-Leu-Leu-$NH_2$, D-Phe-Gln-Trp-Ala-Val-Gly-His-Leu-Leu-$NH_2$, D-Phe-Gln-Trp-Ala-Val-Gly-His-Leu-Met-$NH_2$, D-Cpa-Gln-Trp-Ala-Val-Gly-His-Leu-Met-$NH_2$, D-Cpa-Gln-Trp-Ala-Val-Gly-His-Leu-Leu-$NH_2$, D-Phe-Gln-Trp-Ala-Val-D-Ala-His-Leu-Leu-$NH_2$, D-Phe-Gln-Trp-Ala-Val-D-Ala-His-Leu-Met-$NH_2$, D-Cpa-Gln-Trp-Ala-Val-D-Ala-His-Leu-Met-$NH_2$, pGlu-Gln-Trp-Ala-Val-Gly-His-Phe-Leu-$NH_2$, D-Phe-Gln-Trp-Ala-Val-Gly-His-Phe-Leu-$NH_2$, D-Phe-Gln-Trp-Ala-Val-D-Ala-His-Phe-Met-$NH_2$, D-Phe-Gln-Trp-Ala-Val-D-Ala-His-Phe-Leu-$NH_2$, D-Phe-Gln-Trp-Ala-Val-Gly-His-Leu-Nle-$NH_2$, D-Phe-Gln-Trp-Ala-Val-D-Ala-His-Leu-Nle-$NH_2$, D-Phe-Gln-Trp-Ala-Val-Gly-His-Phe-Nle-$NH_2$, D-Phe-Gln-Trp-Ala-Val-D-Ala-His-Phe-Nle-$NH_2$, D-p-Cl-Phe-Gln-Trp-Ala-Val-Gly-His-Leuc[$CH_2$NH]Phe-$NH_2$, D-Phe-Gln-Trp-Ala-Val-Gly-His-Leu-proplyamide, Ac-His-Trp-Ala-Val-D-Ala-His-Leu-Leu-$NH_2$, D-Phe-Gln-Trp-Ala-Val-Gly-His-CHx-Ala-Leu-$NH_2$, cyclo-D-Phe-Gln-Trp-Ala-Val-Gly-His-Leu-Leu, D-Cys-Asn-Trp-Ala-Val-Gly-His-Leu-Cys-$NH_2$, cyclo-His-Trp-Ala-Val-Gly-His-Leu-Met, Cys-Trp-Ala-Val-Gly-His-Leu-Cys-$NH_2$, cyclo-D-Phe-Gln-Trp-Ala-Val-Gly-His-Leu-Met, cyclo-D-Phe-His-Trp-Ala-Val-Gly-His-Leu-Met, cyclo-Trp-Ala-Val-Gly-His-Leu-Met.

Additional bombesin analogues are described in, for example, WO89/02897, WO91/17181, WO90/03980 and WO91/02746, all of which are herein incorporated by reference thereto.

Examples of ghrelin analogues suitable for use as a TM of the present invention comprise: Tyr-DTrp-DLys-Trp-DPhe-$NH_2$, Tyr-DTrp-Lys-Trp-DPhe-$NH_2$, His-DTrp-DLys-Trp-DPhe-$NH_2$, His-DTrp-DLys-Phe-DTrp-$NH_2$, His-DTrp-DArg-Trp-DPhe-$NH_2$, His-DTrp-DLys-Trp-DPhe-Lys-$NH_2$, DesaminoTyr-DTrp-Ala-Trp-DPhe-$NH_2$, DesaminoTyr-DTrp-DLys-Trp-DPhe-$NH_2$, DeaminoTyr-DTrp-Ser-Trp-DPhe-Lys-$NH_2$, DesaminoTyr-DTrp-Ser-Trp-DPhe-$NH_2$, His-DTrp-DTrp-Phe-Met-$NH_2$, Tyr-DTrp-DTrp-Phe-Phe-$NH_2$, Glyψ[$CH_2$NH]-DβNal-Ala-Trp-DPhe-Lys-$NH_2$, Glyψ[CH2NH]-DbetaNal-DLyS-TrP-DPhe-Lys-$NH_2$, DAla-DbetaNal-DLys-DTrp-Phe-Lys-$NH_2$, His-DbetaNal-DLys-Trp-DPhe-Lys-$NH_2$, Ala-His-DTrp-DLys-Trp-DPhe-Lys-$NH_2$, Alaφ[$CH_2$NH]-DbetaNal-Ala-Trp-DPhe-Lys-$NH_2$, DbetaNal-Ala-Trp-DPhe-Ala-$NH_2$, DAla-DcyclohexylAla-Ala-Phe-DPhe-Nle-$NH_2$, DcyclohexylAla-Ala-Phe-DTrp-Lys-$NH_2$, DAla-DbetaAla-Thr-DThr-Lys-$NH_2$, DcyclohexylAla-Ala-Trp-DPhe-NH2, DAla-DbetaNal-Ala-Ala-DAla-Lys-$NH_2$, DbetaNal-Ala-Trp-DPhe-Leu-$NH_2$, His-DTrp-Phe-Trp-DPhe-Lys-$NH_2$, DAla-DbetaNal-DAla-DTrp-Phe-Lys-$NH_2$, pAla-Trp-DAla-DTrp-Phe-$NH_2$, His-Trp-DAla-DTrp-Phe-LysNH$_2$, DLys-DβNal-Ala-Trp-DPhe-Lys-$NH_2$, DAla-DbetaNal-DLys-DTrp-Phe-Lys-$NH_2$, Tyr-DAla-Phe-Aib-$NH_2$, Tyr-DAla-Sar-NMePhe-$NH_2$, αγAbu-DTrp-DTrp-Ser-$NH_2$, αγAbu-DTrp-DTrp-Lys-$NH_2$, αγAbu-DTrp-DTrp-Orn-$NH_2$, αAbu-DTrp-DTrp-Orn-$NH_2$, DThr-DαNal-DTrp-DPro-Arg-$NH_2$, DAla-Ala-DAla-DTrp-Phe-Lys-$NH_2$, Alaψ[$CH_2$NH]His-DTrp-Ala-Trp-DPhe-Lys-$NH_2$, Lys-DHis-DTrp-Phe-$NH_2$, γAbu-DTrp-DTrp-Orn-$NH_2$, inip-Trp-Trp-Phe-$NH_2$, Ac-DTrp-Phe-DTrp-Leu-$NH_2$, Ac-DTrp-Phe-DTrp-Lys-$NH_2$, Ac-DTrp-DTrp-Lys-$NH_2$, DLys-Tyr-

DTrp-DTrp-Phe-Lys-$NH_2$, Ac-DbetaNal-Leu-Pro-$NH_2$, pAla-Trp-DTrp-DTrp-Orn-$NH_2$, DVal-D$\alpha$Nal-DTrp-Phe-Arg-$NH_2$, DLeu-DaNal-DTrp-Phe-Arg-$NH_2$, CyclohexylAla-D$\alpha$Nal-DTrp- Phe-Arg-$NH_2$, DTp-DaNal-DTrp-Phe-Arg-$NH_2$, DAla-D$\beta$ Nal-DPro-Phe-Arg-$NH_2$, Ac-D$\alpha$Nal-DTrp-Phe-Arg-$NH_2$, DaNal-DTrp-Phe-Arg-$NH_2$, His-DTrp-DTrp-Lys-$NH_2$, Ac-DpNal-DTrp-$NH_2$, $\alpha$Aib-DTrp-DcyclohexylAla-$NH_2$, $\alpha$Aib-DTrp-DAla-cyclohexylAla-$NH_2$, DAla-DcyclohexylAla-Ala-Ala-Phe-DPhe-Nle-$NH_2$, DPhe-Ala-Phe-DPal-$NH_2$, DPhe-Ala-Phe-DPhe-Lys-$NH_2$, DLys-Tyr-DTrp-DTrp-Phe-$NH_2$, Ac-DLys-Tyr-DTrp-DTrp-Phe-$NH_2$, Arg-DTrp-Leu-Tyr-Trp-Pro(cyclic Arg-Pro), Ac-D$\beta$Nal-PicLys-ILys-DPhe-NH2, DPal-Phe-DTrp-Phe-Met-$NH_2$, DPhe-Trp-DPhe-Phe-Met-$NH_2$, DPal-Trp-DPhe-Phe-Met-$NH_2$, pAla-Pal-DTrp-DTrp-Orn-$NH_2$, $\alpha\gamma$Abu-Trp-DTrp-DTrp-Orn-$NH_2$, $\beta$Ala-Trp-DTrp-DTrp-Lys-$NH_2$, $\gamma$Abu-Trp-DTrp-DTrp-Orn-$NH_2$, Ava-Trp-DTrp-DTrp-Orn-$NH_2$, DLys-Tyr-DTrp-Ala-Trp-DPhe-$NH_2$, His-DTrp-DArg-Trp-DPhe-$NH_2$, <Glu-His-Trp-DSer-DArg-$NH_2$, DPhe-DPhe-DTrp-Met-DLys-$NH_2$, 0-(2-methylallyl) benzophonone oxime, (R)-2-amino-3-(1H-indol-3-yl)-l-(4-phenylpiperidin-1-yl)propan-1 -one, N-((R)-1-((R)-1-((S)-3-(1H-indol-3-yl)-1-oxo-1-(4-phenylpiperidin-1-yl)propan-2-ylamino)-6-amino-1-oxohexan-2-ylamino)-3-hydroxy-1-oxopropan-2-yl)benzamide, (S)-N-((S)-3-(1H-indol-3-yl)-1-oxo-1-(4-phenylpiperidin-1-yl)propan-2-yl)-6-acetamido-2-((S)-2-amino-3-(benzyloxy)propanamido)hexanamide, (S)-N-((R)-3-(1H-indol-3-yl)-1-oxo-1-(4-phenylpiperidin-1-yl)propan-2-yl)-2-((S)-2-acetamido-3-(benzyloxy)propanamido)-6-aminohexanamide, (R)-N-(3-(1H-indol-3-yl)-1-(4-(2-methoxyphenyl)piperidin-1-yl)-1-oxopropan-2-yl)-4-aminobutanamide, (R)-N-(3-(1H-indol-3-yl)-1-(4-(2-methoxyphenyl)piperidin-1-yl)-1-oxopropan-2-yl)-2-amino-2-methylpropanamide, methyl 3-(p-tolylcarbamoyl)-2-naphthoate, ethyl 3-(4-(2-methoxyphenyl)piperidine-1-carbonyl)-2-naphthoate, 3-(2-methoxyphenylcarbamoyl)-2-naphthoate, (S)-2,4-diamino-N-((R)-3-(naphthalen-2-ylmethoxy)-1-oxo-1-(4-phenylpiperidin-1 -yl)propan-2-yl)butanamide, naphthalene-2,3-diylbis((4-(2-methoxyphenyl)piperazin-1 - yl)methanone), (R)-2-amino-N-(3-(benzyloxy)-1-oxo-1-(4-phenylpiperazin-1-yl)propan-2-yl)-2-methylpropanamide, or (R)-2-amino-3-(benzyloxy)-1-(4-phenylpiperazin-1-yl)propan-1-one.

Examples of GnRH analogues suitable for use as a TM in the present invention include those known from, for example, EP171477, WO96/033729, WO92/022322, WO92/013883, and WO91/05563, each of which is herein incorporated by reference thereto. Specific examples comprise: (NAcDQal$^{1}$,DPtf$^{2}$,DPAl$^{3}$,cjsPzACAla$^{5}$,DPicLys$^{6}$,DAla$^{10}$)LHRH; NAcDNal$^{1}$,DpClPhe$^{2}$,DPal$^{3}$,Thr$^{4}$, cjsPzACAla$^{5}$,DNicLys$^{6}$,ILys$^{8}$,DAla$^{10}$)LHRH; (NAcDNal$^{1}$,DpClPhe$^{2}$,DPal$^{3}$,Thr$^{4}$,PicLys$^{5}$,DPicLys$^{6}$,ILys$^{8}$,DAla$^{10}$)LHRH; (NAcDNal$^{1}$, DpClPhe$^{2}$,DPal$^{3}$,PicLys$^{5}$,DPicLys$^{6}$,Thr$^{7}$,ILys$^{8}$,DAla$^{10}$)LHRH; (NapDThr$^{1}$, DpClPhe$^{2}$, DPal$^{3}$, PicLys$^{5}$,DPicLys$^{6}$,ILys$^{8}$,DAla$^{10}$)LHRH; (NAcDNal$^{1}$ , DpClPhe$^{2}$,DPal$^{3}$,NicLys$^{5}$,DNicLys$^{6}$,Thr$^{7}$,ILys$^{8}$,DAla$^{10}$)LHRH; (NAcDNal$^{1}$ , DpClPhe$^{2}$,DPal$^{3}$,Thr$^{4}$NicLys$^{5}$,DNicLys$^{6}$,Thr$^{7}$,ILys$^{8}$,DAla$^{10}$)LHRH; (NAcDNal$^{1}$,DpClPhe$^{2}$,DPal$^{3}$,PicLys$^{5}$,D(PicSar)Lys$^{6}$,ILys$^{8}$,DAla$^{10}$)LHRH' (NAcDNal$^{1}$,DpClPhe$^{2}$,DPal$^{3}$,D(PicSar)Lys$^{6}$,ILys$^{8}$,DAla$^{10}$)LHRH; (NAcDNal$^{1}$,DpClPhe$^{2}$,DPal$^{3}$,PicLys$^{5}$,D(6ANic)Lys$^{6}$,ILys$^{8}$,DAla$^{10}$)LHRH; (NAcDNal$^{1}$,DpClPhe$^{2}$,DPal$^{3}$,PicLys$^{5}$,D(6ANic)0rn$^{6}$,ILys$^{8}$,DAla$^{10}$)LHRH; (NAcDQal$^{1}$,DCpa$^{2}$,DPal$^{3}$,cisPzACAla$^{5}$,DPicLys$^{6}$,NLeu$^{7}$,ILys$^{8}$,DAla$^{10}$)LHRH; (NAcDNal$^{1}$,DCpa$^{2}$,DPal$^{3}$ DPicLys$^{5}$,DA-Phe(PicSar)$^{\beta}$,ILys$^{8}$,DAla$^{l0}$)LHRH; NAcDQal$^{1}$,DCpa$^{2}$,DPal$^{3}$,PicLys$^{5}$,DPal$^{6}$,ILys$^{8}$,DAla$^{10}$)LHRH; (NAcDNal$^{1}$,DCpa$^{2}$,DPal$^{3}$,PicLys$^{5}$,DOrn(ACyp)$^{6}$,ILys$^{8}$,DAla$^{10}$)LHRH; N-acetyl-D-beta-Nal-D-Phe-D-Phe-Ser-Tyr-D-Lys(cyclo-pentyl)-Phe-Arg-Pro-D-Ala-$NH_2$; N-acetyl-D-ø-Nal-D-Phe-D-Phe-Ser-Tyr-D-Lys(cyclopentyl)-Phe-Lys(cyclopentyl)-Pro-D-Ala-$NH_2$; N-acetyl-D-beta-Nal-D-Phe-D-Phe-Ser-Tyr-D-Arg-Phe-(isopropyl)D-Lys-Pro-D-Ala-$NH_2$; N-acety1-D-beta-Nal-D-Phe-D-Phe-Ser-Tyr-D-Lys(benzyl)-Phe-Arg-Pro-D-Ala-$NH_2$; N-acetyl-D-beta-Nal-D-Phe-D-Phe-Ser-Tyr-D-Lys(Cl-benzyl)-Phe-Arg-Pro-D-Ala-$NH_2$; N-acetyl-D-beta-Nal-D-Phe-D-Phe-Ser-Tyr-D-Lys(heptyl)-Phe-Arg-Pro-D-Ala-$NH_2$; N-acetyl-D-beta-Nal-D-Phe-D-Phe-Ser-Tyr-D-Arg-Phe-Lys-(t-butylmethyl)-ProD-Ala-$NH_2$; N-acetyl-D-beta-Nal-D-Phe-D-Phe-Ser-Tyr-D-Arg-Phe-Lys-(4-methyl-benzyl)-Pro-D-Ala-$NH_2$; N-acetyl-D-beta-Nal-D-Phe-D-Phe-Ser-Tyr-D-Arg-Phe-Lys-(benzyl)-Pro-D-Ala-$NH_2$; N-acetyl-D-beta-Nal-D-p-Cl-Phe-D-Trp-Ser-Tyr-D-p-$NH_2$-Phe-Phe-(isopropyl)Lys-Pro-D-Ala-$NH_2$; N-acetyl-D-beta-Nal-D-Phe-D-Phe-Ser-Tyr-D-Lys(heptyl)-Phe-Lys-(heptyl)-Pro-D-Ala-$NH_2$; N-acetyl-D-3-Nal-D-Phe-D-Phe-Ser-Tyr-D-Lys(1-butylpentyl)-Phe-Lys(1-butylpentyl)-Arg-Pro-D-Ala-N $H_2$.

Examples of urotensin analogues suitable for use as a TM of the present invention comprise: Cpa-c [D-Cys-Phe-Trp-Lys-Thr-Cys]-Val-NH2; and Asp-c[Cys-Phe- Trp-Lys-Tyr-Cys]-Val-OH.

The polypeptides of the present invention lack a functional $H_C$ domain of a clostridial neurotoxin. Accordingly, said polypeptides are not able to bind rat synaptosomal membranes (via a clostridial $H_c$ component) in binding assays as described in Shone et al. (1985) Eur. J. Biochem. 151, 75-82. In a preferred embodiment, the polypeptides preferably lack the last 50 C-terminal amino acids of a clostridial neurotoxin holotoxin. In another embodiment, the polypeptides preferably lack the last 100, preferably the last 150, more preferably the last 200, particularly preferably the last 250, and most preferably the last 300 C-terminal amino acid residues of a clostridial neurotoxin holotoxin. Alternatively, the Hc binding activity may be negated/ reduced by mutagenesis - by way of example, referring to BoNT/ A for convenience, modification of one or two amino acid residue mutations (W1266 to L and Y1267 to F) in the ganglioside binding pocket causes the $H_c$ region to lose its receptor binding function. Analogous mutations may be made to non-serotype A clostridial peptide components, e.g. a construct based on botulinum B with mutations (W1262 to L and Y1263 to F) or botulinum E (W1224 to L and Y1225 to F). Other mutations to the active site achieve the same ablation of $H_c$ receptor binding activity, e.g. Y1267S in botulinum type A toxin and the corresponding highly conserved residue in the other clostridial neurotoxins. Details of this and other mutations are described in Rummel et al (2004) (Molecular Microbiol. 51:631-634), which is hereby incorporated by reference thereto.

In another embodiment, the polypeptides of the present invention lack a functional $H_C$ domain of a clostridial neurotoxin and also lack any functionally equivalent TM. Accordingly, said polypeptides lack the natural binding function of a clostridial neurotoxin and are not able to bind rat synaptosomal membranes (via a clostridial $H_C$ component, or via any functionally equivalent TM) in binding assays as described in Shone et al. (1985) Eur. J. Biochem. 151, 75-82.

The $H_C$ peptide of a native clostridial neurotoxin comprises approximately 400-440 amino acid residues, and consists of two functionally distinct domains of approximately 25kDa each, namely the N-terminal region (commonly referred to as the $H_{CN}$ peptide or domain) and the C-terminal region (commonly referred to as the $H_{CC}$ peptide or domain). This fact is confirmed by the following publications, each of which is herein incorporated in its entirety by reference thereto: Umland TC (1997) Nat. Struct. Biol. 4: 788-792; Herreros J (2000) Biochem. J. 347: 199-204; Halpern J (1993) J. Biol. Chem. 268: 15, pp. 11188-11192; Rummel A (2007) PNAS 104: 359-364; Lacey DB (1998) Nat. Struct. Biol. 5: 898-902; Knapp (1998) Am. Cryst. Assoc. Abstract Papers 25: 90; Swaminathan and Eswaramoorthy (2000) Nat. Struct. Biol. 7: 1751-1759; and Rummel A (2004) Mol. Microbiol. 51(3), 631-643. Moreover, it has been well documented that the C-terminal region ($H_{CC}$), which constitutes the C-terminal 160-200 amino acid residues, is responsible for binding of a clostridial neurotoxin to its natural cell receptors, namely to nerve terminals at the neuromuscular junction - this fact is also confirmed by the above publications. Thus, reference throughout this specification to a clostridial heavy-chain lacking a functional heavy chain $H_C$ peptide (or domain) such that the heavy-chain is incapable of binding to cell surface receptors to which a native clostridial neurotoxin binds means that the clostridial heavy-chain simply lacks a functional $H_{CC}$ peptide. In other words, the $H_{CC}$ peptide region is either partially or wholly deleted, or otherwise modified (e.g. through conventional chemical or proteolytic treatment) to inactivate its native binding ability for nerve terminals at the neuromuscular junction.

Thus, in one embodiment, a clostridial $H_N$ peptide of the present invention lacks part of a C-terminal peptide portion ($H_{CC}$) of a clostridial neurotoxin and thus lacks the $H_C$ binding function of native clostridial neurotoxin. By way of example, in one embodiment, the C-terminally extended clostridial $H_N$ peptide lacks the C-terminal 40 amino acid residues, or the C-terminal 60 amino acid residues, or the C-terminal 80 amino acid residues, or the C-terminal 100 amino acid residues, or the C-terminal 120 amino acid residues, or the C-terminal 140 amino acid residues, or the C-terminal 150 amino acid residues, or the C-terminal 160 amino acid residues of a clostridial neurotoxin heavy-chain. In another embodiment, the clostridial $H_N$ peptide of the present invention lacks the entire C-terminal peptide portion ($H_{CC}$) of a clostridial neurotoxin and thus lacks the $H_C$ binding function of native clostridial neurotoxin. By way of example, in one embodiment, the clostridial $H_N$ peptide lacks the C-terminal 165 amino acid residues, or the C-terminal 170 amino acid residues, or the C-terminal 175 amino acid residues, or the C-terminal 180 amino acid residues, or the C-terminal 185 amino acid residues, or the C-terminal 190 amino acid residues, or the C-terminal 195 amino acid residues of a clostridial neurotoxin heavy-chain. By way of further example, the clostridial $H_N$ peptide of the present invention lacks a clostridial $H_{CC}$ reference sequence selected from the group consisting of:

Botulinum type A neurotoxin - amino acid residues (Y1111-L1296)
Botulinum type B neurotoxin - amino acid residues (Y1098-E1291)
Botulinum type C neurotoxin - amino acid residues (Y1112-E1291)
Botulinum type D neurotoxin - amino acid residues (Y1099-E1276)
Botulinum type E neurotoxin - amino acid residues (Y1086-K1252)
Botulinum type F neurotoxin - amino acid residues (Y1106-E1274)
Botulinum type G neurotoxin - amino acid residues (Y1106-E1297)
Tetanus neurotoxin - amino acid residues (Y1128-D1315).

The above-identified reference sequences should be considered a guide as slight variations may occur according to sub-serotypes.

The protease of the present invention embraces all non-cytotoxic proteases that are capable of cleaving one or more proteins of the exocytic fusion apparatus in eukaryotic cells.

The protease of the present invention is preferably a bacterial protease (or fragment thereof). More preferably the bacterial protease is selected from the genera *Clostridium* or *Neisseria*/*Streptococcus* (e.g. a clostridial L-chain, or a neisserial IgA protease preferably from *N. gonorrhoeae or S. pneumoniae*).

The present invention also embraces variant non-cytotoxic proteases (ie. variants of naturally-occurring protease molecules), so long as the variant proteases still demonstrate the requisite protease activity. By way of example, a variant may have at least 70%, preferably at least 80%, more preferably at least 90%, and most preferably at least 95 or at least 98% amino acid sequence homology with a reference protease sequence. Thus, the term variant includes non-cytotic proteases having enhanced (or decreased) endopeptidase activity - particular mention here is made to the increased $K_{cat}/K_m$ of BoNT/A mutants Q161A, E54A, and K165L see Ahmed, S.A. (2008) Protein J. DOI 10.1007/s10930-007-9118-8, which is incorporated by reference thereto. The term fragment, when used in relation to a protease, typically means a peptide having at least 150, preferably at least 200, more preferably at least 250, and most preferably at least 300 amino acid residues of the reference protease. As with the TM ’fragment’ component (discussed above), protease ’fragments’ of the present invention embrace fragments of variant proteases based on a reference sequence.

The protease of the present invention preferably demonstrates a serine or metalloprotease activity (e.g. endopeptidase activity). The protease is preferably specific for a SNARE protein (e.g. SNAP-25, synaptobrevin/VAMP, or syntaxin).

Particular mention is made to the protease domains of neurotoxins, for example the protease domains of bacterial neurotoxins. Thus, the present invention embraces the use of neurotoxin domains, which occur in nature, as well as recombinantly prepared versions of said naturally-occurring neurotoxins.

Exemplary neurotoxins are produced by clostridia, and the term clostridial neurotoxin embraces neurotoxins produced by *C. tetani* (TeNT), and by C. *botulinum* (BoNT) serotypes A-G, as well as the closely related BoNT-like neurotoxins produced by *C. baratii* and *C. butyricum.* The above-mentioned abbreviations are used throughout the present specification. For example, the nomenclature BoNT/A denotes the source of neurotoxin as BoNT (serotype A). Corresponding nomenclature applies to other BoNT serotypes.

BoNTs are the most potent toxins known, with median lethal dose (LD50) values for mice ranging from 0.5 to 5 ng/kg depending on the serotype. BoNTs are adsorbed in the gastrointestinal tract, and, after entering the general circulation, bind to the presynaptic membrane of cholinergic nerve terminals and prevent the release of their neurotransmitter acetylcholine. BoNT/B, BoNT/D, BoNT/F and BoNT/G cleave synaptobrevin/vesicle-associated membrane protein (VAMP); BoNT/C, BoNT/A and BoNT/E cleave the synaptosomal-associated protein of 25 kDa (SNAP-25); and BoNT/C cleaves syntaxin.

BoNTs share a common structure, being di-chain proteins of ~150 kDa, consisting of a heavy chain (H-chain) of ~100 kDa covalently joined by a single disulfide bond to a light chain (L-chain) of ~50 kDa. The H-chain consists of two domains, each of ~50 kDa. The C-terminal domain ($H_C$) is required for the high-affinity neuronal binding, whereas the N-terminal domain ($H_N$) is proposed to be involved in membrane translocation. The L-chain is a zinc-dependent metalloprotease responsible for the cleavage of the substrate SNARE protein.

The term L-chain fragment means a component of the L-chain of a neurotoxin, which fragment demonstrates a metalloprotease activity and is capable of proteolytically cleaving a vesicle and/or plasma membrane associated protein involved in cellular exocytosis.

Examples of suitable protease (reference) sequences include:

| | |
|---|---|
| Botulinum type A neurotoxin | - amino acid residues (1-448) |
| Botulinum type B neurotoxin | - amino acid residues (1-440) |
| Botulinum type C neurotoxin | - amino acid residues (1-441) |
| Botulinum type D neurotoxin | - amino acid residues (1-445) |
| Botulinum type E neurotoxin | - amino acid residues (1-422) |
| Botulinum type F neurotoxin | - amino acid residues (1-439) |
| Botulinum type G neurotoxin | - amino acid residues (1-441) |
| Tetanus neurotoxin | - amino acid residues (1-457) |
| IgA protease | - amino acid residues (1-959)* |

* Pohlner, J. et al. (1987). Nature 325, pp. 458-462, which is hereby incorporated by reference thereto.

The above-identified reference sequence should be considered a guide as slight variations may occur according to sub-serotypes. By way of example, US 2007/0166332 (hereby incorporated by reference thereto) cites slightly different clostridial sequences:

| | |
|---|---|
| Botulinum type A neurotoxin | - amino acid residues (M1-K448) |
| Botulinum type B neurotoxin | - amino acid residues (M1-K441) |
| Botulinum type C neurotoxin | - amino acid residues (M1-K449) |
| Botulinum type D neurotoxin | - amino acid residues (M1-R445) |
| Botulinum type E neurotoxin | - amino acid residues (M1-R422) |
| Botulinum type F neurotoxin | - amino acid residues (M1-K439) |
| Botulinum type G neurotoxin | - amino acid residues (M1-K446) |
| Tetanus neurotoxin | - amino acid residues (M1-A457) |

A variety of clostridial toxin fragments comprising the light chain can be useful in aspects of the present invention with the proviso that these light chain fragments can specifically target the core components of the neurotransmitter release apparatus and thus participate in executing the overall cellular mechanism whereby a clostridial toxin proteolytically cleaves a substrate. The light chains of clostridial toxins are approximately 420-460 amino acids in length and comprise an enzymatic domain. Research has shown that the entire length of a clostridial toxin light chain is not necessary for the enzymatic activity of the enzymatic domain. As a non-limiting example, the first eight amino acids of the BoNT/A light chain are not required for enzymatic activity. As another non-limiting example, the first eight amino acids of the TeNT light chain are not required for enzymatic activity. Likewise, the carboxyl-terminus of the light chain is not necessary for activity. As a non-limiting example, the last 32 amino acids of the BoNT/A light chain (residues 417-448) are not required for enzymatic activity. As another non-limiting example, the last 31 amino acids of the TeNT light chain (residues 427-457) are not required for enzymatic activity. Thus, aspects of this embodiment can include clostridial toxin light chains comprising an enzymatic domain having a length of, for example, at least 350 amino acids, at least 375 amino acids, at least 400 amino acids, at least 425 amino acids and at least 450 amino acids. Other aspects of this embodiment can include clostridial toxin light chains comprising an enzymatic domain having a length of, for example, at most 350 amino acids, at most 375 amino acids, at most 400 amino acids, at most 425 amino acids and at most 450 amino acids.

The non-cytotoxic protease component of the present invention preferably comprises a BoNT/A, BoNT/B or BoNT/D serotype L-chain (or fragment or variant thereof).

The polypeptides of the present invention, especially the protease component thereof, may be PEGylated - this may help to increase stability, for example duration of action of the protease component. PEGylation is particularly preferred when the protease comprises a BoNT/A, B or $C_1$ protease. PEGylation preferably includes the addition of PEG to the N-terminus of the protease component. By way of example, the N-terminus of a protease may be extended with one or more amino acid (e.g. cysteine) residues, which may be the same or different. One or more of said amino acid residues may have its own PEG molecule attached (e.g. covalently attached) thereto. An example of this technology is described in WO2007/104567, which is incorporated in its entirety by reference thereto.

A Translocation Domain is a molecule that enables translocation of a protease into a target cell such that a functional expression of protease activity occurs within the cytosol of the target cell. Whether any molecule (e.g. a protein or peptide) possesses the requisite translocation function of the present invention may be confirmed by any one of a number of conventional assays. For example, Shone C. (1987) describes an *in vitro* assay employing liposomes, which are challenged with a test molecule. Presence of the requisite translocation function is confirmed by release from the liposomes of $K^+$ and/ or labelled NAD, which may be readily monitored [see Shone C. (1987) Eur. J. Biochem; vol. 167(1): pp. 175-180].

A further example is provided by Blaustein R. (1987), which describes a simple *in vitro* assay employing planar phospholipid bilayer membranes. The membranes are challenged with a test molecule and the requisite translocation function is confirmed by an increase in conductance across said membranes [see Blaustein (1987) FEBS Letts; vol. 226, no. 1: pp. 115-120].

Additional methodology to enable assessment of membrane fusion and thus identification of Translocation Domains suitable for use in the present invention are provided by Methods in Enzymology Vol 220 and 221, Membrane Fusion Techniques, Parts A and B, Academic Press 1993.

The present invention also embraces variant translocation domains, so long as the variant domains still demonstrate the requisite translocation activity. By way of example, a variant may have at least 70%, preferably at least 80%, more preferably at least 90%, and most preferably at least 95% or at least 98% amino acid sequence homology with a reference translocation domain. The term fragment, when used in relation to a translocation domain, means a peptide having at least 20, preferably at least 40, more preferably at least 80, and most preferably at least 100 amino acid residues of the reference translocation domain. In the case of a clostridial translocation domain, the fragment preferably has at least 100, preferably at least 150, more preferably at least 200, and most preferably at least 250 amino acid residues of the reference translocation domain (eg. $H_N$ domain). As with the TM 'fragment' component (discussed above), translocation 'fragments' of the present invention embrace fragments of variant translocation domains based on the reference sequences.

The Translocation Domain is preferably capable of formation of ion-permeable pores in lipid membranes under conditions of low pH. Preferably it has been found to use only those portions of the protein molecule capable of pore-formation within the endosomal membrane.

The Translocation Domain may be obtained from a microbial protein source, in particular from a bacterial or viral protein source. Hence, in one embodiment, the Translocation Domain is a translocating domain of an enzyme, such as a bacterial toxin or viral protein.

It is well documented that certain domains of bacterial toxin molecules are capable of forming such pores. It is also known that certain translocation domains of virally expressed membrane fusion proteins are capable of forming such pores. Such domains may be employed in the present invention.

The Translocation Domain may be of a clostridial origin, such as the $H_N$ domain (or a functional component thereof). $H_N$ means a portion or fragment of the H-chain of a clostridial neurotoxin approximately equivalent to the amino-terminal half of the H-chain, or the domain corresponding to that fragment in the intact H-chain. The H-chain lacks the natural binding function of the $H_C$ component of the H-chain. In this regard, the $H_C$ function may be removed by deletion of the $H_C$ amino acid sequence (either at the DNA synthesis level, or at the post-synthesis level by nuclease or protease treatment). Alternatively, the $H_C$ function may be inactivated by chemical or biological treatment. Thus, the H-chain is incapable of binding to the Binding Site on a target cell to which native clostridial neurotoxin (i.e. holotoxin) binds.

Examples of suitable (reference) Translocation Domains include:

| | |
|---|---|
| Botulinum type A neurotoxin | - amino acid residues (449-871) |
| Botulinum type B neurotoxin | - amino acid residues (441-858) |
| Botulinum type C neurotoxin | - amino acid residues (442-866) |
| Botulinum type D neurotoxin | - amino acid residues (446-862) |
| Botulinum type E neurotoxin | - amino acid residues (423-845) |
| Botulinum type F neurotoxin | - amino acid residues (440-864) |
| Botulinum type G neurotoxin | - amino acid residues (442-863) |
| Tetanus neurotoxin | - amino acid residues (458-879) |

The above-identified reference sequence should be considered a guide as slight variations may occur according to sub-serotypes. By way of example, US 2007/0166332 (hereby incorporated by reference thereto) cites slightly different clostridial sequences:

| | |
|---|---|
| Botulinum type A neurotoxin | - amino acid residues (A449-K871) |
| Botulinum type B neurotoxin | - amino acid residues (A442-S858) |
| Botulinum type C neurotoxin | - amino acid residues (T450-N866) |
| Botulinum type D neurotoxin | - amino acid residues (D446-N862) |
| Botulinum type E neurotoxin | - amino acid residues (K423-K845) |
| Botulinum type F neurotoxin | - amino acid residues (A440-K864) |
| Botulinum type G neurotoxin | - amino acid residues (S447-S863) |
| Tetanus neurotoxin | - amino acid residues (S458-V879) |

In the context of the present invention, a variety of Clostridial toxin $H_N$ regions comprising a translocation domain can be useful in aspects of the present invention with the proviso that these active fragments can facilitate the release of a non-cytotoxic protease (e.g. a clostridial L-chain) from intracellular vesicles into the cytoplasm of the target cell and thus participate in executing the overall cellular mechanism whereby a clostridial toxin proteolytically cleaves a substrate. The $H_N$ regions from the heavy chains of Clostridial toxins are approximately 410-430 amino acids in length and comprise a translocation domain. Research has shown that the entire length of a $H_N$ region from a Clostridial toxin heavy chain is not necessary for the translocating activity of the translocation domain. Thus, aspects of this embodiment can include clostridial toxin $H_N$ regions comprising a translocation domain having a length of, for example, at least 350 amino acids, at least 375 amino acids, at least 400 amino acids and at least 425 amino acids. Other aspects of this embodiment can include clostridial toxin $H_N$ regions comprising translocation domain having a length of, for example, at most 350 amino acids, at most 375 amino acids, at most 400 amino acids and at most 425 amino acids.

For further details on the genetic basis of toxin production in *Clostridium botulinum* and *C. tetani,* we refer to Henderson et al (1997) in The Clostridia: Molecular Biology and Pathogenesis, Academic press.

The term $H_N$ embraces naturally-occurring neurotoxin $H_N$ portions, and modified $H_N$ portions having amino acid sequences that do not occur in nature and/ or synthetic amino acid residues, so long as the modified $H_N$ portions still demonstrate the above-mentioned translocation function.

Alternatively, the Translocation Domain may be of a non-clostridial origin. Examples of non-clostridial (reference) Translocation Domain origins include, but not be restricted to, the translocation domain of diphtheria toxin [O'Keefe et al., Proc. Natl. Acad. Sci. USA (1992) 89, 6202-6206; Silverman et al., J. Biol. Chem. (1993) 269, 22524-22532; and London, E. (1992) Biochem. Biophys. Acta., 1112, pp.25-51*],* the translocation domain of *Pseudomonas* exotoxin type A [Prior et al. Biochemistry (1992) 31, 3555-3559], the translocation domains of anthrax toxin [Blanke et al. Proc. Natl. Acad. Sci. USA (1996) 93, 8437-8442], a variety of fusogenic or hydrophobic peptides of translocating function [Plank et al. J. Biol. Chem. (1994) 269, 12918-12924; and Wagner et al (1992) PNAS, 89, pp.7934-7938], and amphiphilic peptides [Murata et al (1992) Biochem., 31, pp.1986-1992]. The Translocation Domain may mirror the Translocation Domain present in a naturally-occurring protein, or may include amino acid variations so long as the variations do not destroy the translocating ability of the Translocation Domain.

Particular examples of viral (reference) Translocation Domains suitable for use in the present invention include certain translocating domains of virally expressed membrane fusion proteins. For example, Wagner *et al.* (1992) and Murata *et al.* (1992) describe the translocation (i.e. membrane fusion and vesiculation) function of a number of fusogenic and amphiphilic peptides derived from the N-terminal region of influenza virus haemagglutinin. Other virally expressed membrane fusion proteins known to have the desired translocating activity are a translocating domain of a fusogenic peptide of Semliki Forest Virus (SFV), a translocating domain of vesicular stomatitis virus (VSV) glycoprotein G, a translocating domain of SER virus F protein and a translocating domain of Foamy virus envelope glycoprotein. Virally encoded Aspike proteins have particular application in the context of the present invention, for example, the E1 protein of SFV and the G protein of the G protein of VSV.

Use of the (reference) Translocation Domains listed in Table (below) includes use of sequence variants thereof. A variant may comprise one or more conservative nucleic acid substitutions and/ or nucleic acid deletions or insertions, with the proviso that the variant possesses the requisite translocating function. A variant may also comprise one or more amino acid substitutions and/ or amino acid deletions or insertions, so long as the variant possesses the requisite translocating function.

| **Trans location Domain source** | **Amino acid residues** | **References** |
|---|---|---|
| Diphtheria toxin | 194-380 | Silverman et al., 1994, J. Biol. Chem. 269, 22524-22532<br>London E., 1992, Biochem. Biophys. Acta., 1113, 25-51 |
| Domain II of pseudomonas exotoxin | 405-613 | Prior et al., 1992, Biochemistry 31, 3555-3559<br>Kihara & Pastan, 1994, Bioconj Chem. 5, 532-538 |
| Influenza virus haemagglutinin | GLFGAIAGFIENGWE GMIDGWYG, and Variants thereof | Plank et al., 1994, J. Biol. Chem. 269, 12918-12924<br>Wagner et al., 1992, PNAS, 89, 7934-7938<br>Murata et al., 1992, Biochemistry 31, 1986-1992 |
| Semliki Forest virus fusogenic protein | **Translocation domain** | Kielian et al., 1996, J Cell Biol. 134(4), 863-872 |
| Vesicular Stomatitis virus glycoprotein G | 118-139 | Yao et al., 2003, Virology 310(2), 319-332 |
| SER virus F protein | Translocation domain | Seth et al., 2003, J Virol 77(11) 6520-6527 |
| Foamy virus envelope glycoprotein | Translocation domain | Picard-Maureau et al., 2003, J Virol. 77(8), 4722-4730 |

The polypeptides of the present invention may further comprise a translocation facilitating domain. Said domain facilitates delivery of the non-cytotoxic protease into the cytosol of the target cell and are described, for example, in WO 08/008803 and WO 08/008805, each of which is herein incorporated by reference thereto.

By way of example, suitable translocation facilitating domains include an enveloped virus fusogenic peptide domain, for example, suitable fusogenic peptide domains include influenzavirus fusogenic peptide domain (eg. influenza A virus fusogenic peptide domain of 23 amino acids), alphavirus fusogenic peptide domain (eg. Semliki Forest virus fusogenic peptide domain of 26 amino acids), vesiculovirus fusogenic peptide domain (eg. vesicular stomatitis virus fusogenic peptide domain of 21 amino acids), respirovirus fusogenic peptide domain (eg. Sendai virus fusogenic peptide domain of 25 amino acids), morbiliivirus fusogenic peptide domain (eg. Canine distemper virus fusogenic peptide domain of 25 amino acids), avulavirus fusogenic peptide domain (eg. Newcastle disease virus fusogenic peptide domain of 25 amino acids), henipavirus fusogenic peptide domain (eg. Hendra virus fusogenic peptide domain of 25 amino acids), metapneumovirus fusogenic peptide domain (eg. Human metapneumovirus fusogenic peptide domain of 25 amino acids) or spumavirus fusogenic peptide domain such as simian foamy virus fusogenic peptide domain; or fragments or variants thereof.

By way of further example, a translocation facilitating domain may comprise a Clostridial toxin $H_{CN}$ domain or a fragment or variant thereof. In more detail, a Clostridial toxin $H_{CN}$ translocation facilitating domain may have a length of at least 200 amino acids, at least 225 amino acids, at least 250 amino acids, at least 275 amino acids. In this regard, a Clostridial toxin $H_{CN}$ translocation facilitating domain preferably has a length of at most 200 amino acids, at most 225 amino acids, at most 250 amino acids, or at most 275 amino acids. Specific (reference) examples include:

| | |
|---|---|
| Botulinum type A neurotoxin | - amino acid residues (872-1110) |
| Botulinum type B neurotoxin | - amino acid residues (859-1097) |
| Botulinum type C neurotoxin | - amino acid residues (867-1111) |
| Botulinum type D neurotoxin | - amino acid residues (863-1098) |
| Botulinum type E neurotoxin | - amino acid residues (846-1085) |
| Botulinum type F neurotoxin | - amino acid residues (865-1105) |
| Botulinum type G neurotoxin | - amino acid residues (864-1105) |
| Tetanus neurotoxin | - amino acid residues (880-1127) |

The above sequence positions may vary a little according to serotype/ subtype, and further examples of suitable (reference) Clostridial toxin $H_{CN}$ domains include:

| | |
|---|---|
| Botulinum type A neurotoxin | - amino acid residues (874-1110) |
| Botulinum type B neurotoxin | - amino acid residues (861-1097) |
| Botulinum type C neurotoxin | - amino acid residues (869-1111) |
| Botulinum type D neurotoxin | - amino acid residues (865-1098) |
| Botulinum type E neurotoxin | - amino acid residues (848-1085) |
| Botulinum type F neurotoxin | - amino acid residues (867-1105) |
| Botulinum type G neurotoxin | - amino acid residues (866-1105) |
| Tetanus neurotoxin | - amino acid residues (882-1127) |

Any of the above-described facilitating domains may be combined with any of the previously described translocation domain peptides that are suitable for use in the present invention. Thus, by way of example, a non-clostridial facilitating domain may be combined with non-clostridial translocation domain peptide or with clostridial translocation domain peptide. Alternatively, a Clostridial toxin $H_{CN}$ translocation facilitating domain may be combined with a non-clostridal translocation domain peptide. Alternatively, a Clostridial toxin $H_{CN}$ facilitating domain may be combined or with a clostridial translocation domain peptide, examples of which include:

| | |
|---|---|
| Botulinum type A neurotoxin | - amino acid residues (449-1110) |
| Botulinum type B neurotoxin | - amino acid residues (442-1097) |
| Botulinum type C neurotoxin | - amino acid residues (450-1111) |
| Botulinum type D neurotoxin | - amino acid residues (446-1098) |
| Botulinum type E neurotoxin | - amino acid residues (423-1085) |
| Botulinum type F neurotoxin | - amino acid residues (440-1105) |
| Botulinum type G neurotoxin | - amino acid residues (447-1105) |
| Tetanus neurotoxin | - amino acid residues (458-1127) |

Sequence homology:

Any of a variety of sequence alignment methods can be used to determine percent identity, including, without limitation, global methods, local methods and hybrid methods, such as, e.g., segment approach methods. Protocols to determine percent identity are routine procedures within the scope of one skilled in the. Global methods align sequences from the beginning to the end of the molecule and determine the best alignment by adding up scores of individual residue pairs and by imposing gap penalties. Non-limiting methods include, e.g., CLUSTAL W, see, e.g., Julie D. Thompson et al., CLUSTAL W: Improving the Sensitivity of Progressive Multiple Sequence Alignment Through Sequence Weighting, Position- Specific Gap Penalties and Weight Matrix Choice, 22(22) Nucleic Acids Research 4673-4680 (1994); and iterative refinement, see, e.g., Osamu Gotoh, Significant Improvement in Accuracy of Multiple Protein. Sequence Alignments by Iterative Refinement as Assessed by Reference to Structural Alignments, 264(4) J. Mol. Biol. 823-838 (1996). Local methods align sequences by identifying one or more conserved motifs shared by all of the input sequences. Non-limiting methods include, e.g., Match-box, see, e.g., Eric Depiereux and Ernest Feytmans, Match-Box: A Fundamentally New Algorithm for the Simultaneous Alignment of Several Protein Sequences, 8(5) CABIOS 501 -509 (1992); Gibbs sampling, see, e.g., C. E. Lawrence et al., Detecting Subtle Sequence Signals: A Gibbs Sampling Strategy for Multiple Alignment, 262(5131) Science 208-214 (1993); Align-M, see, e.g., Ivo Van Walle et al., Align-M - A New Algorithm for Multiple Alignment of Highly Divergent Sequences, 20(9) Bioinformatics:1428-1435 (2004).

Thus, percent sequence identity is determined by conventional methods. See, for example, Altschul et al., Bull. Math. Bio. 48: 603-16, 1986 and Henikoff and Henikoff, Proc. Natl. Acad. Sci. USA 89:10915-19, 1992. Briefly, two amino acid sequences are aligned to optimize the alignment scores using a gap opening penalty of 10, a gap extension penalty of 1, and the "blosum 62" scoring matrix of Henikoff and Henikoff (ibid.) as shown below (amino acids are indicated by the standard one-letter codes).

## Alignment scores for determining sequence identity

| | A | R | N | D | C | Q | E | G | H | I | L | K | M | F | P | S | T | W | Y | V |
|---|---|---|---|---|---|---|---|---|---|---|---|---|---|---|---|---|---|---|---|---|
| A | 4 | | | | | | | | | | | | | | | | | | | |
| R | -1 | 5 | | | | | | | | | | | | | | | | | | |
| N | -2 | 0 | 6 | | | | | | | | | | | | | | | | | |
| D | -2 | -2 | 1 | 6 | | | | | | | | | | | | | | | | |
| C | 0 | -3 | -3 | -3 | 9 | | | | | | | | | | | | | | | |
| Q | -1 | 1 | 0 | 0 | -3 | 5 | | | | | | | | | | | | | | |
| E | -1 | 0 | 0 | 2 | -4 | 2 | 5 | | | | | | | | | | | | | |
| G | 0 | -2 | 0 | -1 | -3 | -2 | -2 | 6 | | | | | | | | | | | | |
| H | -2 | 0 | 1 | -1 | -3 | 0 | 0 | -2 | 8 | | | | | | | | | | | |
| I | -1 | -3 | -3 | -3 | -1 | -3 | -3 | -4 | -3 | 4 | | | | | | | | | | |
| L | -1 | -2 | -3 | -4 | -1 | -2 | -3 | -4 | -3 | 2 | 4 | | | | | | | | | |
| K | -1 | 2 | 0 | -1 | -3 | 1 | 1 | -2 | -1 | -3 | -2 | 5 | | | | | | | | |
| M | -1 | -1 | -2 | -3 | -1 | 0 | -2 | -3 | -2 | 1 | 2 | -1 | 5 | | | | | | | |
| F | -2 | -3 | -3 | -3 | -2 | -3 | -3 | -3 | -1 | 0 | 0 | -3 | 0 | 6 | | | | | | |
| P | -1 | -2 | -2 | -1 | -3 | -1 | -1 | -2 | -2 | -3 | -3 | -1 | -2 | -4 | 7 | | | | | |
| S | 1 | -1 | 1 | 0 | -1 | 0 | 0 | 0 | -1 | -2 | -2 | 0 | -1 | -2 | -1 | 4 | | | | |
| T | 0 | -1 | 0 | -1 | -1 | -1 | -1 | -2 | -2 | -1 | -1 | -1 | -1 | -2 | -1 | 1 | 5 | | | |
| W | -3 | -3 | -4 | -4 | -2 | -2 | -3 | -2 | -2 | -3 | -2 | -3 | -1 | 1 | -4 | -3 | -2 | 11 | | |
| Y | -2 | -2 | -2 | -3 | -2 | -1 | -2 | -3 | 2 | -1 | -1 | -2 | -1 | 3 | -3 | -2 | -2 | 2 | 7 | |
| V | 0 | -3 | -3 | -3 | -1 | -2 | -2 | -3 | -3 | 3 | 1 | -2 | 1 | -1 | -2 | -2 | 0 | -3 | -1 | 4 |

The percent identity is then calculated as:

$$\frac{\text{Total number of identical matches}}{\text{[length of the longer sequence plus the number of gaps introduced into the longer sequence in order to align the two sequences]}} \times 100$$

Substantially homologous polypeptides are characterized as having one or more amino acid substitutions, deletions or additions. These changes are preferably of a minor nature, that is conservative amino acid substitutions (see below) and other substitutions that do not significantly affect the folding or activity of the polypeptide; small deletions, typically of one to about 30 amino acids; and small amino- or carboxyl-terminal extensions, such as an amino-terminal methionine residue, a small linker peptide of up to about 20-25 residues, or an affinity tag.

**Conservative amino acid substitutions**

| | |
|---|---|
| Basic: | arginine |
| | lysine |
| | histidine |
| Acidic: | glutamic acid |
| | aspartic acid |
| Polar: | glutamine |
| | asparagine |
| Hydrophobic: | leucine |
| | isoleucine |
| | valine |
| Aromatic: | phenylalanine |
| | tryptophan |
| | tyrosine |
| Small: | glycine |
| | alanine |
| | serine |
| | threonine |
| | methionine |

In addition to the 20 standard amino acids, non-standard amino acids (such as 4-hydroxyproline, 6-*N*-methyl lysine, 2-aminoisobutyric acid, isovaline and $\alpha$-methyl serine) may be substituted for amino acid residues of the polypeptides of the present invention. A limited number of non-conservative amino acids, amino acids that are not encoded by the genetic code, and unnatural amino acids may be substituted for clostridial polypeptide amino acid residues. The polypeptides of the present invention can also comprise non-naturally occurring amino acid residues.

Non-naturally occurring amino acids include, without limitation, trans-3-methylproline, 2,4-methano-proline, cis-4-hydroxyproline, trans-4-hydroxyproline, N-methylglycine, allo-threonine, methyl-threonine, hydroxy-ethylcysteine, hydroxyethylhomo-cysteine, nitro-glutamine, homoglutamine, pipecolic acid, tert-leucine, norvaline, 2-azaphenylalanine, 3-azaphenyl-alanine, 4-azaphenyl-alanine, and 4-fluorophenylalanine. Several methods are known in the art for incorporating non-naturally occurring amino acid residues into proteins. For example, an *in vitro* system can be employed wherein nonsense mutations are suppressed using chemically aminoacylated suppressor tRNAs. Methods for synthesizing amino acids and aminoacylating tRNA are known in the art. Transcription and translation of plasmids containing nonsense mutations is carried out in a cell free system comprising an *E. coli* S30 extract and commercially available enzymes and other reagents. Proteins are purified by chromatography. See, for example, Robertson et al., J. Am. Chem. Soc. 113:2722, 1991; Ellman et al., Methods Enzymol. 202:301, 1991; Chung et al., Science 259:806-9, 1993; and Chung et al., Proc. Natl. Acad. Sci. USA 90:10145-9, 1993). In a second method, translation is carried out in Xenopus oocytes by microinjection of mutated mRNA and chemically aminoacylated suppressor tRNAs (Turcatti et al., J. Biol. Chem. 271:19991-8, 1996). Within a third method, *E. coli* cells are cultured in the absence of a natural amino acid that is to be replaced (e.g., phenylalanine) and in the presence of the desired non-naturally occurring amino acid(s) (e.g., 2-azaphenylalanine, 3-azaphenylalanine, 4-azaphenylalanine, or 4-fluorophenylalanine). The non-naturally occurring amino acid is incorporated into the polypeptdie in place of its natural counterpart. See, Koide et al., Biochem. 33:7470-6, 1994. Naturally occurring amino acid residues can be converted to non-naturally occurring species by *in vitro* chemical modification. Chemical modification can be combined with site-directed mutagenesis to further expand the range of substitutions (Wynn and Richards, Protein Sci. 2:395-403, 1993).

A limited number of non-conservative amino acids, amino acids that are not encoded by the genetic code, non-naturally occurring amino acids, and unnatural amino acids may be substituted for amino acid residues of polypeptides of the present invention.

Essential amino acids in the polypeptides of the present invention can be identified according to procedures known in the art, such as site-directed mutagenesis or alanine-scanning mutagenesis (Cunningham and Wells, Science 244: 1081-5, 1989). Sites of biological interaction can also be determined by physical analysis of structure, as determined by such techniques as nuclear magnetic resonance, crystallography, electron diffraction or photoaffinity labeling, in conjunction with mutation of putative contact site amino acids. See, for example, de Vos et al., Science 255:306-12, 1992; Smith et al., J. Mol. Biol. 224:899-904, 1992; Wlodaver et al., FEBS Lett. 309:59-64, 1992. The identities of essential amino acids can also be inferred from analysis of homologies with related components (e.g. the translocation or protease components) of the polypeptides of the present invention.

Multiple amino acid substitutions can be made and tested using known methods of mutagenesis and screening, such as those disclosed by Reidhaar-Olson and Sauer (Science 241:53-7, 1988) or Bowie and Sauer (Proc. Natl. Acad. Sci. USA 86:2152-6, 1989). Briefly, these authors disclose methods for simultaneously randomizing two or more positions in a polypeptide, selecting for functional polypeptide, and then sequencing the mutagenised polypeptides to determine the spectrum of allowable substitutions at each position. Other methods that can be used include phage display (e.g., Lowman et al., Biochem. 30:10832-7, 1991; Ladner et al., U.S. Patent No. 5,223,409; Huse, WIPO Publication WO 92/06204) and region-directed mutagenesis (Derbyshire et al., Gene 46:145, 1986; Ner et al., DNA 7:127, 1988).

Multiple amino acid substitutions can be made and tested using known methods of mutagenesis and screening, such as those disclosed by Reidhaar-Olson and Sauer (Science 241:53-7, 1988) or Bowie and Sauer (Proc. Natl. Acad. Sci. USA 86:2152-6, 1989). Briefly, these authors disclose methods for simultaneously randomizing two or more positions in a polypeptide, selecting for functional polypeptide, and then sequencing the mutagenized polypeptides to determine the spectrum of allowable substitutions at each position. Other methods that can be used include phage display (e.g., Lowman et al., Biochem. 30:10832-7, 1991; Ladner et al., U.S. Patent No. 5,223,409; Huse, WIPO Publication WO 92/06204) and region-directed mutagenesis (Derbyshire et al., Gene 46:145, 1986; Ner et al., DNA 7:127, 1988).

There now follows a brief description of the Figures, which illustrate aspects and/ or embodiments of the present invention.

**Figure 1** - **Purification of $LH_N$/D-CT-CST28 fusion protein**

Using the methodology outlined in Example 5, a $LH_N$/D-CT-CST28 fusion protein was purified from *E. coli* BL21 (DE3) cells. Briefly, the soluble products obtained following cell disruption were applied to a nickel-charged affinity capture column. Bound proteins were eluted with 200 mM imidazole, treated with enterokinase to activate the fusion protein and then re-applied to a second nickel-charged affinity capture column. Samples from the purification procedure were assessed by SDS-PAGE. *Lane 1*: First nickel chelating Sepharose column eluant, *Lane 2*: Second nickel chelating Sepharose column eluant under non-reducing conditions, *Lane 3:* Second nickel chelating Sepharose column eluant under reducing conditions, *lane 4:* Molecular mass markers (kDa).

**Figure 2** - **Purification of $LH_N$/A-CT-SST14 fusion protein**

Using the methodology outlined in Example 6, an $LH_N$/A-CT-SST14 fusion protein was purified from *E. coli* BL21 (DE3) cells. Briefly, the soluble products obtained following cell disruption were applied to a nickel-charged affinity capture column. Bound proteins were eluted with 200 mM imidazole, treated with Factor Xa to activate the fusion protein and then re-applied to a second nickel-charged affinity capture column. Samples from the purification procedure were assessed by SDS-PAGE. *Lane 1*: First nickel chelating Sepharose column eluant, *Lane 2*: Molecular mass markers (kDa), *Lanes 3-4:* Second nickel chelating Sepharose column eluant under non-reducing conditions, Lanes 5-6: Second nickel chelating Sepharose column eluant under reducing conditions.

**Figure 3** - **Activity of SST-$LH_N$/A in cultured endocrine cells (AtT20)**

Figure 3a shows Inhibition of secretion of ACTH by SST-$LH_N$/A, and Figure 3b shows corresponding cleavage of SNAP-25 by SST-$LH_N$/A.

**Figure 4** - **Activity of SST-$LH_N$/D in cultured endocrine cells (GH3)**

Figure 4 shows the effect of growth hormone release from GH3 cells. Higher administration dosages of SST-$LH_N$/D result in a greater inhibition of growth hormone release.

**Figure 5** - **Activity of CP-GHRH-LHD on rat IGF-1 levels *in vivo***

Figure 5 shows the effects of i.v. administration of CP-GHRH-LHD (SXN101000) on rat IGF-1 levels 5 days after treatment compared to a vehical only control.

**Figure 6** - **Activity of CP-GHRH-LHD on rat IGF-1 levels *in vivo***

Figure 6 shows the effects of i.v. administration of CP-GHRH-LHD (SXN101000) on rat IGF-1 levels on day 1 to 8 days after treatment compared to a vehical only control. Due to the blocking of the cannula on days 9 and 10 have too few an n number to be considered.

**Figure 7** - **Activity of CP-GHRH-LHD on rat growth hormone levels *in vivo***

Figure 7b shows the effects of i.v. administration of CP-GHRH-LHD (SXN101000) on rat growth hormone levels on day 5 days after treatment compared to a vehical only control (figure 7a) and octreotide infusion (figure 7c).

**SEQ ID NOs**

1. DNA sequence of $LH_N$/A
2. DNA sequence of $LH_N$/B
3. DNA sequence of $LH_N$/C
4. DNA sequence of $LH_N$/D
5. DNA sequence of the human CP-EN-GS15-SST28 linker
6. DNA sequence of the human CT-GS20-CST28 linker
7. Protein sequence of the CP-CST14-GS20-LHD fusion
8. Protein sequence of the CP-CST14-GS30-LHD fusion
9. Protein sequence of the CP-CST28-GS20-LHD fusion
10. Protein sequence of the CP-CST28-GS30-LHD fusion
11. Protein sequence of the CP-SST14-GS20-LHD fusion
12. Protein sequence of the CP-SST14-GS30-LHD fusion
13. Protein sequence of the CP-SST28-GS20-LHD fusion
14. Protein sequence of the CP-SST28-GS30-LHD fusion
15. Protein sequence of the CT-CST14-GS20-LHD fusion
16. Protein sequence of the CT-CST14-GS30-LHD fusion
17. DNA sequence of the CT-CST28-GS20-LHD fusion
18. Protein sequence of the CT-CST28-GS20-LHD fusion
19. Protein sequence of the CT-CST28-GS30-LHD fusion
20. Protein sequence of the CT-SST14-GS15-L(#Fxa)HD fusion
21. Protein sequence of the CT-SST14-GS30-LHD fusion
22. Protein sequence of the CT-SST28-GS20-LHD fusion
23. Protein sequence of the CT-SST28-GS30-LHD fusion
24. Protein sequence of the CT-SST14-GS35-LHC fusion
25. DNA sequence of the CP-GS15-SST28-LHA fusion
26. Protein sequence of the CP-GS15-SST28-LHA fusion
27. Protein sequence of the CT-SST28-GS15-LHB fusion
28. Protein sequence of the CT-CST14-GS20-LHC fusion
29. Protein sequence of the CT-CST17-GS25-LHC fusion
30. Protein sequence of the CT-CST29-GS15-LHA fusion
31. Protein sequence of the CT-CST29-GS30-LHB fusion
32. DNA sequence of IgA-$H_N$tet
33. Protein sequence of the CT-GHRP-LHC fusion
34. Protein sequence of the CT-GHRH-LHD fusion
35. Protein sequence of the CT-GHRP-LHD fusion
36. Protein sequence of the CT-ghrelin-LHA fusion

37. Protein sequence of the IgA-$H_N$tet-CT-SST14 Fusion
38. Protein sequence of the IgA-$H_N$tet-CT-GHRP Fusion
39. Protein sequence of the CT-ghrelin S3W-LHA fusion
40. Protein sequence of the CT-GRP-LHD fusion
41. Protein sequence of the CT-GRP-LHB fusion
42. Protein sequence of the CP-qGHRH29-LHD fusion
43. Protein sequence of the CP-qGHRH-LHA fusion
44. Protein sequence of the CP-qGHRH-LHC fusion
45. Protein sequence of the CP-qGHRH-LHD fusion
46. Protein sequence of the CP-qGHRH-LHD N10-PL5 fusion
47. Protein sequence of the CP-qGHRH-LHD N10-HX12 fusion
48. Protein sequence of the CP-UTS-LHA fusion
49. Protein sequence of $LH_N$/A
50. Protein sequence of $LH_N$/B
51. Protein sequence of $LH_N$/C
52. Protein sequence of $LH_N$/D
53. Protein sequence of IgA-$H_N$tet
54.Synthesised Octreotide peptide
55.Synthesised GHRH agonist peptide
56.Synthesised GHRH antagonist peptide
57. Protein sequence of the CP-MCH-LHD fusion
58. Protein sequence of the CT-KISS-LHD fusion
59. Protein sequence of the CT-PrRP-LHA fusion
60. Protein sequence of the CP-HS_GHRH_1-27-LHD fusion
61. Protein sequence of the CP-HS_GHRH_1-28-LHD fusion
62. Protein sequence of the CP-HS_GHRH_1-29-LHD fusion
63. Protein sequence of the CP-HS_GHRH_1-44-LHD fusion
64. Protein sequence of the CP-HS_GHRH_1-40-LHD fusion
65. Protein sequence of the CP-HS_GHRH_Ala9-LHD fusion
66. Protein sequence of the CP-HS_GHRH_Ala22-LHD fusion
67. Protein sequence of the CP-HS_GHRH_Ala8_Lys11_1-29-LHD fusion
68. Protein sequence of the CP-HS_GHRH_Ala8_Lys11_Arg12_1-29-LHD fusion
69. Protein sequence of the CP-HS_GHRH_Ala8_Asn11_1-29-LHD fusion
70. Protein sequence of the CP-HS_GHRH_Ala8_Lys20_1-29-LHD fusion
71. Protein sequence of the CP-HS_GHRH_Ala8_Lys11_Lys20_1-29-LHD fusion
72. Protein sequence of the CP-HS_GHRH_Ala8_Asn20_1-29-LHD fusion
73. Protein sequence of the CP-HS_GHRH_Ala8_Asn12_1-29-LHD fusion
74. Protein sequence of the CP-HS_GHRH_Ala8_Asn21_1-29-LHD fusion
75. Protein sequence of the CP-HS_GHRH_Ala8_Glu_7_1-29-LHD fusion
76. Protein sequence of the CP-HS_GHRH_Ala8_Glu_10_1-29LHD fusion
77. Protein sequence of the CP-HS_GHRH_Ala8_Glu_13_1-29-LHD fusion
78. Protein sequence of the CP-HS_GHRH_Ala8-LHD fusion
79. Protein sequence of the CP-HS_GHRH_Glu8_1-29-LHD fusion
80. Protein sequence of the CP-HS_GHRH_Ala15_1-27-LHD fusion
81. Protein sequence of the CP-HS_GHRH_Ala15-LHD fusion
82. Protein sequence of the CP- HS_GHRH_Ala8_Ala15_1-29-LHD fusion
83. Protein sequence of the CP-HS_GHRH_Ala8_9_15_22_27-LHD fusion
84. Protein sequence of the CP-HS_GHRH_Ala8_9_15_22-LHD fusion
85. Protein sequence of the CP-HS_GHRH_HVQAL_1-32-LHD fusion
86. Protein sequence of the CP-HS_GHRH_HVSAL_1-29-LHD fusion
87. Protein sequence of the CP-HS_GHRH_HVTAL_1-29-LHD fusion
88. Protein sequence of the CP-HS_GHRH_QALN-LHD fusion
89. Protein sequence of the CP-HS_GHRH_QAL-LHD fusion
90. Protein sequence of the CP-hGHRH29 N8A M27L-LHD fusion
91. Protein sequence of the CP-hGHRH29 N8A K12N M27L-LHD fusion
92. Protein sequence of the N-terminal-hGHRH29 N8A M27L-LHD fusion
93. Protein sequence of the human GnRH-C fusion
94. Protein sequence of the human GnRH -D GS 20 fusion

## SUMMARY OF EXAMPLES

Example 1 Preparation of a LHA backbone construct

Example 2 Construction of LHA-CP-SST28

Example 3 Expression and purification of a LHA-CP-SST28 fusion protein

Example 4 Construction of LHD-CT-CST28

Example 5 Expression and purification of a LHD-CT-CST28 fusion protein

Example 6 Chemical conjugation of $LH_N$/A to SST TM

Example 7 Activity of SST-LHA in cultured endocrine cells (AtT20)

Example 8 Activity of SST-LHD in cultured neuroendocrine cells (GH3)

Example 9 Method for alleviating acromegalic symptoms by reducing elevated GH and IGF-1 levels resulting from pituitary adenoma

Example 10 Method for normalising swollen hirsute fingers by reducing elevated GH and IGF-1 levels resulting from pituitary adenoma

Example 11 Method for ameliorating the consequences of re-emerging growth-hormone-secreting pituitary adenoma

Example 12 Method for treating acromegalic patients resistant to somatostatin analogues

Example 13 Method for treating Cushing's disease in patients intolerant of somatostatin analogues

Example 14 Method for reversing female sexual impotence by treating prolactinoma

Example 15 Method for bringing about weight loss by treating insulinoma

Example 16 Method for treating glucagonoma

Example 17 Method for treating diarrhoea and flushing caused by VIPoma Example 18 Method for treating gastrinoma

Example 19 Method for treating thyrotoxicosis caused by thyrotrophinoma

Example 20 Method for treating recurrent soft tissue swelling caused by acromegaly

Example 21 Method for treating excessive facial hirsutism caused by Cushing's disease

Example 22 Method for treating male galactorrhoea caused by prolactinoma

Example 23 Method for treating multiple symptoms caused by insulinoma

Example 24 Method for treating acromegalic patients resistant to somatostatin analogues

Example 25 Method for treating Cushing's disease in patients intolerant of somatostatin analogues

Example 26 Method for reversing female sexual impotence by treating prolactinoma

Example 27 Method for treating Cushing's disease

Example 28 Method for treating gastrinoma

Example 29 Method for alleviating acromegalic symptoms by reducing elevated GH and IGF-1 levels resulting from pituitary adenoma

Example 30 Method for treating acromegalic patients resistant to somatostatin analogues

Example 31 Method for treating acromegaly

Example 32 Activity of CP-GHRH-LHD on rat IGF-1 levels *in vivo*

Example 33 Activity of CP-GHRH-LHD on rat IGF-1 levels *in vivo*

Example 34 Activity of CP-GHRH-LHD on rat growth hormone levels *in vivo*

SEQ IDs

1. DNA sequence of $LH_N$/A

```
ggatccATGGAGTTCGTTAACAAACAGTTCAACTATAAAGACCCAGTTAACGGTGTTGACATTGCTTAC
ATCAAAATCCCGAACGCTGGCCAGATGCAGCCGGTAAAGGCATTCAAAATCCACAACAAAATCTGGGTT
ATCCCGGAACGTGATACCTTTACTAACCCGGAAGAAGGTGACCTGAACCCGCCACCGGAAGCGAAACAG
GTGCCGGTATCTTACTATGACTCCACCTACCTGTCTACCGATAACGAAAAGGACAACTACCTGAAAGGT
GTTACTAAACTGTTCGAGCGTATTTACTCCACCGACCTGGGCCGTATGCTGCTGACTAGCATCGTTCGC
GGTATCCCGTTCTGGGGCGGTTCTACCATCGATACCGAACTGAAAGTAATCGACACTAACTGCATCAAC
GTTATTCAGCCGGACGGTTCCTATCGTTCCGAAGAACTGAACCTGGTGATCATCGGCCCGTCTGCTGAT
ATCATCCAGTTCGAGTGTCTGAGCTTTGGTCACGAAGTTCTGAACCTCACCCGTAACGGCTACGGTTCC
ACTCAGTACATCCGTTTCTCTCCGGACTTCACCTTCGGTTTTGAAGAATCCCTGGAAGTAGACACGAAC
CCACTGCTGGGCGCTGGTAAATTCGCAACTGATCCTGCGGTTACCCTGGCTCACGAACTGATTCATGCA
GGCCACCGCCTGTACGGTATCGCCATCAATCCGAACCGTGTCTTCAAAGTTAACACCAACGCGTATTAC
GAGATGTCCGGTCTGGAAGTTAGCTTCGAAGAACTGCGTACTTTTGGCGGTCACGACGCTAAATTCATC
GACTCTCTGCAAGAAAACGAGTTCCGTCTGTACTACTATAACAAGTTCAAAGATATCGCATCCACCCTG
AACAAAGCGAAATCCATCGTGGGTACCACTGCTTCTCTCCAGTACATGAAGAACGTTTTTAAAGAAAAA
TACCTGCTCAGCGAAGACACCTCCGGCAAATTCTCTGTAGACAAGTTGAAATTCGATAAACTTTACAAA
ATGCTGACTGAAATTTACACCGAAGACAACTTCGTTAAGTTCTTTAAAGTTCTGAACCGCAAAACCTAT
CTGAACTTCGACAAGGCAGTATTCAAAATCAACATCGTGCCGAAAGTTAACTACACTATCTACGATGGT
TTCAACCTGCGTAACACCAACCTGGCTGCTAATTTTAACGGCCAGAACACGGAAATCAACAACATGAAC
TTCACAAAACTGAAAAACTTCACTGGTCTGTTCGAGTTTTACAAGCTGCTGTGCGTCGACGGCATCATT
ACCTCCAAAACTAAATCTGACGATGACGATAAAAACAAAGCGCTGAACCTGCAGTGTATCAAGGTTAAC
AACTGGGATTTATTCTTCAGCCCGAGTGAAGACAACTTCACCAACGACCTGAACAAAGGTGAAGAAATC
ACCTCAGATACTAACATCGAAGCAGCCGAAGAAAACATCTCGCTGGACCTGATCCAGCAGTACTACCTG
ACCTTTAATTTCGACAACGAGCCGGAAAACATTTCTATCGAAAACCTGAGCTCTGATATCATCGGCCAG
CTGGAACTGATGCCGAACATCGAACGTTTCCCAAACGGTAAAAAGTACGAGCTGGACAAATATACCATG
TTCCACTACCTGCGCGCGCAGGAATTTGAACACGGCAAATCCCGTATCGCACTGACTAACTCCGTTAAC
GAAGCTCTGCTCAACCCGTCCCGTGTATACACCTTCTTCTCTAGCGACTACGTGAAAAAGGTCAACAAA
GCGACTGAAGCTGCAATGTTCTTGGGTTGGGTTGAACAGCTTGTTTATGATTTTACCGACGAGACGTCC
GAAGTATCTACTACCGACAAAATTGCGGATATCACTATCATCATCCCGTACATCGGTCCGGCTCTGAAC
ATTGGCAACATGCTGTACAAAGACGACTTCGTTGGCGCACTGATCTTCTCCGGTGCGGTGATCCTGCTG
GAGTTCATCCCGGAAATCGCCATCCCGGTACTGGGCACCTTTGCTCTGGTTTCTTACATTGCAAACAAG
GTTCTGACTGTACAAACCATCGACAACGCGCTGAGCAAACGTAACGAAAAATGGGATGAAGTTTACAAA
TATATCGTGACCAACTGGCTGGCTAAGGTTAATACTCAGATCGACCTCATCCGCAAAAAAATGAAAGAA
GCACTGGAAAACCAGGCGGAAGCTACCAAGGCAATCATTAACTACCAGTACAACCAGTACACCGAGGAA
GAAAAAAACAACATCAACTTCAACATCGACGATCTGTCCTCTAAACTGAACGAATCCATCAACAAAGCT
ATGATCAACATCAACAAGTTCCTGAACCAGTGCTCTGTAAGCTATCTGATGAACTCCATGATCCCGTAC
GGTGTTAAACGTCTGGAGGACTTCGATGCGTCTCTGAAAGACGCCCTGCTGAAATACATTTACGACAAC
CGTGGCACTCTGATCGGTCAGGTTGATCGTCTGAAGGACAAAGTGAACAATACCTTATCGACCGACATC
CCTTTTCAGCTCAGTAAATATGTCGATAACCAACGCCTTTTGTCCACTtaataagctt
```

2. DNA sequence of $LH_N$/B

GGATCCATGCCGGTTACCATCAACAACTTCAACTACAACGACCCGATCGACAACAACAACATCATTATG
ATGGAACCGCCGTTCGCACGTGGTACCGGACGTTACTACAAGGCTTTTAAGATCACCGACCGTATCTGG
ATCATCCCGGAACGTTACACCTTCGGTTACAAACCTGAGGACTTCAACAAGAGTAGCGGGATTTTCAAT
CGTGACGTCTGCGAGTACTATGATCCAGATTATCTGAATACCAACGATAAGAAGAACATATTCCTTCAG
ACTATGATTAAACTCTTCAACCGTATCAAAAGCAAACCGCTCGGTGAAAAACTCCTCGAAATGATTATC
AACGGTATCCCGTACCTCGGTGACCGTCGTGTCCCGCTTGAAGAGTTCAACACCAACATCGCAAGCGTC
ACCGTCAACAAACTCATCAGCAACCCAGGTGAAGTCGAACGTAAAAAAGGTATCTTCGCAAACCTCATC
ATCTTCGGTCCGGGTCCGGTCCTCAACGAAAACGAAACCATCGACATCGGTATCCAGAACCACTTCGCA
AGCCGTGAAGGTTTCGGTGGTATCATGCAGATGAAATTCTGCCCGGAATACGTCAGTGTCTTCAACAAC
GTCCAGGAAAACAAAGGTGCAAGCATCTTCAACCGTCGTGGTTACTTCAGCGACCCGGCACTCATCCTC
ATGCATGAACTCATCCACGTCCTCCACGGTCTCTACGGTATCAAAGTTGACGACCTCCCGATCGTCCCG
AACGAGAAGAAATTCTTCATGCAGAGCACCGACGCAATCCAGGCTGAGGAACTCTACACCTTCGGTGGC
CAAGACCCAAGTATCATAACCCCGTCCACCGACAAAAGCATCTACGACAAAGTCCTCCAGAACTTCAGG
GGTATCGTGGACAGACTCAACAAAGTCCTCGTCTGCATCAGCGACCCGAACATCAATATCAACATATAC
AAGAACAAGTTCAAAGACAAGTACAAATTCGTCGAGGACAGCGAAGGCAAATACAGCATCGACGTAGAA
AGTTTCGACAAGCTCTACAAAAGCCTCATGTTCGGTTTCACCGAAACCAACATCGCCGAGAACTACAAG
ATCAAGACAAGGGCAAGTTACTTCAGCGACAGCCTCCCGCCTGTCAAAATCAAGAACCTCTTAGACAAC
GAGATTTACACAATTGAAGAGGGCTTCAACATCAGTGACAAAGACATGGAGAAGGAATACAGAGGTCAG

AACAAGGCTATCAACAAACAGGCATACGAGGAGATCAGCAAAGAACACCTCGCAGTCTACAAGATCCAG
ATGTGCGTCGACGGCATCATTACCTCCAAAACTAAATCTGACGATGACGATAAAAACAAAGCGCTGAAC
CTGCAGTGCATCGACGTTGACAACGAAGACCTGTTCTTCATCGCTGACAAAAACAGCTTCAGTGACGAC
CTGAGCAAAAACGAACGTATCGAATACAACACCCAGAGCAACTACATCGAAAACGACTTCCCGATCAAC
GAACTGATCCTGGACACCGACCTGATAAGTAAAATCGAACTGCCGAGCGAAAACACCGAAAGTCTGACC
GACTTCAACGTTGACGTTCCGGTTTACGAAAAACAGCCGGCTATCAAGAAAATCTTCACCGACGAAAAC
ACCATCTTCCAGTACCTGTACAGCCAGACCTTCCCGCTGGACATCCGTGACATCAGTCTGACCAGCAGT
TTCGACGACGCTCTGCTGTTCAGCAACAAAGTTTACAGTTTCTTCAGCATGGACTACATCAAAACCGCT
AACAAAGTTGTTGAAGCAGGGCTGTTCGCTGGTTGGGTTAAACAGATCGTTAACGACTTCGTTATCGAA
GCTAACAAAAGCAACACTATGGACAAAATCGCTGACATCAGTCTGATCGTTCCGTACATCGGTCTGGCT
CTGAACGTTGGTAACGAAACCGCTAAAGGTAACTTTGAAAACGCTTTCGAGATCGCTGGTGCAAGCATC
CTGCTGGAGTTCATCCCGGAACTGCTGATCCCGGTTGTTGGTGCTTTCCTGCTGGAAAGTTACATCGAC
AACAAAAACAAGATCATCAAAACCATCGACAACGCTCTGACCAAACGTAACGAAAAATGGAGTGATATG
TACGGTCTGATCGTTGCTCAGTGGCTGAGCACCGTCAACACCCAGTTCTACACCATCAAAGAAGGTATG
TACAAAGCTCTGAACTACCAGGCTCAGGCTCTGGAAGAGATCATCAAATACCGTTACAACATCTACAGT
GAGAAGGAAAAGAGTAACATCAACATCGACTTCAACGACATCAACAGCAAACTGAACGAAGGTATCAAC
CAGGCTATCGACAACATCAACAACTTCATCAACGGTTGCAGTGTTAGCTACCTGATGAAGAAGATGATC
CCGCTGGCTGTTGAAAAACTGCTGGACTTCGACAACACCCTGAAAAAGAACCTGCTGAACTACATCGAC
GAAAACAAGCTGTACCTGATCGGTAGTGCTGAATACGAAAAAGTAAAGTGAACAAATACCTGAAGACC
ATCATGCCGTTCGACCTGAGTATCTACACCAACGACACCATCCTGATCGAAATGTTCAACAAATACAAC
TCTtaataagctt

3. DNA sequence of $LH_N$/C

ggatccATGCCGATCACCATCAACAACTTCAACTACAGCGATCCGGTGGATAACAAAAACATCCTGTAC
CTGGATACCCATCTGAATACCCTGGCGAACGAACCGGAAAAAGCGTTTCGTATCACCGGCAACATTTGG
GTTATTCCGGATCGTTTTAGCCGTAACAGCAACCCGAATCTGAATAAACCGCCGCGTGTTACCAGCCCG
AAAAGCGGTTATTACGATCCGAACTATCTGAGCACCGATAGCGATAAAGATACCTTCCTGAAAGAAATC
ATCAAACTGTTCAAACGCATCAACAGCCGTGAAATTGGCGAAGAACTGATCTATCGCCTGAGCACCGAT
ATTCCGTTTCCGGGCAACAACAACACCCCGATCAACACCTTTGATTTCGATGTGGATTTCAACAGCGTT
GATGTTAAAACCCGCCAGGGTAACAATTGGGTGAAAACCGGCAGCATTAACCCGAGCGTGATTATTACC
GGTCCGCGCGAAAACATTATTGATCCGGAAACCAGCACCTTTAAACTGACCAACAACACCTTTGCGGCG
CAGGAAGGTTTTGGCGCGCTGAGCATTATTAGCATTAGCCCGCGCTTTATGCTGACCTATAGCAACGCG
ACCAACGATGTTGGTGAAGGCCGTTTCAGCAAAAGCGAATTTTGCATGGACCCGATCCTGATCCTGATG
CATGAACTGAACCATGCGATGCATAACCTGTATGGCATCGCGATTCCGAACGATCAGACCATTAGCAGC
GTGACCAGCAACATCTTTTACAGCCAGTACAACGTGAAACTGGAATATGCGGAAATCTATGCGTTTGGC
GGTCCGACCATTGATCTGATTCCGAAAAGCGCGCGCAAATACTTCGAAGAAAAAGCGCTGGATTACTAT
CGCAGCATTGCGAAACGTCTGAACAGCATTACCACCGCGAATCCGAGCAGCTTCAACAAATATATCGGC
GAATATAAACAGAAACTGATCCGCAAATATCGCTTTGTGGTGGAAAGCAGCGGCGAAGTTACCGTTAAC
CGCAATAAATTCGTGGAACTGTACAACGAACTGACCCAGATCTTCACCGAATTTAACTATGCGAAAATC
TATAACGTGCAGAACCGTAAAATCTACCTGAGCAACGTGTATACCCCGGTGACCGCGAATATTCTGGAT
GATAACGTGTACGATATCCAGAACGGCTTTAACATCCCGAAAAGCAACCTGAACGTTCTGTTTATGGGC
CAGAACCTGAGCCGTAATCCGGCGCTGCGTAAAGTGAACCCGGAAAACATGCTGTACCTGTTCACCAAA
TTTTGCGTCGACGCGATTGATGGTCGTAGCCTGTACAACAAAACCCTGCAGTGTCGTGAACTGCTGGTG
AAAAACACCGATCTGCCGTTTATTGGCGATATCAGCGATGTGAAAACCGATATCTTCCTGCGCAAAGAT
ATCAACGAAGAAACCGAAGTGATCTACTACCCGGATAACGTGAGCGTTGATCAGGTGATCCTGAGCAAA
AACACCAGCGAACATGGTCAGCTGGATCTGCTGTATCCGAGCATTGATAGCGAAAGCGAAATTCTGCCG
GGCGAAAACCAGGTGTTTTACGATAACCGTACCCAGAACGTGGATTACCTGAACAGCTATTACTACCTG
GAAAGCCAGAAACTGAGCGATAACGTGGAAGATTTTACCTTTACCCGCAGCATTGAAGAAGCGCTGGAT
AACAGCGCGAAAGTTTACACCTATTTTCCGACCCTGGCGAACAAAGTTAATGCGGGTGTTCAGGGCGGT
CTGTTTCTGATGTGGGCGAACGATGTGGTGGAAGATTTCACCACCAACATCCTGCGTAAAGATACCCTG
GATAAAATCAGCGATGTTAGCGCGATTATTCCGTATATTGGTCCGGCGCTGAACATTAGCAATAGCGTG
CGTCGTGGCAATTTTACCGAAGCGTTTGCGGTTACCGGTGTGACCATTCTGCTGGAAGCGTTTCCGGAA
TTTACCATTCCGGCGCTGGGTGCGTTTGTGATCTATAGCAAAGTGCAGGAACGCAACGAAATCATCAAA
ACCATCGATAACTGCCTGGAACAGCGTATTAAACGCTGGAAAGATAGCTATGAATGGATGATGGGCACC
TGGCTGAGCCGTATTATCACCCAGTTCAACAACATCAGCTACCAGATGTACGATAGCCTGAACTATCAG
GCGGGTGCGATTAAAGCGAAAATCGATCTGGAATACAAAAAATACAGCGGCAGCGATAAAGAAAACATC
AAAAGCCAGGTTGAAAACCTGAAAAACAGCCTGGATGTGAAAATTAGCGAAGCGATGAATAACATCAAC
AAATTCATCCGCGAATGCAGCGTGACCTACCTGTTCAAAAACATGCTGCCGAAAGTGATCGATGAACTG
AACGAATTTGATCGCAACACCAAAGCGAAACTGATCAACCTGATCGATAGCCACAACATTATTCTGGTG
GGCGAAGTGGATAAACTGAAAGCGAAAGTTAACAACAGCTTCCAGAACACCATCCCGTTTAACATCTTC
AGCTATACCAACAACAGCCTGCTGAAAGATATCATCAACGAATACTTCAATtaataagctt

4. DNA sequence of $LH_N$/D

ggatccATGACGTGGCCAGTTAAGGATTTCAACTACTCAGATCCTGTAAATGACAACGATATTCTGTAC
CTTCGCATTCCACAAAATAAACTGATCACCACACCAGTCAAAGCATTCATGATTACTCAAAACATTTGG
GTCATTCCAGAACGCTTTTCTAGTGACACAAATCCGAGTTTATCTAAACCTCCGCGTCCGACGTCCAAA
TATCAGAGCTATTACGATCCCTCATATCTCAGTACGGACGAACAAAAAGATACTTTCCTTAAAGGTATC
ATTAAACTGTTTAAGCGTATTAATGAGCGCGATATCGGGAAAAAGTTGATTAATTATCTTGTTGTGGGT
TCCCCGTTCATGGGCGATAGCTCTACCCCCGAAGACACTTTTGATTTTACCCGTCATACGACAAACATC
GCGGTAGAGAAGTTTGAGAACGGATCGTGGAAAGTCACAAACATCATTACACCTAGCGTCTTAATTTTT
GGTCCGCTGCCAAACATCTTAGATTATACAGCCAGCCTGACTTTGCAGGGGCAACAGTCGAATCCGAGT
TTCGAAGGTTTTGGTACCCTGAGCATTCTGAAAGTTGCCCCGGAATTTCTGCTCACTTTTTCAGATGTC
ACCAGCAACCAGAGCTCAGCAGTATTAGGAAAGTCAATTTTTTGCATGGACCCGGTTATTGCACTGATG
CACGAACTGACGCACTCTCTGCATCAACTGTATGGGATCAACATCCCCAGTGACAAACGTATTCGTCCC
CAGGTGTCTGAAGGATTTTTCTCACAGGATGGGCCGAACGTCCAGTTCGAAGAGTTGTATACTTTCGGA
GGCCTGGACGTAGAGATCATTCCCCAGATTGAGCGCAGTCAGCTGCGTGAGAAGGCATTGGGCCATTAT
AAGGATATTGCAAAACGCCTGAATAACATTAACAAAACGATTCCATCTTCGTGGATCTCGAATATTGAT
AAATATAAGAAAATTTTTAGCGAGAAATATAATTTTGATAAAGATAATACAGGTAACTTTGTGGTTAAC
ATTGACAAATTCAACTCCCTTTACAGTGATTTGACGAATGTAATGAGCGAAGTTGTGTATAGTTCCCAA
TACAACGTTAAGAATCGTACCCATTACTTCTCTCGTCACTACCTGCCGGTTTTCGCGAACATCCTTGAC
GATAATATTTACACTATTCGTGACGGCTTTAACTTGACCAACAAGGGCTTCAATATTGAAAATTCAGGC
CAGAACATTGAACGCAACCCGGCCTTGCAGAAACTGTCGAGTGAATCCGTGGTTGACCTGTTTACCAAA
GTCTGCGTCGACAAAAGCGAAGAGAAGCTGTACGATGACGATGACAAAGATCGTTGGGGATCGTCCCTG
CAGTGTATTAAAGTGAAAAACAATCGGCTGCCTTATGTAGCAGATAAAGATAGCATTAGTCAGGAGATT
TTCGAAAATAAAATTATCACTGACGAAACCAATGTTCAGAATTATTCAGATAAATTTTCACTGGACGAA
AGCATCTTAGATGGCCAAGTTCCGATTAACCCGGAAATTGTTGATCCGTTACTGCCGAACGTGAATATG
GAACCGTTAAACCTCCCTGGCGAAGAGATCGTATTTTATGATGACATTACGAAATATGTGGACTACCTT
AATTCTTATTACTATTTGGAAAGCCAGAAACTGTCCAATAACGTGGAAAACATTACTCTGACCACAAGC
GTGGAAGAGGCTTTAGGCTACTCAAATAAGATTTATACCTTCCTCCCGTCGCTGGCGGAAAAAGTAAAT
AAAGGTGTGCAGGCTGGTCTGTTCCTCAACTGGGCGAATGAAGTTGTCGAAGACTTTACCACGAATATT
ATGAAAAAGGATACCCTGGATAAAATCTCCGACGTCTCGGTTATTATCCCATATATTGGCCCTGCGTTA
AATATCGGTAATAGTGCGCTGCGGGGGAATTTTAACCAGGCCTTTGCTACCGCGGGCGTCGCGTTCCTC
CTGGAGGGCTTTCCTGAATTTACTATCCCGGCGCTCGGTGTTTTTACATTTTACTCTTCCATCCAGGAG
CGTGAGAAAATTATCAAAACCATCGAAAACTGCCTGGAGCAGCGGGTGAAACGCTGGAAAGATTCTTAT
CAATGGATGGTGTCAAACTGGTTATCTCGCATCACGACCCAATTCAACCATATTAATTACCAGATGTAT
GATAGTCTGTCGTACCAAGCTGACGCCATTAAAGCCAAAATTGATCTGGAATATAAAAAGTACTCTGGT
AGCGATAAGGAGAACATCAAAAGCCAGGTGGAGAACCTTAAGAATAGTCTGGATGTGAAAATCTCTGAA
GCTATGAATAACATTAACAAATTCATTCGTGAATGTTCGGTGACGTACCTGTTCAAGAATATGCTGCCA
AAAGTTATTGATGAACTGAATAAATTTGATCTGCGTACCAAAACCGAACTTATCAACCTCATCGACTCC
CACAACATTATCCTTGTGGGCGAAGTGGATCGTCTGAAGGCCAAAGTAAACGAGAGCTTTGAAAATACG
ATGCCGTTTAATATTTTTTCATATACCAATAACTCCTTGCTGAAAGATATCATCAATGAATATTTCAAT
taataagctt

5. DNA sequence of the human CP-EN-GS15-SST28 linker

CATATGGGATCCGGTTTAAACGTCGACGGCATCATTACCTCCAAAACTAAATCTGACGATGACGATAAA
AGCGCCAATTCAAATCCTGCAATGGCGCCACGCGAACGCAAAGCTGGTTGCAAAAACTTCTTCTGGAAA
ACCTTCACCTCTTGCGCGCTAGCGGGCGGTGGCGGTAGCGGCGGTGGCGGTAGCGGCGGTGGCGGTAGC
GCACTAGTGCTGCAGCTAGAATAATGAAAGCTT

6. DNA sequence of the Human CT-GS20-CST28 linker

GGATCCGTCGACCTGCAGGGTCTAGAAGGCGGTGGCGGTAGCGGCGGTGGCGGTAGCGGCGGTGGCGGT
AGCGGCGGTGGCGGTAGCGCACTAGTGCAGGAAAGACCTCCATTACAACAACCTCCACATCGCGATAAG
AAACCATGTAAGAATTTCTTTTGGAAAACATTTAGCAGTTGCAAATGATAAAAGCTT

7. Protein sequence of the CP-CST14-GS20-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKPCKNFFWKTFSSCKALAGGGGSGGGGSGGGG
SALVLQCIKVKNNRLPYVADKDSISQEIFENKIITDETNVQNYSDKFSLDESILDGQVPINPEIVDPLL

PNVNMEPLNLPGEEIVFYDDITKYVDYLNSYYYLESQKLSNNVENITLTTSVEEALGYSNKIYTFLPSL
AEKVNKGVQAGLFLNWANEVVEDFTTNIMKKDTLDKISDVSVIIPYIGPALNIGNSALRGNFNQAFATA
GVAFLLEGFPEFTIPALGVFTFYSSIQEREKIIKTIENCLEQRVKRWKDSYQWMVSNWLSRITTQFNHI
NYQMYDSLSYQADAIKAKIDLEYKKYSGSDKENIKSQVENLKNSLDVKISEAMNNINKFIRECSVTYLF
KNMLPKVIDELNKFDLRTKTELINLIDSHNIILVGEVDRLKAKVNESFENTMPFNIFSYTNNSLLKDII
NEYFN

8. Protein sequence of the CP-CST14-GS30-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKPCKNFFWKTFSSCKALAGGGGSGGGGSGGGG
SGGGGSGGGGSALALQCIKVKNNRLPYVADKDSISQEIFENKIITDETNVQNYSDKFSLDESILDGQVP
INPEIVDPLLPNVNMEPLNLPGEEIVFYDDITKYVDYLNSYYYLESQKLSNNVENITLTTSVEEALGYS
NKIYTFLPSLAEKVNKGVQAGLFLNWANEVVEDFTTNIMKKDTLDKISDVSVIIPYIGPALNIGNSALR
GNFNQAFATAGVAFLLEGFPEFTIPALGVFTFYSSIQEREKIIKTIENCLEQRVKRWKDSYQWMVSNWL
SRITTQFNHINYQMYDSLSYQADAIKAKIDLEYKKYSGSDKENIKSQVENLKNSLDVKISEAMNNINKF
IRECSVTYLFKNMLPKVIDELNKFDLRTKTELINLIDSHNIILVGEVDRLKAKVNESFENTMPFNIFSY
TNNSLLKDIINEYFN

9. Protein sequence of the CP-CST28-GS20-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKQERPPLQQPPHRDKKPCKNFFWKTFSSCKAL
AGGGGSGGGGSGGGGSALVLQCIKVKNNRLPYVADKDSISQEIFENKIITDETNVQNYSDKFSLDESIL
DGQVPINPEIVDPLLPNVNMEPLNLPGEEIVFYDDITKYVDYLNSYYYLESQKLSNNVENITLTTSVEE
ALGYSNKIYTFLPSLAEKVNKGVQAGLFLNWANEVVEDFTTNIMKKDTLDKISDVSVIIPYIGPALNIG
NSALRGNFNQAFATAGVAFLLEGFPEFTIPALGVFTFYSSIQEREKIIKTIENCLEQRVKRWKDSYQWM
VSNWLSRITTQFNHINYQMYDSLSYQADAIKAKIDLEYKKYSGSDKENIKSQVENLKNSLDVKISEAMN
NINKFIRECSVTYLFKNMLPKVIDELNKFDLRTKTELINLIDSHNIILVGEVDRLKAKVNESFENTMPF
NIFSYTNNSLLKDIINEYFN

10. Protein sequence of the CP-CST28-GS30-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKQERPPLQQPPHRDKKPCKNFFWKTFSSCKAL
AGGGGSGGGGSGGGGSGGGGSGGGGSALVLQCIKVKNNRLPYVADKDSISQEIFENKIITDETNVQNYS
DKFSLDESILDGQVPINPEIVDPLLPNVNMEPLNLPGEEIVFYDDITKYVDYLNSYYYLESQKLSNNVE
NITLTTSVEEALGYSNKIYTFLPSLAEKVNKGVQAGLFLNWANEVVEDFTTNIMKKDTLDKISDVSVII
PYIGPALNIGNSALRGNFNQAFATAGVAFLLEGFPEFTIPALGVFTFYSSIQEREKIIKTIENCLEQRV
KRWKDSYQWMVSNWLSRITTQFNHINYQMYDSLSYQADAIKAKIDLEYKKYSGSDKENIKSQVENLKNS
LDVKISEAMNNINKFIRECSVTYLFKNMLPKVIDELNKFDLRTKTELINLIDSHNIILVGEVDRLKAKV
NESFENTMPFNIFSYTNNSLLKDIINEYFN

11. Protein sequence of the CP-SST14-GS20-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD

VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKAGCKNFFWKTFTSCALAGGGGSGGGGSGGGG
SALVLQCIKVKNNRLPYVADKDSISQEIFENKIITDETNVQNYSDKFSLDESILDGQVPINPEIVDPLL
PNVNMEPLNLPGEEIVFYDDITKYVDYLNSYYYLESQKLSNNVENITLTTSVEEALGYSNKIYTFLPSL
AEKVNKGVQAGLFLNWANEVVEDFTTNIMKKDTLDKISDVSVIIPYIGPALNIGNSALRGNFNQAFATA
GVAFLLEGFPEFTIPALGVFTFYSSIQEREKIIKTIENCLEQRVKRWKDSYQWMVSNWLSRITTQFNHI
NYQMYDSLSYQADAIKAKIDLEYKKYSGSDKENIKSQVENLKNSLDVKISEAMNNINKFIRECSVTYLF
KNMLPKVIDELNKFDLRTKTELINLIDSHNIILVGEVDRLKAKVNESFENTMPFNIFSYTNNSLLKDII
NEYFN

12. Protein sequence of the CP-SST14-GS30-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKAGCKNFFWKTFTSCALAGGGGSGGGGSGGGG
SGGGGSGGGGSALALQCIKVKNNRLPYVADKDSISQEIFENKIITDETNVQNYSDKFSLDESILDGQVP
INPEIVDPLLPNVNMEPLNLPGEEIVFYDDITKYVDYLNSYYYLESQKLSNNVENITLTTSVEEALGYS
NKIYTFLPSLAEKVNKGVQAGLFLNWANEVVEDFTTNIMKKDTLDKISDVSVIIPYIGPALNIGNSALR
GNFNQAFATAGVAFLLEGFPEFTIPALGVFTFYSSIQEREKIIKTIENCLEQRVKRWKDSYQWMVSNWL
SRITTQFNHINYQMYDSLSYQADAIKAKIDLEYKKYSGSDKENIKSQVENLKNSLDVKISEAMNNINKF
IRECSVTYLFKNMLPKVIDELNKFDLRTKTELINLIDSHNIILVGEVDRLKAKVNESFENTMPFNIFSY
TNNSLLKDIINEYFN

13. Protein sequence of the CP-SST28-GS20-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKSANSNPAMAPRERKAGCKNFFWKTFTSCALA
GGGGSGGGGSGGGGSALVLQCIKVKNNRLPYVADKDSISQEIFENKIITDETNVQNYSDKFSLDESILD
GQVPINPEIVDPLLPNVNMEPLNLPGEEIVFYDDITKYVDYLNSYYYLESQKLSNNVENITLTTSVEEA
LGYSNKIYTFLPSLAEKVNKGVQAGLFLNWANEVVEDFTTNIMKKDTLDKISDVSVIIPYIGPALNIGN
SALRGNFNQAFATAGVAFLLEGFPEFTIPALGVFTFYSSIQEREKIIKTIENCLEQRVKRWKDSYQWMV
SNWLSRITTQFNHINYQMYDSLSYQADAIKAKIDLEYKKYSGSDKENIKSQVENLKNSLDVKISEAMNN
INKFIRECSVTYLFKNMLPKVIDELNKFDLRTKTELINLIDSHNIILVGEVDRLKAKVNESFENTMPFN
IFSYTNNSLLKDIINEYFN

14. Protein sequence of the CP-SST28-GS30-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKSANSNPAMAPRERKAGCKNFFWKTFTSCALA
GGGGSGGGGSGGGGSGGGGSGGGGSALALQCIKVKNNRLPYVADKDSISQEIFENKIITDETNVQNYSD
KFSLDESILDGQVPINPEIVDPLLPNVNMEPLNLPGEEIVFYDDITKYVDYLNSYYYLESQKLSNNVEN
ITLTTSVEEALGYSNKIYTFLPSLAEKVNKGVQAGLFLNWANEVVEDFTTNIMKKDTLDKISDVSVIIP
YIGPALNIGNSALRGNFNQAFATAGVAFLLEGFPEFTIPALGVFTFYSSIQEREKIIKTIENCLEQRVK
RWKDSYQWMVSNWLSRITTQFNHINYQMYDSLSYQADAIKAKIDLEYKKYSGSDKENIKSQVENLKNSL
DVKISEAMNNINKFIRECSVTYLFKNMLPKVIDELNKFDLRTKTELINLIDSHNIILVGEVDRLKAKVN
ESFENTMPFNIFSYTNNSLLKDIINEYFN

15. Protein sequence of the CT-CST14-GS20-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDKSEEKLYDDDDKDRWGSSLQCIKVKNNRLPYVADKDSISQEIFEN
KIITDETNVQNYSDKFSLDESILDGQVPINPEIVDPLLPNVNMEPLNLPGEEIVFYDDITKYVDYLNSY
YYLESQKLSNNVENITLTTSVEEALGYSNKIYTFLPSLAEKVNKGVQAGLFLNWANEVVEDFTTNIMKK
DTLDKISDVSVIIPYIGPALNIGNSALRGNFNQAFATAGVAFLLEGFPEFTIPALGVFTFYSSIQEREK
IIKTIENCLEQRVKRWKDSYQWMVSNWLSRITTQFNHINYQMYDSLSYQADAIKAKIDLEYKKYSGSDK
ENIKSQVENLKNSLDVKISEAMNNINKFIRECSVTYLFKNMLPKVIDELNKFDLRTKTELINLIDSHNI
ILVGEVDRLKAKVNESFENTMPFNIFSYTNNSLLKDIINEYFNLEGGGGSGGGGSGGGGSALVPCKNFF
WKTFSSCK

16. Protein sequence of the CT-CST14-GS30-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDKSEEKLYDDDDKDRWGSSLQCIKVKNNRLPYVADKDSISQEIFEN
KIITDETNVQNYSDKFSLDESILDGQVPINPEIVDPLLPNVNMEPLNLPGEEIVFYDDITKYVDYLNSY
YYLESQKLSNNVENITLTTSVEEALGYSNKIYTFLPSLAEKVNKGVQAGLFLNWANEVVEDFTTNIMKK
DTLDKISDVSVIIPYIGPALNIGNSALRGNFNQAFATAGVAFLLEGFPEFTIPALGVFTFYSSIQEREK
IIKTIENCLEQRVKRWKDSYQWMVSNWLSRITTQFNHINYQMYDSLSYQADAIKAKIDLEYKKYSGSDK
ENIKSQVENLKNSLDVKISEAMNNINKFIRECSVTYLFKNMLPKVIDELNKFDLRTKTELINLIDSHNI
ILVGEVDRLKAKVNESFENTMPFNIFSYTNNSLLKDIINEYFNLEGGGGSGGGGSGGGGSGGGGSGGGG
SALVPCKNFFWKTFSSCK

17. DNA sequence of the CT-CST28-GS20-LHD fusion

GGATCCATGACGTGGCCAGTTAAGGATTTCAACTACTCAGATCCTGTAAATGACAACGATATTCTGTAC
CTTCGCATTCCACAAAATAAACTGATCACCACACCAGTCAAAGCATTCATGATTACTCAAAACATTTGG
GTCATTCCAGAACGCTTTTCTAGTGACACAAATCCGAGTTTATCTAAACCTCCGCGTCCGACGTCCAAA
TATCAGAGCTATTACGATCCCTCATATCTCAGTACGGACGAACAAAAAGATACTTTCCTTAAAGGTATC
ATTAAACTGTTTAAGCGTATTAATGAGCGCGATATCGGGAAAAAGTTGATTAATTATCTTGTTGTGGGT
TCCCCGTTCATGGGCGATAGCTCTACCCCCGAAGACACTTTTGATTTTACCCGTCATACGACAAACATC
GCGGTAGAGAAGTTTGAGAACGGATCGTGGAAAGTCACAAACATCATTACACCTAGCGTCTTAATTTTT
GGTCCGCTGCCAAACATCTTAGATTATACAGCCAGCCTGACTTTGCAGGGGCAACAGTCGAATCCGAGT
TTCGAAGGTTTTGGTACCCTGAGCATTCTGAAAGTTGCCCCGGAATTTCTGCTCACTTTTTCAGATGTC
ACCAGCAACCAGAGCTCAGCAGTATTAGGAAAGTCAATTTTTTGCATGGACCCGGTTATTGCACTGATG
CACGAACTGACGCACTCTCTGCATCAACTGTATGGGATCAACATCCCCAGTGACAAACGTATTCGTCCC
CAGGTGTCTGAAGGATTTTTCTCACAGGATGGGCCGAACGTCCAGTTCGAAGAGTTGTATACTTTCGGA
GGCCTGGACGTAGAGATCATTCCCCAGATTGAGCGCAGTCAGCTGCGTGAGAAGGCATTGGGCCATTAT
AAGGATATTGCAAAACGCCTGAATAACATTAACAAAACGATTCCATCTTCGTGGATCTCGAATATTGAT
AAATATAAGAAAATTTTTAGCGAGAAATATAATTTTGATAAAGATAATACAGGTAACTTTGTGGTTAAC
ATTGACAAATTCAACTCCCTTTACAGTGATTTGACGAATGTAATGAGCGAAGTTGTGTATAGTTCCCAA
TACAACGTTAAGAATCGTACCCATTACTTCTCTCGTCACTACCTGCCGGTTTTCGCGAACATCCTTGAC
GATAATATTTACACTATTCGTGACGGCTTTAACTTGACCAACAAGGGCTTCAATATTGAAAATTCAGGC
CAGAACATTGAACGCAACCCGGCCTTGCAGAAACTGTCGAGTGAATCCGTGGTTGACCTGTTTACCAAA
GTCTGCGTCGACAAAAGCGAAGAGAAGCTGTACGATGACGATGACAAAGATCGTTGGGGATCGTCCCTG
CAGTGTATTAAAGTGAAAAACAATCGGCTGCCTTATGTAGCAGATAAAGATAGCATTAGTCAGGAGATT
TTCGAAAATAAAATTATCACTGACGAAACCAATGTTCAGAATTATTCAGATAAATTTTCACTGGACGAA
AGCATCTTAGATGGCCAAGTTCCGATTAACCCGGAAATTGTTGATCCGTTACTGCCGAACGTGAATATG
GAACCGTTAAACCTCCCTGGCGAAGAGATCGTATTTTATGATGACATTACGAAATATGTGGACTACCTT
AATTCTTATTACTATTTGGAAAGCCAGAAACTGTCCAATAACGTGGAAAACATTACTCTGACCACAAGC
GTGGAAGAGGCTTTAGGCTACTCAAATAAGATTTATACCTTCCTCCCGTCGCTGGCGGAAAAAGTAAAT
AAAGGTGTGCAGGCTGGTCTGTTCCTCAACTGGGCGAATGAAGTTGTCGAAGACTTTACCACGAATATT
ATGAAAAAGGATACCCTGGATAAAATCTCCGACGTCTCGGTTATTATCCCATATATTGGCCCTGCGTTA
AATATCGGTAATAGTGCGCTGCGGGGGAATTTTAACCAGGCCTTTGCTACCGCGGGCGTCGCGTTCCTC
CTGGAGGGCTTTCCTGAATTTACTATCCCGGCGCTCGGTGTTTTTACATTTTACTCTTCCATCCAGGAG

CGTGAGAAAATTATCAAAACCATCGAAAACTGCCTGGAGCAGCGGGTGAAACGCTGGAAAGATTCTTAT
CAATGGATGGTGTCAAACTGGTTATCTCGCATCACGACCCAATTCAACCATATTAATTACCAGATGTAT
GATAGTCTGTCGTACCAAGCTGACGCCATTAAAGCCAAAATTGATCTGGAATATAAAAAGTACTCTGGT
AGCGATAAGGAGAACATCAAAAGCCAGGTGGAGAACCTTAAGAATAGTCTGGATGTGAAAATCTCTGAA
GCTATGAATAACATTAACAAATTCATTCGTGAATGTTCGGTGACGTACCTGTTCAAGAATATGCTGCCA
AAAGTTATTGATGAACTGAATAAATTTGATCTGCGTACCAAAACCGAACTTATCAACCTCATCGACTCC
CACAACATTATCCTTGTGGGCGAAGTGGATCGTCTGAAGGCCAAAGTAAACGAGAGCTTTGAAAATACG
ATGCCGTTTAATATTTTTTCATATACCAATAACTCCTTGCTGAAAGATATCATCAATGAATATTTCAAT
CTAGAAGGCGGTGGCGGTAGCGGCGGTGGCGGTAGCGGCGGTGGCGGTAGCGCACTAGTGCAGGAAAGA
CCTCCATTACAACAACCTCCACATCGCGATAAGAAACCATGTAAGAATTTCTTTTGGAAAACATTTAGC
AGTTGCAAAtaataagctt

18. Protein sequence of the CT-CST28-GS20-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDKSEEKLYDDDDKDRWGSSLQCIKVKNNRLPYVADKDSISQEIFEN
KIITDETNVQNYSDKFSLDESILDGQVPINPEIVDPLLPNVNMEPLNLPGEEIVFYDDITKYVDYLNSY
YYLESQKLSNNVENITLTTSVEEALGYSNKIYTFLPSLAEKVNKGVQAGLFLNWANEVVEDFTTNIMKK
DTLDKISDVSVIIPYIGPALNIGNSALRGNFNQAFATAGVAFLLEGFPEFTIPALGVFTFYSSIQEREK
IIKTIENCLEQRVKRWKDSYQWMVSNWLSRITTQFNHINYQMYDSLSYQADAIKAKIDLEYKKYSGSDK
ENIKSQVENLKNSLDVKISEAMNNINKFIRECSVTYLFKNMLPKVIDELNKFDLRTKTELINLIDSHNI
ILVGEVDRLKAKVNESFENTMPFNIFSYTNNSLLKDIINEYFNLEGGGGSGGGGSGGGGSALVQERPPL
QQPPHRDKKPCKNFFWKTFSSCK

19. Protein sequence of the CT-CST28-GS30-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDKSEEKLYDDDDKDRWGSSLQCIKVKNNRLPYVADKDSISQEIFEN
KIITDETNVQNYSDKFSLDESILDGQVPINPEIVDPLLPNVNMEPLNLPGEEIVFYDDITKYVDYLNSY
YYLESQKLSNNVENITLTTSVEEALGYSNKIYTFLPSLAEKVNKGVQAGLFLNWANEVVEDFTTNIMKK
DTLDKISDVSVIIPYIGPALNIGNSALRGNFNQAFATAGVAFLLEGFPEFTIPALGVFTFYSSIQEREK
IIKTIENCLEQRVKRWKDSYQWMVSNWLSRITTQFNHINYQMYDSLSYQADAIKAKIDLEYKKYSGSDK
ENIKSQVENLKNSLDVKISEAMNNINKFIRECSVTYLFKNMLPKVIDELNKFDLRTKTELINLIDSHNI
ILVGEVDRLKAKVNESFENTMPFNIFSYTNNSLLKDIINEYFNLEGGGGSGGGGSGGGGSGGGGSGGGG
SALVQERPPLQQPPHRDKKPCKNFFWKTFSSCK

20. Protein sequence of the CT-SST14-GS15-L(#Fxa)HD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSIDGRNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDKSEEKLYIDGRWGSSLQCIKVKNNRLPYVADKDSISQEIFENKII
TDETNVQNYSDKFSLDESILDGQVPINPEIVDPLLPNVNMEPLNLPGEEIVFYDDITKYVDYLNSYYYL
ESQKLSNNVENITLTTSVEEALGYSNKIYTFLPSLAEKVNKGVQAGLFLNWANEVVEDFTTNIMKKDTL
DKISDVSVIIPYIGPALNIGNSALRGNFNQAFATAGVAFLLEGFPEFTIPALGVFTFYSSIQEREKIIK
TIENCLEQRVKRWKDSYQWMVSNWLSRITTQFNHINYQMYDSLSYQADAIKAKIDLEYKKYSGSDKENI
KSQVENLKNSLDVKISEAMNNINKFIRECSVTYLFKNMLPKVIDELNKFDLRTKTELINLIDSHNIILV
GEVDRLKAKVNESFENTMPFNIFSYTNNSLLKDIINEYFNLEGGGGSGGGGSGGGGSALVAGCKNFFWK
TFTSC

21. Protein sequence of the CT-SST14-GS30-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDKSEEKLYDDDDKDRWGSSLQCIKVKNNRLPYVADKDSISQEIFEN
KIITDETNVQNYSDKFSLDESILDGQVPINPEIVDPLLPNVNMEPLNLPGEEIVFYDDITKYVDYLNSY
YYLESQKLSNNVENITLTTSVEEALGYSNKIYTFLPSLAEKVNKGVQAGLFLNWANEVVEDFTTNIMKK
DTLDKISDVSVIIPYIGPALNIGNSALRGNFNQAFATAGVAFLLEGFPEFTIPALGVFTFYSSIQEREK
IIKTIENCLEQRVKRWKDSYQWMVSNWLSRITTQFNHINYQMYDSLSYQADAIKAKIDLEYKKYSGSDK
ENIKSQVENLKNSLDVKISEAMNNINKFIRECSVTYLFKNMLPKVIDELNKFDLRTKTELINLIDSHNI
ILVGEVDRLKAKVNESFENTMPFNIFSYTNNSLLKDIINEYFNLEGGGGSGGGGSGGGGSGGGGSGGGG
SALVAGCKNFFWKTFTSC

22. Protein sequence of the CT-SST28-GS20-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDKSEEKLYDDDDKDRWGSSLQCIKVKNNRLPYVADKDSISQEIFEN
KIITDETNVQNYSDKFSLDESILDGQVPINPEIVDPLLPNVNMEPLNLPGEEIVFYDDITKYVDYLNSY
YYLESQKLSNNVENITLTTSVEEALGYSNKIYTFLPSLAEKVNKGVQAGLFLNWANEVVEDFTTNIMKK
DTLDKISDVSVIIPYIGPALNIGNSALRGNFNQAFATAGVAFLLEGFPEFTIPALGVFTFYSSIQEREK
IIKTIENCLEQRVKRWKDSYQWMVSNWLSRITTQFNHINYQMYDSLSYQADAIKAKIDLEYKKYSGSDK
ENIKSQVENLKNSLDVKISEAMNNINKFIRECSVTYLFKNMLPKVIDELNKFDLRTKTELINLIDSHNI
ILVGEVDRLKAKVNESFENTMPFNIFSYTNNSLLKDIINEYFNLEGGGGSGGGGSGGGGSALVSANSNP
AMAPRERKAGCKNFFWKTFTSC

23. Protein sequence of the CT-SST28-GS30-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDKSEEKLYDDDDKDRWGSSLQCIKVKNNRLPYVADKDSISQEIFEN
KIITDETNVQNYSDKFSLDESILDGQVPINPEIVDPLLPNVNMEPLNLPGEEIVFYDDITKYVDYLNSY
YYLESQKLSNNVENITLTTSVEEALGYSNKIYTFLPSLAEKVNKGVQAGLFLNWANEVVEDFTTNIMKK
DTLDKISDVSVIIPYIGPALNIGNSALRGNFNQAFATAGVAFLLEGFPEFTIPALGVFTFYSSIQEREK
IIKTIENCLEQRVKRWKDSYQWMVSNWLSRITTQFNHINYQMYDSLSYQADAIKAKIDLEYKKYSGSDK
ENIKSQVENLKNSLDVKISEAMNNINKFIRECSVTYLFKNMLPKVIDELNKFDLRTKTELINLIDSHNI
ILVGEVDRLKAKVNESFENTMPFNIFSYTNNSLLKDIINEYFNLEGGGGSGGGGSGGGGSGGGGSGGGG
SALVSANSNPAMAPRERKAGCKNFFWKTFTSC

24. Protein sequence of the CT-SST14-GS35-LHC fusion

PITINNFNYSDPVDNKNILYLDTHLNTLANEPEKAFRITGNIWVIPDRFSRNSNPNLNKPPRVTSPKSG
YYDPNYLSTDSDKDTFLKEIIKLFKRINSREIGEELIYRLSTDIPFPGNNNTPINTFDFDVDFNSVDVK
TRQGNNWVKTGSINPSVIITGPRENIIDPETSTFKLTNNTFAAQEGFGALSIISISPRFMLTYSNATND
VGEGRFSKSEFCMDPILILMHELNHAMHNLYGIAIPNDQTISSVTSNIFYSQYNVKLEYAEIYAFGGPT
IDLIPKSARKYFEEKALDYYRSIAKRLNSITTANPSSFNKYIGEYKQKLIRKYRFVVESSGEVTVNRNK
FVELYNELTQIFTEFNYAKIYNVQNRKIYLSNVYTPVTANILDDNVYDIQNGFNIPKSNLNVLFMGQNL
SRNPALRKVNPENMLYLFTKFCVDAIDGRSLYNKTLQCRELLVKNTDLPFIGDISDVKTDIFLRKDINE
ETEVIYYPDNVSVDQVILSKNTSEHGQLDLLYPSIDSESEILPGENQVFYDNRTQNVDYLNSYYYLESQ
KLSDNVEDFTFTRSIEEALDNSAKVYTYFPTLANKVNAGVQGGLFLMWANDVVEDFTTNILRKDTLDKI
SDVSAIIPYIGPALNISNSVRRGNFTEAFAVTGVTILLEAFPEFTIPALGAFVIYSKVQERNEIIKTID
NCLEQRIKRWKDSYEWMMGTWLSRIITQFNNISYQMYDSLNYQAGAIKAKIDLEYKKYSGSDKENIKSQ
VENLKNSLDVKISEAMNNINKFIRECSVTYLFKNMLPKVIDELNEFDRNTKAKLINLIDSHNIILVGEV

DKLKAKVNNSFQNTIPFNIFSYTNNSLLKDIINEYFNLEGGGGSGGGGSGGGGSGGGGSGGGGSGGGGS
GGGGSALVAGCKNFFWKTFTSC

25. DNA sequence of the CP-SST28-GS15-LHA fusion

ggatccATGGAGTTCGTTAACAAACAGTTCAACTATAAAGACCCAGTTAACGGTGTTGACATTGCTTAC
ATCAAAATCCCGAACGCTGGCCAGATGCAGCCGGTAAAGGCATTCAAAATCCACAACAAAATCTGGGTT
ATCCCGGAACGTGATACCTTTACTAACCCGGAAGAAGGTGACCTGAACCCGCCACCGGAAGCGAAACAG
GTGCCGGTATCTTACTATGACTCCACCTACCTGTCTACCGATAACGAAAAGGACAACTACCTGAAAGGT
GTTACTAAACTGTTCGAGCGTATTTACTCCACCGACCTGGGCCGTATGCTGCTGACTAGCATCGTTCGC
GGTATCCCGTTCTGGGGCGGTTCTACCATCGATACCGAACTGAAAGTAATCGACACTAACTGCATCAAC
GTTATTCAGCCGGACGGTTCCTATCGTTCCGAAGAACTGAACCTGGTGATCATCGGCCCGTCTGCTGAT
ATCATCCAGTTCGAGTGTCTGAGCTTTGGTCACGAAGTTCTGAACCTCACCCGTAACGGCTACGGTTCC
ACTCAGTACATCCGTTTCTCTCCGGACTTCACCTTCGGTTTTGAAGAATCCCTGGAAGTAGACACGAAC
CCACTGCTGGGCGCTGGTAAATTCGCAACTGATCCTGCGGTTACCCTGGCTCACGAACTGATTCATGCA
GGCCACCGCCTGTACGGTATCGCCATCAATCCGAACCGTGTCTTCAAAGTTAACACCAACGCGTATTAC
GAGATGTCCGGTCTGGAAGTTAGCTTCGAAGAACTGCGTACTTTTGGCGGTCACGACGCTAAATTCATC
GACTCTCTGCAAGAAAACGAGTTCCGTCTGTACTACTATAACAAGTTCAAAGATATCGCATCCACCCTG
AACAAAGCGAAATCCATCGTGGGTACCACTGCTTCTCTCCAGTACATGAAGAACGTTTTTAAAGAAAAA
TACCTGCTCAGCGAAGACACCTCCGGCAAATTCTCTGTAGACAAGTTGAAATTCGATAAACTTTACAAA
ATGCTGACTGAAATTTACACCGAAGACAACTTCGTTAAGTTCTTTAAAGTTCTGAACCGCAAAACCTAT
CTGAACTTCGACAAGGCAGTATTCAAAATCAACATCGTGCCGAAAGTTAACTACACTATCTACGATGGT
TTCAACCTGCGTAACACCAACCTGGCTGCTAATTTTAACGGCCAGAACACGGAAATCAACAACATGAAC
TTCACAAAACTGAAAAACTTCACTGGTCTGTTCGAGTTTTACAAGCTGCTGTGCGTCGACGGCATCATT
ACCTCCAAAACTAAATCTGACGATGACGATAAAAGCGCCAATTCAAATCCTGCAATGGCGCCACGCGAA
CGCAAAGCTGGATGCAAAAACTTCTTTTGGAAGACATTTACTAGTTGTGCGCTAGCGGGCGGTGGCGGT
AGCGGCGGTGGCGGTAGCGGCGGTGGCGGTAGCGCACTAGTGCTGCAGTGTATCAAGGTTAACAACTGG
GATTTATTCTTCAGCCCGAGTGAAGACAACTTCACCAACGACCTGAACAAAGGTGAAGAAATCACCTCA
GATACTAACATCGAAGCAGCCGAAGAAAACATCTCGCTGGACCTGATCCAGCAGTACTACCTGACCTTT
AATTTCGACAACGAGCCGGAAAACATTTCTATCGAAAACCTGAGCTCTGATATCATCGGCCAGCTGGAA
CTGATGCCGAACATCGAACGTTTCCCAAACGGTAAAAAGTACGAGCTGGACAAATATACCATGTTCCAC
TACCTGCGCGCGCAGGAATTTGAACACGGCAAATCCCGTATCGCACTGACTAACTCCGTTAACGAAGCT
CTGCTCAACCCGTCCCGTGTATACACCTTCTTCTCTAGCGACTACGTGAAAAAGGTCAACAAAGCGACT
GAAGCTGCAATGTTCTTGGGTTGGGTTGAACAGCTTGTTTATGATTTTACCGACGAGACGTCCGAAGTA
TCTACTACCGACAAAATTGCGGATATCACTATCATCATCCCGTACATCGGTCCGGCTCTGAACATTGGC
AACATGCTGTACAAAGACGACTTCGTTGGCGCACTGATCTTCTCCGGTGCGGTGATCCTGCTGGAGTTC
ATCCCGGAAATCGCCATCCCGGTACTGGGCACCTTTGCTCTGGTTTCTTACATTGCAAACAAGGTTCTG
ACTGTACAAACCATCGACAACGCGCTGAGCAAACGTAACGAAAAATGGGATGAAGTTTACAAATATATC
GTGACCAACTGGCTGGCTAAGGTTAATACTCAGATCGACCTCATCCGCAAAAAAATGAAAGAAGCACTG
GAAAACCAGGCGGAAGCTACCAAGGCAATCATTAACTACCAGTACAACCAGTACACCGAGGAAGAAAAA
AACAACATCAACTTCAACATCGACGATCTGTCCTCTAAACTGAACGAATCCATCAACAAAGCTATGATC
AACATCAACAAGTTCCTGAACCAGTGCTCTGTAAGCTATCTGATGAACTCCATGATCCCGTACGGTGTT
AAACGTCTGGAGGACTTCGATGCGTCTCTGAAAGACGCCCTGCTGAAATACATTTACGACAACCGTGGC
ACTCTGATCGGTCAGGTTGATCGTCTGAAGGACAAAGTGAACAATACCTTATCGACCGACATCCCTTTT
CAGCTCAGTAAATATGTCGATAACCAACGCCTTTTGTCCACTtaataagctt

26. Protein sequence of the CP-SST28-GS15-LHA fusion

EFVNKQFNYKDPVNGVDIAYIKIPNAGQMQPVKAFKIHNKIWVIPERDTFTNPEEGDLNPPPEAKQVPV
SYYDSTYLSTDNEKDNYLKGVTKLFERIYSTDLGRMLLTSIVRGIPFWGGSTIDTELKVIDTNCINVIQ
PDGSYRSEELNLVIIGPSADIIQFECLSFGHEVLNLTRNGYGSTQYIRFSPDFTFGFEESLEVDTNPLL
GAGKFATDPAVTLAHELIHAGHRLYGIAINPNRVFKVNTNAYYEMSGLEVSFEELRTFGGHDAKFIDSL
QENEFRLYYYNKFKDIASTLNKAKSIVGTTASLQYMKNVFKEKYLLSEDTSGKFSVDKLKFDKLYKMLT
EIYTEDNFVKFFKVLNRKTYLNFDKAVFKINIVPKVNYTIYDGFNLRNTNLAANFNGQNTEINNMNFTK
LKNFTGLFEFYKLLCVDGIITSKTKSDDDDKSANSNPAMAPRERKAGCKNFFWKTFTSCALAGGGGSGG
GGSGGGGSALVLQCIKVNNWDLFFSPSEDNFTNDLNKGEEITSDTNIEAAEENISLDLIQQYYLTFNFD
NEPENISIENLSSDIIGQLELMPNIERFPNGKKYELDKYTMFHYLRAQEFEHGKSRIALTNSVNEALLN
PSRVYTFFSSDYVKKVNKATEAAMFLGWVEQLVYDFTDETSEVSTTDKIADITIIIPYIGPALNIGNML
YKDDFVGALIFSGAVILLEFIPEIAIPVLGTFALVSYIANKVLTVQTIDNALSKRNEKWDEVYKYIVTN
WLAKVNTQIDLIRKKMKEALENQAEATKAIINYQYNQYTEEEKNNINFNIDDLSSKLNESINKAMININ
KFLNQCSVSYLMNSMIPYGVKRLEDFDASLKDALLKYIYDNRGTLIGQVDRLKDKVNNTLSTDIPFQLS
KYVDNQRLLST

27. Protein sequence of the CT-SST28-GS15-LHB fusion

PVTINNFNYNDPIDNNNIIMMEPPFARGTGRYYKAFKITDRIWIIPERYTFGYKPEDFNKSSGIFNRDV
CEYYDPDYLNTNDKKNIFLQTMIKLFNRIKSKPLGEKLLEMIINGIPYLGDRRVPLEEFNTNIASVTVN
KLISNPGEVERKKGIFANLIIFGPGPVLNENETIDIGIQNHFASREGFGGIMQMKFCPEYVSVFNNVQE
NKGASIFNRRGYFSDPALILMHELIHVLHGLYGIKVDDLPIVPNEKKFFMQSTDAIQAEELYTFGGQDP
SIITPSTDKSIYDKVLQNFRGIVDRLNKVLVCISDPNININIYKNKFKDKYKFVEDSEGKYSIDVESFD
KLYKSLMFGFTETNIAENYKIKTRASYFSDSLPPVKIKNLLDNEIYTIEEGFNISDKDMEKEYRGQNKA
INKQAYEEISKEHLAVYKIQMCVDGIITSKTKSDDDDKNKALNLQCIDVDNEDLFFIADKNSFSDDLSK
NERIEYNTQSNYIENDFPINELILDTDLISKIELPSENTESLTDFNVDVPVYEKQPAIKKIFTDENTIF
QYLYSQTFPLDIRDISLTSSFDDALLFSNKVYSFFSMDYIKTANKVVEAGLFAGWVKQIVNDFVIEANK
SNTMDKIADISLIVPYIGLALNVGNETAKGNFENAFEIAGASILLEFIPELLIPVVGAFLLESYIDNKN
KIIKTIDNALTKRNEKWSDMYGLIVAQWLSTVNTQFYTIKEGMYKALNYQAQALEEIIKYRYNIYSEKE
KSNINIDFNDINSKLNEGINQAIDNINNFINGCSVSYLMKKMIPLAVEKLLDFDNTLKKNLLNYIDENK
LYLIGSAEYEKSKVNKYLKTIMPFDLSIYTNDTILIEMFNKYNSLEGGGGSGGGGSGGGGSALDSANSN
PAMAPRERKAGCKNFFWKTFTSC

28. Protein sequence of the CT-CST14-GS20-LHC fusion

PITINNFNYSDPVDNKNILYLDTHLNTLANEPEKAFRITGNIWVIPDRFSRNSNPNLNKPPRVTSPKSG
YYDPNYLSTDSDKDTFLKEIIKLFKRINSREIGEELIYRLSTDIPFPGNNNTPINTFDFDVDFNSVDVK
TRQGNNWVKTGSINPSVIITGPRENIIDPETSTFKLTNNTFAAQEGFGALSIISISPRFMLTYSNATND
VGEGRFSKSEFCMDPILILMHELNHAMHNLYGIAIPNDQTISSVTSNIFYSQYNVKLEYAEIYAFGGPT
IDLIPKSARKYFEEKALDYYRSIAKRLNSITTANPSSFNKYIGEYKQKLIRKYRFVVESSGEVTVNRNK
FVELYNELTQIFTEFNYAKIYNVQNRKIYLSNVYTPVTANILDDNVYDIQNGFNIPKSNLNVLFMGQNL
SRNPALRKVNPENMLYLFTKFCVDAIDGRSLYNKTLQCRELLVKNTDLPFIGDISDVKTDIFLRKDINE
ETEVIYYPDNVSVDQVILSKNTSEHGQLDLLYPSIDSESEILPGENQVFYDNRTQNVDYLNSYYYLESQ
KLSDNVEDFTFTRSIEEALDNSAKVYTYFPTLANKVNAGVQGGLFLMWANDVVEDFTTNILRKDTLDKI
SDVSAIIPYIGPALNISNSVRRGNFTEAFAVTGVTILLEAFPEFTIPALGAFVIYSKVQERNEIIKTID
NCLEQRIKRWKDSYEWMMGTWLSRIITQFNNISYQMYDSLNYQAGAIKAKIDLEYKKYSGSDKENIKSQ
VENLKNSLDVKISEAMNNINKFIRECSVTYLFKNMLPKVIDELNEFDRNTKAKLINLIDSHNIILVGEV
DKLKAKVNNSFQNTIPFNIFSYTNNSLLKDIINEYFNLEGGGGSGGGGSGGGGSGGGGSALVAGCKNFF
WKTFTSC

29. Protein sequence of the CT-CST17-GS25-LHC fusion

PITINNFNYSDPVDNKNILYLDTHLNTLANEPEKAFRITGNIWVIPDRFSRNSNPNLNKPPRVTSPKSG
YYDPNYLSTDSDKDTFLKEIIKLFKRINSREIGEELIYRLSTDIPFPGNNNTPINTFDFDVDFNSVDVK
TRQGNNWVKTGSINPSVIITGPRENIIDPETSTFKLTNNTFAAQEGFGALSIISISPRFMLTYSNATND
VGEGRFSKSEFCMDPILILMHELNHAMHNLYGIAIPNDQTISSVTSNIFYSQYNVKLEYAEIYAFGGPT
IDLIPKSARKYFEEKALDYYRSIAKRLNSITTANPSSFNKYIGEYKQKLIRKYRFVVESSGEVTVNRNK
FVELYNELTQIFTEFNYAKIYNVQNRKIYLSNVYTPVTANILDDNVYDIQNGFNIPKSNLNVLFMGQNL
SRNPALRKVNPENMLYLFTKFCVDAIDGRSLYNKTLQCRELLVKNTDLPFIGDISDVKTDIFLRKDINE
ETEVIYYPDNVSVDQVILSKNTSEHGQLDLLYPSIDSESEILPGENQVFYDNRTQNVDYLNSYYYLESQ
KLSDNVEDFTFTRSIEEALDNSAKVYTYFPTLANKVNAGVQGGLFLMWANDVVEDFTTNILRKDTLDKI
SDVSAIIPYIGPALNISNSVRRGNFTEAFAVTGVTILLEAFPEFTIPALGAFVIYSKVQERNEIIKTID
NCLEQRIKRWKDSYEWMMGTWLSRIITQFNNISYQMYDSLNYQAGAIKAKIDLEYKKYSGSDKENIKSQ
VENLKNSLDVKISEAMNNINKFIRECSVTYLFKNMLPKVIDELNEFDRNTKAKLINLIDSHNIILVGEV
DKLKAKVNNSFQNTIPFNIFSYTNNSLLKDIINEYFNLEGGGGSGGGGSGGGGSGGGGSGGGGSALVDR
MPCRNFFWKTFSSCK

30. Protein sequence of the CT-CST29-GS15-LHA fusion

EFVNKQFNYKDPVNGVDIAYIKIPNAGQMQPVKAFKIHNKIWVIPERDTFTNPEEGDLNPPPEAKQVPV
SYYDSTYLSTDNEKDNYLKGVTKLFERIYSTDLGRMLLTSIVRGIPFWGGSTIDTELKVIDTNCINVIQ
PDGSYRSEELNLVIIGPSADIIQFECLSFGHEVLNLTRNGYGSTQYIRFSPDFTFGFEESLEVDTNPLL
GAGKFATDPAVTLAHELIHAGHRLYGIAINPNRVFKVNTNAYYEMSGLEVSFEELRTFGGHDAKFIDSL
QENEFRLYYYNKFKDIASTLNKAKSIVGTTASLQYMKNVFKEKYLLSEDTSGKFSVDKLKFDKLYKMLT
EIYTEDNFVKFFKVLNRKTYLNFDKAVFKINIVPKVNYTIYDGFNLRNTNLAANFNGQNTEINNMNFTK
LKNFTGLFEFYKLLCVDGIITSKTKSDDDDKNKALNLQCIKVNNWDLFFSPSEDNFTNDLNKGEEITSD
TNIEAAEENISLDLIQQYYLTFNFDNEPENISIENLSSDIIGQLELMPNIERFPNGKKYELDKYTMFHY
LRAQEFEHGKSRIALTNSVNEALLNPSRVYTFFSSDYVKKVNKATEAAMFLGWVEQLVYDFTDETSEVS
TTDKIADITIIIPYIGPALNIGNMLYKDDFVGALIFSGAVILLEFIPEIAIPVLGTFALVSYIANKVLT
VQTIDNALSKRNEKWDEVYKYIVTNWLAKVNTQIDLIRKKMKEALENQAEATKAIINYQYNQYTEEEKN
NINFNIDDLSSKLNESINKAMININKFLNQCSVSYLMNSMIPYGVKRLEDFDASLKDALLKYIYDNRGT

LIGQVDRLKDKVNNTLSTDIPFQLSKYVDNQRLLSTLEGGGGSGGGGSGGGGSALVQEGAPPQQSARRD
RMPCRNFFWKTFSSCK

31. Protein sequence of the CT-CST29-GS30-LHB fusion

PVTINNFNYNDPIDNNNIIMMEPPFARGTGRYYKAFKITDRIWIIPERYTFGYKPEDFNKSSGIFNRDV
CEYYDPDYLNTNDKKNIFLQTMIKLFNRIKSKPLGEKLLEMIINGIPYLGDRRVPLEEFNTNIASVTVN
KLISNPGEVERKKGIFANLIIFGPGPVLNENETIDIGIQNHFASREGFGGIMQMKFCPEYVSVFNNVQE
NKGASIFNRRGYFSDPALILMHELIHVLHGLYGIKVDDLPIVPNEKKFFMQSTDAIQAEELYTFGGQDP
SIITPSTDKSIYDKVLQNFRGIVDRLNKVLVCISDPNININIYKNKFKDKYKFVEDSEGKYSIDVESFD
KLYKSLMFGFTETNIAENYKIKTRASYFSDSLPPVKIKNLLDNEIYTIEEGFNISDKDMEKEYRGQNKA
INKQAYEEISKEHLAVYKIQMCVDGIITSKTKSDDDDKNKALNLQCIDVDNEDLFFIADKNSFSDDLSK
NERIEYNTQSNYIENDFPINELILDTDLISKIELPSENTESLTDFNVDVPVYEKQPAIKKIFTDENTIF
QYLYSQTFPLDIRDISLTSSFDDALLFSNKVYSFFSMDYIKTANKVVEAGLFAGWVKQIVNDFVIEANK
SNTMDKIADISLIVPYIGLALNVGNETAKGNFENAFEIAGASILLEFIPELLIPVVGAFLLESYIDNKN
KIIKTIDNALTKRNEKWSDMYGLIVAQWLSTVNTQFYTIKEGMYKALNYQAQALEEIIKYRYNIYSEKE
KSNINIDFNDINSKLNEGINQAIDNINNFINGCSVSYLMKKMIPLAVEKLLDFDNTLKKNLLNYIDENK
LYLIGSAEYEKSKVNKYLKTIMPFDLSIYTNDTILIEMFNKYNSLEGGGGSGGGGSGGGGSGGGGSGGG
GSGGGGSALDQEGAPPQQSARRDRMPCRNFFWKTFSSCK

32. DNA sequence of IgA-$H_N$tet

ggatccATGGAGTCCAATCAGCCGGAAAAAAATGGAACCGCGACTAAACCCGAGAATTCGGGGAACACT
ACGTCGGAAAACGGCCAGACGGAACCTGAGAAGAAACTGGAACTACGAAATGTGTCCGATATCGAGCTA
TACTCTCAAACCAATGGAACCTATAGGCAGCATGTTTCATTGGACGGAATCCCAGAAAATACGGATACA
TATTTCGTCAAAGTGAAGTCTAGCGCATTCAAGGATGTATATATCCCCGTTGCGAGTATTACAGAAGAG
AAGCGGAACGGTCAAAGCGTTTATAAGATTACAGCAAAGGCCGAAAAGTTACAACAGGAGTTAGAAAAC
AAATACGTTGACAATTTCACTTTTTATCTCGATAAAAAGGCTAAAGAGGAAAACACGAACTTCACGTCA
TTTAGTAATCTGGTCAAAGCCATAAATCAAAATCCATCTGGTACATACCATCTCGCGGCAAGTCTAAAC
GCGAATGAAGTAGAACTTGGCCCGGACGAGCGTTCATACATTAAGGATACCTTTACTGGCAGACTCATA
GGGGAAAAAGACGGTAAGAACTATGCTATATACAATTTGAAAAAGCCTTTATTTGAGAACCTGTCGGGC
GCCACCGTCGAGAAATTGTCCCTTAAAAACGTAGCTATAAGCGGAAAGAATGACATCGGTAGTCTTGCA
AACGAGGCTACTAACGGGACAAAGATTAAACAAGTGCACGTAGATGGGtgtgtcgacggcatcattacc
tccaaaactaaatctgacgatgacgataaaaacaaagcgctgaacctgcagtgcattaaaataaagaat
gaggatttgacattcatcgcagaaaaaaatagcttcagcgaagagccgttccaagatgagatagtaagc
tacaacaccaagaacaagccgcttaattttaattactcgttagataaaatcatagttgactacaacctt
caatcgaagatcacgttaccgaatgacagaacaactcctgtcacaaaaggaattccctatgcacctgag
tataagtcaaatgccgcgtcaacaatagagattcataatatagatgacaacaccatctatcaatatctg
tacgctcagaaaagtccaacaactcttcagcgtataacaatgaccaatagtgtcgatgacgcattgata
aattctaccaagatatactcttatttcccgagcgtcatctccaaagttaatcaaggtgctcaaggcatt
ctatttttgcaatgggtccgagacatcatagatgacttcactaatgagtcgtctcagaaaaccacgatt
gataaaatatcagatgtttccaccatcgtcccctacatcggacctgcgcttaacattgtgaagcagggg
tatgaggggaattttatcggagcgttagaaactacggggttgtgctattacttgaatacataccagag
ataacattgcccgttatagcggccctcagtatcgcagaatcaagtacacaaaaagaaaagataatcaaa
acaatcgacaacttcctagaaaagaggtacgaaaaatggatagaggtttataaactcgtgaaagcgaaa
tggttaggcactgttaatacgcagttccaaaagagatcctatcaaatgtatagatcactggagtaccag
gtggatgccataaagaaaattatcgactatgaatataaaatatattcaggtccagataaggagcagata
gctgatgaaataaacaatttaaaaaacaaacttgaagagaaggcgaataaggccatgatcaatatcaat
atttttatgcgagaatcttcacgatctttttttggtaaatcagatgattaacgaagccaaaaagcagctg
cttgagttcgacacacagtccaaaaacatactaatgcaatatatcaaagcaaactcaaaattcattgga
attactgagctgaagaaactggaatccaaaataaataaagtattctctaccccgatcccgttctcttac
tctaaaaaccttgactgctgggtagataacgaagaagatattgacgttctagagtaataagctt

33. Protein sequence of the CT-GHRP-LHC fusion

PITINNFNYSDPVDNKNILYLDTHLNTLANEPEKAFRITGNIWVIPDRFSRNSNPNLNKPPRVTSPKSG
YYDPNYLSTDSDKDTFLKEIIKLFKRINSREIGEELIYRLSTDIPFPGNNNTPINTFDFDVDFNSVDVK
TRQGNNWVKTGSINPSVIITGPRENIIDPETSTFKLTNNTFAAQEGFGALSIISISPRFMLTYSNATND
VGEGRFSKSEFCMDPILILMHELNHAMHNLYGIAIPNDQTISSVTSNIFYSQYNVKLEYAEIYAFGGPT
IDLIPKSARKYFEEKALDYYRSIAKRLNSITTANPSSFNKYIGEYKQKLIRKYRFVVESSGEVTVNRNK
FVELYNELTQIFTEFNYAKIYNVQNRKIYLSNVYTPVTANILDDNVYDIQNGFNIPKSNLNVLFMGQNL
SRNPALRKVNPENMLYLFTKFCVDAIDGRSLYNKTLQCRELLVKNTDLPFIGDISDVKTDIFLRKDINE
ETEVIYYPDNVSVDQVILSKNTSEHGQLDLLYPSIDSESEILPGENQVFYDNRTQNVDYLNSYYYLESQ
KLSDNVEDFTFTRSIEEALDNSAKVYTYFPTLANKVNAGVQGGLFLMWANDVVEDFTTNILRKDTLDKI

SDVSAIIPYIGPALNISNSVRRGNFTEAFAVTGVTILLEAFPEFTIPALGAFVIYSKVQERNEIIKTID
NCLEQRIKRWKDSYEWMMGTWLSRIITQFNNISYQMYDSLNYQAGAIKAKIDLEYKKYSGSDKENIKSQ
VENLKNSLDVKISEAMNNINKFIRECSVTYLFKNMLPKVIDELNEFDRNTKAKLINLIDSHNIILVGEV
DKLKAKVNNSFQNTIPFNIFSYTNNSLLKDIINEYFNLEGGGGSGGGGSGGGGSALVGSSFLSPEHQRV
QQRKESKKPPAKLQPR

34. Protein sequence of the CT-GHRH-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDKSEEKLYDDDDKDRWGSSLQCIKVKNNRLPYVADKDSISQEIFEN
KIITDETNVQNYSDKFSLDESILDGQVPINPEIVDPLLPNVNMEPLNLPGEEIVFYDDITKYVDYLNSY
YYLESQKLSNNVENITLTTSVEEALGYSNKIYTFLPSLAEKVNKGVQAGLFLNWANEVVEDFTTNIMKK
DTLDKISDVSVIIPYIGPALNIGNSALRGNFNQAFATAGVAFLLEGFPEFTIPALGVFTFYSSIQEREK
IIKTIENCLEQRVKRWKDSYQWMVSNWLSRITTQFNHINYQMYDSLSYQADAIKAKIDLEYKKYSGSDK
ENIKSQVENLKNSLDVKISEAMNNINKFIRECSVTYLFKNMLPKVIDELNKFDLRTKTELINLIDSHNI
ILVGEVDRLKAKVNESFENTMPFNIFSYTNNSLLKDIINEYFNLEGGGGSGGGGSGGGGSALVYADAIF
TNSYRKVLGQLSARKLLQDIMSRQQGESNQERGA

35. Protein sequence of the CT-GHRP-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDKSEEKLYDDDDKDRWGSSLQCIKVKNNRLPYVADKDSISQEIFEN
KIITDETNVQNYSDKFSLDESILDGQVPINPEIVDPLLPNVNMEPLNLPGEEIVFYDDITKYVDYLNSY
YYLESQKLSNNVENITLTTSVEEALGYSNKIYTFLPSLAEKVNKGVQAGLFLNWANEVVEDFTTNIMKK
DTLDKISDVSVIIPYIGPALNIGNSALRGNFNQAFATAGVAFLLEGFPEFTIPALGVFTFYSSIQEREK
IIKTIENCLEQRVKRWKDSYQWMVSNWLSRITTQFNHINYQMYDSLSYQADAIKAKIDLEYKKYSGSDK
ENIKSQVENLKNSLDVKISEAMNNINKFIRECSVTYLFKNMLPKVIDELNKFDLRTKTELINLIDSHNI
ILVGEVDRLKAKVNESFENTMPFNIFSYTNNSLLKDIINEYFNLEGGGGSGGGGSGGGGSALVGSSFLS
PEHQRVQQRKESKKPPAKLQPR

36. Protein sequence of the CT-ghrelin-LHA fusion

EFVNKQFNYKDPVNGVDIAYIKIPNAGQMQPVKAFKIHNKIWVIPERDTFTNPEEGDLNPPPEAKQVPV
SYYDSTYLSTDNEKDNYLKGVTKLFERIYSTDLGRMLLTSIVRGIPFWGGSTIDTELKVIDTNCINVIQ
PDGSYRSEELNLVIIGPSADIIQFECKSFGHEVLNLTRNGYGSTQYIRFSPDFTFGFEESLEVDTNPLL
GAGKFATDPAVTLAHELIHAGHRLYGIAINPNRVFKVNTNAYYEMSGLEVSFEELRTFGGHDAKFIDSL
QENEFRLYYYNKFKDIASTLNKAKSIVGTTASLQYMKNVFKEKYLLSEDTSGKFSVDKLKFDKLYKMLT
EIYTEDNFVKFFKVLNRKTYLNFDKAVFKINIVPKVNYTIYDGFNLRNTNLAANFNGQNTEINNMNFTK
LKNFTGLFEFYKLLCVDGIITSKTKSDDDDKNKALNLQCIKVNNWDLFFSPSEDNFTNDLNKGEEITSD
TNIEAAEENISLDLIQQYYLTFNFDNEPENISIENLSSDIIGQLELMPNIERFPNGKKYELDKYTMFHY
LRAQEFEHGKSRIALTNSVNEALLNPSRVYTFFSSDYVKKVNKATEAAMFLGWVEQLVYDFTDETSEVS
TTDKIADITIIIPYIGPALNIGNMLYKDDFVGALIFSGAVILLEFIPEIAIPVLGTFALVSYIANKVLT
VQTIDNALSKRNEKWDEVYKYIVTNWLAKVNTQIDLIRKKMKEALENQAEATKAIINYQYNQYTEEEKN
NINFNIDDLSSKLNESINKAMININKFLNQCSVSYLMNSMIPYGVKRLEDFDASLKDALLKYIYDNRGT
LIGQVDRLKDKVNNTLSTDIPFQLSKYVDNQRLLSTLEGGGGSGGGGSGGGGSALVGSSFLSPEHQRVQ
QRKESKKPPAKLQPR

37. Protein sequence of the IgA-$H_N$tet-CT-SST14 Fusion

ESNQPEKNGTATKPENSGNTTSENGQTEPEKKLELRNVSDIELYSQTNGTYRQHVSLDGIPENTDTYFVKV
KSSAFKDVYIPVASITEEKRNGQSVYKITAKAEKLQQELENKYVDNFTFYLDKKAKEENTNFTSFSNLVKA
INQNPSGTYHLAASLNANEVELGPDERSYIKDTFTGRLIGEKDGKNYAIYNLKKPLFENLSGATVEKLSLK
NVAISGKNDIGSLANEATNGTKIKQVHVDGCVDGIITSKTKSDDDDKNKALNLQCIKIKNEDLTFIAEKNS
FSEEPFQDEIVSYNTKNKPLNFNYSLDKIIVDYNLQSKITLPNDRTTPVTKGIPYAPEYKSNAASTIEIHN

IDDNTIYQYLYAQKSPTTLQRITMTNSVDDALINSTKIYSYFPSVISKVNQGAQGILFLQWVRDIIDDFTN
ESSQKTTIDKISDVSTIVPYIGPALNIVKQGYEGNFIGALETTGVVLLLEYIPEITLPVIAALSIAESSTQ
KEKIIKTIDNFLEKRYEKWIEVYKLVKAKWLGTVNTQFQKRSYQMYRSLEYQVDAIKKIIDYEYKIYSGPD
KEQIADEINNLKNKLEEKANKAMININIFMRESSRSFLVNQMINEAKKQLLEFDTQSKNILMQYIKANSKF
IGITELKKLESKINKVFSTPIPFSYSKNLDCWVDNEEDIDVLEGGGGSGGGGSGGGGSALVAGCKNFFWKT
FTSC

38. Protein sequence of the IgA-$H_N$tet-CT-GHRP Fusion

ESNQPEKNGTATKPENSGNTTSENGQTEPEKKLELRNVSDIELYSQTNGTYRQHVSLDGIPENTDTYFVKV
KSSAFKDVYIPVASITEEKRNGQSVYKITAKAEKLQQELENKYVDNFTFYLDKKAKEENTNFTSFSNLVKA
INQNPSGTYHLAASLNANEVELGPDERSYIKDTFTGRLIGEKDGKNYAIYNLKKPLFENLSGATVEKLSLK
NVAISGKNDIGSLANEATNGTKIKQVHVDGCVDGIITSKTKSDDDDKNKALNLQCIKIKNEDLTFIAEKNS
FSEEPFQDEIVSYNTKNKPLNFNYSLDKIIVDYNLQSKITLPNDRTTPVTKGIPYAPEYKSNAASTIEIHN
IDDNTIYQYLYAQKSPTTLQRITMTNSVDDALINSTKIYSYFPSVISKVNQGAQGILFLQWVRDIIDDFTN
ESSQKTTIDKISDVSTIVPYIGPALNIVKQGYEGNFIGALETTGVVLLLEYIPEITLPVIAALSIAESSTQ
KEKIIKTIDNFLEKRYEKWIEVYKLVKAKWLGTVNTQFQKRSYQMYRSLEYQVDAIKKIIDYEYKIYSGPD
KEQIADEINNLKNKLEEKANKAMININIFMRESSRSFLVNQMINEAKKQLLEFDTQSKNILMQYIKANSKF
IGITELKKLESKINKVFSTPIPFSYSKNLDCWVDNEEDIDVLEGGGGSGGGGSGGGGSALVGSSFLSPEHQ
RVQQRKESKKPPAKLQPR

39. Protein sequence of the CT-ghrelin S3W-LHA fusion

EFVNKQFNYKDPVNGVDIAYIKIPNAGQMQPVKAFKIHNKIWVIPERDTFTNPEEGDLNPPPEAKQVPVSY
YDSTYLSTDNEKDNYLKGVTKLFERIYSTDLGRMLLTSIVRGIPFWGGSTIDTELKVIDTNCINVIQPDGS
YRSEELNLVIIGPSADIIQFECKSFGHEVLNLTRNGYGSTQYIRFSPDFTFGFEESLEVDTNPLLGAGKFA
TDPAVTLAHELIHAGHRLYGIAINPNRVFKVNTNAYYEMSGLEVSFEELRTFGGHDAKFIDSLQENEFRLY
YYNKFKDIASTLNKAKSIVGTTASLQYMKNVFKEKYLLSEDTSGKFSVDKLKFDKLYKMLTEIYTEDNFVK
FFKVLNRKTYLNFDKAVFKINIVPKVNYTIYDGFNLRNTNLAANFNGQNTEINNMNFTKLKNFTGLFEFYK
LLCVDGIITSKTKSDDDDKNKALNLQCIKVNNWDLFFSPSEDNFTNDLNKGEEITSDTNIEAAEENISLDL
IQQYYLTFNFDNEPENISIENLSSDIIGQLELMPNIERFPNGKKYELDKYTMFHYLRAQEFEHGKSRIALT
NSVNEALLNPSRVYTFFSSDYVKKVNKATEAAMFLGWVEQLVYDFTDETSEVSTTDKIADITIIIPYIGPA
LNIGNMLYKDDFVGALIFSGAVILLEFIPEIAIPVLGTFALVSYIANKVLTVQTIDNALSKRNEKWDEVYK
YIVTNWLAKVNTQIDLIRKKMKEALENQAEATKAIINYQYNQYTEEEKNNINFNIDDLSSKLNESINKAMI
NINKFLNQCSVSYLMNSMIPYGVKRLEDFDASLKDALLKYIYDNRGTLIGQVDRLKDKVNNTLSTDIPFQL
SKYVDNQRLLSTLEIYALVGSWFLSPEHQRVQQRKESKKPPAKLQPR

40. Protein sequence of the CT-GRP-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDKSEEKLYDDDDKDRWGSSLQCIKVKNNRLPYVADKDSISQEIFEN
KIITDETNVQNYSDKFSLDESILDGQVPINPEIVDPLLPNVNMEPLNLPGEEIVFYDDITKYVDYLNSY
YYLESQKLSNNVENITLTTSVEEALGYSNKIYTFLPSLAEKVNKGVQAGLFLNWANEVVEDFTTNIMKK
DTLDKISDVSVIIPYIGPALNIGNSALRGNFNQAFATAGVAFLLEGFPEFTIPALGVFTFYSSIQEREK
IIKTIENCLEQRVKRWKDSYQWMVSNWLSRITTQFNHINYQMYDSLSYQADAIKAKIDLEYKKYSGSDK
ENIKSQVENLKNSLDVKISEAMNNINKFIRECSVTYLFKNMLPKVIDELNKFDLRTKTELINLIDSHNI
ILVGEVDRLKAKVNESFENTMPFNIFSYTNNSLLKDIINEYFNLEGGGGSGGGGSGGGGSALVGNHWAV
GHLM

41. Protein sequence of the CT-GRP-LHB fusion

PVTINNFNYNDPIDNNNIIMMEPPFARGTGRYYKAFKITDRIWIIPERYTFGYKPEDFNKSSGIFNRDV
CEYYDPDYLNTNDKKNIFLQTMIKLFNRIKSKPLGEKLLEMIINGIPYLGDRRVPLEEFNTNIASVTVN
KLISNPGEVERKKGIFANLIIFGPGPVLNENETIDIGIQNHFASREGFGGIMQMKFCPEYVSVFNNVQE
NKGASIFNRRGYFSDPALILMHELIHVLHGLYGIKVDDLPIVPNEKKFFMQSTDAIQAEELYTFGGQDP
SIITPSTDKSIYDKVLQNFRGIVDRLNKVLVCISDPNININIYKNKFKDKYKFVEDSEGKYSIDVESFD
KLYKSLMFGFTETNIAENYKIKTRASYFSDSLPPVKIKNLLDNEIYTIEEGFNISDKDMEKEYRGQNKA
INKQAYEEISKEHLAVYKIQMCVDEEKLYDDDDKDRWGSSLQCIDVDNEDLFFIADKNSFSDDLSKNER
IEYNTQSNYIENDFPINELILDTDLISKIELPSENTESLTDFNVDVPVYEKQPAIKKIFTDENTIFQYL

YSQTFPLDIRDISLTSSFDDALLFSNKVYSFFSMDYIKTANKVVEAGLFAGWVKQIVNDFVIEANKSNT
MDAIADISLIVPYIGLALNVGNETAKGNFENAFEIAGASILLEFIPELLIPVVGAFLLESYIDNKNKII
KTIDNALTKRNEKWSDMYGLIVAQWLSTVNTQFYTIKEGMYKALNYQAQALEEIIKYRYNIYSEKEKSN
INIDFNDINSKLNEGINQAIDNINNFINGCSVSYLMKKMIPLAVEKLLDFDNTLKKNLLNYIDENKLYL
IGSAEYEKSKVNKYLKTIMPFDLSIYTNDTILIEMFNKYNSLEGGGGSGGGGSGGGGSALVGNHWAVGH
LM

42. Protein sequence of the CP-qGHRH29-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDNNNNNNNNNNDDDDKHVDAIFTQSYRKVLAQLSARKLLQDILNRA
EAAAKEAAAKALQCIKVKNNRLPYVADKDSISQEIFENKIITDETNVQNYSDKFSLDESILDGQVPINP
EIVDPLLPNVNMEPLNLPGEEIVFYDDITKYVDYLNSYYYLESQKLSNNVENITLTTSVEEALGYSNKI
YTFLPSLAEKVNKGVQAGLFLNWANEVVEDFTTNIMKKDTLDKISDVSVIIPYIGPALNIGNSALRGNF
NQAFATAGVAFLLEGFPEFTIPALGVFTFYSSIQEREKIIKTIENCLEQRVKRWKDSYQWMVSNWLSRI
TTQFNHINYQMYDSLSYQADAIKAKIDLEYKKYSGSDKENIKSQVENLKNSLDVKISEAMNNINKFIRE
CSVTYLFKNMLPKVIDELNKFDLRTKTELINLIDSHNIILVGEVDRLKAKVNESFENTMPFNIFSYTNN
SLLKDIINEYFN

43. Protein sequence of the CP-qGHRH-LHA fusion EFVNKQFNYKDPVNGVDIAYIKIPNAGQMQPVKAFKIHNK-
IWVIPERDTFTNPEEGDLNPPPEAKQVPV SYYDSTYLSTDNEKDNYLKGVTKLFERIYSTDLGRMLLTSIVRGIP-
FWGGSTIDTELKVIDTNCINVIQ PDGSYRSEELNLVIIGPSADIIQFECKSFGHEVLNLTRNGYGSTQYIRFSPDFTF-
GFEESLEVDTNPLL GAGKFATDPAVTLAHELIHAGHRLYGIAINPNRVFKVNTNAYYEMSGLEVSFEELRTFGGH-
DAKFIDSL QENEFRLYYYNKFKDIASTLNKAKSIVGTTASLQYMKNVFKEKYLLSEDTSGKFSVDKLKFDKLYKMLT
EIYTEDNFVKFFKVLNRKTYLNFDKAVFKINIVPKVNYTIYDGFNLRNTNLAANFNGQNTEINNMNFTK
LKNFTGLFEFYKLLCVDGIITSKTKSLIEGRHVDAIFTQSYRKVLAQLSARKLLQDILNRQQGERNQEQ
GALAGGGGSGGGGSGGGGSALVLQCIKVNNWDLFFSPSEDNFTNDLNKGEEITSDTNIEAAEENISLDL
IQQYYLTFNFDNEPENISIENLSSDIIGQLELMPNIERFPNGKKYELDKYTMFHYLRAQEFEHGKSRIA
LTNSVNEALLNPSRVYTFFSSDYVKKVNKATEAAMFLGWVEQLVYDFTDETSEVSTTDKIADITIIIPY
IGPALNIGNMLYKDDFVGALIFSGAVILLEFIPEIAIPVLGTFALVSYIANKVLTVQTIDNALSKRNEK
WDEVYKYIVTNWLAKVNTQIDLIRKKMKEALENQAEATKAIINYQYNQYTEEEKNNINFNIDDLSSKLN
ESINKAMININKFLNQCSVSYLMNSMIPYGVKRLEDFDASLKDALLKYIYDNRGTLIGQVDRLKDKVNN TLSTDIPFQLSKYVDNQRLLST

44. Protein sequence of the CP-qGHRH-LHC fusion

PITINNFNYSDPVDNKNILYLDTHLNTLANEPEKAFRITGNIWVIPDRFSRNSNPNLNKPPRVTSPKSG
YYDPNYLSTDSDKDTFLKEIIKLFKRINSREIGEELIYRLSTDIPFPGNNNTPINTFDFDVDFNSVDVK
TRQGNNWVKTGSINPSVIITGPRENIIDPETSTFKLTNNTFAAQEGFGALSIISISPRFMLTYSNATND
VGEGRFSKSEFCMDPILILMHELNHAMHNLYGIAIPNDQTISSVTSNIFYSQYNVKLEYAEIYAFGGPT
IDLIPKSARKYFEEKALDYYRSIAKRLNSITTANPSSFNKYIGEYKQKLIRKYRFVVESSGEVTVNRNK
FVELYNELTQIFTEFNYAKIYNVQNRKIYLSNVYTPVTANILDDNVYDIQNGFNIPKSNLNVLFMGQNL
SRNPALRKVNPENMLYLFTKFCVDAIDGRHVDAIFTQSYRKVLAQLSARKLLQDILNRQQGERNQEQGA
LAGGGGSGGGGSGGGGSALVLQCRELLVKNTDLPFIGDISDVKTDIFLRKDINEETEVIYYPDNVSVDQ
VILSKNTSEHGQLDLLYPSIDSESEILPGENQVFYDNRTQNVDYLNSYYYLESQKLSDNVEDFTFTRSI
EEALDNSAKVYTYFPTLANKVNAGVQGGLFLMWANDVVEDFTTNILRKDTLDKISDVSAIIPYIGPALN
ISNSVRRGNFTEAFAVTGVTILLEAFPEFTIPALGAFVIYSKVQERNEIIKTIDNCLEQRIKRWKDSYE
WMMGTWLSRIITQFNNISYQMYDSLNYQAGAIKAKIDLEYKKYSGSDKENIKSQVENLKNSLDVKISEA
MNNINKFIRECSVTYLFKNMLPKVIDELNEFDRNTKAKLINLIDSHNIILVGEVDKLKAKVNNSFQNTI
PFNIFSYTNNSLLKDIINEYFN

45. Protein sequence of the CP-qGHRH-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD

VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKHVDAIFTQSYRKVLAQLSARKLLQDILNRQQ
GERNQEQGAALAGGGGSGGGGSGGGGSALALQCIKVKNNRLPYVADKDSISQEIFENKIITDETNVQNY
SDKFSLDESILDGQVPINPEIVDPLLPNVNMEPLNLPGEEIVFYDDITKYVDYLNSYYYLESQKLSNNV
ENITLTTSVEEALGYSNKIYTFLPSLAEKVNKGVQAGLFLNWANEVVEDFTTNIMKKDTLDKISDVSVI
IPYIGPALNIGNSALRGNFNQAFATAGVAFLLEGFPEFTIPALGVFTFYSSIQEREKIIKTIENCLEQR
VKRWKDSYQWMVSNWLSRITTQFNHINYQMYDSLSYQADAIKAKIDLEYKKYSGSDKENIKSQVENLKN
SLDVKISEAMNNINKFIRECSVTYLFKNMLPKVIDELNKFDLRTKTELINLIDSHNIILVGEVDRLKAK
VNESFENTMPFNIFSYTNNSLLKDIINEYFN

46. Protein sequence of the CP-qGHRH-LHD N10-PL5 fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDNNNNNNNNNNDDDDKHVDAIFTQSYRKVLAQLSARKLLQDILNRQ
QGERNQEQGAPAPAPLQCIKVKNNRLPYVADKDSISQEIFENKIITDETNVQNYSDKFSLDESILDGQV
PINPEIVDPLLPNVNMEPLNLPGEEIVFYDDITKYVDYLNSYYYLESQKLSNNVENITLTTSVEEALGY
SNKIYTFLPSLAEKVNKGVQAGLFLNWANEVVEDFTTNIMKKDTLDKISDVSVIIPYIGPALNIGNSAL
RGNFNQAFATAGVAFLLEGFPEFTIPALGVFTFYSSIQEREKIIKTIENCLEQRVKRWKDSYQWMVSNW
LSRITTQFNHINYQMYDSLSYQADAIKAKIDLEYKKYSGSDKENIKSQVENLKNSLDVKISEAMNNINK
FIRECSVTYLFKNMLPKVIDELNKFDLRTKTELINLIDSHNIILVGEVDRLKAKVNESFENTMPFNIFS
YTNNSLLKDIINEYFN

47. Protein sequence of the CP-qGHRH-LHD N10-HX12 fusion

TWPVKDFNYSDPVNDNDILYLRIPQNKLITTPVKAFMITQNIWVIPERFSSDTNPSLSKPPRPTSKYQSYY
DPSYLSTDEQKDTFLKGIIKLFKRINERDIGKKLINYLVVGSPFMGDSSTPEDTFDFTRHTTNIAVEKFEN
GSWKVTNIITPSVLIFGPLPNILDYTASLTLQGQQSNPSFEGFGTLSILKVAPEFLLTFSDVTSNQSSAVL
GKSIFCMDPVIALMHELTHSLHQLYGINIPSDKRIRPQVSEGFFSQDGPNVQFEELYTFGGLDVEIIPQIE
RSQLREKALGHYKDIAKRLNNINKTIPSSWISNIDKYKKIFSEKYNFDKDNTGNFVVNIDKFNSLYSDLTN
VMSEVVYSSQYNVKNRTHYFSRHYLPVFANILDDNIYTIRDGFNLTNKGFNIENSGQNIERNPALQKLSSE
SVVDLFTKVCVDNNNNNNNNNNDDDDKHVDAIFTQSYRKVLAQLSARKLLQDILNRQQGERNQEQGAEAAA
KEAAAKALQCIKVKNNRLPYVADKDSISQEIFENKIITDETNVQNYSDKFSLDESILDGQVPINPEIVDPL
LPNVNMEPLNLPGEEIVFYDDITKYVDYLNSYYYLESQKLSNNVENITLTTSVEEALGYSNKIYTFLPSLA
EKVNKGVQAGLFLNWANEVVEDFTTNIMKKDTLDKISDVSVIIPYIGPALNIGNSALRGNFNQAFATAGVA
FLLEGFPEFTIPALGVFTFYSSIQEREKIIKTIENCLEQRVKRWKDSYQWMVSNWLSRITTQFNHINYQMY
DSLSYQADAIKAKIDLEYKKYSGSDKENIKSQVENLKNSLDVKISEAMNNINKFIRECSVTYLFKNMLPKV
IDELNKFDLRTKTELINLIDSHNIILVGEVDRLKAKVNESFENTMPFNIFSYTNNSLLKDIINEYFN

48. Protein sequence of the CP-UTS-LHA fusion

EFVNKQFNYKDPVNGVDIAYIKIPNAGQMQPVKAFKIHNKIWVIPERDTFTNPEEGDLNPPPEAKQVPV
SYYDSTYLSTDNEKDNYLKGVTKLFERIYSTDLGRMLLTSIVRGIPFWGGSTIDTELKVIDTNCINVIQ
PDGSYRSEELNLVIIGPSADIIQFECKSFGHEVLNLTRNGYGSTQYIRFSPDFTFGFEESLEVDTNPLL
GAGKFATDPAVTLAHELIHAGHRLYGIAINPNRVFKVNTNAYYEMSGLEVSFEELRTFGGHDAKFIDSL
QENEFRLYYYNKFKDIASTLNKAKSIVGTTASLQYMKNVFKEKYLLSEDTSGKFSVDKLKFDKLYKMLT
EIYTEDNFVKFFKVLNRKTYLNFDKAVFKINIVPKVNYTIYDGFNLRNTNLAANFNGQNTEINNMNFTK
LKNFTGLFEFYKLLCVDGGGGSADDDDKNDDPPISIDLTFHLLRNMIEMARIENEREQAGLNRKYLDEV
ALAGGGGSGGGGSGGGGSALVLQCIKVNNWDLFFSPSEDNFTNDLNKGEEITSDTNIEAAEENISLDLI
QQYYLTFNFDNEPENISIENLSSDIIGQLELMPNIERFPNGKKYELDKYTMFHYLRAQEFEHGKSRIAL
TNSVNEALLNPSRVYTFFSSDYVKKVNKATEAAMFLGWVEQLVYDFTDETSEVSTTDKIADITIIIPYI
GPALNIGNMLYKDDFVGALIFSGAVILLEFIPEIAIPVLGTFALVSYIANKVLTVQTIDNALSKRNEKW
DEVYKYIVTNWLAKVNTQIDLIRKKMKEALENQAEATKAIINYQYNQYTEEEKNNINFNIDDLSSKLNE
SINKAMININKFLNQCSVSYLMNSMIPYGVKRLEDFDASLKDALLKYIYDNRGTLIGQVDRLKDKVNNT
LSTDIPFQLSKYVDNQRLLST

49. Protein sequence of $LH_N/A$

EFVNKQFNYKDPVNGVDIAYIKIPNAGQMQPVKAFKIHNKIWVIPERDTFTNPEEGDLNPPPEAKQVPV
SYYDSTYLSTDNEKDNYLKGVTKLFERIYSTDLGRMLLTSIVRGIPFWGGSTIDTELKVIDTNCINVIQ
PDGSYRSEELNLVIIGPSADIIQFECKSFGHEVLNLTRNGYGSTQYIRFSPDFTFGFEESLEVDTNPLL
GAGKFATDPAVTLAHELIHAGHRLYGIAINPNRVFKVNTNAYYEMSGLEVSFEELRTFGGHDAKFIDSL
QENEFRLYYYNKFKDIASTLNKAKSIVGTTASLQYMKNVFKEKYLLSEDTSGKFSVDKLKFDKLYKMLT
EIYTEDNFVKFFKVLNRKTYLNFDKAVFKINIVPKVNYTIYDGFNLRNTNLAANFNGQNTEINNMNFTK
LKNFTGLFEFYKLLCVDGIITSKTKSDDDDKNKALNLQCIKVNNWDLFFSPSEDNFTNDLNKGEEITSD
TNIEAAEENISLDLIQQYYLTFNFDNEPENISIENLSSDIIGQLELMPNIERFPNGKKYELDKYTMFHY
LRAQEFEHGKSRIALTNSVNEALLNPSRVYTFFSSDYVKKVNKATEAAMFLGWVEQLVYDFTDETSEVS
TTDKIADITIIIPYIGPALNIGNMLYKDDFVGALIFSGAVILLEFIPEIAIPVLGTFALVSYIANKVLT
VQTIDNALSKRNEKWDEVYKYIVTNWLAKVNTQIDLIRKKMKEALENQAEATKAIINYQYNQYTEEEKN
NINFNIDDLSSKLNESINKAMININKFLNQCSVSYLMNSMIPYGVKRLEDFDASLKDALLKYIYDNRGT
LIGQVDRLKDKVNNTLSTDIPFQLSKYVDNQRLLST

50. Protein sequence of $LH_N/B$

PVTINNFNYNDPIDNNNIIMMEPPFARGTGRYYKAFKITDRIWIIPERYTFGYKPEDFNKSSGIFNRDV
CEYYDPDYLNTNDKKNIFLQTMIKLFNRIKSKPLGEKLLEMIINGIPYLGDRRVPLEEFNTNIASVTVN
KLISNPGEVERKKGIFANLIIFGPGPVLNENETIDIGIQNHFASREGFGGIMQMKFCPEYVSVFNNVQE
NKGASIFNRRGYFSDPALILMHELIHVLHGLYGIKVDDLPIVPNEKKFFMQSTDAIQAEELYTFGGQDP
SIITPSTDKSIYDKVLQNFRGIVDRLNKVLVCISDPNININIYKNKFKDKYKFVEDSEGKYSIDVESFD
KLYKSLMFGFTETNIAENYKIKTRASYFSDSLPPVKIKNLLDNEIYTIEEGFNISDKDMEKEYRGQNKA
INKQAYEEISKEHLAVYKIQMCVDEEKLYDDDDKDRWGSSLQCIDVDNEDLFFIADKNSFSDDLSKNER
IEYNTQSNYIENDFPINELILDTDLISKIELPSENTESLTDFNVDVPVYEKQPAIKKIFTDENTIFQYL
YSQTFPLDIRDISLTSSFDDALLFSNKVYSFFSMDYIKTANKVVEAGLFAGWVKQIVNDFVIEANKSNT
MDAIADISLIVPYIGLALNVGNETAKGNFENAFEIAGASILLEFIPELLIPVVGAFLLESYIDNKNKII
KTIDNALTKRNEKWSDMYGLIVAQWLSTVNTQFYTIKEGMYKALNYQAQALEEIIKYRYNIYSEKEKSN
INIDFNDINSKLNEGINQAIDNINNFINGCSVSYLMKKMIPLAVEKLLDFDNTLKKNLLNYIDENKLYL
IGSAEYEKSKVNKYLKTIMPFDLSIYTNDTILIEMFNKYNS

51. Protein sequence of $LH_N$/C

PITINNFNYSDPVDNKNILYLDTHLNTLANEPEKAFRITGNIWVIPDRFSRNSNPNLNKPPRVTSPKSG
YYDPNYLSTDSDKDTFLKEIIKLFKRINSREIGEELIYRLSTDIPFPGNNNTPINTFDFDVDFNSVDVK
TRQGNNWVKTGSINPSVIITGPRENIIDPETSTFKLTNNTFAAQEGFGALSIISISPRFMLTYSNATND
VGEGRFSKSEFCMDPILILMHELNHAMHNLYGIAIPNDQTISSVTSNIFYSQYNVKLEYAEIYAFGGPT
IDLIPKSARKYFEEKALDYYRSIAKRLNSITTANPSSFNKYIGEYKQKLIRKYRFVVESSGEVTVNRNK
FVELYNELTQIFTEFNYAKIYNVQNRKIYLSNVYTPVTANILDDNVYDIQNGFNIPKSNLNVLFMGQNL
SRNPALRKVNPENMLYLFTKFCVDAIDGRSLYNKTLQCRELLVKNTDLPFIGDISDVKTDIFLRKDINE
ETEVIYYPDNVSVDQVILSKNTSEHGQLDLLYPSIDSESEILPGENQVFYDNRTQNVDYLNSYYYLESQ
KLSDNVEDFTFTRSIEEALDNSAKVYTYFPTLANKVNAGVQGGLFLMWANDVVEDFTTNILRKDTLDKI
SDVSAIIPYIGPALNISNSVRRGNFTEAFAVTGVTILLEAFPEFTIPALGAFVIYSKVQERNEIIKTID
NCLEQRIKRWKDSYEWMMGTWLSRIITQFNNISYQMYDSLNYQAGAIKAKIDLEYKKYSGSDKENIKSQ
VENLKNSLDVKISEAMNNINKFIRECSVTYLFKNMLPKVIDELNEFDRNTKAKLINLIDSHNIILVGEV
DKLKAKVNNSFQNTIPFNIFSYTNNSLLKDIINEYFN

52. Protein sequence of $LH_N$/D

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDKSEEKLYDDDDKDRWGSSLQCIKVKNNRLPYVADKDSISQEIFEN
KIITDETNVQNYSDKFSLDESILDGQVPINPEIVDPLLPNVNMEPLNLPGEEIVFYDDITKYVDYLNSY
YYLESQKLSNNVENITLTTSVEEALGYSNKIYTFLPSLAEKVNKGVQAGLFLNWANEVVEDFTTNIMKK
DTLDKISDVSVIIPYIGPALNIGNSALRGNFNQAFATAGVAFLLEGFPEFTIPALGVFTFYSSIQEREK
IIKTIENCLEQRVKRWKDSYQWMVSNWLSRITTQFNHINYQMYDSLSYQADAIKAKIDLEYKKYSGSDK
ENIKSQVENLKNSLDVKISEAMNNINKFIRECSVTYLFKNMLPKVIDELNKFDLRTKTELINLIDSHNI
ILVGEVDRLKAKVNESFENTMPFNIFSYTNNSLLKDIINEYFN

53. Protein sequence of IgA-$H_N$tet

ESNQPEKNGTATKPENSGNTTSENGQTEPEKKLELRNVSDIELYSQTNGTYRQHVSLDGIPENTDTYFV
KVKSSAFKDVYIPVASITEEKRNGQSVYKITAKAEKLQQELENKYVDNFTFYLDKKAKEENTNFTSFSN
LVKAINQNPSGTYHLAASLNANEVELGPDERSYIKDTFTGRLIGEKDGKNYAIYNLKKPLFENLSGATV
EKLSLKNVAISGKNDIGSLANEATNGTKIKQVHVDGCVDGIITSKTKSDDDDKNKALNLQCIKIKNEDL
TFIAEKNSFSEEPFQDEIVSYNTKNKPLNFNYSLDKIIVDYNLQSKITLPNDRTTPVTKGIPYAPEYKS
NAASTIEIHNIDDNTIYQYLYAQKSPTTLQRITMTNSVDDALINSTKIYSYFPSVISKVNQGAQGILFL
QWVRDIIDDFTNESSQKTTIDKISDVSTIVPYIGPALNIVKQGYEGNFIGALETTGVVLLLEYIPEITL
PVIAALSIAESSTQKEKIIKTIDNFLEKRYEKWIEVYKLVKAKWLGTVNTQFQKRSYQMYRSLEYQVDA
IKKIIDYEYKIYSGPDKEQIADEINNLKNKLEEKANKAMININIFMRESSRSFLVNQMINEAKKQLLEF
DTQSKNILMQYIKANSKFIGITELKKLESKINKVFSTPIPFSYSKNLDCWVDNEEDIDV

54. Synthesised Octreotide peptide
Cys-Dphe-Cys-Phe-Dtrp-Lys-Thr-Cys-Thr-ol
55. Synthesised GHRH agonist peptide

HIS-ALA-ASP-ALA-ILE-PHE-THR-ASN-SER-TYR-ARG-LYS-VAL-LEU-GLY-GLN-LEU-SER-ALA-ARG-LYS-LEU-LEU-GLN-ASP-ILE-NLE-SER-ARG-CYS

56. Synthesised GHRH antagonist peptide

PhAc-Tyr-D-Arg-Asp-Ala-Ile-Phe(4-Cl)-Thr-Ala-Har-Tyr(Me)-His-Lys-Val-Leu-Abu-Gln-Leu-Ser-Ala-His-Lys-Leu-Leu-Gln-Asp-Ile-Nle-D-Arg-Har-CYS

57. Protein sequence of CP-MCH-LHD

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKDFDMLRCMLGRVYRPCWQVALAKRLVLQCIK
VKNNRLPYVADKDSISQEIFENKIITDETNVQNYSDKFSLDESILDGQVPINPEIVDPLLPNVNMEPLN
LPGEEIVFYDDITKYVDYLNSYYYLESQKLSNNVENITLTTSVEEALGYSNKIYTFLPSLAEKVNKGVQ
AGLFLNWANEVVEDFTTNIMKKDTLDKISDVSVIIPYIGPALNIGNSALRGNFNQAFATAGVAFLLEGF
PEFTIPALGVFTFYSSIQEREKIIKTIENCLEQRVKRWKDSYQWMVSNWLSRITTQFNHINYQMYDSLS
YQADAIKAKIDLEYKKYSGSDKENIKSQVENLKNSLDVKISEAMNNINKFIRECSVTYLFKNMLPKVID
ELNKFDLRTKTELINLIDSHNIILVGEVDRLKAKVNESFENTMPFNIFSYTNNSLLKDIINEYFN

58. Protein sequence of CT-KISS-LHD

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDKSEEKLYDDDDKDRWGSSLQCIKVKNNRLPYVADKDSISQEIFEN
KIITDETNVQNYSDKFSLDESILDGQVPINPEIVDPLLPNVNMEPLNLPGEEIVFYDDITKYVDYLNSY
YYLESQKLSNNVENITLTTSVEEALGYSNKIYTFLPSLAEKVNKGVQAGLFLNWANEVVEDFTTNIMKK
DTLDKISDVSVIIPYIGPALNIGNSALRGNFNQAFATAGVAFLLEGFPEFTIPALGVFTFYSSIQEREK
IIKTIENCLEQRVKRWKDSYQWMVSNWLSRITTQFNHINYQMYDSLSYQADAIKAKIDLEYKKYSGSDK
ENIKSQVENLKNSLDVKISEAMNNINKFIRECSVTYLFKNMLPKVIDELNKFDLRTKTELINLIDSHNI
ILVGEVDRLKAKVNESFENTMPFNIFSYTNNSLLKDIINEYFNLEGGGGSGGGGSGGGGSALVYNWNSF
GLRFG

59. Protein sequence of CT-PrRP-LHA

EFVNKQFNYKDPVNGVDIAYIKIPNAGQMQPVKAFKIHNKIWVIPERDTFTNPEEGDLNPPPEAKQVPV
SYYDSTYLSTDNEKDNYLKGVTKLFERIYSTDLGRMLLTSIVRGIPFWGGSTIDTELKVIDTNCINVIQ
PDGSYRSEELNLVIIGPSADIIQFECKSFGHEVLNLTRNGYGSTQYIRFSPDFTFGFEESLEVDTNPLL
GAGKFATDPAVTLAHELIHAGHRLYGIAINPNRVFKVNTNAYYEMSGLEVSFEELRTFGGHDAKFIDSL

QENEFRLYYYNKFKDIASTLNKAKSIVGTTASLQYMKNVFKEKYLLSEDTSGKFSVDKLKFDKLYKMLT
EIYTEDNFVKFFKVLNRKTYLNFDKAVFKINIVPKVNYTIYDGFNLRNTNLAANFNGQNTEINNMNFTK
LKNFTGLFEFYKLLCVDGIITSKTKSDDDDKNKALNLQCIKVNNWDLFFSPSEDNFTNDLNKGEEITSD
TNIEAAEENISLDLIQQYYLTFNFDNEPENISIENLSSDIIGQLELMPNIERFPNGKKYELDKYTMFHY
LRAQEFEHGKSRIALTNSVNEALLNPSRVYTFFSSDYVKKVNKATEAAMFLGWVEQLVYDFTDETSEVS
TTDKIADITIIIPYIGPALNIGNMLYKDDFVGALIFSGAVILLEFIPEIAIPVLGTFALVSYIANKVLT
VQTIDNALSKRNEKWDEVYKYIVTNWLAKVNTQIDLIRKKMKEALENQAEATKAIINYQYNQYTEEEKN
NINFNIDDLSSKLNESINKAMININKFLNQCSVSYLMNSMIPYGVKRLEDFDASLKDALLKYIYDNRGT
LIGQVDRLKDKVNNTLSTDIPFQLSKYVDNQRLLSTLEGGGGSGGGGSGGGGSALVTPDINPAWYASRG
IRPVGRFG

60. Protein sequence of CP-HS_GHRH_1-27-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFTNSYRKVLGQLSARKLLQDIMALAG
GGGSGGGGSGGGGSALALQCIKVKNNRLPYVADKDSISQEIFENKIITDETNVQNYSDKFSLDESILDG
QVPINPEIVDPLLPNVNMEPLNLPGEEIVFYDDITKYVDYLNSYYYLESQKLSNNVENITLTTSVEEAL
GYSNKIYTFLPSLAEKVNKGVQAGLFLNWANEVVEDFTTNIMKKDTLDKISDVSVIIPYIGPALNIGNS
ALRGNFNQAFATAGVAFLLEGFPEFTIPALGVFTFYSSIQEREKIIKTIENCLEQRVKRWKDSYQWMVS
NWLSRITTQFNHINYQMYDSLSYQADAIKAKIDLEYKKYSGSDKENIKSQVENLKNSLDVKISEAMNNI
NKFIRECSVTYLFKNMLPKVIDELNKFDLRTKTELINLIDSHNIILVGEVDRLKAKVNESFENTMPFNI
FSYTNNSLLKDIINEYFN

61. Protein sequence of the CP-HS_GHRH_1-28-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFTNSYRKVLGQLSARKLLQDIMSALA
GGGGSGGGGSGGGGSALALQCIKVKNNRLPYVADKDSISQEIFENKIITDETNVQNYSDKFSLDESILD
GQVPINPEIVDPLLPNVNMEPLNLPGEEIVFYDDITKYVDYLNSYYYLESQKLSNNVENITLTTSVEEA
LGYSNKIYTFLPSLAEKVNKGVQAGLFLNWANEVVEDFTTNIMKKDTLDKISDVSVIIPYIGPALNIGN
SALRGNFNQAFATAGVAFLLEGFPEFTIPALGVFTFYSSIQEREKIIKTIENCLEQRVKRWKDSYQWMV
SNWLSRITTQFNHINYQMYDSLSYQADAIKAKIDLEYKKYSGSDKENIKSQVENLKNSLDVKISEAMNN
INKFIRECSVTYLFKNMLPKVIDELNKFDLRTKTELINLIDSHNIILVGEVDRLKAKVNESFENTMPFN
IFSYTNNSLLKDIINEYFN

62. Protein sequence of the CP-HS_GHRH_1-29-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFTNSYRKVLGQLSARKLLQDIMSRAL
AGGGGSGGGGSGGGGSALALQCIKVKNNRLPYVADKDSISQEIFENKIITDETNVQNYSDKFSLDESIL
DGQVPINPEIVDPLLPNVNMEPLNLPGEEIVFYDDITKYVDYLNSYYYLESQKLSNNVENITLTTSVEE
ALGYSNKIYTFLPSLAEKVNKGVQAGLFLNWANEVVEDFTTNIMKKDTLDKISDVSVIIPYIGPALNIG
NSALRGNFNQAFATAGVAFLLEGFPEFTIPALGVFTFYSSIQEREKIIKTIENCLEQRVKRWKDSYQWM
VSNWLSRITTQFNHINYQMYDSLSYQADAIKAKIDLEYKKYSGSDKENIKSQVENLKNSLDVKISEAMN
NINKFIRECSVTYLFKNMLPKVIDELNKFDLRTKTELINLIDSHNIILVGEVDRLKAKVNESFENTMPF
NIFSYTNNSLLKDIINEYFN

63. Protein sequence of the CP-HS_GHRH_1-44-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFTNSYRKVLGQLSARKLLQDIMSRQQ
GESNQERGARARLALAGGGGSGGGGSGGGGSALALQCIKVKNNRLPYVADKDSISQEIFENKIITDETN
VQNYSDKFSLDESILDGQVPINPEIVDPLLPNVNMEPLNLPGEEIVFYDDITKYVDYLNSYYYLESQKL
SNNVENITLTTSVEEALGYSNKIYTFLPSLAEKVNKGVQAGLFLNWANEVVEDFTTNIMKKDTLDKISD
VSVIIPYIGPALNIGNSALRGNFNQAFATAGVAFLLEGFPEFTIPALGVFTFYSSIQEREKIIKTIENC
LEQRVKRWKDSYQWMVSNWLSRITTQFNHINYQMYDSLSYQADAIKAKIDLEYKKYSGSDKENIKSQVE
NLKNSLDVKISEAMNNINKFIRECSVTYLFKNMLPKVIDELNKFDLRTKTELINLIDSHNIILVGEVDR
LKAKVNESFENTMPFNIFSYTNNSLLKDIINEYFN

64. Protein sequence of the CP-HS_GHRH_1-40-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFTNSYRKVLGQLSARKLLQDIMSRQQ
GESNQERGALAGGGGSGGGGSGGGGSALALQCIKVKNNRLPYVADKDSISQEIFENKIITDETNVQNYS

DKFSLDESILDGQVPINPEIVDPLLPNVNMEPLNLPGEEIVFYDDITKYVDYLNSYYYLESQKLSNNVE
NITLTTSVEEALGYSNKIYTFLPSLAEKVNKGVQAGLFLNWANEVVEDFTTNIMKKDTLDKISDVSVII
PYIGPALNIGNSALRGNFNQAFATAGVAFLLEGFPEFTIPALGVFTFYSSIQEREKIIKTIENCLEQRV
KRWKDSYQWMVSNWLSRITTQFNHINYQMYDSLSYQADAIKAKIDLEYKKYSGSDKENIKSQVENLKNS
LDVKISEAMNNINKFIRECSVTYLFKNMLPKVIDELNKFDLRTKTELINLIDSHNIILVGEVDRLKAKV
NESFENTMPFNIFSYTNNSLLKDIINEYFN

65. Protein sequence of the CP-HS_GHRH_Ala9-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFTNAYRKVLGQLSARKLLQDIMSRAL
AGGGGSGGGGSGGGGSALALQCIKVKNNRLPYVADKDSISQEIFENKIITDETNVQNYSDKFSLDESIL
DGQVPINPEIVDPLLPNVNMEPLNLPGEEIVFYDDITKYVDYLNSYYYLESQKLSNNVENITLTTSVEE
ALGYSNKIYTFLPSLAEKVNKGVQAGLFLNWANEVVEDFTTNIMKKDTLDKISDVSVIIPYIGPALNIG
NSALRGNFNQAFATAGVAFLLEGFPEFTIPALGVFTFYSSIQEREKIIKTIENCLEQRVKRWKDSYQWM
VSNWLSRITTQFNHINYQMYDSLSYQADAIKAKIDLEYKKYSGSDKENIKSQVENLKNSLDVKISEAMN
NINKFIRECSVTYLFKNMLPKVIDELNKFDLRTKTELINLIDSHNIILVGEVDRLKAKVNESFENTMPF
NIFSYTNNSLLKDIINEYFN

66. Protein sequence of the CP-HS_GHRH_Ala22-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFTNSYRKVLGQLSARKALQDIMSRAL
AGGGGSGGGGSGGGGSALALQCIKVKNNRLPYVADKDSISQEIFENKIITDETNVQNYSDKFSLDESIL
DGQVPINPEIVDPLLPNVNMEPLNLPGEEIVFYDDITKYVDYLNSYYYLESQKLSNNVENITLTTSVEE
ALGYSNKIYTFLPSLAEKVNKGVQAGLFLNWANEVVEDFTTNIMKKDTLDKISDVSVIIPYIGPALNIG
NSALRGNFNQAFATAGVAFLLEGFPEFTIPALGVFTFYSSIQEREKIIKTIENCLEQRVKRWKDSYQWM
VSNWLSRITTQFNHINYQMYDSLSYQADAIKAKIDLEYKKYSGSDKENIKSQVENLKNSLDVKISEAMN
NINKFIRECSVTYLFKNMLPKVIDELNKFDLRTKTELINLIDSHNIILVGEVDRLKAKVNESFENTMPF
NIFSYTNNSLLKDIINEYFN

67. Protein sequence of the CP-HS_GHRH_Ala8_Lys11_1-29-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFTASYKKVLGQLSARKLLQDIMSRAL
AGGGGSGGGGSGGGGSALALQCIKVKNNRLPYVADKDSISQEIFENKIITDETNVQNYSDKFSLDESIL
DGQVPINPEIVDPLLPNVNMEPLNLPGEEIVFYDDITKYVDYLNSYYYLESQKLSNNVENITLTTSVEE
ALGYSNKIYTFLPSLAEKVNKGVQAGLFLNWANEVVEDFTTNIMKKDTLDKISDVSVIIPYIGPALNIG
NSALRGNFNQAFATAGVAFLLEGFPEFTIPALGVFTFYSSIQEREKIIKTIENCLEQRVKRWKDSYQWM
VSNWLSRITTQFNHINYQMYDSLSYQADAIKAKIDLEYKKYSGSDKENIKSQVENLKNSLDVKISEAMN
NINKFIRECSVTYLFKNMLPKVIDELNKFDLRTKTELINLIDSHNIILVGEVDRLKAKVNESFENTMPF
NIFSYTNNSLLKDIINEYFN

68. Protein sequence of the CP-HS_GHRH_Ala8_Lys11_Arg12_1-29-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFTASYKRVLGQLSARKLLQDIMSRAL
AGGGGSGGGGSGGGGSALALQCIKVKNNRLPYVADKDSISQEIFENKIITDETNVQNYSDKFSLDESIL
DGQVPINPEIVDPLLPNVNMEPLNLPGEEIVFYDDITKYVDYLNSYYYLESQKLSNNVENITLTTSVEE
ALGYSNKIYTFLPSLAEKVNKGVQAGLFLNWANEVVEDFTTNIMKKDTLDKISDVSVIIPYIGPALNIG
NSALRGNFNQAFATAGVAFLLEGFPEFTIPALGVFTFYSSIQEREKIIKTIENCLEQRVKRWKDSYQWM
VSNWLSRITTQFNHINYQMYDSLSYQADAIKAKIDLEYKKYSGSDKENIKSQVENLKNSLDVKISEAMN
NINKFIRECSVTYLFKNMLPKVIDELNKFDLRTKTELINLIDSHNIILVGEVDRLKAKVNESFENTMPF
NIFSYTNNSLLKDIINEYFN

69. Protein sequence of the CP-HS_GHRH_Ala8_Asn11_1-29-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI

ERNPALQKLSSESVVDLFTKVCVDGIITSKTKSDDDDKYADAIFTASYNKVLGQLSARKLLQDIMSRAL AGGGGSGGGGSGGGGSALALQCIKVKNNRLPYVADKDSISQEIFENKIITDETNVQNYSDKFSLDESIL DGQVPINPEIVDPLLPNVNMEPLNLPGEEIVFYDDITKYVDYLNSYYYLESQKLSNNVENITLTTSVEE ALGYSNKIYTFLPSLAEKVNKGVQAGLFLNWANEVVEDFTTNIMKKDTLDKISDVSVIIPYIGPALNIG NSALRGNFNQAFATAGVAFLLEGFPEFTIPALGVFTFYSSIQEREKIIKTIENCLEQRVKRWKDSYQWM VSNWLSRITTQFNHINYQMYDSLSYQADAIKAKIDLEYKKYSGSDKENIKSQVENLKNSLDVKISEAMN NINKFIRECSVTYLFKNMLPKVIDELNKFDLRTKTELINLIDSHNIILVGEVDRLKAKVNESFENTMPF NIFSYTNNSLLKDIINEYFN

70. Protein sequence of the CP-HS_GHRH_Ala8_Lys20_1-29-LHD fusion

TWPVKDFNYSDPVNDNDILYLRIPQNKLITTPVKAFMITQNIWVIPERFSSDTNPSLSKPPRPTSKYQS YYDPSYLSTDEQKDTFLKGIIKLFKRINERDIGKKLINYLVVGSPFMGDSSTPEDTFDFTRHTTNIAVE KFENGSWKVTNIITPSVLIFGPLPNILDYTASLTLQGQQSNPSFEGFGTLSILKVAPEFLLTFSDVTSN QSSAVLGKSIFCMDPVIALMHELTHSLHQLYGINIPSDKRIRPQVSEGFFSQDGPNVQFEELYTFGGLD VEIIPQIERSQLREKALGHYKDIAKRLNNINKTIPSSWISNIDKYKKIFSEKYNFDKDNTGNFVVNIDK FNSLYSDLTNVMSEVVYSSQYNVKNRTHYFSRHYLPVFANILDDNIYTIRDGFNLTNKGFNIENSGQNI ERNPALQKLSSESVVDLFTKVCVDGIITSKTKSDDDDKYADAIFTASYRKVLGQLSAKKLLQDIMSRAL AGGGGSGGGGSGGGGSALALQCIKVKNNRLPYVADKDSISQEIFENKIITDETNVQNYSDKFSLDESIL DGQVPINPEIVDPLLPNVNMEPLNLPGEEIVFYDDITKYVDYLNSYYYLESQKLSNNVENITLTTSVEE ALGYSNKIYTFLPSLAEKVNKGVQAGLFLNWANEVVEDFTTNIMKKDTLDKISDVSVIIPYIGPALNIG NSALRGNFNQAFATAGVAFLLEGFPEFTIPALGVFTFYSSIQEREKIIKTIENCLEQRVKRWKDSYQWM VSNWLSRITTQFNHINYQMYDSLSYQADAIKAKIDLEYKKYSGSDKENIKSQVENLKNSLDVKISEAMN NINKFIRECSVTYLFKNMLPKVIDELNKFDLRTKTELINLIDSHNIILVGEVDRLKAKVNESFENTMPF NIFSYTNNSLLKDIINEYFN

71. Protein sequence of the CP-HS_GHRH_Ala8_Lys11_Lys20_1-29-LHD fusion

TWPVKDFNYSDPVNDNDILYLRIPQNKLITTPVKAFMITQNIWVIPERFSSDTNPSLSKPPRPTSKYQS YYDPSYLSTDEQKDTFLKGIIKLFKRINERDIGKKLINYLVVGSPFMGDSSTPEDTFDFTRHTTNIAVE KFENGSWKVTNIITPSVLIFGPLPNILDYTASLTLQGQQSNPSFEGFGTLSILKVAPEFLLTFSDVTSN QSSAVLGKSIFCMDPVIALMHELTHSLHQLYGINIPSDKRIRPQVSEGFFSQDGPNVQFEELYTFGGLD VEIIPQIERSQLREKALGHYKDIAKRLNNINKTIPSSWISNIDKYKKIFSEKYNFDKDNTGNFVVNIDK FNSLYSDLTNVMSEVVYSSQYNVKNRTHYFSRHYLPVFANILDDNIYTIRDGFNLTNKGFNIENSGQNI ERNPALQKLSSESVVDLFTKVCVDGIITSKTKSDDDDKYADAIFTASYKKVLGQLSAKKLLQDIMSRAL AGGGGSGGGGSGGGGSALALQCIKVKNNRLPYVADKDSISQEIFENKIITDETNVQNYSDKFSLDESIL DGQVPINPEIVDPLLPNVNMEPLNLPGEEIVFYDDITKYVDYLNSYYYLESQKLSNNVENITLTTSVEE ALGYSNKIYTFLPSLAEKVNKGVQAGLFLNWANEVVEDFTTNIMKKDTLDKISDVSVIIPYIGPALNIG NSALRGNFNQAFATAGVAFLLEGFPEFTIPALGVFTFYSSIQEREKIIKTIENCLEQRVKRWKDSYQWM VSNWLSRITTQFNHINYQMYDSLSYQADAIKAKIDLEYKKYSGSDKENIKSQVENLKNSLDVKISEAMN NINKFIRECSVTYLFKNMLPKVIDELNKFDLRTKTELINLIDSHNIILVGEVDRLKAKVNESFENTMPF

NIFSYTNNSLLKDIINEYFN

72. Protein sequence of the CP-HS_GHRH_Ala8_Asn20_1-29-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFTASYRKVLGQLSANKLLQDIMSRAL
AGGGGSGGGGSGGGGSALALQCIKVKNNRLPYVADKDSISQEIFENKIITDETNVQNYSDKFSLDESIL
DGQVPINPEIVDPLLPNVNMEPLNLPGEEIVFYDDITKYVDYLNSYYYLESQKLSNNVENITLTTSVEE
ALGYSNKIYTFLPSLAEKVNKGVQAGLFLNWANEVVEDFTTNIMKKDTLDKISDVSVIIPYIGPALNIG
NSALRGNFNQAFATAGVAFLLEGFPEFTIPALGVFTFYSSIQEREKIIKTIENCLEQRVKRWKDSYQWM
VSNWLSRITTQFNHINYQMYDSLSYQADAIKAKIDLEYKKYSGSDKENIKSQVENLKNSLDVKISEAMN
NINKFIRECSVTYLFKNMLPKVIDELNKFDLRTKTELINLIDSHNIILVGEVDRLKAKVNESFENTMPF
NIFSYTNNSLLKDIINEYFN

73. Protein sequence of the CP-HS_GHRH_Ala8_Asn12_1-29-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFTASYRNVLGQLSARKLLQDIMSRAL
AGGGGSGGGGSGGGGSALALQCIKVKNNRLPYVADKDSISQEIFENKIITDETNVQNYSDKFSLDESIL
DGQVPINPEIVDPLLPNVNMEPLNLPGEEIVFYDDITKYVDYLNSYYYLESQKLSNNVENITLTTSVEE
ALGYSNKIYTFLPSLAEKVNKGVQAGLFLNWANEVVEDFTTNIMKKDTLDKISDVSVIIPYIGPALNIG
NSALRGNFNQAFATAGVAFLLEGFPEFTIPALGVFTFYSSIQEREKIIKTIENCLEQRVKRWKDSYQWM
VSNWLSRITTQFNHINYQMYDSLSYQADAIKAKIDLEYKKYSGSDKENIKSQVENLKNSLDVKISEAMN
NINKFIRECSVTYLFKNMLPKVIDELNKFDLRTKTELINLIDSHNIILVGEVDRLKAKVNESFENTMPF
NIFSYTNNSLLKDIINEYFN

74. Protein sequence of the CP-HS_GHRH_Ala8_Asn21_1-29-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE

KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFTASYRKVLGQLSARNLLQDIMSRAL
AGGGGSGGGGSGGGGSALALQCIKVKNNRLPYVADKDSISQEIFENKIITDETNVQNYSDKFSLDESIL
DGQVPINPEIVDPLLPNVNMEPLNLPGEEIVFYDDITKYVDYLNSYYYLESQKLSNNVENITLTTSVEE
ALGYSNKIYTFLPSLAEKVNKGVQAGLFLNWANEVVEDFTTNIMKKDTLDKISDVSVIIPYIGPALNIG
NSALRGNFNQAFATAGVAFLLEGFPEFTIPALGVFTFYSSIQEREKIIKTIENCLEQRVKRWKDSYQWM
VSNWLSRITTQFNHINYQMYDSLSYQADAIKAKIDLEYKKYSGSDKENIKSQVENLKNSLDVKISEAMN
NINKFIRECSVTYLFKNMLPKVIDELNKFDLRTKTELINLIDSHNIILVGEVDRLKAKVNESFENTMPF
NIFSYTNNSLLKDIINEYFN

75. Protein sequence of the CP-HS_GHRH_Ala8_Glu_7_1-29-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFEASYRKVLGQLSARKLLQDIMSRAL
AGGGGSGGGGSGGGGSALALQCIKVKNNRLPYVADKDSISQEIFENKIITDETNVQNYSDKFSLDESIL
DGQVPINPEIVDPLLPNVNMEPLNLPGEEIVFYDDITKYVDYLNSYYYLESQKLSNNVENITLTTSVEE
ALGYSNKIYTFLPSLAEKVNKGVQAGLFLNWANEVVEDFTTNIMKKDTLDKISDVSVIIPYIGPALNIG
NSALRGNFNQAFATAGVAFLLEGFPEFTIPALGVFTFYSSIQEREKIIKTIENCLEQRVKRWKDSYQWM
VSNWLSRITTQFNHINYQMYDSLSYQADAIKAKIDLEYKKYSGSDKENIKSQVENLKNSLDVKISEAMN
NINKFIRECSVTYLFKNMLPKVIDELNKFDLRTKTELINLIDSHNIILVGEVDRLKAKVNESFENTMPF
NIFSYTNNSLLKDIINEYFN

76. Protein sequence of the CP-HS_GHRH_Ala8_Glu_10_1-29LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFTASERKVLGQLSARKLLQDIMSRAL
AGGGGSGGGGSGGGGSALALQCIKVKNNRLPYVADKDSISQEIFENKIITDETNVQNYSDKFSLDESIL
DGQVPINPEIVDPLLPNVNMEPLNLPGEEIVFYDDITKYVDYLNSYYYLESQKLSNNVENITLTTSVEE

ALGYSNKIYTFLPSLAEKVNKGVQAGLFLNWANEVVEDFTTNIMKKDTLDKISDVSVIIPYIGPALNIG
NSALRGNFNQAFATAGVAFLLEGFPEFTIPALGVFTFYSSIQEREKIIKTIENCLEQRVKRWKDSYQWM
VSNWLSRITTQFNHINYQMYDSLSYQADAIKAKIDLEYKKYSGSDKENIKSQVENLKNSLDVKISEAMN
NINKFIRECSVTYLFKNMLPKVIDELNKFDLRTKTELINLIDSHNIILVGEVDRLKAKVNESFENTMPF
NIFSYTNNSLLKDIINEYFN

77. Protein sequence of the CP-HS_GHRH_Ala8_Glu_13_1-29-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFTASYRKELGQLSARKLLQDIMSRAL
AGGGGSGGGGSGGGGSALALQCIKVKNNRLPYVADKDSISQEIFENKIITDETNVQNYSDKFSLDESIL
DGQVPINPEIVDPLLPNVNMEPLNLPGEEIVFYDDITKYVDYLNSYYYLESQKLSNNVENITLTTSVEE
ALGYSNKIYTFLPSLAEKVNKGVQAGLFLNWANEVVEDFTTNIMKKDTLDKISDVSVIIPYIGPALNIG
NSALRGNFNQAFATAGVAFLLEGFPEFTIPALGVFTFYSSIQEREKIIKTIENCLEQRVKRWKDSYQWM
VSNWLSRITTQFNHINYQMYDSLSYQADAIKAKIDLEYKKYSGSDKENIKSQVENLKNSLDVKISEAMN
NINKFIRECSVTYLFKNMLPKVIDELNKFDLRTKTELINLIDSHNIILVGEVDRLKAKVNESFENTMPF
NIFSYTNNSLLKDIINEYFN

78. Protein sequence of the CP-HS_GHRH_Ala8-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFTASYRKVLGQLSARKLLQDIMSRAL
AGGGGSGGGGSGGGGSALALQCIKVKNNRLPYVADKDSISQEIFENKIITDETNVQNYSDKFSLDESIL
DGQVPINPEIVDPLLPNVNMEPLNLPGEEIVFYDDITKYVDYLNSYYYLESQKLSNNVENITLTTSVEE
ALGYSNKIYTFLPSLAEKVNKGVQAGLFLNWANEVVEDFTTNIMKKDTLDKISDVSVIIPYIGPALNIG
NSALRGNFNQAFATAGVAFLLEGFPEFTIPALGVFTFYSSIQEREKIIKTIENCLEQRVKRWKDSYQWM
VSNWLSRITTQFNHINYQMYDSLSYQADAIKAKIDLEYKKYSGSDKENIKSQVENLKNSLDVKISEAMN
NINKFIRECSVTYLFKNMLPKVIDELNKFDLRTKTELINLIDSHNIILVGEVDRLKAKVNESFENTMPF
NIFSYTNNSLLKDIINEYFN

79. Protein sequence of the CP-HS_GHRH_Glu8_1-29-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFTESYRKVLGQLSARKLLQDIMSRAL
AGGGGSGGGGSGGGGSALALQCIKVKNNRLPYVADKDSISQEIFENKIITDETNVQNYSDKFSLDESIL
DGQVPINPEIVDPLLPNVNMEPLNLPGEEIVFYDDITKYVDYLNSYYYLESQKLSNNVENITLTTSVEE
ALGYSNKIYTFLPSLAEKVNKGVQAGLFLNWANEVVEDFTTNIMKKDTLDKISDVSVIIPYIGPALNIG
NSALRGNFNQAFATAGVAFLLEGFPEFTIPALGVFTFYSSIQEREKIIKTIENCLEQRVKRWKDSYQWM
VSNWLSRITTQFNHINYQMYDSLSYQADAIKAKIDLEYKKYSGSDKENIKSQVENLKNSLDVKISEAMN
NINKFIRECSVTYLFKNMLPKVIDELNKFDLRTKTELINLIDSHNIILVGEVDRLKAKVNESFENTMPF
NIFSYTNNSLLKDIINEYFN

80. Protein sequence of the CP-HS_GHRH_Ala15_1-27-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFTNSYRKVLAQLSARKLLQDIMALAG
GGGSGGGGSGGGGSALALQCIKVKNNRLPYVADKDSISQEIFENKIITDETNVQNYSDKFSLDESILDG
QVPINPEIVDPLLPNVNMEPLNLPGEEIVFYDDITKYVDYLNSYYYLESQKLSNNVENITLTTSVEEAL
GYSNKIYTFLPSLAEKVNKGVQAGLFLNWANEVVEDFTTNIMKKDTLDKISDVSVIIPYIGPALNIGNS
ALRGNFNQAFATAGVAFLLEGFPEFTIPALGVFTFYSSIQEREKIIKTIENCLEQRVKRWKDSYQWMVS
NWLSRITTQFNHINYQMYDSLSYQADAIKAKIDLEYKKYSGSDKENIKSQVENLKNSLDVKISEAMNNI
NKFIRECSVTYLFKNMLPKVIDELNKFDLRTKTELINLIDSHNIILVGEVDRLKAKVNESFENTMPFNI
FSYTNNSLLKDIINEYFN

81. Protein sequence of the CP-HS_GHRH_Ala15-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFTNSYRKVLAQLSARKLLQDIMSRAL
AGGGGSGGGGSGGGGSALALQCIKVKNNRLPYVADKDSISQEIFENKIITDETNVQNYSDKFSLDESIL
DGQVPINPEIVDPLLPNVNMEPLNLPGEEIVFYDDITKYVDYLNSYYYLESQKLSNNVENITLTTSVEE

ALGYSNKIYTFLPSLAEKVNKGVQAGLFLNWANEVVEDFTTNIMKKDTLDKISDVSVIIPYIGPALNIG
NSALRGNFNQAFATAGVAFLLEGFPEFTIPALGVFTFYSSIQEREKIIKTIENCLEQRVKRWKDSYQWM
VSNWLSRITTQFNHINYQMYDSLSYQADAIKAKIDLEYKKYSGSDKENIKSQVENLKNSLDVKISEAMN
NINKFIRECSVTYLFKNMLPKVIDELNKFDLRTKTELINLIDSHNIILVGEVDRLKAKVNESFENTMPF
NIFSYTNNSLLKDIINEYFN

82. Protein sequence of the CP- HS_GHRH_Ala8_Ala15_1-29-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFTASYRKVLAQLSARKLLQDIMSRAL
AGGGGSGGGGSGGGGSALALQCIKVKNNRLPYVADKDSISQEIFENKIITDETNVQNYSDKFSLDESIL
DGQVPINPEIVDPLLPNVNMEPLNLPGEEIVFYDDITKYVDYLNSYYYLESQKLSNNVENITLTTSVEE
ALGYSNKIYTFLPSLAEKVNKGVQAGLFLNWANEVVEDFTTNIMKKDTLDKISDVSVIIPYIGPALNIG
NSALRGNFNQAFATAGVAFLLEGFPEFTIPALGVFTFYSSIQEREKIIKTIENCLEQRVKRWKDSYQWM
VSNWLSRITTQFNHINYQMYDSLSYQADAIKAKIDLEYKKYSGSDKENIKSQVENLKNSLDVKISEAMN
NINKFIRECSVTYLFKNMLPKVIDELNKFDLRTKTELINLIDSHNIILVGEVDRLKAKVNESFENTMPF
NIFSYTNNSLLKDIINEYFN

83. Protein sequence of the CP-HS_GHRH_Ala8_9_15_22_27-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFTAAYRKVLAQLSARKALQDIASRAL
AGGGGSGGGGSGGGGSALALQCIKVKNNRLPYVADKDSISQEIFENKIITDETNVQNYSDKFSLDESIL
DGQVPINPEIVDPLLPNVNMEPLNLPGEEIVFYDDITKYVDYLNSYYYLESQKLSNNVENITLTTSVEE
ALGYSNKIYTFLPSLAEKVNKGVQAGLFLNWANEVVEDFTTNIMKKDTLDKISDVSVIIPYIGPALNIG
NSALRGNFNQAFATAGVAFLLEGFPEFTIPALGVFTFYSSIQEREKIIKTIENCLEQRVKRWKDSYQWM
VSNWLSRITTQFNHINYQMYDSLSYQADAIKAKIDLEYKKYSGSDKENIKSQVENLKNSLDVKISEAMN
NINKFIRECSVTYLFKNMLPKVIDELNKFDLRTKTELINLIDSHNIILVGEVDRLKAKVNESFENTMPF
NIFSYTNNSLLKDIINEYFN

84. Protein sequence of the CP-HS_GHRH_Ala8_9_15_22-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFTAAYRKVLAQLSARKALQDIMSRAL
AGGGGSGGGGSGGGGSALALQCIKVKNNRLPYVADKDSISQEIFENKIITDETNVQNYSDKFSLDESIL
DGQVPINPEIVDPLLPNVNMEPLNLPGEEIVFYDDITKYVDYLNSYYYLESQKLSNNVENITLTTSVEE
ALGYSNKIYTFLPSLAEKVNKGVQAGLFLNWANEVVEDFTTNIMKKDTLDKISDVSVIIPYIGPALNIG
NSALRGNFNQAFATAGVAFLLEGFPEFTIPALGVFTFYSSIQEREKIIKTIENCLEQRVKRWKDSYQWM
VSNWLSRITTQFNHINYQMYDSLSYQADAIKAKIDLEYKKYSGSDKENIKSQVENLKNSLDVKISEAMN
NINKFIRECSVTYLFKNMLPKVIDELNKFDLRTKTELINLIDSHNIILVGEVDRLKAKVNESFENTMPF
NIFSYTNNSLLKDIINEYFN

85. Protein sequence of the CP-HS_GHRH_HVQAL_1-32-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKHVDAIFTQSYRKVLAQLSARKALQDILSRQQ
GALAGGGGSGGGGSGGGGSALALQCIKVKNNRLPYVADKDSISQEIFENKIITDETNVQNYSDKFSLDE
SILDGQVPINPEIVDPLLPNVNMEPLNLPGEEIVFYDDITKYVDYLNSYYYLESQKLSNNVENITLTTS
VEEALGYSNKIYTFLPSLAEKVNKGVQAGLFLNWANEVVEDFTTNIMKKDTLDKISDVSVIIPYIGPAL
NIGNSALRGNFNQAFATAGVAFLLEGFPEFTIPALGVFTFYSSIQEREKIIKTIENCLEQRVKRWKDSY
QWMVSNWLSRITTQFNHINYQMYDSLSYQADAIKAKIDLEYKKYSGSDKENIKSQVENLKNSLDVKISE
AMNNINKFIRECSVTYLFKNMLPKVIDELNKFDLRTKTELINLIDSHNIILVGEVDRLKAKVNESFENT
MPFNIFSYTNNSLLKDIINEYFN

86. Protein sequence of the CP-HS_GHRH_HVSAL_1-29-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKHVDAIFTSSYRKVLAQLSARKLLQDILSRAL

AGGGGSGGGGSGGGGSALALQCIKVKNNRLPYVADKDSISQEIFENKIITDETNVQNYSDKFSLDESIL
DGQVPINPEIVDPLLPNVNMEPLNLPGEEIVFYDDITKYVDYLNSYYYLESQKLSNNVENITLTTSVEE
ALGYSNKIYTFLPSLAEKVNKGVQAGLFLNWANEVVEDFTTNIMKKDTLDKISDVSVIIPYIGPALNIG
NSALRGNFNQAFATAGVAFLLEGFPEFTIPALGVFTFYSSIQEREKIIKTIENCLEQRVKRWKDSYQWM
VSNWLSRITTQFNHINYQMYDSLSYQADAIKAKIDLEYKKYSGSDKENIKSQVENLKNSLDVKISEAMN
NINKFIRECSVTYLFKNMLPKVIDELNKFDLRTKTELINLIDSHNIILVGEVDRLKAKVNESFENTMPF
NIFSYTNNSLLKDIINEYFN

87. Protein sequence of the CP-HS_GHRH_HVTAL_1-29-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKHVDAIFTTSYRKVLAQLSARKLLQDILSRAL
AGGGGSGGGGSGGGGSALALQCIKVKNNRLPYVADKDSISQEIFENKIITDETNVQNYSDKFSLDESIL
DGQVPINPEIVDPLLPNVNMEPLNLPGEEIVFYDDITKYVDYLNSYYYLESQKLSNNVENITLTTSVEE
ALGYSNKIYTFLPSLAEKVNKGVQAGLFLNWANEVVEDFTTNIMKKDTLDKISDVSVIIPYIGPALNIG
NSALRGNFNQAFATAGVAFLLEGFPEFTIPALGVFTFYSSIQEREKIIKTIENCLEQRVKRWKDSYQWM
VSNWLSRITTQFNHINYQMYDSLSYQADAIKAKIDLEYKKYSGSDKENIKSQVENLKNSLDVKISEAMN
NINKFIRECSVTYLFKNMLPKVIDELNKFDLRTKTELINLIDSHNIILVGEVDRLKAKVNESFENTMPF
NIFSYTNNSLLKDIINEYFN

88. Protein sequence of the CP-HS_GHRH_QALN-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFTQSYRKVLAQLSARKALQDILNRAL
AGGGGSGGGGSGGGGSALALQCIKVKNNRLPYVADKDSISQEIFENKIITDETNVQNYSDKFSLDESIL
DGQVPINPEIVDPLLPNVNMEPLNLPGEEIVFYDDITKYVDYLNSYYYLESQKLSNNVENITLTTSVEE
ALGYSNKIYTFLPSLAEKVNKGVQAGLFLNWANEVVEDFTTNIMKKDTLDKISDVSVIIPYIGPALNIG
NSALRGNFNQAFATAGVAFLLEGFPEFTIPALGVFTFYSSIQEREKIIKTIENCLEQRVKRWKDSYQWM
VSNWLSRITTQFNHINYQMYDSLSYQADAIKAKIDLEYKKYSGSDKENIKSQVENLKNSLDVKISEAMN
NINKFIRECSVTYLFKNMLPKVIDELNKFDLRTKTELINLIDSHNIILVGEVDRLKAKVNESFENTMPF
NIFSYTNNSLLKDIINEYFN

89. Protein sequence of the CP-HS_GHRH_QAL-LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSDDDDKYADAIFTQSYRKVLAQLSARKALQDILSRAL
AGGGGSGGGGSGGGGSALALQCIKVKNNRLPYVADKDSISQEIFENKIITDETNVQNYSDKFSLDESIL
DGQVPINPEIVDPLLPNVNMEPLNLPGEEIVFYDDITKYVDYLNSYYYLESQKLSNNVENITLTTSVEE
ALGYSNKIYTFLPSLAEKVNKGVQAGLFLNWANEVVEDFTTNIMKKDTLDKISDVSVIIPYIGPALNIG
NSALRGNFNQAFATAGVAFLLEGFPEFTIPALGVFTFYSSIQEREKIIKTIENCLEQRVKRWKDSYQWM
VSNWLSRITTQFNHINYQMYDSLSYQADAIKAKIDLEYKKYSGSDKENIKSQVENLKNSLDVKISEAMN
NINKFIRECSVTYLFKNMLPKVIDELNKFDLRTKTELINLIDSHNIILVGEVDRLKAKVNESFENTMPF
NIFSYTNNSLLKDIINEYFN

90. Protein sequence of the CP-hGHRH29 N8A M27L -LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSIEGRYADAIFTASYRKVLGQLSARKLLQDILSR
ALAGGGGSGGGGSGGGGSALALQCIKVKNNRLPYVADKDSISQEIFENKIITDETNVQNYSDKFSLDES
ILDGQVPINPEIVDPLLPNVNMEPLNLPGEEIVFYDDITKYVDYLNSYYYLESQKLSNNVENITLTTSV
EEALGYSNKIYTFLPSLAEKVNKGVQAGLFLNWANEVVEDFTTNIMKKDTLDKISDVSVIIPYIGPALN
IGNSALRGNFNQAFATAGVAFLLEGFPEFTIPALGVFTFYSSIQEREKIIKTIENCLEQRVKRWKDSYQ
WMVSNWLSRITTQFNHINYQMYDSLSYQADAIKAKIDLEYKKYSGSDKENIKSQVENLKNSLDVKISEA
MNNINKFIRECSVTYLFKNMLPKVIDELNKFDLRTKTELINLIDSHNIILVGEVDRLKAKVNESFENTM
PFNIFSYTNNSLLKDIINEYFN

91. Protein sequence of the CP-hGHRH29 N8A K12N M27L -LHD fusion

TWPVKDFNYSDPVNDNDILYLRIPQNKLITTPVKAFMITQNIWVIPERFSSDTNPSLSKPPRPTSKYQS
YYDPSYLSTDEQKDTFLKGIIKLFKRINERDIGKKLINYLVVGSPFMGDSSTPEDTFDFTRHTTNIAVE
KFENGSWKVTNIITPSVLIFGPLPNILDYTASLTLQGQQSNPSFEGFGTLSILKVAPEFLLTFSDVTSN
QSSAVLGKSIFCMDPVIALMHELTHSLHQLYGINIPSDKRIRPQVSEGFFSQDGPNVQFEELYTFGGLD
VEIIPQIERSQLREKALGHYKDIAKRLNNINKTIPSSWISNIDKYKKIFSEKYNFDKDNTGNFVVNIDK
FNSLYSDLTNVMSEVVYSSQYNVKNRTHYFSRHYLPVFANILDDNIYTIRDGFNLTNKGFNIENSGQNI
ERNPALQKLSSESVVDLFTKVCVDGIITSKTKSIEGR YADAIFTASYRNVLGQLSARKLLQDILSR

ALAGGGGSGGGGSGGGGSALALQCIKVKNNRLPYVADKDSISQEIFENKIITDETNVQNYSDKFSLDESILDGQVPINPEIVDPLLPNVNMEPLNLPGEEIVFYDDITKYVDYLNSYYYLESQKLSNNVENITLTTSVEEALGYSNKIYTFLPSLAEKVNKGVQAGLFLNWANEVVEDFTTNIMKKDTLDKISDVSVIIPYIGPALNIGNSALRGNFNQAFATAGVAFLLEGFPEFTIPALGVFTFYSSIQEREKIIKTIENCLEQRVKRWKDSYQWMVSNWLSRITTQFNHINYQMYDSLSYQADAIKAKIDLEYKKYSGSDKENIKSQVENLKNSLDVKISEAMNNINKFIRECSVTYLFKNMLPKVIDELNKFDLRTKTELINLIDSHNIILVGEVDRLKAKVNESFENTMPFNIFSYTNNSLLKDIINEYFN

92. Protein sequence of the N-termianal-hGHRH29 N8A M27L -LHD fusion

HVDAIFTQSYRKVLAQLSARKLLQDILNRNNNNNNNNNNTWPVKDFNYSDPVNDNDILYLRIPQNKLITTPVKAFMITQNIWVIPERFSSDTNPSLSKPPRPTSKYQSYYDPSYLSTDEQKDTFLKGIIKLFKRINERDIGKKLINYLVVGSPFMGDSSTPEDTFDFTRHTTNIAVEKFENGSWKVTNIITPSVLIFGPLPNILDYTASLTLQGQQSNPSFEGFGTLSILKVAPEFLLTFSDVTSNQSSAVLGKSIFCMDPVIALMHELTHSLHQLYGINIPSDKRIRPQVSEGFFSQDGPNVQFEELYTFGGLDVEIIPQIERSQLREKALGHYKDIAKRLNNINKTIPSSWISNIDKYKKIFSEKYNFDKDNTGNFVVNIDKFNSLYSDLTNVMSEVVYSSQYNVKNRTHYFSRHYLPVFANILDDNIYTIRDGFNLTNKGFNIENSGQNIERNPALQKLSSESVVDLFTKVCVDKSEEKLYDDDDKDRWGSSLQCIKVKNNRLPYVADKDSISQEIFENKIITDETNVQNYSDKFSLDESILDGQVPINPEIVDPLLPNVNMEPLNLPGEEIVFYDDITKYVDYLNSYYYLESQKLSNNVENITLTTSVEEALGYSNKIYTFLPSLAEKVNKGVQAGLFLNWANEVVEDFTTNIMKKDTLDKISDVSVIIPYIGPALNIGNSALRGNFNQAFATAGVAFLLEGFPEFTIPALGVFTFYSSIQEREKIIKTIENCLEQRVKRWKDSYQWMVSNWLSRITTQFNHINYQMYDSLSYQADAIKAKIDLEYKKYSGSDKENIKSQVENLKNSLDVKISEAMNNINKFIRECSVTYLFKNMLPKVIDELNKFDLRTKTELINLIDSHNIILVGEVDRLKAKVNESFENTMPFNIFSYTNNSLLKDIINEYFN

SEQ ID93 GnRH-C fusion protein

PITINNFNYSDPVDNKNILYLDTHLNTLANEPEKAFRITGNIWVIPDRFSRNSNPNLNKPPRVT
SPKSGYYDPNYLSTDSDKDTFLKEIIKLFKRINSREIGEELIYRLSTDIPFPGNNNTPINTFDFDV
DFNSVDVKTRQGNNWVKTGSINPSVIITGPRENIIDPETSTFKLTNNTFAAQEGFGALSIISISP
RFMLTYSNATNDVGEGRFSKSEFCMDPILILMHELNHAMHNLYGIAIPNDQTISSVTSNIFYSQ
YNVKLEYAEIYAFGGPTIDLIPKSARKYFEEKALDYYRSIAKRLNSITTANPSSFNKYIGEYKQK
LIRKYRFVVESSGEVTVNRNKFVELYNELTQIFTEFNYAKIYNVQNRKIYLSNVYTPVTANILDD
NVYDIQNGFNIPKSNLNVLFMGQNLSRNPALRKVNPENMLYLFTKFCVDAIDGRSLYNKTLQ
CRELLVKNTDLPFIGDISDVKTDIFLRKDINEETEVIYYPDNVSVDQVILSKNTSEHGQLDLLYP
SIDSESEILPGENQVFYDNRTQNVDYLNSYYYLESQKLSDNVEDFTFTRSIEEALDNSAKVYT
YFPTLANKVNAGVQGGLFLMWANDVVEDFTTNILRKDTLDKISDVSAIIPYIGPALNISNSVRR
GNFTEAFAVTGVTILLEAFPEFTIPALGAFVIYSKVQERNEIIKTIDNCLEQRIKRWKDSYEWM
MGTWLSRIITQFNNISYQMYDSLNYQAGAIKAKIDLEYKKYSGSDKENIKSQVENLKNSLDVKI
SEAMNNINKFIRECSVTYLFKNMLPKVIDELNEFDRNTKAKLINLIDSHNIILVGEVDKLKAKVN
NSFQNTIPFNIFSYTNNSLLKDIINEYFNLEGGGGSGGGGSGGGGSALVMKPIQKLLAGLILLT
WCVEGCSSQHWSYGLRPGGKRDAENLIDSFQEIVKEVGQLAETQRFECTTHQPRSPLRDLK
GALESLIEEETGQKKI

SEQ ID94 GnRH-D fusion

TWPVKDFNYSDPVNDNDILYLRIPQNKLITTPVKAFMITQNIWVIPERFSSDTNPSLSKPPRPT
SKYQSYYDPSYLSTDEQKDTFLKGIIKLFKRINERDIGKKLINYLVVGSPFMGDSSTPEDTFDF
TRHTTNIAVEKFENGSWKVTNIITPSVLIFGPLPNILDYTASLTLQGQQSNPSFEGFGTLSILKV
APEFLLTFSDVTSNQSSAVLGKSIFCMDPVIALMHELTHSLHQLYGINIPSDKRIRPQVSEGFF
SQDGPNVQFEELYTFGGLDVEIIPQIERSQLREKALGHYKDIAKRLNNINKTIPSSWISNIDKYK
KIFSEKYNFDKDNTGNFVVNIDKFNSLYSDLTNVMSEVVYSSQYNVKNRTHYFSRHYLPVFA
NILDDNIYTIRDGFNLTNKGFNIENSGQNIERNPALQKLSSESVVDLFTKVCVDKSEEKLYDDD
DKDRWGSSLQCIKVKNNRLPYVADKDSISQEIFENKIITDETNVQNYSDKFSLDESILDGQVPI
NPEIVDPLLPNVNMEPLNLPGEEIVFYDDITKYVDYLNSYYYLESQKLSNNVENITLTTSVEEA
LGYSNKIYTFLPSLAEKVNKGVQAGLFLNWANEVVEDFTTNIMKKDTLDKISDVSVIIPYIGPAL
NIGNSALRGNFNQAFATAGVAFLLEGFPEFTIPALGVFTFYSSIQEREKIIKTIENCLEQRVKR
WKDSYQWMVSNWLSRITTQFNHINYQMYDSLSYQADAIKAKIDLEYKKYSGSDKENIKSQVE
NLKNSLDVKISEAMNNINKFIRECSVTYLFKNMLPKVIDELNKFDLRTKTELINLIDSHNIILVGE
VDRLKAKVNESFENTMPFNIFSYTNNSLLKDIINEYFNLEGGGGSGGGGSGGGGSGGGGSA
LVMKPIQKLLAGLILLTWCVEGCSSQHWSYGLRPGGKRDAENLIDSFQEIVKEVGQLAETQR
FECTTHQPRSPLRDLKGALESLIEEETGQKKI

## Example 1 Preparation of a $LH_N$/A backbone construct

The following procedure creates a clone for use as an expression backbone for multidomain protein expression. This example is based on preparation of a serotype A based clone (SEQ ID1), though the procedures and methods are equally applicable to all $LH_N$ serotypes such as serotype B (SEQ ID2), serotype C (SEQ ID3) and serotype D (SEQ ID4) and other protease or translocation domains such as IgA and Tetanus $H_N$ by using the appropriate published sequence for synthesis (SEQ ID32).

*Preparation of cloning and expression vectors*

pCR 4 (Invitrogen) is the chosen standard cloning vector chosen due to the lack of restriction sequences within the vector and adjacent sequencing primer sites for easy construct confirmation. The expression vector is based on the pET (Novagen) expression vector which has been modified to contain the multiple cloning site *Nde*I-*Bam*HI-*Sal*I-*Pst*I-*Xba*I-*Hind*III for construct insertion, a fragment of the expression vector has been removed to create a non-mobilisable plasmid, a variety of different fusion tags have been inserted to increase purification options and an existing *XbaI* site in the vector backbone has been removed to simplify sub-cloning.

*Preparation of LC/A*

The DNA sequence is designed by back translation of the LC/A amino acid sequence (obtained from freely available database sources such as GenBank (accession number P10845) using one of a variety of reverse translation software tools (for example Backtranslation tool v2.0 (Entelechon)). *Bam*HI/*Sal*I recognition sequences are incorporated at the 5' and 3' ends respectively of the sequence maintaining the correct reading frame. The DNA sequence is screened (using software such as SeqBuilder, DNASTAR Inc.) for restriction enzyme cleavage sequences incorporated during the back translation. Any cleavage sequences that are found to be common to those required by the cloning system are removed by the Backtranslation tool from the proposed coding sequence ensuring common *E. coli* codon usage is maintained. *E. coli* codon usage is assessed by reference to software programs such as Graphical Codon Usage Analyser (Geneart), and the %GC content and codon usage ratio assessed by reference to published codon usage tables (for example GenBank Release 143, September 13 2004). This optimised DNA sequence containing the LC/A open reading frame (ORF) is then commercially synthesized (for example by Entelechon, Geneart or Sigma-Genosys) and is provided in the pCR 4 vector.

*Preparation of $H_N$/A insert*

The DNA sequence is designed by back translation of the $H_N$/A amino acid sequence (obtained from freely available database sources such as GenBank (accession number P10845) using one of a variety of reverse translation software tools (for example Back translation tool v2.0 (Entelechon)). A *Pst*I restriction sequence added to the N-terminus and *Xba*I-stop codon-*Hind*III to the C-terminus ensuring the correct reading frame in maintained. The DNA sequence is screened (using software such as SeqBuilder, DNASTAR Inc.) for restriction enzyme cleavage sequences incorporated during the back translation. Any sequences that are found to be common to those required by the cloning system are removed by the Backtranslation tool from the proposed coding sequence ensuring common *E. coli* codon usage is maintained. *E. coli* codon usage is assessed by reference to software programs such as Graphical Codon Usage Analyser (Geneart), and the %GC content and codon usage ratio assessed by reference to published codon usage tables (for example GenBank Release 143, September 13 2004). This optimised DNA sequence is then commercially synthesized (for example by Entelechon, Geneart or Sigma-Genosys) and is provided in the pCR 4 vector.

*Preparation of the interdomain (LC-$H_N$ linker*)

The LC-$H_N$ linker can be designed from first principle, using the existing sequence information for the linker as the template. For example, the serotype A linker (in this case defined as the inter-domain polypeptide region that exists between the cysteines of the disulphide bridge between LC and $H_N$) has the sequence VRGIIPFKTKSLDEGYNKALNDL. This sequence information is freely available from available database sources such as GenBank (accession number P10845). For generation of a specific protease cleavage site, the native recognition sequence for Factor Xa can be used in the modified sequence VDGIITSKTKSLIEGR or an enterokinase recognition sequence is inserted into the activation loop to generate the sequence VDGIITSKTKSDDDDKNKALNLQ. Using one of a variety of reverse translation software tools (for example Backtranslation tool v2.0 (Entelechon), the DNA sequence encoding the linker region is determined. *Bam*HI/*Sal*I and *Pst*I/*Xba*I/stop codon/*Hind*III restriction enzyme sequences are incorporated at either end, in the correct reading frames. The DNA sequence is screened (using software such as Seqbuilder, DNASTAR Inc.) for restriction enzyme cleavage sequences incorporated during the back translation. Any sequences that are found to be common to those required by the cloning system are removed by the Backtranslation tool from the proposed coding sequence ensuring common *E. coli* codon usage is maintained. *E. coli* codon usage is assessed by reference to software programs such as Graphical Codon Usage Analyser (Geneart), and the %GC content and codon usage ratio assessed by reference to published codon usage tables (for example GenBank Release 143, September 13 2004). This optimised DNA sequence is then commercially synthesized (for example by Entelechon, Geneart or Sigma-Genosys) and is provided in the pCR 4 vector.

*Assembly and confirmation of the backbone clone*

Due to the small size, the activation linker must be transferred using a two step process. The pCR-4 linker vector is cleaved with *Bam*HI + *Sal*I combination restriction enzymes and the cleaved linker vector then serves as the recipient for *Bam*HI + *Sal*I restriction enzyme cleaved LC DNA. Once the LC encoding DNA is inserted upstream of the linker DNA, the entire LC-linker DNA fragment can then be isolated and transferred to the pET expression vector MCS. The LC-linker is cut out from the pCR 4 cloning vector using *Bam*HI/*Pst*I restriction enzymes digests. The pET expression vector is digested with the same enzymes but is also treated with antarctic phosphatase as an extra precaution to prevent re-circularisation. The LC-linker and the pET vector backbone are gel purified and the purified insert and vector backbone are ligated together using T4 DNA ligase. The product is transformed with TOP10 cells which are then screened for LC-linker using *Bam*HI/*Pst*I restriction digestion. The process is then repeated for the $H_N$ insertion into the *Pst*I/*Hin*dIII restriction sites of the pET-LC-linker construct. Screening with restriction enzymes is sufficient to ensure the final backbone is correct as all components are already sequenced confirmed during synthesis. However, during the sub-cloning of some components into the backbone, where similar size fragments are being removed and inserted, sequencing of a small region to confirm correct insertion is required.

**Example 2 Construction of $LH_N$/AA-CP-GS15-SST28**

The following procedure creates a clone for use as an expression construct for multidomain fusion expression where the targeting moiety (TM) is presented centrally between the protease and translocation domain. This example is based on preparation of the $LH_N$/A-CP-GS15-SST28 fusion (SEQ ID25), though the procedures and methods are equally applicable to create other protease, translocation and TM fusions, where the TM is N-terminal to the translocation domain. In this example, a flanking 15 amino acid glycine-serine spacer $(G_4S)3$ is engineered into the interdomain sequence ensure accessibility of the ligand to its receptor, but other spacers are applicable.

*Preparation of spacer-human SST28 insert*

The LC-$H_N$ inter-domain polypeptide linker region exists between the cysteines of the disulphide bridge between LC and $H_N$. For insertion of a protease cleavage site, spacer and a targeting moiety (TM) region into the activation loop, one of a variety of reverse translation software tools (for example Backtranslation tool v2.0 (Entelechon) are used to determine the DNA sequence encoding the linker region. For central presentation of an SST28 sequence at the N-terminus of the $H_N$ domain, a DNA sequence is designed for the GS spacer and targeting moiety (TM) regions allowing incorporation into the backbone clone (SEQ ID1). The DNA sequence can be arranged as *Bam*HI-*Sal*I-spacer-protease activation site-*SST28-spacer-Pst*I-XbaI-stop codon-*Hin*dIII (SEQ ID5). Once the TM DNA is designed, the additional DNA required to encode the preferred spacer is created *in silico*. It is important to ensure the correct reading frame is maintained for the spacer, SST28 and restriction sequences and that the *XbaI* sequence is not preceded by the bases TC, which would result in DAM methylation. The DNA sequence is screened for restriction sequence incorporated and any additional sites are removed manually from the remaining sequence ensuring common *E. coli* codon usage is maintained. *E. coli* codon usage is assessed by reference to software programs such as Graphical Codon Usage Analyser (Geneart), and the %GC content and codon usage ratio assessed by reference to published codon usage tables (for example GenBank Release 143, September 13 2004). This optimised DNA sequence is then commercially synthesized (for example by Entelechon, Geneart or Sigma-Genosys) and is provided in the pCR 4 vector.

*Assembly and confirmation of the backbone clone*

In order to create a LC-spacer-activation site-SST28-spacer-$H_N$ construct (SEQ ID25) using the backbone construct (SEQ ID1) and the newly synthesised pCR 4-spacer-activation site-TM-spacer vector encoding the SST28 TM (SEQ ID5), a one or two step method can be used; typically the two step method is used when the TM DNA is less than 100 base pairs. Using the one step method the SST28 linker region can be inserted directly into the backbone construct buy cutting the pCR 4- spacer-activation site-TM-spacer vector with *Sal*I and *Pst*I restriction enzymes and inserting the TM encoding DNA fragment into a similarly cut pET backbone construct. Using the two-step method the LC domain is excised from the backbone clone using restriction enzymes *Bam*HI and *Sal*I and ligated into similarly digested pCR 4-spacer-activation site-TM-spacer vector. This creates a LC-spacer-activation site-SST28-spacer ORF in pCR 4 that can be excised from the vector using restriction enzymes *Bam*HI and *Pst*I for subsequent ligation into similarly pET expression construct. The final construct contains the LC-spacer-activation site-SST28-spacer-$H_N$ DNA (SEQ ID25) which will result in a fusion protein containing the sequence illustrated in SEQ ID26.

**Example 3 Expression and purification of a $LH_N$/A-CP-SST28 fusion protein**

This example is based on preparation of an $LH_N$/A protein that incorporates a SST28 TM polypeptide into the interdomain linker region (SEQ ID26), where the pET expression vector ORF also encodes a histidine purification tag. These procedures and methods are equally applicable to the other fusion protein such as those shown in SEQ ID7-14, 42-48, 57, 60-91. Where appropriate, the activation enzyme should be selected to be compatible with the protease activation site within each sequence

*Expression of $LH_N$/A-CP-SST28*

Expression of the $LH_N$/A-CP-SST28 protein is achieved using the following protocol. Inoculate 100 ml of modified TB containing 0.2 % glucosamine and 30 μg/ml kanamycin in a 250 ml flask with a single colony from the LHA-CP-SST28 expression strain. Grow the culture at 37°C, 225 rpm for 16 hours. Inoculate 1 L of modified TB containing 0.2 % glucosamine and 30 μg/ml kanamycin in a 2 L flask with 10 ml of overnight culture. Grow cultures at 37°C until an approximate $OD_{600}$ nm of 0.5 is reached at which point reduce the temperature to 16°C. After 1 hour induce the cultures with 1 mM IPTG and grow at 16°C for a further 16 hours.

*Purification of $LH_N$/A-CP-SST28 protein*

Defrost falcon tube containing 35 ml 50 mM HEPES pH 7.2 200 mM NaCl and approximately 10 g of E. coli BL21 (DE3) cell paste. Homogenise the cell paste (20 psi) ensuring the sample remains cool. Spin the lysed cells at 18 000 rpm, 4°C for 30 minutes. Load the supernatant onto a 0.1 M NiSO4 charged Chelating column (20-30 ml column is sufficient) equilibrated with 50 mM HEPES pH 7.2 200 mM NaCl. Using a step gradient of 10, 40 and 100 mM imidazole, wash away the non-specific bound protein and elute the fusion protein with 200 mM imidazole. The eluted fusion protein is dialysed against 5 L of 50 mM HEPES pH 7.2 200 mM NaCl at 4°C overnight and the $OD_{280}$ nm measured to establish the protein concentration. Add 3.2 μl enterokinase (New England Biolabs) per mg fusion protein and incubate static overnight at 25°C. Load onto a 0.1 M NiSO4 charged Chelating column (20-30 ml column is sufficient) equilibrated with 50 mM HEPES pH 7.2 200 mM NaCl. Wash column to baseline with 50 mM HEPES pH 7.2 200 mM NaCl. Using a step gradient of 10, 40 and 100 mM imidazole, wash away the non-specific bound protein and elute the fusion protein with 200 mM imidazole. Dialyse the eluted fusion protein against 5L of 50 mM HEPES pH 7.2 150 mM NaCl at 4°C overnight and concentrate the fusion to about 2 mg/ml, aliquot sample and freeze at -20°C. Test purified protein using $OD_{280}$, BCA and purity analysis.

**Example 4 Construction of $LH_N$/D-CT-GS20-CST28**

The following procedure creates a clone for use as an expression construct for multidomain fusion expression where the targeting moiety (TM) is presented C-terminally to the translocation domain. This example is based on preparation of the $LH_N$/D-CT-GS20-CST28 fusion (SEQ ID17), though the procedures and methods are equally applicable to create other protease, translocation and TM fusions, where the TM of C-terminal to the translocation domain. In this example, a flanking 20 amino acid glycine-serine spacer is engineered into the interdomain sequence ensure accessibility of the ligand to its receptor, but other spacers are applicable.

*Preparation of spacer-human CST28 insert*

For presentation of a CST28 sequence at the C-terminus of the $H_N$ domain, a DNA sequence is designed to flank the spacer and targeting moiety (TM) regions allowing incorporation into the backbone clone (SEQ ID4). The DNA sequence can be arranged as *Bam*HI-*Sal*I-*Pst*I-*Xba*I-spacer-*CST28*-stop codon-*Hin*dIII (SEQ ID6). The DNA sequence can be designed using one of a variety of reverse translation software tools (for example EditSeq best *E. coli* reverse translation (DNASTAR Inc.), or Backtranslation tool v2.0 (Entelechon)). Once the TM DNA is designed, the additional DNA required to encode the preferred spacer is created *in silico.* It is important to ensure the correct reading frame is maintained for the spacer, CST28 and restriction sequences and that the *Xba*I sequence is not preceded by the bases TC, which would result on DAM methylation. The DNA sequence is screened for restriction sequences incorporated and any additional sequences are removed manually from the remaining sequence ensuring common *E. coli* codon usage is maintained. *E. coli* codon usage is assessed by reference to software programs such as Graphical Codon Usage Analyser (Geneart), and the %GC content and codon usage ratio assessed by reference to published codon usage tables (for example GenBank Release 143, September 13 2004). This optimised DNA sequence is then commercially synthesized (for example by Entelechon, Geneart or Sigma-Genosys) and is provided in the pCR 4 vector.

*Assembly and confirmation of the backbone clone*

In order to create a $LH_N$/D-GS20-CST28 construct (SEQ ID17) using the backbone construct (SEQ ID4) and the newly synthesised pCR 4-spacer-TM vector encoding the CST28 TM (SEQ ID6), a one or two step method can be used; typically the two step method is used when the TM DNA is less than 100 base pairs. Using the one step method the CST28 can be inserted directly into the backbone construct buy cutting the pCR 4-spacer-TM vector with *Xba*I and *Hind*III restriction enzymes and inserting the TM encoding DNA fragment into a similarly cut pET backbone construct. Using the two-step method the $LH_N$ domain is excised from the backbone clone using restriction enzymes *Bam*HI and *XbaI* and ligated into similarly digested pCR 4-spacer-CST28 vector. This creates an $LH_N$-spacer-CST28 ORF in pCR 4 that can be excised from the vector using restriction enzymes *Bam*HI and *Hind*III for subsequent ligation into the similarly cleaved pET expression construct. The final construct contains the LC-linker-$H_N$-spacer-CST28 DNA (SEQ ID17) which will result in a fusion protein containing the sequence illustrated in SEQ ID18.

**Example 5 Expression and purification of a $LH_N$/D-CT-CST28 fusion protein**

This example is based on preparation of an $LH_N$/D protein that incorporates a CST28 TM polypeptide at the carboxyl terminus of the $H_N$ domain (SEQ ID 18), where the pET expression vector ORF also encodes a histidine purification tag. These procedures and methods are equally applicable to fusion protein sequences such as those shown in SEQ ID15, 16, 18-24, 27-31, 33-41, 58-59, and 93-94. Where appropriate, the activation enzyme should be selected to be compatible with the protease activation site within each sequence.

*Expression of $LH_N$/D-CT-CST28*

Expression of the $LH_N$/D-CT-CST28 protein is achieved using the following protocol. Inoculate 100 ml of modified TB containing 0.2 % glucosamine and 30 μg/ml kanamycin in a 250 ml flask with a single colony from the $LH_N$/D-CT-CST28 expression strain. Grow the culture at 37°C, 225 rpm for 16 hours. Inoculate 1 L of modified TB containing 0.2 % glucosamine and 30 μg/ml kanamycin in a 2 L flask with 10 ml of overnight culture. Grow cultures at 37°C until an approximate $OD_{600}$ nm of 0.5 is reached at which point reduce the temperature to 16°C. After 1 hour induce the cultures with 1 mM IPTG and grow at 16°C for a further 16 hours.

*Purification of $LH_N$/D-CT-CST28 protein*

Defrost falcon tube containing 35 ml 50 mM HEPES pH 7.2 200 mM NaCl and approximately 10 g of *E. coli* BL21 (DE3) cell paste. Homogenise the cell paste (20psi) ensuring the sample remains cool. Spin the lysed cells at 18 000 rpm, 4°C for 30 minutes. Load the supernatant onto a 0.1 M $NiSO_4$ charged Chelating column (20-30 ml column is sufficient) equilibrated with 50 mM HEPES pH 7.2 200 mM NaCl. Using a step gradient of 10, 40 and 100 mM imidazole, wash away the non-specific bound protein and elute the fusion protein with 200 mM imidazole. The eluted fusion protein is dialysed against 5 L of 50 mM HEPES pH 7.2 200 mM NaCl at 4°C overnight and the $OD_{280}$ nm measured to establish the protein concentration. Add 3.2 μl enterokinase (New England Biolabs) per mg fusion protein and incubate static overnight at 25°C. Load onto a 0.1 M $NiSO_4$ charged Chelating column (20-30 ml column is sufficient) equilibrated with 50 mM HEPES pH 7.2 200 mM NaCl. Wash column to baseline with 50 mM HEPES pH 7.2 200 mM NaCl. Using a step gradient of 10, 40 and 100 mM imidazole, wash away the non-specific bound protein and elute the fusion protein with 200 mM imidazole. Dialyse the eluted fusion protein against 5 L of 50 mM HEPES pH 7.2 150 mM NaCl at 4°C overnight and concentrate the fusion to about 2 mg/ml, aliquot sample and freeze at -20°C. Test purified protein using $OD_{280}$, BCA and purity analysis. Figures 1 and 2 demonstrate purification of fusion proteins as analysed by SDS-PAGE.

**Example 6 Chemical conjugation of $LH_N$/A to SST TM**

The following procedure creates a chemically conjugated molecule containing the $LH_N$/A amino acid sequence (SEQ ID49), prepared from SEQ ID1 using the production method outlined in example 3, and a SST Octreotide peptide which has been chemically synthesised (SEQ ID54). However, the procedures and methods are equally applicable for the conjugation of other peptides such as SEQ ID55 and SEQ ID56 to other protease/translocation domain proteins such as those containing the amino acid sequences SEQ ID50, 51, 52 and 53.

The $LH_N$/A protein was buffer exchanged from 50 mM Hepes 150 mM salt into PBSE (100mM 14.2g NA2HPO4, 100mM 5.85g NaCl, 1mM $EDTANa_2$ pH 7.5 with 1M HCl) using the Bio-rad PD10 column. This was done by washing one column volume of PBSE through the PD10 column, the protein was then added to the column until no more drops exit the end of the PD10 column. 8 mls of PBSE was then added and 0.5ml fractions are collected. The collected fractions are the measured using the $A_{280}$ reading and fractions containing protein are pooled. A concentration of 1.55 mg/ml of $LH_N$/A was obtained from the buffer exchange step and this was used to set up the following reactions:

| $LH_N$/A 1.55 mg/ml | 20 mM SPDP or Sulfo-LC-SPDP |
|---|---|
| A 200 μl | 0 |
| B 200 μl | 4 fold increase 0.62 μl |
| C 200 μl | 8 fold increase 1.24 μl |

Sample were left to tumble at RT for 3 hours before being passed down another PD10 column to buffer exchange into PBSE and the protein containing fractions pooled. A final concentration of 25 mM DTT was then added to derivatised protein and then the samples left at room temperature for 10 minutes. $A_{280}$ and $A_{343}$ readings were then taken to work out the ratio of SPDP:$LH_N$/A interaction and the reaction which resulted in a derivatisation ration of between 1 and 3 was used for the peptide conjugation. The SPDP reagent binds to the primary amines of the $LH_N$/A via an N-hydroxysuccinimide (NHS) ester, leaving the sulphydryl-reactive portion to form a disulphide bond to the free SH group on the free cysteine on the synthesised peptide. In this case the peptide sequence is Octreotide which has been synthesised with a free cysteine on the N-terminus (SEQ ID91). The SPDP-derivatised $LH_N$/A was mixed with a 4-fold excess of the Octreotide ligand and the reaction was then left at RT for 90 minutes whilst tumbling. The excess octreotide was then removed using either a PD10 column leaving $LH_N$/A-Octreotide conjugated molecule.

**Example 7 Activity of SST-$LH_N$/A in cultured endocrine cells (AtT20)**

The rat pituitary tumour cell line AtT20 is an example of a cell line of endocrine origin. It thus represents a model cell line for the investigation of inhibition-of-release effects of the agents.

AtT20 cells possess surface receptors that allow for the binding, and internalisation, of SST-$LH_N$/A. In contrast, AtT20 cells lack suitable receptors for clostridial neurotoxins and are therefore not susceptible to botulinum neurotoxins (BoNTs).

Figure 3 (a) illustrates the inhibition of release of ACTH from AtT20 cells after prior incubation with SST-$LH_N$/A. It is clear that dose-dependent inhibition is observed, indicating that SST-$LH_N$/A can inhibit the release of ACTH from an endocrine cell model. Inhibition of ACTH release was demonstrated to correlate with cleavage of the SNARE protein SNAP25 (Fig 3 (a) and (b)) Thus, inhibition of release of chemical messenger is due to a clostridial endopeptidase-mediated effect of SNARE-protein cleavage.

*Materials and Methods*

ACTH enzyme immunoassay kits were obtained from Bachem Research Inc., CA, USA. Western blotting reagents were obtained from Invitrogen and Sigma. AtT20 cells were seeded onto 12 well plates and cultured in DMEM containing 10% foetal bovine serum, 4 mM Glutamax. After 1 day SST-$LH_N$/A was applied for 72 hours then the cells washed to remove unbound SST-$LH_N$/A. Secretion of ACTH was stimulated by elevating the concentration of extracellular potassium (60 mM KCl) and calcium (5 mM $CaCl_2$) for 30 min. The medium was harvested from the cells and stored at -20°C until assayed for ACTH content using the immunoassay kit and following the manufacturer's instructions. Cells were solubilised in 1x LDS electrophoresis reducing sample buffer, heated for 10 minutes at 90°C then stored at -20°c until used for Western blotting. Stimulated secretion was calculated by subtracting basal release from total release under stimulating conditions. Solubilised cell samples were separated by SDS-PAGE and transferred to nitrocellulose membrane. Proteolysis of SNAP-25, a crucial component of the neurosecretory process and the substrate for the zinc-dependent endopeptidase activity of BoNT/A, was then detected by probing with an antibody that recognises both the intact and cleaved forms of SNAP-25. Quantitation of proteolysis was achieved by image analysis using a Synoptics Syngene GeneGnome imaging system and GeneTools software.

**Example 8 Activity of SST-$LH_N$/D in cultured neuroendocrine cells (GH3)**

The rat pituitary cell line GH3 is an example of a cell line of neuroendocrine origin. It thus represents a model cell line for the investigation of inhibition-of-release effects of the agents.

GH3 cells possess surface receptors that allow for the binding, and internalisation of SST-$LH_N$/D. In contrast, GH3 cells lack suitable receptors for clostridial neurotoxins and are therefore not susceptible to botulinum neurotoxins (BoNTs).

Figure 4 illustrates the inhibition of release of growth hormone (GH) from GH3 cells after prior incubation with SST-$LH_N$/D It is clear that dose-dependent inhibition is observed, indicating that SST-$LH_N$/D can inhibit the release of GH from a neuroendocrine cell model.

Comparison of the inhibition effects observed with conjugate and the untargeted $LH_N$/D demonstrate the contribution of the targeting moiety (TM) to efficient inhibition of transmitter release.

*Materials and Methods*

GH enzyme immunoassay kits were obtained from Millipore, MA, USA. GH3 cells were cultured on 24 well plates in F-10 nutrient mixture (Ham) supplemented with 15 % Horse Serum, 2.5 % FBS, 2 mM L-Glutamine. Cells were treated with SST-$LH_N$/D or $LH_N$/D for 72 hours then the cells washed to remove unbound SST-$LH_N$/D. Secretion was stimulated by exposing the cells to 10 μM tetradecanoyl phorbol acetate (TPA, PMA) over 30 min. The medium was harvested from the cells and stored at -20°C until assayed for GH content using the immunoassay kit and following the manufacturer's instructions. Stimulated secretion was calculated by subtracting basal release from total release under stimulating conditions.

**Example 9 Method for alleviating acromegalic symptoms by reducing elevated GH and IGF-1 levels resulting from pituitary adenoma**

A 35 year old male member of a regional badminton team undergoes a spinal X-ray for lower back pain. The consultant notices abnormal bone growth and, on questioning, the man reports increasing incidents of sleep apnoea and also increasingly oily skin.

The physician recommends measurement of circulating IGF-1 and these are found to be elevated. Subsequent tests also show above-normal circulating GH levels so a cranial MRI scan is carried out. This shows a pituitary tumour of 9mm diameter. The patient is treated with a cortistatin or somatostatin peptide TM fusion protein (eg. SEQ ID 7-16, 18-24, 26-31) by i.v. injection.

At intervals of 1 week circulating IGF-1 levels are measured and are seen to be lower at the first measurement and to reduce steadily to 15% above normal over the following six weeks. The level of circulating GH is found to be normal at this time. A further dose of the medication with two-weekly IGF-1 measurements shows this hormone to have stabilised at the upper end of normal. At six weeks after the second treatment a cranial MRI scan reveals shrinkage of the tumour to 6 mm. The therapy is continued at a reduced dosage at two-monthly intervals with IGF-1 and GH levels measured on the seventh week. These are both stable in the normal range and the sleep apnoea and oily skin are now absent. A spinal X-ray at one year following the first treatment shows no increased bone size from the original observation.

**Example 10 Method for normalising swollen hirsute fingers by reducing elevated GH and IGF-1 levels resulting from pituitary adenoma**

A 50 year old female confectionery worker has increasing difficulty removing her wedding ring and eventually visits her medical practitioner. The physician also notices the patient's fingers are hairier than expected and, on questioning, the patient admits that both these conditions have arisen gradually. Subsequent clinical tests reveal a higher-than-average level of circulating GH that does not change following a high-glucose drink. An acromegalic condition is suspected and a cranial CT scan confirms the presence of a small pituitary tumour.

Surgery is considered inappropriate so the patient is treated with an i.v. injection of a somatostatin or cortistatin peptide TM fusion protein (eg. SEQ ID 7-16, 18-24, 26-31). Within four weeks the glucose tolerance test shows a response in GH levels and IGF-1 levels are near normal. Treatment continues at six-weekly intervals and by the end of the eighteenth week the patient is able to remove her ring easily and the hirsutism has disappeared.

**Example 11 Method for ameliorating the consequences of re-emerging growth-hormone-secreting pituitary adenoma**

A 52 year-old male scuba diver presents with increasingly noticeable acromegalic symptoms, including soft tissue swelling and enlargement of the extremities. Thorough tests confirm the presence of a 12mm pituitary adenoma. Somatostatin analogues are poorly tolerated by the patient so the tumour is resected and regular tests over 2 years show circulating GH and IGF-1 levels to be in the upper range of normal and no further medication is given. Eighteen months later, upon presenting with hyperhydrosis and moderate hypertension, GH and IGF-1 levels are found to be above normal and a CT scan reveals regrowth of the pituitary adenoma. Repeat resection is considered undesirable.

The man is treated by i.v. administration of a somatostatin or cortistatin peptide TM fusion protein (eg. SEQ ID 7-16, 18-24, 26-31). A course of radiotherapy is also given and after four weeks the hyperhydrosis and hypertension are near normal as are the GH and IGF-1 levels. Over the next three years symptoms do not recur and there is no tumour regrowth at five years post-treatment.

**Example 12 Method for treating acromegalic patients resistant to somatostatin analogues**

After six years' successful control of circulating GH and IGF-1 by somatostatin analogues, a 60-year-old acromegalic fairground tarot reader reports increasingly obvious oily skin and also prominent body odour as a result of hyperhydrosis. She is found to be glucose-intolerant and to have elevated circulating IGF-1 levels and raising the SSA dosage does not control these.

She is treated by localised injection of a somatostatin or cortistatin peptide TM fusion protein (eg. SEQ ID 7-16, 18-24, 26-31). Within 14 days the patient reports a significant reduction in sweating. Over the following month her oily skin returns to normal and at this time her GH and IGF-1 levels are both within the normal range. This situation remains over the next five years.

**Example 13 Method for treating Cushing's disease in patients intolerant of somatostatin analogues**

A 30 year old female mature student visits her GP to request treatment for anxiety and depression. The physician observes the woman has a rounded face with increased fat around the neck and also thinner than normal arms and legs. Upon questioning she confirms an irregular menstrual cycle. A 24-hour urinary free cortisol level of 150μg is measured suggesting Cushing's syndrome. Abdominal MRI scan shows no adrenal tumours to be present but cranial MRI scan reveals a small pituitary tumour.

The patient is considered unsuitable for surgical intervention so is treated with a somatostatin or cortistatin peptide TM fusion protein (eg. SEQ ID 7-16, 18-24, 26-31).

**Example 14 Method for reversing female sexual impotence by treating prolactinoma**

A 36 year old woman visits her doctor, worried about her recent expression of breast milk, despite her negative pregnancy test. Examination also indicates vaginal dryness and she confirms that she has lost her libido. Clinical test results are largely normal with the notable exception of moderate hyperprolactinaemia. A cranial MRI scan indicates a pituitary adenoma, consistent with the elevated prolactin levels.

She is treated by oral administration with a preparation of a somatostatin or cortistatin peptide TM fusion protein (eg. SEQ ID 7-16, 18-24, 26-31). After eight days she no longer expresses breast milk and her vaginal moisture levels have significantly improved. After seven weeks the dryness begins to return but is almost immediately reversed by a second treatment. Treatments continue at six-weekly visits to the sexual health clinic where the woman reports a return to normal sexual activity.

**Example 15 Method for bringing about weight loss by treating insulinoma**

A 64 year old female with a BMI of 39 has been diagnosed with inoperable insulinoma. She wishes to achieve a sustained reduction in appetite and weight to enable her to maintain an active interest in aerobics so is treated by a systemic injection of a fusion protein comprising a somatostatin or cortistatin peptide TM (eg. SEQ ID 7-16, 18-24, 26-31). Within 10 to 14 days following treatment her weight gain has stabilised and by 30 days weight loss has occurred. The patient maintains a significant weight loss provided medication continues as a series of 24-weekly injections

**Example 16 Method for treating glucagonoma**

A 63-year-old woman visits her doctor in a distressed state, having had rashes develop on her buttocks, around her groin and on her lower legs. Blood tests show her to be anaemic and diabetic. She also has frequent diarrhoeal episodes. The physician suspects the presence of glucagonoma and a CT scan confirms the existence of a tumour in the tail of the pancreas.

The patient is treated with a fusion protein comprising a somatostatin or cortistatin peptide TM (eg. SEQ ID 7-16, 18-24, 26-31). After 4 weeks the diarrhoeal episodes have subsided and the rashes have cleared significantly. Her red-cell count has also returned to near normal. The treatment is repeated at six-weekly intervals and the symptoms remain largely under control.

**Example 17 Method for treating diarrhoea and flushing caused by VIPoma**

A 49 year old man suffers from secretory diarrhoea associated with chronic flushing. Clinical tests indicate metabolic acidosis, and an abdominal CT scan reveals a tumour - almost certainly a VIPoma - near the pancreas.

Surgery is not available to the patient so he is treated with a fusion protein comprising a somatostatin or cortistatin peptide TM (eg. SEQ ID 7-16, 18-24, 26-31). Within 3 weeks the flushing has stopped and the diarrhoea has become less frequent. By seven weeks after treatment all symptoms have disappeared and remain absent providing therapy is repeated at approximately 8-week intervals.

**Example 18 Method for treating gastrinoma**

A 47-year-old man suffers from severe peptic ulceration that causes debilitating abdominal pain. He also experiences unexplained diarrhoeal episodes and eventually is diagnosed with intrapancreatic gastrinoma by blood tests and abdominal ultrasound study.

He is treated by intra-tumoural injection of a medication consisting of a fusion protein comprising a somatostatin or cortistatin peptide TM (eg. SEQ ID 7-16, 18-24, 26-31), or fusion comprising a GnRH peptide TM (eg. SEQ ID 93-94). Within a week painful gastric symptoms start to improve. The hypergastrinaemia has subsided and the diarrhoeal episodes have reduced in severity and frequency. This status pertains for 7 weeks but blood gastrin levels start to rise thereafter. The therapy is repeated at 7 week intervals and this maintains blood gastrin at normal levels and no other symptoms recur.

**Example 19 Method for treating thyrotoxicosis caused by thyrotrophinoma**

A 39-year-old female airline cabin crew member visits her physician complaining of excessive sweating, coupled with previously unknown nervousness, that have started to affect her ability to perform her job. During the consultation a fine tremor is evident and the doctor suspects thyrotoxicosis. The woman is referred to an endocrinologist who carries out a number of blood tests. The major abnormalities detected are elevated thyroxine levels but also elevated TSH (thyrotrophin) levels, indicative of a thyrotrophinoma. An MRI scan of the head confirms the presence of a pituitary tumour.

The woman is treated with a medication consisting of a fusion protein comprising somatostatin or cortistatin peptide TM (eg. SEQ ID 7-16, 18-24, 26-31). Both the sweating and nervousness decline over the following two weeks. Two-weekly follow-up blood tests show both thyroxine and thyrotrophin levels falling and they reach normal levels by six weeks. The patient is able to resume full employment activity.

**Example 20 Method for treating recurrent soft tissue swelling caused by acromegaly**

A 72-year-old woman, having already had transsphenoidal surgery to remove a pituitary macroadenoma, shows recurrence of acromegalic symptoms (primarily swelling of fingers and tongue and increasing tiredness and lethargy). Cranial MRI scanning reveals the presence of a putative pituitary microadenoma and subsequent blood tests confirm elevated circulating GH and IGF-1 levels.

Surgery is deemed incompatible with pre-existing medical conditions so she is treated with a medication consisting of a fusion protein comprising a somatostatin or cortistatin peptide TM (eg. SEQ ID 7-16, 18-24, 26-31). After a week she reports feeling generally more active and that the swelling of her fingers and tongue has reduced noticeably. By three weeks the recurrent symptoms have reverted completely and endocrinological examination confirms a normalisation of GH and IGF-1 levels. She is monitored on a monthly basis and given repeat treatments at 10-weekly intervals. This dosage regimen keeps the hormone levels within the normal range and prevents recurrence of symptoms.

**Example 21 Method for treating excessive facial hirsutism caused by Cushing's Disease**

A 27-rear-old beauty consultant starts to develop noticeable facial hair growth. This is not adequately treated by standard hair-removal methods and is causing her severe psychological problems (anxiety, depression) in relation to both her employment and her personal life. Her physician suspects Cushing's syndrome so she is referred to an endocrinologist. Blood and urine tests show elevated levels of cortisol and ACTH levels, and a CRH stimulation test proves positive, confirming the likelihood of an ACTH-secreting pituitary tumour. Adrenal and pituitary CT-scans confirm the presence of a pituitary tumour but no adrenal abnormality.

Following discussions with consultants the patient opts for medical intervention and is treated with a medication consisting of a fusion protein comprising a somatostatin or cortistatin peptide TM (eg. SEQ ID 7-16, 18-24, 26-31), or fusion comprising a GnRH peptide TM (eg. SEQ ID 93-94). Within ten days the woman is starting to feel more positive and by the two week time point she has to use hair bleaching or depilatory creams with much lower frequency. The symptoms start to reappear at around ten to twelve weeks so a second treatment is given. A similar pattern of symptom remission, gradual reappearance and treatment occurs. During the third treatment, the patient elects for surgical removal of the pituitary tumour. Follow-up monitoring for the next two years shows no recurrence of symptoms or tumour.

**Example 22 Method for treating male galactorrhea caused by prolactinoma**

A 40-year-old male rugby player has been worried for some time about increasing breast size beyond that expected from training. He becomes highly stressed when a trickle of milk appears at the left breast. His physician immediately suspects the existence of a pituitary prolactinoma and refers him to a radiologist and endocrinologist. Blood tests show hyperprolactinaemia but normal thyroid function. A cranial MRI scan shows a pituitary tumour to be present.

In the absence of any tumour-mass effect the man is treated with a medication consisting of a fusion protein comprising a somatostatin or cortistatin peptide TM (eg. SEQ ID 7-16, 18-24, 26-31). After only four days the milk expression has ceased and after six weeks there has been a measurable reduction in non-muscle breast tissue. During this period the blood prolactin levels were measured fortnightly and had returned to normal by the four-week measurement. The treatment is repeated at 12-week intervals during which time there is no recurrence of symptoms and no indication of tumour growth. Surgery or other tumour-reduction treatment is considered unnecessary while these conditions pertain.

**Example 23 Method for treating multiple symptoms caused by insulinoma**

A 51-year-old man is diagnosed with insulinoma after presenting to the doctor with a variety of recently occurring conditions including blurred vision, palpitations, weakness, amnesia and, on two occasions in three months has passed out. The diagnosis is confirmed by endocrinological and radiographic tests.

He is treated with a medication consisting of a fusion protein comprising a somatostatin or cortistatin peptide TM (eg. SEQ ID 7-16, 18-24, 26-31), or fusion comprising a GnRH peptide TM (eg. SEQ ID 93-94). Within a week his vision and energy levels have returned to near normal and continue to improve over the following fortnight. At four weeks he is no longer hypoglycaemic and at that point laparoscopic enucleation of a pancreatic head tumour is performed. Subsequent patient monitoring records no return of symptoms or tumour mass and the patient remains healthy after three years.

**Example 24 Method for treating acromegalic patients resistant to somatostatin analogues**

After 3 years' successful control of circulating GH and IGF-1 by somatostatin analogues, a 54-year-old acromegalic office worker reports increasingly obvious oily skin and also prominent body odour as a result of hyperhydrosis. She is found to be glucose-intolerant and to have elevated circulating IGF-1 levels and raising the SSA dosage does not control these.

She is treated by intravenous injection of a fusion protein comprising a growth hormone releasing hormone peptide TM (eg. SEQ ID 34, 42-47, 60-92). Within 14 days the patient reports a significant reduction in sweating. Over the following month her oily skin returns to normal and at this time her GH and IGF-1 levels are both within the normal range. This situation remains over the next five years.

**Example 25 Method for treating Cushing's disease in patients intolerant of somatostatin analogues**

A 37 year old female receptionist visits her GP to request treatment for anxiety and depression. The physician observes the woman has a rounded face with increased fat around the neck and also thinner than normal arms and legs. Upon questioning she confirms an irregular menstrual cycle. A 24-hour urinary free cortisol level of 150 $\mu$g is measured suggesting Cushing's syndrome. Abdominal MRI scan shows no adrenal tumours to be present but cranial MRI scan reveals a small pituitary tumour.

The patient is considered unsuitable for surgical intervention so is treated with an intravenous injection of fusion protein comprising a urotensin peptide TM (eg. SEQ ID 48).

**Example 26 Method for reversing female sexual impotence by treating prolactinoma**

A 28 year old woman visits her doctor, worried about her recent expression of breast milk, despite her negative pregnancy test. Examination also indicates vaginal dryness and she confirms that she has lost her libido. Clinical test results are largely normal with the notable exception of moderate hyperprolactinaemia. A cranial MRI scan indicates a pituitary adenoma, consistent with the elevated prolactin levels.

She is treated by an intravenous injection of a fusion protein comprising a ghrelin peptide (GHRP) TM (eg. SEQ ID 33, 35, 38), or fusion comprising a GnRH peptide TM (eg. SEQ ID 93-94). After four days she no longer expresses breast milk and her vaginal moisture levels have significantly improved. After thirteen weeks the dryness begins to return but is almost immediately reversed by a second treatment. Treatments continue at twelve-weekly visits to the sexual health clinic where the woman reports a return to normal sexual activity.

**Example 27 Method for treating Cushing's disease**

A 30 year old female typist visits her GP to request treatment for anxiety and depression. The physician observes the woman has a rounded face with increased fat around the neck and also thinner than normal arms and legs. Upon questioning she confirms an irregular menstrual cycle. A 24-hour urinary free cortisol level of 200 μg is measured suggesting Cushing's syndrome. Abdominal MRI scan shows no adrenal tumours to be present but cranial MRI scan reveals a small pituitary tumour.

The patient is considered unsuitable for surgical intervention so is treated with a fusion protein comprising a bombesin peptide (GRP) TM (eg. SEQ ID 40-41), or fusion comprising a GnRH peptide TM (eg. SEQ ID 93-94).

**Example 28 Method for treating gastrinoma**

A 63-year-old man suffers from severe peptic ulceration that causes debilitating abdominal pain. He also experiences unexplained diarrhoeal episodes and eventually is diagnosed with intrapancreatic gastrinoma by blood tests and abdominal ultrasound study.

He is treated by intra-tumoural injection of a medication consisting of a fusion protein comprising a somatostatin or cortistatin peptide TM analogue (octreotide - SEQ ID 54), which has been chemically conjugated to the protease-translocation protein (eg. SEQ ID 49-53). Within a week painful gastric symptoms start to improve. The hypergastrinaemia has subsided and the diarrhoeal episodes have reduced in severity and frequency. This status pertains for 8 weeks but blood gastrin levels start to rise thereafter. The therapy is repeated at 8 week intervals and this maintains blood gastrin at normal levels and no other symptoms recur.

**Example 29 Method for alleviating acromegalic symptoms by reducing elevated GH and IGF-1 levels resulting from pituitary adenoma**

A 50 year old female reports to her GP increasing incidents of sleep apnoea and also increasingly oily skin and the GP observes abnormal bone growth. The GP recommends measurement of circulating IGF-1 and these are found to be elevated. Subsequent tests also show above-normal circulating GH levels so a cranial MRI scan is carried out. This shows a pituitary tumour of 5mm diameter. The patient is treated with a MCH fusion protein (eg. SEQ ID 57) by i.v. injection.

At intervals of 1 week circulating IGF-1 levels are measured and are seen to be lower at the first measurement and to reduce steadily to 5% above normal over the following eight weeks. The level of circulating GH is found to be normal at this time. A further dose of the medication with two-weekly IGF-1 measurements shows this hormone to have stabilised at the upper end of normal. At six weeks after the second treatment a cranial MRI scan reveals shrinkage of the tumour to 3 mm. The therapy is continued at a reduced dosage at two-monthly intervals with IGF-1 and GH levels measured on the seventh week. These are both stable in the normal range and the sleep apnoea and oily skin are now absent.

**Example 30 Method for treating acromegalic patients resistant to somatostatin analogues**

After 1 years' successful control of circulating GH and IGF-1 by somatostatin analogues, a 40-year-old acromegalic digger driver reports increasingly obvious oily skin and also prominent body odour as a result of hyperhydrosis. He is found to be glucose-intolerant and to have elevated circulating IGF-1 levels and raising the SSA dosage does not control these.

He is treated by intravenous injection of a fusion protein comprising a KISS1R binding peptide TM (eg. SEQ ID 58), or fusion comprising a GnRH peptide TM (eg. SEQ ID 93-94). Within 14 days the patient reports a significant reduction in sweating. Over the following month his oily skin returns to normal and at this time her GH and IGF-1 levels are both within the normal range. This situation remains over the next five years.

**Example 31 Method for treating acromegaly**

A patient reports to her GP that she can no longer fit into her size 8 shoes, a size she have worn for the past 25 years, and that her wedding ring will no longer fit. After ruling out obesity, the GP suspects this could be the result of a pituitary disorder the GP refers the patient for tests which confirm significantly elevated IGF-1 and GH levels. A cranial MRI confirms the presence of a pituitary adenoma.

She is treated by intravenous injection of a fusion protein comprising a prolactin releasing hormone receptor binding peptide TM (eg. SEQ ID 59). Over the following months GH and IGF-1 levels return to normal and this is maintained by a quarterly injection on the fusion protein.

**Example 32 Activity of CP-GHRH-LHD on rat IGF-1 levels *in vivo***

*Aims*

To assess the impact of *i.v.* adminisation of CP-GHRH-LHD fusion on IGF-1 levels in rats five days after treatment compared with vehicle only treated control.

*Materials and Methods*

Animals: Adult male Sprague-Dawley rats maintained under standard housing conditions with lights on at 05.00h (14L:10D), food and water available ad libitum and habituated to housing conditions for at least 1 week prior to surgery.

Surgery: On day 1 of the study rats (200-250g) will be anaesthetised with a combination of Hypnorm (0.32 mg/kg fentanyl citrate and 10 mg/kg fluanisone, i.m.) and diazepam (2.6 mg/kg i.p.). The right jugular vein is exposed and a silastic tipped (i.d. 0.50 mm, o.d. 0.93 mm) polythene cannula (Portex, UK) inserted into the vessel until it lies close to the entrance of the right atrium. Cannulae will be prefilled with heparinised (10IU/ml) isotonic saline. The free end of the cannulae will be exteriorised through a scalp incision and then tunnelled through a protective spring anchored to the skull using two stainless steel screws and self-curing dental acrylic. Following recovery animals are housed in individual cages in the automated blood sampling room. The end of the protective spring is attached to a mechanical swivel that allows the animal maximum freedom of movement. Cannulae are flushed daily with heparinised saline to maintain patency.

Treatment: At 09:00 on day 2 of the study rats will receive in i.v. injection of CP-GHRH-LHD or vehicle only control.

Sampling: The automated blood-sampling system (ABS) has been previously described (Clark et al., 1986; Windle et al., 1997). Three to four days after surgery the jugular vein cannula of each animal will be connected to the automated blood-sampling system. At 07:00 on day 6 sampling will begin. Blood samples will be collected at 10 minute intervals using the automated system for a 24 hour period. A total of 144 blood samples will be collected for each will contain no more than 38μl of whole blood.

*Results*

The IGF-1 levels were measure using an IGF-1 ELISA kit. Figure 5 illistrates a statistically significant reduction in the IGF-1 levels in the fusion treated rats compared to the vehicle only control with a t-test P value = 0.0416 after only five days.

**Example 33 Activity of CP-GHRH-LHD on rat IGF-1 levels *in vivo***

*Aims:*

This study is designed to investigate the activity timecourse for CP-GHRH-LHD fusion identifying the time delay between administration and initall effect of the compound in IGF-1 levels.

*Materials and Methods:*

Animals: Adult male Sprague-Dawley rats maintained under standard housing conditions with lights on at 05.00h (14L:10D), food and water available ad libitum and habituated to housing conditions for at least 1 week prior to surgery.

Surgery: On day 1 of the study rats (260-280g) will be anaesthetised with a combination of Hypnorm and diazepam. The right jugular vein is then exposed and a silastic tipped (i.d. 0.50 mm, o.d. 0.93 mm) polythene cannula (Portex, UK) inserted into the vessel until it lies close to the entrance of the right. Cannulae will be prefilled with heparinised (10 IU/ml) isotonic saline. The free end of the cannulae will be exteriorised through a scalp incision and passed through a spring anchored to the skull using stainless steel screws and dental cement. Following recovery animals will be housed in individual cages in the ABS room. The spring will be attached to a swivel that allows the animal maximum freedom of movement. Cannulae will be flushed daily with heparinised saline to maintain patency.

Treatment: At 10:00h on day 5 of the study rats will receive in i.v. injection of the CP-GHRH-LHD or vehicle (sterile saline).

Blood sampling: After flushing the cannulae a single manual blood sample (100μl) will be taken from each rat at 09.30h. Samples will be taken from day 5 to day 18 of the experiment (or until the cannulae block). Plasma from blood samples will be stored at -20C for later analysis of IGF-1 content by ELISA kit.

*Results*

Figure 6 illustrates a statistically significant reduction in the IGF-1 levels in the fusion treated rats compared to the vehicle only control from day four after treatment.

**Example 34 Activity of CP-GHRH-LHD on rat growth hormone levels *in vivo***

*Aims*

To assess the impact of *i.v.* adminisation of CP-GHRH-LHD fusion on growth hormone levels in rats five days after treatment compared with vehicle only treated and Octreotide infusion controls.

*Materials and Methods*

Animals: Adult male Sprague-Dawley rats maintained under standard housing conditions with lights on at 05.00h (14L:10D), food and water available ad libitum and habituated to housing conditions for at least 1 week prior to surgery.

Surgery: On day 1 of the study rats (200-250g) will be anaesthetised with a combination of Hypnorm (0.32 mg/kg fentanyl citrate and 10 mg/kg fluanisone, i.m.) and diazepam (2.6 mg/kg i.p.). The right jugular vein is exposed and a silastic tipped (i.d. 0.50 mm, o.d. 0.93 mm) polythene cannula (Portex, UK) inserted into the vessel until it lies close to the entrance of the right atrium. Cannulae will be prefilled with heparinised (10IU/ml) isotonic saline. The free end of the cannulae will be exteriorised through a scalp incision and then tunnelled through a protective spring anchored to the skull using two stainless steel screws and self-curing dental acrylic. Following recovery animals are housed in individual cages in the automated blood sampling room. The end of the protective spring is attached to a mechanical swivel that allows the animal maximum freedom of movement. Cannulae are flushed daily with heparinised saline to maintain patency.

Treatment: At 09:00 on day 2 of the study rats will receive in i.v. injection of the Syntaxin active compound or vehicle. A 12 hour infusion of somatostatin (or an analogue) will begin 6 hours after the start of sampling (administered via one of the dual cannulae lines) and will continue for 12 hours only. [This infusion timing should be an excellent GH assay control as we should see baseline secretion then complete inhibition and then rapid recovery/rebound]

Sampling: The automated blood-sampling system (ABS) has been previously described (Clark et al., 1986; Windle et al., 1997). Three to four days after surgery the jugular vein cannula of each animal will be connected to the automated blood-sampling system. At 07:00 on day 6 sampling will begin. Blood samples will be collected at 10 minute intervals using the automated system for a 24 hour period. A total of 144 blood samples will be collected for each will contain no more than 38μl of whole blood.

*Results*

The growth hormone levels were measure using an RIA assay. Figure 7a illistrates the vehical treated animals which show typical pulsatile release of growth hormone, figure 7b illustrates the complete ablation of the pulsatile growth hormone release after treatment with GHRH-LHD chimera and figure 7c shows the blocking of the pulsatile growth hormone release and subsequent recovery when the Octreotide infusion is stopped.

**CLAUSES**

1. A polypeptide for use in suppressing secretion from a neuroendocrine tumour cell, said polypeptide comprising:

   a. a non-cytotoxic protease, which protease is capable of cleaving a protein of the exocytic fusion apparatus in a neuroendocrine tumour cell;
   b. a Targeting Moiety (TM) that binds to a Binding Site on a neuroendocrine tumour cell, which Binding Site is capable of undergoing endocytosis to be incorporated into an endosome within the neuroendocrine tumour cell; and
   c. a translocation domain that translocates the protease from within the endosome, across the endosomal membrane and into the cytosol of said neuroendocrine tumour cell.

2. A polypeptide for use according to Clause 1, wherein the neuroendocrine tumour cell is selected from the group consisting of cells derived from or contributing to: pituitary tumours; non-carcinoid gastroenteropancreatic neuroendocrine tumours; carcinoid tumours; and phaeochromocytomas.

3. A polypeptide for use according to Clause 1 or 2, wherein the neuroendocrine tumour cell is selected from the group consisting of cells derived from or contributing to somatotrophinomas, insulinomas, gastrinomas, VIPomas, glucagonomas, prolactinomas, corticotrophinomas, thyrotrophinomas, and phaeochromocytomas.

4. A polypeptide for use according to any preceding Clause, wherein the TM binds to a receptor selected from the group comprising: a growth hormone-releasing hormone (GHRH) receptor; a somatostatin (SST) receptor, a cortistatin (CST) receptor; a ghrelin receptor; a bombesin receptor; a urotensin receptor; a melanin-concentrating hormone receptor 1; a KiSS-1 receptor; a gonadotropin-releasing hormone (GnRH) receptor; and/or a prolactin-releasing peptide receptor.

5. A polypeptide for use according to any preceding Clause, wherein the TM comprises a growth hormone releasing hormone (GHRH) peptide, a somatostatin peptide, a cortistatin peptide, a ghrelin peptide, a bombesin peptide, a urotensin peptide, melanin-concentrating hormone peptide, a KISS-1 peptide, a gonadotropin-releasing hormone (GnRH) peptide, or a prolactin-releasing peptide.

6. A polypeptide for use according to any preceding Clause, wherein the non-cytotoxic protease comprises a clostridial neurotoxin L-chain or an IgA protease.

7. A polypeptide for use according to any preceding Clause, wherein the translocation domain comprises a clostridial neurotoxin translocation domain.

8. A polypeptide comprising:

a. a non-cytotoxic protease, which protease is capable of cleaving a protein of the exocytic fusion apparatus in a neuroendocrine tumour cell;
b. a Targeting Moiety (TM) that binds to a Binding Site on a neuroendocrine tumour cell, which Binding Site is capable of undergoing endocytosis to be incorporated into an endosome within the neuroendocrine tumour cell; and
c. a translocation domain that translocates the protease from within the endosome, across the endosomal membrane and into the cytosol of said neuroendocrine tumour cell.

9. A polypeptide according to Clause 8, wherein the neuroendocrine tumour cell is selected from the group consisting of cells derived from or contributing to: pituitary tumours, non-carcinoid gastroenteropancreatic neuroendocrine tumours; carcinoid tumours; phaeochromocytomas, insulinomas, gastrinomas, VIPomas, glucagonomas, prolactinomas, somatotrophinomas, corticotrophinomas, thyrotrophinomas and phaeochromocytomas.

10. A polypeptide according to Clause 8 or Clause 9, wherein the TM binds to a receptor selected from the group comprising: a growth hormone-releasing hormone (GHRH) receptor; a somatostatin (SST) receptor, a cortistatin (CST) receptor; a ghrelin receptor; a bombesin receptor (eg. BRS-1, BRS-2, or BRS-3); a urotensin receptor (eg. a urotensin II receptor); a melanin-concentrating hormone receptor 1; a KiSS-1 receptor; a gonadotropin-releasing hormone (GnRH) receptor; and/or a prolactin-releasing peptide receptor.

11. A polypeptide according to any of Clauses 8-10, wherein the TM comprises a growth hormone releasing hormone (GHRH) peptide, a somatostatin peptide, a cortistatin peptide, a ghrelin peptide, a bombesin peptide, a urotensin peptide, melanin-concentrating hormone peptide, a KISS-1 peptide, a gonadotropin-releasing hormone (GnRH) peptide, or a prolactin-releasing peptide.

12. A polypeptide according to any of Clauses 8-11, wherein the translocation domain comprises a clostridial neurotoxin translocation domain; and/ or wherein the non-cytotoxic protease comprises a clostridial neurotoxin protease or an IgA protease.

13. A polypeptide according to any of Clauses 8-12, wherein said polypeptide comprises an amino acid sequence having at least 90-92%, or at least 95-97%, or at least 98-99% sequence identity to any one of SEQ ID NOs: 7, 8, 9, 10, 11, 12, 13, 14, 15, 16, 18, 19, 20, 21, 22, 23, 24, 26, 27, 28, 29, 30, 31, 33, 34, 35, 36, 37, 38, 39, 40, 41, 42, 43, 44, 45, 46, 47, 48, 57, 58, 59. 60, 61, 62, 63, 64, 65, 66, 67, 68, 69, 70, 71, 72, 73, 74, .75, 76, 77, 78, 79, 80, 81, 82, 83, 84, 85, 86, 87, 88, 89, 90, 91, 92, 93 or 94.

14. A nucleic acid encoding a polypeptide according to any of Clauses 8-13.

15. A nucleic acid encoding a polypeptide according to Clause 8, wherein said nucleic acid comprises a nucleic acid sequence having at least 90-94%, or at least 95-97%, or at least 98-99% sequence identity to any one of SEQ ID NOs: 17 or 25.

16. A method of suppressing secretion from a neuroendocrine tumour cell in a patient, comprising administering to the patient an effective amount of a polypeptide according to any of Clauses 8-13 or a nucleic acid according to Clause 14 or Clause 15.

17. A method according to Clause 16, for suppressing secretion from a neuroendocrine tumour cell selected from the group consisting of cells derived from or contributing to: pituitary tumours, non-carcinoid gastroenteropancreatic neuroendocrine tumours; carcinoid tumours; insulinomas, gastrinomas, VIPomas, glucagonomas, prolactinomas, somatotrophinomas, corticotrophinomas, thyrotrophinomas and phaeochromocytomas.

18. A polypeptide for use according to any of Clauses 1-7 or a method according to Clause 17, for treating Cushing's disease, acromegaly, carcinoid syndrome, hypoglycaemic syndrome, necrolytic migratory erythema, Zollinger-Ellison syndrome and Verner-Morrison syndrome, hepatoma, VIPoma, nesidoblastosis, hyperinsulinism, gastrinoma, hypersecretory diarrhea, irritable bowel syndrome, upper gastrointestinal bleeding, postprandial portal venous hypertension, especially in cirrhotic patients, complications of portal hypertension, small bowel obstruction, diabetic neuropathy, and cancer cachexia; accelerated growth of a solid primary or metastatic tumour resulting from tissue trauma caused surgically, non-surgically, or by tissue ulceration; and epithelial tumours (tumours of epithelial tissues) such as prostate tumour, breast tumour, colon tumour, lung tumour, and melanoma.

## Claims

1. A polypeptide for use in suppressing secretion from a pituitary tumour cell for treating Cushing's disease, said polypeptide comprising:

a a non-cytotoxic protease, which protease is capable of cleaving a protein of the exocytic fusion apparatus in a pituitary tumour cell;
b. a Targeting Moiety (TM) that binds to a Binding Site on a pituitary tumour cell, which Binding Site is capable of undergoing endocytosis to be incorporated into an endosome within the pituitary tumour cell; and
c a translocation domain that translocates the protease from within the endosome, across the endosomal membrane and into the cytosol of said pituitary tumour cell;

wherein the polypeptide lacks a functional $H_c$ domain of a clostridial neurotoxin.

**2.** The polypeptide for use according to claim 1, wherein the pituitary tumour cell is derived from or contributes to corticotrophinomas.

**3.** The polypeptide for use according to claim 1 or 2, wherein the TM binds to a receptor selected from the group comprising: a growth hormone-releasing hormone (GHRH) receptor; a somatostatin (SST) receptor, a cortistatin (CST) receptor; a ghrelin receptor; a bombesin receptor; a urotensin receptor; a melanin-concentrating hormone receptor 1; a KiSS-1 receptor; a gonadotropin-releasing hormone (GnRH) receptor; and/or a prolactin-releasing peptide receptor.

**4.** The polypeptide for use according to any preceding claim, wherein the TM comprises a growth hormone releasing hormone (GHRH) peptide, a somatostatin peptide, a cortistatin peptide, a ghrelin peptide, a bombesin peptide, a urotensin peptide, melanin-concentrating hormone peptide, a KISS-1 peptide, a gonadotropin-releasing hormone (GnRH) peptide, or a prolactin-releasing peptide.

**5.** The polypeptide for use according to any preceding claim, wherein the non-cytotoxic protease comprises a clostridial neurotoxin L-chain or an IgA protease.

**6.** The polypeptide for use according to any preceding claim, wherein the translocation domain comprises a clostridial neurotoxin translocation domain.

**7.** A polypeptide comprising:

a. a non-cytotoxic protease, which protease is capable of cleaving a protein of the exocytic fusion apparatus in a pituitary tumour cell;
b. a Targeting Moiety (TM) that binds to a Binding Site on a pituitary tumour cell, which Binding Site is capable of undergoing endocytosis to be incorporated into an endosome within the pituitary tumour cell; and
c. a translocation domain that translocates the protease from within the endosome, across the endosomal membrane and into the cytosol of said pituitary tumour cell;

wherein the polypeptide lacks a functional $H_c$ domain of a clostridial neurotoxin; and
wherein the polypeptide comprises an amino acid sequence having at least 90-92%, or at least 95-97%, or at least 98-99% sequence identity to any one of SEQ ID NOs: 33, 35, 36, 38, 39, 40, 41, 48, 57, 58, 59, 93 or 94.

**8.** The polypeptide according to claim 7, wherein the TM binds to a receptor selected from the group comprising: a ghrelin receptor; a bombesin receptor (eg. BRS-1, BRS-2, or BRS-3); a urotensin receptor (eg. a urotensin II receptor); a melanin-concentrating hormone receptor 1; a KiSS-1 receptor; a gonadotropin-releasing hormone (GnRH) receptor; and/or a prolactin-releasing peptide receptor.

**9.** The polypeptide according to claim 7 or 8, wherein the TM comprises a ghrelin peptide, a bombesin peptide, a urotensin peptide, melanin-concentrating hormone peptide, a KISS-1 peptide, a gonadotropin-releasing hormone (GnRH) peptide, or a prolactin-releasing peptide.

**10.** The polypeptide according to any of claims 7-9, wherein the translocation domain comprises a clostridial neurotoxin translocation domain; and/ or wherein the non-cytotoxic protease comprises a clostridial neurotoxin protease or an IgA protease.

**11.** A nucleic acid encoding a polypeptide according to any of claims 7-10.

**12.** A method of activating a polypeptide according to any of claims 7-10, said method comprising contacting the polypeptide with a protease that cleaves the polypeptide at a recognition site (cleavage site) located between the non-cytotoxic protease component and the translocation component, and converting the polypeptide into a di-chain polypeptide wherein the non-cytotoxic protease component and the translocation component are joined together by a disulphide bond.

**13.** A di-chain polypeptide obtainable by the method of claim 12.

Figure 1

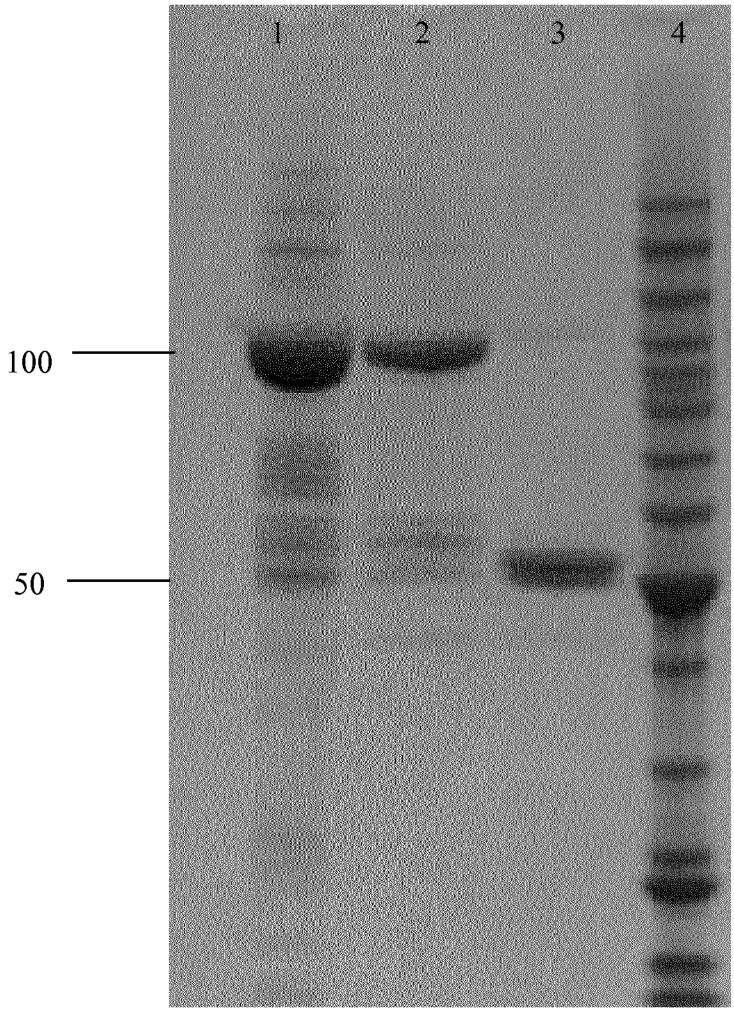

Figure 2

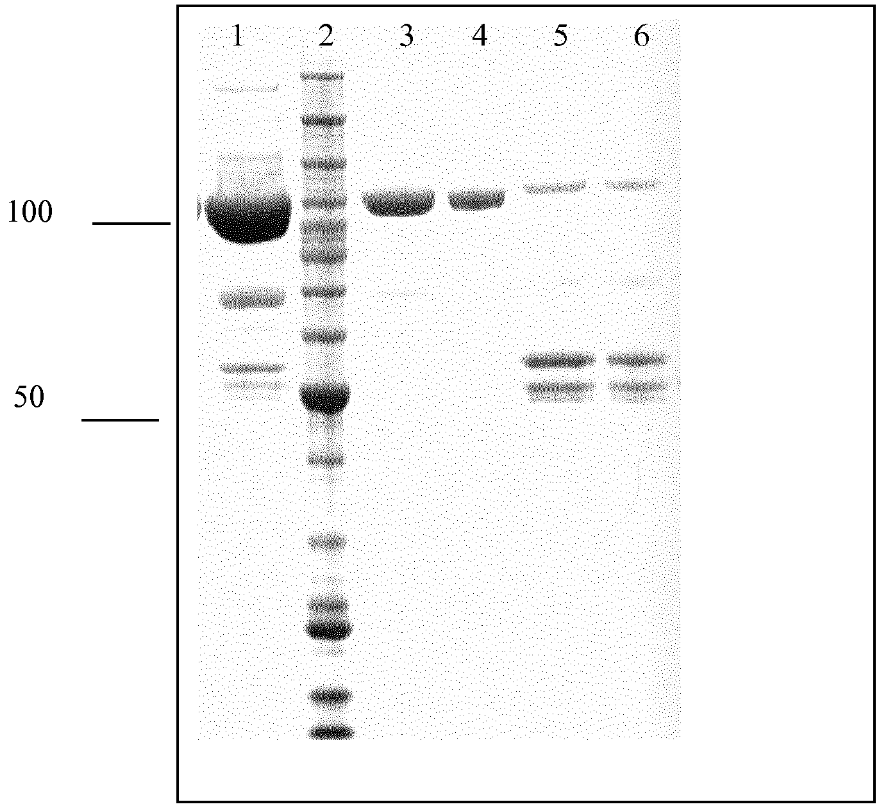

Figure 3a

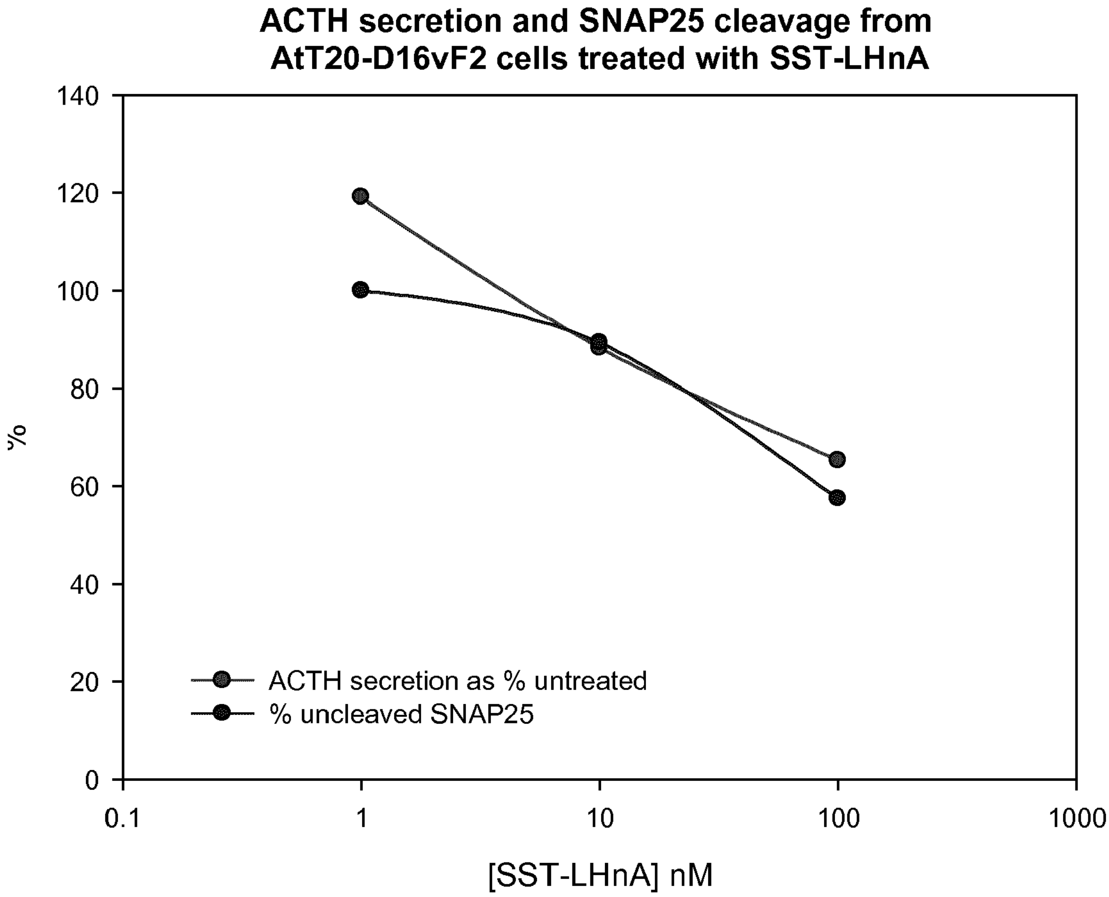


Figure 3b

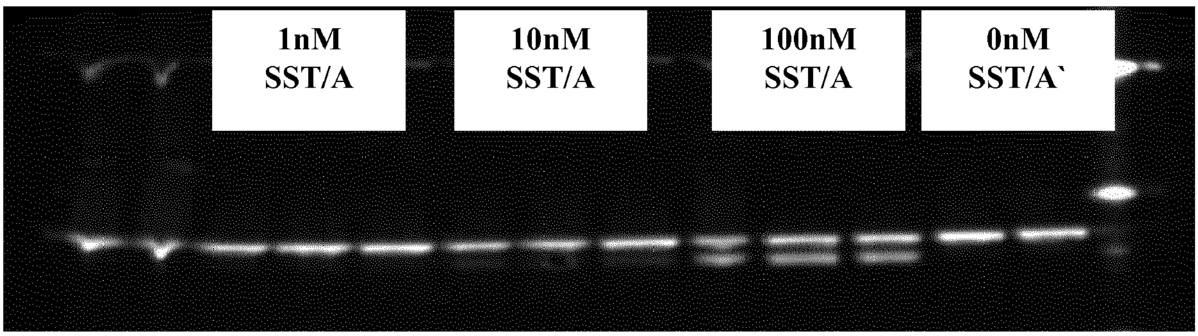

Figure 4

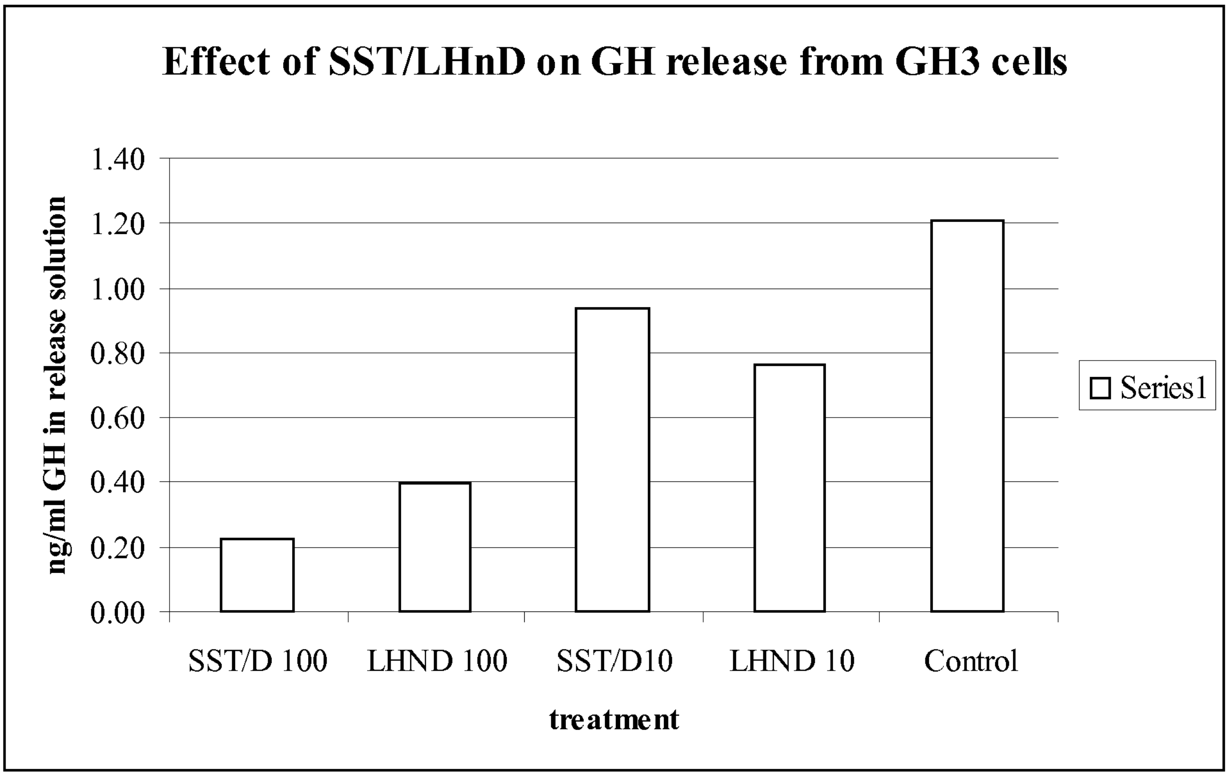
Effect of SST/LHnD on GH release from GH3 cells
ng/ml GH in release solution
1.40
1.20
1.00
0.80
0.60
0.40
0.20
0.00
SST/D 100
LHND 100
SST/D10
LHND 10
Control
treatment
Series1

Figure 5

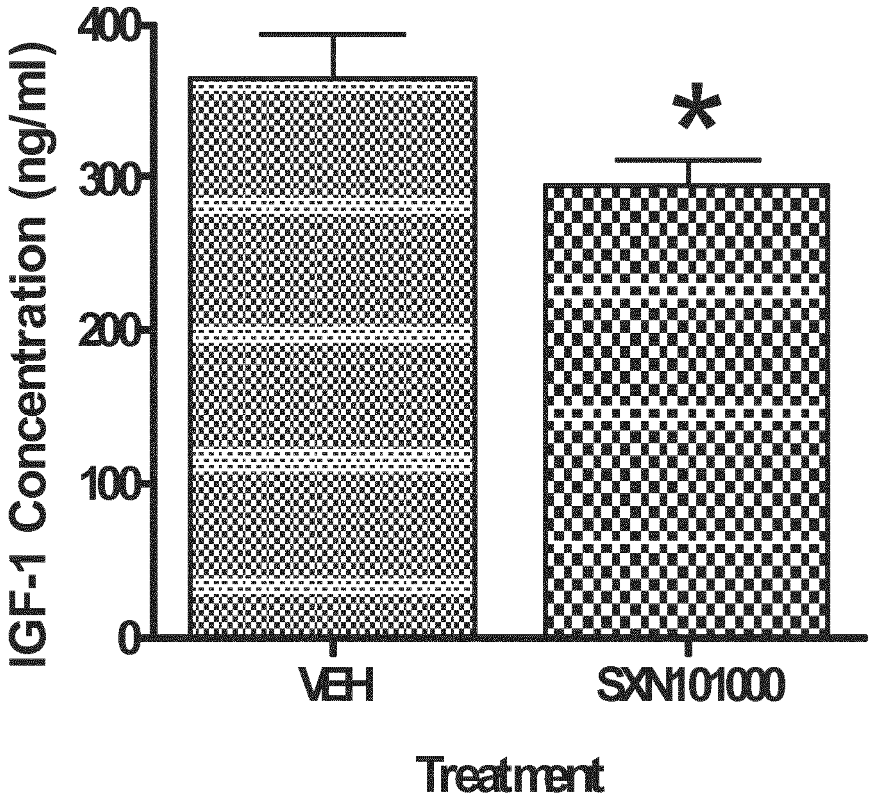
IGF-1 Concentration (ng/ml)
400
300
200
100
0
*
VEH
SXN101000
Treatment

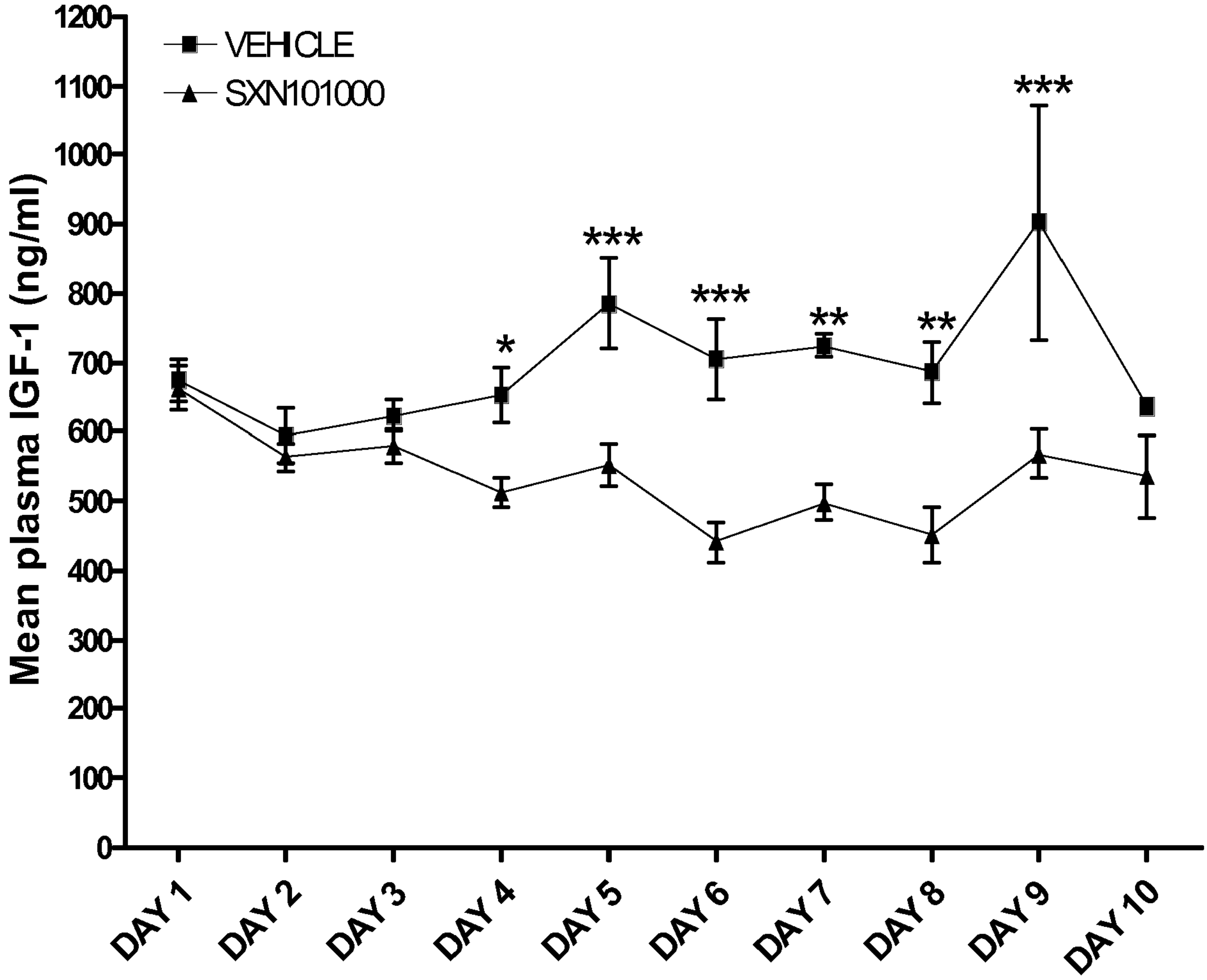
1200
1100
1000
900
800
700
600
500
400
300
200
100
0
Mean plasma IGF-1 (ng/ml)
VEHICLE
SXN101000
*
***
***
**
**
***
DAY 1
DAY 2
DAY 3
DAY 4
DAY 5
DAY 6
DAY 7
DAY 8
DAY 9
DAY 10

Figure 6

Figure 7a

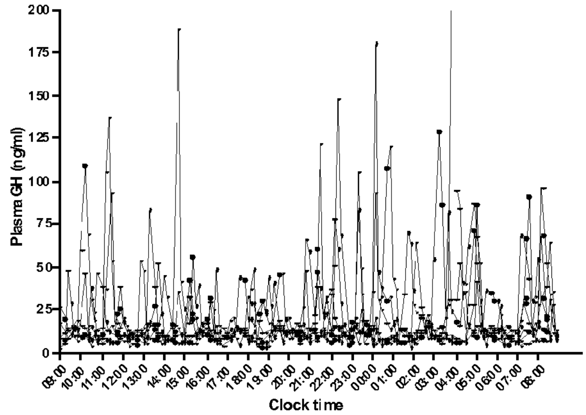


Figure 7b

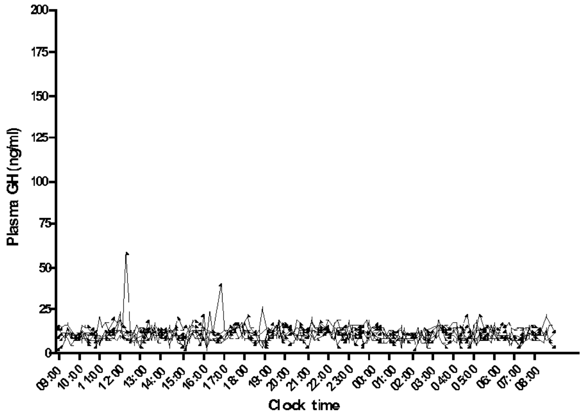


Figure 7c

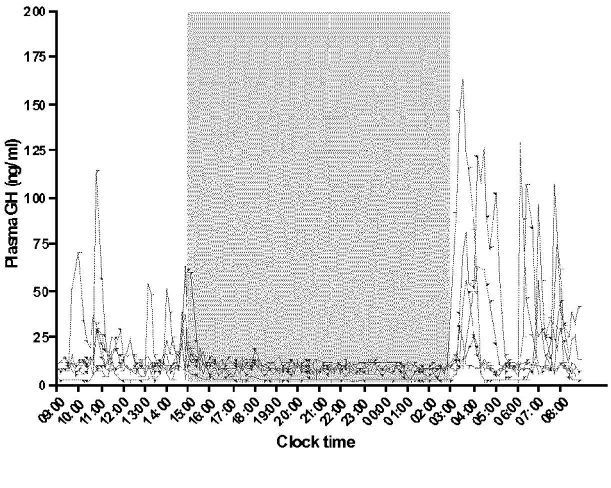

Europäisches Patentamt
European Patent Office
Office européen des brevets

# EUROPEAN SEARCH REPORT

Application Number
EP 19 17 5007

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 01/21213 A2 (MICROBIOLOGICAL RES AUTHORITY [GB]) 29 March 2001 (2001-03-29)<br>* claims 1-39 *<br>----- | 1-11<br>1-11 | INV.<br>C07K14/195<br>A61P35/00<br>C12N9/52<br>A61K38/48<br>C07K1/00 |
| X<br>Y | US 2003/180289 A1 (FOSTER KEITH ALAN [GB] ET AL) 25 September 2003 (2003-09-25)<br>* claims 1-6 *<br>----- | 1-11<br>1-11 | |
| X<br>Y | WO 2006/059113 A2 (HEALTH PROT AGENCY [GB]; FOSTER KEITH [GB] ET AL.) 8 June 2006 (2006-06-08)<br>* claims 1-23 *<br>----- | 12,13<br>1-11 | |
| Y | WO 2006/099590 A2 (ALLERGAN INC [US]; STEWARD LANCE E [US] ET AL.) 21 September 2006 (2006-09-21)<br>* paragraph [0004] - paragraph [0006] *<br>* paragraph [0129] *<br>----- | 1-11 | |
| Y | US 2005/031648 A1 (BRIN MITCHELL F [US] ET AL) 10 February 2005 (2005-02-10)<br>* claims 12,28,26,33,40,52 *<br>----- | 1-11 | TECHNICAL FIELDS SEARCHED (IPC) |
| Y | WO 2006/025976 A1 (ALLERGAN INC [US]; BRIN MITCHELL F [US]; DONOVAN STEPHEN [US]) 9 March 2006 (2006-03-09)<br>* page 55, line 24 - line 26; claim 1 *<br>* page 61, line 23 - line 25 *<br>* page 70, line 24 - line 27 *<br>-----<br>-/-- | 1-11 | A61K<br>A61P<br>C07K |

The present search report has been drawn up for all claims

2

| Place of search | Date of completion of the search | Examiner |
|---|---|---|
| Munich | 5 November 2019 | Rodrigo-Simón, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 19 17 5007

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CHADDOCK J A ET AL: "Clostridial neurotoxins: structure-function led design of new therapeutics", CELLULAR AND MOLECULAR LIFE SCIENCES, BIRKHÄUSER-VERLAG, BA, vol. 63, no. 5, 1 March 2006 (2006-03-01), pages 540-551, XP019201042, ISSN: 1420-9071, DOI: 10.1007/S00018-005-5505-5 * page 546, column 2 * ----- | 1-11 | |
| X | WO 2006/059093 A2 (HEALTH PROT AGENCY [GB]; ALLERGAN INC [US] ET AL.) 8 June 2006 (2006-06-08) * page 21, line 2; claims 1,20; figures 1,2 * ----- | 12,13 | |
| X | US 2008/032931 A1 (STEWARD LANCE E [US] ET AL) 7 February 2008 (2008-02-07) * claims 1,47 * ----- | 12,13 | |
| L | EP 2 719 392 A1 (SYNTAXIN LTD [GB]) 16 April 2014 (2014-04-16) * the whole document * ----- | 1-13 | TECHNICAL FIELDS SEARCHED (IPC) |
| L | EP 2 310 028 A2 (SYNTAXIN LTD [GB]) 20 April 2011 (2011-04-20) * the whole document * ----- | | |
| L | EP 2 310 029 A2 (SYNTAXIN LTD [GB]) 20 April 2011 (2011-04-20) * the whole document * ----- | 1-13 | |

The present search report has been drawn up for all claims

2

| Place of search | Date of completion of the search | Examiner |
|---|---|---|
| Munich | 5 November 2019 | Rodrigo-Simón, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 19 17 5007

This annex lists the patent family members relating to the patent documents cited in the above-mentioned European search report. The members are as contained in the European Patent Office EDP file on
The European Patent Office is in no way liable for these particulars which are merely given for the purpose of information.

05-11-2019

| Patent document cited in search report | Publication date | Patent family member(s) | Publication date |
|---|---|---|---|
| WO 0121213 A2 | 29-03-2001 | AU 782457 B2 | 28-07-2005 |
| | | CA 2383470 A1 | 29-03-2001 |
| | | CA 2805979 A1 | 29-03-2001 |
| | | CA 2847143 A1 | 29-03-2001 |
| | | DK 1235594 T3 | 23-07-2012 |
| | | EP 1235594 A2 | 04-09-2002 |
| | | EP 2110142 A2 | 21-10-2009 |
| | | ES 2386838 T3 | 03-09-2012 |
| | | JP 5416876 B2 | 12-02-2014 |
| | | JP 5514128 B2 | 04-06-2014 |
| | | JP 2003509476 A | 11-03-2003 |
| | | JP 2011074091 A | 14-04-2011 |
| | | JP 2011184459 A | 22-09-2011 |
| | | JP 2014037429 A | 27-02-2014 |
| | | WO 0121213 A2 | 29-03-2001 |
| US 2003180289 A1 | 25-09-2003 | US 2003180289 A1 | 25-09-2003 |
| | | US 2006216283 A1 | 28-09-2006 |
| WO 2006059113 A2 | 08-06-2006 | AU 2005311106 A1 | 08-06-2006 |
| | | BR PI0515760 A | 29-07-2008 |
| | | CA 2593707 A1 | 08-06-2006 |
| | | CN 101098885 A | 02-01-2008 |
| | | CY 1114839 T1 | 14-12-2016 |
| | | DK 1874807 T3 | 09-12-2013 |
| | | EP 1874807 A2 | 09-01-2008 |
| | | ES 2431841 T3 | 28-11-2013 |
| | | JP 5438901 B2 | 12-03-2014 |
| | | JP 2008521428 A | 26-06-2008 |
| | | PT 1874807 E | 09-10-2013 |
| | | SI 1874807 T1 | 31-12-2013 |
| | | US 2008064092 A1 | 13-03-2008 |
| | | US 2009004174 A1 | 01-01-2009 |
| | | US 2012149084 A1 | 14-06-2012 |
| | | US 2013115682 A1 | 09-05-2013 |
| | | WO 2006059113 A2 | 08-06-2006 |
| WO 2006099590 A2 | 21-09-2006 | AT 514709 T | 15-07-2011 |
| | | AU 2006225116 A1 | 21-09-2006 |
| | | AU 2006227816 A1 | 28-09-2006 |
| | | AU 2012203366 A1 | 28-06-2012 |
| | | CA 2601577 A1 | 21-09-2006 |
| | | CA 2601592 A1 | 28-09-2006 |
| | | EP 1861419 A1 | 05-12-2007 |
| | | EP 1871789 A2 | 02-01-2008 |
| | | EP 2316847 A2 | 04-05-2011 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 19 17 5007

This annex lists the patent family members relating to the patent documents cited in the above-mentioned European search report. The members are as contained in the European Patent Office EDP file on
The European Patent Office is in no way liable for these particulars which are merely given for the purpose of information.

05-11-2019

| Patent document cited in search report | Publication date | Patent family member(s) | Publication date |
|---|---|---|---|
| | | JP 2008532549 A | 21-08-2008 |
| | | JP 2008535486 A | 04-09-2008 |
| | | US 2008096248 A1 | 24-04-2008 |
| | | US 2008161543 A1 | 03-07-2008 |
| | | US 2012052548 A1 | 01-03-2012 |
| | | US 2012178140 A1 | 12-07-2012 |
| | | WO 2006099590 A2 | 21-09-2006 |
| | | WO 2006101809 A1 | 28-09-2006 |
| US 2005031648 A1 | 10-02-2005 | AR 050713 A1 | 15-11-2006 |
| | | AT 416786 T | 15-12-2008 |
| | | AU 2005280574 A1 | 09-03-2006 |
| | | BR PI0515352 A | 15-07-2008 |
| | | CA 2580748 A1 | 09-03-2006 |
| | | CN 101031317 A | 05-09-2007 |
| | | EP 1804827 A1 | 11-07-2007 |
| | | EP 1990059 A2 | 12-11-2008 |
| | | ES 2317284 T3 | 16-04-2009 |
| | | JP 4922167 B2 | 25-04-2012 |
| | | JP 2008511627 A | 17-04-2008 |
| | | KR 20070045292 A | 02-05-2007 |
| | | TW I364293 B | 21-05-2012 |
| | | US 2005031648 A1 | 10-02-2005 |
| | | WO 2006025976 A1 | 09-03-2006 |
| WO 2006025976 A1 | 09-03-2006 | AR 050713 A1 | 15-11-2006 |
| | | AT 416786 T | 15-12-2008 |
| | | AU 2005280574 A1 | 09-03-2006 |
| | | BR PI0515352 A | 15-07-2008 |
| | | CA 2580748 A1 | 09-03-2006 |
| | | CN 101031317 A | 05-09-2007 |
| | | EP 1804827 A1 | 11-07-2007 |
| | | EP 1990059 A2 | 12-11-2008 |
| | | ES 2317284 T3 | 16-04-2009 |
| | | JP 4922167 B2 | 25-04-2012 |
| | | JP 2008511627 A | 17-04-2008 |
| | | KR 20070045292 A | 02-05-2007 |
| | | TW I364293 B | 21-05-2012 |
| | | US 2005031648 A1 | 10-02-2005 |
| | | WO 2006025976 A1 | 09-03-2006 |
| WO 2006059093 A2 | 08-06-2006 | AU 2005311086 A1 | 08-06-2006 |
| | | CA 2595115 A1 | 08-06-2006 |
| | | EP 1830872 A2 | 12-09-2007 |
| | | PL 1830872 T3 | 30-09-2011 |
| | | WO 2006059093 A2 | 08-06-2006 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 19 17 5007

This annex lists the patent family members relating to the patent documents cited in the above-mentioned European search report. The members are as contained in the European Patent Office EDP file on
The European Patent Office is in no way liable for these particulars which are merely given for the purpose of information.

05-11-2019

| Patent document cited in search report | Publication date | Patent family member(s) | Publication date |
|---|---|---|---|
| US 2008032931 A1 | 07-02-2008 | NONE | |
| EP 2719392 A1 | 16-04-2014 | AU 2009259033 A1 | 17-12-2009 |
| | | CA 2727082 A1 | 17-12-2009 |
| | | CN 102112145 A | 29-06-2011 |
| | | CN 104328100 A | 04-02-2015 |
| | | EP 2310028 A2 | 20-04-2011 |
| | | EP 2719392 A1 | 16-04-2014 |
| | | ES 2614990 T3 | 02-06-2017 |
| | | IL 209855 A | 30-09-2013 |
| | | JP 5728380 B2 | 03-06-2015 |
| | | JP 5891258 B2 | 22-03-2016 |
| | | JP 2011522560 A | 04-08-2011 |
| | | JP 2014195454 A | 16-10-2014 |
| | | KR 20110038017 A | 13-04-2011 |
| | | US 2011171191 A1 | 14-07-2011 |
| | | WO 2009150469 A2 | 17-12-2009 |
| | | ZA 201008536 B | 30-05-2012 |
| EP 2310028 A2 | 20-04-2011 | AU 2009259033 A1 | 17-12-2009 |
| | | CA 2727082 A1 | 17-12-2009 |
| | | CN 102112145 A | 29-06-2011 |
| | | CN 104328100 A | 04-02-2015 |
| | | EP 2310028 A2 | 20-04-2011 |
| | | EP 2719392 A1 | 16-04-2014 |
| | | ES 2614990 T3 | 02-06-2017 |
| | | IL 209855 A | 30-09-2013 |
| | | JP 5728380 B2 | 03-06-2015 |
| | | JP 5891258 B2 | 22-03-2016 |
| | | JP 2011522560 A | 04-08-2011 |
| | | JP 2014195454 A | 16-10-2014 |
| | | KR 20110038017 A | 13-04-2011 |
| | | US 2011171191 A1 | 14-07-2011 |
| | | WO 2009150469 A2 | 17-12-2009 |
| | | ZA 201008536 B | 30-05-2012 |
| EP 2310029 A2 | 20-04-2011 | AU 2009259034 A1 | 17-12-2009 |
| | | BR PI0915142 A2 | 16-02-2016 |
| | | CA 2726092 A1 | 17-12-2009 |
| | | CN 102083451 A | 01-06-2011 |
| | | CN 107446053 A | 08-12-2017 |
| | | EP 2310029 A2 | 20-04-2011 |
| | | EP 3473643 A1 | 24-04-2019 |
| | | IL 209854 A | 30-09-2013 |
| | | JP 5799397 B2 | 28-10-2015 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 19 17 5007

This annex lists the patent family members relating to the patent documents cited in the above-mentioned European search report.
The members are as contained in the European Patent Office EDP file on
The European Patent Office is in no way liable for these particulars which are merely given for the purpose of information.

05-11-2019

| Patent document cited in search report | Publication date | Patent family member(s) | Publication date |
|---|---|---|---|
| | | JP 2011523859 A | 25-08-2011 |
| | | KR 20110028492 A | 18-03-2011 |
| | | US 2011165137 A1 | 07-07-2011 |
| | | US 2014219983 A1 | 07-08-2014 |
| | | WO 2009150470 A2 | 17-12-2009 |
| | | ZA 201008425 B | 25-04-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9958571 A
- WO 9421300 A
- WO 9633273 A
- WO 9807864 A
- WO 0010598 A
- WO 0121213 A
- WO 06059093 A
- WO 0062814 A
- WO 0004926 A
- WO 9315766 A
- WO 0061192 A
- EP 0257742 A
- US 20070166332 A
- US 5506339 A
- EP 0363589 A
- US 4904642 A
- US 4871717 A
- US 4725577 A
- US 4684620 A
- US 4650787 A
- US 4585755 A
- US 4522813 A
- US 4369179 A
- US 4360516 A
- US 4328214 A
- US 4316890 A
- US 4310518 A
- US 4291022 A
- US 4238481 A
- US 4235886 A
- US 4211693 A
- US 4190648 A
- US 4146612 A
- US 4133782 A
- US 4261885 A
- US 4282143 A
- US 4190575 A
- US 5552520 A
- EP 0389180 A
- EP 0505680 A
- US 4603120 A
- EP 0030920 A
- US 4853371 A
- WO 9012811 A
- WO 9701579 A
- WO 9118016 A
- WO 9808529 A
- WO 9808528 A
- WO 0075186 A
- WO 0006185 A
- WO 9956769 A
- FR 2522655
- EP 0395417 A1
- WO 8802756 A
- EP 0329295 A
- US 5240561 A
- US 4659693 A
- WO 9116923 A
- US 5084555 A
- US 5550212 A
- US 5942489 A
- US 6057422 A
- WO 96032126 A
- WO 96022782 A
- WO 96016707 A
- WO 94011397 A
- WO 94011396 A
- US 502438 A
- US 397169 A
- US 376555 A
- US 394727 A
- US 317941 A
- US 282328 A
- US 257998 A
- US 248771 A
- US 207759 A
- US 204171 A
- US 173311 A
- US 100571 A
- US 520225 A
- US 440039 A
- WO 9220363 A
- EP 0737691 A
- WO 8902897 A
- WO 9117181 A
- WO 9003980 A
- WO 9102746 A
- EP 171477 A
- WO 96033729 A
- WO 92022322 A
- WO 92013883 A
- WO 9105563 A
- WO 2007104567 A
- WO 08008803 A
- WO 08008805 A
- US 5223409 A, Ladner
- WO 9206204 A

**Non-patent literature cited in the description**


- **GERALD K.** Cell and Molecular Biology. John Wiley & Sons, Inc, 2002
- **HERMANSON, G.T.** Bioconjugate techniques. Academic Press, 1996
- **WONG, S.S.** Chemistry of protein conjugation and cross-linking. CRC Press, 1991
- **NAGY et al.** *PNAS,* 1998, vol. 95, 1794-99
- Receptor-binding studies, a brief outline. **HULME, E.C.** Receptor biochemistry, A Practical Approach. Oxford University Press, 1990, 303-311
- **VAN BINST, G. et al.** *Peptide Research,* 1992, vol. 5, 8
- **HORVATH, A. et al.** Conformations of Somatostatin Analogs Having Antitumor Activity. *22nd European peptide Symposium,* 13 September 1992
- **ZACHARY et al.** *Proc. Nat. Aca. Sci.,* 1985, vol. 82, 7616
- Synthetic Peptides: Approaches to Biological Problems. **HEIMBROOK et al.** UCLA Symposium on Mol. and Cell. Biol. New Series. vol. 86
- **HEINZ-ERIAN et al.** *Am. J. Physiol.,* 1986, vol. G439
- **MARTINEZ et al.** *J. Med. Chem.,* 1985, vol. 28, 1874
- **GARGOSKY et al.** *Biochem. J.,* 1987, vol. 247, 427
- **DUBREUIL et al.** Drug Design and Delivery. Harwood Academic Publishers, 1987, vol. 2, 49
- **HEIKKILA et al.** *J. Biol. Chem.,* 1987, vol. 262, 16456
- **CARANIKAS et al.** *J. Med. Chem.,* 1982, vol. 25, 1313
- **SAEED et al.** *Peptides,* 1989, vol. 10, 597
- **ROSELL et al.** *Trends in Pharmacological Sciences,* 1982, vol. 3, 211
- **LUNDBERG et al.** *Proc. Nat. Aca. Sci.,* 1983, vol. 80, 1120
- **ENGBERG et al.** *Nature,* 1984, vol. 293, 222
- **MIZRAHI et al.** *Euro. J. Pharma.,* 1982, vol. 82, 101
- **LEANDER et al.** *Nature,* 1981, vol. 294, 467
- **WOLL et al.** *Biochem. Biophys. Res. Comm.,* 1988, vol. 155, 359
- **RIVIER et al.** *Biochem.,* 1978, vol. 17, 1766
- **CUTTITTA et al.** *Cancer Surveys,* 1985, vol. 4, 707
- **AUMELAS et al.** *Int. J. Peptide Res.,* 1987, vol. 30, 596
- **SHONE et al.** *Eur. J. Biochem.,* 1985, vol. 151, 75-82
- **RUMMEL et al.** *Molecular Microbiol.,* 2004, vol. 51, 631-634
- **UMLAND TC.** *Nat. Struct. Biol.,* 1997, vol. 4, 788-792
- **HERREROS J.** *Biochem. J.,* 2000, vol. 347, 199-204
- **HALPERN J.** *J. Biol. Chem.,* 1993, vol. 268 (15), 11188-11192
- **RUMMEL A.** *PNAS,* 2007, vol. 104, 359-364
- **LACEY DB.** *Nat. Struct. Biol.,* 1998, vol. 5, 898-902
- **KNAPP.** *Am. Cryst. Assoc. Abstract Papers,* 1998, vol. 25, 90
- **SWAMINATHAN ; ESWARAMOORTHY.** *Nat. Struct. Biol.,* 2000, vol. 7, 1751-1759
- **RUMMEL A.** *Mol. Microbiol.,* 2004, vol. 51 (3), 631-643
- **AHMED, S.A.** *Protein J.,* 2008
- **POHLNER, J. et al.** *Nature,* 1987, vol. 325, 458-462
- **SHONE C.** *Eur. J. Biochem,* 1987, vol. 167 (1), 175-180
- **BLAUSTEIN.** *FEBS Letts,* 1987, vol. 226 (1), 115-120
- Membrane Fusion Techniques, Parts A and B. Methods in Enzymology. Academic Press, 1993, vol. 220, 221
- **HENDERSON et al.** The Clostridia: Molecular Biology and Pathogenesis. Academic press, 1997
- **O'KEEFE et al.** *Proc. Natl. Acad. Sci. USA,* 1992, vol. 89, 6202-6206
- **SILVERMAN et al.** *J. Biol. Chem.,* 1993, vol. 269, 22524-22532
- **LONDON, E.** *Biochem. Biophys. Acta.,* 1992, vol. 1112, 25-51
- **PRIOR et al.** *Biochemistry,* 1992, vol. 31, 3555-3559
- **BLANKE et al.** *Proc. Natl. Acad. Sci. USA,* 1996, vol. 93, 8437-8442
- **PLANK et al.** *J. Biol. Chem.,* 1994, vol. 269, 12918-12924
- **WAGNER et al.** *PNAS,* 1992, vol. 89, 7934-7938
- **MURATA et al.** *Biochem.,* 1992, vol. 31, 1986-1992
- **SILVERMAN et al.** *J. Biol. Chem.,* 1994, vol. 269, 22524-22532
- **LONDON E.** *Biochem. Biophys. Acta.,* 1992, vol. 1113, 25-51
- **KIHARA ; PASTAN.** *Bioconj Chem.,* 1994, vol. 5, 532-538
- **MURATA et al.** *Biochemistry,* 1992, vol. 31, 1986-1992
- **KIELIAN et al.** *J Cell Biol.,* 1996, vol. 134 (4), 863-872
- **YAO et al.** *Virology,* 2003, vol. 310 (2), 319-332
- **SETH et al.** *J Virol,* 2003, vol. 77 (11), 6520-6527
- **PICARD-MAUREAU et al.** *J Virol.,* 2003, vol. 77 (8), 4722-4730

- **JULIE D. THOMPSON et al.** CLUSTAL W: Improving the Sensitivity of Progressive Multiple Sequence Alignment Through Sequence Weighting, Position-Specific Gap Penalties and Weight Matrix Choice. *Nucleic Acids Research,* 1994, vol. 22 (22), 4673-4680
- **OSAMU GOTOH.** Significant Improvement in Accuracy of Multiple Protein. Sequence Alignments by Iterative Refinement as Assessed by Reference to Structural Alignments. *J. Mol. Biol.,* 1996, vol. 264 (4), 823-838
- **ERIC DEPIEREUX ; ERNEST FEYTMANS.** Match-Box: A Fundamentally New Algorithm for the Simultaneous Alignment of Several Protein Sequences. *CABIOS,* 1992, vol. 8 (5), 501-509
- **C. E. LAWRENCE et al.** Detecting Subtle Sequence Signals: A Gibbs Sampling Strategy for Multiple Alignment. *Science,* 1993, vol. 262 (5131), 208-214
- **IVO VAN WALLE et al.** Align-M - A New Algorithm for Multiple Alignment of Highly Divergent Sequences. *Bioinformatics,* 2004, vol. 20 (9), 1428-1435
- **ALTSCHUL et al.** *Bull. Math. Bio.,* 1986, vol. 48, 603-16
- **HENIKOFF ; HENIKOFF.** *Proc. Natl. Acad. Sci. USA,* 1992, vol. 89, 10915-19
- **ROBERTSON et al.** *J. Am. Chem. Soc.,* 1991, vol. 113, 2722
- **ELLMAN et al.** *Methods Enzymol,* 1991, vol. 202, 301
- **CHUNG et al.** *Science,* 1993, vol. 259, 806-9
- **CHUNG et al.** *Proc. Natl. Acad. Sci. USA,* 1993, vol. 90, 10145-9
- **TURCATTI et al.** *J. Biol. Chem.,* 1996, vol. 271, 19991-8
- **KOIDE et al.** *Biochem,* 1994, vol. 33, 7470-6
- **WYNN ; RICHARDS.** *Protein Sci.,* 1993, vol. 2, 395-403
- **CUNNINGHAM ; WELLS.** *Science,* 1989, vol. 244, 1081-5
- **DE VOS et al.** *Science,* 1992, vol. 255, 306-12
- **SMITH et al.** *J. Mol. Biol,* 1992, vol. 224, 899-904
- **WLODAVER et al.** *FEBS Lett.,* 1992, vol. 309, 59-64
- **REIDHAAR-OLSON ; SAUER.** *Science,* 1988, vol. 241, 53-7
- **BOWIE ; SAUER.** *Proc. Natl. Acad. Sci. USA,* 1989, vol. 86, 2152-6
- **LOWMAN et al.** *Biochem.,* 1991, vol. 30, 10832-7
- **DERBYSHIRE et al.** *Gene,* 1986, vol. 46, 145
- **NER et al.** *DNA,* 1988, vol. 7, 127